(19) Europäisches Patentamt
European Patent Office
Office européen des brevets

(11) EP 1 982 970 A1

(12) EUROPEAN PATENT APPLICATION

(43) Date of publication:
**22.10.2008 Bulletin 2008/43**

(21) Application number: **08251149.4**

(22) Date of filing: **28.03.2008**

(51) Int Cl.:
***C07C 35/44*** *(2006.01)* ***C07C 61/125*** *(2006.01)*
***C07C 207/02*** *(2006.01)* ***C07C 211/19*** *(2006.01)*
***C07C 211/38*** *(2006.01)* ***C07C 233/06*** *(2006.01)*
***C07C 233/41*** *(2006.01)* *A61K 31/16 (2006.01)*
*A61K 31/13 (2006.01)* *A61K 31/045 (2006.01)*
*A61K 31/19 (2006.01)* *A61P 25/28 (2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **17.04.2007 US 787695**

(71) Applicant: **Chevron U.S.A. Inc.**
**San Ramon, CA 94583-2324 (US)**

(72) Inventors:
- **LIU, Shenggao**
  **Hercules, CA 94547 (US)**
- **LAM, Frederick W.**
  **Piedmont, CA 94611 (US)**
- **SCIAMANNA, Steven F.**
  **Orinda, CA 94563 (US)**
- **CARLTON, Robert M.**
  **Petaluma, CA 94952 (US)**
- **DAHL, Jeremy E.**
  **Palo Alto, CA 94305 (US)**

(74) Representative: **Nash, David Allan**
**HASELTINE LAKE**
**Redcliff Quay**
**120 Redcliff Street**
**Bristol BS1 6HU (GB)**

(54) **Diamondoid derivatives possessing therapeutic activity in the treatment of neurologic disorders**

(57) This invention relates to diamondoid derivatives which exhibit therapeutic activity. Specifically, the diamondoid derivatives herein exhibit therapeutic effects in the treatment of neurologic disorders. Also provided are methods of treatment, prevention and inhibition of neurologic disorders in a subject in need.

EP 1 982 970 A1

Printed by Jouve, 75001 PARIS (FR)

## Description

## BACKGROUND OF THE INVENTION

This application is a continuation-in-part of U.S. application Serial No. 11/430,464, filed May 8, 2006 which claims priority to U.S. Provisional Application No. 60/678,169, filed May 6, 2005 and U.S. Provisional Application No. 60/782,265, filed March 15, 2006 all of which are herein incorporated by reference in their entirety.

### Field of the Invention

This invention relates to diamondoid derivatives which exhibit therapeutic activity. Specifically, the diamondoid derivatives herein exhibit therapeutic effects in the treatment of neurologic disorders. Also provided are methods of treatment, prevention and inhibition of neurologic disorders in a subject in need.

### State of the Art

Neurologic disorders are among the most common in clinical medicine. Neurologic disorders can affect perception, memory, cognitive function, interaction with others, cause disturbances in language, and cause symptoms affecting the brain, spinal cord, nerves, and muscles. More serious neurologic disorders cause seizure, coma, loss of mobility, chronic pain, and even death. For example, in Western countries, stroke is the third most common cause of death and the second most common cause of neurologic disability after Alzheimer's disease. Neurologic disease remains the leading cause of institutional placement for loss of independence among adults. HARRISON'S PRINCIPLES OF INTERNAL MEDICINE, Isselbacher ed. 13th Ed. (1994) New York: McGraw-Hill, Inc.: 2203-2204.

Diamondoids are cage-shaped hydrocarbon molecules possessing rigid structures, resembling tiny fragments of a diamond crystal lattice. *See* Fort, Jr., et al., Adamantane: Consequences of the Diamondoid Structure, Chem. Rev., 64:277-300 (1964). Adamantane is the smallest member of the diamondoid series and consists of one diamond crystal subunit. Diamantane contains two diamond subunits, triamantane contain three, and so on.

Adamantane, which is currently commercially available, has been studied extensively with regard to thermodynamic stability and functionalization, as well as to properties of adamantane containing materials. It has been found that derivatives containing adamantane have certain pharmaceutical uses, including anti-viral properties and uses as blocking agents and protecting groups in biochemical syntheses. For example, *alpha*-methyl-1-adamantanemethylamine hydrochloride (Flumadine® (remantidine) Forest Pharmaceuticals, Inc.) and 1-aminoadamantane hydrochloride (Symmetrel® (amantadine) Endo Laboratories, Inc.) may be used to treat influenza. Adamantanes are also useful in the treatment of Parkinson diseases.

However, though research has addressed the application of adamantane derivatives, studies on derivatives of the other two lower diamondoids (diamantane or triamantane) are very limited. U.S. Patent No. 5,576,355 discloses the preparation of adamantane and diamantane alcohol, ketone, ketone derivatives, adamantyl amino acid, quaternary salt or combinations thereof which have antiviral properties. U.S. Patent No. 4,600,782 describes the preparation of substituted spiro[oxazolidine-5,2'-adamantane] compounds useful as antiinflammatory agent. U.S. Patent No. 3,657,273 discloses the preparation of antibiotic adamantane-1,3-dicarboxamides having antibacterial, antifungal, antialgal, antiprotozoal, and antiinflammatory properties, as well as having analgesic and antihypertensive properties.

A large body of evidence has shown that the neurotransmitter glutamate is a key mediator involved in both normal functions of the brain (e.g., movement, learning and memory) and in pathological damage (e.g., chronic and acute neurotoxicity, such as cell death following Alzheimer's dementia and stroke, respectively). Low affinity, uncompetitive inhibitors blocking the ion channel pore of glutaminergic NMDA receptors, such as the adamantane derivative memantine, have shown efficacy in treating a variety of neurological disorders. However, the therapeutic window between inhibition of pathological excess glutamate receptor activity and interference with normal glutaminergic function is narrow. New agents, compositions and methods for using these agents and compositions that inhibit and treat neurologic disorders are needed, which can be used alone or in combination with other agents.

### Structure Function and Pharmacology of Glutamate Receptors

Among the many chemicals mediating synaptic transmission between neurons, glutamate has secured a place as the primary excitatory neurotransmitter. Studies on the structure, function and pharmacology of glutamate receptors have shown that they are large multi-subunit transmembrane proteins that are subject to multiple, interacting types of regulation. They can be divided into two major families: ionotrophic and metabotrophic. The ionotrophic family is composed of three major pharmacologically and genetically defined sub-families of ligand-gated ion channels known as AMPA receptors (4 genes: GluR1-4), kainate receptors (5 genes: GluR5-7, KA1 and KA2) and NMDA receptors (7

genes: NR1, NR2A-D, NR3A and NR3B). The NMDA receptors (NRs) are unique in requiring two obligatory co-agonists, glutamate (binds NR2) and glycine (binds NR1), in order to open the ion channel and permit an influx of Ca++ ions. The channel opening, or gating, is affected by binding of a number allosteric modulators: high affinity inhibition by Zn++ (NR2A), current enhancement by low concentrations of polyamines such as spermine (NR1). Both of these effects are pH dependent (H+ ion effect) [reviewed in Mayer, M. L. and N. Armstrong (2004). "Structure and function of glutamate receptor ion channels." Annu Rev Physiol 66: 161-81.; Herin, G. A. and E. Aizenman (2004). "Amino terminal domain regulation of NMDA receptor function." Eur J Pharmacol 500(1-3): 101-11.; Mayer, M. L. (2005). "Glutamate receptor ion channels." Curr Opin Neurobiol 15(3): 282-8.; Kew, J. N. and J. A. Kemp (2005). "Ionotropic and metabotropic glutamate receptor structure and pharmacology." Psychopharmacology(Berl) 179(1): 4-29.; Watkins, J. C. and D. E. Jane (2006). "The glutamate story." Br J Pharmacol 147 Suppl 1: S100-8.]. Furthermore, inhibition of resting NRs by endogenous Mg++ binding in the pore channel provide the characteristic voltage dependence of Ca++ ion flow, although the mechanistic details are still controversial [MacDonald 2006; Qian 2005;Vargas-Caballero 2004].

Evidence now suggests that the original concept of ligand binding leading to channel opening and ion passage is too simplistic. Rather the combined effect of multiple ligands and effectors produces a variety of partially to fully activated receptors with different conformations resulting in different Ca++ channel characteristics, all of which are kinetically interconvertable. Evidence includes a combination of binding and functional assays that combine pharmacologic agents and recombinant receptors with either specific protein mutations in the agonist sites (glycine and glutamate) [Kalbaugh, T. L., H. M. VanDongen, et al. (2004). "Ligand-binding residues integrate affinity and efficacy in the NMDA receptor." Mol Pharmacol 66(2): 209-19.; Chen, P. E. and D. J. Wyllie (2006). "Pharmacological insights obtained from structure-function studies of ionotropic glutamate receptors." Br J Pharmacol. 147: 839.] or the use of single channel recording techniques [reviewed by Gibb, A. J. (2004). "NMDA receptor subunit gating--uncovered." Trends Neurosci 27 (1): 7-10; discussion 10.; Magleby, K. L. (2004). "Modal gating of NMDA receptors." Trends Neurosci 27(5): 231-3.].

The NR sub-family of glutamate receptors in the brain (central nervous system, CNS) is crucial in maintaining normal cognitive functions. These include a) declarative memory (conscious recollection of autobiographical events or facts), including consolidation of memory from visual recognition or spatial learning; b) associative conditioning (such as spatial learning in a water-maze escape task), including acquisition (encoding / consolidation) of appetitive and aversive conditioning or extinction (when the reinforcer, e.g. food or shock, associated with learning a particular task or response is withdrawn), but not maintenance of already established performance, and; c) executive functions, such as retrieval (working memory) and discriminative learning [Robbins & Murphy 2006].

During the same time period that the role of glutamate as a key excitatory neurotransmitter was being discovered, its role in 'excitotoxicity' was also being defined. The first experimental demonstration of the phenomena was in 1957 by Lucas and Newhouse who injected glutamate subcutaneously into animals and found specific damage to retinal ganglion cells, although the term 'excitotoxicity' was not developed until over 10 years later by Olney. Since then, it has been seen that a variety of insults to the brain, from acute head trauma and stroke to chronic progressive dementias such as Alzheimer's and Parkinson's, involve excessive extracellular accumulation of glutamate and thus over-stimulation of glutamate receptors and neuronal cell death by over accumulation of Ca++. The concept thus arose of neuroprotection as a property of drugs that could antagonize glutamate receptors in order to limit excitotoxicity and preserve neuronal function. Based on these fundamental studies a variety of therapeutic applications are being pursued for antagonists of glutamate receptors, and NRs in particular [see reviews: Danysz W and Parsons CG, 2002 "Neuroprotective potential of ionotrophic glutamate receptor antagonists Neurotox Res 4: 119; Lipton SA, 2004 "Failures and successes ofNMDA receptor antagonists" NeuroRx 1: 101; Lipton SA, 2006 "Paradigm shift in neuroprotection by NMDA receptor blockade: Memantine and beyond" Nat Rev Drug Disc 5:160].

Despite the biochemical and pharmacologic complexities of how glutamate regulates Ca++ ion flow into and out of neurons, the control of neurotransmission by NR is widely recognized as an important potential means of treating many neurologic disorders. Perhaps the most fruitful pharmacologic regulation of NR channel activity to date has come from drugs that interact directly with the channel pore. Over a dozen such drugs (see table below) are either undergoing clinical trials or marketed for a variety of indications [Bleich, S., K. Romer, et al. (2003). "Glutamate and the glutamate receptor system: a target for drug action." Int J Geriatr Psychiatry 18(Suppl 1): S33-40.; Tariot PN, Farlow MR, et. al. 2004 Memantine treatment in patients with moderate to severe Alzheimer disease already receiving donepezil: a randomized controlled trial JAMA. Jan 21;291(3):317-24; Foster, A. C. and J. A. Kemp (2006). "Glutamate- and GABA-based CNS therapeutics." Curr Opin Pharmacol 6(1): 7-17.; Muir, K. W. (2006). "Glutamate-based therapeutic approaches: clinical trials with NMDA antagonists." Curr Opin Pharmacol 6(1): 53-60.; Lepeintre JF, et. al. 2005 "Neuroprotective effect of gacyclidine. A multicenter double-blind pilot trial in patients with acute traumatic brain injury" Neurochirurgie 50:83.]. Other clinical studies have involved high affinity, selective glutamate receptor antagonists that show either competitive or noncompetitive binding to the glycine or glutamate co-activation sites, as well as antagonists binding to the polyamine site and subunit selective drugs. Regardless of the mode of inhibition, in most cases the results have shown inadequate therapeutic utility and/or excessive adverse events, both cognitive (hallucinations, delirium, psychosis or coma) and physical (nausea, vomiting or nystagmus) [Rogawski, M. A. (2000). "Low affinity channel blocking (uncompetitive) NMDA receptor antagonists as therapeutic agents--toward an understanding of their favorable tolerability." Amino Acids 19(1): 133-49.; Calabresi, P., D. Centonze, et al. (2003). "Ionotropic glutamate receptors: still a target for neuroprotection in brain ischemia? Insights from in vitro studies." Neurobiol Dis 12(1): 82-8.; Hoyte, L., P. A. Barber, et al. (2004). "The rise and fall of NMDA antagonists for ischemic stroke." Curr Mol Med 4(2): 131-6.; Johnson, J. W. and S. E. Kotermanski (2006). "Mechanism of action of memantine." Curr Opin Pharmacol 6(1): 61-7.; Lipton, S. A. (2006). "Paradigm shift in neuroprotection by NMDA receptor blockade: memantine and beyond." Nat Rev Drug Discov 5(2): 160-70.]. Inhibitors with the best clinical profiles were most often found to be low affinity, uncompetitive channel blockers. In the table below these are memantine, remacemide, budipine, amantadine, AR-R15896AR, gacyclidine, MRZ 2/579, ketamine, dextromethorphan, and ADCI.

Table

| Category | Disease | Drug (Trade Name) | Company |
|---|---|---|---|
| **Neurodegenerative** | Alzheimer’s | Memantine (Namenda) | Forest Pharmaceuticals, Inc. |
| | Parkinson’s | Remacemide | AstraZeneca |
| | | Budipine | Byk Gulden |
| | | Amantadine (Symmetrel) | Endo Laboratories, Inc. |
| | Stroke | AR-R15896AR, was ARL 15896AR) | AstraZeneca |
| | Stroke, Traumatic Brain Injury (TBI) | CNS 1102, Aptiganel (Cerestat) | Cambridge Neurosciences |
| | TBI | HU 211 (Dexanabinol) | Pharmos |
| | | GT11 (Gacyclidine) | Beaufour-Ipsen |
| | Huntington’s | Remacemide | AstraZeneca |
| | | Memantine (Namenda) | Forest Pharmaceuticals, Inc. |
| **Psychiatric** | Substance Abuse | MRZ 2/579 (Neramexane) | Forest / Merz |
| **Pain** | Neuropathic Pain | Ketamine (Ketalar) | Parke-Davis |
| | | Dextropmethorphan | many |
| | | Memantine (Namenda) | Forest Pharmaceuticals, Inc. |
| | | CNS 5101 | Cambridge Neuroscience |
| **Epilepsy** | | ADCI | NIH, |

Despite these advances, problems still persist. For instance, a meta analysis of the clinical studies for several forms of dementia dementia [Areosa SA, Sherriff F, McShane R. 2005 Memantine for dementia. Cochrane Database Syst Rev. Jul 20;(3):CD003154.] finds only a small or transitory beneficial effect. A similar meta study found insufficient evidence for the safety and efficacy of amantadine, an adamantane derivative, in treatment of idiopathic Parkinson’s Disease [Crosby N, Deane KH, Clarke CE. 2003 Amantadine in Parkinson’s disease Cochrane Database Syst Rev.; (1):CD003468.]. Although some success for ketamine and dextromethorphan in decreasing the use of opioids or local anesthetics has been found, including the novel approach of adding a co-drug to decrease dextromethorphan metabolism and thus decrease the dose, other studies found no consistent effect with these agents. [Legge J et, al. 2006 "The potential role of ketamine in hospice analgesia: a literature review" Consult Pharm 21: 51-7; Yeh CC, et. al. 2005, "Preincisional dextromethorphan combined with thoracic epidural anesthesia and analgesia improves postoperative pain and bowel function in patients undergoing colonic surgery" Anesth Analg 100: 1384-9.; Wu CT, et. al. 2005, "The interaction effect of perioperative cotreatment with dextromethorphan and intravenous lidocaine on pain relief and recovery of bowel function after laparoscopic cholecystectomy" Anesth Analg 100: 448-53. Weinbroum AA and Ben-Abraham R, 2001 "Dextromethorphan and dexmedetomidine: new agents for the control of perioperative pain" Eur J Surg. 167: 563-9; Duedahl, T H et. al. 2006 "A qualitative systematic review of perioperative dextromethorphan in post-operative pain." Acta Anaesthesiol Scand 50: 1-13; Hempenstall K, 2005 "Analgesic therapy in postherpetic neuralgia: a quantitative systematic review" PLoS Med 2: et al; Galer BS, et. al. 2005 "MorphiDex (morphine sulfate/dextromethorphan hydrobromide combination) in the treatment of chronic pain: three multicenter, randomized, double-blind, controlled clinical trials fail to demonstrate enhanced opioid analgesia or reduction in tolerance" Pain 115: 284-95; [(anon)

2005 "Dextromethorphan/quinidine: AVP 923, dextromethorphan/cytochrome P450-2D6 inhibitor, quinidine/dextromethorphan" Drugs R D. 6(3): 174-7]. Evidence of neuroprotection in models mimicking nerve-agent induced seizures, as may occur in wartime or from terrorist attack, has been obtained for memantine, MK-801, ketamine, gacyclidine (GK11) and HU-211 in animal models, but higher doses of MK-801 induced neuronal degeneration in some brain areas [Filbert J el. al. 2005 Med Chem Biol Radiol Def online at http://jmedhchemdef.org].

Thus the need remains for improved agents, compositions and methods for using these agents and compositions that inhibit and treat neurologic disorders, which can be used alone or in concert with other agents.

## SUMMARY OF THE INVENTION

The present invention provides diamondoid derivatives which exhibit pharmaceutical activity in the treatment, inhibition, and prevention of neurologic disorders. In particular, the present invention relates to derivatives of diamantane and triamantane, which may be used in the treatment, inhibition, and prevention of neurologic disorders. In its composition aspects, diamantane derivatives within the scope of the present invention include compounds of Formula I and II and triamantane derivatives within the scope of the present invention include compounds of Formula III.

In one of its composition aspects, this invention is directed to a compound of Formula I:

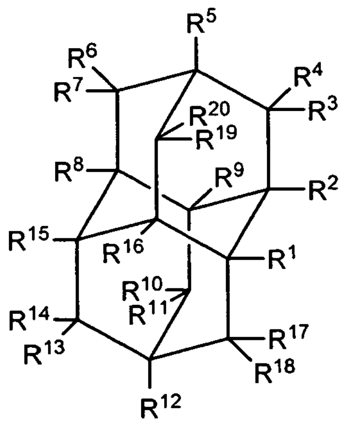

Formula I

wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;
provided that at least two of $R^1$, $R^2$, $R^5$, $R^8$, $R^9$, $R^{12}$, $R^{15}$, and $R^{16}$ are not hydrogen; and
that both $R^5$ and $R^{12}$ or $R^1$ and $R^8$ are not identical when the remaining of $R^1$, $R^2$, $R^8$, $R^9$, $R^{15}$, and $R^{16}$ are hydrogen;
and pharmaceutically acceptable salts thereof.

In another of its composition aspects, this invention is directed to a compound of Formula I wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;
provided that at least two of $R^1$, $R^2$, $R^5$, $R^8$, $R^9$, $R^{12}$, $R^{15}$, and $R^{16}$ are not hydrogen; and
that both $R^5$ and $R^{12}$ are not identical when $R^1$, $R^2$, $R^8$, $R^9$, $R^{15}$ and $R^{16}$ are hydrogen; and
that both $R^1$ and $R^8$ are not identical when $R^2$, $R^5$, $R^9$, $R^{12}$, $R^{15}$ and $R^{16}$ are hydrogen;
and pharmaceutically acceptable salts thereof.

In one embodiment of the compounds of Formula I, at least three of $R^1$, $R^2$, $R^5$, $R^8$, $R^9$, $R^{12}$, $R^{15}$, and $R^{16}$ are not hydrogen. In another embodiment of the compounds of Formula I, at least four of $R^1$, $R^2$, $R^5$, $R^8$, $R^9$, $R^{12}$, $R^{15}$, and $R^{16}$ are not hydrogen. In yet another embodiment of the compounds of Formula I, five of $R^1$, $R^2$, $R^5$, $R^8$, $R^9$, $R^{12}$, $R^{15}$,

and $R^{16}$ are not hydrogen.

In one preferred embodiment of the compounds of Formula I, $R^1$ and $R^5$ are aminoacyl and $R^2$, $R^8$, $R^9$, $R^{12}$, $R^{15}$, and $R^{16}$ are hydrogen or lower alkyl. In another preferred embodiment of the compounds of Formula I, $R^5$ is amino and two of $R^1$, $R^2$, $R^8$ and $R^{15}$ are lower alkyl, preferably methyl. In yet another embodiment of the compounds of Formula I, $R^5$ is amino and two of $R^1$, $R^2$, $R^8$ and $R^{15}$ are lower alkyl. In a preferred embodiment $R^1$ and $R^8$ are methyl and in another preferred embodiment $R^1$ and $R^{15}$ are methyl.

In a further embodiment of the compounds of Formula I, $R^9$ or $R^{15}$ is amino and $R^1$ is methyl. In another embodiment of the compounds of Formula I, $R^2$ or $R^{16}$ is amino and $R^1$ and $R^8$ are methyl.

In another embodiment of the compounds of Formula I, at least one of $R^1$, $R^2$, $R^5$, $R^8$, $R^9$, $R^{12}$, $R^{15}$, and $R^{16}$ is independently selected from the group consisting of amino, nitroso, nitro, and aminoacyl and at least one of the remaining of $R^1$, $R^2$, $R^5$, $R^8$, $R^9$, $R^{12}$, $R^{15}$, and $R^{16}$ are lower alkyl. In a preferred embodiment, at least two of the remaining of $R^1$, $R^2$, $R^5$, $R^8$, $R^9$, $R^{12}$, $R^{15}$, and $R^{16}$ are lower alkyl. In another preferred embodiment, three of the remaining of $R^1$, $R^2$, $R^5$, $R^8$, $R^9$, $R^{12}$, $R^{15}$, and $R^{16}$ are lower alkyl.

In an embodiment of the compounds of Formula I, at least one of $R^5$ and $R^{12}$ is independently selected from the group consisting of amino, nitroso, nitro, and aminoacyl and at least one of $R^1$, $R^2$, $R^8$, $R^9$, $R^{15}$, and $R^{16}$ is lower alkyl. In a preferred embodiment, at least two of $R^1$, $R^2$, $R^8$, $R^9$, $R^{15}$, and $R^{16}$ are lower alkyl. In another preferred embodiment, three of $R^1$, $R^2$, $R^8$, $R^9$, $R^{15}$, and $R^{16}$ are lower alkyl.

In an embodiment of the compounds of Formula I, at least one of $R^1$, $R^2$, $R^5$, $R^8$, $R^9$, $R^{12}$, $R^{15}$, and $R^{16}$ is substituted lower alkyl. In a preferred embodiment, two of $R^1$, $R^2$, $R^5$, $R^8$, $R^9$, $R^{12}$, $R^{15}$, and $R^{16}$ are substituted lower alkyl.

In another embodiment of the compounds of Fomula I, at least one of $R^1$, $R^2$, $R^5$, $R^8$, $R^9$, $R^{12}$, $R^{15}$, and $R^{16}$ is substituted lower alkyl and at least one of the remaining of $R^1$, $R^2$, $R^5$, $R^8$, $R^9$, $R^{12}$, $R^{15}$, and $R^{16}$ are independently selected from the group consisting of amino, nitroso, nitro, and aminoacyl.

In another of its composition aspects, this invention is directed to a compound of Formula II:

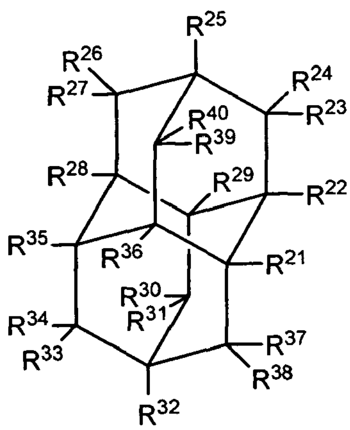

Formula II

wherein:

$R^{21}$, $R^{22}$, $R^{25}$, $R^{28}$, $R^{29}$, $R^{32}$, $R^{35}$, and $R^{36}$ are independently selected from the group consisting of hydrogen or substituted lower alkyl;
$R^{23}$, $R^{24}$, $R^{26}$, $R^{27}$, $R^{30}$, $R^{31}$, $R^{33}$, $R^{34}$, $R^{37}$, $R^{38}$, $R^{39}$, and $R^{40}$ are hydrogen;
provided that at least at least one of $R^{21}$, $R^{22}$, $R^{25}$, $R^{28}$, $R^{29}$, $R^{32}$, $R^{35}$, and $R^{36}$ is substituted lower alkyl;
and pharmaceutically acceptable salts thereof.

In a preferred embodiment of the compounds of Formula II, the substituted lower alkyl group is substituted with one substitutent selected from the group consisting of amino, hydroxy, halo, nitroso, nitro, carboxy, acyloxy, acyl, aminoacyl, and aminocarbonyloxy. In a more preferred embodiment of the compounds of Formula II, the substituted lower alkyl group is substituted with one substitutent selected from the group consisting of amino, nitroso, nitro, and aminoacyl.

In one embodiment of the compounds of Formula II, $R^{25}$ is substituted lower alkyl and $R^{21}$, $R^{22}$, $R^{28}$, $R^{29}$, $R^{32}$, $R^{35}$, and $R^{36}$ are hydrogen.

In another embodiment of the compounds of Formula II, $R^{25}$ and $R^{32}$ are substituted lower alkyl.

In yet another embodiment of the compounds of Formula II, $R^{21}$ is substituted lower alkyl and $R^{22}$, $R^{25}$, $R^{28}$, $R^{29}$, $R^{32}$, $R^{35}$, and $R^{36}$ are hydrogen.

In one embodiment of the compounds of Formula II, $R^{25}$ and $R^{21}$ are substituted lower alkyl.

In another embodiment of the compounds of Formula II, $R^{32}$ and $R^{21}$ are substituted lower alkyl.

In another of its composition aspects, this invention is directed to a compound having the structure:

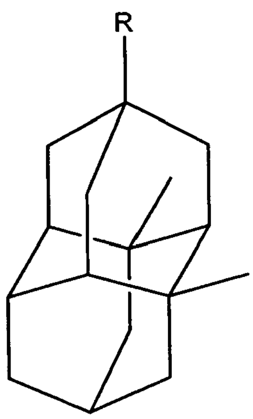
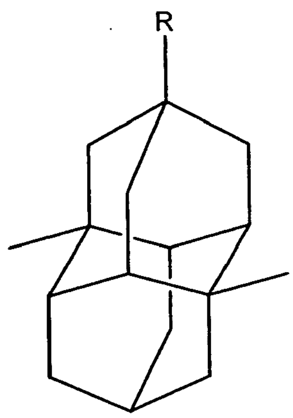
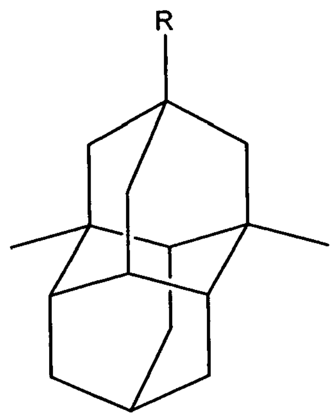
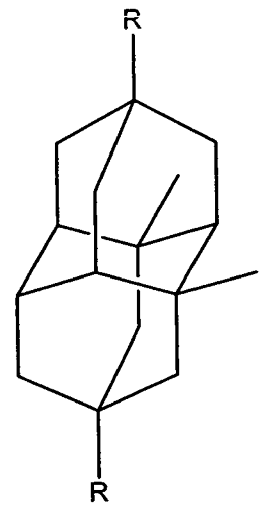
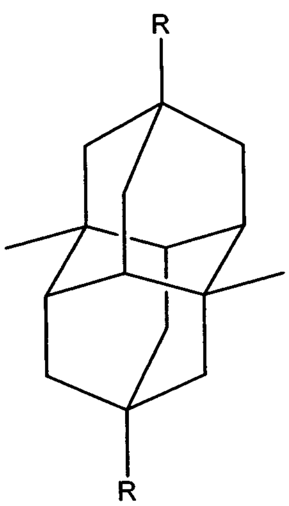
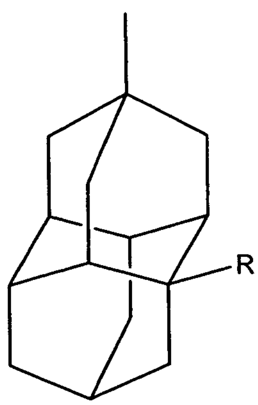
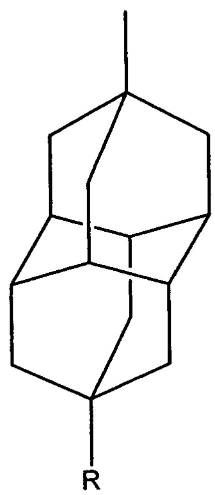
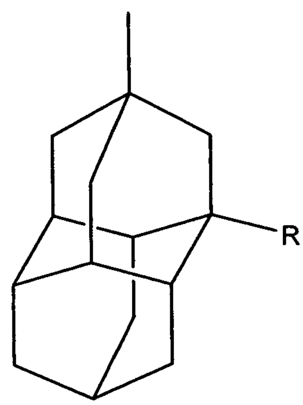

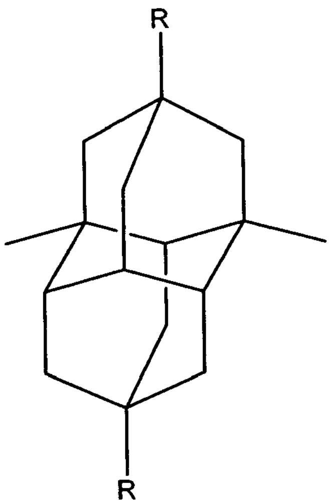

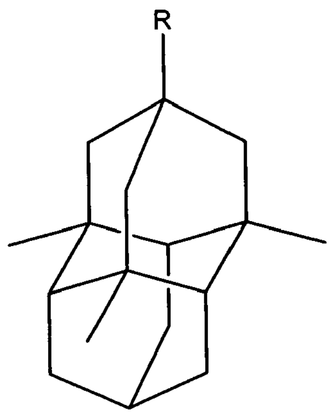

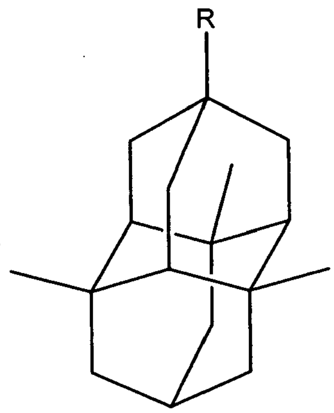

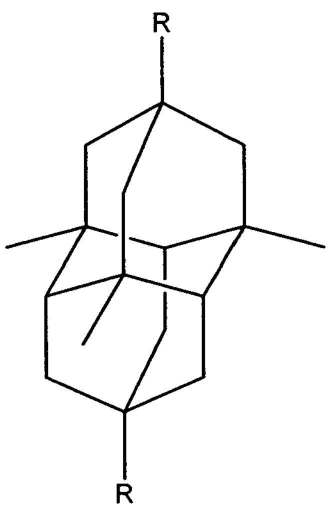

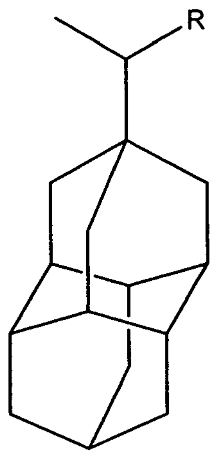

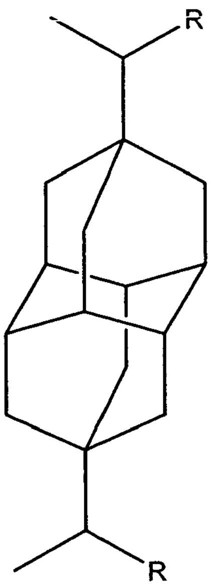

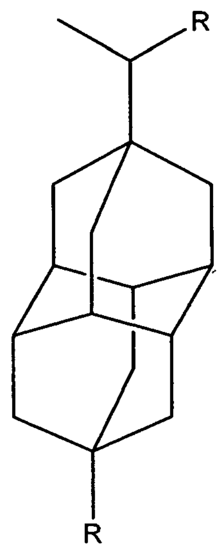

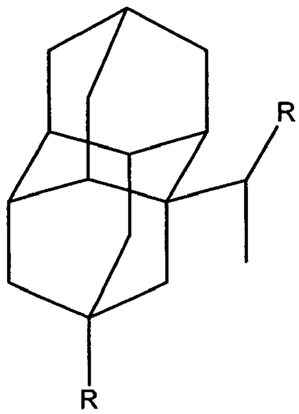
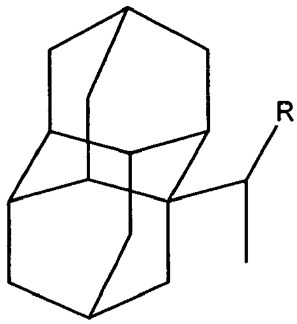
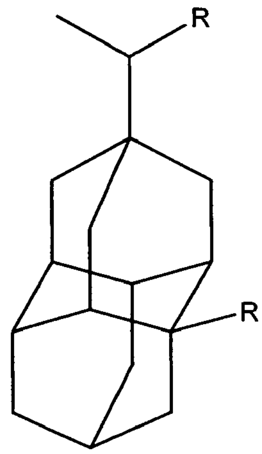
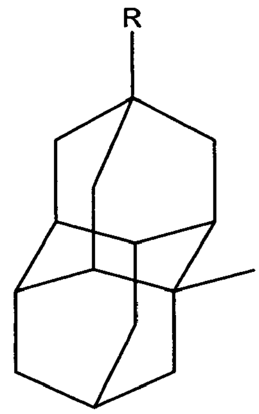

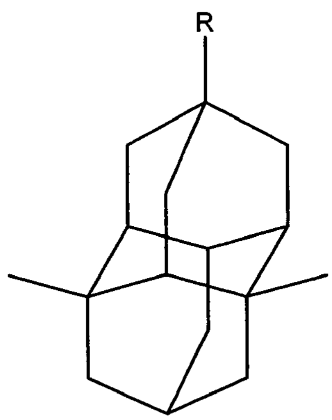
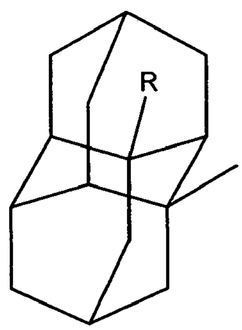
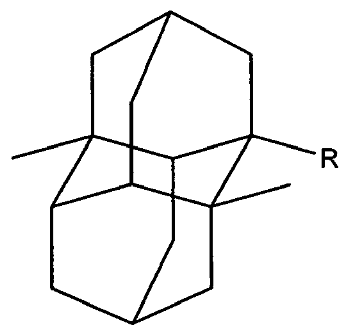

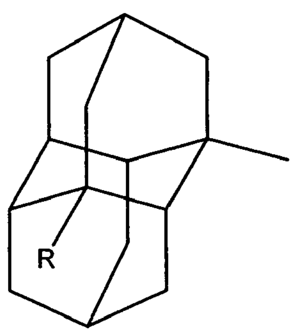
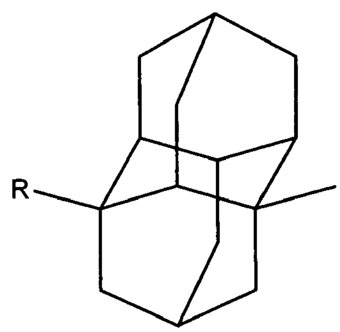
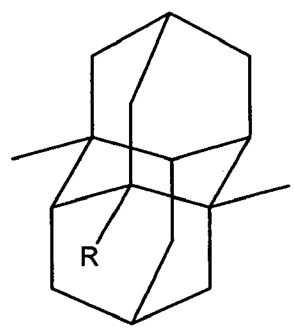

or

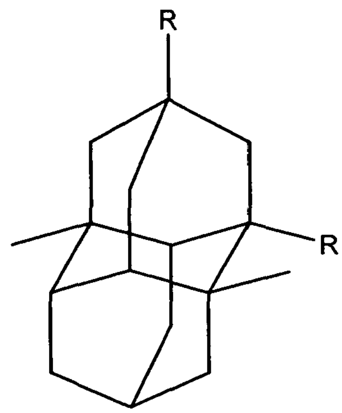

wherein R is independently hydroxy, carboxy, amino, nitroso, nitro or aminoacyl. In one embodiment of the above compounds, R is hydroxy or carboxy. In another embodiment of the above compounds, R is independently amino, nitroso, nitro or aminoacyl. In a preferred embodiment, R is amino or aminoacyl.

In yet another of its composition aspects, this invention is directed to a compound of Formula III:

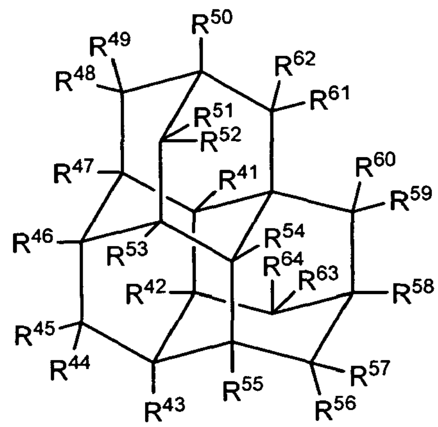

Formula III

wherein:

$R^{41}$, $R^{42}$, $R^{43}$, $R^{46}$, $R^{47}$, $R^{50}$, $R^{53}$, $R^{54}$, $R^{55}$, and $R^{58}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^{44}$, $R^{45}$, $R^{48}$, $R^{49}$, $R^{51}$, $R^{52}$, $R^{56}$, $R^{57}$, $R^{59}$, $R^{60}$, $R^{61}$, $R^{62}$, $R^{63}$, and $R^{64}$ are hydrogen;
provided that at least one of $R^{41}$, $R^{42}$, $R^{43}$, $R^{46}$, $R^{47}$, $R^{50}$, $R^{53}$ , $R^{54}$, $R^{55}$, and $R^{58}$ is not hydrogen;
and pharmaceutically acceptable salts thereof.

In one embodiment of the compounds of Formula III, at least two of $R^{41}$, $R^{42}$, $R^{43}$, $R^{46}$, $R^{47}$, $R^{50}$, $R^{53}$, $R^{54}$, $R^{55}$, and $R^{58}$ are not hydrogen. In another embodiment of the compounds of Formula III, at least three of $R^{41}$, $R^{42}$, $R^{43}$, $R^{46}$, $R^{47}$, $R^{50}$, $R^{53}$, $R^{54}$, $R^{55}$, and $R^{58}$ are not hydrogen.

In one embodiment of the compounds of Formula III, $R^{50}$ is selected from the group consisting of amino, nitroso, nitro, and aminoacyl and at least one of $R^{41}$, $R^{42}$, $R^{43}$, $R^{46}$, $R^{47}$, $R^{50}$, $R^{53}$, $R^{54}$, $R^{55}$, and $R^{58}$ is lower alkyl. In a preferred embodiment, at least two of $R^{41}$, $R^{42}$, $R^{43}$, $R^{46}$, $R^{47}$, $R^{50}$, $R^{53}$, $R^{54}$, $R^{55}$, and $R^{58}$ are lower alkyl.

In another aspect, this invention provides for a method for treating a neurologic disorder in a subject in need thereof, comprising administering a therapeutically effective amount of a compound of Formula Ia:

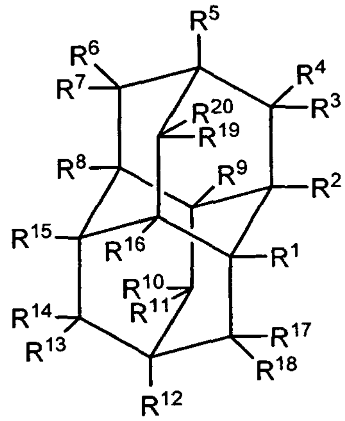

Formula Ia

wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;

$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;

provided that at least one of $R^1$, $R^2$, $R^5$, $R^8$, $R^9$, $R^{12}$, $R^{15}$, and $R^{16}$ are not hydrogen;

and pharmaceutically acceptable salts thereof.

In one embodiment of the compounds of Formula Ia, at least two of $R^1$, $R^2$, $R^5$, $R^8$, $R^9$, $R^{12}$, $R^{15}$, and $R^{16}$ are not hydrogen. In another embodiment of the compounds of Formula Ia, at least three of $R^1$, $R^2$, $R^5$, $R^8$, $R^9$, $R^{12}$, $R^{15}$, and $R^{16}$ are not hydrogen. In another embodiment of the compounds of Formula I, at least four of $R^1$, $R^2$, $R^5$, $R^8$, $R^9$, $R^{12}$, $R^{15}$, and $R^{16}$ are not hydrogen. In yet another embodiment of the compounds of Formula I, five of $R^1$, $R^2$, $R^5$, $R^8$, $R^9$, $R^{12}$, $R^{15}$, and $R^{16}$ are not hydrogen.

In another embodiment of the compounds of Formula Ia, $R^1$ and $R^5$ are aminoacyl and $R^2$, $R^8$, $R^9$, $R^{12}$, $R^{15}$, and $R^{16}$ are hydrogen or lower alkyl. In another preferred embodiment of the compounds of Formula Ia $R^5$ is amino and two of $R^1$, $R^2$, $R^8$ and $R^{15}$ are lower alkyl, preferably methyl. In yet another embodiment of the compounds of Formula Ia $R^5$ is amino and two of $R^1$, $R^2$, $R^8$ and $R^{15}$ are lower alkyl. In a further embodiment of the compounds of Formula Ia, $R^9$ or $R^{15}$ is amino and $R^1$ is methyl. In another embodiment of the compounds of Formula Ia, $R^2$ is amino, $R^1$ is methyl, and $R^8$ or $R^{15}$ is methyl.

In another embodiment of the compounds of Formula Ia, at least one of $R^1$, $R^2$, $R^5$, $R^8$, $R^9$, $R^{12}$, $R^{15}$, and $R^{16}$ is independently selected from the group consisting of amino, nitroso, nitro, and aminoacyl and at least one of the remaining of $R^1$, $R^2$, $R^5$, $R^8$, $R^9$, $R^{12}$, $R^{15}$, and $R^{16}$ are lower alkyl. In a preferred embodiment, at least two of the remaining of $R^1$, $R^2$, $R^5$, $R^8$, $R^9$, $R^{12}$, $R^{15}$, and $R^{16}$ are lower alkyl. In another preferred embodiment, three of the remaining of $R^1$, $R^2$, $R^5$, $R^8$, $R^9$, $R^{12}$, $R^{15}$, and $R^{16}$ are lower alkyl.

In one embodiment of the compounds of Formula Ia, at least one of $R^5$ and $R^{12}$ is independently selected from the group consisting of amino, nitroso, nitro, and aminoacyl and at least one of $R^1$, $R^2$, $R^8$, $R^9$, $R^{15}$, and $R^{16}$ is lower alkyl. In a preferred embodiment, at least two of $R^1$, $R^2$, $R^8$, $R^9$, $R^{15}$, and $R^{16}$ are lower alkyl. In another preferred embodiment, three of $R^1$, $R^2$, $R^8$, $R^9$, $R^{15}$, and $R^{16}$ are lower alkyl.

In one embodiment of the compounds of Formula Ia, at least one of $R^1$, $R^2$, $R^5$, $R^8$, $R^9$, $R^{12}$, $R^{15}$, and $R^{16}$ is substituted lower alkyl. In a preferred embodiment, two of $R^1$, $R^2$, $R^5$, $R^8$, $R^9$, $R^{12}$, $R^{15}$, and $R^{16}$ are substituted lower alkyl. In another preferred embodiment, $R^5$ is substituted lower alkyl and $R^1$, $R^2$, $R^8$, $R^9$, $R^{12}$, $R^{15}$, and $R^{16}$ are hydrogen. In yet another preferred embodiment, $R^5$ and $R^{12}$ are substituted lower alkyl. In another preferred embodiment, $R^1$ is substituted lower alkyl and $R^2$, $R^5$, $R^8$, $R^9$, $R^{12}$, $R^{15}$, and $R^{16}$ are hydrogen. In yet another preferred embodiment, $R^5$ and $R^1$ are substituted lower alkyl. In another embodiment, $R^{12}$ and $R^1$ are substituted lower alkyl.

In one embodiment of the compounds of Formula Ia, at least one of $R^1$, $R^2$, $R^5$, $R^8$, $R^9$, $R^{12}$, $R^{15}$, and $R^{16}$ is substituted lower alkyl and at least one of the remaining of $R^1$, $R^2$, $R^5$, $R^8$, $R^9$, $R^{12}$, $R^{15}$, and $R^{16}$ are independently selected from the group consisting of amino, nitroso, nitro, and aminoacyl.

In a preferred embodiment of the compounds of Formula Ia, the substituted lower alkyl group is substituted with one substitutent selected from the group consisting of amino, hydroxy, halo, nitroso, nitro, carboxy, acyloxy, acyl, aminoacyl, and aminocarbonyloxy. In a more preferred embodiment, the substituted lower alkyl group is substituted with one substitutent selected from the group consisting of amino, nitroso, nitro, and aminoacyl.

In yet another aspect, this invention provides for a method for treating a neurologic disorder in a subject in need thereof, comprising administering a therapeutically effective amount of a compound of Formula III as defined above.
In a preferred embodiment, the neurologic disorder is epilepsy, narcolepsy, neurodegenerative disorders, pain, and psychiatric disorders. Preferably, the neurodegenerative disorder may include Alzheimer's Disease, Parkinson's Disease, stroke, AIDS related dementia, traumatic brain injury (TBI), and Huntington's Disease. Preferably, the psychiatric disorder is substance abuse.
In another aspect, this invention provides pharmaceutical compositions comprising a pharmaceutically acceptable carrier and a therapeutically effective amount of the compounds defined herein.
In yet another aspect, the present invention provides processes for preparing compounds of Formula I, Ia, II, and III.

## BRIEF DESCRIPTION OF THE FIGURES

FIG. 1 illustrates synthetic pathways by which diamantane may be derivatized to provide a compound according to the present invention.
FIGs. 2-16 illustrate synthetic pathways by which derivatized diamantane and triamantane compounds may be prepared from diamantane and triamantane.
FIG. 17- is GC MS,$^1$H-NMR, or $^{13}$C-NMR data corresponding to the Examples.
FIG. 18 is the crystal structure of 1,6-dibromodiamantane.
FIGs. 19-32 are GC MS, HPLC, $^1$H-NMR, or $^{13}$C-NMR data corresponding to the Examples.
FIG. 33 shows the effect of diamantine compounds on NMDA evoked currents.
FIGs. 34-47 are GC MS, $^1$H-NMR, $^{13}$C-NMR, $^{13}$C-$^1$H Cosy NMR, Dept-135 NMR or IR data corresponding to the Examples.
FIG. 48 is the crystal structure of 4-amino-1,6-dimethyldiamantane.
FIGs. 49-71 are GC MS, mass spectrometry, HPLC, $^1$H-NMR, $^{13}$C-NMR, $^{13}$C-$^1$H Cosy NMR, $^1$H-$^1$H Cosy NMR, $^1$H-$^{13}$C HMQC NMR, Dept-135 NMR or IR data corresponding to the Examples.
FIG. 72 is the crystal structure of 1-methyl-9-aminodiamantane.
FIGs. 73-74 are GC MS data corresponding to the Examples.
FIG. 75 is the crystal structure of 1-methyl-4-aminodiamantane.
FIGs. 76-84 are GC MS, MRMS, mass spectrometry, $^1$H-NMR, $^{13}$C-NMR, Dept-135 NMR or IR data corresponding to the Examples.
FIG. 85 is the crystal structure of 1-amino-2-methyldiamantane.
FIG. 86 shows the structure of a racemic mixture of 1-amino-2-methyldiamantane and 1-amino-12-methyldiamantane.
FIGs. 87-131 are GC MS, HRMS, mass spectrometry, $^1$H-NMR, $^{13}$C-NMR or Dept-135 NMR data corresponding to the Examples.
FIGs. 132-138 are dose response curves for MDT-22, MDT-23, MDT-24, MDT-30, MDT-43, MDT-50 and MDT-51 in the NMDA-induced LDH release assay.

## DETAILED DESCRIPTION OF THE INVENTION

As described above, this invention relates to diamondoid derivatives which exhibit pharmaceutical activity, useful for the treatment, inhibition, and/or prevention of neurologic conditions. However, prior to describing this invention in further detail, the following terms will first be defined.

Definitions

In accordance with this detailed description, the following abbreviations and definitions apply. It must be noted that as used herein, the singular forms "a", "an", and "the" include plural referents unless the context clearly dictates otherwise. Thus, for example, reference to "compounds" includes a plurality of such compounds and reference to "the dosage" includes reference to one or more dosages and equivalents thereof known to those skilled in the art, and so forth.
The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates, which may need to be independently confirmed.
Unless otherwise stated, the following terms used in the specification and claims have the meanings given below:

"Halo" means fluoro, chloro, bromo, or iodo.
"Nitro" means the group $-NO_2$.
"Nitroso" means the group -NO.
"Hydroxy" means the group =OH.
"Carboxy" means the group -COOH.
"Lower alkyl" refers to monovalent alkyl groups having from 1 to 6 carbon atoms including straight and branched chain alkyl groups. This term is exemplified by groups such as methyl, ethyl, *iso*-propyl, *n*-propyl, *n*-butyl, *iso*-butyl, *sec*-butyl, *t*-butyl, *n*-pentyl and the like.
"Substituted lower alkyl" means an alkyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy. "Lower alkenyl" means a linear unsaturated monovalent hydrocarbon radical of two to six carbon atoms or a branched monovalent hydrocarbon radical of three to eight carbon atoms containing at least one double bond, (-C=C-). Examples of alkenyl groups include, but are not limited to, allyl, vinyl, 2-butenyl, and the like.
"Substituted lower alkenyl" means an alkenyl group with one or more substituents, preferably one to three substituents, wherein the substitutents are selected from the group consisting of amino, nitroso, nitro, halo, hydroxy, carboxy, acyloxy, acyl, aminoacyl, and aminocarbonyloxy.

The term "cycloalkyl" refers to cyclic alkyl groups of from 3 to 6 carbon atoms having a single cyclic ring including, by way of example, cyclopropyl, cyclobutyl, cyclopentyl and cyclohexyl.

"Alkoxy" refers to the group "lower alkyl-O-" which includes, by way of example, methoxy, ethoxy, n-propoxy, isopropoxy, n-butoxy, tert-butoxy, sec-butoxy, n-pentoxy, 1,2-dimethylbutoxy, and the like.
"Amino" refers to the group $NR^ak^b$, wherein $R^a$ and $R^b$ are independently selected from hydrogen, lower alkyl, substituted lower alkyl, and cycloalkyl.
"Acyloxy" refers to the groups H-C(O)O-, lower alkyl-C(O)O-, substituted lower alkyl-C(O)O-, lower alkenyl-C(O)O-, substituted lower alkenyl-C(O)O- and cycloalkyl-C(O)O-, wherein lower alkyl, substituted lower alkyl, lower alkenyl, substituted lower alkenyl, and cycloalkyl are as defined herein.
"Acyl" refers to the groups H-C(O)-, lower alkyl-C(O)-, substituted lower alkyl-C(O)-, lower alkenyl-C(O)-, substituted lower alkenyl-C(O)- , cycloalkyl-C(O)-, wherein lower alkyl, substituted lower alkyl, lower alkenyl, substituted lower alkenyl, and cycloalkyl are as defined herein.
"Aminoacyl" refers to the groups -NRC(O)lower alkyl, -NRC(O)substituted lower alkyl, -NRC(O)cycloalkyl, -NRC(O) lower alkenyl, and -NRC(O)substituted lower alkenyl, wherein R is hydrogen or lower alkyl and wherein lower alkyl, substituted lower alkyl, lower alkenyl, substituted lower alkenyl, and cycloalkyl are as defined herein.
"Aminocarbonyloxy" refers to the groups -NRC(O)O-lower alkyl, -NRC(O)O-substituted lower alkyl, -NRC(O)O-lower alkenyl, -NRC(O)O-substituted lower alkenyl, - NRC(O)O-cycloalkyl, wherein R is hydrogen or lower alkyl and wherein lower alkyl, substituted lower alkyl, lower alkenyl, substituted lower alkenyl, and cycloalkyl are as defined herein.
"Pharmaceutically acceptable carrier" means a carrier that is useful in preparing a pharmaceutical composition that is generally safe, non-toxic and neither biologically nor otherwise undesirable, and includes a carrier that is acceptable for veterinary use as well as human pharmaceutical use. "A pharmaceutically acceptable carrier" as used in the specification and claims includes both one and more than one such carrier.
"Neurologic disorder" or "neurological disorder" means any condition, disease and/or disorder affecting or related to the central nervous system, the brain, nerves, and muscles. These disorders include, but are not limited to, central nervous system disorders, disorders of the brain, disorders of nerves and muscles, psychiatric disorders, chronic fatigue disorders, and alcohol and/or drug dependency.
"Treating" or "treatment" of a disease includes:

(1) preventing the disease, i.e., causing the clinical symptoms of the disease not to develop in a mammal that may be exposed to or predisposed to the disease but does not yet experience or display symptoms of the disease,
(2) inhibiting the disease, *i.e.,* arresting or reducing the development of the disease or its clinical symptoms, or
(3) relieving the disease, *i.e.,* causing regression of the disease or its clinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.
"Pharmaceutically acceptable salt" refers to pharmaceutically acceptable salts of a compound of Formula I which salts are derived from a variety of organic and inorganic counter ions well known in the art and include, by way

of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like. Preferably, the pharmaceutically acceptable salts are of inorganic acid salts, such as hydrochloride.

"Optional" or "optionally" means that the subsequently described event or circumstance may, but need not, occur, and that the description includes instances where the event or circumstance occurs and instances in which it does not. For example, "aryl group optionally mono- or di- substituted with an alkyl group" means that the alkyl may but need not be present, and the description includes situations where the aryl group is mono- or disubstituted with an alkyl group and situations where the aryl group is not substituted with the alkyl group.

The term "mammal" refers to all mammals including humans, livestock, and companion animals.

The compounds of the present invention are generally named according to the IUPAC or CAS nomenclature system. Abbreviations which are well known to one of ordinary skill in the art may be used (e. g. , "Ph" for phenyl, "Me" for methyl, "Et" for ethyl, "h" for hour or hours and "rt" for room temperature).

In naming the compounds of the present invention, the numbering scheme used for the diamantane ring system ($C_{14}H_{20}$) is as follows:

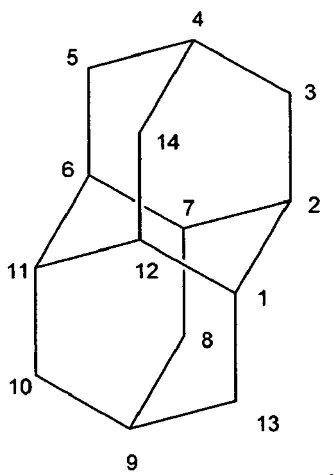

Positions 1, 2, 4, 6, 7, 9, 11, and 12 are bridgehead positions and the substituents at these positions are as defined for the compounds of Formula I, Ia, and II. It is to be understood that in naming the compounds based upon the above positions, the compounds may be racemic mixtures of enantiomers (e.g., the enantiomers 1,6-dimethyl-2-amino diamantane and 1,6-dimethyl-12-amino diamantane and the enantiomers 1-methyl-7-amino diamantane and 1-methyl-11-amino diamantane).

In naming the compounds of the present invention, the numbering scheme used for the triamantane ring system ($C_{18}H_{24}$) is as follows:

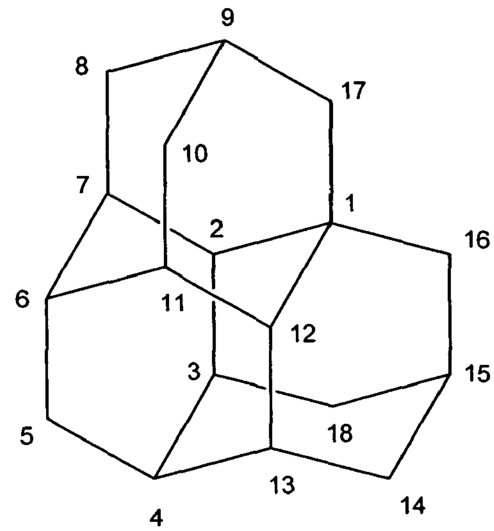

Positions 1, 2, 3, 4, 6, 7, 9, 11, 12, 13, and 15 are bridgehead positions and the substituents at these positions are as defined for the compounds of Formula III.

Diamantane derivatives within the scope of this invention, including those of Formula I, Ia, and II, include those set forth in Table I as follows. The substituents at positions 1, 2, 4, 6, 7, 9, 11, and 12 are defined in the Table. The substituents at positions 3, 5, 8, 10, 13, and 14 are all hydrogen.

Table I

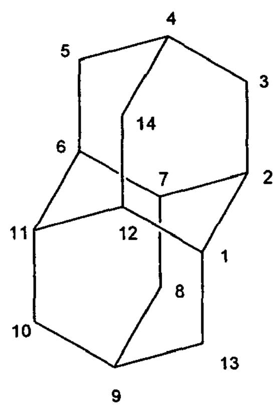

| 1 | 2 | 4 | 6 | 7 | 9 | 11 | 12 |
|---|---|---|---|---|---|---|---|
| -H | -H | $-NH_2$ | -H | -H | -H | -H | -H |
| $-NH_2$ | -H | -H | -H | -H | -H | -H | -H |
| $-NH_2$ | -H | -H | $-NH_2$ | -H | -H | -H | -H |
| -H | -H | $-NHCOCH_3$ | -H | -H | -H | -H | -H |
| $-NHCOCH_3$ | -H | -H | -H | -H | -H | -H | -H |
| $-NHCOCH_3$ | -H | -H | $-NHCOCH_3$ | -H | -H | -H | -H |
| $-NHCOCH_3$ | -H | $-NHCOCH_3$ | -H | -H | -H | -H | -H |
| $-CH_3$ | -H | $-NH_2$ | -H | -H | -H | -H | -H |
| -H | -H | $-NH_2$ | -H | -H | $-NH_2$ | -H | -H |
| $-CH_3$ | $-NH_2$ | -H | $-CH_3$ | -H | -H | -H | -H |

(continued)

| 1 | 2 | 4 | 6 | 7 | 9 | 11 | 12 |
|---|---|---|---|---|---|---|---|
| $-CH_3$ | -H | $-NH_2$ | $-CH_3$ | -H | -H | -H | -H |
| $-CH_3$ | $-NH_2$ | $-NH_2$ | $-CH_3$ | -H | -H | -H. | -H |
| $-CH_3$ | -H | $-NH_2$ | -H | $-CH_3$ | -H | -H | -H |
| $-CH_3$ | -H | -H | -H | $-NH_2$ | -H | -H | -H |
| $-CH_3$ | -H | -H | -H | -H | -H | $-NH_2$ | -H |
| $-CH_3$ | -H | -H | $-CH_3$ | -H | -H | -H | $-NH_2$ |
| $-CH_3$ | $NH_2$ | -H | -H | -H | -H | -H | -H |
| $-CH_3$ | -H | -H | $-NH_2$ | -H | -H | -H | -H |
| $-CH_3$ | -H | -H | -H | -H | $-NH_2$ | -H | -H |
| $-CH_3$ | $NH_2$ | $-NH_2$ | -H | -H | -H | -H | -H |
| $-CH_3$ | -H | $-NH_2$ | $-NH_2$ | -H | -H | -H | -H |
| $-CH_3$ | -H | $-NH_2$ | -H | -H | $-NH_2$ | -H | -H |
| $-NH_2$ | $-CH_3$ | -H | -H | -H | -H | -H | -H |
| $-NH_2$ | -H | $-CH_3$ | -H | -H | -H | -H | -H |
| -H | $NH_2$ | $-CH_3$ | -H | -H | -H | -H | -H |
| -H | -H | $-CH_3$ | -H | -H | $-NH_2$ | -H | -H |
| $-CH_3$ | -OH |  | $-CH_3$ | -H | -H | -H | -H |
| $-CH_3$ | -H | -OH | $-CH_3$ | -H | -H | -H | -H |
| $-CH_3$ | -H | -COOH | $-CH_3$ | -H | -H | -H | -H |
| -OH | -H | $-CH_3$ | -H | -H | $-CH_3$ | -H | -H |
| $-NH_2$ | -H | $-CH_3$ | -H | -H | $-CH_3$ | -H | -H |
| -COOH | -H | $-CH_3$ | -H | -H | $-CH_3$ | -H | -H |
| $-NH_2$ | -H | $-CH_3$ | $-NH_2$ | -H | $-CH_3$ | -H | -H |
| -OH | -H | -H | -H | -H | -H | -H | -H |
| -H | -H | -OH | -H | -H | -H | -H | -H |
| -OH | -H | -H | -OH | -H | -H | -H | -H |
| -OH | -H | -H | -H | -OH | -H | -H | -H |
| -H | -H | -OH | -H | -H | -OH | -H | -H |
| -COOH | -H | -H | -H | -H | -H | -H | -H |
| -COONa | -H | -H | -H | -H | -H | -H | -H |
| -H | -H | -COOH | -H | -H | -H | -H | -H |
| -COOH | -H | -H | -COOH | -H | -H | -H | -H |
| -COONa | -H | -H | -COONa | -H | -H | -H | -H |
| -H | -H | -COOH | -H | -H | -COOH | -H | -H |
| -ONO | -H | -H | -H | -H | -H | -H | -H |
| -H | -H | -ONO | -H | -H | -H | -H | -H |
| $-NH_2$ | -H | -H | -Br | -H | -H | -H | -H |
| $-CH_2NH_2$ | -H | -H | -H | -H | -H | -H | -H |

(continued)

| 1 | 2 | 4 | 6 | 7 | 9 | 11 | 12 |
|---|---|---|---|---|---|---|---|
| $-CHCH_3NH_2$ | -H | -H | -H | -H | -H | -H | -H |
| -H | -H | $-CH_2NH_2$ | -H | -H | -H | -H | -H |
| -H | -H | $-CHCH_3NH_2$ | -H | -H | -H | -H | -H |
| $-CH_2NH_2$ | -H | $-CH_3$ | -H | -H | $-CH_3$ | -H | -H |
| $-CHNH_2CH_2CH_3$ | -H | -H | -H | -H | -H | -H | -H |
| $-CH_3$ | -H | $-CH_2NH_2$ | $-CH_3$ | -H | -H | -H | -H |
| -H | -H | $-CHNH_2CH_2CH_3$ | -H | -H | -H | -H | -H |
| $-CHCH_3NH_2$ | -H | $-CH_3$ | -H | -H | $-CH_3$ | -H | -H |
| $-CH_3$ | -H | $-CHCH_3NH_2$ | $-CH_3$ | -H | -H | -H | -H |

Diamantane derivatives within the scope of this invention, including those of Formula I, Ia, and II, also include the following:

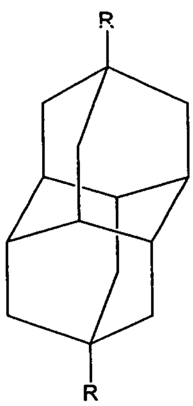
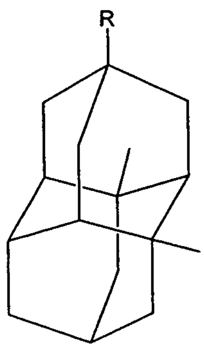
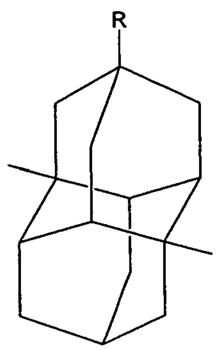
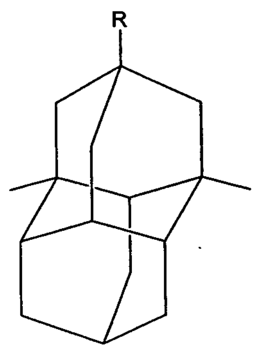

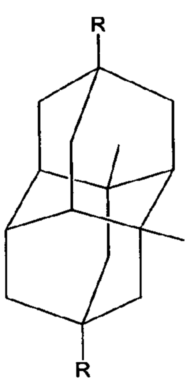
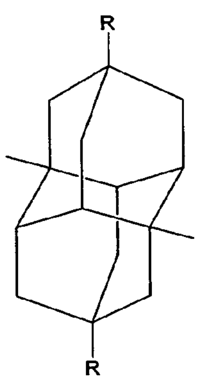
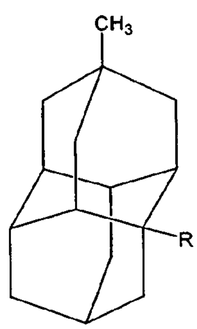
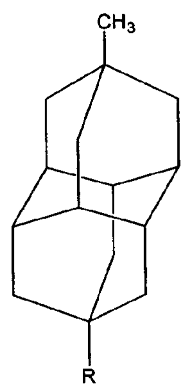
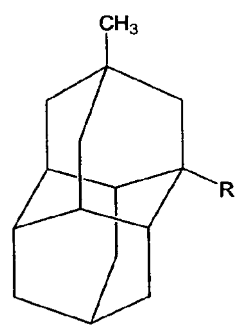

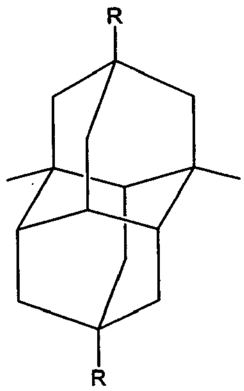
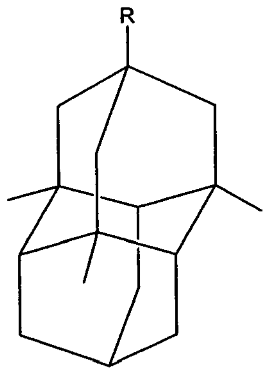
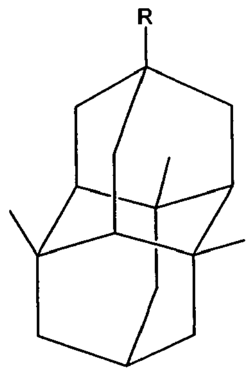
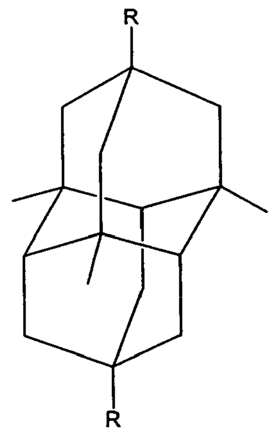

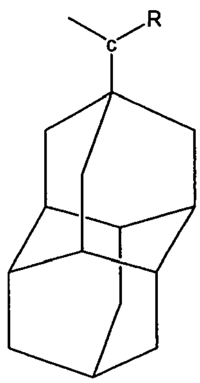
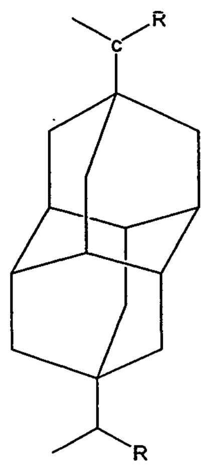
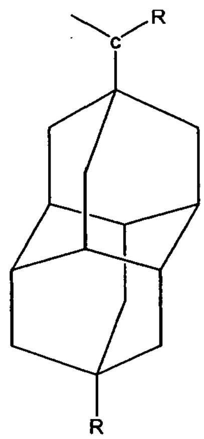

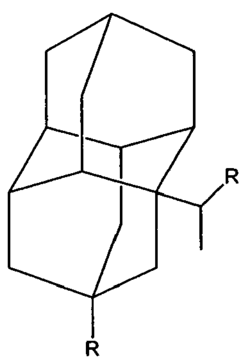
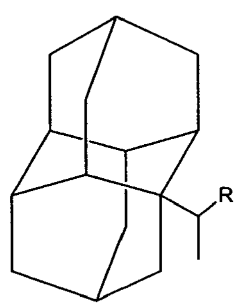
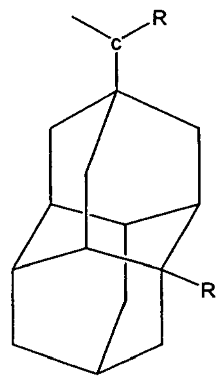

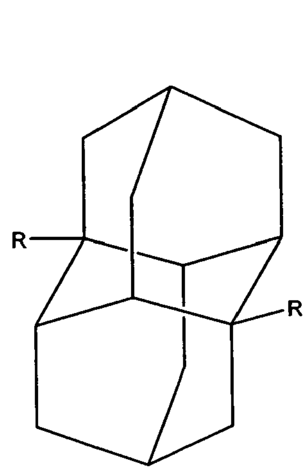

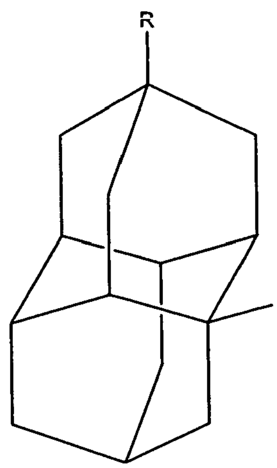

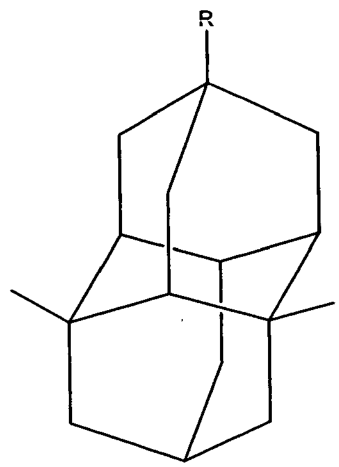

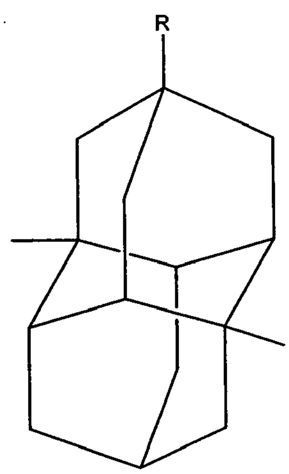

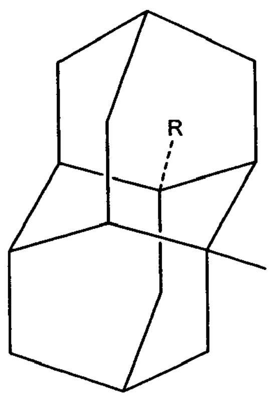

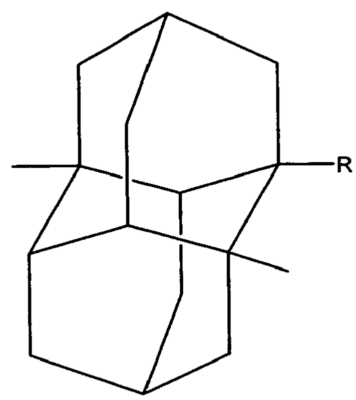

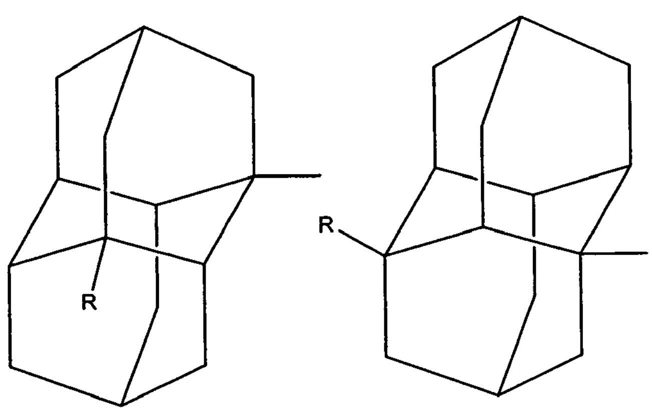

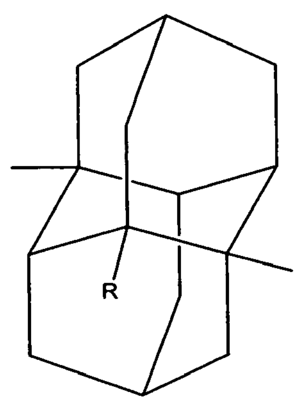

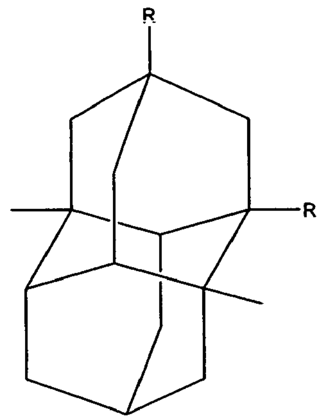

wherein R is independently hydroxy, carboxy, amino, when amino preferably $-NH_2$, nitroso, nitro, or aminoacyl, when aminoacyl preferably acetamino. Preferably R is hydroxy, carboxy, amino or aminoacyl.

Specific compounds within the scope of this invention include, for example, the following compounds: 1-aminodiamantane; 4-aminodiamantane; 1,6-diaminodiamantane; 4,9-diaminodiamantane; 1-methyl-2-aminodiamantane; 1-methyl-4-aminodiamantane; 1-methyl-6-aminodiamantane; 1-methyl-7-aminodiamantane; 1-methyl-9-aminodiamantane; 1-methyl-11-aminodiamantane; 1-methyl-2,4-diaminodiamantane; 1-methyl-4,6-diaminodiamantane; 1-methyl-4,9-diaminodiamantane; 1-amino-2-methyldiamantane; 1-amino-4-methyldiamantane; 2-amino-4-methyldiamantane; 4-methyl-9-aminodiamantane; 1,6-dimethyl-2-aminodiamantane; 1,6-dimethyl-4-aminodiamantane; 1,6-dimethyl-12-aminodiamantane; 1,6-dimethyl-2,4-diaminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 4,9-dimethyl-1-aminodiamantane; 4,9-dimethyl-1-diamantanecarboxylic acid; 4,9-dimethyl-1,6-diaminodiamantane; 1,7-dimethyl-4-aminodiamantane; 1-acetaminodiamantane; 4-acetaminodiamantane; 1,4-diacetaminodiamantane; 1,6-diacetaminodiamantane; 1-hydroxydiamantane; 4-hydroxydiamantane; 1,6-dihydroxydiamantane; 1,7-dihydroxydiamantane; 4,9-dihydroxydiamantane; 1-diamantanecarboxylic acid; sodium 1-diamantanecarboxylate; 4-diamantanecarboxylic acid; 1,6-diamantanedicarboxylic acid; sodium 1,6-diamantanedicarboxylate; 4,9-diamantanedicarboxylic acid; 1-nitrosodiamantane; 4-nitrosodiamantane; 6-bromo-1-aminodiamantane; 1-aminomethyl-diamantane; 1-(1-aminoethyl)-diamantane; 4-aminomethyl-diamantane; 4-(1-aminoethyl)-diamantane; 1-aminomethyl-4,9-dimethyl-diamantane; 1-(1-aminopropyl)-diamantane; 4-aminomethyl-1,6-dimethyl-diamantane; 4-(1-aminopropyl)-diamantane; 1-(1-aminoethyl)-4,9-dimethyl-diamantane; 4-(1-aminoethyl)-1,6-dimethyl-diamantane; and pharmaceutically acceptable salts thereof. Preferred pharmaceutically acceptable salts thereof include hydrochloride salts.

Triamantane derivatives within the scope of this invention include those as illustrated below. The substituents at positions 5, 8, 10, 14, 16, 17, and 18 are all hydrogen.

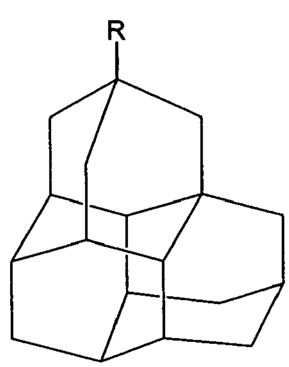
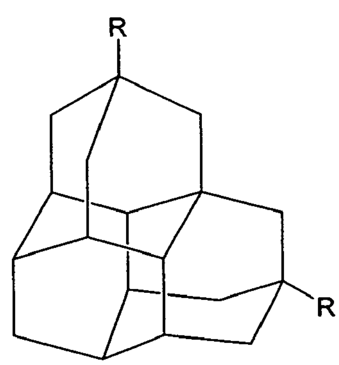
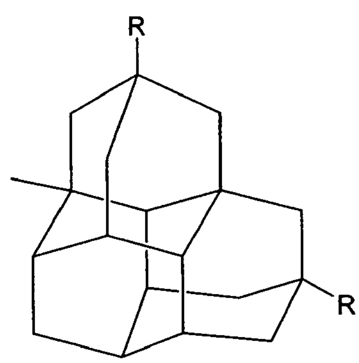

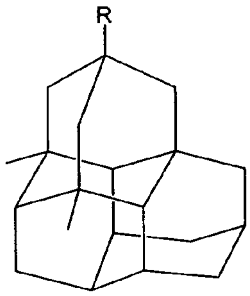 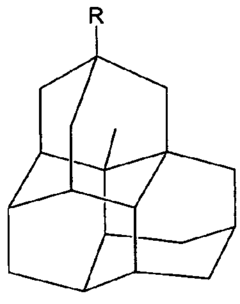 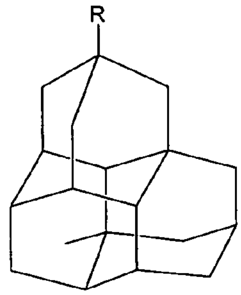 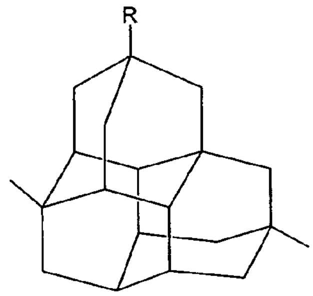

wherein R is independently amino, when amino preferably $-NH_2$, nitroso, nitro, or aminoacyl, when aminoacyl preferably acetamino.

Specific compounds within the scope of this invention include, for example, the following compounds: 2-hydroxytriamantane; 3-hydroxytriamantane; 9-hydroxytriamantane; 9,15-dihydroxytriamantane; 2-aminotriamantane; 3-aminotriamantane; 9-aminotriamantane; 9,15-diaminotriamantane; and pharmaceutically acceptable salts thereof. Preferred pharmaceutically acceptable salts thereof include hydrochloride salts.

General Synthetic Schemes

Unsubstituted diamantane and triamantane may be synthesized by methods well known to those of skill in the art. For example, diamantane may be synthesized as described in Organic Syntheses, Vol 53, 30-34 (1973); Tetrahedron Letters, No. 44, 3877-3880 (1970); and Journal of the American Chemical Society, 87:4, 917-918 (1965). Triamantane may be synthesized as described in Journal of the American Chemical Society, 88:16, 3862-3863 (1966).

Furthermore, unsubstituted or alkylated diamantane and triamantane can be recovered from readily available feedstocks using methods and procedures well known to those of skill in the art. For example, unsubstituted or alkylated diamantane and triamantane can be isolated from suitable feedstock compositions by methods as described in U.S. Patent No. 5,414,189, herein incorporated by reference in its entirety. Furthermore, unsubstituted or alkylated diamantane and triamantane can be isolated from suitable feedstock compositions by methods as described for higher diamondoids in U.S. Patent No. 6,861,569, herein incorporated by reference in its entirety. It will be appreciated that where typical or preferred process conditions (*i.e.,* reaction temperatures, times, solvents, pressures, etc.) are given, other process conditions can also be used unless otherwise stated. Optimum reaction conditions may vary with feedstocks, but such conditions can be determined by one skilled in the art by routine optimization procedures. Suitable feedstocks are selected such that the feedstock comprises recoverable amounts of unsubstituted diamondoids selected from the group consisting of diamantane, triamanate, and mixtures thereof. Preferred feedstocks include, for example, natural gas condensates and refinery streams, including hydrocarbonaceous streams recoverable from cracking processes, distillations, coking, and the like. Preferred feedstocks include condensate fractions recovered from the Norphlet Formation in the Gulf of Mexico and from the LeDuc Formation in Canada.

Diamantane, isolated as described above, may be derivatized to provide a compound of Formula I, Ia, or II according to the present invention by synthetic pathways as illustrated in FIG. 1 and as described in further detail in the following examples.

Representative examples of derivatized diamantane and triamantane compounds may be prepared from diamantane and triamantane, isolated as described above, by synthetic pathways as illustrated in FIGs. 2-16, wherein D represents diamantane, triamantane, and their alkylated analogs.

The reagents used in preparing the compounds of Formula I, Ia, II, and III are either available from commercial suppliers such as Toronto Research Chemicals (North York, ON Canada), Aldrich Chemical Co. (Milwaukee, Wisconsin, USA), Bachem (Torrance, California, USA), Emka-Chemie, or Sigma (St. Louis, Missouri, USA) or are prepared by methods known to those skilled in the art following procedures set forth in references such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure.

As it will be apparent to those skilled in the art, conventional protecting groups may be necessary to prevent certain functional groups from undergoing undesired reactions. Suitable protecting groups for various functional groups, as well as suitable conditions for protecting and deprotecting particular function groups are well known in the art. For

example, numerous protecting groups are described in T.W. Greene and G.M. Wuts, Protecting Groups in Organic Synthesis, Second Edition, Wiley, New York, 1991, and references cited therein.

The starting materials and the intermediates of the reaction may be isolated and purified if desired using conventional techniques, including but not limited to filtration, distillation, crystallization, chromatography, and the like. Such materials may be characterized using conventional means, including physical constants and spectral data.

FIG. 2 shows some representative primary derivatives of diamondoids and the corresponding reactions. As shown in FIG. 2, there are, in general, three major reactions for the derivatization of diamondoids sorted by mechanism: nucleophilic ($S_N$1-type) and electrophilic ($S_E$2-type) substitution reactions, and free radical reaction (details for such reactions and their use with adamantane are shown, for instance in, "Recent developments in the adamantane and related polycyclic hydrocarbons " by R. C. Bingham and P. v. R. Schleyer as a chapter of the book entitled "Chemistry of Adamantanes", Springer-Verlag, Berlin Heidelberg New York, 1971 and in; "Reactions of adamantanes in electrophilic media" by I. K. Moiseev, N. V. Makarova, M. N. Zemtsova published in Russian Chemical Review, 68(12), 1001-1020 (1999); "Cage hydrocarbons" edited by George A. Olah, John Wiley & Son, Inc., New York, 1990).

$S_N$1 reactions involve the generation of diamondoids carbocations (there are several different ways to generate the diamondoid carbocations, for instance, the carbocation is generated from a parent diamantane or triamantane, a hydroxylated diamantane or triamantane or a halogenated diamantane or triamantane, shown in FIG. 3), which subsequently react with various nucleophiles. Some representative examples are shown in FIG. 4. Such nucleophiles include, for instance, the following: water (providing hydroxylated diamantane or triamantane); halide ions (providing halogenated diamantane or triamantane); ammonia (providing aminated diamantane or triamantane); azide (providing azidylated diamantane or triamantane); nitriles (the Ritter reaction, providing aminated diamantane or triamantane after hydrolysis); carbon monoxide (the Koch-Haaf reaction, providing carboxylated diamantane or triamantane after hydrolysis); olefins (providing alkenylated diamantane or triamantane after deprotonation); and aromatic reagents (providing arylated diamantane or triamantane after deprotonation). The reaction occurs similarly to those of open chain alkyl systems, such as t-butyl, t-cumyl and cycloalkyl systems. Since tertiary (bridgehead) carbons of diamondoids are considerably more reactive than secondary carbons under $S_N$1 reaction conditions, substitution at the tertiary carbons is favored.

$S_E$2-type reactions (*i.e.,* electrophile substitution of a C-H bond via a five-coordinate carbocation intermediate) include, for instance, the following reactions: hydrogen-deuterium exchange upon treatment with deuterated superacids (e.g., DF-$SbF_5$ or $DSO_3F$-$SbF_5$); nitration upon treatment with nitronium salts, such as $NO_2^+BF_4^-$ or $NO_2^+PF_6^-$ in the presence of superacids (e.g., $CF_3SO_3H$); halogenation upon, for instance, reaction with $Cl_2$+$AgSbF_6$; alkylation of the bridgehead carbons under the Friedel-Crafts conditions (i.e., $S_E$2-type σ alkylation ); carboxylation under the Koch reaction conditions; and, oxygenation under $S_E$2-type σ hydroxylation conditions (e.g., hydrogen peroxide or ozone using superacid catalysis involving $H_3O_2^+$ or $HO_3^+$, respectively). Some representative $S_E$2-type reactions are shown in FIG. 5.

Of those $S_N$1 and $S_E$2 reactions, $S_N$1-type reactions are the most frequently used for the derivatization of diamondoids. However, such reactions produce the derivatives mainly substituted at the tertiary carbons. Substitution at the secondary carbons of diamondoids is not easy in carbonium ion processes since secondary carbons are considerably less reactive than the bridgehead positions (tertiary carbons) in ionic processes. Free radical reactions provide a method for the preparation of a greater number of the possible isomers of a given diamondoids than might be available by ionic processes. The complex product mixtures and/or isomers which result, however, are generally difficult to separate.

FIG. 6 shows some representative pathways for the preparation of brominated diamantane or triamantane derivatives. Mono- and multi-brominated diamondoids are some of the most versatile intermediates in the derivative chemistry of diamondoids. These intermediates are used in, for example, the Koch-Haaf, the Ritter, and the Friedel-Crafts alkylation/arylation reactions. Brominated diamondoids are prepared by two different general routes. One involves direct bromination of diamantane or triamantane with elemental bromine in the presence or absence of a Lewis acid (e.g., $BBr_3$-$AlBr_3$) catalyst. The other involves the substitution reaction of hydroxylated diamantane or triamantane with hydrobromic acid.

Direct bromination of diamantane or triamantane is highly selective resulting in substitution at the bridgehead (tertiary) carbons. By proper choice of catalyst and conditions, one, two, three, four, or more bromines can be introduced sequentially into the molecule, all at bridgehead positions. Without a catalyst, the mono-bromo derivative is the major product with minor amounts of higher bromination products being formed. By use of suitable catalysts, however, di-, tri-, and tetra-, penta-, and higher bromide derivatives are isolated as major products in the bromination (e.g., adding catalyst mixture of boron bromide and aluminum bromide with different molar ratios into the bromine reaction mixture). Typically, tetrabromo or higher bromo derivatives are synthesized at higher temperatures in a sealed tube.

Bromination reactions of diamondoids are usually worked up by pouring the reaction mixture onto ice or ice water and adding a suitable amount of chloroform or ethyl ether or carbon tetrachloride to the ice mixture. Excess bromine is removed by distillation under vacuum and addition of solid sodium disulfide or sodium hydrogen sulfide. The organic layer is separated and the aqueous layer is extracted by chloroform or ethyl ether or carbon tetrachloride for an additional

2-3 times. The organic layers are then combined and washed with aqueous sodium hydrogen carbonate and water, and finally dried.

To isolate the brominated derivatives, the solvent is removed under vacuum. Typically, the reaction mixture is purified by subjecting it to column chromatography on either alumina or silica gel using standard elution conditions (e.g., eluting with light petroleum ether, n-hexane, or cyclohexane or their mixtures with ethyl ether). Separation by preparative gas chromatography (GC) or high performance liquid chromatography (HPLC) is used where normal column chromatography is difficult and/or the reaction is performed on extremely small quantities of material.

Similarly to bromination reactions, diamantanes and triamantanes are chlorinated or photochlorinated to provide a variety of mono-, di-, tri-, or even higher chlorinated derivatives of the diamondoids. FIG. 7 shows some representative pathways for the synthesis of chlorinated diamondoid derivatives.

FIG. 8 shows some representative pathways for the synthesis of hydroxylated diamantane or triamantane. Direct hydroxylation is also effected on diamantane or triamantane upon treatment with *N*-hydroxyphthalimide and a binary co-catalyst in acetic acid. Hydroxylation is a very important way of activating the diamondoid nuclei for further derivatizations, such as the generation of diamondoid carbocations under acidic conditions, which undergo the $S_N1$ reaction to provide a variety of diamondoid derivatives. In addition, hydroxylated derivatives are very important nucleophilic agents, by which a variety of diamondoid derivatives are produced. For instance, the hydroxylated derivatives are esterified under standard conditions such as reaction with an activated acid derivative. Alkylation to prepare ethers is performed on the hydroxylated derivatives through nucleophilic substitution on appropriate alkyl halides.

The above described three core derivatives (hydroxylated diamondoids and halogenated, especially brominated and chlorinated, diamondoids), in addition to the parent diamondoids or substituted diamondoids directly separated from the feedstocks as described above, are most frequently used for further derivatizations of diamantane or triamantane, such as hydroxylated and halogenated derivatives at the tertiary carbons are very important precursors for the generation of diamondiod carbocations, which undergo the $S_N1$ reaction to provide a variety of diamondoid derivatives thanks to the tertiary nature of the bromide or chloride or alcohol and the absence of skeletal rearrangements in the subsequent reactions. Examples are given below.

FIG. 9 shows some representative pathways for the synthesis of carboxylated diamondoids, such as the Koch-Haaf reaction, starting from hydroxylated or brominated diamantane or triamantane. It should be mentioned that for most cases, using hydroxylated precursors get better yields than using brominated diamantane or triamantane. For instance, carboxylated derivatives are obtained from the reaction of hydroxylated derivatives with formic acid after hydrolysis. The carboxylated derivatives are further esterified through activation (*e.g.*, conversion to acid chloride) and subsequent exposure to an appropriate alcohol. Those esters are reduced to provide the corresponding hydroxymethyl diamantanes or triamantanes (diamantane or triamantane substituted methyl alcohols, $D\text{-}CH_2OH$). Amide formation is also performed through activation of the carboxylated derivative and reaction with a suitable amine. Reduction of the diamondoid carboxamide with reducing agents (e.g., lithium aluminum hydride) provides the corresponding aminomethyl diamondoids (diamantane or triamantane substituted methylamines, $D\text{-}CH_2NH_2$).

FIG. 10 shows some representative pathways for the synthesis of acylaminated diamondoids, such as the Ritter reaction starting from hydroxylated or brominated diamondoids. Similarly to the Koch-Haaf reaction, using hydroxylated precursors get better yields than using brominated diamondoids in most cases. Acylaminated diamondoids are converted to amino derivatives after alkaline hydrolysis. Amino diamondoids are further converted to, without purification in most cases, amino diamondoid hydrochloride by introducing hydrochloride gas into the aminated derivatives solution. Amino diamondoids are some of very important precursors. They are also prepared from the reduction of nitrated compounds. FIG. 11 shows some representative pathways for the synthesis of nitro diamondoid derivatives. Diamondoids are nitrated by concentrated nitric acid in the presence of glacial acetic acid under high temperature and pressure. The nitrated diamondoids are reduced to provide the corresponding amino derivatives. In turn, for some cases, amino diamondoids are oxidized to the corresponding nitro derivatives if necessary. The amino derivatives are also synthesized from the brominated derivatives by heating them in the presence of formamide and subsequently hydrolyzing the resultant amide.

Similarly to the hydroxylated compounds, amino diamondoids are acylated or alkylated. For instance, reaction of an amino diamondoid with an activated acid derivative produces the corresponding amide. Alkylation is typically performed by reacting the amine with a suitable carbonyl containing compound in the presence of a reducing agent (e.g., lithium aluminum hydride). The amino diamondoids undergo condensation reactions with carbamates such as appropriately substituted ethyl *N*-arylsulfonylcarbamates in hot toluene to provide, for instance, *N*-arylsulfonyl-*N*'- diamondoidylureas.

FIG. 12 presents some representative pathways for the synthesis of alkylated, alkenylated, alkynylated and arylated diamondoids, such as the Friedel-Crafts reaction. Ethenylated diamondoid derivatives are synthesized by reacting a brominated diamondoid with ethylene in the presence of $AlBr_3$ followed by dehydrogen bromide with potassium hydroxide (or the like). The ethenylated compound is transformed into the corresponding epoxide under standard reaction conditions (e.g., 3-chloroperbenzoic acid). Oxidative cleavage (e.g., ozonolysis) of the ethenylated diamondoid affords

the related aldehyde. The ethynylated diamondoid derivatives are obtained by treating a brominated diamondoid with vinyl bromide in the presence of $AlBr_3$. The resultant product is dehydrogen bromide using KOH or potassium t-butoxide to provide the desired compound.

More reactions are illustrative of methods which can be used to functionalize diamondoids. For instance, fluorination of a diamondoid is carried out by reacting the diamondoid with a mixture of poly(hydrogen fluoride) and pyridine (30% Py, 70% HF) in the presence of nitronium tetrafluoroborate. Sulfur tetrafluoride reacts with a diamondoid in the presence of sulfur monochloride to afford a mixture of mono-, di-, tri- and even higher fluorinated diamondoids. Iodo diamondoids are obtained by a substitutive iodination of chloro, bromo or hydroxyl diamondoids.

Reaction of the brominated derivatives with hydrochloric acid in dimethylformamide (DMF) converts the compounds to the corresponding hydroxylated derivatives. Brominated or iodinated diamondoids are converted to thiolated diamondoids by way of, for instance, reacting with thioacetic acid to form diamondoid thioacetates followed by removal of the acetate group under basic conditions. Brominated diamondoids, e.g., D-Br, are heated under reflux with an excess (10 fold) of hydroxyalkylamine, *e.g.*, $HO\text{-}CH_2CH_2\text{-}NH_2$, in the presence of a base, e.g., triethylamine, diamondoidyloxyalkylamine, *e.g.*, $D\text{-}O\text{-}CH_2CH_2\text{-}NH_2$, is obtained. On acetylation of the amines with acetic anhydride and pyridine, a variety of N-acetyl derivatives are obtained. Direct substitution reaction of brominated diamondoids, *e.g.*, D-Br, with sodium azide in dipolar aprotic solvents, *e.g.*, DMF, to afford the azido diamondoids, *e.g.*, $D\text{-}N_3$.

Diamondoid carboxylic acid hydrazides are prepared by conversion of diamondoid carboxylic acid into a chloroanhydride by thionyl chloride and condensation with isonicotinic or nicotinic acid hydrazide (FIG. 13).

Diamondoidones or "diamondoid oxides" are synthesized by photooxidation of diamondoids in the presence of peracetic acid followed by treatment with a mixture of chromic acid-sulfuric acid. Diamondoidones are reduced by, for instance, $LiAlH_4$, to diamondoidols hydroxylated at the secondary carbons. Diamondoidones also undergo acid-catalyzed (HCl-catalyzed) condensation reaction with, for example, excess phenol or aniline in the presence of hydrogen chloride to form 2,2-bis(4-hydroxyphenyl) diamondoids or 2,2-bis(4-aminophenyl) diamondoids.

Diamondoidones (*e.g.*, D=O) are treated with RCN (R = hydrogen, alkyl, aryl, etc.) and reduced with $LiAlH_4$ to give the corresponding C-2-aminomethyl-C-2-D-OH, which are heated with $COCl_2$ or $CSCl_2$ in toluene to afford the following derivatives shown in formula IV (where Z = O or S):

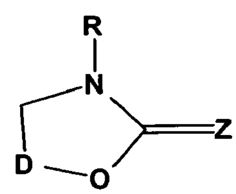

IV

Diamondoidones react with a suitable primary amine in an appropriate solvent to form the corresponding imines. Hydrogenation of the imines in ethanol using Pd/C as the catalyst at about 50˚C to afford the corresponding secondary amines. Methylation of the secondary amines following general procedures (see, for instance, H. W. Geluk and V. G. Keiser, Organic Synthesis, 53:8 (1973)) to give the corresponding tertiary amines. Quaternization of the tertiary amines by, for instance, slowly dropping $CH_3I$ (excess) into an ethanol solution of the amine at around 35˚C to form the corresponding quaternary amines.

C-2 derivatives of diamondoids, C-2 D-R' (R'=alkyl, alkoxy, halo, OH, Ph, COOH, $CH_2COOH$, $NHCOCH_3$, $CF_3COOH$) are prepared by nucleophilic substitution of diamondoid-C-2-spiro-C-3-diazirine in solution at 0-80˚C in the presence of an acid catalyst.

N-sulfinyl diamondoids [$D\text{-}(NSO)_n$, n=1, 2, 3, 4,...] are prepared by refluxing the diamondoid-HCl with $SOCl_2$ in benzene for about half an hour to several hours afording mono-, di, tri-, or higher N-sulfinyl diamondoid derivatives.

Treatment of D-Br and/or D-Cl with $HCONH_2$ (wt. ratio not >1:2) at <195˚C followed by hydrolysis of the formylamino diamondoids D-NHCHO with <20% HCl at <110˚C affords the amino diamondoid hydrochloride $D\text{-}NH_2HCl$.

Diamondoid dicarboxamides are prepared by the reaction of diamondoid dicarbonyl chloride or diamondoid diacetyl chloride with aminoalkylamines. For instance, $D\text{-}(COCl)_2$ [from $SOCl_2$ and the corresponding dicarboxylic acid $D\text{-}(COOH)_2$] are treated with $(CH_3)_2NCH_2CH_2CH_2NH_2$ in $C_5H_5N\text{-}C_6H_6$ to give N,N'-bis(dimethylaminopropyl) diamondoid dicarboxamide.

Aminoethoxyacetylamino diamondoids are prepared from chloroacetylamino diamondoids and $HOCH_2CH_2NR'R''$. Thus, for instance, amino diamondoids, $D\text{-}NH_2$, and $ClCH_2COCl$ in benzene, is added to $(CH_3)_2NCH_2CH_2ONa$ in xylene and refluxed for about 10 hours to give aminoethoxyacetylamino diamondoids

(R'=R"=$CH_3$).

Ritter reaction of C-3 D-OH and HCN gives D-$NH_2$; the preparation of D-NHCHO from diamondoids and HCN; the reaction of diamondoids with nitriles gives D-NHCHO and D-$NH_2$; the preparation of aza diamondoids from nitriles and compounds containing unsaturated OH groups, and SH groups, and so on.

Hydroxylated diamondoids, *e.g.*, D-OH, react with $COCl_2$ or $CSCl_2$ to afford the diamondoidyloxycarbonyl derivatives, *e.g.*, D-O-C(O)Cl or D-O-C(S)Cl the former being an important blocking group in biochemical syntheses.

FIG. 14 shows representative reactions starting from D-$NH_2$ and D-$CONH_2$ and the corresponding derivatives.

FIG. 15 shows representative reactions starting from D-$POCl_2$ and the corresponding derivatives.

FIG. 16 shows representative reactions starting from D-SH or D-SOCl and the corresponding derivatives.

It is noted that many of the derivatizations described herein are merely exemplary and provide guidance to the skilled artisan for synthesizing diamantane and triamantane derivatives of the Formula I, Ia, II, and III according to the present invention.

Utility

The derivatives of diamantane and triamantane of the subject invention exhibit pharmaceutical activity, useful in the treatment, inhibition and/or prevention of neurologic disorders.

The diamantane and triamantane analogs of the present invention exhibit activity against neurologic disorders. Because diamantane and traimanatane are larger than adamantane, the diffusivity of diamantane, triamantane and their derivatives will be lower than that of adamantane and its corresponding derivatives. This will lead to a slower release of the blocking agent from the ion channel.

In addition, substituting two amino groups onto the diamantane structure, as opposed to one amino group, improves the aqueous solubility, and decreases the lipid solubility, which will improve the bioavailability of the molecule. As diamantane and triamantane have rigid structures, they exhibit excellent bioavailability, as well as the ability to pass through the blood-brain barrier.

One mechanism of action of the present compounds includes the regulation of glutamate. Glutamate is the main neurotransmitter in the brain. Glutamatergic overstimulation results in neuronal damage and a condition termed excitotoxicity. The excitotoxicity leads to neuronal calcium overload and has been implicated in neurodegenerative disorders such as Alzheimer's disease. Glutamate stimulates a number of receptors, including the N-methyl-D-aspartate (NMDA) receptor. NMDA receptors are activated by concentrations of glutamate. In order to prevent excessive influx, the ion channel is blocked by a Mg++ under resting conditions.

Overexcitation of NMDA receptors by glutamate may play a role in Alzheimer's disease, as glutamate plays an integral role in the neural pathways associated with learning and memory, and is likely implicated or is affected by many neurologic disorders. The excitotoxicity produced by excessive amounts of glutamate is thought to contribute to neuronal cell death observed in Alzheimer's disease. The compounds of the subject invention are useful in selectively blocking the excitotoxic effects associated with excessive transmission of glutamate, while still allowing enough glutamate activation to preserve normal cell functioning.

For example, it was recently discovered that 1-amino-3,5-dimethyladamantane (Namenda™ (memantine) Forest Pharmaceuticals, Inc.) was effective at treating moderate to severe Alzheimer's disease. Numerous studies have demonstrated the effectiveness of memantine therapy including significant improvement in patients with vascular dementia and significant improvement in motor functions, cognition and social behaviors. However, although adamantanes such as amantadine and rimantadine have not shown great efficacy as NMDA channel blockers, the present diamantane and triamantane derivatives show improvement in this regard, especially as to regulating the Ca++ influx.

Memantine is a prototypical comparator in pre-clinical studies seeking new chemical entities which share a similar low affinity, uncompetitive mode of inhibition. Memantine was synthesized in the 1960s, although it's primary mode of action was not recognized as an NR inhibitor until the late 1980s. During this time an extensive clinical history has shown memantine to have some efficacy with minimal side effects [Rogawski, M. A. (2000). "Low affinity channel blocking (uncompetitive) NMDA receptor antagonists as therapeutic agents-toward an understanding of their favorable tolerability." Amino Acids 19(1): 133-49.; Lipton, S. A. (2006). "Paradigm shift in neuroprotection by NMDA receptor blockade: memantine and beyond." Nat Rev Drug Discov 5(2): 160-70.]. This comparative approach to drug discovery has demonstrated pre-clinical utility of compounds suitable for a broad spectrum of neurologic disorders which are being pursued in the clinic to treat Parkinson's disease [Danysz, W., C. G. Parsons, et al. (1997). "Aminoadamantanes as NMDA receptor antagonists and antiparkinsonian agents--preclinical studies." Neurosci Biobehav Rev 21(4): 455-68.], Alzheimer's disease [Lipton, S. A. (2005). "The molecular basis of memantine action in Alzheimer's disease and other neurologic disorders: low-affinity, uncompetitive antagonism." Curr Alzheimer Res 2(2): 155-65.], Rogawski, M. A. and G. L. Wenk (2003). "The neuropharmacological basis for the use of memantine in the treatment of Alzheimer's disease." CNS Drug Rev 9(3): 275-308.], a variety of acute and chronic neurologic insults [Lipton, S. A. (2004). "Failures and successes of NMDA receptor antagonists: molecular basis for the use of open-channel blockers like memantine in the

treatment of acute and chronic neurologic insults." NeuroRx 1(1): 101-10.], HIV-associated dementia or HAD [Anderson ER 2004 Memantine protects hippocampal neuronal function in muringe human immunodeficiency virus type I encephalitis J Neurosci 24: 7194; Alisky, J. M. (2005). "Could cholinesterase inhibitors and memantine alleviate HIV dementia?" J Acquir Immune Defic Syndr 38(1): 113-4.; Kaul, M., J. Zheng, et al. (2005). "HIV-1 infection and AIDS: consequences for the central nervous system." Cell Death Differ 12 Suppl 1: 878-92.], neuropathic pain [Parsons, C. G. (2001). "NMDA receptors as targets for drug action in neuropathic pain." Eur J Pharmacol 429(1-3): 71-8.], and substance abuse [Danysz, W., C. G. Parsons, et al. (2002). "Amino-alkyl-cyclohexanes as a novel class of uncompetitive NMDA receptor antagonists." Curr Pharm Des 8(10): 835-43.]. Interestingly, although this search strategy has been pursued with adamantane derivatives [Losi G 2006 "Functional in vitro characterization of CR 3394: A novel voltage dependent N-metliyl-D-aspartate (NMDA) receptor antagonist" Neuropharmacol 50: 277.], most work has lead to a variety of chemical structures which lack the adamantane nucleus [Parsons 2001 "NMDA receptors as targets for drug action in neuropathic pain" Eur J Pharmacol 429: 71.; Danysz W and Parsons CG, 2002 "Neuroprotective potential of ionotrophic glutamate receptor antagonists Neurotox Res 4:119; Planells-Cases R et. al. 2002 "A novel N-methyl-D-aspartate receptor open channel blocker with in vivo neuroprotectant activity" J Pharmacol Exp Thera 302: 163. ; Bleich S et. al. 2003 "Glutamate and the glutamate receptor system: a target for drug action" Int J Geriatr Psychiatry 18: S33. ; Muir KW 2006 "Glutamate-based therapeutic approaches: clinical trials with NMDA antagonists" Curr Opin Pharmacol 6:53 ].

The mechanistic understanding of why low affinity, uncompetitive NR antagonists are preferred clinical candidates is a subject of much experimentation and debate. No less than eight mechanisms are discussed in a recent review [Johnson JW and Kotermanski SE 2006 "Mechanism of action of memantine" Cur Opin Pharmacol 6:61].

1. the ability to bind only (or preferentially) to open channels;
2. the tendency to inhibit faster, or with higher affinity, at higher agonist concentrations;
3. a relatively low affinity of inhibition;
4. relatively fast unblocking kinetics;
5. relatively strong voltage dependence;
6. an ability to be trapped in some but not all receptors;
7. an ability to inhibit at two different sites;
8. and NMDAR subtype specificity, or lack thereof.

Current experiments are defining the role that specific amino acid residues lining the NR pore have on key functions affected by channel-binding inhibitors: gating and desensitization [Chen N et. al. 2004 "Site within N-methyl-D-aspartate receptor pore modulates channel gating" Mol Pharmacol 65:157; Yuan H et. al. 2005 "Conserved structural and functional control of N-methyl-d-aspartate receptor gating by transmembrane domain M3" J Biol Chem 280:29708. ; Thomas CG, et al. 2006 "Probing N-methyl-D-aspartate receptor desensitization with the substituted-cysteine accessibility method" Mol Pharmacol. 2006 Apr;69(4):1296-303]. There is also increasing evidence for two binding sites in the NR pore, one near the vestibule or pore entrance and a second deep within the pore near the selectivity filter, and evidence that inhibitors may bind each with differing affinities and functional consequences [Sobolevsky A and Koshelev 1998 "Two blocking sites of amino-adamantane derivatives in open N-methyl-d-aspartate channels" Biophysical J 74: 1305.; Kashiwagi K, et. al. 2002 "Channel blockers acting at N-methyl-D-aspartate receptors: Differential effects of mutation in the vestibule and ion channel pore" Mol Pharmacol 61:533; Chen H-S and Lipton SA 2005 "Pharmacological implications of two distinct mechanism of interaction of memantine with N-methyl-d-aspartate-gated channels" J Pharmacol Exp Thera 314:961; Bolshakov KV et. al. 2005 "Design of antagonists for NMDA and AMPA receptors" Neuropharmacol 42: 144.].

The compounds of the present invention may be used to treat, manage, and prevent neurologic disorders, including those associated with excessive activity of the NMDA receptor. If the NMDA receptor is activated by glutamate continuously, the influx of calcium increases which produces enhanced noise. This noise greatly reduces the chance of the receptor recognizing the relevant signal once it arrives, and cognitive and neuronal function decreases. The following neurologic diseases and conditions relate to the overexcitation of the NMDA receptor, and thus may be treated by the present compounds.

The term neurologic disorder embraces a collection of diseases and conditions, with each type consisting of numerous subsets. Preferred neurologic disorders to be treated, inhibited, and/or prevented with the triamantane and diamantane derivatives set forth herein, include but are not limited to, epilepsy, narcolepsy, neurodegenerative disorders, pain, and psychiatric disorders.

Pain includes both acute pain and chronic pain. Acute pain is pain that lasts or is anticipated to last a short time, typically less than one month. Chronic pain is pain persisting greater than one month beyond the resolution of an acute tissue injury, pain persisting or recurring for more than three months, or pain associated with tissue injury that is expected to continue. Pain may include neuropathic pain, including acute pain where the present compounds may also be used as an adjunct to other analgesic agents as well as administered alone.

Neurodegenerative disorders may include Alzheimer's Disease, Parkinson's Disease, stroke, AIDS related dementia, traumatic brain injury (TBI), and Huntington's Disease.

A stroke occurs when the blood supply to part of the brain is suddenly interrupted or when a blood vessel in the brain bursts, spilling blood into the spaces surrounding brain cells. Brain cells die when they no longer receive oxygen and nutrients from the blood or there is sudden bleeding into or around the brain. The symptoms of a stroke include sudden numbness or weakness, sudden confusion or trouble speaking or understanding speech; sudden trouble seeing in one or both eyes; sudden trouble with walking, dizziness, or loss of balance or coordination; or sudden severe headache with no known cause. Generally, there are three treatment stages for stroke: prevention, including therapy immediately after the stroke, and post-stroke rehabilitation. The most popular classes of drugs used to prevent or treat stroke are antithrombotics (antiplatelet agents and anticoagulants) and thrombolytics. Recurrent stroke is frequent; about 25 percent of people who recover from their first stroke will have another stroke within five years.

TBI is characterized by is caused by a blow or jolt to the head or a penetrating head injury that disrupts the normal function of the brain. The severity of a TBI may be mild, resulting in only a brief change in mental status or consciousness to severe, with extended results including amnesia and unconsciousness or amnesia after the injury. Severe neural degeneration may occur following a brain injury, and is believed to evolve in a biphasic manner consisting of the primary mechanical insult and then a progressive secondary necrosis. Symptoms of a traumatic brain injury include functional changes affecting thinking, sensation, language, and/or emotions.Psychiatric disorders may include substance abuse. The substance abuse may include drug abuse and/or alcohol abuse.

Epilepsy is a recurrent, paroxysmal disorder of cerebral function characterized by sudden, brief attacks of altered consciousness, motor activity, sensory phenomena, or inappropriate behavior caused by excessive discharge of the cerebral neurons.

Narcolepsy is a recurrent disorder characterized by an pathologic increase in absolute sleep hours, usually by greater than 25 percent. See, for example, Xie et al., GABAB receptor-mediated modulation of hypocretin/orexin neurones in mouse hypothalamus. J Physiol, 2006, which as showing that NMDA responsive cells are implicated in narcolepsy. Narcolepsy is not definitively diagnosed in most patients until 10 to 15 years after the first symptoms appear. There is no cure for narcolepsy.

These diseases are divisible into two groups. In one group, the process inevitably produces dementia if it progresses through its full course; these are the conditions thought to affect the brain primarily or exclusively, such as Alzheimer's disease, Huntington's disease, and Parkinson-dementia complex. Other diseases may or may not produce dementia, depending upon whether or how the brain is affected. Examples are liver disease with portacaval encephalopathy, metabolic disorders such as hypothyroidism, or infectious disorders such as syphilis or acquired immune deficiency syndrome. Dementia, a clinical syndrome, can be produced by numerous pathological states that affect the brain. These pathological states can be divided into those that appearto be primary in the brain, such as Alzheimer's disease, and those which are outside the brain and affect it secondarily.

Certain chronic viral illnesses, such as human immunodeficiency virus, are known to produce dementia with great frequency. Thiamine deficiency produces Wernicke-Korsakoffs encephalopathy, which may cause Korsakoff dementia. Thiamine deficiency is a preventable nutritional deficiency seen in the context of alcoholism, pernicious vomiting of pregnancy, depression, or any other condition in which this deficiency occurs.

Management of the underlying states can arrest and sometimes reverse the dementias of cardiovascular origin. Hypertension, especially severe hypertension, is one of the most frequent causes of dementia. Other causes are atherosclerosis and arteriosclerosis without hypertension, vasculitis, and emboli from the heart or elsewhere in the vascular system. Cardiac disease also produces dementia by single or repeated episodes of cerebral ischemia and hypoxia due to acute or intermittent disorders of cardiac function.

Alzheimer's Disease is the most common of all the dementing diseases. Other dementing diseases include those of the basal ganglia, (such as Parkinson's Disease and Huntington's Disease), of the cerebellum (cerebellar and spinocerebellar degenerations, olivopontocerebellar degeneration), and of the motor neurone (amyotrophic lateral sclerosis). Pharmaceutical Formulations

In general, the compounds of the subject invention will be administered in a therapeutically effective amount by any of the accepted modes of administration for these compounds. The compounds can be administered by a variety of routes, including, but not limited to, oral, parenteral (e.g., subcutaneous, subdural, intravenous, intramuscular, intrathecal, intraperitoneal, intracerebral, intraarterial, or intralesional routes of administration), topical, intranasal, localized (e.g., surgical application or surgical suppository), rectal, and pulmonary (e.g., aerosols, inhalation, or powder). Accordingly, these compounds are effective as both injectable and oral compositions. Preferably, the compounds are administered by oral route. Also preferably, the compounds are administered by parenteral routes. The compounds can be administered continuously by infusion or by bolus injection. More preferably, the compounds are administered by intravenous routes. Such compositions are prepared in a manner well known in the pharmaceutical art.

The actual amount of the compound of the subject invention, i.e., the active ingredient, will depend on a number of factors, such as the severity of the disease, i.e., the condition or disease to be treated, the age and relative health of

the subject, the potency of the compound used, the route and form of administration, and other factors.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are preferred.

The data obtained from cell culture assays and animal studies can be used in formulating a range of dosage for use in humans and other animal patients. The dosage of such compounds lies preferably within a range of circulating concentrations that include the $ED_{50}$ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range which includes the $IC_{50}$ (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography. The effective blood level of the compounds of the subject invention is preferably greater than or equal to 40 ng/ml.

The amount of the pharmaceutical composition administered to the patient will vary depending upon what is being administered, the purpose of the administration, such as prophylaxis or therapy, the state of the patient, the manner of administration, and the like. In therapeutic applications, compositions are administered to a patient already suffering from a disease in an amount sufficient to cure or at least partially arrest the symptoms of the disease and its complications. An amount adequate to accomplish this is defined as "therapeutically effective dose." Amounts effective for this use will depend on the disease condition being treated as well as by the judgment of the attending clinician depending upon factors such as the severity of the inflammation, the age, weight and general condition of the patient, and the like.

The compositions administered to a patient are in the form of pharmaceutical compositions described *supra.* These compositions may be sterilized by conventional sterilization techniques, or may be sterile filtered. The resulting aqueous solutions may be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The active compound is effective over a wide dosage range and is generally administered in a pharmaceutically or therapeutically effective amount. The therapeutic dosage of the compounds of the present invention will vary according to, for example, the particular use for which the treatment is made, the manner of administration of the compound, the health and condition of the patient, and the judgment of the prescribing physician. For example, for oral administration, the dose will typicallu be in the range of about 5 mg to about 300 mg per day, preferably about 100 mg to about 200 mg per day. For intravenous administration, the dose will typically be in the range of about 0.5 mg to about 50 mg per kilogram body weight, preferably about 2 mg to about 20 mg per kilogram body weight. Effective doses can be extrapolated from dose-response curves derived from *in vitro* or animal model test systems. Typically, the clinician will administer the compound until a dosage is reached that achieves the desired effect.

When employed as pharmaceuticals, the compounds of the subject invention are usually administered in the form of pharmaceutical compositions. This invention also includes pharmaceutical compositions, which contain as the active ingredient, one or more of the compounds of the subject invention above, associated with one or more pharmaceutically acceptable carriers or excipients. The excipient employed is typically one suitable for administration to human subjects or other mammals. In making the compositions of this invention, the active ingredient is usually mixed with an excipient, diluted by an excipient or enclosed within a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g., about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, sterile water, syrup, and methyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of

the invention can be formulated so as to provide quick, sustained or delayed release of the active ingredient after administration to the patient by employing procedures known in the art.

The quantity of active compound in the pharmaceutical composition and unit dosage form thereof may be varied or adjusted widely depending upon the particular application, the manner or introduction, the potency of the particular compound, and the desired concentration. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. The concentration of therapeutically active compound may vary from about 0.5 mg/ml to 500 g/ml.

Preferably, the compound can be formulated for parenteral administration in a suitable inert carrier, such as a sterile physiological saline solution. For example, the concentration of compound in the carrier solution is typically between about 1-100 mg/ml. The dose administered will be determined by route of administration. Preferred routes of administration include parenteral or intravenous administration. A therapeutically effective dose is a dose effective to produce a significant steroid tapering. Preferably, the amount is sufficient to produce a statistically significant amount of steroid tapering in a subject.

By way of example, for preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer, which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as corn oil, cottonseed oil, sesame oil, coconut oil, or peanut oil, as well as elixirs and similar pharmaceutical vehicles. Syrups are preferred.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* The compositions may be administered by the oral or nasal respiratory route for local or systemic effect. Compositions in preferably pharmaceutically acceptable solvents may be nebulized by use of inert gases. Nebulized solutions may be inhaled directly from the nebulizing device or the nebulizing device may be attached to a face mask tent, or intermittent positive pressure breathing machine. Solution, suspension, or powder compositions may be administered, preferably orally or nasally, from devices which deliver the formulation in an appropriate manner.

The compounds of this invention can be administered in a sustained release form. Suitable examples of sustained-release preparations include semipermeable matrices of solid hydrophobic polymers containing the protein, which matrices are in the form of shaped articles, *e.g.,* films, or microcapsules. Examples of sustained-release matrices include polyesters, hydrogels (e.g., poly(2-hydroxyethyl-methacrylate) as described by Langer et al., J. Biomed. Mater. Res. 15: 167-277 (1981) and Langer, Chem. Tech. 12: 98-105 (1982) or poly(vinyl alcohol)), polylactides (U.S. Patent No. 3,773,919), copolymers ofL-glutamic acid and gamma ethyl-L-glutamate (Sidman et al., Biopolymers 22: 547-556, 1983), nondegradable ethylene-vinyl acetate (Langer *et al., supra),* degradable lactic acid-glycolic acid copolymers such as the LUPRON DEPOT™ (i.e., injectable microspheres composed of lactic acid-glycolic acid copolymer and leuprolide acetate), and poly-D-(-)-3-hydroxybutyric acid (EP 133,988).

The compounds of this invention can be administered in a sustained release form, for example a depot injection, implant preparation, or osmotic pump, which can be formulated in such a manner as to permit a sustained release of the active ingredient. Implants for sustained release formulations are well-known in the art. Implants may be formulated as, including but not limited to, microspheres, slabs, with biodegradable or non-biodegradable polymers. For example, polymers of lactic acid and/or glycolic acid form an erodible polymer that is well-tolerated by the host. The implant is placed in proximity to the site of protein deposits (e.g., the site of formation of amyloid deposits associated with neurodegenerative disorders), so that the local concentration of active agent is increased at that site relative to the rest of the body.

The following formulation examples illustrate pharmaceutical compositions of the present invention.

Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/capsule) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/capsule) |
|---|---|
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinyl-pyrrolidone is mixed with the resultant powders, which are then passed through a 16 mesh

U.S. sieve. The granules so produced are dried at 50° to 60°C and passed through a 16 mesh U.S. sieve. The sodium carboxymethyl starch, magnesium stearate, and talc, previously passed through a No. 30 mesh U.S. sieve, are then added to the granules, which after mixing, are compressed on a tablet machine to yield tablets each weighing 150 mg.

Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, an magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
|---|---|
| Active Ingredient | 25 mg |
| Saturated fatty acid glycerides | to 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) Microcrystalline cellulose (89%) | 50.0 mg |
| Sucrose | 1.75 g |
| Sodium benzoate | 10.0 mg |
| Flavor and Color | q.v. |
| Purified water | to 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Hard gelatin tablets, each containing 15 mg of active ingredient are made as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S. sieve, and filled into hard gelatin capsules in 560 mg quantities.

Formulation Example 9

An intravenous formulation may be prepared as follows:

| Ingredient | Quantity |
|---|---|
| Active Ingredient | 250.0 mg |
| Isotonic saline | 1000 ml |

Therapeutic compound compositions generally are placed into a container having a sterile access port, for example, an intravenous solution bag or vial having a stopper pierceable by a hypodermic injection needle or similar sharp instrument.

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid paraffin and emulsifying wax are incorporated and stirred until dissolved. The active ingredient is added and stirring is continued until dispersed. The mixture is then cooled until solid.

Formulation Example 11

An aerosol formulation may be prepared as follows:
A solution of the candidate compound in 0.5% sodium bicarbonate/saline (w/v) at a concentration of 30.0 mg/mL is prepared using the following procedure:

A. Preparation of 0.5% Sodium Bicarbonate / Saline Stock Solution: 100.0 mL

| Ingredient | Gram / 100.0 mL | Final Concentration |
|---|---|---|
| Sodium Bicarbonate | 0.5 g | 0.5% |
| Saline | q.s. ad 100.0 mL | q.s. ad 100% |

Procedure:

1. Add 0.5g sodium bicarbonate into a 100 mL volumetric flask.
2. Add approximately 90.0 mL saline and sonicate until dissolved.
3. Q.S. to 100.0 mL with saline and mix thoroughly.

B. Preparation of 30.0 mg/mL Candidate Compound: 10.0 mL

| Ingredient | Gram / 10.0 mL | Final Concentration |
|---|---|---|
| Candidate Compound | 0.300 g | 30.0 mg/mL |
| 0.5% Sodium Bicarbonate / Saline Stock Solution | q.s. ad 10.0 mL | q.s ad 100% |

Procedure:

1. Add 0.300 g of the candidate compound into a 10.0 mL volumetric flask.
2. Add approximately 9.7 mL of 0.5% sodium bicarbonate / saline stock solution.
3. Sonicate until the candidate compound is completely dissolved.
4. Q.S. to 10.0 mL with 0.5% sodium bicarbonate / saline stock solution and mix thoroughly.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. *See, e.g.,* U.S. Patent No. 5,023,252, issued June 11, 1991, herein incorporated by reference in its entirety for or all purposes. Such patches may be constructed for continuous, pulsatile, or on demand delivery of pharmaceutical agents.

Direct or indirect placement techniques may be used when it is desirable or necessary to introduce the pharmaceutical composition to the brain. Direct techniques usually involve placement of a drug delivery catheter into the host's ventricular system to bypass the blood-brain barrier. One such implantable delivery system used for the transport of biological factors to specific anatomical regions of the body is described in U.S. Patent No. 5,011,472, which is herein incorporated by reference in its entirety for all purposes.

Indirect techniques, which are generally preferred, usually involve formulating the compositions to provide for drug latentiation by the conversion of hydrophilic drugs into lipid-soluble drugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

According to one aspect of the invention, the compound may be administered alone, as a combination of compounds, or in combination with anti-alpha-4-antibodies. The compounds of the present invention may also be administered in combination with an immunosuppressant, wherein the immunosuppressant is not a steroid, an anti-TNF composition, a 5-ASA composition, and combinations thereof, wherein the immunosuppressant, anti-TNF composition, and 5-ASA composition are typically used to treat the condition or disease for which the compound of the present invention is being administed. The immunosuppressant may be azathioprine, 6-mercaptopurine, methotrexate, or mycophenolate. The anti-TNF composition may be infliximab. The 5-ASA agent may be mesalazine or osalazine.

When administered in combination, the small compounds may be administered in the same formulation as these other compounds or compositions, or in a separate formulation. When administered in combinations, the steroid sparing agents may be administered prior to, following, or concurrently with the other compounds and compositions.

Pharmaceutical compositions of the invention are suitable for use in a variety of drug delivery systems. Suitable formulations for use in the present invention are found in REMINGTON'S PHARMACEUTICAL SCIENCES, Mace Publishing Company, Philadelphia, PA, 17th ed. (1985).

In order to enhance serum half-life, the compounds may be encapsulated, introduced into the lumen of liposomes, prepared as a colloid, or other conventional techniques may be employed which provide an extended serum

half-life of the compounds. A variety of methods are available for preparing liposomes, as described in, *e.g.,* Szoka *et al.,* U.S. Patent Nos. 4,235,871, 4,501,728 and 4,837,028 each of which is incorporated herein by reference in its entirety for all purposes.

The following synthetic and biological examples are offered to illustrate this invention and are not to be construed in any way as limiting the scope of this invention.

**Example 1:**

**Preparation of Hydroxy, Amino, and Aminoacyl Derivatives of Diamantane**

***Experimental***

**Part I. GC-MS Instrumentation and Analytical Methods**

Diamondoids and most of their derivatives can be conveniently detected and analyzed by gas chromatography-mass spectrometry (GC-MS) to confirm the presence of a diamondoid compound as well as its purity. Appropriate GC-MS systems include HP 5890 Series II Chromatography connected to an HP 5973 Series MSD (mass selective detector).

*Detailed GC-MS Methods for the Analysis of Diamondoid Derivatives*

*1. Column details and dimensions*

Column: HP-5ms 30M x 0.25 mm ID and 0.25 μm film thickness. In addition, we also used DB-1 column (15M x 0.25mm ID with film thickness of 0.1 μm), and we found that AT-1HT 15M x 0.25mm ID with film thickness 0.1 μm worked even better than the DB-1 columns.

*2. Injector temperature, injection volume and split ratio*

Injector temp.: 320˚C; injection volume: 0.2 μL; splitless or split

*3. Carrier gas flow rate*

Flow rate: 1.2 mL/min.

*4. Oven program*

150˚C hold for 1 min., then 10˚C per min. to 320˚C; hold at 320˚C for 15 min.

*5. Detector Temperature (if the analysis was run using FID detection)*

320˚C transfer temp.

*6. MS conditions*

Mode of analysis: EI mode; full scan with mass range of 50 to 550. SIM was not used.

*7. Sample preparation conditions*

Dissolve very little compound in suitable solvents.

**Part II. Reactions and Product Analysis**

***Hydroxylation Reaction of Diamantane to Prepare Hydroxyl Diamantanes***

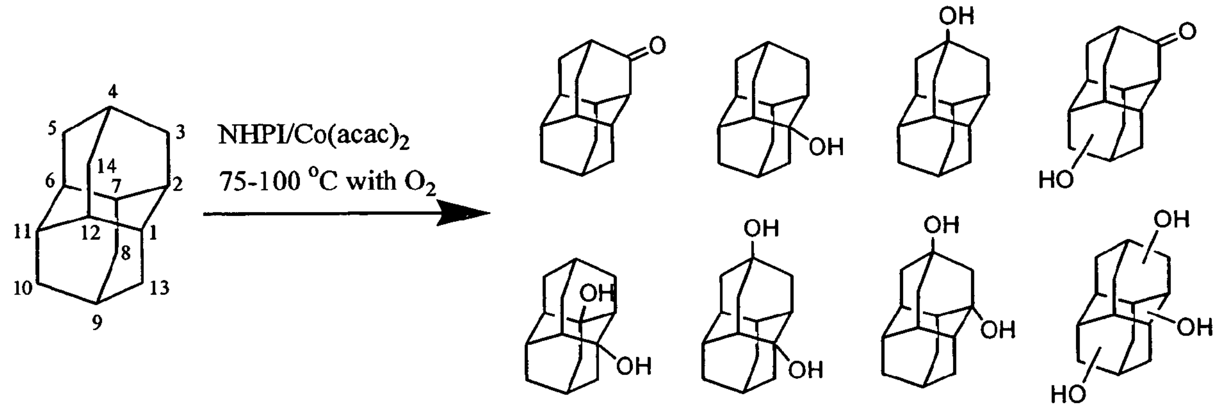


Scheme 1. Synthesis of diamantane alcohols

| Reagent | Source/Cat. No. | MW | Amount | Moles | Eq. |
|---|---|---|---|---|---|
| Diamantane | ChevronTexaco/2010780 | 188.31 | 300g | 1.59 | 1.0 |
| N-Hydroxyphthalimide | Aldrich/H53704 | 163.13 | 26.1g | 0.59 | 0.1 |
| Co(acac)2 | Aldrich/22,712-9 | 257.15 | 2.04g | 0.0079 | 0.005 |
| Acetic acid | Aldrich/32009-9 | 60.05 | 2500mL | | 8.3 v. |
| Oxygen | Airgas | 31.998 | Excess | | |

## General Procedure

A 4 liter, multi-neck reactor, fitted with mechanical stirrer, reflux condenser, thermometer, and gas inlet, was charged diamantane, *N*-hydroxyphthalimide (NHPI), Co(acac)$_2$ (cobalt (II) acetylacetonate), and acetic acid in quantities indicated in the above table. The mixture was stirred for about 23 hours at 75-100˚C in a bubbling oxygen atmosphere until all the diamantane was dissolved and resulted in a clear red solution without any visible solid in the flask. During the reaction, an additional portion (duplicate to triplicate) ofNHPI and Co(acac)$_2$ were added as before. GCMS analysis of the reaction mixture showed significant proportion of diamantane mono-alcohols, dialcohols (diols), and tri-alcohols (triols) in the mixture and the reaction was continued as GCMS indicated increased yields of the alcohols until the desired yields were achieved or until GCMS analysis indicated no increase in the proportion of desired products was being achieved. Then the reaction was stopped and let the reaction mixture cool to room temperature. GCMS analysis of the resulting reaction mixture showed the total ion chromatogram (TIC) of the resulting reaction mixture confirming the presence of diamantane hydroxys in the mixture, shown in FIG. 17.

After the reaction mixture was cooled to room temperature (20˚C), precipitated unreacted diamantane was removed by filtration (very little, if any), and the red colored reaction mixture was concentrated with rotary evaporator (rotovap) to give a dark red oily liquid. The dark red oily liquid was dissolved in dichloromethane (DCM). The DCM solution of the reaction mixture was first extracted with water for three times. The combined water layers were then back extracted with DCM for three times and the DCM layers were combined. The combined DCM layers were dried over $Na_2SO_4$, filtered, and evaporated to give a dark, brown oil, which was purified and separated into a series of diamantane diols and less polar products as described below by column chromatography.

The aqueous extract was concentrated with rotovap to afford a thick red oil. This material was then dissolved in ethanol and decolorizing charcoal added, stirring for 4 hours at room temperature. The mixture was filtered through celite and stripped to dryness to give a colorless oily liquid. This material was then dissolved in DCM/THF (2:1) and placed on the top of a large dry column of silica. The column was eluted with the same solvent mixture to remove a little less polar components and then eluted with THF/ethanol (4:1) to collect the triols. GC/MS: 218, 200, 236 ($M^+$).

A portion of the above crude oily mixture from DCM extract was dissolved into DCM and adsorbed onto double the mass of silica before placing on top of a large silica gel dry column (in this case: 200g crude mixed with 400g silica and the column has 1.2Kg of silica). The column was flushed first with DCM (2 L) and then eluted with 5-15% THF in

DCM which resulted in the less polar components containing diamantanone, mono-alcohols, and mono-keto alcohols, amongst some other unidentified products, being quickly eluted off together. Elution of the di-alcohols was then achieved using 20-50% THF. The 1,7-dialcohol eluted first followed by the 1,4-/2,4- di-alcohol mixture.

Further purification of the 1,7-dialcohol was carried out as following: a 6g sample of less pure 1,7-dialcohol was adsorbed onto silica and purified through a silica gel column, eluting first with DCM (1 L) then running a gradient of 1-5% MeOH in DCM. Impurities of higher and lower $R_f$ spots were removed and the fractions containing predominantly the pure product spots were combined and evaporated to dryness.

| | |
|---|---|
| 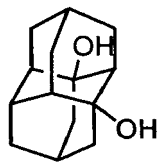 | Analysis of Diamantane-1,7-diol<br><br>R*f*=0.42 (DCM/THF, 95:5)<br>GC/MS: 202 (M-18$^+$)<br><br>$^1$H NMR ($CDCl_3$, 500 MHz): δ 3.9 (s, 2H), 2.25 (m, 4H), 1.9 (s, 2H), 1.55 (m, 10H), 1.4 (d, 2H)<br><br>$^{13}$C NMR ($CDCl_3$, 126 MHz): δ 72, 49, 45, 42, 40, 36, 32, 31, 27,25 |

A further attempted separation of 2g of the two closely related diols (1,4- and 2,4-) was carried out using a silica gel column chromatography eluting with 10-20% THF/DCM. Partial separation of the two isomers was observed, enabling fractions containing predominatly upper and lower spots to be combined separately and evaporated to dryness. The top diol was believed to be the 1,4-isomer and the lower spot to be the 2,4-isomer. A very small amount of very pure 1,4-diol was obtained with a further very careful column chromatography. GC/MS: 202, 220(M$^+$). Due to the extremely difficult nature of this separation, less pure 1,4- and 2,4-isomer were collected and characterized.

| | |
|---|---|
| 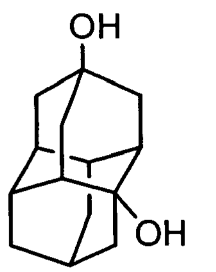 | Analysis of Diamantane-1,4-diol<br><br>$R_f$=0.62 (DCM/THF, 80:20)<br>GC/MS: 202,220(M$^+$)<br><br>$^1$H NMR (DMSO, 500 MHz): δ 4.0 (s, 2H), 2.0 (d, 2H), 1.8 (d, 2H), 1.7 (s, 2H), 1.5 (m, 10H), 1.1 (d, 2H)<br><br>$^{13}$C NMR (DMSO, 126 MHz): δ 68, 65, 45, 38, 36, 29 |
| 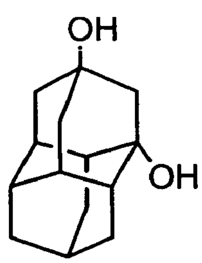 | Analysis of Diamantane-2,4-diol<br><br>$R_f$=0.53 (DCM/THF, 80:20)<br>GC/MS: 202,220(M$^+$)<br><br>$^1$H NMR (DMSO, 500 MHz): δ 4.3 (s), 4.1 (s), 2.0 (m), 1.8 (s),<br>1.5 (m), 1.4 (t), 1.25 (m)<br><br>$^{13}$C NMR (DMSO, 126 MHz): δ 70, 68, 54, 45, 41, 37, 35, 31, 24 |

Further purification of the less polar mixtures containing keto, mono-alcohols, mono-keto alcohols was carried out as following: 50g of the lass polar mixtures was adsorbed onto silica and placed on top of a large silica gel column. The column was eluted with DCM (100%) to DCM/THF (85/15) gradient elution. A series of separated samples were isolated and collected from this column, identifying fractions containing diamantanone, 1-hydroxyl diamantane, 4-hydroxyl diamantane. Or a silica gel column chromatography of a portion of the crude mixture containing less polar components using 0-10% MTBE in hexane gradient elution, afforded a sample of pure diamantanone.

| | |
|---|---|
| 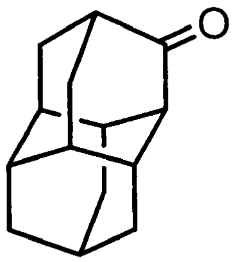 | Analysis of Diamantanone<br><br>$R_f$=0.49 (1% MeOH in DCM)<br>GC/MS: 202 ($M^+$)<br><br>$^1H$ NMR ($CDCl_3$, 500 MHz): δ 2.4 (d, 2H), 2.1 (s, 2H), 1.9 (m, 8H), 1.8 (s, 4H), 1.7 (s, 2H)<br><br>$^{13}C$ NMR ($CDCl_3$, 126 MHz): δ 218, 56, 43, 39.5, 38, 37.5, 36.5, 32, 30, 25 |

The next identifiable sample to be collected from the crude mixture was the monoalcohol with substitution at the 1 position, *i.e.,* diamantane-1-ol. A silica column purification of the crude residences containing the 1-ol from other attempted purifications of the crude mixture was carried out using 1-20% MTBE (methyl tertiary-butyl ether) in hexane gradient elution. The desired compound eluted between 10-15% MTBE. Any pure fractions were evaporated to afford the 1-ol as a white solid.

| | |
|---|---|
| 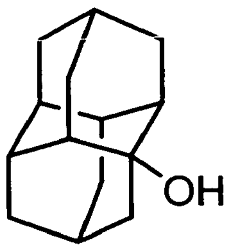 | Analysis of Diamantane-1-ol<br><br>$R_f$=0.40 (hexane/MTBE, 75:25) GC/MS: 186, 204($M^+$)<br><br>$^1H$ NMR ($CDCl_3$, 500 MHz): δ 3.2 (s, 1H), 2.0 (d, 2H), 1.9 (s, 1H), 1.85 (s, 2H), 1.65 (s, 1H), 1.55 (m, 10H), 1.3 (m, 2H)<br><br>$^{13}C$ NMR ($CDCl_3$, 126MHz): δ 71, 47, 43, 39.5, 38, 37.5, 36.5, 32, 30, 25 |

The next identifiable sample to be collected from the crude mixture was the monoalcohol with substitution at the 4 position, i.e., diamantane-4-ol. Several batches containing the crude 4-ol from previous columns (6.5g) were combined and adsorbed onto silica (15g) and placed on a silica gel column. Higher $R_f$ material was flushed off using a gradient of 2-10% MTBE in hexane. Fractions containing the desired 4-ol were then collected and evaporated to dryness. GC-MS and NMR analyses of the sample indicated that there were still significant levels of impurity present that were not visible by TLC. A further silica column purification was carried out using 0-1% MeOH in DCM gradient elution. Any pure fractions were combined and evaporated to dryness to afford the 4-ol as a white solid. Still some impurities were observed in proton NMR spectrum but no solvent system could be found yet to separate these from the target alcohol.

| | |
|---|---|
| 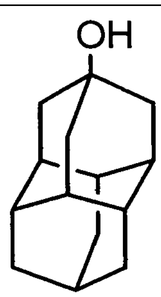 | Analysis of Diamantane-4-ol<br><br>$R_f$=0.36 (1% MeOH in DCM)<br>GC/MS: 204($M^+$)<br><br>$^1H$ NMR ($CDCl_3$, 500 MHz): δ 2.0 (m, 6H), 1.7 (m, 12H), 1.45 (m, 1H)<br><br>$^{13}C$ NMR ($CDCl_3$, 126 MHz): δ 134, 123.5, 45.5, 40, 37, 36, 26 |

After the diamantane-4-ol, there were a series of closely eluting spots believed to contain a mixture of keto alcohol isomers. Fractions containing keto-alcohols from the original column were combined, adsorbed onto silica and purified using a gradient of MTBE in hexane. The gradient was increased to 20% MTBE until the desired compounds begin to elute. The polarity was gradually increased to 50:50. The products obtained were predominantly a mixture of keto-alcohols with other unresolved impurities. The combined fractions containing keto-alcohol products were subjected to further purification using a gradient elution of MeOH (0-2%) in DCM. Two samples were isolated that were purer than before. The smaller sample contained mainly keto-alcohols and the larger, keto-alcohols with another lower $R_f$ impurity present on GC-MS. It is impossible to separate the various keto-alcohol isomers by gravity column chromatography.

| | |
|---|---|
| 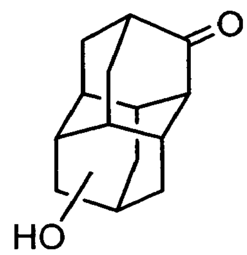 | Analysis of Diamantane Keto-Alcohol<br><br>$R_f$=0.34 (1% MeOH in DCM)<br>GC/MS: 218 ($M^+$), 200; GC/MS: 218 ($M^+$), 200<br><br>$^1$H NMR ($CDCl_3$, 500 MHz): $\delta$ 3.2 (s, 1H), 2.5 (m, 1H), 2.4 (s, 1H), 2.25 (d, 2H), 1.75 (m, 12H) |

***Acetamination of Diamantane Hydroxys to Prepare Acetaminated Diamantanes***

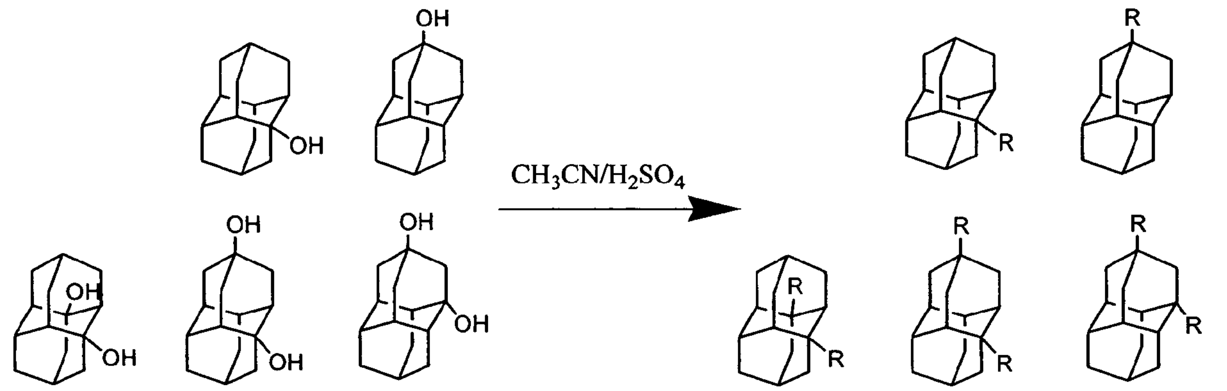


R=NHCOCH$_3$

Scheme 2. Synthesis of acetaminated diamantanes

7 g hydroxylated diamantane (mono- and di-hydroxyl mixtures) was dissolved in 25 mL acetonitrile (HPLC grade) while stirring at room temperature, 25 mL concentrated sulfuric acid was slowly added to the mixture, whereby the mixture heats up by the reraction (note: if $H_2SO_4$ added too fast, $CH_3CN$ will boil). The color of the mixture changed deeper and deeper as the reaction proceeds. After the mixture was stirred for 20 hours (the mixture became brownish with lots of solids precipitated), the mixture was poured onto 200 mL ice. Filtrating the water mixture by suction under an in house vacuum provided white to off-white solids, which were washed twice with water and then air dried to give 3 g of white solids. GC-MS showed high purity of 1- and 4-acetamino diamantane isomer mixture with MW of 245. GC/MS: 245($M^+$); GC/MS: 245($M^+$).

The aqueous solution (400 mL) above was extracted with ethyl acetate for 3 times (3x200 mL) and combined the three extracts together to give a pale yellow clear solution, which was dried with $Na_2SO_4$. After filtration off the $Na_2SO_4xH_2O$, the pale yellow ethyl acetate solution was concentrated under vacuum with rotovap to dryness to give 3 g of oil and solids mixture product. GC-MS analysis of the crude product showed mainly di-acetamino diamantane with minor mono-acetamino diamantane and other impurities, which could be further purified by washing with acetone. To another portion of the aqueous solution (120 mL) was added 100 mL water and extracted with $CH_2Cl_2$ for three times ($3 \times 100$ mL). The combined $CH_2Cl_2$ extracts were then back extracted with water for three times (3x100 mL) and then dried with $Na_2SO_4$. After filtration, the $CH_2Cl_2$ solution was concentrated with rotovap to dryness to give an off-white solid, which GC-MS confirmed to be di-acetamino diamantane. The aqueous solution was extracted with $CH_2Cl_2$ for three times (3x100 mL). The combined $CH_2Cl_2$ extracts were dried with $Na_2SO_4$. Removing the solvent by rotovap gave 1.5 g brown liquid. 10 mL acetone was added into the liquid and a lot of solid was precipitated. The solid was collected by filtration and washed with acetone for three times (3x5 mL. *note:* after filtration, 670 mg of off-white solid was collected. GC-MS analysis of the solid showed di-acetamino diamantane, which was further purified by washing methanol and acetone) and air dried to afford a white solid, which was also characterized as pure di-acetamino diamantane. GC/MS: 302 ($M^+$); GC/MS: 302 ($M^+$).

***Hydroxylation of Acetaminated Diamantanes to Prepare Aminated Diamantanes***

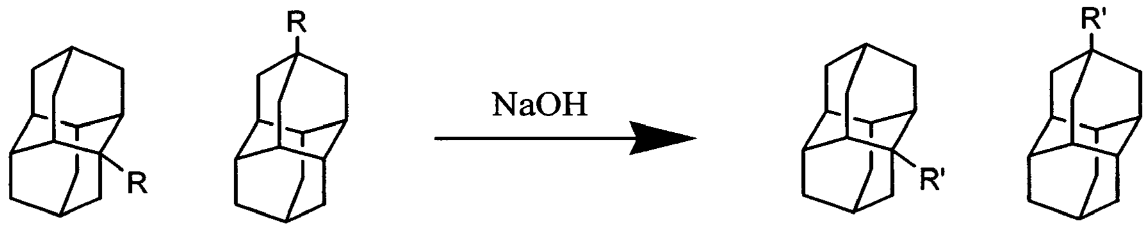


**R=NHCOCH$_3$** **R'=NH$_2$**

Scheme 3. Synthesis of aminated diamantanes

To 3 g of 1- and 4-acetamino diamantane isomer mixture product was added 23 g diethylene glycol (b.p. 245 ˚C) and 2 g NaOH solid (20-40 mesh beads). The mixture was then heated to 200 ˚C and stirred for 5 hours (as the reaction proceeded and the temperature increased, the color of the mixture became deep dark red). The reaction mixture was then poured into 100 mL water. Filtration was conducted to collect the water insoluble solids and washed with plenty of water and dried in air to give 400 mg solid product. GC-MS analysis showed the formation of 1- and 4-amino diamantane with some unreacted mono-acetamino diamantane. It indicated that the reaction needed a longer period of time to go to completion. GC/MS: 203($M^+$); GC/MS: 203($M^+$), 186.

Likewise, starting with 1-acetamino diamantane or 1,6-diacetamino diamantane afforded 1-amino diamantane or 1,6-diamino diamantane. GC/MS: 203 ($M^+$); GC/MS: 218 ($M^+$), respectively.

**Example 2:**

**Preparation of 4-Aminodiamantane Using Trichloramine Reagent**

The 4-amino derivative of diamantane was prepared by the method of Cahill (1) in which an aluminum chloride - $NCl_3$ adduct directs attack to the 4 position of diamantane.

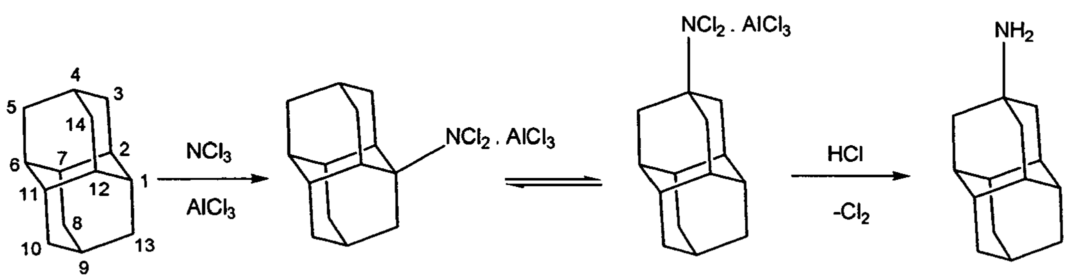


Scheme 4. Synthesis of 4-aminodiamantane

This method uses the trichloramine reagent developed by Kovacic (2, 3), which has been previously used to prepare 1-aminoadiamantane (3, 4) as well as 4-aminodiamantane (1).

**Materials**

***(Ordered from Sigma Aldrich, unless otherwise noted)***

Diamantane (99.9%), FW 188.314, isolated by Shenggao Liu, ChevronTexaco Dichloromethane (HPLC grade),

from Fischer Scientific
1,2-dichloroethane [107-06-2], Cat. No. 27,057-1
Aluminum trichloride, FW133.34 [7446-70-0], Cat. No. 29,471-3
NaOH (50% in water, [1310-73-2], Cat. No. 41,541-3
HCl 37% [7647-01-0], Cat. No. 33,925-3
$Na_2SO_4$ anhydrous, granular [7757-82-6], 23,931-3

***Materials Used to Prepare the Trichloramine ($NCl_3$) Reagent***

Calcium hypochlorite, $Ca(OCl)_2$, FW 142.99 [7778-54-3], Cat. No. 21,138-9
Ammonium chloride, $NH_4Cl$, FW 53.49 [12125-02-9], Cat. No. 21,333-0
Hydrochloric acid (conc.) - see above
HPLC water - Fischer Scientific

Reagent for measuring $NCl_3$ content of the reagent

Sodium iodide, 95.5% [7681-82-5], Cat. No. 38,311-2
Sodium thiosulfate, 0.1N solution [10102-17-7], Cat. No. 31,954-6

**Procedure used to prepare the trichloramine reagent (modified from ref. (2)):**

1. 10 g (0.07mole) of $Ca(OCl)_2$ was suspended in 20 mL of HPLC grade water in a 250 mL 3-neck round-bottom flask (14/20 ground glass joints) with condenser, cooled (Kontes Article No. 633070-0050) addition funnel, thermometer in 14/20 adapter, and magnetic stirring bar.
2. The flask was cooled to ice bath temperatures (~ 4˚C) and 30 mL of cold $CH_2Cl_2$, was added. With the flask and additional funnel at ~ 4˚C, a solution of 2.2 g (0.04 mole) of $NH_4Cl$ in 5 mL of concentrated HCl and 15 mL of HPLC grade water was slowly added to the flask over 30 min. (addition rate = 6 drops/min.) The in-flask thermometer read 40˚F through-out the reaction. A bright yellow color appeared.
3. When the addition was completed, the reaction mixture was stirred an additional 15 min., and then the contents of the flask were poured into a 125 mL separatory funnel. The bottom bright yellow organic phase was separated, washed with HPLC water, and dried over anhydrous $Na_2SO_4$. (The reagent was stored at ice bath temperatures until used).

**Procedure for titrating the trichloramine:**

1. A 1.00 mL aliquot of the $NCl_3$ solution was added to 2 g of sodium iodide in 50 mL of 80% acetic acid.
2. 5.00 mL of this solution was titrated with 0.100 N sodium thiosulfate solution to reduce the liberated iodine to iodide. Two titrations were performed: #1 required 5.25 mL of the 0.100 N sodium thiosulfate, #2: #1 required 5.30 mL of the 0.100 N sodium thiosulfate.

**Procedure for preparing 4-aminodiamantane (modified from ref. (1)):**

1. 1.00 g (5.32 mmol) of the diamantane was dissolved in 60 mL of *dry* 1,2-dichloroethane and placed in a 3-neck 250 mL round-bottom flask (14/20 ground glass joints) with condenser, cooled addition funnel (o-xylene/dry ice, -29˚C), $N_2$ purge line/thermometer adapter and magnetic stirring bar, all placed inside a Dewar cold bath containing o-xylene/dry ice (-29˚C), behind a shield.
2. With the flask cold (-29˚C), 0.75 g (5.5 mmol) of aluminum trichloride was added with a slow $N_2$ purge.
3. 25 mL of cold (-29˚C) $CH_2Cl_2$ was added to 5.32 mL of the cold $NCl_3$ reagent(-29˚C) $CH_2Cl_2$ and placed in the

cooled (-29°C) addition funnel. This cold $NCl_3$-$CH_2Cl_2$ solution was slowly added to the flask drop-wise over a 2 hr. time period.

4. The reaction mixture was brought to -10°C (ethylene glycol/ dry ice, -10.5°C) and stirred at this temperature for 1 hr.

5. The reaction was then quenched by the rapid addition of 80 mL of cold (ice bath temperature) 18% HCL with rapid $N_2$ purging (to remove the $Cl_2$ formed).

6. The aqueous layer was collected and washed once using 60 mL $CH_2Cl_2$ and 60 mL diethylether.

7. The water solution was brought to pH of 9 by the addition of 50% NaOH, extracted three times with 40 mL each of $CH_2Cl_2$, and the extract dried using anhydrous $Na_2SO_4$.

8. The solvent was removed in a rotary evaporator and the product (white solid) stored cold in absence of light and oxygen. Product yield was 130 mg.

9. GCMS analysis showed the product to be 79% 4-aminodiamantane (MS characteristic of 4-aminodiamantane (1)), 8% 1-aminodiamantane, and 13% aminochlorodiamantane. Specifically, the GCMS data for the reaction product provided a total ion chromatogram showing GC peaks at 5.32, 5.41, and 7.35 min., and the mass spectrum of the component (79%) eluting at 5.32 min. showed it to be 4-aminodiamantane (M+ = 203 m/z).

**Example 3:**

**Synthesis of 1,6-Dimethyl-4-aminodiamantane**

Two synthetic routes were designed for the synthesis of 1,6-dimethyl-4-amino-diamantane, shown in Scheme 5 below.

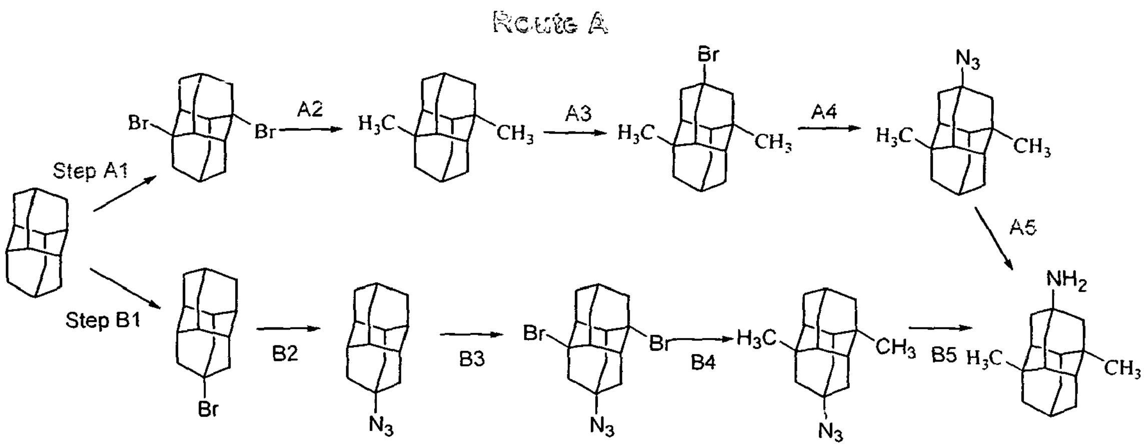


**Route B**

Scheme 5. Synthesis of 1,6-dimethyl-4-aminodiamantane

Proceeding by Route A, the selective di-bromination at the medial positions C-1 and C-6 of diamantane is easier to control, and usually with high yields, when compared with Route B. However, synthesis of the target product via Route B was initially attempted because it was expected that the selective mono-bromination at the apical position C-4 of 1,6-dimethyldiamantane in Route A would be difficult to control and the yield would not be satisfactory based on prior experience of the selective mono-bromination of diamantane at the apical C-4 position to synthesize 4-bromodiamantane. Therefore, proceeding by Route B, 10s of grams of 4-bromodiamantane and then 10s of grams of 4-azidodiamantane were produced. However, at the third reaction step (Step B3 in Scheme 1), an unexpected problem was encountered. When reacting 4-azidodiamantane reacted the neat bromine, the azide group was eliminated during the bromination reaction under conditions thought to be best suitable. Hence, synthesis via Route A was undertaken. Although the target product was successfully synthesized through the five-step Route A, the separation and purification of the intermediate 1,6-dimethyldiamantane of Route A is difficult due to competing of the coupling reaction (methylation) with the elimination of the two bromines of 1,6-dibromodiamantane. More details are set forth below.

***Step 1. Synthesis of 1,6-dibromodiamantane***

| Reagent | MW | Amount | Moles | Eq. |
|---|---|---|---|---|
| Diamantane | 188.3 | 10.0g | 0.053 | 1 |
| Neat Bromine | 159.8 | 25.0ml | 0.488 | 9.2 |
| $NaHSO_3$ | 104.06 | 50.0g | | |
| $CHCl_3$ | 117.91 | 100.0ml | | |
| $Na_2CO_3$ | 105.99 | 50g | | |

Under vigorous stirring, bromine (25.0 ml) was added dropwise to diamantane (10.0 g, 0.053 mole) in a 100 ml three-necked flask equipped with a thermometer and a gas outlet leading to a $Na_2CO_3$ solution and cooled in an ice-bath. The ice-bath was removed after the addition was completed in about 30 min. The reaction mixture was stirred for another 6 h at about 20 ˚C. The mixture was then heated and refluxed for 24 h. After being cooled to room temperature, the mixture was poured onto frozen aqueous sodium hydrogen bisulfite solution. $CHCl_3$ (40.0 ml) was added and the organic layer was separated. The aqueous solution was extracted with $CHCl_3$ (3×20ml). The combined $CHCl_3$ solution was washed with water and dried with anhydrous $CaCl_2$. Evaporation under reduced pressure gave crude product (21.24g). Fractional recrystallization from $CHCl_3$ gave colorless crystals (8.30g, 0.024mol). The mother liquid was concentrated and the mixture was separated by flash column chromatography (silica gel; solvent: petroleum ether), an additional 0.35g 1,6-dibromodiamantane was obtained. The overall yield of the product 1,6-dibromodiamantane was 47.4%. *m.p.* 272˚C. IR ($cm^{-1}$): 2900 (vs), 2854 (s), 1441 (m), 1286 (m), 1068 (m), 972, 877 (m), 795 (m), 715 (m). $^1H$-NMR ($CDCl_3$, ppm): 2.48 (m, 8H), 2.36 (s, 4H), 1.95 (t, 2H), 1.69 (d, 4H). $^{13}C$-NMR ($CDCl_3$, ppm): 52.08, 48.91, 34.15, 30.44. The crystal structure of 1,6-dibromodiamantane with atom numbering is shown in Figure 18.

**Table 2**

| Atomic coordinates (x $10^4$) and equivalent isotropic displacement parameters ($A^2$ x $10^3$) for 1,6-dibromodiamantane. U(eq) is defined as one third of the trace of the orthogonalized Uij tensor. | | | | |
|---|---|---|---|---|
| | **x** | **y** | **z** | **U(eq.)** |
| Br(1) | 1818(1) | 1655(1) | 4984(1) | 56(1) |
| C(1) | 3646(5) | 3646(5) | 5320(3) | 30(1) |
| C(2) | 5730(5) | 3192(5) | 3192(5) | 30(1) |
| C(3) | 5816(6) | 2710(6) | 3886(3) | 41(1) |
| C(4) | 5086(6) | 4263(6) | 3132(3) | 43(1) |
| C(5) | 2986(6) | 4746(6) | 3295(3) | 40(1) |
| C(6) | 2918(5) | 5216(5) | 4529(3) | 31(1) |
| C(7) | 3571(6) | 4169(6) | 6550(3) | 45(1) |

**Table 3**

| Bond lengths [A] and angles [deg] for 1,6-dibromodiamantane | |
|---|---|
| **Bond Lengths** | **Angstrom** |
| Br(1)-C(1) | 2.002(4) |
| C(1)-C(7) | 1.521(5) |
| C(1)-C(6) | 1.529(5) |
| C(1)-C(2) | 1.529(5) |
| C(2)-C(3) | 1.527(5) |
| C(2)-C(6)#1 | 1.547(5) |
| C(3)-C(4) | 1.528(6) |

(continued)

| Bond lengths [A] and angles [deg] for 1,6-dibromodiamantane | |
|---|---|
| **Bond Lengths** | **Angstrom** |
| C(4)-C(7)#1 | 1.522(6) |
| C(4)-C(5) | 1.522(6) |
| C(5)-C(6) | 1.526(5) |
| C(6)-C(2)#1 | 1.547(5) |
| C(7)-C(4)#1 | 1.522(6) |
| **Bond Angles** | |
| C(7)-C(1)-C(6) | 111.5(3) |
| C(7)-C(1)-C(2) | 111.9(3) |
| C(6)-C(1)-C(2) | 108.8(3) |
| C(7)-C(1)-Br(1) | 105.7(2) |
| C(6)-C(1)-Br(1) | 109.0(2) |
| C(2)-C(1)-Br(1) | 109.9(2) |
| C(3)-C(2)-C(1) | 112.2(3) |
| C(3)-C(2)-C(6)#1 | 110.3(3) |
| C(1)-C(2)-C(6)#1 | 107.0(3) |
| C(2)-C(3)-C(4) | 109.2(3) |
| C(7)#1-C(4)-C(5) | 108.9(4) |
| C(7)#1-C(4)-C(3) | 109.0(3) |
| C(5)-C(4)-C(3) | 110.3(3) |
| C(4)-C(5)-C(6) | 109.9(3) |
| C(1)-C(6)-C(5) | 111.6(3) |
| C(1)-C(6)-C(2)#1 | 107.2(3) |
| C(5)-C(6)-C(2)#1 | 110.3(3) |
| C(1)-C(7)-C(4)#1 | 108.9(3) |

Symmetry transformations used to generate equivalent atoms:

#1 -x+1, -y+1, -z+1

**Table 4**

| Anisotropic displacement parameters ($A^2 \times 10^3$) for 1,6-dibromodiamantane. The anisotropic displacement factor exponent takes the form: -2 pi^2 [h^2 a*^2 U11 + ... + 2 h k a* b* U12 ] | | | | | | |
|---|---|---|---|---|---|---|
| | **U11** | **U22** | **U33** | **U23** | **U13** | **U12** |
| Br(1) | 45(1) | 38(1) | 85(1) | 3(1) | 12(1) | -14(1) |
| C(1) | 30(2) | 26(2) | 34(2) | 2(1) | 6(1) | -3(1) |
| C(2) | 32(2) | 25(2) | 32(2) | 4(1) | 2(1) | 4(1) |
| C(3) | 38(2) | 41(2) | 44(2) | -8(2) | 7(2) | 5(2) |
| C(4) | 56(2) | 53(3) | 21(2) | -6(2) | 7(2) | 3(2) |

(continued)

| Anisotropic displacement parameters ($A^2 \times 10^3$) for 1,6-dibromodiamantane. The anisotropic displacement factor exponent takes the form: -2 pi^2 [h^2 a*^2 U11 + ... + 2 h k a* b* U12 ] | | | | | | |
|---|---|---|---|---|---|---|
| | **U11** | **U22** | **U33** | **U23** | **U13** | **U12** |
| C(5) | 44(2) | 44(2) | 29(2) | 0(2) | -8(2) | 2(2) |
| C(6) | 25(2) | 31(2) | 37(2) | 1(2) | 4(1) | 1(1) |
| C(7) | 47(2) | 53(3) | 37(2) | 10(2) | 18(2) | 0(2) |

**Table 5**

| Hydrogen coordinates (x $10^4$) and isotropic displacement parameters ($A^2 \times 10^3$) for 1,6-dibromodiamantane | | | | |
|---|---|---|---|---|
| | **x** | **y** | **z** | **U(eq)** |
| H(2A) | 6181 | 2188 | 5600 | 36 |
| H(3A) | 4992 | 1692 | 3678 | 49 |
| H(3B) | 7162 | 2421 | 3788 | 49 |
| H(4A) | 5119 | 3954 | 2338 | 52 |
| H(5A) | 2110 | 3765 | 3077 | 48 |
| H(5B) | 2539 | 5737 | 2816 | 48 |
| H(6A) | 1557 | 5509 | 4627 | 37 |
| H(7A) | 4006 | 3177 | 7031 | 54 |
| H(8A) | 2227 | 4454 | 6654 | 54 |

***Step 2. Synthesis of 1,6-dimethyldiamantane and other methylated diamantanes***

| **Reagent** | **Amount** | **M.W.** | **moles** | **e.q.** |
|---|---|---|---|---|
| 1,6-dibromodiamantane | 10 g | 346.3 | 0.029 | 1 |
| $CH_3MgI$ (freshly prepared) | 33 g | 165.9 | 0.2 | 6.9 |
| Ethyl ether | 200 ml | | | |
| $CH_2Cl_2$ | 100 ml | | | |
| Mg | 10 g | 24 | 0.4 | |
| $CH_3I$ | 13 ml | 146.9 | 0.2 | |

Grignard reagent was freshly prepared by a common method. Mg (10 g, 0.4 mol) and $CH_3I$ (13 ml, 0.2 mol) were stirred in ethyl ether (200 ml). The ethyl ether was then removed by evaporation under reduced pressure.

Under nitrogen atmosphere, 1,6-dibromodiamantane (10 g, 0.029 mol) and the freshly prepared Grignard reagent (33 g, 0.2 mol) were added to 100 ml anhydrous $CH_2Cl_2$. After refluxing for about 48 hours, the reaction was quenched by pouring the mixture onto ice, and extracted by $CH_2Cl_2$ (5×300ml). The combined extracts were dried and concentrated. The mixture was then subjected to column chromatography (silica gel; solvent: petroleum ether), however all methylated diamantanes eluted at almost the same time, showing poor separation by column chromatography. Therefore, only mixtures containing 1,6-dimethyldiamantane (5.30 g) were obtained. $R_f$= 0.98 (petroleum ether). $^1$H-NMR ($CDCl_3$, 300MHz) δ (ppm): 2.07 (d, *J*=13.00 Hz, 6 H), 1.79 (m, 2 H), 1.66 (m, 1 H), 1.39 (s, 9 H), 0.91 (s, 6 H). $^{13}$C-NMR ($CDCl_3$, 75MHz) δ (ppm): 47.59, 42.91, 33.78, 33.24, 28.13, 26.15.

1,6-dimethyldiamantane was successfully synthesized by reacting the 1,6-dibromodiamantane with the Grig-

nard reagent. $^1H$-NMR showed that methyl groups (0.91ppm, s, 6H) were successfully bonded to the diamantane cage. The mixture should contain 1,6-dimethyldiamantane, 1-methyldiamantane, 1-methyl-6-bromodiamantane, and diamantane because, in principal, when the 1,6-dibromodiamantane reacted with the Grignard reagent, there should be at least four possible products (see below). Three of the products (1,6-dimethyldiamantane, 1-methyldiamantane, and diamantane) are similar non-polar compounds and, thus, it is difficult to separate them by column chromatography. Consequently, it was decided to proceed to the next bromination reaction without further purification of the methyldiamantanes mixture.

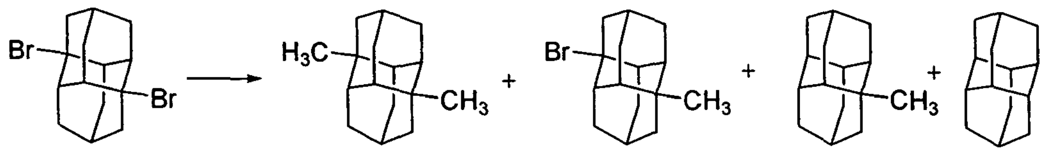

**Some possible alkylation products of 1,6-dibromodiamantane with the Grignard reagent**

***Step 3. Synthesis of 1,6-dimethyldiamantane bromides mixture***

| Reagent | Amount | M.W. | moles | e.q. |
|---|---|---|---|---|
| 1,6-dimethyldiamantane mixture | 5 g | 216.3 | 0.023 | 1.0 |
| *t*-BuBr | 4 g | 137 | 0.03 | 1.30 |
| $AlBr_3$ | 0.2 g | 263 | 0.0008 | 0.03 |
| Anhydrous cyclohexane | 50 ml | 84 | | |

The mixture from step 2 containing 1,6-dimethyldiamantane (5.0 g, 0.023 mol) was dissolved in 50 ml anhydrous cyclohexane. *t*-BuBr (4 g, 0.03 mol) was then added to the solution. Under argon atmosphere, $AlBr_3$ (0.1 g) was added to the mixture, stirred for about 6 hours at about 0°C . Then a second batch of the $AlBr_3$ catalyst (0.1 g) was added. After stirring for an additional 2 hours, the reaction was quenched by pouring the reaction mixture into ice-water followed by extraction with $CH_2Cl_2$ (3×200ml). The combined $CH_2Cl_2$ extract was dried and concentrated. The product 1,6-dimethyl-2,4-dibromodiamantane (2.5 g, 0.007 mol) was crystallized out from the mother liquid. $R_f$=0.32 (petroleum ether). $^1H$-NMR ($CDCl_3$, 300MHz) δ (ppm): 2.63 (d, *J*=12.83 Hz, 4 H), 2.27 (s, 5 H), 2.04 (m, 3 H), 1.62 (m, 4 H), 1.05 (m, 6 H). $^{13}C$-NMR ($CDCl_3$, 75MHz) δ (ppm): 63.70, 63.38, 56.77, 56.13, 49.07, 48.44, 45.09, 44.28, 43.81, 43.75, 40.52, 39.35, 37.76, 37.30, 25.92, 25.57. $EI^+$: 373 [M+H] +

After filtration and collection of the 1,6-dimethyl-2,4-dibromodiamantane, the mother liquid was further concentrated by rota evaporation under reduced pressure. The concentrated mother liquid was subjected to flash column chromatography (silica gel; solvent: petroleum ether), resulting in a mixture (2.0 g) of 1,6-dimethyl-2-bromodiamantane and 1,6-dimethyl-4-bromodiamantane with other mono-methylated diamantane bromides, which are difficult to separate by column chromatography. Therefore, the methyl diamantane bromides mixture was not further purified and was directly used for next reaction step in order to produce a variety of compounds with the hope of separating each of the methyl azidodiamantanes.

***Step 4. Synthesis of 1,6-dimethyl-2,4-diazidodiamantane***

| Reagent | Amount | M.W. | moles | e.q. |
|---|---|---|---|---|
| 1,6-dimethyl-2,4-dibromodiamantane | 1 g 372 | | 0.003 | 1.0 |
| TMSA | 1.8 g | 115 | 0.015 | 5.0 |
| Anhydrous $SnCl_4$ | 1 ml | | | |

1,6-dimethyl-2,4-dibromodiamantane (1.0 g, 0.003 mol) was dissolved in 20 ml anhydrous $CH_2Cl_2$, TMSA (1.8

g, 0.015 mol) and anhydrous $SnCl_4$ (1 ml) were then added to the solution. Under argon atmosphere, the reaction solution was heated to reflux for about 5 hours. Iit was quenched by pouring the reaction mixture into ice-water, and then extracted with $CH_2Cl_2$ (3×50 ml). The combined $CH_2Cl_2$ extracts were concentrated by rota evaporation under reduced pressure to collect the 1,6-dimethyl-2,4-diazido- diamantane. IR (KBr; $cm^{-1}$): 2923 (m), 2971 (m), 2095 (s, $-N_3$). Strong absorption at 2095 $cm^{-1}$ indicates the presence of the azide group. It was directly reduced to the diamine without purification.

***Step 5. Synthesis of 1,6-dimethyl-2,4-diaminodiamantane***

The above 1,6-dimethyl-2,4-diazidodiamantane mixture was dissolved in 20 ml methanol followed by adding Pd/C (50 mg) as the reducing reagent. The mixture was stirred in hydrogen atmosphere for about 12 hours, then concentrated by rota evaporation under reduced pressure giving the 1,6-dimethyl-2,4-diaminodiamantane as a white solid (200 mg, 0.0008 mol) without purification $R_f$=0.17 (MeOH:EA=1:3). $^1H$-NMR ($CD_3OD$, 300MHz) δ (ppm): 1.89 (m, 4 H), 1.79 (s, 2 H), 1.68 (s, 1 H), 1.52 (m, 6 H), 0.87 (m, 1 H), 1.27 (d, J=10.03 Hz, 8 H), 1.38 (m, 6 H). $^{13}C$-NMR ($CD_3OD$, 75MHz) δ (ppm): 54.68, 54.15, 48.55, 47.46, 46.27, 45.66, 44.82, 43.82, 40.99, 40.82, 40.39, 39.06, 36.08, 35.65, 27.04, 26.69. $EI^+$: 246 $[M]^+$. This product was a mixture of compounds and was designated as MDT-9. Figure 19 shows the GC-MS total ion chromatogram (TIC) for the final crude synthetic product of MDT-9.

***Step 6. Synthesis of 1,6-dimethyl-4-azidodiamantane mixture***

The mixture of 1,6-dmicthyl-4-bromodiamantane and 1,6-dimethyl-2-bromodiamantane with their mono-methylated analogs were reacted in the same way as the 1,6-dimethyl-2,4-dibromodiamantane described in Step 4 above. Therefore, more mixed dimethyl or monomethyl azidodiamantane derivatives including the 1,6-dimethyl-2-azidodiamantane and the 1,6-dimethyl-4-azidodiamantane were obtained. The mixture from the bromination of 1,6-dimethyldiamantane mixture after removing the 1,6-dimethyl-2,4-dibromodiamantane mainly contained 1,6-dimethyl-4-bromodiamantane, 1,6-dimethyl-2-bromodiamantane and their mono-methyl bromides. It was directly reacted with TMSA without further purification. After reacting with TMSA, four compounds from the reaction mixture were separated by column chromatography on silica gel and all of them were characterized to have the azide group by IR analysis, which showed a strong characteristic absorption of the azide group at around 2095 $cm^{-1}$. TLC showed only one spot for each fraction, but the $^1H$- and $^{13}C$-NMR spectra were so complex that it is believed each fraction is still a mixture. The four fractions were designated as 1,6-dimethyl-4-azidodiamantane, 1,6-dimethyl-2-azidodiamantane, 1,6-dimethyl-mono-azidodiamantane, and 1,6- or 1,7-dimethyl-mono-azidodiamantane based on preliminary $^{13}C$-NMR analysis. These four fractions were not further purified.

***Step 7. Synthesis of 1,6-dimethyl-2-aminodiamantane mixture***

The above azidodiamantane mixture designated 1,6-dimethyl-2-azidodiamantane was reduced by Pd/C in $H_2$ environment to produce the corresponding amino compound mixture as described in Step 5. Mass spectra showed that it was a 1,6-dimethyl mono-aminodiamantane. TLC showed only one clear spot, but the $^{13}C$-NMR spectra were so complex that it is believed the product is still a mixture containing compounds such as 1-methyl-2-aminodiamantane. EI+:231 $[M]^+$. This product was designated as MDT-6. Figure 20 shows the GC-MS total ion chromatogram (TIC) for the final crude synthetic product of MDT-6.

***Step 8. Synthesis of 1,6-dimethyl-mono-aminodiamantane mixture***

The above azidodiamantane mixture designated 1,6-dimethyl-mono-azidodiamantane was reduced by Pd/C in $H_2$ environment to produce the corresponding amino compound mixture as described in Step 5. Mass spectra showed that it was a dimethyl mono-aminodiamantane. TLC showed only one clear spot, but the $^{13}C$-NMR spectra were so complex that it is believed the product is still a mixture. EI+: 231 $[M]^+$.

***Step 9. Synthesis of 1,6- or 1,7-dimethyl-mono-aminodiamantane mixture***

The above azidodiamantane mixture designated 1,6- or 1,7-dimethyl-mono-azidodiamantane was reduced by Pd/C in $H_2$ environment to produce the corresponding amino compound mixture as described in Step 5. Mass spectra showed a *m*/*z* at 231. TLC showed only one clear spot, but the $^{13}C$-NMR spectra were so complex that it is believed the product is still a mixture. EI+: 231 $[M]^+$. During the multiple step reaction, it was observed that the methyl group may be rearranged to a different position. Therefore, the methyldiamantane mixtures may contain 1,7-dimethyldiamantane derivatives even though the starting precursor was 1,6-dibromodiamantane.

***Step 10. Synthesis of 1,6-dimethyl-4-aminodiamantane mixture***

The above azidodiamantane mixture designated 1,6-dimethyl-4-azidodiamantane was reduced by Pd/C in $H_2$ environment to produce the corresponding amino compound mixture as described in Step 5. TLC showed only one clear spot, but the $^{13}C$-NMR spectra were so complex that it is believed the product is still a mixture. This crude synthetic product is referred to herein as MDT-7. Mass spectrum (m/z: 231, 217, below) showed that it contained dimethyl mono-aminodiamantane with m/z at 231. In addition, the mass spectrum showed a strong peak at m/z 217 which represented a mono-methyl-mono-aminodiamantane.

**Example 4:**

**Purification of MDT-21, MDT-22 and MDT-23**

***Instrumentation/Equipment***

Gas Chromatographic Mass Spectral (GC-MS) analysis was performed on an Agilent model 6890 gas chromatograph equipped with an Agilent model 7683 autosampler and an Agilent model 5973 Network Mass Selective Detector. The GC was run in the splitless mode in an Agilent HP-MS5 column (30m by 0.25 mm I.D., 0.25μ phase thickness) with helium carrier gas at a flow rate of 1.2 mL/min (constant flow mode) and inlet pressure of 16 psi. During GC-MS analyses, the GC oven had a 1.0 min initial hold time at 150°C, followed by oven programming at 10°C/min to 320°C with a final hold time of 15 min. The GC-MS transfer line temperature was maintained at 320 °C. A solvent delay of 2.5 min was applied for GC-MS data accumulation. Trimethylsilyl (TMS) ether derivatives of the amines were prepared using standard procedures (N,O-bis-(trimethylsilyl)-trifluoroacetamide(BSTFA), Pierce Chemical Company, Rockford, IL).

High-resolution mass spectra where measured on Micromass GCT TOFMS (time-of-flight mass spectrometer).

High Performance Liquid Chromatography (HPLC): the HPLC system consisted of a Waters Prep LC 4000 solvent pumping system (Waters Corporation, Milford, Massachusetts) in line with a Rheodyne Model 7125 sample injection valve (Rheodyne LLC, Cotati, California) fitted with a 50 microliter injection loop, used to inject samples into an in-line Hypercarb 10 mm I.D. by 250 mm long HPLC column (ThermoElectron Corporation, Bellefonte, Pennsylvania) containing 5- micron particle-size Hypercarb packing. The HPLC detector was a Waters Model 2410 Differential Refractometer, and HPLC chromatograms were collected using a Hewlett-Packard Chemstation Data system (Chemstation Rev. A.05.02 [273] software running on a Hewlett-Packard Vectra computer). Fractions were collected manually into Fisher 10 mm by 150 mm tubes and analyzed using the GCMS system described above. The mobile phase developed for the separation herein consisted of a mixture of methanol, water, and triethylamine, in the 95/5/1 volume ratios. A basic mobile phase prepared without inorganic salts made it possible to retrieve isolated methyldiamantane amines by simple solvent removal under a stream of dry nitrogen. Fisher solvents (Fisher Scientific, Chicago, Illinois) were consistently used for this application. It is desirable, in reversed-phase HPLC separations of amines, to use a high-pH material (in this case triethylamine) in the mobile phase to insure that amines remained unprotonated (uncharged) so that effective interactions with the hydrophobic HPLC column packing occur. A polar, water-containing mobile phase forces the methylated aminodiamantanes to interact with the hydrophobic stationary phase, thus effecting separation. A Hypercarb HPLC column was used because it is especially effective at providing separations of closely related isomer mixtures.

The Carbon-13 ($^{13}C$) and Hydrogen-1 ($^1H$) nuclear magnetic resonance (NMR) spectra were recorded for MDT-22 and MDT-23. The NMR spectra were recorded at room temperature on a Bruker AVANCE 500 spectrometer operating at 125.7537 MHz for $^{13}C$ nuclei and 500.115 MHz for $^1H$ nuclei. Both spectra were obtained using deuterated chloroform ($CDCl_3$) as the solvent and TMS as the reference.

***Separation and Purification***

The final crude synthesis product from step 10 of Example 3 was a mixture of diamantane compounds believed to include a dimethyl mono-aminodiamantane and a mono-methyl-mono-aminodiamantane, among other compounds. This product is also designated herein as MDT-7. MDT-7 was subjected to further purification to provide fractions designated as MDT-21 (an intermediate purity MDT-22), MDT-22 and MDT-23, the details of which are provided below.

Gas Chromatographic Mass Spectral (GCMS) analysis of the reaction product MDT-7 showed three major components and some minor components including unmethylated, monomethyl-, dimethyl-, trimethyl-, tetramethyl-, pentamethyl-, and hexamethyl-monoaminodiamantanes. Figure 21 shows the GC-MS total ion chromatogram (TIC) for the final crude synthetic product of MDT-7. The TIC peaks corresponding to MDT-22 and MDT-23 are indicted on Figure 21. This crude synthetic product was then purified by HPLC using the method described above.

HPLC separations were run at a mobile phase flow rate of 1.5 mL/ min. Figure 22 shows the HPLC chromatogram of the crude product (i.e., MDT-7). In Figure 22, 301 indicates the HPLC peak corresponding to the elution time of MDT-22 and 307 indicates the HPLC peak corresponding to the elution time of MDT-23.

A preparative HPLC isolation of MDT-21, MDT-22 and MDT-23 was undertaken using a high-sample loading of 43 mg of the crude product in the preparative HPLC runs. Five HPLC runs were made in which fractions were taken at elution times shown at 302, 303, 304, 305, and 306 in Figure 22. The fractions corresponding to 302 in Figure 22 from the various HPLC runs were combined into a single sample (15.1 mg) for MDT-21. This sample of MDT-21 was converted into the hydrochloride salt and submitted for biological testing. Figure 23 shows the GC-MS total ion chromatogram (TIC) for the final crude synthetic product of MDT-21 indicating it is an intermediate purity sample of MDT-22.

A second HPLC fraction corresponding to cut 303 in Figure 22 from the series of preparative runs was collected and combined to give a total of 21 mg of a product enriched in MDT-22. This product was further purified using the same HPLC system, but at a lower sample loading (~3.5 mg per run) giving improved separations. Tight fractions were taken at the elution time corresponding to peak 301 in Figure 22. Five separate HPLC runs were carried-out. The early-eluting fractions rich in MDT-22 were combined to provide a single sample of MDT-22 (later-eluting fractions were retained for use in the preparation of a sample of MDT-23). This sample of MDT-22 was converted into the hydrochloride salt and submitted for biological testing.

A third HPLC fraction corresponding to cut 306 in Figure 22 from the 5 series of preparative runs was collected and combined to give a sample of MDT-23 (4.7mg) . This sample was converted into the hydrochloride salt and submitted for biological testing. Figure 24 shows the GC-MS total ion chromatogram (TIC) of MDT-23 used for biological testing. Fractions 304 and 305 and late-eluting fractions retained from the final series of HPLC runs used to purify MDT-22, contained MDT-23. These fractions were further purified using the same HPLC system, but at a lower sample loading giving improved separations. Tight fractions were taken at the elution time corresponding to peak 307 in Figure 22. Fractions richest in MDT-23 were identified by GC-MS analyses, and were combined to provide a sample of MDT-23 submitted for additional spectral analyses.

***Spectral analysis of AMT-22 and MDT-23***

Figures 25 and 26 show the GC-MS TIC trace and mass spectrum, respectively, of MDT-22. The peak corresponding to MDT-22 is indicated in the Figure 25 TIC. Figure 26 shows the mass spectrum of MDT-22 with a molecular ion at m/z 217 and base peak at m/z 120. High-resolution mass spectral analyses showed the molecular ion of MDT-22 to have a mass of 217.1900 (calculated 217.1830 for $C_{15}H_{23}N$). A sample of MDT-22 was derivatized to form the trimethylsilyl (TMS) ether. GC-MS analysis of the TMS product showed comparable purity to the GC-MS TIC of the MDT-22 free amine shown in Figure 25. The mass spectrum of the major TMS ether product showed a molecular ion of m/z 289, increased over the free amine by 72 mass units by the TMS moiety, further demonstrating the presence of the amine group in MDT-22. The $^1$H- and $^{13}$C-NMR spectra of MDT-22 are shown in Figures 29 and 30, respectively.

The GCMS TIC of MDT-23 is shown in Figure 27 with the corresponding mass spectrum shown in Figure 28. The peak corresponding to MDT-23 is indicated in the Figure 27 TIC. Figure 28 shows the mass spectrum of MDT-23 with a molecular ion at m/z 231 and base peak at m/z 120. High-resolution mass spectral analyses showed the molecular ion of MDT-23 to have a mass of 231.2036 (calculated 231.1987 for $C_{16}H_{25}N$). A sample of MDT-23 was derivatized to form the trimethylsilyl (TMS) ether. GC-MS analysis of the TMS product showed comparable purity as the GC-MS TIC of the MDT-23 free amine shown in Figure 27. The mass spectrum of the major product showed a molecular ion of m/z 303, increased by 72 mass units by the TMS moiety, demonstrating the presence of the amine group in MDT-23. Figures 31 and 32 show the $^1$H- and $^{13}$C-NMRs, respectively, of MDT-23.

***Preparation of the hydrochloride salts from the free amines***

For testing, MDT-21, MDT-22 and MDT-23 were further converted into the watersoluble hydrochloride salt. The free amine was dissolved in dry diethyl ether, capped and place in an ice bath to cool. 1M HCl in diethyl ether was also capped and cooled in the ice bath. The molar-equivalent of 1M HCl in diethyl ether was added to the solution of the free amine, and a white precipitate formed at varying rates depending on the composition of the amine and its concentration in the ether. For quantities of amine greater than -10 mg, the solution containing the precipitate can be poured into a Millipore filter (0.5micron Teflon). The filtered precipitate is then washed with excess dry diethyl ether, dried on the filter and transferred to a tightly capped vial. For amounts smaller than ~10 mg, it can be difficult to retrieve the precipitate from the Millipore filter. In those cases, the precipitate was not filtered, but allowed to coagulate and settle to the bottom of the capped tube in which it was formed. The HCl-containing ether was then carefully decanted, and the precipitate resuspended in dry ether. This process was repeated until no acid could be detected (using pH paper) in the vapor emitted when the ether solution was slowly evaporated in a stream of dry nitrogen. When acid could no longer be detected, then the remaining ether was removed by evaporation in a gentle stream of dry nitrogen at ambient

temperature, yielding a bright white, powdery solid.

**Example 5:**

**Alternate synthesis, purification and structure of MDT-23**

A modification of Route A of Scheme 5 (see Example 3 above) was developed which resulted in the production of MDT-23 of greater purity. The details of this alternate synthetic route are set forth below.

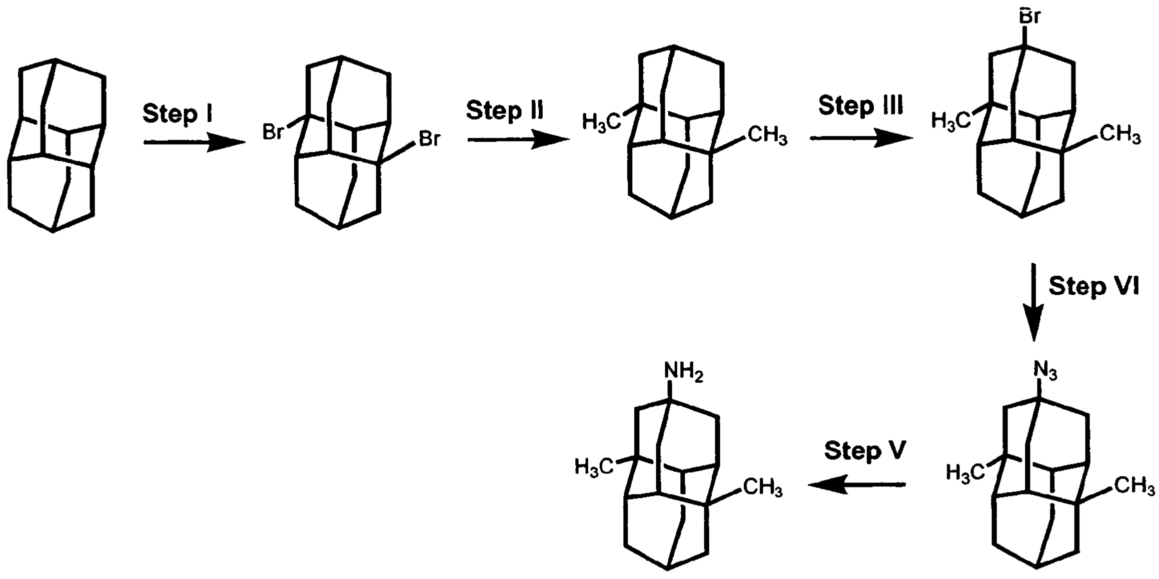


MDT-23

Scheme 6. Synthesis of MDT-23

***Step L Synthesis of 1,6-dibromo-diamantane***

1,6-Dibromo-diamantane was synthesized according to the procedure set forth in Step 1 of Examiple 3. The IR and $^1$H-, $^{13}$C- and $^{13}$C-$^1$H Cosy NMR spectra of 1,6-dibromo-diamantane are shown in Figures 34, 35, 36 and 37, respectively.

***Step II. Synthesis of 1,6-dimethyl-diamantane***

To a solution of 1,6-dibromo-diamantane (40 g, 0.11 mol) in *n*-$Bu_2O$ was added a solution of methyl magnesium bromide in *n*-butyl ether (3 M, 310 mL, 0.93 mmol) at 140 °C under argon atmosphere. Stirring was continued for 2h and then cooled down to room temperature. The reaction mixture was quenched by the addition of saturated aqueous solution of $NH_4Cl$, followed by petroleum ether extraction (3×150 mL). The combined organic layers were dried over anhydrous $Na_2SO_4$, filtered, and then the solvent was evaporated to obtain a crude product. The crude product was further purified by recrystallization to give 1,6-dimethyl-diamantane as a white solid (24.1 g, 96.5%). The GC-MS, $^1$H- and $^{13}$C-NMR spectra of 1,6-dimethyl-diamantane are shown in Figures 38, 39 and 40, respectively. $R_f$=0.92 (petroleum ether). $^1$H-NMR (300 MHz, $CDCl_3$, δ, ppm): 0.91 (s, 6 H), 1.36-1.40 (m, 12 H), 1.78 (d, *J*=2.79 Hz, 2 H), 2.07 (d, *J*=13.13 Hz, 4 H). $^{13}$C-NMR (75 MHz, $CDCl_3$, δ, ppm): 26.17, 28.14, 33.25, 33.79, 42.92, 47.60. GC-MS: 216, 201 (base peak). Purity is approximately 93.5% by GC-MS.

***Step III. Synthesis of 4-bromo-1,6-dimethyl-diamantane***

1,6-Dimethyl-diamantane (4.0 g, 18.5 mmol) was dissolved in dry chloroform (100 mL) in a flask accommodated in a cooling bath at -10°C. Anhydrous $AlBr_3$ (49 mg, 0.185 mmol) and a solution of bromine (1.14 mL, 22.2 mmol) in anhydrous DCM (20 mL) were added and the mixture was stirred for about 30 min. The reaction mixture was poured into an ice-$NaHSO_3$ mixture. The organic layer was separated and the aqueous layer was extracted 2 times with DCM.

The combined extract was washed with saturated aqueous solution of $NaHCO_3$, then NaCl aqueous solution, and finally was dried over anhydrous $Na_2SO_4$. The solvent was then evaporated to obtain a crude product. The crude product was further purified by flash chromatography using petroleum ether as the eluent to give 4-bromo-1,6-dimethyl-diamantane as a white solid (2.5 g, 46.5%). The $^1$H- and $^{13}$C-NMR spectra of 4-bromo-1,6-dimethyl-diamantane are shown in Figures 41 and 42, respectively. $R_f$=0.72 (petroleum ether). $^1$H-NMR (300 MHz, $CDCl_3$, δ, ppm): 0.97,0.98 (s, s, 3 H, 3 H), 1.41-1.45 (m, 6 H), 1.79-1.80 (m, 1 H), 1.65 (s, 1 H), 1.80-1.82 (m, 1 H), 2.01-2.05 (m, 6 H), 2.69 (d, *J*=11.67 Hz, 4 H). $^{13}$C-NMR (75 MHz, $CDCl_3$, δ, ppm): 25.71, 26.05, 27.39, 32.23, 32.60, 38.68, 41.16, 44.84, 46.34, 46.99, 58.04, 66.55.

***Step IV. Synthesis of 4-azido-1,6-dimethyl-diamantane***

To a stirred solution of 4-bromo-1,6-dimethyl-diamantane (1.0 g, 3.4 mmol) and trimethylsilyl azide (1.34 mL, 6.8 mmol) in dry DCM (50 mL), stannic chloride (0.34 mL) was added under nitrogen at 0 ˚C. The temperature of the mixture was then raised to room temperature, followed by prolonged stirring for 4 h. After the reaction was complete, the mixture was quenched with ice-water (30 mL), followed by DCM extraction (2×30 mL). The combined organic layers were dried over anhydrous $MgSO_4$ and the solvent was evaporated to obtain a crude product. The crude product was further purified by silica gel column chromatography using petroleum ether as the eluent to give 4-azido-1,6-dimethyl-diamantane as a white solid (0.78 g, 89.3%). The $^1$H- and $^{13}$C-NMR spectra of 4-azido-1,6-dimethyl-diamantane are shown in Figures 43 and 44, respectively. $R_f$=0.58 (petroleum ether).$^1$H-NMR (300 MHz, $CDCl_3$, δ, ppm): 0.94, 0.99 (s, s, 3 H, 3 H), 1.40-1.49 (m, 9 H), 1.59 (s, 1 H), 1.65 (s, 1 H), 1.80-1.82 (m, 1 H), 2.10 (dd, *J*=12.15, 23.96 Hz, 4 H). $^{13}$C-NMR (75 MHz, $CDCl_3$, δ, ppm): 25.83, 25.93, 27.56, 32.26, 32.87, 35.86, 37.04, 41.51, 44.43, 46.36, 50.41, 59.31.

***Step V. Synthesis of 4-amino-1,6-dimethyl-diamantane***

A solution of 4-azido-1,6-dimethyl-diamantane (0.57 g, 2.2 mmol) in methanol (35 mL) was stirred vigorously with Pd-C (10%, 60 mg) in a hydrogen atmosphere for 12 h. Filtration and evaporation of the solvent *in vacuo* yielded 4-amino-1,6-dimethyl-diamantane as a white solid (0.51 g, 100%). The GC-MS, $^1$H- and $^{13}$C-NMR, and Dept-135-NMR spectra of 4-amino-1,6-dimethyl-diamantane are shown in Figures 45, 46 and 47, respectively. Prior to analysis by $^1$H- and $^{13}$C-NMR, the 4-amino-1,6-dimethyl-diamantane was converted to the hydrochloride salt. The hydrochloride salt was also used for the the X-ray crystallographic analysis and biological testing described below. $^1$H-NMR (300 MHz, $CD_3OD$, δ, ppm): 0.98 (s, 3 H), 1.04 (s, 3 H), 1.45-1.58 (m, 10 H), 1.71 (s, 2 H), 1.83 (s, 1 H), 2.14 (d, *J*=12.86 Hz, 2 H), 2.25 (d, *J*=11.88 Hz, 2 H). $^{13}$C-NMR (75 MHz, $CD_3OD$, δ, ppm): 26.13, 26.31, 28.93, 33.14, 33.71, 36.40, 37.04, 42.61, 44.97, 47.25, 50.39, 53.24. The crystal structure of 4-amino-1,6-dimethyl-diamantane with atom numbering is shown in Figure 48.

**Table 6**

Atomic coordinates (x $10^4$) and equivalent isotropic displacement parameters (Å$^2$ x $10^3$) for 4-amino-1,6-dimethyldiamantane hydrochloride monohydrate. U(eq) is defined as one third of the trace of the orthogonalized $U^{ij}$ tensor.

| | x | y | z | U(eq) |
|---|---|---|---|---|
| Cl(1) | 6068(1) | 2070(1) | 4263(1) | 19(1) |
| O(1A) | 12241(3) | 2703(1) | 3372(2) | 20(1) |
| O(1B) | 11748(6) | 2576(1) | 3166(4) | 24(1) |
| N(1) | 9261(2) | 2861(1) | 4909(1) | 16(1) |
| C(1) | 8058(1) | 4212(1) | 5278(1) | 13(1) |
| C(2) | 6152(1) | 3896(1) | 4692(1) | 14(1) |
| C(3) | 6903(2) | 3434(1) | 5240(1) | 14(1) |
| C(4) | 8449(2) | 3307(1) | 4312(1) | 13(1) |
| C(5) | 7298(2) | 3319(1) | 2262(1) | 15(1) |
| C(6) | 6483(2) | 3778(1) | 1641(1) | 14(1) |
| C(7) | 4966(1) | 3922(1) | 2611(1) | 15(1) |
| C(8) | 4175(2) | 4384(1) | 2072(1) | 19(1) |
| C(9) | 6076(2) | 4692(1) | 2612(1) | 18(1) |
| C(10) | 7621(2) | 4559(1) | 1681(1) | 18(1) |
| C(11) | 8382(2) | 4096(1) | 2216(1) | 14(1) |
| C(12) | 9562(1) | 4070(1) | 4296(1) | 13(1) |

(continued)

Atomic coordinates (x $10^4$) and equivalent isotropic displacement parameters (Å$^2$ x $10^3$) for 4-amino-1,6-dimethyldiamantane hydrochloride monohydrate. U(eq) is defined as one third of the trace of the orthogonalized $U^{ij}$ tensor.

| | x | y | z | U(eq) |
|---|---|---|---|---|
| C(13) | 7231(2) | 4668(1) | 4666(1) | 17(1) |
| C(14) | 10363(1) | 3611(1) | 4841(1) | 14(1) |
| C(15) | 9222(2) | 4225(1) | 7342(1) | 18(1) |
| C(16) | 5354(2) | 3760(1) | -428(1) | 21(1) |

**Table 7**

Bond lengths [Å] and angles [deg] for 4-amino-1,6-dimethyldiamantane hydrochloride monohydrate.

| | | | |
|---|---|---|---|
| O(1-A-H(1D) | 0.901(16) | C(7)-C(8) | 1.5374(14) |
| O(1A)-H(1E) | 0.905(16) | C(7)-H(7) | 0.981(15) |
| O(1B)-H(1D) | 0.963/(16) | C(8)-C(9) | 1.5346(15) |
| O(1B)-H(1E) | 0.936(17) | C(8)-H(8A) | 0.967(16) |
| N(1)-C(4) | 1.5026(12) | C(8)-H(8B) | 0.980(16) |
| N(1)-H(1A) | 0.885(17) | C(9)-C(13) | 1.5283(14) |
| N(1)-H(1B) | 0.930(17) | C(9)-C(10) | 1.5367(15) |
| N(1)-H(1C) | 0.911(17) | C(9)-H(9) | 0.977(16) |
| C(1)-C(15) | 1.5343(13) | C(10)-C(11) | 1.5375(14) |
| C(1)-C(13) | 1.5365(13) | C(10)-H(10A) | 0.982(15) |
| C(1)-C(12) | 1.5499(13) | C(10)-H(10B) | 0.986(15) |
| C(1)-C(2) | 1.5520(13) | C(11)-C(12) | 1.5485(13) |
| C(2)-C(3) | 1.5332(13) | C(11)-H(11) | 0.982(14) |
| C(2)-C(7) | 1.5496(13) | C(12)-C(14) | 1.5330(13) |
| C(2)-H(2) | 0.954(15) | C(12)-H(12) | 0.994(14) |
| C(3)-C(4) | 1.5303(13) | C(13)-H(13A) | 0.983(15) |
| C(3)-H(3A) | 0.943(15) | C(13)-H(13B) | 0.982(16) |
| C(3)-H(3B) | 0.971(15) | C(14)-H(14A) | 0.976(14) |
| C(4)-C(5) | 1.5233(13) | C(14)-H(14B) | 0.955(14 |
| C(4)-C(14) | 1.5320(13) | C(15)-H(15A) | 0.966(17) |
| C(5)-C(6) | 1.5434(13) | C(15)-H(15B) | 0.988(17) |
| C(5)-H(5A) | 0.975(15) | C(15)-H(15C) | 0.975(17) |
| C(5)-H(5B) | 0.989(15) | C(16)-H(16A) | 0.949(18) |
| C(6)-C(16) | 1.5354(13) | C(16)-H(16B) | 0.970(17) |
| C(6)-C(11) | 1.5513(13) | C(16)-H(16C) | 0.976(18) |
| C(6)-C(7) | 1.5530(13) | | |
| | | | |
| H(1D)-O(1A)-H(1E) | 104.7(14) | H(1A)-N(1)-H(1C) | 109.1(15) |
| H(1D)-O(1B)-H(1E) | 97.7(14) | H(1B)-N91)-H(1C) | 107.5(14) |
| C(4)-N(1)-H(1A) | 111.6(11) | C(15)-C(1)-C(13) | 107.30(8) |
| C(4)-N(1)-H(1B) | 112.6(10) | C(15)-C(1)-C(12) | 111.41(8) |
| H(IA)-N(1)-H(1B) | 106.9(14) | C(13)-C(1)-C(12) | 109.52(8) |
| C(4)-N(1)-H(1C) | 109.1(10) | C(15)-C(1)-C(2) | 112.61(8) |
| C(13)-C(1)-C(2) | 108.83(8) | C(2)-C(7)-H(7) | 109.3(9) |
| C(12)-C(1)-C(2) | 107.13(7) | C(6)-C(7)-H(7) | 107.7(9) |
| C(3)-C(2)-C(7) | 109.93(8) | C(9)-C(8)-C(7) | 109.55(8) |
| C(3)-C(2)-C(1) | 110.82(7) | C(9)-C(8)-H(8A) | 111.5(9) |
| C(7)-C(2)-C(1) | 109.69(7) | C(7)-C(8)-H(8A) | 110.7(9) |

(continued)

Bond lengths [Å] and angles [deg] for 4-amino-1,6-dimethyldiamantane hydrochloride monohydrate.

| | | | |
|---|---|---|---|
| C(3)-C(2)-H(2) | 108.8(9) | C(9)-C(8)-H(8B) | 109.7(9) |
| C(7)-C(2)-H(2) | 108.1 (9) | C(7)-C(8)-H(8B) | 109.0(9) |
| C(1)-C(2)-H(2) | 109.4(9) | H(8A)-C(8)-H(8B) | 106.4(13) |
| C(4)-C(3)-C(2) | 108.58(7) | C(13)-C(9)-C(8) | 108.55(8) |
| C(4)-C(3)-H(3A) | 110.2(9) | C(13)-C(9)-C(10) | 108.80(8) |
| C(2)-C(3)-H(3A) | 112.6(9) | C(8)-C(9)-C(10) | 110.13(8) |
| C(4)-C(3)-H(3B) | 107.6(9) | C(13)-C(9)-H(9) | 109.0(9) |
| C(2)-C(3)-H(3B) | 112.0(9) | C(8)-C(9)-H(9) | 109.6(9) |
| H(3A)-C(3)-H(3B) | 105.9(12) | C(10)-C(9)-H(9) | 110.7(9) |
| N(1)-C(4)-C(5) | 110.18(7) | C(9)-C(10)-C(11) | 109.76(8) |
| N(1)-C(4)-C(3) | 108.74(7) | C(9)-C(10)-H(10A) | 109.5(9) |
| C(5)-C(4)-C(3) | 109.30(8) | C(11)-C(10)-H(10A) | 109.9(9) |
| N(1)-C(4)-C(14) | 107.88(7) | C(9)-C(10)-H(10B) | 110.7(9) |
| C(5)-C(4)-C(14) | 109.25(7) | C(11)-C(10)-H(10B) | 110.3(9) |
| C(3)-C(4)-C(14) | 111.48(7) | H(10A)-C(10)-H-10B) | 106.6(12) |
| C(4)-C(5)-C(6) | 110.34(7) | C(10)-C(11)-C(12) | 109.41(8) |
| C(4)-C(5)-H(5A) | 110.1(9) | C(10)-C(11)-C(6) | 110.98(8) |
| C(6)-C(5)-H(5A) | 110.9(9) | C(12)-C(11)-C(6) | 109.34(7) |
| C(4)-C(5)-H(5B) | 110.4(9) | C(10)-C(11)-H(11) | 109.7(9) |
| C(6)-C(5)-H(5B) | 108.4(9) | C(12)-C(11)-H(11) | 108.7(9) |
| H(5A)-C(5)-H(5B) | 106.6(12) | C(6)-C(11)-H(11) | 108.7(9) |
| C(16)-C(6)-C(5) | 106.67(8) | C(14)-C(12)-C(11) | 109.86(7) |
| C(16)-C(6)-C(11) | 112.00(8) | C(14)-C(12)-C(1) | 110.64(7) |
| C(5)-C(6)-C(11) | 109.49(7) | C(11)-C(12)-C(1) | 109.85(7) |
| C(16)-C(6)-C(7) | 112.07(8) | C(14)-C(12)H(12) | 108.2(8) |
| C(5)-C(6)-C(7) | 109.43(8) | C(11)-C(12)-H(12) | 109.7(8) |
| C(11)-C(6)-C(7) | 107.18(7) | C(1)-C(12)-H(12) | 108.5(8) |
| C(8)-C(7)-C(2) | 109.76(8) | C(9)-C(13)-C-(1) | 111.59(8) |
| C(8)-C(7)-C(6) | 110.98(8) | C(9)-C(13)-H(13A) | 110.4(9) |
| C(2)-C(7)-C(6) | 109.26(7) | C(1)-C(13)-H(13A) | 107.8(9) |
| C(8)-C(7)-H(7) | 109.9(9) | C(9)-C(13)-H(13B) | 111.1(9) |
| C(1)-C(13)-H(13B) | 108.9(9) | H(15A)-C(15)-H(15B) | 107.0(14) |
| H(13A)-C(13)-H(13B) | 106.8(12) | C(1)-C(15)-H(15C) | 115.4(10) |
| C(4)-C(14)-C-(12) | 108.71(7) | H(15A)-C(15)-H(15C) | 109.0(14) |
| C(4)-C(14)-H(14A) | 109.6(9) | H(15B)-C(15)-H(15C) | 104.7(14) |
| C(12)-C(14)-H(14A) | 109.4(9) | C(6)-C(16)-H(16A) | 109.4(10) |
| C(4)-C(14)-H(14B) | 111.3(9) | C(6)-C(16)-H(16B) | 113.4(10) |
| C(12)-C(14)-H(14B) | 111.1(9) | H(16A)-C(16)-H(16B) | 106.2(15) |
| H(14A)-C(14)-H(14B) | 106.7(12) | C(6)-C(16)-H(16C) | 110.4(10) |
| C(1)-C(15)-H(15A) | 109.3(10) | H(16A)-C(16)-H(16C) | 107.3(15) |
| C(1)-C(15)-H(15B) | 110.9(10) | H(16B)-C(16)-H(16C) | 109.7(14) |

**Table 8**

Anisotropic displacement parameters ($Å^2 \times 10^3$) for 4-amino-1,6-dimethyldiamantane hydrochloride monohydrate. The anisotropic displacement factor exponent takes the form: $-2 \pi^2[h^2a^{*2}U^{11}+...+2hka^*b^*U^{12}]$

| | $U^{11}$ | $U^{22}$ | $U^{33}$ | $U^{23}$ | $U^{13}$ | $U^{12}$ |
|---|---|---|---|---|---|---|
| C1(1) | 20(1) | 18(1) | 16(1) | -2(1) | 4(1) | 0(1) |
| N(1) | 21(1) | 14(1) | 15(1) | 1(1) | 7(1) | 2(1) |
| C(1) | 13(1) | 14(1) | 12(1) | -1(1) | 4(1) | 1(1) |

(continued)

Anisotropic displacement parameters ($Å^2 \times 10^3$) for 4-amino-1,6-dimethyldiamantane hydrochloride monohydrate. The anisotropic displacement factor exponent takes the form: $-2 \pi^2[h^2a^{*2}U^{11}+...+2hka^*b^*U^{12}]$

| | $U^{11}$ | $U^{22}$ | $U^{33}$ | $U^{23}$ | $U^{13}$ | $U^{12}$ |
|---|---|---|---|---|---|---|
| C(2) | 12(1) | 16(1) | 13(1) | 0(1) | 5(1) | 1(1) |
| C(3) | 15(1) | 16(1) | 13(1) | 0(1) | 7(1) | -1(1) |
| C(4) | 14(1) | 12(1) | 12(1) | 0(1) | 5(1) | 0(1) |
| C(5) | 17(1) | 15(1) | 12(1) | -2(1) | 5(1) | -2(1) |
| C(6) | 13(1) | 17(1) | 10(1) | 0(1) | 3(1) | -1(1) |
| C(7) | 11(1) | 19(1) | 14(1) | 0(1) | 3(1) | 0(1) |
| C(8) | 14(1) | 23(1) | 18(1) | 3(1) | 3(1) | 4(1) |
| C(9) | 19(1) | 16(1) | 18(1) | 3(1) | 5(1) | 4(1) |
| C(10) | 20(1) | 17(1) | 16(1) | 4(1) | 6(1) | 1(1) |
| C(11) | 14(1) | 15(1) | 12(1) | 1(1) | 5(1) | -1(1) |
| C(12) | 11(1) | 13(1) | 13(1) | 0(1) | 3(1) | 0(1) |
| C(13) | 18(1) | 15(1) | 17(1) | -1(1) | 5(1) | 3(1) |
| C(14) | 12(1) | 14(1) | 14(1) | 0(1) | 4(1) | 1(1) |
| C(15) | 21(1) | 19(1) | 13(1) | -3(1) | 4(1) | 1(1) |
| C(16) | 22(1) | 25(1) | 12(1) | 0(1) | 3(1) | -3(1) |

**Table 9**

Hydrogen coordinates ($\times 10^4$) and isotropic displacement parameters ($Å^2 \times 10^3$) for 4-amino-1,6-dimethyldiamantane hydrochloride monohydrate

| | x | y | z | U(eq) |
|---|---|---|---|---|
| H(1D) | 11830(30) | 2741(5) | 2160(20) | 24 |
| H(1E) | 13150(30) | 2479(5) | 3620(20) | 24 |
| H(1A) | 10170(30) | 2777(5) | 4410(20) | 25 |
| H(1B) | 8180(30) | 2658(5) | 4600(20) | 25 |
| H(1C) | 9910(30) | 2858(5) | 6140(20) | 25 |
| H(2) | 5180(20) | 3975(5) | 5260(20) | 16 |
| H(3A) | 7550(20) | 3400(5) | 6510(20) | 17 |
| H(3B) | 5730(20) | 3230(5) | 4860(20) | 17 |
| H(5A) | 6130(20) | 3113(5) | 1900(20) | 18 |
| H(5B) | 8270(20) | 3234(5) | 1640(20) | 18 |
| H(7) | 3760(20) | 3723(5) | 2250(20) | 18 |
| H(8A) | 3340(30) | 4401(5) | 790(20) | 23 |
| H(8B) | 3220(30) | 4465(5) | 2700(20) | 23 |
| H(9) | 5570(20) | 4986(5) | 2280(20) | 22 |
| H(10A) | 8840(20) | 4756(5) | 2050(20) | 21 |
| H(10B) | 6940(20) | 4584(5) | 350(20) | 21 |
| H(11) | 9370(20) | 4011(5) | 1620(20) | 17 |
| H(12) | 10810(20) | 4266(5) | 4667(19) | 15 |
| H(13A) | 6270(20) | 4747(5) | 5290(20) | 20 |
| H(13B) | 8430(20) | 4870(5) | 5080(20) | 20 |
| H(14A) | 11300(20) | 3527(5) | 4205(19) | 16 |
| H(14B) | 11180(20) | 3593(5) | 6110(20) | 16 |
| H(15A) | 8280(30) | 4342(5) | 7900(20) | 28 |
| H(15B) | 10470(30) | 4417(5) | 7670(20) | 28 |
| H(15C) | 9780(30) | 3950(5) | 7910(20) | 28 |
| H(16A) | 6330(30) | 3668(6) | -970(20) | 31 |

(continued)

Hydrogen coordinates (x $10^4$) and isotropic displacement parameters ($Å^2$ x $10^3$) for 4-amino-1,6-dimethyldiamantane hydrochloride monohydrate

| | x | y | z | U(eq) |
|---|---|---|---|---|
| H(16B) | 4820(30) | 4036(6) | -970(20) | 31 |
| H(16C) | 4200(30) | 3550(6) | -760(20) | 31 |

**Table 10**

Hydrogen bonds for 4-amino-1,6-dimethyldiamantane hydrochloride monohydrate [Å and deg]

| D-H...A | d(D-H) | d(H...A) | d(D...A) | <(DHA) |
|---|---|---|---|---|
| O(1A)-H(1D)...C1(1)#1 | 0.901(16) | 2.240(17) | 3.1345(14) | 171.4(15) |
| O(1A)-H(1E)...C1(1)#2 | 0.905(16) | 2.241(17) | 3.1201(14) | 163.9(14) |
| N(1)-H(1A)...O(1B) | 0.885(17) | 1.802(17) | 2.682(3) | 172.7(16) |
| N(1)-H(1A)...O(1A) | 0.885(17) | 1.873(17) | 2.7483(17) | 169.7(16) |
| N(1)-H(1B)...C1(1) | 0.930(17) | 2.274(17) | 3.1893(9) | 168.0(14) |
| N(1)-H(1C)...C1(1)#3 | 0.911(17) | 2.317(17) | 3.2164(9) | 169.5(15) |

Symmetry transformations used to generate equivalent atoms:
#1 x+1/2,-y+1/2,z-1/2 #2 x+1,y,z #3 x+1/2,-y+1/2,z+1/2

**Example 6:**

**Alternate synthesis of MDT-22 and MDT-24**

The final crude synthesis product from step 10 of Example 3 was a mixture of diamantane compounds which was further purified in Example 4 to provide, *inter alia,* a fraction designated as MDT-22, as well as a component designated below as MDT-24. An alternate synthesis for making MDT-22 and MDT-24 was developed which resulted in the production of MDT-22 and MDT-24 of greater purity. The details of this alternate synthetic route are set forth below.

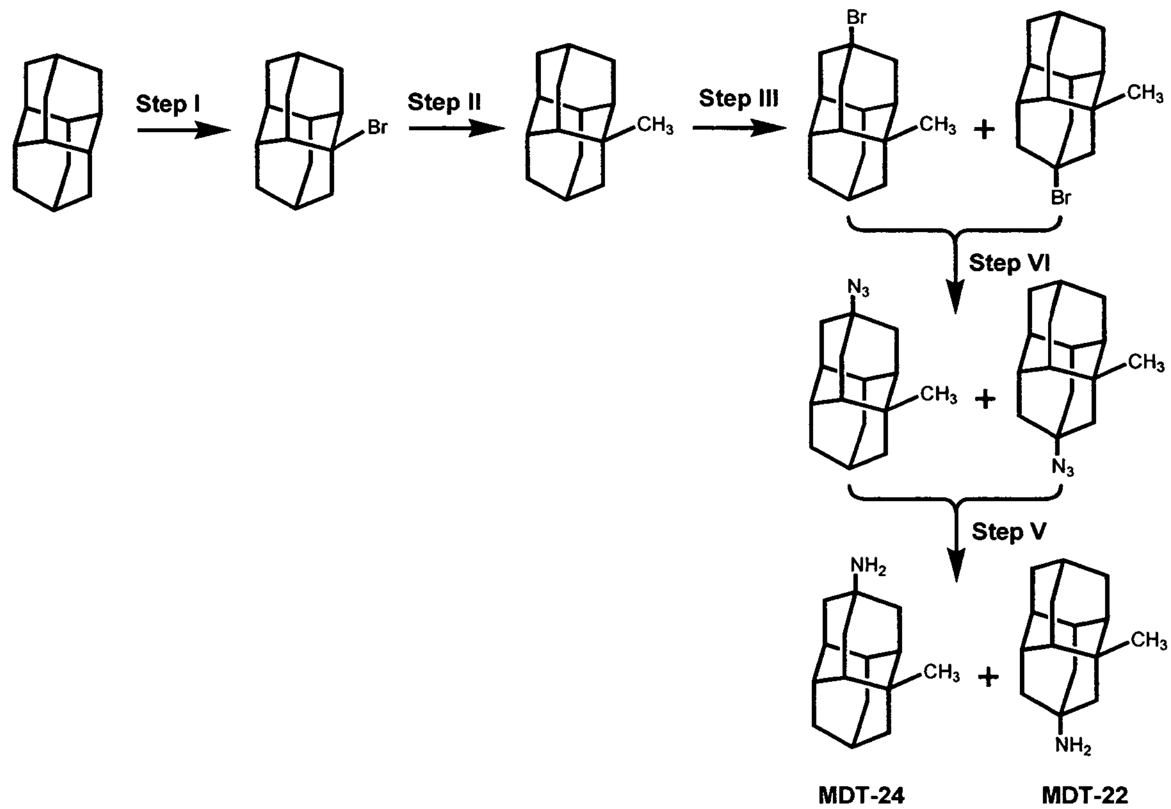


Scheme 7. Synthesis of MDT-22 and MDT-24 mixture

***Step I. Synthesis of 1-bromodiamantane***

Bromine (46.0 ml) was added dropwise to diamantane (14.05 g, 0.075 mol) in a three-necked flask (150 ml) with a thermometer and a gas outlet leading to a $Na_2CO_3$ solution under vigorous stirring and cooled in an ice-bath in a period of about 40 min. The ice-bath was removed after the addition of bromine was completed. The reaction mixture was stirred for another 6 h at about 20 °C and then poured onto frozen aqueous sodium hydrogen bisulfite solution. $CH_2Cl_2$ (40.00 ml) was added and the organic layer was separated. The aqueous solution was extracted with $CH_2Cl_2$ (3×20 ml). The combined $CH_2Cl_2$ solution was washed with water and dried with anhydrous $CaCl_2$. Evaporation under reduced pressure gave crude product (19.11 g). Fractional recrystallization from $CH_2Cl_2$ and washing with $CH_3OH$ (3×5.00 ml) gave colorless crystals (14.83 g, 0.053 mol), m.p. 221-223 °C. (lit. m.p. 222-224°C). The IR and $^1H$-, $^{13}C$- and $^{13}C$-$^1H$ Cosy NMR, and mass spectra of 1-bromodiamantane are shown in Figures 49, 50, 51, 52 and 53, respectively.

***Step II. Synthesis of 1-methyl-diamantane***

To a solution of 1-bromodiamantane (13.6 g, 51 mmol) in dry *n*-butyl ether (250 mL) was added a solution of methyl magnesium iodide in diethyl ether (3 M, 68 mL, Aldrich) under argon atmosphere. The reaction mixture was warmed slowly to 90 °C and was stirred at about 90±5 °C for 30 min. The reaction mixture was then cooled down to room temperature and was quenched by addition of cold saturated $NH_4Cl$ solution. The organic layer was separated, dried ($Na_2SO_4$) and concentrated *in vacuo.* The residue was purified by column chromatography (silica gel, petroleum ether) to give 1-methyldiamantane as a white solid (9.32g, 90.5%). The $^1H$- and $^{13}C$-NMR spectra of 1-methyldiamantane are shown in Figures 54 and 55, respectively.

***Step III. Synthesis of 1-methyl-4-bromo-diamantane and 1-methyl-9-bromo-diamantane mixture***

To a stirred solution of 1-methyldiamantane (6.9 g, 34.2 mmol) and $AlBr_3$ (100 mg) in chloroform (300 mL) was added dropwise a chloroform (10 mL) solution of bromine (1.4 mL, 27.4 mmol) under ice-salt condition. TLC was used to monitor the progress of the reaction. After 10 min, the reaction was stopped and quenched by pouring the reaction mixture into a mixture of solid sodium bisulphate and crushed ice. The mixture was then extracted by chloroform (2×80

mL), and washed with saturated aqueous solution of sodium bicarbonate and water. The organic layer was dried over sodium sulfate. After evaporating the solvent, the resulting residue was purified by silica gel column chromatography. Elution with petroleum ether gave 1.0 g (10%) of starting material, and further elution gave a mixture of 1-methyl-4-bromo-diamantane and 1-methyl-9-bromo-diamantane (5.0 g, 52%). Melting range: 50-67 °C. The $^1$H- and $^{13}$C-NMR spectra of the mixture of 1-methyl-4-bromodiamantane and 1-methyl-9-bromodiamantane are shown in Figures 56 and 57, respectively.

***Step IV, Synthesis of 1-methyl-4-azido-diamantane and 1-methyl-9-azido-diamantane mixture***

Tin tetrachloride (0.35 mL) was added under nitrogen atmosphere at ice-bath temperature to a solution of the mixture of 1-methyl-4-bromo-diamantane and 1-methyl-9-bromo-diamantane (1.88 g, 6.71 mmol) and azido trimethylsilylate (2.65 mL, 20.15 mmol) in dry dichloromethane (DCM) (160 mL). The reaction mixture was stirred overnight at room temperature. The reaction mixture was quenched by saturated aqueous solution of ammonium chloride. The mixture was then extracted with DCM (2×50 mL). The combined organic layers were dried with sodium sulfite and filtered. The filtrate was evaporated and the residue was purified by silica gel column chromatography. Elution with petroleum ether gave the desired mixture of 1-methyl-4-azido-diamantane and 1-methyl-9-azido-diamantane as a white solid (1.2 g, 73%). Melting range: 60-75 °C. The IR, $^1$H- and $^{13}$C-NMR spectra of the mixture of 1-methyl-4-azidodiamantane and 1-methyl-9-azidodiamantane are shown in Figures 58, 59 and 60, respectively.

***Step V. Synthesis of synthesis of 1-methyl-4-amino-diamantane and 1-methyl-9-amino-diamantane mixture***

To a suspension of the mixture of 1-methyl-4-azido-diamantane and 1-methyl-9-azido-diamantane (1.26 g, 5.18 mmol) in dry methanol (20 mL) was added 10% palladium carbon (Pd-C) (0.023 g). The reaction mixture was stirred overnight under hydrogen atmosphere at room temperature. After completion of reaction, the mixture was filtered through Celite. After removal of the solvent, the mixture of 1-methyl-4-amino-diamantane and 1-methyl-9-amino-diamantane was obtained as a white solid (1.13 g, 100%). Melting range: 81-95°C. The GC-MS, $^1$H- and $^{13}$C-NMR spectra of the mixture of 1-methyl-4-aminodiamantane (MDT-24) and 1-methyl-9-aminodiamantane (MDT-22) are shown in Figures 61, 62 and 63, respectively. Figure 61A shows the TIC of the synthetic product, with MDT-24 eluting first, followed by MDT-22. Figure 61B shows the mass spectrum of MDT-22, with molecular ion at m/z 217, and base peak at mz 120. Figure 61C shows the mass spectrum of MDT-24, with molecular ion at m/z 217, and large fragment ion at mz 106.

**Example 7:**

**Purification and structure of MDT-22 and MDT-24**

The mixture of 1-methyl-4-aminodiamantane (MDT-24) and 1-methyl-9-aminodiamantane (MDT-22) was subjected to HPLC in order to separate the two compounds. Preparative HPLC was performed on a Hypercarb (7μ) 30 mm I.D. by 250 mm long column (with 30 mm I.D. by 50 mm long guard column). The mobile phase was methanol-water-triethylamine (95/5/1, by volume) at 13.5 mL/min and approximately 840 psi. In a typical HPLC separation, a sample of 35 mg of the MDT-22 and MDT-24 mixture in a 2.00 mL mobile phase was applied to the column. Fractions (30 sec each) were collected starting at 30.0 min and continuing until 60 min. A total of 60 fractions were collected. A representative chromatogram is shown in Figure 64. Fractions 10-12 and 20-22 were assayed by GC-MS to monitor sample purity. Fractions 11, 12 and 20 were found to contain MDT-22 that was >95+% pure. Thus, Fractions 11-20 were combined, used for additional GC-MS and NMR analysis and converted to the hydrochloride salt for use in biological testing. Fractions 10 and 21 were retained for reprocessing for MDT-22. MDT-24 (Fractions 22-50) had less than 95% purity. Therefore, MDT-24 was subjected to one additional HPLC run using the same method in order to obtain material that had 95+% purity. This purified MDT-24 was used for additional GC-MS and NMR analysis and then converted to the hydrochloride salt for use in biological testing.

Figures 65 and 66 show the GC-MS analysis of purified MDT-22. Figure 65A is the TIC of the purified MDT-22, showing only a single component peak (MDT-22). The mass spectrum of this component is Figure 65B, showing ions characteristic of MDT-22. Figure 66A shows an expansion of the TIC between 5.0 to 7.0 minutes, showing only a single, symmetrical TIC peak indicative of pure MDT-22. Figure 66B is a selected ion chromatogram for m/z 120 (characteristic of MDT-22, and m/z 106 (characteristic of MDT-24), illustrating that the purified MDT-22 contained no detectable MDT-24. Figures 67, 68, 69, 70 and 71 show $^1$H-, $^{13}$C-, $^{13}$C DEPT-135, $^1$H-$^1$H COSY and $^1$H-$^{13}$C HMQC NMR, respectively, for the purified MDT-22. The crystal structure of MDT-22 with atom numbering is shown in Figure 72. MDT-22 has the structure 1-methyl-9-amino-diamantane, although Figure 72 shows the atom numbering for 2-methyl-4-amino-diamantane. 1-Methyl-9-amino-diamantane and 2-methyl-4-amino-diamantane are alternative names for the same structure; however naming of the structure as 1-methyl-9-amino-diamantane is the preferred nomenclature.

**Table 11**

Atomic coordinates (x $10^4$) and equivalent isotropic displacement parameters ($Å^2$ x $10^3$) for 2-methyl-4-amino-diamantane. U(eq) is defined as one third of the trace of the orthogonalized $U^{ij}$ tensor.

| | x | y | z | U(eq) |
|---|---|---|---|---|
| C1(1) | -1783(1) | 4624(1) | 2109(1) | 22(1) |
| O(1) | -181(2) | 1425(1) | 2343(1) | 29(1) |
| N(1) | 1826(1) | 3267(1) | 2092(1) | 19(1) |
| C(1) | 3783(1) | 2096(1) | 896(1) | 17(1) |
| C(2) | 5191(1) | 2512(1) | 1219(1) | 16(1) |
| C(3) | 4339(1) | 2541(1) | 1661(1) | 16(1) |
| C(4) | 2671(1) | 3269(1) | 1664(1) | 14(1) |
| C(5) | 3205(2) | 4445(1) | 1549(1) | 17(1) |
| C(6) | 4020(2) | 4436(1) | 1107(1) | 16(1) |
| C(7) | 5717(1) | 3703(1) | 1089(1) | 17(1) |
| C(8) | 6496(2) | 3731(1) | 641(1) | 22(1) |
| C(9) | 5092(2) | 3290(1) | 329(1) | 23(1) |
| C(10) | 3420(2) | 4024(1) | 344(1) | 23(1) |
| C(11) | 2625(2) | 4010(1) | 788(1) | 17(1) |
| C(12) | 2101(1) | 2831(1) | 914(1) | 16(1) |
| C(13) | 4568(2) | 2111(1) | 450(1) | 22(1) |
| C(14) | 1278(1) | 2830(1) | 1354(1) | 16(1) |
| C(15) | 6819(2) | 1742(1) | 1244(1) | 25(1) |

**Table 12**

Bond lengths [Å] and angles [deg] for 2-methyl-4-amino-diamantane.

| | | | |
|---|---|---|---|
| O(1)-H(1E) | 0.86(2) | C(6)-H(6) | 0.959(14) |
| O(1)-H(1F) | 0.80(2) | C(7)-C(8) | 1.5404(16) |
| N(1)-C(4) | 1.5000(13) | C(7)-H(7) | 0.950(15) |
| N(1)-H(1A) | 0.921(17) | C(8)-C(9) | 1.5362(17) |
| N(1)-H(1B) | 0.874(17) | C(8)-H(8A) | 0.975(16) |
| N(1)-H(1C) | 0.914(17) | C(8)-H(8B) | 0.955(16) |
| C(1)-C(12) | 1.5360(15) | C(9)-C(10) | 1.5295(18) |
| C(1)-C(13) | 1.5364(15) | C(9)-C(13) | 1.5346(17) |
| C(1)-C(2) | 1.5508(15) | C(9)-H(9) | 0.997(16) |
| C(1)-H(1D) | 0.976(15) | C(10)-C(11) | 1.5322(16) |
| C(2)-C(15) | 1.5318(16) | C(10)-H(10A) | 0.990(16) |
| C(2)-C(3) | 1.5446(15) | C(10)-H(10B) | 0.998(16) |
| C(2)-C(7) | 1.5553(15) | C(11)-C(12) | 1.5384(15) |
| C(3)-C(4) | 1.5217(15) | C(11)-H(11) | 0.988(14) |
| C(3)-H(3A) | 0.964(14) | C(12)-C(14) | 1.5296(15) |
| C(3)-H(3B) | 0.989(14) | C(12)-H(12) | 0.982(15) |
| C(4)-C(14) | 1.5279(15) | C(13)-H(13A) | 0.989(16) |
| C(4)-C(5) | 1.5283(14) | C(13)-H(13B) | 1.000(16) |
| C(5)-C(6) | 1.5319(15) | C(14)-H(14A) | 0.969(14) |
| C(5)-H(5A) | 0.960(15) | C(14)-H(14B) | 0.976(14) |
| C(5)-H(5B) | 0.977(15) | C(15)-H(15A) | 0.994(18) |
| C(6)-C(11) | 1.5417(15) | C(15)-H(15B) | 1.006(18) |
| C(6)-C(7) | 1.5440(15) | C(15)-H(15C) | 0.971(18) |
| H(1E)-O(1)-H(1F) | 103.8(19) | C(12)-C(1)-H(1D) | 108.3(8) |

(continued)

Bond lengths [Å] and angles [deg] for 2-methyl-4-amino-diamantane.

| | | | |
|---|---|---|---|
| C(4)-N(1)-H(1A) | 109.9(10) | C(13)-C(1)-H(1D) | 109.1(9) |
| C(4)-N(1)-H(1B) | 110.9(11) | C(2)-C(1)-H(1D) | 109.7(9) |
| H(1A)-N(1)-H(1B) | 108.3(14) | C(15)-C(2)-C(3) | 106.80(9) |
| C(4)-N(1)-H(1C) | 112.2(10) | C(15)-C(2)-C(1) | 111.55(9) |
| H(1A)-N(1)-H(1C) | 105.4(14) | C(3)-C(2)-C(1) | 109.63(8) |
| H(1B)-N(1)-H(1C) | 109.9(14) | C(15)-C(2)-C(7) | 112.65(9) |
| C(12)-C(1)-C(13) | 109.54(9) | C(3)-C(2)-C(7) | 108.93(8) |
| C(12)-C(1)-C(2) | 109.51(8) | C(1)-C(2)-C(7) | 107.26(8) |
| C(13)-C(1)-C(2) | 110.71(9) | C(4)-C(3)-C(2) | 110.65(8) |
| C(4)-C(3)-H(3A) | 110.1(9) | C(10)-C(9)-C(8) | 109.10(10) |
| C(2)-C(3)-H(3A) | 109.5(9) | C(13)-C(9)-C(8) | 109.58(10) |
| C(4)-C(3)-H(3B) | 109.7(8) | C(10)-C(9)-H(9) | 111.6(9) |
| C(2)-C(3)-H(3B) | 109.7(8) | C(13)-C(9)-H(9) | 107.5(9) |
| H(3A)-C(3)-H(3B) | 107.2(12) | C(8)-C(9)-H(9) | 109.7(9) |
| N(1)-C(4)-C(3) | 110.21(9) | C(9)-C(10)-C(11) | 109.55(9) |
| N(1)-C(4)-C(14) | 107.72(8) | C(9)-C(10)-H(10A) | 108.8(9) |
| C(3)-C(4)-C(14) | 110.06(8) | C(11)-C(10)-H(10A) | 110.2(9) |
| N(1)-C(4)-C(5) | 109.20(8) | C(9)-C(10)-H(10B) | 111.0(9) |
| C(3)-C(4)-C(5) | 109.34(9) | C(11)-C(10)-H(10B) | 108.2(9) |
| C(14)-C(4)-C(5) | 110.30(9) | H(10A)-C(10)-H(10B) | 109.1(13) |
| C(4)-C(5)-C(6) | 108.46(8) | C(10)-C(11)-C(12) | 110.40(9) |
| C(4)-C(5)-H(5A) | 107.4(9) | C(10)-C(11)-C(6) | 110.28(9) |
| C(6)-C(5)-H(5A) | 110.5(9) | C(12)-C(11)-C(6) | 108.09(8) |
| C(4)-C(5)-H(5B) | 109.0(8) | C(10)-C(11)-H(11) | 110.9(9) |
| C(6)-C(5)-H(5B) | 109.5(9) | C(12)-C(11)-H(11) | 107.6(9) |
| H(5A)-C(5)-H(5B) | 111.8(12) | C(6)-C(11)-H(11) | 109.5(9) |
| C(5)-C(6)-C(11) | 110.08(9) | C(14)-C(12)-C(1) | 110.94(9) |
| C(5)-C(6)-C(7) | 111.17(9) | C(14)-C(12)-C(11) | 109.93(8) |
| C(11)-C(6)-C(7) | 109.23(8) | C(1)-C(12)-C(11) | 109,10(9) |
| C(5)-C(6)-H(6) | 107.5(8) | C(14)-C(12)-H(12) | 108.0(8) |
| C(11)-C(6)-H(6) | 110.2(8) | C(1)-C(12)-H(12) | 110.1(8) |
| C(7)-C(6)-H(6) | 108.6(9) | C(11)-C(12)-H(12) | 108.8(9) |
| C(8)-C(7)-C(6) | 109.17(9) | C(9)-C(13)-C(1) | 109.89(9) |
| C(8)-C(7)-C(2) | 111.19(9) | C(9)-C(13)-H(13A) | 110.7(9) |
| C(6)-C(7)-C(2) | 108.79(8) | C(1)-C(13)-H(13A) | 109.6(9) |
| C(8)-C(7)-H(7) | 108.4(9) | C(9)-C(13)-H(13B) | 110.4(9) |
| C(6)-C(7)-H(7) | 109.0(9) | C(1)-C(13)-H(13B) | 108.8(9) |
| C(2)-C(7)-H(7) | 110.3(9) | H(13A)-C(13)-H(13B) | 107.4(13) |
| C(9)-C(8)-C(7) | 109.70(9) | C(4)-C(14)-C(12) | 108.82(8) |
| C(9)-C(8)-H(8A) | 108.1(9) | C(4)-C(14)-H(14A) | 110.7(8) |
| C(7)-C(8)-H(8A) | 109.2(9) | C(12)-C(14)-H(14A) | 112.0(9) |
| C(9)-C(8)-H(8B) | 109.4(9) | C(4)-C(14)-H(14B) | 108.8(8) |
| C(7)-C(8)-H(8B) | 112.9(9) | C(12)-C(14)-H(14B) | 110.7(8) |
| H(8A)-C(8)-H(8B) | 107.4(13) | H(14A)-C(14)-H(14B) | 105.9(12) |
| C(10)-C(9)-C(13) | 109.33(10) | C(2)-C(15)-H(15A) | 110.3(10) |
| C(2)-C(15)-H(15B) | 114.1(10) | H(15A)-C(15)-H(15C) | 104.7(14) |
| H(15A)-C(15)-H(15B) | 109.9(14) | H(15B)-C(15)-H(15C) | 107.9(14) |
| C(2)-C(15)-H(15C) | 109.6(10) | | |

**Table 13**

Anisotropic displacement parameters ($Å^2 \times 10^3$) for 2-methyl-4-amino-diamantane. The anisotropic displacement factor exponent takes the form: $-2 \pi^2[h^2 a^{*2} U^{11} + ... + 2 h k a^* b^* U^{12}]$

| | $U^{11}$ | $U^{22}$ | $U^{33}$ | $U^{23}$ | $U^{13}$ | $U^{12}$ |
|---|---|---|---|---|---|---|
| C1(1) | 25(1) | 22(1) | 18(1) | 1(1) | 1(1) | 3(1) |
| O(1) | 37(1) | 22(1) | 29(1) | 5(1) | -6(1) | -9(1) |
| N(1) | 22(1) | 18(1) | 16(1) | -1(1) | 3(1) | -2(1) |
| C(1) | 18(1) | 14(1) | 18(1) | -3(1) | 2(1) | -1(1) |
| C(2) | 14(1) | 15(1) | 18(1) | 2(1) | 1(1) | 1(1) |
| C(3) | 17(1) | 16(1) | 17(1) | 3(1) | -1(1) | -1(1) |
| C(4) | 17(1) | 13(1) | 13(1) | -1(1) | 2(1) | -2(1) |
| C(5) | 22(1) | 12(1) | 16(1) | -2(1) | 0(1) | -2(1) |
| C(6) | 21(1) | 12(1) | 15(1) | 1(1) | -1(1) | -2(1) |
| C(7) | 16(1) | 17(1) | 17(1) | 1(1) | -1(1) | -5(1) |
| C(8) | 21(1) | 23(1) | 22(1) | 2(1) | 4(1) | -3(1) |
| C(9) | 26(1) | 26(1) | 16(1) | -1(1) | 5(1) | -3(1) |
| C(10) | 27(1) | 26(1) | 15(1) | 3(1) | -2(1) | -1(1) |
| C(11) | 19(1) | 18(1) | 15(1) | 1(1) | -2(1) | 1(1) |
| C(12) | 16(1) | 17(1) | 16(1) | -3(1) | -1(1) | -2(1) |
| C(13) | 25(1) | 23(1) | 19(1) | -6(1) | 5(1) | -1(1) |
| C(14) | 15(1) | 15(1) | 18(1) | -2(1) | 1(1) | -2(1) |
| C(15) | 19(1) | 24(1) | 32(1) | 6(1) | 3(1) | 4(1) |

**Table 14**

Hydrogen coordinates ($\times 10^4$) and isotropic displacement parameters ($Å^2 \times 10^3$) for 2-methyl-4-amino-diamantane

| | x | y | z | U(eq) |
|---|---|---|---|---|
| H(1E) | 400(30) | 950(16) | 2489(6) | 49(5) |
| H(1F) | -980(30) | 1077(17) | 2240(6) | 47(6) |
| H(1A) | 820(20) | 3711(14) | 2092(5) | 28 |
| H(1B) | 2580(20) | 3514(13) | 2280(5) | 28 |
| H(1C) | 1420(20) | 2585(15) | 2167(5) | 28 |
| H(1D) | 3425(19) | 1346(12) | 967(5) | 20 |
| H(3A) | 4027(19) | 1805(12) | 1746(5) | 20 |
| H(3B) | 5226(18) | 2823(11) | 1866(5) | 20 |
| H(5A) | 2130(20) | 4884(12) | 1555(4) | 20 |
| H(5B) | 4100(20) | 4708(11) | 1749(5) | 20 |
| H(6) | 4359(19) | 5176(12) | 1040(4) | 19 |
| H(7) | 6596(19) | 3989(12) | 1276(5) | 20 |
| H(8A) | 6769(19) | 4489(13) | 565(5) | 26 |
| H(8B) | 7580(20) | 3317(13) | 614(5) | 26 |
| H(9) | 5620(20) | 3264(13) | 42(5) | 27 |
| H(10A) | 2530(20) | 3744(13) | 140(5) | 27 |
| H(10B) | 3730(20) | 4800(13) | 272(5) | 27 |
| H(11) | 1526(19) | 4467(12) | 803(5) | 21 |
| H(12) | 1187(19) | 2559(12) | 717(5) | 19 |
| H(13A) | 5630(20) | 1619(13) | 437(5) | 27 |
| H(13B) | 3640(20) | 1817(13) | 251(5) | 27 |
| H(14A) | 181(19) | 3260(12) | 1366(4) | 19 |

(continued)

Hydrogen coordinates (x $10^4$) and isotropic displacement parameters (Å $^2$ x $10^3$) for 2-methyl-4-amino-diamantane

| | x | y | z | U(eq) |
|---|---|---|---|---|
| H(14B) | 940(19) | 2085(12) | 1437(5) | 19 |
| H(15A) | 7610(20) | 1958(14) | 1481(6) | 37 |
| H(15B) | 7540(20) | 1697(14) | 978(5) | 37 |
| H(15C) | 6420(20) | 1004(15) | 1314(5) | 37 |

**Table 15**

Hydrogen bonds for 2-methyl-4-amino-diamantane [Å and deg]

| D-H...A | d(D-H) | d(H...A) | d(D...A) | <(DHA) |
|---|---|---|---|---|
| O(1)-H(1E)...C1(1)#1 | 0.86(2) | 2.30(2) | 3.1575(11) | 176.3(18) |
| O(1)-H(1F)...C1(1)#2 | 0.80(2) | 2.46(2) | 3.2299(10) | 161.9(19) |
| N(1)-H(1B)...C1(1)#3 | 0.874(17) | 2.417(17) | 3.2072(10) | 150.7(14) |
| N(1)-H(1A)...C1(1) | 0.921(17) | 2.230(18) | 3.1470(11) | 173.8(14) |
| N(1)-H(1C)...O(1) | 0.914(17) | 1.929(17) | 2.8069(14) | 160.5(15) |

Symmetry transformations used to generate equivalent atoms:
#1 -x,y-1/2,-z+1/2 #2 -x-1/2,y-1/2,z #3 x+1/2,y,-z+1/2

Figures 73 and 74 show GC-MS analyses of the purified MDT-24. Figure 73A is the TIC of the purified MDT-24, showing only a single major component peak (MDT-24). The mass spectrum of this component is Figure 73B, showing ions characteristic of MDT-24. Figure 74A shows an expansion of the TIC between 5.0 to 7.0 minutes, showing only a single, nearly symmetrical TIC peak indicative of high-purity MDT-24. Figure 74B is a selected ion chromatogram for m/z 120 (characteristic of MDT-22, and m/z 106 (characteristic of MDT-24), illustrating that the purified MDT-24 contained only a small amount of MDT-22. The crystal structure of MDT-24 with atom numbering is shown in Figure 75. MDT-24 has the structure 1-methyl-4-amino-diamantane.

**Table 16**

Atomic coordinates (x $10^4$) and equivalent isotropic displacement parameters (Å$^2$ x $10^3$) for 1-methyl-4-amino-diamantane. U(eq) is defined as one third of the trace of the orthogonalized $U^{ij}$ tensor.

| | x | y | z | U(eq) |
|---|---|---|---|---|
| Cl(1) | -287(1) | 3169(1) | 2500 | 21(1) |
| Cl(2) | -5127(1) | 2500 | 10000 | 18(1) |
| N(1) | 3264(2) | 3022(1) | 2500 | 18(1) |
| C(1) | 3319(2) | 4435(1) | 2500 | 15(1) |
| C(2) | 3909(1) | 4185(1) | 1268(1) | 16(1) |
| C(3) | 3319(1) | 3713(1) | 1258(1) | 16(1) |
| C(4) | 3897(2) | 3479(1) | 2500 | 14(1) |
| C(5) | 5658(2) | 3464(1) | 2500 | 17(1) |
| C(6) | 6264(2) | 3935(1) | 2500 | 18(1) |
| C(9) | 5784(2) | 4887(1) | 2500 | 24(1) |
| C(10) | 6346(1) | 4646(1) | 3734(1) | 25(1) |
| C(11) | 5695(1) | 4182(1) | 3734(1) | 19(1) |
| C(13) | 4014(2) | 4897(1) | 2500 | 19(1) |
| C(15) | 1553(2) | 4480(1) | 2500 | 20(1) |
| N(1') | -2790(2) | 2567(1) | 7500 | 17(1) |
| C(1') | 1050(2) | 3471(1) | 7500 | 14(1) |
| C(2') | -7(1) | 3441(1) | 8730(1) | 16(1) |
| C(3') | -930(1) | 3013(1) | 8745(1) | 17(1) |
| C(4') | -1930(2) | 2992(1) | 7500 | 15(1) |

(continued)

Atomic coordinates (x $10^4$) and equivalent isotropic displacement parameters ($Å^2$ x $10^3$) for 1-methyl-4-amino-diamantane. U(eq) is defined as one third of the trace of the orthogonalized $U^{ij}$ tensor.

| | x | y | z | U(eq) |
|---|---|---|---|---|
| C(5') | -3090(2) | 3368(1) | 7500 | 20(1) |
| C(6') | -2182(2) | 3797(1) | 7500 | 21(1) |
| C(9') | 728(2) | 4295(1) | 7500 | 23(1) |
| C(10') | -278(1) | 4263(1) | 6259(1) | 26(1) |
| C(11') | -1147(1) | 3828(1) | 6265(1) | 21(1) |
| C(15') | 2282(2) | 3116(1) | 7500 | 21(1) |
| C(13') | 1882(2) | 3917(1) | 7500 | 19(1) |

**Table 17**

Bond lengths [Å] and angles [deg] for 1-methyl-4-amino-diamantane.

| | | | |
|---|---|---|---|
| N(1)-C(4) | 1.5051(19) | N(1')-C(4') | 1.5034(18) |
| N(1)-H(1A) | 0.913(16) | N(1')-H(1B') | 0.86(2) |
| N(1)-H(1B) | 0.88(2) | N(1')-H(1A') | 0.915(16) |
| C(1)-C(15) | 1.536(2) | C(1')-C(15') | 1.527(2) |
| C(1)-C(13) | 1.539(2) | C(1')-C(13') | 1.545(2) |
| C(1)-C(2)#1 | 1.5492(13) | C(1')-C(2')#2 | 1.5463(13) |
| C(1)-C(2) | 1.5492(13) | C(1')-C(2') | 1.5463(13) |
| C(2)-C(3) | 1.5345(14) | C(2')-C(3') | 1.5376(14) |
| C(2)-C(11)#1 | 1.5472(14) | C(2')-C(11')#2 | 1.5432(15) |
| C(2)-H(2) | 0.993(14) | C(2')-H(2') | 0.946(15) |
| C(3)-C(4) | 1.5306(13) | C(3')-C(4') | 1.5289(13) |
| C(3)-H(3A) | 0.980(15) | C(3')-H(3'A) | 0.962(15) |
| C(3)-H(3B) | 0.991(15) | C(3')-H(3'B) | 0.975(15) |
| C(4)-C(5) | 1.526(2) | C(4')-C(5') | 1.529(2) |
| C(4)-C(3)#1 | 1.5306(13) | C(4')-C(3')#2 | 1.5289(13) |
| C(5)-C(6) | 1.535(2) | C(5')-C(6') | 1.532(2) |
| C(5)-H(5) | 0.942(15) | C(5')-H(5') | 0.957(15) |
| C(6)-C(11)#1 | 1.5404(14) | C(6')-C(11')#2 | 1.5396(14) |
| C(6)-C(11) | 1.5404(14) | C(6')-C(11') | 1.5396(14) |
| C(6)-H(6) | 1.03(2) | C(6')-H(6') | 0.99(2) |
| C(9)-C(10)#1 | 1.5296(16) | C(9')-C(10') | 1.5304(16) |
| C(9)-C(10) | 1.5296(16) | C(9')-C(10')#2 | 1.5304(16) |
| C(9)-C(13) | 1.533(2) | C(9')-C(13') | 1.531 (2) |
| C(9)-H(9) | 0.98(2) | C(9')-H(9') | 0.96(2) |
| C(10)-C(11) | 1.5309(15) | C(10')-C(11') | 1.5321(16) |
| C(10)-H(10A) | 0.988(16) | C(10')-H(10C) | 0.986(18) |
| C(10)-H(10B) | 0.994(17) | C(10')-H(10D) | 0.979(17) |
| C(11)-C(2)#1 | 1.5472(14) | C(11')-C(2')#2 | 1.5432(15) |
| C(11)-H(11) | 0.964(16) | C(11')-H(11') | 0.977(16) |
| C(13)-H(13) | 0.958(15) | C(15')-H(15C) | 0.97(2) |
| C(15)-H(15A) | 0.990(16) | C(15')-H(15D) | 0.985(17) |
| C(15)-H(15B) | 0.97(2) | C(13')-H(13') | 0.973(15) |
| C(4)-N(1)-H(1A) | 109.6(10) | C(10)#1-C(9)-C(10) | 109.17(14) |
| C(4)-N(1)-H(1B) | 110.7(14) | C(10)#1-C(9)-C(13) | 109.13(9) |
| H(1A)-N(1)-H(1B) | 110.5(12) | C(10)-C(9)-C(13) | 109.13(9) |
| C(15)-C(1)-C(13) | 107.90(11) | C(10)#1-C(9)-H(9) | 108.9(7) |

(continued)

Bond lengths [Å] and angles [deg] for 1-methyl-4-amino-diamantane.

| | | | |
|---|---|---|---|
| C(15)-C(1)-C(2)#1 | 111.91(8) | C(10)-C(9)-H(9) | 108.9(7) |
| C(13)-C(1)-C(2)#1 | 109.08(8) | C(13)-C(9)-H(9) | 111.6(13) |
| C(15)-C(1)-C(2) | 111.91(8) | C(9)-C(10)-C(11) | 109.46(10) |
| C(13)-C(1)-C(2) | 109.08(8) | C(9)-C(10)-H(10A) | 110.5(9) |
| C(2)#1-C(1)-C(2) | 106.91(11) | C(11)-C(10)-H(10A) | 109.5(9) |
| C(3)-C(2)-C(11)#1 | 109.10(8) | C(9)-C(10)-H(10B) | 112.5(9) |
| C(3)-C(2)-C(1) | 111.29(9) | C(11)-C(10)-H(10B) | 109.8(9) |
| C(11)#1-C(2)-C(1) | 109.54(9) | H(10A)-C(10)-H(10B) | 105.0(13) |
| C(3)-C(2)-H(2) | 108.5(8) | C(10)-C(11)-C(6) | 109.81(10) |
| C(11)#1-C(2)-H(2) | 109.3(8) | C(10)-C(11)-C(2)#1 | 111.28(8) |
| C(1)-C(2)-H(2) | 109.1(8) | C(6)-C(11)-C(2)#1 | 108.76(9) |
| C(4)-C(3)-C(2) | 109.17(9) | C(10)-C(11)-H(11) | 109.9(9) |
| C(4)-C(3)-H(3A) | 109.9(8) | C(6)-C(11)-H(11) | 109.0(9) |
| C(2)-C(3)-H(3A) | 111.0(8) | C(2)#1-C(11)-H(11) | 108.0(9) |
| C(4)-C(3)-H(3B) | 109.7(8) | C(9)-C(13)-C(1) | 111.95(12) |
| C(2)-C(3)-H(3B) | 110.7(8) | C(9)-C(13)-H(13) | 110.1(9) |
| H(3A)-C(3)-H(3B) | 106.3(12) | C(1)-C(13)-H(13) | 108.6(9) |
| N(1)-C(4)-C(5) | 109.70(11) | C(1)-C(15)-H(15A) | 109.2(9) |
| N(1)-C(4)-C(3) | 108.56(7) | C(1)-C(15)-H(15B) | 114.7(14) |
| C(5)-C(4)-C(3) | 109.90(7) | H(15A)-C(15)-H(15B) | 108.1(12) |
| N(1)-C(4)-C(3)#1 | 108.56(7) | C(4')-N(1')-H(1B') | 109.0(15) |
| C(5)-C(4)-C(3)#1 | 109.90(7) | C(4')-N(1')-H(1A') | 110.3(9) |
| C(3)-C(4)-C(3)#1 | 110.17(11) | H(1B')-N(1')-H(1A') | 108.0(12) |
| C(4)-C(5)-C(6) | 108.34(11) | C(15')-C(1')-C(13') | 107.79(11) |
| C(4)-C(5)-H(5) | 110.9(9) | C(15')-C(1')-C(2')#2 | 111.80(8) |
| C(6)-C(5)-H(5) | 109.4(9) | C(13')-C(1')-C(2')#2 | 109.21(8) |
| C(5)-C(6)-C(11)#1 | 110.66(8) | C(15')-C(1')-C(2') | 111.80(8) |
| C(5)-C(6)-C(11) | 110.66(8) | C(13')-C(1')-C(2') | 109.21(8) |
| C(11)#1-C(6)-C(11) | 108.13(12) | C(2')#2-C(1')-C(2') | 107.00(11) |
| C(5)-C(6)-H(6) | 107.0(11) | C(3')-C(2')-C(11')#2 | 108.87(8) |
| C(11)#1-C(6)-H(6) | 110.2(6) | C(3')-C(2')-C(1') | 111.51(9) |
| C(11)-C(6)-H(6) | 110.2(6) | C(11')#2-C(2')-C(1') | 109.61(9) |
| C(3')-C(2')-H(2') | 109.3(9) | C(10')-C(9')-C(10')#2 | 110.07(14) |
| C(11')#2-C(2')-H(2') | 107.4(9) | C(10')-C(9')-C(13') | 108.82(9) |
| C(1')-C(2')-H(2') | 110.1(9) | C(10')#2-C(9')-C(13') | 108.82(9) |
| C(4')-C(3')-C(2') | 108.80(9) | C(10')-C(9')-H(9') | 109.6(7) |
| C(4')-C(3')-H(3'A) | 110.7(9) | C(10')#2-C(9')-H(9') | 109.6(7) |
| C(2')-C(3')-H(3'A) | 111.0(9) | C(13')-C(9')-H(9') | 110.0(13) |
| C(4')-C(3')-H(3'B) | 109.7(9) | C(9')-C(10')-C(11') | 109.45(10) |
| C(2')-C(3')-H(3'B) | 108.2(8) | C(9')-C(10')-H(10C) | 110.6(10) |
| H(3'A)-C(3')-H(3'B) | 108.4(12) | C(11')-C(10')-H(10C) | 110.3(10) |
| N(1')-C(4')-C(5') | 109.20(11) | C(9')-C(10')-H(10D) | 110.1(10) |
| N(1')-C(4')-C(3')#2 | 108.49(7) | C(11')-C(10')-H(10D) | 109.5(10) |
| C(5')-C(4')-C(3')#2 | 109.92(8) | H(10C)-C(10')-H(10D) | 106.9(13) |
| N(1')-C(4')-C(3') | 108.49(7) | C(10')-C(11')-C(6') | 110.11(10) |
| C(5')-C(4')-C(3') | 109.92(8) | C(10')-C(11')-C(2')#2 | 110.76(9) |
| C(3')#2-C(4')-C(3') | 110.77(11) | C(6')-C(11')-C(2')#2 | 108.78(9) |
| C(4')-C(5')-C(6') | 108.01(12) | C(10')-C(11')-H(11') | 107.6(9) |
| C(4')-C(5')-H(5') | 110.2(9) | C(6')-C(11')-H(11') | 111.1(9) |
| C(6')-C(5')-H(5') | 110.9(9) | C(2')#2-C(11')-H(11') | 108.5(9) |

(continued)

Bond lengths [Å] and angles [deg] for 1-methyl-4-amino-diamantane.

| | | | |
|---|---|---|---|
| C(5')-C(6')-C(11')#2 | 110.64(8) | C(1')-C(15')-H(15C) | 112.9(14) |
| C(5')-C(6')-C(11') | 110.64(8) | C(1')-C(15')-H(15D) | 111.4(9) |
| C(11')#2-C(6')-C(11') | 108.33(12) | H(15C)-C(15')-H(15D) | 106.6(12) |
| C(5')-C(6')-H(6') | 106.7(13) | C(9')-C(13')-C(1') | 111.43(12) |
| C(11')#2-C(6')-H(6') | 110.3(6) | C(9')-C(13')-H(13') | 111.7(8) |
| C(11')-C(6')-H(6') | 110.3(6) | C(1')-C(13')-H(13') | 107.4(8) |

Symmetry transformations used to generate equivalent atoms:
#1 x,y,-z+1/2 #2 x,y,-z+3/2

**Table 18**

Anisotropic displacement parameters ($Å^2 \times 10^3$) for 1-methyl-4-amino-diamantane. The anisotropic displacement factor exponent takes the form: $-2 \pi^2[h^2a^{*2} U^{11} + ... + 2 h k a^* b^* U^{12}]$

| | $U^{11}$ | $U^{22}$ | $U^{33}$ | $U^{23}$ | $U^{13}$ | $U^{12}$ |
|---|---|---|---|---|---|---|
| C1(1) | 12(1) | 23(1) | 27(1) | 0 | 0 | 0(1) |
| C1(2) | 14(1) | 21(1) | 18(1) | -1(1) | 0 | 0 |
| N(1) | 13(1) | 17(1) | 24(1) | 0 | 0 | 1(1) |
| C(1) | 12(1) | 16(1) | 16(1) | 0 | 0 | 2(1) |
| C(2) | 14(1) | 19(1) | 14(1) | 1(1) | 0(1) | 2(1) |
| C(3) | 15(1) | 18(1) | 15(1) | -2(1) | -2(1) | 2(1) |
| C(4) | 10(1) | 15(1) | 18(1) | 0 | 0 | 1(1) |
| C(5) | 11(1) | 18(1) | 21(1) | 0 | 0 | 4(1) |
| C(6) | 10(1) | 19(1) | 25(1) | 0 | 0 | 1(1) |
| C(9) | 17(1) | 19(1) | 36(1) | 0 | 0 | -2(1) |
| C(10) | 18(1) | 22(1) | 34(1) | -5(1) | -7(1) | 0(1) |
| C(11) | 15(1) | 21(1) | 20(1) | -1(1) | -5(1) | 2(1) |
| C(13) | 18(1) | 17(1) | 23(1) | 0 | 0 | 2(1) |
| C(15) | 14(1) | 21(1) | 24(1) | 0 | 0 | 5(1) |
| N(1') | 13(1) | 18(1) | 19(1) | 0 | 0 | -3(1) |
| C(1') | 10(1) | 17(1) | 16(1) | 0 | 0 | -1(1) |
| C(2') | 13(1) | 20(1) | 15(1) | -2(1) | 0(1) | -2(1) |
| C(3') | 14(1) | 20(1) | 15(1) | 1(1) | 0(1) | -3(1) |
| C(4') | 11(1) | 15(1) | 18(1) | 0 | 0 | -3(1) |
| C(5') | 10(1) | 20(1) | 31(1) | 0 | 0 | 0(1) |
| C(6') | 11(1) | 18(1) | 35(1) | 0 | 0 | 2(1) |
| C(9') | 16(1) | 16(1) | 36(1) | 0 | 0 | -4(1) |
| C(10') | 20(1) | 20(1) | 38(1) | 10(1) | -4(1) | -2(1) |
| C(11') | 15(1) | 21(1) | 27(1) | 7(1) | -6(1) | -1(1) |
| C(15') | 12(1) | 21(1) | 29(1) | 0 | 0 | 2(1) |
| C(13') | 12(1) | 20(1) | 23(1) | 0 | 0 | -3(1) |

**Table 19**

Hydrogen coordinates ($\times 10^4$) and isotropic displacement parameters ($Å^2 \times 10^3$) for 1-methyl-4-amino-diamantane

| | x | y | z | U(eq) |
|---|---|---|---|---|
| H(1A) | 3627(17) | 2874(5) | 3220(16) | 27 |
| H(1B) | 2250(30) | 3025(7) | 2500 | 27 |
| H(2) | 3532(16) | 4333(5) | 457(14) | 19 |
| H(3A) | 2189(17) | 3704(4) | 1226(14) | 19 |

(continued)

Hydrogen coordinates (x $10^4$) and isotropic displacement parameters (Å $^2$ x $10^3$) for 1-methyl-4-amino-diamantane

| | x | y | z | U(eq) |
|---|---|---|---|---|
| H(3B) | 3686(16) | 3557(5) | 459(15) | 19 |
| H(5) | 6031(17) | 3317(S) | 1747(15) | 20 |
| H(6) | 7460(20) | 3917(6) | 2500 | 21 |
| H(9) | 6220(30) | 5182(7) | 2500 | 28 |
| H(10A) | 7487(19) | 4633(5) | 3749(15) | 30 |
| H(10B) | 6053(19) | 4797(5) | 4567(17) | 30 |
| H(11) | 6033(17) | 4029(5) | 4515(16) | 22 |
| H(13) | 3645(17) | 5049(5) | 3264(16) | 23 |
| H(15A) | 1230(18) | 4648(5) | 1711(17) | 29 |
| H(15B) | 990(30) | 4204(7) | 2500 | 29 |
| H(1B') | -2130(30) | 2356(7) | 7500 | 25 |
| H(1A') | -3380(18) | 2542(5) | 8247(16) | 25 |
| H(2') | 591(17) | 3461(4) | 9512(15) | 19 |
| H(3'A) | -253(17) | 2765(5) | 8793(15) | 20 |
| H(3'B) | -1588(16) | 3013(5) | 9528(15) | 20 |
| H(5') | -3746(17) | 3349(5) | 8260(15) | 24 |
| H(6') | -2950(30) | 4035(7) | 7500 | 26 |
| H(9') | 1270(30) | 4569(7) | 7500 | 27 |
| H(10C) | 360(19) | 4288(5) | 5454(18) | 31 |
| H(10D) | -1020(19) | 4503(5) | 6238(16) | 31 |
| H(11') | -1760(18) | 3814(5) | 5454(16) | 25 |
| H(15C) | 1850(30) | 2825(7) | 7500 | 31 |
| H(15D) | 2950(20) | 3137(5) | 6713(17) | 31 |
| H(13') | 2550(17) | 3925(4) | 6726(15) | 22 |

**Table 20**

Hydrogen bonds for 1-methyl-4-amino-diamantane [Å and deg]

| D-H...A | d(D-H) | d(H...A) | d(D...A) | <(DHA) |
|---|---|---|---|---|
| N(1)-H(1A)...Cl(2)#3 | 0.913(16) | 2.391(16) | 3.2996(9) | 173.3(13) |
| N(1)-H(1B)...C1(1) | 0.88(2) | 2.24(2) | 3.1090(14) | 169.2(19) |
| N(1')-H(1B')...C1(1)#4 | 0.86(2) | 2.27(2) | 3.1295(14) | 177(2) |
| N(1')-H(lA')...C1(2) | 0.915(16) | 2.333(16) | 3.2439(8) | 173.5(14) |

Symmetry transformations used to generate equivalent atoms:
#1 x,y,-z+1/2 #2 x,y,-z+3/2 #3 x+1,y,-z+3/2 #4 x,-y+1/2,z+1/2

**Example 8:**

**Synthesis of MDT-28 (1-methyl-4,9-diaminodiamantane)**

The compound designated as MDT-28 (1-methyl-4,9-diaminodiamantane) was synthesized via the synthetic route depicted in Scheme 8.

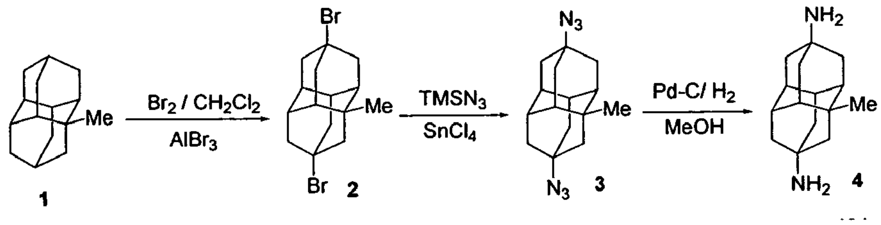


Scheme 8. Synthesis of MDT-28

***Step 1: Synthesis of 1-methyl-4,9-dibromodiamantane***

At room temperature, aluminum bromide (0.10 g) was added into a solution of 6.06 g (30 mmol) 1-methyldiamantane in 120 ml of dichloromethane. A solution of bromine (1 ml) in 5 ml of anhydrous dichloromethane was added dropwise to the above solution and finished in 30 min. The reaction was then quenched with saturated sodium hydrogen sulfite (cold ice/water mixture). The reaction mixture was extracted with dichloromethane (3x50 ml) and washed with 40 ml saturated sodium bicarbonate, and saturated sodium chloride. The solution was concentrated under vacuum and the residue was purified by column chromatography to give 1-methyl-4,9-dibromodiamantane (2), 2.49 g, yield 30% (2.27 g of unreacted compound 1 was recovered). White rhombic crystalloids, m.p.: 205-207˚C. $^1$H-NMR ($CDCl_3$, 300M), δ(ppm) : 2.65-2.61 (d, J = 12, 2H), 2.29-2.02 (s, 10H), 1.64-1.61 (d, J = 9, 2H), 1.04 (s, 3H). $^{13}$C-NMR ($CDCl_3$, 75M), δ (ppm), 63.47, 56.28, 56.12, 49.07, 48.43, 44.26, 43.97, 43.80, 40.51, 39.60, 39.34, 37.77, 25.94. Dept-135 ($CDCl_3$, 300M), δ(ppm), positive: 56.12, 49.07, 48.03, 43.80. negative: 44.26, 40.51, 39.34, 25.94. HRMS (SIMS), m/e: 358.9835 (100) $[M]^+$(C15H19 79Br 81Br requires 358.9833). IR ($cm^{-1}$): 2941,2885 and 1445.

***Step 2. Synthesis of 1-methyl-4,9-diazidodiamantane***

Azidotrimethyl silane (0.32 ml) was added to a solution of 4,9-dibromo-1-methyldiamantane (0.40 g) in 50 ml of dichloromethane (dried by refluxing with calcium hydroxide for 12 h). The solution was stirred at ice-bath temperature in an argon atmosphere for 10 min, then 0.4 ml of tin tetrachloride was injected into the above mixed solution. The reaction was continued for an additional 5 min. TLC indicated the reaction was completed. The reaction was quenched with 20 g crushed ice. The reaction mixture was extracted with 3×20ml dichloromethane. After removal of solvents, the residue was purified by column chromatography to give 1-methyl-4,9-diazidodiamantane (3) (0.09 g, 29%). $^1$H-NMR ($CDCl_3$, 300M), δ(ppm) : 2.13-2.10 (d, J=9, 2H), 2.01-1.79 (m, 10H), 1.61-1.54 (m, 7H), 1.01(s, 3H). $^{13}$C-NMR ($CDCl_3$, 75M), δ(ppm), 57.29, 56.58, 47.86, 41.20, 40.48, 39.89, 37.64, 36.36, 35.78, 35.34, 34.46, 24.96. Dept-135($CDCl_3$, 300M), δ(ppm), positive: 42.19, 41.93, 38.64, 37.35, 25.96, negative: 48.85, 41.47, 40.89, 36.34.

***Step 3. Synthesis of 1-methyl-4,9-diaminodiamantane***

A mixture of 0.06 g of 1-methyl-4,9-diazidodiamantane and 0.05 g of Pd/C in methanol (20 ml) was stirred in a hydrogen atmosphere overnight. The mixture was filtered through a Celite pad and the filtrate was concentrated. The resulting crude product was dissolved in 1% hydrochloride methanol solution. The solution was evaporated *in vacuo* to give a white solid which was washed with ethyl acetate. The white solid was carefully neutralized with concentrated aqueous ammonia solution, and then extracted with ethyl acetate to yield compound 4 (0.03 g free base, 61 %).

The $^1$H-, $^{13}$C- and Dept-135 NMR, and GC-MS, HRMS (SIMS) and IR spectra of MDT-28 are shown in Figures 76, 77, 78, 79, 80 and 81, respectively. White solid, m.p. > 300˚C. $^1$H-NMR (MeOD, 300M), δ(ppm), 7.90 (s, 4H), 2.26-2.23 (d, J=9, 2H), 2.15-1.90 (m, 8H), 1.74-1.67 (t, J=9, 6H), 1.08 (s, 3H). $^{13}$C-NMR ($CDCl_3$, 75M), δ(ppm), 52.46, 51.64, 42.31, 41.32, 40.70, 38.76, 37.43, 36.77, 36.57, 36.15, 35.80, 26.07. Dept-135 (MeOD, 300M), δ(ppm), positive: 40.90, 37.34, 36.02, 24.66, negative: 47.05, 39.91, 39.29, 34.73. HRMS (SIMS), *m/e*: 232.1937 $[M]^+$(C15H24N2 requires 232.1939). GC-MS, 85.07(area%). IR ($cm^{-1}$): 3430, 3020, 2879 and 2358.

**Example 9:**

**Synthesis of MDT-30 (racemic mixture of 1-amino-2-methyldiamantane and 1-amino-12-methyldiamantane) and MDT-31 (mixture of 1-methyl-2-aminodiamantane and 1-methyl-6-aminodiamantane)**

The compounds designated as MDT-30 (racemic mixture of 1-amino-2-methyldiamantane and 1-amino-12-methyldiamantane) and MDT-31 (mixture of 1-methyl-2-aminodiamantane and 1-methyl-6-aminodamantane) were synthesized via the synthetic route depicted in Scheme 9.

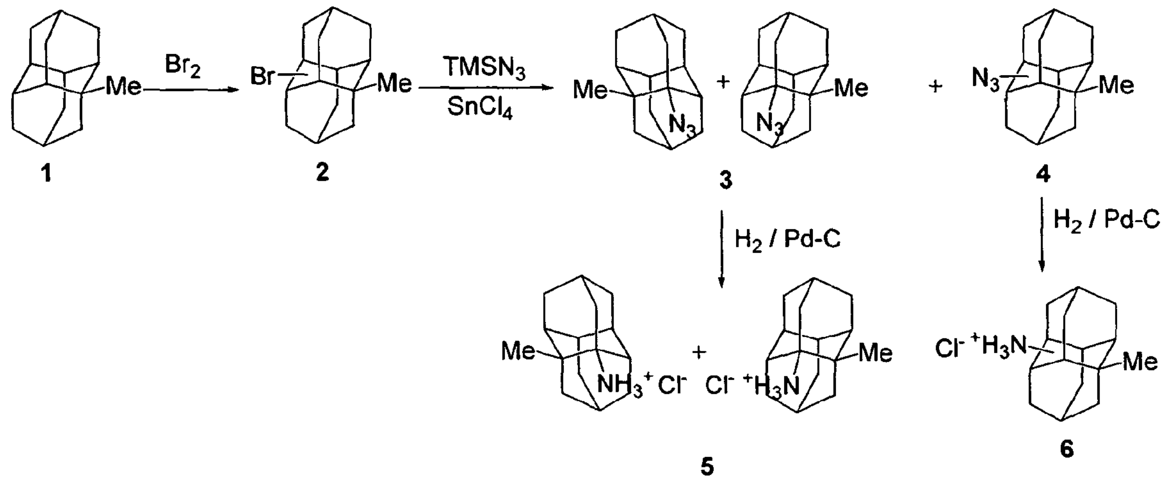


Scheme 9. Synthesis of MDT-30 and MDT-31

The bromination of **1** resulted in a mixture of three mono-brominated diamantanes **2.** The mixture **2** was treated with $TMSN_3$ to yield a major product **3** (tentatively assigned structure) and a mixture of two mono-azido-substituted diamantanes **4.** Hydrogenation of **3** and **4** provided the diamantine mono-amines 5 (MDT-30) and **6** (MDT-31), respectively.

***Step 1: Synthesis of mixture 2***

A solution of bromine (22 ml) in methylene chloride (40 ml) was added to a well-stirred solution of 1-methyl diamantane (8.69 g, 43 mmol) in 100ml methylene chloride in an ice-water bath. The reaction mixture was then warmed to 40 ˚C. The reaction was quenched by saturated $Na_2SO_3$. The organic layer was separated, washed with saturated $NaHCO_3$, dried over $Na_2SO_4$ and concentrated. The residue was purified by column chromatography to give 7.28 g (60.3%) of mixture of 1-methyl bromodiamantane 2 with recovery of 80 mg (4%) of the starting material **1.**

***Step 2: Synthesis of mixture 3 and 4***

$SnCl_4$ (1.31 ml) was added slowly to a well-stirred, ice-water bath cooled solution of $TMSN_3$ (2.58 ml, 19.65 mmol) and 1-methyl-7-bromodiamantane (3.68 g, 13.1 mmol) in methylene chloride (30 ml). The reaction mixture was stirred at room temperature overnight and then was poured into 20 ml of ice-water. The organic layer was separated, washed with saturated $NaHCO_3$, dried over $Na_2SO_4$ and concentrated. The residue was purified by column chromatography to give 0.73 g (23%) of 1-methyl-7-azido diamantane **3** and also a mixture of products **4** 0.19 g (6%). Characterization of compound **3:** mp: 148˚C. $^1H$ NMR ($CDCl_3$, 300 MHz) δ (ppm): 0.91 (s, 3H), 1.12~1.16 (m, 1H), 1.36~1.41 (m, 1H), 1.44~1.49 (m, 1H), 1.49~1.56 (m, 1H), 1.60~1.64 (m, 4H), 1.72 (s, 1H), 1.80~1.88 (m, 3H), 1.96~2.00 (m, 1H), 2.05~2.09 (m, 2H), 2.18~2.22 (m, 1H). $^{13}C$ NMR ($CDCl_3$, 75 MHz) δ (ppm): 22.23, 27.24, 29.24, 32.12, 33.08, 36.49, 37.81, 37.83, 38.04, 39.87, 41.25, 41.33, 44.34, 66.09. IR (KBr) ν ($cm^{-1}$): 703.8, 903.4, 1016.3, 1256.4, 1439.6, 2086.6, 2913.9. MS : m/z (%): 243(1), 215(17), 201(100). Characterization of compound 4: $^1H$ NMR ($CDCl_3$, 300 MHz)δ (ppm): 0.92 (s,1H), 1.00 (s,2H), 1.38~1.56 (m, 7H), 1.56~1.86 (m, 11H), 1.97~2.13 (m,5H). $^{13}C$ NMR ($CDCl_3$, 75 MHz) δ (ppm): 25.16, 25.61, 25.87, 27.02, 27.48, 28.99, 29.81, 32.08, 32.72, 33.06, 33.48, 33.55, 36.28, 37.17, 39.58, 40.24, 41.03, 41.11, 41.89, 42.39, 43.21, 44.78, 45.76, 46.45, 63.79, 64.60.

***Step 3: Synthesis of mixture 5 (MDT-30)***

Pd/C (200 mg) was added to a solution of 1-methyl 2-azido-diamantane (400 mg) in methylene chloride (10 ml) and methanol (40 ml). The reaction mixture was stirred under hydrogen atmosphere at room temperature overnight. The solid was then filtered off and the filtrate was concentrated to give 350 mg (98%) of 1- methyl-2-amino-diamantane 5.

The $^1H$-, $^{13}C$-NMR, and MS spectra of MDT-30 are shown in Figures 82, 83 and 84, respectively. Mp: >300 ˚C. $^1H$-NMR ($CDCl_3$, 300 MHz): δ (ppm) 1.02 (s, 3H), 1.22~1.26 (d, 1H), 1.37~1.41 (d, 2H), 1.51~1.75 (m, 10H), 1.90 (s, 2H), 1.99~2.05 (m, 3H), 2.11~2.30 (m, 3H). $^{13}C$-NMR ($CDCl_3$, 75 MHz) δ (ppm): 22.13, 27.24, 28.79, 32.17, 32.29,

37.22, 37.91,38.18,38.33,39.58,40.70,44.55. IR (KBr) ν ($cm^{-1}$): 752.6, 1513.4, 2910.9, 3033.2, 3673.6. MS : m/z (%): 217(12), 200(100), 185(3). The crystal structure of MDT-30 with atom numbering is shown in Figure 85. MDT-30 is actually a racemic mixture of 1-amino-2-methyldiamantane and 1-amino-12-methyldiamantane (Figure 86), although Figure 85 only depicts the structure for 1-amino-2-methyldiamantane.

**Table 21**

Atomic coordinates (x $10^4$) and equivalent isotropic displacement parameters (Å$^2$ x $10^3$) for 1-amino-2-methyldiamantane hydrochloride monohydrate. U(eq) is defined as one third of the trace of the orthogonalized $U^{ij}$ tensor.

| | x | y | z | U(eq) |
|---|---|---|---|---|
| C1(1) | 4636(1) | 2069(1) | 2095(1) | 17(1) |
| O(1) | 2851(2) | 5315(1) | 2570(1) | 25(1) |
| N(1) | 3085(3) | 3308(2) | 3012(1) | 15(1) |
| C(1) | 4152(3) | 3497(2) | 3463(1) | 15(1) |
| C(2) | 4404(3) | 2488(2) | 3769(1) | 15(1) |
| C(3) | 2755(3) | 2128(2) | 3996(1) | 19(1) |
| C(4) | 1972(3) | 2978(2) | 4317(1) | 20(1) |
| C(5) | 3150(3) | 3242(2) | 4737(1) | 23(1) |
| C(6) | 4783(3) | 3632(2) | 4523(1) | 20(1) |
| C(7) | 5590(3) | 2795(2) | 4196(1) | 17(1) |
| C(8) | 7244(3) | 3210(2) | 4002(1) | 20(1) |
| C(9) | 6941(3) | 4186(2) | 3689(1) | 19(1) |
| C(10) | 6140(3) | 5039(2) | 4007(1) | 22(1) |
| C(11) | 4507(3) | 4619(2) | 4210(1) | 19(1) |
| C(12) | 3313(3) | 4336(2) | 3784(1) | 17(1) |
| C(13) | 5793(3) | 3918(2) | 3262(1) | 18(1) |
| C(14) | 1678(3) | 3941(2) | 3997(1) | 20(1) |
| C(15) | 5108(3) | 1577(2) | 3468(1) | 21(1) |

**Table 22**

Bond lengths [Å] and angles [deg] for 1-amino-2-methyldiamantane hydrochloride monohydrate.

| | | | |
|---|---|---|---|
| 0(1)-H(1D) | 0.819(10) | C(6)-H(6) | 1.00(2) |
| O(1)-H(1E) | 0.820(10) | C(7)-C(8) | 1.537(3) |
| N(1)-C(1) | 1.517(3) | C(7)-H(7) | 0.98(2) |
| N(1)-H(IA) | 0.95(3) | C(8)-C(9) | 1.530(3) |
| N(1)-H(1B) | 0.93(3) | C(8)-H(8A) | 1.03(2) |
| N(1)-H(1C) | 0.93(3) | C(8)-H(8B) | 0.97(3) |
| C(1)-C(13) | 1.537(3) | C(9)-C(13) | 1.526(3) |
| C(1)-C(12) | 1.541(3) | C(9)-C(10) | 1.535(3) |
| C(1)-C(2) | 1.548(3) | C(9)-H(9) | 0.96(2) |
| C(2)-C(15) | 1.533(3) | C(10)-C(11) | 1.533(4) |
| C(2)-C(3) | 1.543(3) | C(10)-H(10A) | 1.08(2) |
| C(2)-C(7) | 1.558(3) | C(10)-H(10B) | 0.97(3) |
| C(3)-C(4) | 1.532(3) | C(11)-C(12) | 1.550(3) |
| C(3)-H(3A) | 1.01(2) | C(11)-H(11) | 0.96(3) |
| C(3)-H(3B) | 0.98(3) | C(12)-C(14) | 1.533(3) |
| C(4)-C(5) | 1.524(3) | C(12)-H(12) | 0.98(2) |
| C(4)-C(14) | 1.526(3) | C(13)-H(13A) | 1.02(2) |
| C(4)-H(4) | 1.02(3) | C(13)-H(13B) | 0.98(2) |
| C(5)-C(6) | 1.531(3) | C(14)-H(14A) | 0.98(3) |
| C(5)-H(5A) | 1.00(3) | C(14)-H(14B) | 0.99(3) |

(continued)

Bond lengths [Å] and angles [deg] for 1-amino-2-methyldiamantane hydrochloride monohydrate.

| | | | |
|---|---|---|---|
| C(5)-H(5B) | 0.98(3) | C(15)-H(15A) | 0.97(3) |
| C(6)-C(7) | 1.537(3) | C(15)-H(15B) | 1.01(3) |
| C(6)-C(11) | 1.538(3) | C(15)-H(15C) | 0.99(3) |
| H(1D)-O(1)-H(1E) | 105(3) | C(13)-C(1)-C(12) | 109.86(19) |
| C(1)-N(1)-H(1A) | 114.6(14) | N(1)-C(1)-C(2) | 111.97(18) |
| C(1)-N(1)-H(1B) | 112.3(15) | C(13)-C(1)-C(2) | 111.59(18) |
| H(IA)-N(1)-H(1B) | 106(2) | C(12)-C(1)-C(2) | 109.65(18) |
| C(1)-N(1)-H(1C) | 108.5(15) | C(15)-C(2)-C(3) | 108.0(2) |
| H(1A)-N(1)-H(IC) | 110(2) | C(15)-C(2)-C(1) | 113.45(19) |
| H(1B)-N(1)-H(1C) | 105(2) | C(3)-C(2)-C(1) | 110.33(19) |
| N(1)-C(1)-C(13) | 105.37(18) | C(15)-C(2)-C(7) | 110.92(19) |
| N(1)-C(1)-C(12) | 108.26(18) | C(3)-C(2)-C(7) | 108.34(18) |
| C(1)-C(2)-C(7) | 105.67(18) | H(8A)-C(8)-H(8B) | 112(2) |
| C(4)-C(3)-C(2) | 112.1(2) | C(13)-C(9)-C(8) | 109.7(2) |
| C(4)-C(3)-H(3A) | 109.3(14) | C(13)-C(9)-C(10) | 109.0(2) |
| C(2)-C(3)-H(3A) | 110.3(13) | C(8)-C(9)-C(10) | 109.7(2) |
| C(4)-C(3)-H(3B) | 110.7(14) | C(13)-C(9)-H(9) | 108.4(14) |
| C(2)-C(3)-H(3B) | 107.3(14) | C(8)-C(9)-H(9) | 108.9(15) |
| H(3A)-C(3)-H(3B) | 107.0(19) | C(10)-C(9)-H(9) | 111.1(15) |
| C(5)-C(4)-C(14) | 110.1(2) | C(11)-C(10)-C(9) | 108.7(2) |
| C(5)-C(4)-C(3) | 108.7(2) | C(11)-C(10)-H(10A) | 109.6(13) |
| C(14)-C(4)-C(3) | 108.30(19) | C(9)-C(10)-H(10A) | 109.7(13) |
| C(5)-C(4)-H(4) | 109.9(13) | C(11)-C(10)-H(10B) | 112.3(15) |
| C(14)-C(4)-H(4) | 108.8(14) | C(9)-C(10)-H(10B) | 109.7(14) |
| C(3)-C(4)-H(4) | 110.9(14) | H(10A)-C(10)-H(10B) | 107(2) |
| C(4)-C(5)-C(6) | 109.5(2) | C(10)-C(11)-C(6) | 111.1(2) |
| C(4)-C(5)-H(5A) | 109.9(15) | C(10)-C(11)-C(12) | 110.79(19) |
| C(6)-C(5)-H(5A) | 110.3(15) | C(6)-C(11)-C(12) | 108.04(19) |
| C(4)-C(5)-H(5B) | 110.7(15) | C(10)-C(11)-H(11) | 110.6(15) |
| C(6)-C(5)-H(5B) | 108.9(15) | C(6)-C(11)-H(11) | 107.8(15) |
| H(5A)-C(5)-H(5B) | 108(2) | C(12)-C(11)-H(11) | 108.4(15) |
| C(5)-C(6)-C(7) | 111.1(2) | C(14)-C(12)-C(1) | 111.51(19) |
| C(5)-C(6)-C(11) | 110.5(2) | C(14)-C(12)-C(11) | 109.82(19) |
| C(7)-C(6)-C(11) | 108.44(19) | C(1)-C(12)-C(11) | 107.89(19) |
| C(5)-C(6)-H(6) | 111.8(14) | C(14)-C(12)-H(12) | 109.1(14) |
| C(7)-C(6)-H(6) | 106.6(14) | C(1)-C(12)-H(12) | 109.8(14) |
| C(11)-C(6)-H(6) | 108.3(14) | C(11)-C(12)-H(12) | 108.7(14) |
| C(6)-C(7)-C(8) | 109.2(2) | C(9)-C(13)-C(1) | 109.82(19) |
| C(6)-C(7)-C(2) | 109.94(19) | C(9)-C(13)-H(13A) | 111.9(14) |
| C(8)-C(7)-C(2) | 111.86(19) | C(1)-C(13)-H(13A) | 110.5(14) |
| C(6)-C(7)-H(7) | 109.2(14) | C(9)-C(13)-H(13B) | 107.4(14) |
| C(8)-C(7)-H(7) | 111.2(14) | C(1)-C(13)-H(13B) | 107.8(15) |
| C(2)-C(7)-H(7) | 105.3(14) | H(13A)-C(13)-H(13B) | 109.2(19) |
| C(9)-C(8)-C(7) | 109.4(2) | C(4)-C(14)-C(12) | 110.1(2) |
| C(9)-C(8)-H(8A) | 106.9(14) | C(4)-C(14)-H(14A) | 109.8(14) |
| C(7)-C(8)-H(8A) | 106.9(13) | C(12)-C(14)-H(14A) | 110.2(15) |
| C(9)-C(8)-H(8B) | 110.0(15) | C(4)-C(14)-H(14B) | 111.4(15) |
| C(7)-C(8)-H(8B) | 111.4(15) | C(12)-C(14)-H(14B) | 110.1(14) |
| H(14A)-C(14)-H(14B) | 105(2) | C(2)-C(15)-H(15C) | 110.3(15) |
| C(2)-C(15)-H(15A) | 110.1(16) | H(15A)-C(15)-H(15C) | 111(2) |

(continued)

Bond lengths [Å] and angles [deg] for 1-amino-2-methyldiamantane hydrochloride monohydrate.

| | | | |
|---|---|---|---|
| C(2)-C(15)-H(15B) | 108.1(15) | H(15B)-C(15)-H(15C) | 112(2) |
| H(15A)-C(15)-H(15B) | 105(2) | | |

**Table 23**

Anisotropic displacement parameters ($Å^2 \times 10^3$) for 1-amino-2-methyldiamantane hydrochloride monohydrate. The anisotropic displacement factor exponent takes the form: $-2 \pi^2[h^2a^{*2}U^{11}+...+2hka^*b^*U^{12}]$.

| | $U^{11}$ | $U^{22}$ | $U^{33}$ | $U^{23}$ | $U^{13}$ | $U^{12}$ |
|---|---|---|---|---|---|---|
| C1(1) | 16(1) | 16(1) | 19(1) | 0(1) | 1(1) | -1(1) |
| O(1) | 25(1) | 17(1) | 33(1) | 5(1) | -11(1) | -3(1) |
| N(1) | 16(1) | 13(1) | 16(1) | 1(1) | -1(1) | 0(1) |
| C(1) | 14(1) | 14(1) | 16(1) | 0(1) | -2(1) | 0(1) |
| C(2) | 15(1) | 13(1) | 17(1) | 2(1) | 0(1) | 1(1) |
| C(3) | 18(1) | 16(1) | 22(1) | 4(1) | -1(1) | -2(1) |
| C(4) | 17(1) | 23(1) | 20(1) | 4(1) | 4(1) | 2(1) |
| C(5) | 24(1) | 25(2) | 19(1) | 1(1) | 4(1) | 3(1) |
| C(6) | 22(1) | 21(1) | 16(1) | -1(1) | -2(1) | 0(1) |
| C(7) | 19(1) | 14(1) | 19(1) | 3(1) | -3(1) | 1(1) |
| C(8) | 15(1) | 22(1) | 22(1) | 0(1) | -2(1) | 0(1) |
| C(9) | 14(1) | 21(1) | 23(1) | 3(1) | -1(1) | -5(1) |
| C(10) | 26(2) | 14(1) | 25(1) | -1(1) | -6(1) | -5(1) |
| C(11) | 23(1) | 13(1) | 22(1) | -3(1) | -3(1) | 2(1) |
| C(12) | 19(1) | 12(1) | 20(1) | 1(1) | -1(1) | 1(1) |
| C(13) | 17(1) | 17(1) | 20(1) | 2(1) | 0(1) | 0(1) |
| C(14) | 18(1) | 20(1) | 22(1) | -2(1) | 1(1) | 3(1) |
| C(15) | 24(2) | 16(1) | 23(1) | -2(1) | -2(1) | 1(1) |

**Table 24**

Hydrogen coordinates ($\times 10^4$) and isotropic displacement parameters ($Å^2 \times 10^3$) for 1-amino-2-methyldiamantane hydrochloride monohydrate.

| | x | y | z | U(eq) |
|---|---|---|---|---|
| H(1D) | 3520(30) | 5737(17) | 2676(9) | 30 |
| H(1E) | 2160(30) | 5667(18) | 2424(8) | 30 |
| H(1A) | 2060(30) | 2980(20) | 3079(8) | 23 |
| H(1B) | 3610(30) | 2900(20) | 2777(9) | 23 |
| H(1C) | 2910(30) | 3950(20) | 2855(9) | 23 |
| H(3A) | 1960(30) | 1920(18) | 3727(9) | 22 |
| H(3B) | 2980(30) | 1500(20) | 4191(8) | 22 |
| H(4) | 870(30) | 2732(19) | 4458(9) | 24 |
| H(5A) | 3340(30) | 2610(20) | 4947(9) | 27 |
| H(5B) | 2680(30) | 3790(20) | 4949(9) | 27 |
| H(6) | 5590(30) | 3802(19) | 4788(9) | 24 |
| H(7) | 5740(30) | 2155(19) | 4389(8) | 21 |
| H(8A) | 7910(30) | 3443(19) | 4304(9) | 24 |
| H(8B) | 7810(30) | 2690(20) | 3810(9) | 24 |
| H(9) | 7970(30) | 4419(19) | 3555(8) | 23 |
| H(10A) | 6950(30) | 5239(19) | 4309(9) | 26 |
| H(10B) | 5990(30) | 5670(20) | 3811(9) | 26 |

(continued)

Hydrogen coordinates (x $10^4$) and isotropic displacement parameters ($Å^2$ x $10^3$) for 1-amino-2-methyldiamantane hydrochloride monohydrate.

| | x | y | z | U(eq) |
|---|---|---|---|---|
| H(11) | 3990(30) | 5130(20) | 4415(9) | 23 |
| H(12) | 3110(30) | 4961(19) | 3588(8) | 20 |
| H(13A) | 6310(30) | 3390(20) | 3025(8) | 22 |
| H(13B) | 5560(30) | 4570(20) | 3085(9) | 22 |
| H(14A) | 1150(30) | 4490(20) | 4193(9) | 24 |
| H(14B) | 900(30) | 3792(19) | 3728(9) | 24 |
| H(15A) | 4280(30) | 1310(20) | 3243(9) | 31 |
| H(15B) | 5340(30) | 980(20) | 3701(9) | 31 |
| H(15C) | 6100(30) | 1800(20) | 3287(9) | 31 |

**Table 25**

Hydrogen bonds for 1-amino-2-methyldiamantane hydrochloride monohydrate [Å and deg].

| D-H...A | d(D-H) | d(H...A) | d(D...A) | <(DHA) |
|---|---|---|---|---|
| O(1)-H(1D)...Cl(1)#1 | 0.819(10) | 2.351(10) | 3.1661(19) | 173(3) |
| O(1)-H(1E)...Cl(1)#2 | 0.820(10) | 2.478(12) | 3.2808(18) | 167(3) |
| N(1)-H(1A)...Cl(1)#3 | 0.95(3) | 2.34(3) | 3.233(2) | 157.0(19) |
| N(1)-H(1B)...Cl(1) | 0.93(3) | 2.29(3) | 3.206(2) | 169(2) |
| N(1)-H(1C)...O(1) | 0.93(3) | 1.91(3) | 2.840(3) | 172(2) |

Symmetry transformations used to generate equivalent atoms:
#1 -x+1,y+1/2,-z+1/2 #2 -x+1/2,y+1/2,z #3 x-1/2,y,-z+1/2

***Step 4: Synthesis of mixture 6 (MDT 31)***

Pd/C (200 mg) was added to a solution of 1-methyl azidodiamantane (100 mg) in methylene chloride (10 ml) and methanol (40 ml). The reaction mixture was stirred under hydrogen atmosphere at room temperature overnight. The solid was then filtered off and the filtrate was concentrated to give 90 mg of 1-methyl amino diamantane mixture product 6.

The $^1H$- and $^{13}C$-NMR spectra of MDT-31 are shown in Figures 87 and 88, respectively. Mp: >300 ˚C . $^1H$ NMR ($CDCl_3$, 300 MHz)δ (ppm): 0.92 (s,1H), 1.00 (s,2H), 1.37~1.57 (m, 8H), 1.57~1.68 (m, 2H), 1.82~2.06 (m, 9H), 2.29~2.33 (m,1H), 6.66 (s,3H). $^{13}C$ NMR ($CDCl_3$, 75 MHz) δ (ppm): 24.49, 25.05, 25.23, 25.81, 26.31, 27.75, 28.56, 29.81, 31.35, 31.44, 31.86, 32.37, 32.82, 35.36, 36.48 37.53, 38.72, 39.57, 40.38, 40.53, 41.16, 42.23, 42.59, 42.66, 44.15, 45.06, 46.12, 55.03, 55.83.

**Example 10:**

**Synthesis of MDT-32 (mixture of 1-amino-4-methyldiamantane and 2-amino-4-methyldiamantane)**

The compounds designated as MDT-32 (mixture of 1-amino-4-methyldiamantane and 2-amino-4-methyldiamantane) were synthesized via the synthetic route depicted in Scheme 10.

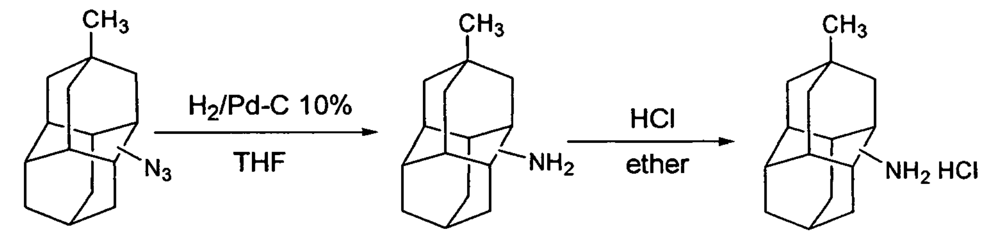


1 2 3

Scheme 10. Synthesis of MDT-32

***Synthesis of mixture 1***

4-Methyldiamantane was separated and purified by high resolution distillation and recrystallization from acetone several times. The 4-methyldiamantane was brominated in neat bromine at room temperature for about 7 hours. After work-up and column chromatography purification on silica gel, the mixture of 4-methyl-1-bromo-diamantane and 4-methyl-2-bromo-diamantane was converted to a mixture of 4-methyl-1-azido-diamantane and 4-methyl-2-azido-diamantane under $SnCl_4/TMSN_3$ conditions as described in this application.

***Synthesis of mixture 2 and 3***

The mixture of 4-methyl-1-azido-diamantane and 4-methyl-2-azido-diamantane (5.4 g) was dissolved in 50 ml of tetrahydrofuran. Pd-C (0.5 g ; 10% wt) was then added, and the mixture sealed in a Parr Bomb with mechanic stirring. The hydrogen pressure was set at 40 psi for 23 hrs at room temperature.

The reaction was stopped with the slow release of the pressure. Hexane (50 ml) was added to the reaction and the catalyst was filtered off with normal filter paper. The solvent was removed under vacuum. The clear oil obtained was then neutralized with hydrogen chloride in ether, forming a white precipitate. The ether was filtered off, and the white powder was washed with acetone and dried in air. Yield: 2.15 g.

The GC-MS and MS spectra of MDT-32 are shown in Figure 89. GC-MS test on the free amine form: t=5.57, 5.61; Mr=217 D2-NH2 (4-methyl-1(2)-diamantane). Purity of the HCl salt is higher than 95%.

**Example 11:**

**Synthesis of MDT-33 (1,6-dimethyl-4-aminodiamantane)**

The compound designated as MDT-33 (1,6-dimethyl-4-aminodiamantane) was synthesized via the synthetic route depicted in Scheme 11.

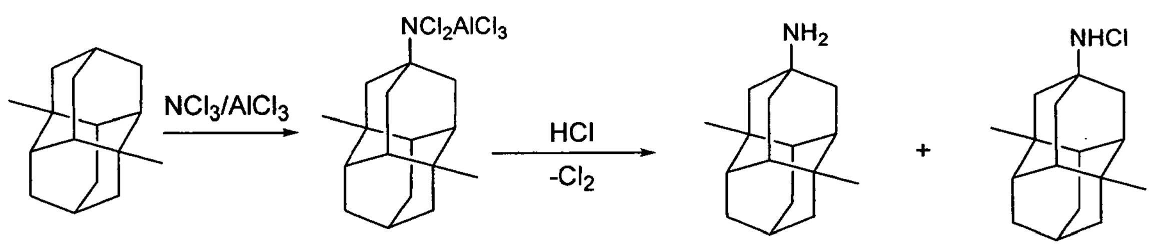


1 2 3

Scheme 11. Synthesis of MDT-33

***Synthesis of $NCl_3$***

$Ca(OCl)_2$ (10 g; 0.07 mol) was charged into 20 ml water (HPLC grade) in a 250 ml three-neck round bottom flask with condenser, addition funnel, thermometer and magnetic stirring bar. The flask was cooled to ice bath temperature (4 ˚C) and 30 ml $CH_2Cl_2$ was added. A solution of 2.2 g (0.04 mol) of $NH_4Cl$ in 5 ml of concentrated HCl and 15 ml of HPLC grade water was slowly added to the flask over 30 min. The temperature kept at 40 ˚F throughout the reaction. A bright yellow color appeared. When the addition was completed, the reaction mixture was stirred an additional 15 min., and then poured into a 125 ml separatory funnel. The bottom bright yellow organic phase was removed, washed with HPLC water, and dried over anhydrous $Na_2SO_4$ for 1 hr.

In order to standardize the concentration of $NCl_3$, a 1 ml aliquot of the $NCl_3$ solution was added to 2 g of NaI in 50 ml of 80% acetic acid. This solution will be violet after all the $I_2$ has been liberated from the reaction. 5.0 ml of this solution was titrated with 0.1 N $Na_2S_2O_3$ (sodium thiosulfate) solution to reduce the $I_2$ to $I^-$. Typically, two titrations were performed. The final concentration of the $NCl_3$ was determined to be about 5 M. ***Synthesis of apical amine from $NCl_3$-$AlCl_3$ catalytic reaction***

1,6-Dimethyl-diamantane (2.16 g; 10 mmol) was dissolved in 150 ml dry 1,2-dichloroethane and charged into a three-neck 250 ml round-bottom flask equipped with condenser, pre-cooled addition funnel, $N_2$ purge line/thermometer adapter and magnetic stirring bar. All were placed inside a round-bottom Dewar cold bath containing o-xylene/dry ice (-29 ˚C) inside a hood.

Anhydrous $AlCl_3$ (1.56 g; 11 mmol) was added under nitrogen. $CH_2Cl_2$ (25 ml) and $NCl_3$ (10.5 ml; 52.5 mmol) were added from the addition funnel slowly over 2 hrs. The temperature was elevated to 0 ˚C (ice bath) for 1hr.

The reaction was quenched with 150 ml 18% HCl water solution with fast $N_2$ purge to remove the $Cl_2$ generated in the quenching process. A white solution formed and was allowed to stand for 20 min at room temperature. The aqueous layer was collected and washed with 150 ml $CH_2Cl_2$ and 150 ml ether once. At this stage, foam formed during separation. Generally, the solution must stand for a longer time until good separation occurs.

The aqueous solution was treated with ammonium hydroxide to pH 14, then extracted with 100 ml $CH_2Cl_2$ three times. All the organic phases were combined and dried with $Na_2SO_4$ for 0.5 hr. The solvent was removed under rotavap, and a clear oil was obtained. Yield: 0.8 g, 34.6%.

The GC-MS spectrum of MDT-33 is shown in Figure 90. GC-MS: t=6.31; Mr=231 2CH3-D2NH2 (1,6-dimethyl-4-amino-diamantane); t=8.2 2CH3-D2-NHCl; Mr=265 (1,6-dimethyl-4-aminochloro diamantane). Purity higher than 90% for the two components.

**Example 12:**

**Synthesis of MDT-34 (mixture of 4-methyl-9-aminodiamantane and 4-aminodiamantane)**

The compounds designated as MDT-34 (mixture of 4-methyl-9-aminodiamantane and 4-aminodiamantane) were synthesized via the synthetic route depicted in Scheme 12.

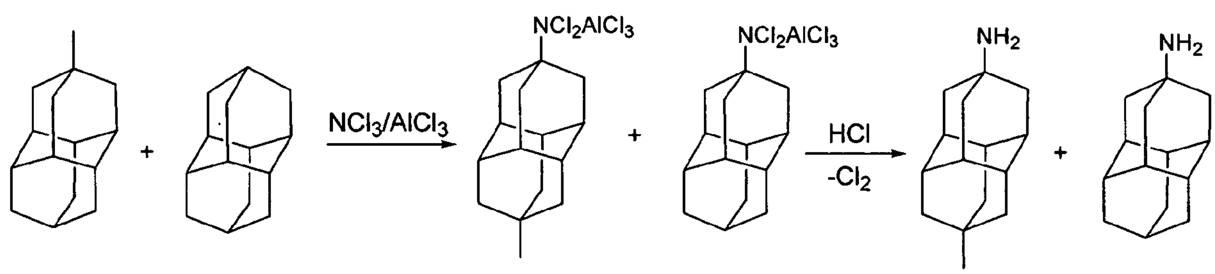


1 2 3

Scheme 12. Synthesis of MDT-34

***Synthesis of $NCl_3$***

$Ca(OCl)_2$ (10 g; 0.07 mol) was charged into 20 ml water (HPLC grade) in a 250 ml three-neck round bottom

flask with condenser, addition funnel, thermometer and magnetic stirring bar. The flask was cooled to ice bath temperature (4 ˚C) and 30 ml $CH_2Cl_2$ was added. A solution of 2.2 g (0.04 mol) of $NH_4Cl$ in 5 ml of concentrated HCl and 15 ml of HPLC grade water was slowly added to the flask over 30 min. The temperature kept at 40 ˚F throughout the reaction. A bright yellow color appeared. When the addition was completed, the reaction mixture was stirred an additional 15 min., and then poured into a 125 ml separatory funnel. The bottom bright yellow organic phase was removed, washed with HPLC water, and dried over anhydrous $Na_2SO_4$ for 1hr.

In order to standardize the concentration of $NCl_3$, a 1 ml aliquot of the $NCl_3$ solution was added to 2 g of NaI in 50 ml of 80% acetic acid. This solution will be violet after all the $I_2$ has been liberated from the reaction. 5.0 ml of this solution was titrated with 0.1 N $Na_2S_2O_3$ (sodium thiosulfate) solution to reduce the $I_2$ to $I^-$. Typically, two titrations were performed. The final concentration of the $NCl_3$ was determined to be about 5 M.

***Synthesis of apical amines from $NCl_3$-$AlCl_3$ catalytic reaction***

4-Methyl-diamantane/diamantane mixture (Batch #2) (4 g; 20mmol) was dissolved in 200 ml dry 1,2-dichloroethane and charged into a three-neck 500 ml round-bottom flask equipped with condenser, pre-cooled addition funnel, $N_2$ purge line/thermometer adapter and magnetic stirring bar. All were placed inside a round-bottom Dewar cold bath containing o-xylene/dry ice (-29 ˚C) inside a hood.

Anhydrous $AlCl_3$ (3 g; 20 mmol) was added under nitrogen. $CH_2Cl_2$ (25 ml) and $NCl_3$ (13 ml; 65 mmol) were added from the additional funnel slowly over 2 hrs. The temperature was elevated to 0 ˚C (ice bath) for 1 hr.

The reaction was quenched with 150 ml 18% HCl water solution with fast $N_2$ purge to remove the $Cl_2$ generated in the quenching process. A white solution formed and was allowed to stand for 20 min at room temperature. The aqueous layer was collected and washed with 150 ml $CH_2Cl_2$ and 150 ml ether once. At this stage, foam formed during separation. Generally, the solution must stand for a longer time until good separation occurs.

The aqueous solution was treated with ammonium hydroxide to pH 14, then extracted with 100 ml $CH_2Cl_2$ three times. All the organic phases were combined and dried with $Na_2SO_4$ for 0.5 hr. The solvent was removed under rotavap, and a white powder was obtained. Yield: 1.5 g (free base), 36.4%.

The GC-MS spectrum of MDT-34 is shown in Figure 91, and the MS spectra of 4-aminodiamantane and 4-methyl-9-aminodiamantane are shown in Figures 92 and 93, respectively. GC-MS: t=5.44; Mr=203 D2-NH2 (4-amino-diamantane); t=5.54; Mr=217 4-CH3-D2-NHCl (4-methyl-9-amino diamantane). The ratio of the 4-methyl-9-amino diamantane: 4-amino-diamantane=75:25.

**Example 13:**

**Synthesis of MDT-43 (1,6-dimethyl-2-aminodiamantane)**

The compound designated as MDT-43 (1,6-dimethyl-2-aminodiamantane hydrochloride salt) was synthesized via the synthetic route depicted in Scheme 13.

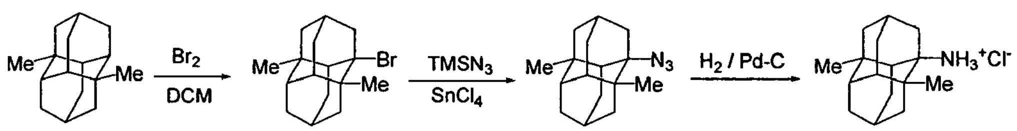


1 2 3 4

Scheme 13. Synthesis of MDT-43

***Steps 1 and 2: Synthesis of compound 3***

1,6-Dimethyldiamantane 1 (2 g; 9.26 mmol) was dissolved in 50 ml of dichloromethane, to which was added dropwise a solution of 1 ml of bromine in 10 ml of dichloromethane at ice-bath temperature. After stirring at room temperature overnight, the reaction was quenched with saturated sodium hydrogen sulfite/ice-water. The mixture was extracted with dichloromethane (3×30ml), and washed with saturated sodium bicarbonate solution (2×20ml), and then saturated sodium chloride solution. Concentration of the organic layer and evaporation of the solvents in vacuo gave the crude product 2 (1.73 g, yield 60%). This material was used in the azide formation directly without further purification.

Azidotrimethyl silane (1 ml) was added to the solution of 2-bromo-1,6-dimethyldiamantane 2 (0.48 g, 1.6 mmol) in 30 ml of dichloromethane. The reaction mixture was stirred at ice-bath temperature in an argon atmosphere for 10 min. Then tin tetrachloride (0.1 ml) was injected into the reaction system. The ice bath was removed after 5 min. When

TLC showed completion of the reaction, the solution was poured into 20 g of ice, and extracted with dichloromethane (3x15ml). After evaporation of the solvents, the residue was purified by column chromatography to give compound 3 (0.39 g, 93%). White solid, m.p.: 118-120 °C.

$^1$H-NMR ($CDCl_3$, 300M), δ(ppm): 2.24-2.19 (d, J=15, 1H, 2.08-2.00 (m, 3H), 1.86-1.34 (m, 14H), 1.13-1.08 (d, J=15, 1H), 1.01 (s, 3H), 0.91 (s,3H). $^{13}$C-NMR ($CDCl_3$, 75 M), δ(ppm), 66.72, 46.51, 45.64, 44.74, 42.22, 41.84, 37.76, 36.77, 36.43, 32.84, 32.09, 29.79, 27.37,27.27,25.70, and 21.98. Dept-135 ($CDCl_3$, 300M), δ(ppm), positive: 46.52, 41.85, 36.43, 32.84, 32.10, 27.37; negative: 45.66, 44.76, 42.23, 29.80, 27.28, 25.69, 21.97. HR-MS (SIMS), m/e: 257.1894 (100) $[M]^+$($C_{16}H_{23}N_3$ requires 257.1982). IR ($cm^{-1}$): 2912, 2088 and 1247.

***Step 3: Synthesis of compound 4, MDT-43***

A mixture of 0.18 g of 1,6-dimethyl-2-azidodiamantane 3 (0.7 mmol) and 0.05 g of Pd/C in 40 ml of methanol was stirred in a hydrogen atmosphere overnight. The mixture was filtered and the filtrate was concentrate in vacuo to give a crude free base (0.113 g, 70%). The crude product was dissolved in 1% methanolic hydrochloride. After evaporation of the solvents, a white solid was obtained which was washed with ethyl acetate to yield compound 4 (0.09 g, 60%). White solid, m.p.: 242-243°C.

The $^1$H-, $^{13}$C- and Dept-135 NMR, and GC-MS and HRMS (SIMS) spectra of MDT-43 are shown in Figures 94, 95, 96, 97 and 98, respectively. $^1$H-NMR ($CDCl_3$, 300M), δ (ppm): 8.32 (s, 3H), 2.47-2.43 (d, J=12,1H), 2.35-2.31 (d, J=12, 1H), 2.10-1.87 (m, 7H), 1.73-1.21 (m, 10H), 1.09 (s,3H), 1.01 (s, 3H). $^{13}$C-NMR ($CDCl_3$, 75M), δ (ppm), 60.06, 45.96, 45.01, 44.82, 42.36, 40.83, 37.05, 36.33, 36.19, 32.89, 31.84, 28.93, 26.88, 26.00, 25.41, 22.15. Dept-135 ($CDCl_3$, 300M), δ (ppm), positive: 44.99, 44.81, 42.34, 28.92, 26.87, 25.42,22.16; negative: 45.94,40.82,37.04,32.88,31.84,25.99. HRMS (SIMS), m/e: 231.1985 (100) $[M]^+$(C16H25N requires 231.1987). IR ($cm^{-1}$): 3192, 3007, 2912, 2861 and 1518. GC-MS: 91.28 (Area %).

**Example 14:**

**Synthesis of MDT-44 (1,6-dimethyl-2-hydroxydiamantane), MDT-45 (1,6-dimethyl-4-hydroxydiamantane) and MDT-46 (1,6-dimethyl-4-diamantanecarboxylic acid)**

The compounds designated as MDT-44 (1,6-dimethyl-2-hydroxydiamantane), MDT-45 (1,6-dimethyl-4-hydroxydiamantane) and MDT-46 (1,6-dimethyl-4-diamantanecarboxylic acid) were synthesized via the synthetic route depicted in Scheme 14.

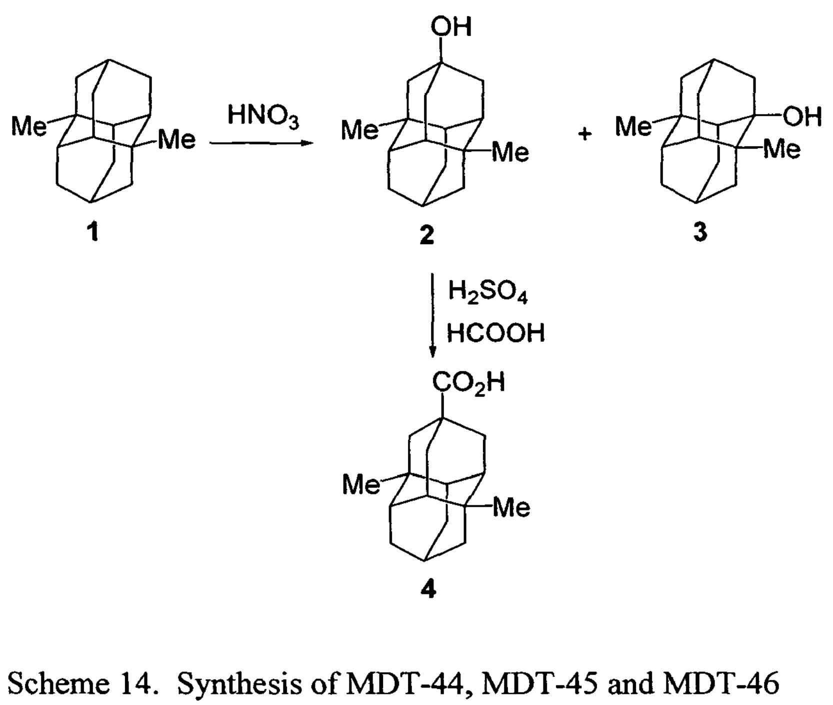


Scheme 14. Synthesis of MDT-44, MDT-45 and MDT-46

***Step 1: Synthesis of MDT-44 and MDT-45***

1,6-Dimethyldiamantane 1 (2.00 g, 9.26 mmol), $CH_2Cl_2$ (16.04 ml) and 98% $HN0_3$ (2.206 ml, 44.12 mmol) were mixed at 0°C and stirred for 6 hrs at 0 °C . The reaction was kept at room temperature for another 10 hrs. The solution was then diluted with water (10 ml), $CH_2Cl_2$ was removed, and the reaction mixture was refluxed for 6 hrs. After the solution was cooled to room temperature, ice-water was added to the solution. The solution was extracted with $CH_2Cl_2$ (3×50mL), dried with $CaCl_2$ and concentrated. The residue was purified by column chromatography to give 1.6 g (76%) of 1,6-dimethyl-4-hydroxydiamantane (2) (MDT-45) and 1,6-dimethyl-2-hydroxydiamantane (3) (MDT-44) (100 mg, 5%).

The $^1$H-, $^{13}$C- and Dept-135 NMR, and MS spectra of MDT-45 are shown in Figures 99, 100, 101 and 102, respectively. Mp:140~145°C. $^1$H-NMR($CDCl_3$, 300 MHz) δ (ppm): 0.93 (s,3H), 0.97 (s,3H), 1.32~1.48 (m, 11H), 1.64 (s, 2H), 1.78~1.80 (s, 1H), 2.03~2.09 (m,4H). $^{13}$C NMR ($CDCl_3$, 75 MHz) δ (ppm): 26.04, 26.16, 27.79, 32.33, 32.94, 36.49, 40.70, 41.64, 45.48, 46.48, 54.50, 68.64. IR (KBr) ν (cm$^{-1}$): 1043.3, 1118.51, 1339.32, 1440.56, 1591.95, 2886.92, 3258.14. MS: m/z (%): 232(9), 217(100), 199(9).

The $^1$H-, $^{13}$C- and Dept-135 NMR, and GC-MS spectra of MDT-44 are shown in Figures 103, 104, 105 and 106, respectively. Mp: 138~142. $^1$H NMR ($CDCl_3$, 300 MHz): δ (ppm) 0.93 (s, 3H), 0.99 (s, 3H), 1.08~1.23 (d, 1H), 1.30~1.40 (m, 8H), 1.58 (s, 1H), 1.76~1.84 (m, 1H), 1.84~1.90 (m, 3H), 1.99~2.06 (m, 4H). $^{13}$C NMR ($CDCl_3$, 75 MHz) δ (ppm): 21.18, 5.3, 26.74, 27.46, 30.67, 32.37, 32.85, 37.27, 38.12, 40.97, 41.47, 42.54, 45.24, 46.94,48.94. IR (KBr) ν (cm$^{-1}$): 737.64, 1028.84, 1442.49, 2906.2, 3458.71.

***Step 2: Synthesis of MDT-46***

1,6-Dimethyl-4-hydroxydiamantane 2 (350 mg, 1.5 mmol) was mixed with HCOOH (2 g, 45.2 mmol) and the solution was stirred for 10 min. Then at 10 °C, 98% $H_2SO_4$ (11.04 g, 6 ml) was added slowly to the reaction mixture. The reaction mixture was stirred at room temperature overnight and was quenched by pouring into 50 ml ice-water. After filtration, the crude cake was dissolved into aqueous NaOH solution. The filtrate was treated with HCl until the pH was 2~3. The resulting precipitate was filtered, washed with water, then dried in vacuum to give 1,6-dimethyl-4-diamantanecarboxylic acid 4 (MDT-46) (260 mg, 66%) as a white solid.

The $^1$H-, $^{13}$C- and Dept-135 NMR, and GC-MS spectra of MDT-46 are shown in Figures 107, 108, 109 and 110, respectively. M.p: 290~295°C. $^1$H -NMR ($CDCl_3$, 300 MHz): δ (ppm) 0.88~0.92 (d, 6H), 1.37~1.52 (m, 12H), 1.77 (s, 1H), 2.01~2.13 (m, 4H), 1.78~1.80 (s, 1H), 12.00 (s,1H). $^{13}$C NMR ($CDCl_3$, 75 MHz) δ (ppm): 25.37, 25.60, 25.68, 27.18, 32.22, 32.51, 32.64, 33.38, 33.84, 34.24, 41.27, 42.17, 46.40, 47.46, 48.00, 178.11. IR (KBr) ν (cm$^{-1}$): 485.009, 943.02, 1295.93, 1441.53, 1691.27, 2866.67.

**Example 15:**

**Synthesis of MDT-47 (4,9-dimethyl-1-hydroxydiamantane)**

The compound designated as MDT-47 (4,9-dimethyl-1-hydroxydiamantane) was synthesized via the synthetic route depicted in Scheme 15.

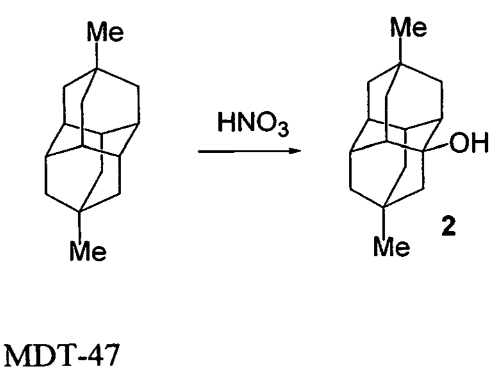


2

Scheme 15. Synthesis of MDT-47

***Synthesis of MDT-47***

To a solution of 4,9-dimethyldiamantane (649 mg, 3 mmol) in $CH_2Cl_2$ (6 ml) was added concentrated $HNO_3$ (0.6 ml) at 0 ˚C . The reaction mixture was stirred for 1 h at 0 ˚C, and then for 24 h at room temperature. The reaction mixture was diluted with 3 ml $H_2O$ then removed $CH_2Cl_2$ in vacuum. Finally, the reaction mixture was refluxed overnight. After the reaction was cooled to room temperature, it was quenched with ice-water, extracted with methylene chloride (4× 10 ml), dried over $Na_2SO_4$ and concentrated. The residue was purified by column chromatography to give 440 mg (63%) of 4,9-dimethyl-1-hydroxydiamantane (MDT-47) as a white solid.

The $^1$H-, $^{13}$C- and Dept-135 NMR, and GC-MS spectra of MDT-47 are shown in Figures 111, 112, 113 and 114, respectively. Mp: 70~71 ˚C. $^1$H NMR ($CDCl_3$, 300 MHz) δ (ppm): 0.8 (m, 3H), 0.85 (m,3H), 1.18~1.23 (d, 2H), 1.39 (s, 9H), 1.57 (m, 3H), 1.87 (m, 4H). $^{13}$C NMR ($CDCl_3$, 75 MHz) δ (ppm): 27.28, 29.60, 29.85, 32.99, 36.55, 39.21, 39.58, 43.25, 44.04, 44.50, 52.72, 70.52. IR (KBr) ν (cm$^{-1}$): 1454.06, 2891.74, 2842.56, 3366.14.

**Example 16:**

**Synthesis of MDT-50 (2-aminotriamantane)**

The compound designated as MDT-5047 (2-aminotriamantane hydrochloride salt) was synthesized via the synthetic route depicted in Scheme 16.

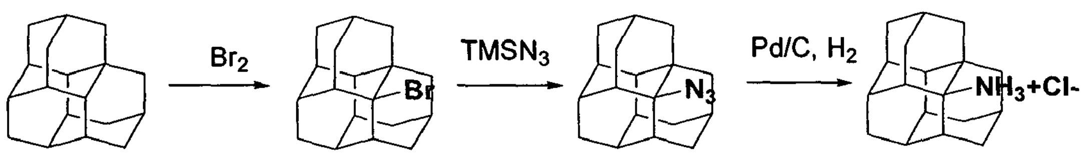


1 2 3 4

Scheme 16. Synthesis of MDT-50

***Step 1: Synthesis of 2-bromotriamantane 2***

To a mixture of 6.0 g (0.025 mol) of triamantane (1) in 10 ml of $CHCl_3$ was added 15 ml (0.3 mol) of neat bromine (distilled) dropwise during 5 min at 0 ˚C. The reaction mixture was stirred for 15 min at 0 ˚C, quenched with $NaHSO_3$ solution, and extracted with 4×20ml of $CHCl_3$. The combined extracts were washed with brine and dried over $Na_2SO_4$. The crude product was purified by column chromatography, yield 30%. (lit, Peter R. Schreiner et al. J. Org. Chem. 2006, 71(18), 6709-6720). $^1$H-NMR (300 MHz, $CDCl_3$), δ (ppm), 2.49-2.48 (m, 2H), 2.42-2.38 (m, 2H), 2.05 (s, 1H), 1.99-1.96 (m, 2H), 1.86-1.67 (m, 1OH), 1.56-1.26 (m, 6H). $^{13}$C-NMR (75 MHz, $CDCl_3$), δ (ppm), 80.23, 54.96, 51.63, 45.80, 44.79, 44.46, 44.05, 41.71, 38.87, 38.05, 37.50, 37.47, 37.19, 35.43, 34.62, 34.37, 32.45, 27.52. Dept-135 ($CDCl_3$, 300M), δ (ppm), positive, 51.62, 44.79, 44.45, 38.04, 37.50, 35.42. Negative, 54.95, 45.78, 44.04, 41.70, 38.04, 34.62, 34.39, 32.45 and 27.52. IR (cm$^{-1}$): 2893, 2851 and 1272.

***Step 2: Synthesis of 2-azidotriamantane 3***

Azidotrimethyl silane (0.35 ml, 3.0 mmol) was added to a solution of 2 (230 mg, 0.72 mmol) in dichloromethane (20ml). After it was cooled to 0 ˚C with ice-bath, tin tetrachloride (0.1 ml, 0.85 mmol) was then added to the reaction mixture slowly. After the disappearance of the starting material, the reaction system was quenched with ice. Dichloromethane (10ml) was added, then the organic layer was separated, washed with saturated sodium chloride, dried over anhydrous $NaSO_4$ and concentrated in vacuo. The residue was purified with chromatography (PE) to yield 3 as white solid (140 mg, 69%). $^1$H-NMR (300 MHz, $CDCl_3$), δ (ppm), 2.11-2.04 (m, 3H), 1.86 (s, 4H), 1.67-1.28 (m, 16H). $^{13}$C-NMR (75 MHz, $CDCl_3$), δ (ppm), 64.45, 49.17, 45.95, 44.50, 44.30, 41.86, 39.25, 38.18, 37.55, 37.41, 36.95, 35.56, 34.40, 33.49, 32.64, 29.79, 27.42. Dept-135 ($CDCl_3$, 300M), δ (ppm), positive, 44.49, 44.29, 41.86, 37.55, 37.41, 36.94, 33.49. Negative, 49.15, 45.93, 39.24, 38.17, 37.39, 34.39, 32.64, 29.78 and 27.41.

***Step 3: Synthesis of 2-aminotriamantane hydrochloride salt (MDT-50)***

10% Pd/C (30 mg) was added to a solution of 3 (100 mg, 0.36 mmol) in methanol (20ml). The suspension was stirred under $H_2$ at room temperature overnight. After the disappearance of starting material, the reaction mixture was filtered through Celite and 20 ml, 1% hydrogen chloride in methanol was added to the filtrate. The solvent was evaporated to yield **4** (MDT-50) as white solid (101 mg, 98%).

The $^1$H-, $^{13}$C- and Dept-135 NMR, and GC-MS spectra of MDT-50 are shown in Figures 115, 116, 117 and 118, respectively. $^1$H-NMR (300 MHz, MeOD), δ (ppm), 1.24-1.33 (m, 2H), 1.66-1.88 (m, 19), 1.92-1.99 (m, 2H), 2.16-2.20 (m, 2H). $^{13}$C-NMR (75 MHz, MeOD), δ (ppm), 28.26, 32.55, 36.52, 38.26, 38.89, 38.93, 39.31, 41.59, 50.38 and 62.75. IR: (KBr, cm$^{-1}$) 3032.5 (s), 2908.1 (vs), 1464.7 (s), 1402 (vs). EI, m/e, 239(100) $[M-NH_2]^+$.

**Example 17:**

**Synthesis of MDT-51 (4,9-dimethyl-1-aminodiamantane)**

The compound designated as MDT-51 (4,9-dimethyl-1-aminodiamantane hydrochloride salt) was synthesized via the synthetic route depicted in Scheme 17.

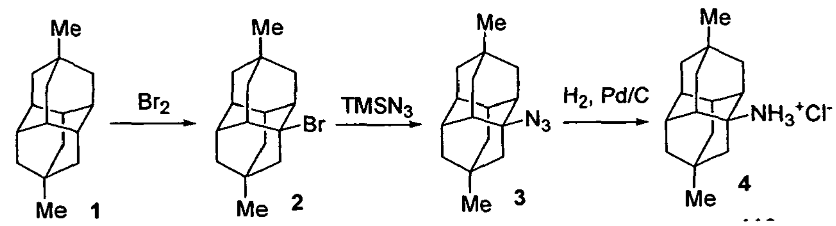

1 2 3 4

Scheme 17. Synthesis of MDT-51

***Step 1: Synthesis of 1-bromo-4,9-dimethyldiamantane 2***

4,9-Dimethyldiamantane **1** (3.0 g, 13.9 mmol) was dissolved in dry chloroform (80 ml) in a flask accommodated in an ice-bath. Bromine (7.15 ml, 139.0 mmol) was added and the mixture was stirred for 55 hrs. The reaction mixture was poured into an ice-$NaHSO_3$ mixture. The organic layer was separated and the aqueous layer was extracted with methylene chloride for 2 x ml. The combined extracts were washed with saturated aqueous $NaHCO_3$ and NaCl, and then dried over anhydrous $Na_2SO_4$. The solvent was evaporated to give the crude product. The crude product was further purified by flash column chromatography using petroleum ether as the eluant to give 1-bromo-4,9-dimethyldiamantane **2** as white solid (2.17 g, 52.9 % yield). $^1$H-NMR (300 MHz, $CDCl_3$, δ, ppm), 0.85 (s, 3H), 0.86 (s, 3H), 1.33 (s, 1H) 1.38 (s, 1H), 1.46-1.53 (m, 6H), 1.65-1.67 (m, 1H), 1.99 (s, 2H), 2.07 (d, J=1.69Hz, 2H), 2.16 (s, 1H), 2.20-2.23 (m, 3H). $^{13}$C-NMR (75 MHz, $CDCl_3$, δ, ppm), 27.50, 29.04, 29.49, 34.09, 36.49, 41.17, 41.37, 43.85, 45.25, 46.16, 57.86.

***Step 2: Synthesis of 1-azido-4,9-dimethyldiamanetane 3***

To a stirring solution of 1-bromo-4,9-dimethyldiamanetane 2 (0.20 g, 0.68 mmol) and trimethylsilyl azide (0.40 ml, 2.04 mmol) in dry dichloromethane (20 ml), tin tetrachloride (0.07 ml, mmol) was added under nitrogen at 0 ˚C. The temperature of the mixture was then raised to room temperature, followed by prolonged stirring for 4 hrs. After the reaction was complete, the mixture was quenched with 30 ml ice-water, followed by dichloromethane extraction (2× 10 ml). The organic layer was dried over anhydrous $MgSO_4$ and evaporated to obtain the crude product. The crude product was further purified on a silica-gel column using petroleum ether as the eluant, to give 1-azido-4,9-dimethyldiamanetane 3 as a white solid (0.10 g, 57.4 % yield). $^1$H-NMR (300 MHz, $CDCl_3$, δ, ppm), 0.81 (s, 3H), 0.89 (s, 3H), 1.21 (s, 1H), 1.26 (s, 2H), 1.43 (s, 6H), 1.57 (s, 2H), 1.63 (s, 1H), 1.68 (s, 2H), 1.80 (s, 1H), 1.84 (s, 1H), 1.89 (s, 2H). $^{13}$C-NMR (75 MHz, $CDCl_3$, δ, ppm), 27.18, 29.44, 29.65, 31.79, 36.51, 38.92, 39.66, 40.28, 43.70, 44.45, 48.08, 63.56. This material was used directly in the next step without further characterization.

**Step 3: Synthesis of 1-ammonium-1,6-dimethyldiamantane chloride 4 (MDT-51)**

A solution of 1-azido-4,9-dimethyldiamanetane **3** (0.11 g, 0.43 mmol) in 1% HCl-MeOH (20 ml) was stirred vigorously with Pd-C (10%, 30 mg) in an $H_2$ atmosphere for 12 hrs. Filtration and evaporation of the solvent in vacuo yielded 1-ammonium-1,6-dimethyldiamantane chloride **4** (MDT-51) as a white solid (0.10 g, 100% yield).
The $^1$H- and $^{13}$C-NMR spectra of MDT-51 are shown in Figures 119 and 120, respectively. $^1$H-NMR (300 MHz, $CDCl_3$, δ, ppm), 0.88, 0.89 (s, s, 6H), 1.32-1.55 (m, 8H), 1.65 (s, 1H), 1.81-1.91 (m, 4H), 2.04-2.15 (m, 4H), 8.33 (s, br, 3H). $^{13}$C-NMR (75 MHz, $CDCl_3$, δ, ppm), 27.31, 29.08, 29.16, 31.05, 36.35, 38.25, 38.65, 39.16, 43.29, 44.79, 47.63, 57.11. GC-MS purity (free base): 96.34%, 231 (m peak), 214, 109 (base peak).

**Example 18:**

**Synthesis of MDT-52 (4,9-dimethyl-1-diamantanecarboxylic acid)**

The compound designated as MDT-52 (4,9-dimethyl-1-diamantanecarboxylic acid) was synthesized via the synthetic route depicted in Scheme 18.

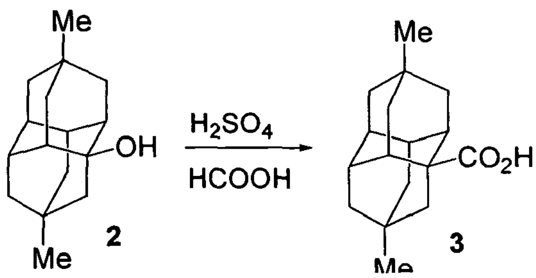


Scheme 18. Synthesis of MDT-52

***Synthesis of MDT-52***

4,9-Dimethyl-l-hydroxydiamantane (116 mg, 0.5 mmol) was mixed with HCOOH (690 mg, 15 mmol), stirred for 1min, and then concentrated $H_2SO_4$ (3.68 g, 2 ml) was added slowly to the reaction system at 10 ˚C. The reaction mixture was stirred at 10 ˚C overnight and then was poured into ice-water (10 ml). After filtration, the crude cake was dissolved in aqueous NaOH solution. After another filtration, the filtrate was treated with HCl until the pH was 2~3. The resulting white precipitate was filtered, washed with water, and dried under vacuum to give the product MDT-52 (102 mg, yield 78%, white solid). Mp: 172~174˚C.
The $^1$H-, $^{13}$C- and Dept-135 NMR, and GC-MS spectra of MDT-52 are shown in Figures 121, 122, 123 and 124, respectively. $^1$H NMR ($CDCl_3$, 300 MHz) δ (ppm): 0.73 (m, 3H), 0.79 (m, 3H), 1.24~1.28 (d, 2H), 1.38~1.47 (m, 8H), 1.51 (s, 2H), 1.59 (s, 1H), 1.72 (m, 2H), 1.96 (m, 2H), 11.98 (s, 1H). $^{13}$C NMR ($CDCl_3$, 75 MHz) δ (ppm): 26.84,28.07,29.57, 29.97, 36.06, 36.84, 36.94, 41.43, 43.60, 43.98, 45.06, 47.57. IR (KBr) ν ($cm^{-1}$): 2943.8, 2914.88, 1693.19.

**Example 19:**

**Synthesis of MDT-53 (4,9-dimethyl-1,6-diaminodiamantane)**

The compound designated as MDT-53 (4,9-dimethyl-1,6-diaminodiamantane dihydrochloride salt) was synthesized via the synthetic route depicted in Scheme 19.

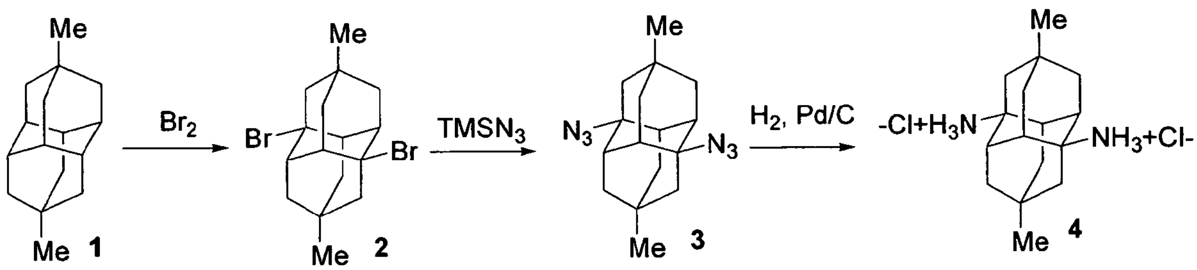


1 2 3 4

Scheme 19. Synthesis of MDT-53

***Step 1: Synthesis of 1,6-dibromo-4,9-dimethyldiamantane 2***

4,9-Dimethyldiamantane **1** (3.0 g, 13.9 mmol) was dissolved in dry chloroform (80 ml) in a flask accommodated in an ice-bath. Bromine (7.15 ml, 139.0 mmol) was added and the mixture was refluxed for 12 h. The reaction mixture was cooled to room temperature and poured into an ice-$NaHSO_3$ mixture. The organic layer was separated and the aqueous layer was extracted with methylene chloride for 2 times. The combined extracts were washed with saturated aqueous $NaHCO_3$ and NaCl, and then dried over anhydrous $Na_2SO_4$. The solvent was then evaporated to give the crude product. The crude product was further purified by flash column chromatography using petroleum ether as the eluant to give 1,6-dibromo-4,9-dimethyldiamantane **2** as a white solid (1.57 g, 30.2 % yield). $^1H$ NMR (300 MHz, $CDCl_3$, $\delta$, ppm) 0.92 (s, 6H), 1.47 (d, J=12.21 Hz, 4H), 2.26 (d, J=10.66 Hz, 12H). $^{13}C$NMR (75 MHz, $CDCl_3$, $\delta$, ppm) 27.45, 33.40, 40.56, 48.54, 58.23, 73.96.

***Step 2: Synthesis of 1,6-diazido-4,9-dimethyldiamantane 3***

1,6-Dibromo-4,9-dimethyldiamantane **2** (150 mg, 0.4 mmol) was dissolved into dried dichloromethane (50 ml) in a well-dried two-necked flask fitted with $Ar_2$ balloon. Trimethylsilyl azide (0.45 ml, 3.39 mmol) was injected slowly into the reaction mixture. After stirring for 10 minutes, 0.15 ml (1.28 mmol) of $SnCl_4$ was added. Three hours later, the reaction mixture was poured into crushed ice and was extracted with dichloromethane twice. The organic layer was dried over $MgSO_4$ (anhydrous) and evaporated to give 1,6-diazido-4,9-dimethyldiamantane **3** as white solid (110 mg, yield 92.13%). M.P. 85~88 ˚C. IR (KBr; $cm^{-1\ 1}$): 2920.66, 2357.55, 2087.52, 1458.10, 1258.64. $^1H$-NMR ($CDCl_3$, 300 MHZ) (ppm): 0.92 (s, 6H), 3.54 (s, 6H), 1.59 (s, 4H), 1.85 (m, 6H). $^{13}C$-NMR ($CDCl_3$, 75MHZ): $\delta$ (ppm) 28.9 (2C, 2-$CH_3$), 31.2 (2C; C-4, C-9), 38.5 (4C; C-3, C-8, C-10, C-14), 41.4 (4C; C-2, C-7, C-11, C-12), 47.69 (2C; C-5, C-13), 62.30 (2C; C-1, C-6), 47.69 (2C; C-5, C-13), 62.30 (2C; C-1, C-6). HRMS (TOF-MS): m/z 298.1909 $[M]^+$, (C16H22N6 requires 298.1906).

***Step 3: Synthesis of 1,6-diamino-4,9-dimethyldiamantane dihydrochloride 4 (MDT-53)***

Compound **3** (100 mg, 0.33 mmol) was added into 20 ml of MeOH. 11.2 mg of Pd-C was also added into the reaction system, and the reaction mixture was stirred in an atmosphere of hydrogen. The reaction was monitored by TLC. After 28 hours, the reaction was completed. The reaction mixture was filtered, the filtrate was concentrated, and then 1 M hydrogen chloride acid in ether was added. 1,6-Diamino-4,9-dimethyldiamantane dihydrochloride **4** was obtained as a white solid (92.3 mg, yield: 86.2%).

The $^1H$-, $^{13}C$- and Dept-135 NMR, and GC-MS spectra of MDT-53 are shown in Figures 125, 126, 127 and 128, respectively. M.P. 258~260 ˚C. IR (KBr; $cm^{-1}$): 3434.6, 3018.05, 2913.91, 1392.35. $^1H$-NMR ($D_2O$, 300 MHz) (ppm): 0.85 (s, 6H), 1.42 (d, 4H), 1.56 (S, 4H), 1.70 (d,4H), 2.01 (s,4H). $^{13}C$-NMR($D_2O$,75 MHz) $\delta$ (ppm): 27.46 (2C; - $CH_3$), 29.13 (2C; C-4, C-9), 36.21 (4C; C-3, C-5, C-10, C-13), 39.84 (2C; C-2, C-11), 54.94 (2C; C-1, C-6), 46.90 (2C; C-8, C-14). GCMS: 246 m/z.

**Example 20:**

**Synthesis of diamantane rimantadine analogs**

Diamantane rimantadine analogs can be synthesized according to the synthetic routes depicted in Scheme 20.

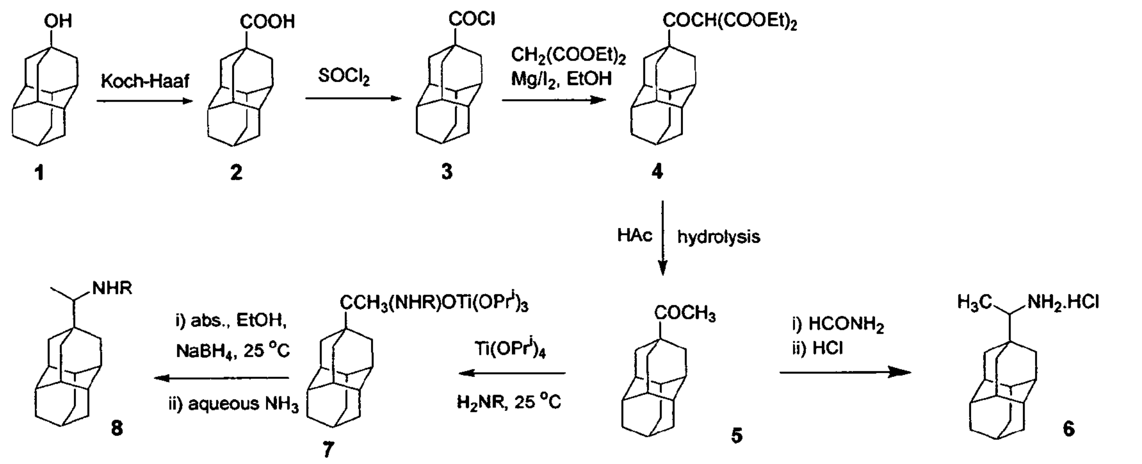


Route 2. one pot 2 step reaction with a total yield of about 80-93%.

Route 1. 6 step reaction with a total yield of about 40%.

Scheme 15. Synthesis of diamantane rimantadine analogs

Two synthetic routes are presented for the preparation of diamantane rimantadine analogs. Both routes have the key intermediate acetyldiamantane, for example, intermediate 4-acetyldiamantane **5.**

***Synthesis of Compound*** **3:**

A 100 ml round-bottom flask is equipped with a stir bar and charged with 0.01 mol 2 and 36 ml $SOCl_2$ (0.5 mol). A condenser and $N_2$ bubbler are attached and the mixture is heated to 80 ˚C for 1 h. The reaction is cooled to room temperature and filtered on a medium porosity glass frit. Removal of excess $SOCl_2$ (room temperature, 10 torr) and extraction of the residual with benzene gives the acid chloride, which may be used directly for the next reaction step without further purification.

***Synthesis of Compound 4:***

A 100 ml round-bottom flask is equipped with a stir bar and a condenser, and is charged with $N_2$, Mg powder (2.4 g; 0.1 mol), $I_2$ (1.2 g), anhydrous ethanol (1 ml), and benzene (11 ml). The mixture is stirred and slowly heated during dropwise addition of a mixture of benzene (30 ml), $CH_2(COOCH_2CH_3)_2$ (24 g, 0.015 mol) and anhydrous ethanol (8 ml) for about 30 minutes until the mixture solution becomes clear. The clear solution is cooled and then solution of 3 in benzene is added dropwise. After addition, the mixture is heated to reflux for about 2 h, then poured onto ice water. The organic layer is separated and the aqueous phase is extracted with benzene (3x50 ml). The combined organic phases are washed with water to neutral and dried. Evaporation of the solvent under reduced pressure gives a crude product of **4**, which may be used directly for the next step reaction without further purification.

***Synthesis of Compound 5:***

To the above crude product is added glacial acetic acid (50 ml), water (30 ml), and concentrated $H_2SO_4$ (3 ml). The mixture is heated to reflux until no gas comes out, then is cooled and poured onto ice water (300 g). A light yellow solid is obtained by filtration.

***Synthesis of Compound 6:***

A round-bottom flask is charged with 0.04 mol of 5 and $HCONH_2$ (40 g, 0.9 mol). The mixture is heated to 160-180 ˚C for about 3 h. After cooling, the mixture is poured onto water (2x vol.) and allowed to stand for a while. The organic layer is separated and the aqueous layer is extracted with benzene (4x50 ml). The organic phases are combined. After evaporation of the solvent (benzene), to the residual is added concentrated HCl (50 ml) followed by heating to 105 ˚C to reflux for about 3 h. After cooling to room temperature, a white solid is precipitated out. The white solid is collected

by filtration and purified by recrystallization in ethyl acetate.

***Synthesis of Compound 8 from Compound 5:***

A mixture of 4-acetyldiamantane (10mmol), titanium (IV) isopropoxide (5.7 g, 20 mmol) and the starting amine ($H_2NR$)(20mmol) is stirred at 25 °C for 5-6 hours. Sodium borohydride (0.76 g, 20 mmol) and absolute ethanol (15 ml) are added to the mixture which is then stirred for a further 12-13 hours at 25 °C. The reaction is quenched with aqueous ammonia (2 M, 30ml) and the resulting inorganic precipitate is filtered off and washed with diethyl ether (50ml); the aqueous solution is extracted with diethyl ether (2x50 ml). The combined organic washings and extracts are next extracted with hydrochloric acid (1 M, 2x10 ml) to separate the neutral materials. The acidic aqueous solution is made alkaline (pH 10) by slow addition of aqueous NaOH (10% w/v) and extracted with dichloromethane (2x50 ml). The combined extracts are dried ($K_2CO_3$) and concentrated under reduced pressure to give pure 4-diamantylethylamines.

***Synthesis of Compound 8 from Compound 5 via Compound 7:***

For preparations involving amine salts, the same general procedure is used except that a mixture of 4-acetyldiamantane (10 mmol), ammonium chloride or methylamine hydrochloride (20 mmol), triethylamine (25 mmol) and titanium (IV) isopropoxide (5.7 g, 20 mmol) in absolute ethanol (15ml) is stirred at room temperature for 12 h to form the intermediate adduct. Sodium borohydride (0.76 g, 20 mmol) is added to the mixture which is then further stirred for 12 h with isolation of the products as described above.

**Example 21**:

**Synthesis of MDT-69 (mixture of methylated triamantane apical amines)**

The compounds designated as MDT-69 (mixture of methylated triamantane apical amines) were synthesized via the synthetic route depicted in Scheme 20.

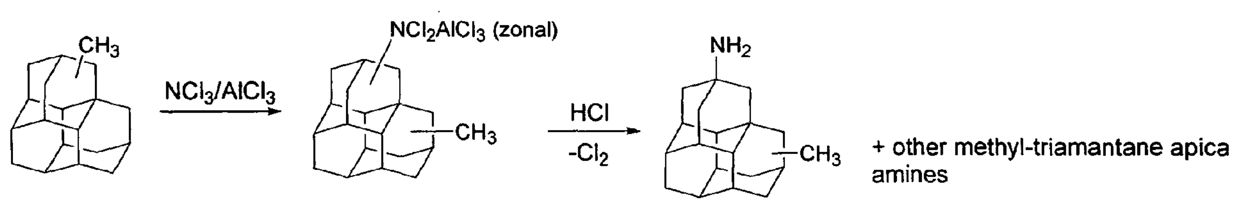


1

2

Scheme 20. Synthesis of MDT-69

***Step 1: Synthesis of component 1***

Compound 1 was prepared by high resolution distillation, followed by hydroprocessing, purification by different solvent extraction, and finally decolorization with activated carbon and silica gel column chromatography. The GC-MS spectrum of the methylated triamantane mixture is shown in Figure 129.

***Step 2: Synthesis of $NCl_3$***

$Ca(OCl)_2$ (10 g; 0.07 mol) was charged into 20 ml water (HPLC grade) in a 250 ml three-neck round bottom flask with condenser, addition funnel, thermometer and magnetic stirring bar. The Husk was cooled to ice bath temperature (4 °C) and 30 ml $CH_2Cl_2$ was added. A solution of 2.2 g (0.04 mol) of $NH_4Cl$ in 5 ml of concentrated HCl and 15 ml of HPLC grade water was slowly added to the flask over 30 min. The temperature kept at 40 °F throughout the reaction. A bright yellow color appeared. When the addition was completed, the reaction mixture was stirred an additional 15 min., and then poured into a 125 ml separatory funnel. The bottom bright yellow organic phase was removed, washed with HPLC water, and dried over anhydrous $Na_2SO_4$ for 1hr. The drying agent was filtered off, and 20 ml of $CH_2Cl_2$ was added.

In order to standardize the concentration of $NCl_3$, a 1 ml aliquot of the $NCl_3$ solution was added to 2 g ofNaI in 50 ml of 80% acetic acid. This solution will be violet after all the $I_2$ has been liberated from the reaction. 5.0 ml of this solution was titrated with 0.1 N $Na_2S_2O_3$ (sodium thiosulfate) solution to reduce the $I_2$ to $I^-$. Typically, two titrations were performed. The final concentration of the $NCl_3$ was determined to be about 2 M.

*Step 3: Synthesis of apical amines from $NCl_3$-$AlCl_3$ catalytic reaction*

The precursor-methylated triamantane mixture was Rotavaped before reaction under vacuum at 60˚C for 10hrs in order to get rid of moisture. Methylated triamantane mixture (3 g; 11 mmol) was dissolved in 50 ml of dry 1,2-dichloroethane and charged into a three-neck 250 ml round-bottom flask equipped with condenser, pre-cooled addition funnel, $N_2$ purge line/thermometer adapter and magnetic stirring bar. All were placed inside a round-bottom Dewar cold bath containing o-xylene/dry ice (-29 ˚C) inside a hood.

Anhydrous $AlCl_3$ (2.5 g; 19 mmol) was added under nitrogen. $CH_2Cl_2$ (50 ml) and $NCl_3$ (26.5 ml, 53 mmol) were added from the addition funnel slowly over 2 hrs. The temperature was elevated to 0 ˚C (ice bath) for 1hr.

The reaction was quenched with 150 ml 18% HCl water solution with fast $N_2$ purge to remove the $Cl_2$ generated in the quenching process. A white solution formed and was allowed to stand for 40 min at room temperature. The aqueous layer was collected and washed with 150 ml $CH_2Cl_2$ and 150 ml ether once. At this stage, foam formed during separation. Generally, the solution must stand for a longer time until good separation occurs.

The aqueous solution was treated with ammonium hydroxide to pH 10, then extracted with 100 ml $CH_2Cl_2$ three times. All the organic phases were combined and dried with $Na_2SO_4$ for 0.5 hr. The solvent was removed under rotavap, and a clear oil was obtained. An HCl 1 M ether solution was added to the clear oil to precipitate a white powder. The white powder was filtered, washed with acetone and dried at room temperature. Yield: 0.21 g (HCl salt), fine white powder. Figure 130 shows the GC-MS spectrum of the aminated methylated triamantane mixture (free base) in $CH_2Cl_2$. Figure 131 shows the GC-MS of the aminated methylated triamantane mixture (HCl salt) in ethanol (after HCl salt formation, the mixture was neutralized with $NH_4OH$, dried then dissolved into ethanol).

**Example 22:**

**Binding analysis of diamondoid compounds**

***NMDA receptors (NMDARS)***

NMDARs are one of three subtypes of glutamate receptors, along with kainite and quisqulate receptors. The NMDAR appears to be unique in that activation is dependent upon simultaneous activation with glutamate and glycine, or perhaps D-serine (Dingledine et al., 1990, Mothet et al., 2000). These receptors are ligand-gated ion channels that have an important role in the regulation of synaptic function in the CNS. This regulatory role originates from their high permeability to $Ca^{2+}$ ions upon receptor activation. Dysregulation of NMDAR-mediated calcium ion influx is implicated in many brain disorders, such as stroke, epilepsy, Huntington disease, Alzheimer disease and AIDS related dementia. In each of these diseases the common feature is the neuronal injury caused by the overstimulation of the glutamate receptors, especially of the NMDA subtype. NMDAR antagonists could therefore be of therapeutic use in several neurological disorders. Only those compounds that block the excessive activation of the NMDAR while leaving the normal function intact are useful in the clinic, as they will not cause unwanted side effects. For this reason, a non-competitive open-channel blocker would be an effective approach to maintain the normal physiological activity of the brain even in a diseased state.

A high affinity, selective PCP analog [$^3$H]MK-801 binds to an allosteric site on the NMDA receptor (Lodge and Anis 1982). Because of its high affinity, MK-801 has been widely used for binding studies in search for additional NMDAR antagonists.

***NMDA receptors in guinea pig brain***

Hartley guinea pigs were sacrificed, and their brains were quickly removed and weighed. The brains then were homogenized in 50 mM Tris HCl buffer, pH 7.7, using a Polytron homogenizer. The homogenate was centrifuged at 40,000 x *g* for 15 min, rehomogenized, and centrifuged again. The final pellet was resuspended in Tris-HCl, pH 7.7, at a final concentration of 6.67 mg original wet weight of tissue/ml. The radioligand used for the binding assay was [$^3$H] MK-801 (1 nM). The guinea pig brain membrane suspension (0.8 ml) was incubated in 5 mM Tris-HCl, pH 7.7, for 1 h at 25˚C with 100 $\mu$l of radioligand and 100 $\mu$l of test compound at concentrations ranging from $10^{-3}$ to $10^{-8}$ M. Nonspecific binding was determined by incubation in the presence of 1 $\mu$M of the "cold" unlabeled MK-801. The samples were then filtered through glass fiber filters on a Tomtec cell harvester. The filters were washed 3 times with 3 ml of cold buffer. Filters were dried overnight and counted next day on a Wallac Betaplate Reader.

The binding experiments were conducted as follows. Competition curves with standard and test compounds included at least six concentrations, with at least four concentrations yielding greater than 20% but less than 80% inhibition. For each compound, graphs were prepared containing individual competition curves obtained for that compound. $IC_{50}$ values and Hill coefficients were calculated using the program Prism. $K_i$ values were calculated using the

Chang Prusoff transformation:

$$K_i = IC_{50}/(1 + L/K_d)$$

where L is radioligand concentration and $K_d$ is the binding affinity of the radioligand, as determined previously by saturation analysis. Experiments for those compounds were repeated if it was found to have an $IC_{50}$ value of less than 100 μM. In each experiment, one standard compound was simultaneously run on each 96 well plate. If the standard compound did not have an $IC_{50}$ value close to the established average for that compound (maximum 3-fold difference), the entire experiment was discarded.

***Results***

To establish this assay, a saturation experiment was conducted on guinea pig brain membranes, which provided a good correlation for the values obtained previously for rat brain membranes (see Table 26). Even the affinity of the standard MK-801 was very close in both systems (2.84 nM in rats and 1.45 nM in the guinea pig).

**Table 26**

| Result of saturation experiments on rat and guinea pig brain homogenates for [$^3$H]MK-801 | | |
|---|---|---|
| **Species** | **$K_d$ (nM)** | **$B_{max}$ (fmol/mg)** |
| Rat | 2.11 | 8655 |
| Guinea pig | 2.19 | 11730 |

Once this information was pbtained we could proceed to test the other standards selected for these assays. The results are listed in Table 27. All of the standard compounds showed low affinity for the NMDARs, except MK-801. Each of these values correlates well with the binding affinities reported in the literature.

**Table 27**

| $K_i$ values for selected standard compounds at the NMDA site | | |
|---|---|---|
| **Standard** | **$K_i$(nM)** | **Hill Slope** |
| MK-801 | 4.17 ± 0.23 | 0.99 ± 0.07 (n=3) |
| Memantine | 733 ± 130 | 1.00 ± 0.05 (n=3) |
| Amantadine | 16,1437 ± 3,454 | 1.20 ± 0.45 (n=2) |
| NMDA | 595,785 ± 82,446 | 0.80 ± 0.04 (n=2) |

Following assay establishment, the test compounds were tested for binding affinities. The concentrations selected for the experiments were chosen according to what was found for memantine in our assay. The first task was to dissolve the compounds and make up the 10 mM stock solution for further experiments. Those compounds that were made into salt format were easy to dissolve in deionized water, but the other compounds were difficult to bring into solution. Several "assay friendly" solvents have been tried, such as molecusol, acetic acid and propylene glycol followed by putting the vial into hot water. Most compounds went into solution using this approach. Others, however, (i.e., MDT-17, MDT-19, and MDT-20) did not go into solution, or came out with time and the binding data are not reported.

Table 28 sets forth the various diamondoid compounds tested, and Table 29 lists the results of the binding experiments.

Table 28

| **Identifier** | **Compound** | **Form** |
|---|---|---|
| MDT-1 | 1-aminodiamantane | hydrochloride salt |
| MDT-2 | 1-aminodiamantane | hydrochloride salt |

(continued)

| Identifier | Compound | Form |
|---|---|---|
| MDT-3 | 4-aminodiamantane | hydrochloride salt |
| MDT-4 | 1,6-diaminodiamantane | hydrochloride salt |
| MDT-5 | 4,9-diaminodiamantane | hydrochloride salt |
| MDT-6 | 1,6-dimethyl-2-aminodiamantane mixture | free amine |
| MDT-7 | 1,6-dimethyl-4-aminodiamantane mixture | hydrochloride salt |
| MDT-9 | Mixture of 1-methyl-2,4- diaminodiamantane and 1,6-dimethyl-2,4-diaminodiamantane | hydrochloride salt |
| MDT-10 | 1-hydroxydiamantane | not ionizable |
| MDT-11 | 4-hydroxydiamantane | not ionizable |
| MDT-12 | 1,6-dihydroxydiamantane | not ionizable |
| MDT-13 | 1,7-dihydroxydiamantane | not ionizable |
| MDT-14 | 4,9-dihydroxydiamantane | not ionizable |
| MDT-15 | 9,15-dihydroxytriamantane | not ionizable |
| MDT-16 | Trihydroxydiamantane mixture | not ionizable |
| MDT-17 | 1-diamantanecarboxylic acid | free acid |
| MDT-19 | 1,6-diamantanedicarboxylic acid | free acid |
| MDT-20 | 4,9-diamantanedicarboxylic acid | free acid |
| MDT-21 | HPLC-purified fraction of MDT-7 | hydrochloride salt |
| MDT-22 | 2-methyl-4-aminodiamantane | hydrochloride salt |
| MDT-23 | 1,6-dimethyl-4-aminodiamantane | hydrochloride salt |
| MDT-24 | 1-methyl-4-aminodiamantane | hydrochloride salt |
| MDT-26 | 1-nitrosodiamantane | not ionizable |
| MDT-27 | 4-nitrosodiamanane | not ionizable |
| MDT-28 | 1-methyl-4,9-diaminodiamantane | hydrochloride salt |
| MDT-29 | 1-methyl-4,6-diaminodiamantane | hydrochloride salt |
| MDT-30 | 1-amino-12-methyldiamantane | hydrochloride salt |
| MDT-31 | Mixture of 1-methyl-2- aminodiamantane and 1-methyl-6-aminodiamantane | hydrochloride salt |
| MDT-32 | Mixture of 1-amino-4- methyldiamantane and 2-amino-4-methyldiamantane | hydrochloride salt |
| MDT-33 | 1,6-dimethyl-4-aminodiamantane | hydrochloride salt |
| MDT-34 | Mixture of 4-methyl-9- aminodiamantane and 4-aminodiamantane | hydrochloride salt |
| MDT-38 | sodium 1-diamantanecarboxylate | sodium salt |
| MDT-39 | sodium 1,6- diamantanedicarboxylate | sodium salt |
| MDT-40 | 2-hydroxytriamantane | not ionizable |
| MDT-41 | 3-hydroxytriamantane | not ionizable |
| MDT-42 | 9-hydroxytriamantane | not ionizable |
| MDT-43 | 1,6-dimethyl-2-aminodiamantane | hydrochloride salt |
| MDT-44 | 1,6-dimethyl-2-hydroxydiamantane | not ionizable |
| MDT-45 | 1,6-dimethyl-4-hydroxydiamantane | not ionizable |

(continued)

| Identifier | Compound | Form |
|---|---|---|
| MDT-46 | 1,6-dimethyl-4- diamantanecarboxylic acid | sodium salt |
| MDT-47 | 4,9-dimethyl-1-hydroxydiamantane | not ionizable |
| MDT-48 | 3-aminotriamantane | hydrochloride salt |
| MDT-49 | 9-aminotriamantane | hydrochloride salt |
| MDT-50 | 2-aminotriamantane | hydrochloride salt |
| MDT-51 | 4,9-dimethyl-1-aminodiamantane | hydrochloride salt |
| MDT-52 | 4,9-dimethyl-1- diamantanecarboxylic acid | sodium salt |
| MDT-53 | 4,9-dimethyl-1,6- diaminodiamantane | hydrochloride salt |
| MDT-56 | 1-diamantanemethylamine | hydrochloride salt |
| MDT-57 | 1-(1-aminoethyl)diamantane | hydrochloride salt |
| MDT-58 | hydroxy-3-diamantanone mixture | not ionizable |
| MDT-59 | 3-diamantanone | not ionizable |
| MDT-60 | 4-methyldiamantane | not ionizable |
| MDT-61 | 4-diamantanemethylamine | hydrochloride salt |
| MDT-62 | 4-(4-aminoethyl)diamantane | hydrochloride salt |
| MDT-63 | 4,9-dimethyl-1- diamantanemethyleneamine | hydrochloride salt |
| MDT-65 | 1,6-dimethyl-4- diamantanemethyleneamine | hydrochloride salt |
| MDT-67 | 4,9-dimethyl-1-diamantane-1'- methyl-methyleneamine | hydrochloride salt |
| MDT-68 | 1,6-dimethyl-4-diamantane-4'- methyl-methyleneamine | hydrochloride salt |
| MDT-69 | methylated triamantane apical- amine mixture | hydrochloride salt |

**Table 29**

| $K_i$ values and Hill coefficients for diamondoid compounds at the NMDA receptor in guinea pig brain membrane preparation | | | |
|---|---|---|---|
| **Compound** | **$K_i$ ($\mu$M)** | **Hill Slope** | **Relative affinity*** |
| Memantine | 0.73 ± 0.013 | 1.00 ± 0.05 | 1 |
| MDT-1 | 7.5 ± 2.4 | 1.28 ± 0.10 | 0.097 |
| MDT-2 | 6.4 ± 0.13 | 1.21 ± 0.00 | 0.114 |
| MDT-3 | 34.6 ± 3.7 | 1.55 ± 0.10 | 0.021 |
| MDT-4 | 99.6 ± 7.6 | 0.65 ± 0.06 | 0.007 |
| MDT-5 | 268 ± 19 | 1.05 ± 0.30 | 0.003 |
| MDT-6 | 4.6 ± 0.18 | 1.30 ± 0.07 | 0.159 |
| MDT-7 | 9.9 ± 0.45 | 2.05 ± 0.34 | 0.074 |
| MDT-9 | 22.5 ± 0.90 | 0.87 ± 0.14 | 0.032 |
| MDT-10 | > 1000 | --- | --- |
| MDT-11 | >1000 | --- | --- |
| MDT-13 | >1000 | --- | --- |
| MDT-14 | >1000 | --- | --- |

(continued)

| Compound | $K_i$ (μM) | Hill Slope | Relative affinity* |
|---|---|---|---|
| MDT-15 | >1000 | --- | --- |
| MDT-16 | >1000 | --- | --- |
| MDT-21 | 9.6 ± 0.80 | 0.97 ± 0.16 | 0.076 |
| MDT-22 (prep from Example 4) | 2.2 ± 0.90 | 0.47 ± 0.03 | 0.332 |
| MDT-22 (prep from Example 7) | 10.5 ± 1.0 | 1.18 ± 0.72 | 0.070 |
| MDT-23 (prep from Example 4) | 3.9 ± 0.07 | 0.83 ± 0.00 | 0.187 |
| MDT-23 (prep from Example 5) | 3.0 ± 0.90 | 0.97 ± 0.05 | 0.243 |
| MDT-24 | 13.4 ± 5.1 | 1.47 ± 0.38 | 0.045 |
| MDT-26 | > 1000 | --- | --- |
| MDT-27 | > 1000 | --- | --- |
| MDT-28 | >1000 | --- | --- |
| MDT-29 | 41.0 ± 10.7 | 0.67 ± 0.15 | 0.018 |
| MDT-30 | 2.9 ± 0.7 | 0.93 ± 0.11 | 0.252 |
| MDT-31 | 3.9 ± 0.2 | 0.93 ± 0.02 | 0.187 |
| MDT-32 | 11.4 ± 3.7 | 1.42 ± 0.31 | 0.064 |
| MDT-33 | 7.8 ± 0.9 | 1.13 ± 0.09 | 0.094 |
| MDT-34 | >1000 | --- | --- |
| MDT-38 | >1000 | --- | --- |
| MDT-39 | >1000 | --- | --- |
| N4DT-40 | >1000 | --- | --- |
| MDT-41 | >1000 | --- | --- |
| MDT-42 | >1000 | --- | --- |
| MDT-43 | 5.1 ± 0.7 | 0.92 ± 0.1 | 0.143 |
| MDT-44 | > 1000 | --- | --- |
| MDT-45 | 98.6 ± 0.1 | --- | 0.007 |
| MDT-46 | > 1000 | --- | --- |
| NmT-47 | > 1000 | --- | --- |
| MDT-48 | 14.0 ± 3.5 | 1.27 ± 0.20 | 0.052 |
| MDT-49 | 389 ± 92 | --- | 0.002 |
| MDT-50 | 19.1 ± 1.7 | 1.07 ± 0.06 | 0.038 |
| MDT-51 | 4.7 ± 2.4 | 0.78 ± 0.17 | 0.155 |
| MDT-52 | >1000 | --- | --- |
| MDT-53 | 112 ± 14 | 1.12 ± 0.03 | 0.007 |

$K_i$ values and Hill coefficients for diamondoid compounds at the NMDA receptor in guinea pig brain membrane preparation

(continued)

| Compound | $K_i$ (μM) | Hill Slope | Relative affinity* |
|---|---|---|---|
| MDT-56 | 9.8 ± 4.5 | 0.51 ± 0.09 | 0.074 |
| MDT-57 | 4.0 ± 0.1 | 0.87 ± 0.07 | 0.183 |
| MDT-58 | >1000 | --- | --- |
| MDT-59 | >1000 | --- | --- |
| MDT-60 | >1000 | --- | --- |
| MDT-61 | 8.0 ± 0.9 | 1.50 ± 0.40 | 0.091 |
| MDT-62 | 8.6 ± 2.6 | 1.27 ± 0.06 | 0.085 |
| MDT-63 | 5.1 ± 0.3 | 1.18 ± 0.01 | 0.143 |
| MDT-65 | 7.3 ± 1.1 | 1.11 ± 0.11 | 0.100 |
| MDT-67 | 1.1 ± 0.1 | 1.18 ± 0.20 | 0.664 |
| MDT-68 | 2.5 ± 0.1 | 1.02 ± 0.12 | 0.292 |
| MDT-69 | 26.0 ± 11.2 | 1.45 ± 0.13 | 0.028 |

$K_i$ values and Hill coefficients for diamondoid compounds at the NMDA receptor in guinea pig brain membrane preparation

* Relative affinity was compared to that of Memantine.

About half of the compounds that could be solubilized inhibited binding of [$^3$H]MK-801 to the NMDA receptor to some extent. MDT-67 had the highest affinity, 1.1 μM, which is very close to that of the clinically useful drug memantine, 0.73 μM. Seven other MDT compounds had a binding affinity ≤ 5 μM (MDT-23, MDT-30, MDT-43, MDT-51, MDT-57, MDT-63 and MDT-68) and another six had $K_i$ values in the range of 5 to 10 μM (MDT-1, MDT-22, MDT-56, MDT-61, MDT-62 and MDT-65). Although not quite as potent as these amino-diamantane derivatives, two of the three amino-traimantanes tested had moderate affinities (2-amino-triamantane, MDT-50, $K_i$=19.1 μM; and 3-amino-triamantane, MDT-48, $K_i$=14.0 μM), while the third had very little affinity (9-amino-triamantane, MDT-49, $K_i$=389 μM). These results indicate that a variety of diamondoid compounds may act as neuroprotectants.

**References**

Dingledine, R., N. W. Kleckner, and C. J. McBain. 1990. The glycine coagonist site of the NMDA receptor. Adv. Exp. Med. Biol. 268: 17-26.

Lodge D., and N.A. Anis. 1982. Effects of phencyclidine on excitatory amino acid activation of spinal interneurones in the cat. Eur. J. Pharmacol. 77:203-204.

Mothet, J. P., A. T. Parent, H. Wolosker, R. O. Brady, D. J. Linden, C. D. Ferris, M. A. Rogawski, and S. H. Snyder. 2000. D-serine is an endogenous ligand for the glycine site of the N-methyl-D-aspartate receptor. Proc. Natl. Acad. Sci. USA 97:4926-4931.

**Example 23:**

**Diamondoid compound modulation of NMDA-induced currents in mammalian cells using whole-cell voltage-clamp recordings**

Cognitive disability characterizes the most common neurodegenerative diseases, i.e., Alzheimer's (AD), Huntington's, and Parkinson's [1-5], and also is a prominent component of neuropsychiatric disorders such as schizophrenia, depression, anxiety, and chronic sleep disorders. Current medications are relatively ineffective in improving cognition [1, 6]. Moreover, most therapeutics are not disease modifying. Neuroprotective drugs tested in clinical trials, particularly

those that block N-methyl-D-aspartate-sensitive glutamate receptors (NMDARs), have failed at least in part due to intolerable side effects. However, memantine was recently approved by the European Union and the US FDA for the treatment of dementia following the discovery of its clinically tolerated mechanism of action. The mechanism of action of memantine has been shown to preferentially block excessive NMDA receptor activity without disrupting normal activity [7].

The chemical structure of memantine is a low molecule of diamondoids. The present application describes additional diamondoid compounds for treatment of neurological disorders. At least some of these molecules are capable of modulating NMDA receptor-induced currents and could be potentially neuroprotective through the modulation of NMDA receptor-mediated activity. The experiments below describe standard whole-cell-voltage-clamp recordings from mouse hypocretin (Hcrt) neurons, cells that are lost likely via excitotoxicity in narcoleptics, found in hypothalamic brain slices to investigate the effects of diamondoid compounds and compare with MK-801 and memantine.

**Methods**

***Slice preparation***

We prepared sections of the hypothalamus from day 21-26 Hcrt-EGFP (enhanced green fluorescent protein) mice as described previously [8]. Male and female Hcrt/EGFP mice, in which the human prepro-orexin promoter drives expression of EGFP were used for experiments. In brief, mice were anesthetized with isoflurane before decapitation. A block of tissue containing the hypothalamus was dissected and then sliced in the coronal plane (250 $\mu$m) using a vibratome (VT-1000S, Leica Instruments) in ice-cold sucrose solution (containing in mM: 220 sucrose, 2.5 KCl, 1.25 $NaH_2PO_4$, 6 $MgCl_2$, 1 $CaCl_2$, and 26 $NaHCO_3$). Slices were transferred to a holding chamber containing artificial cerebrospinal fluid (aCSF, in mM: 126NaCl, 2.5 KCl, 1.2 $NaH_2PO_4$, 1.2 $MgCl_2$, 2.4 $CaCl_2$, 21.4 $NaHCO_3$ and 11.1 glucose) and allowed to recover at room temperature for at least 1 h. The slices were then individually transferred to the recording chamber and perfused at a rate of 2 ml/min with $MgCl_2$-free recording solution (containing in mM: 126 NaCl, 2.5 KCl, 2.4 $CaCl_2$, 1.2 $NaH_2PO_4$, 21.4 $NaHCO_3$, and 11 glucose). The $MgCl_2$-free solution also contained 10 $\mu$M glycine and 500 nM TTX (tetrodotoxin). All solutions had an osmolarity of 290-300 mOsm and were bubbled with 95% $O_2$ / 5% $CO_2$.

***Whole-cell patch clamp recordings***

Cells were visualized with an upright microscope (Leica DM LFSA, Leica Instruments) using both fluorescent microscopy and infrared illumination. Recording pipettes (8-10 M ohms) contained in mM: 145 KCl, 10 HEPES, 1.1 EGTA, 1 $MgCl_2$, 2 MgATP, 0.5 $Na_2$GTP, pH 7.2-7.4, 280-290 mOsm. Recording pipettes were advanced towards individual fluorescent cells in the slice under positive pressure and, on contact, tight seals between the pipette and the cell membrane (~ 1 G ohms) were made by negative pressure. The membrane patch was then ruptured by suction and membrane currents were monitored using an Axopatch ID amplifier (Molecular Devices, formerly Axon Instruments). Neurons were voltage-clamped at -60mV.

***Local NMDA application***

NMDA evoked currents were elicited using an eight-channel local perfusion system (BPS-8, ALA-Scientific). The local perfusion needle was placed just above the tissue near the cell being recorded. Discrete currents were evoked by 80-180 ms application of 300 $\mu$M NMDA, immediately followed by a 540 ms application of $MgCl_2$-free recording solution. NMDA evoked currents were elicited every 20-30 seconds. The NMDA containing solution was made up from a 10 mM stock solution that was diluted to 300 $\mu$M in $MgCl_2$-free recording solution. As mentioned above, the $MgCl_2$-free solution also contained 10 $\mu$M glycine and 500 nM TTX.

***Bath application of antagonists***

All of the antagonists tested including MK-801, memantine, MDT-9, MDT-3, MDT-23 and MDT-22 (see Table 28 above for a description of the diamondoid compounds) were prepared as 10 mM stock solutions in $ddH_2O$. The stock solutions were then diluted to their final concentrations in $MgCl_2$-free recording solution. Following the establishment of a stable baseline (at least five consistent consecutive NMDA-evoked currents) the antagonists were applied to the slices via the bath using a 4-barrel gravity perfusion system (ALA-Scientific) at a rate of 2-3 ml/min.

***Analysis***

NMDA evoked currents were filtered at 1-2 kHz, digitized at 10 kHz and stored using pClamp 9.0 software

(Molecular Devices). Peak amplitude values were determined using pClampfit software 9.0 (Molecular Devices). The percent inhibition produced by the antagonists was calculated as the change in NMDA-evoked current peak amplitude from baseline. All values are expressed as mean ± SEM. Statistical significance was assessed using one-tailed Student's *t*-tests.

## Results

To establish this assay we first demonstrated that local application of NMDA could produce inward currents in hypocretin neurons in the hypothalamus under voltage clamp at - 60 mV in $MgCl_2$-free solution. It was further demonstrated that these currents were specific to the application of NMDA since local application of control aCSF instead of NMDA did not induce an inward current in these neurons.

These initial studies demonstrated that the amplitude and kinetics (shape) of the NMDA evoked currents was dependent on the proximity of the local perfusion needle to the cell being recorded. Thus there were considerable differences in the responses between experiments. However, subsequent experiments demonstrated that the percent inhibition of NMDA evoked currents, induced by several NMDA receptor antagonists was similar between the cells.

The effect of known NMDA receptor antagonists was then tested on the inward current induced in hyprocretin neurons by the local application of NMDA. Bath application of both MK-801 and memantine significantly inhibited the NMDA evoked currents. The parameters of the experiment did not allow differentiation of the control compounds based on voltage dependence or changes in the kinetics of the response but we were able to differentiate the compounds based on their potency. MK-801 (100 nM) produced a significant inhibition of the peak amplitude of NMDA evoked currents, 58 ± 9.4% (mean ± SEM, n=3). Memantine produced a concentration dependent and reversible inhibition of NMDA evoked currents, with 10 μM producing a 41.3 ± 10% (n=3) inhibition and 30 μM producing a 52.7 ± 7.4% (n=3) inhibition. Thus, consistent with the literature, there was a considerable difference in the potency of the two control compounds, with MK-801 being the most potent.

MDT-3, MDT-9, MDT-22 and MDT-23 were then tested to determine if they could inhibit the NMDA evoked currents. The effect of two concentrations (10 and 100 μM) of each compound on the peak amplitude of NMDA currents was examined.

Bath application of MDT-3 (10 and 100 μM) did not produce inhibition ofNMDA evoked currents.

Bath application of 10 μM MDT-9 produced a small and statistically insignificant inhibition of the NMDA evoked currents (13.2 ± 9%, n=3). At 100 μM, MDT-9 still produced a small but significant inhibition of these currents (15.2 ± 4.8%, n=3).

In contrast, bath application of MDT-22 produced significant inhibition of the peak amplitude of NMDA evoked currents at both concentrations tested. The effect was concentration dependent with 10 μM producing a 19 ± 3.1 % inhibition (n=4) and 100 μM producing a 45 ± 12.1 % inhibition (n=2). The effect was reversible upon washout of the compound.

Bath application of 10 μM MDT-23 did not significantly inhibit NMDA evoked currents (11.6 ± 4.3%, n=3). In contrast, the highest concentration of MDT-23 tested (100 μM) did significantly inhibit the amplitude of NMDA currents (22.9 ± 9.3%, n=4). However, this inhibition was not reversible. NMDA currents did not return to baseline upon washout of this compound.

The percent inhibition produced by the two control compounds (MK-801 and memantine) and the four test compounds (MDT-3, MDT-9, MDT-22 and MDT-23) are summarized in Figure 33. Figure 33 is a summary bar graph demonstrating the % inhibition of the peak amplitude of NMDA evoked currents produced by all the compounds tested. The concentration used for each compound is listed under the corresponding bar. The asterisks indicate that the compound produced a significant inhibition $p \leq 0.05$. In all cases, except for MDT-3, the number of cells tested was between 3-4 for each compound. For MDT-3, n=1.

Of the four test compounds tested, MDT-22 produced substantial and significant inhibition of NMDA evoked currents in hypocretin neurons. The modulation of these currents was specific to the application of MDT-22 since it was reversible upon washout of the compound. In addition, the inhibition of NMDA evoked currents induced by MDT-22 was concentration dependent. Moreover, the percent inhibition produced by the 100 μM concentration of this compound was similar to the percent inhibition produced by the control antagonists MK-801 and memantine. MDT-22 is capable of modulating NMDA receptor-induced currents and could be potentially neuroprotective through the modulation of NMDA receptor-mediated activity. MDT-23 and MDT-9 also inhibited NMDA evoked current and, thus, are also potentially useful in this regard.

## References

1 Evans, J.G., G. Wilcock, and J. Birks, Evidence-based pharmacotherapy of Alzheimer's disease. Int J Neuropsychopharmacol, 2004. 7(3): p. 351-69.

2. Mahant, N., et al., Huntington's disease: clinical correlates of disability and progression. Neurology, 2003. 61(8): p. 1085-92.

3. Henry, J.D. and J.R. Crawford, Verbal fluency deficits in Parkinson's disease: a meta-analysis. J Int Neuropsychol Soc, 2004. 10(4): p. 608-22.

4. Downes, J.J., et al., Impaired extra-dimensional shift performance in medicated and unmedicated Parkinson's disease: evidence for a specific attentional dysfunction. Neuropsychologia, 1989. 27(11-12): p. 1329-43.

5. Lawrence, A.D., et al., Visual object and visuospatial cognition in Huntington's disease: implications for information processing in corticostriatal circuits. Brain, 2000. 123 (Pt 7) : p. 1349-64.

6. Farlow, M.R., NMDA receptor antagonists. A new therapeutic approach for Alzheimer's disease. Geriatrics, 2004. 59(6): p. 22-7.

7. Lipton SA. Paradigm shift in neuroprotection by NMDA receptor blockade: memantine and beyond. Nat Rev Drug Discov. 2006 Feb;S(2):160-70. Review.

8. Xie, X., Crowder, T.L., Yamanaka, A., Morairty, S.R., LeWinter, R.D., Sakurai, T., and T.S. Lilduff, GABAB receptor-mediated modulation of hypocretin/orexin neurones in mouse hypothalamus. J Physiol, 2006, online DOI: 10.1113/jphysio1.2006.108266.

**Example 24:**

**Neuroprotection in NMDA-induced LDH release model for MDT-22, MDT-23, MDT-24, MDT-30, MDT-43, MDT-50 and MDT-51**

***In Vitro Assay for Acute Neuroprotection***

**1. Glutamate Receptors and Neurotoxicity**

Glutamate receptors are essential for the normal functioning of the central nervous system (CNS). Excessive activation of these receptors by excitatory amino acids such as glutamate itself can lead to neuronal damage, contributing to various neurodegenerative conditions such as Alzheimer's disease, Parkinson's disease, Huntington's disease and amyotrophic lateral sclerosis. Glutamate receptors are functionally classified as ligand-gated ion channels or "ionotropic" receptors, and G-protein coupled "metabotropic" receptors. Based on the selective agonists that activate the receptor subtype, ionotropic receptors are further classified as AMPA ($\alpha$-amino-3-hydroxy-5-methyl-4-isoxazole propionic acid), kainite and NMDA (N-methyl-D aspartate) receptors. Exposure of neurons to high concentrations of glutamate for even a few minutes can lead to cell death (Meldrum and Garthwaite, 1990). This process is similar to the neurotoxicity that occurs after ischemia or hypoxia, where a massive release and impaired reuptake of glutamate in the synapse leads to excess stimulation of NMDA receptors, influx of $Ca^{+2}$ into the neurons and subsequent cell death. There is considerable therapeutic potential for the use of NMDA antagonists as neuroprotectants. MK-801, a non-competitive NMDA antagonist which has potent anti-convulsant, central sympathomimetic and anxiolytic effects was found to be neuroprotective (Wong et al., 1986, Woodruff et al, 1987). Memantine, discovered 10 years later and also a non-competitive NMDA antagonist, was recently approved for use in the treatment of Alzheimer's disease (Reisberg et al, 2003). We have used MK-801 and memantine in the neuroprotection assays described below, to antagonize the effects of NMDA-mediated LDH release by cortical neurons.

**2. Quantitative determination of neurotoxicity/neuroprotection by LDH assay**

The neuroprotective properties of drugs have been studied in vitro, using neurons cultured from cortex, cerebellum or retina. In this study, we have cultured primary neurons from rat cerebral cortex and used these cultures to test the neuroprotective potential of MDT compounds compared to memantine and/or MK-801, during brief NMDA exposure. Neurotoxicity is determined in this assay by measuring lactate dehydrogenase (LDH), a method which is simple, accurate and reproducible. We conducted pilot studies to test if poly-lysine-coated plates would be suitable as a matrix upon which to plate fetal neurons, compared to astrocyte-coated plates. We also compared the use of growth medium to neurobasal medium supplemented with B27 in the pilot experiments. The concentrations of NMDA (300-500 $\mu$M), glycine (10-50 $\mu$M), and memantine (10-100 $\mu$M) were varied, and optimum concentrations and times determined from these pilot studies were then chosen for the main study. The assay is described below.

### 3. Preparation of Cortical Cultures

Fetal rat neocortical cells were cultured on a mouse astroglial feeder layer. To obtain mouse astroglial cells for the feeder layer, cortices were harvested from 1-day old Swiss Webster mice (Charles River, Hollister, CA) according to the procedure described by Rose et al (1993). After dissecting cortices in ice-cold dissecting medium, they were minced and incubated in 0.09% trypsin in media stock for 1 hr, followed by resuspension in growth medium containing 10% equine serum and 10% fetal bovine serum. A single-cell suspension was obtained by triturating through fire-bored glass pipettes, and the resulting cell suspension was plated at a density of 0.5-0.75 hemispheres/24-well plate/9.6 ml, with 0.4 ml/well. Plates were incubated in a humidified incubator at 37˚C, 5% $CO_2$. Astroglial cells become confluent in about 2-3 weeks, at which time these cultures are ready to be used as feeder layers upon which neuronal cultures can be plated. Neuronal cells were obtained from gestational Sprague-Dawley rats E16-E18, using a similar dissection procedure. Rat embryos were dissected to obtain cortical tissue, which was plated as a single-cell suspension at a density of 1.4 hemispheres/24-well plate/9.6ml, with 0.4 ml/well. These cells were plated on the existing astroglial feeder layer, and were incubated at 37˚C, 5% $CO_2$. After 5 days in culture, cytosine arabinoside (10 μM) was added to the wells to inhibit glial cell proliferation, and after 24 hours, the medium was replaced with fresh growth medium. Cells were fed with fresh growth medium every 2 days, until the experiments were performed. For coating plates with poly-lysine, we used poly-d-lysine hydrobromide (Sigma Cat # P7280) at a concentration of 0.1 mg/ml to coat each well using a minimum volume of 0.2 ml/well. After coating for 5 minutes, the solution was aspirated and wells were rinsed thoroughly with sterile tissue culture grade water. After drying for 2 hours, plates were ready for use. Neuronal cultures were plated onto poly-lysine-coated plates at the same density as above. For the pilot study, some of the cultures were fed with 1: 1 ratio of growth medium: neurobasal medium (GIBCO) + B-27 supplement (2%), while the rest of the cultures were fed with growth medium alone (Losi et al., 2006). For the main study, we used astroglial feeder layers to plate the neuronal cultures, and used a mix (1:1) of growth medium with neurobasal medium + B-27 supplement for feeding the cultures.

### 4. NMDA-induced LDH release

After 11-14 days in culture, neuronal cells were confluent and experiments were performed. Cells were washed twice using sterile Locke's buffer (154 mM NaCl, 5.6 mM KCl, 2.3 mM $CaCl_2$, 3.6 mM $NaHCO_3$, 5.5 mM D-glucose, 5 mM HEPES, pH 7.4), and then exposed to the various treatment compounds. The antagonists (MK-801, memantine, or MDT compounds) were added first, followed by 300 uM NMDA + 50 μM glycine. After 15 minutes, cultures were washed twice and incubated overnight in media stock. After 24 hours, supernatants and lysates were collected to measure LDH activity. LDH activity was measured by the reduction of NAD, which was utilized in the stoichiometric conversion of a tetrazolium dye using a kit (Sigma TOX-7). Supernatants were removed from the wells and centrifuged before LDH measurements. The adherent cells were lysed to obtain total LDH. Percent LDH was calculated as follows: 100 x [LDH from the supernatant (secreted) / (total + secreted LDH)]. Incubation times after NMDA exposure were varied in the pilot experiments, to determine the optimum length of time after which LDH measurements should be made (24 hours - 40 hours). Tables 30-34 show the results for the pilot experiments and Figures 132-138 show the results from the main study experiments.

### 5. Results

Percent LDH was calculated as follows: 100 x [LDH from the supernatant (secreted) / (total + secreted LDH)]. The average value for the control wells (wash alone, no treatment or NMDA exposure) was designated as a baseline of 100%. From this, we calculated the value for NMDA exposure (which was usually the highest), as well as the values for the standard compounds memantine and MK801, and the test MDT compounds (all of which fall between the baseline and maximum). The graphs have percent of control LDH as the ordinate, and concentration of the MDT drugs as the abscissa, and were prepared using Graphpad Prism (San Diego, CA). Each graph provides data from two separate experiments for each MDT compound, and the memantine data is plotted to provide a comparison. The graphs provide $EC_{50}$ values for memantine and the MDT drug.

From the results of the pilot studies, astrocyte-coated plates which were fed using the mixed medium (see methods above) showed the highest difference between control (100%) and NMDA treatment. When viewed under a phase-contrast microscope, the neuronal layer appeared healthier with more neuronal processes when the cells were grown on an astroglial layer as compared to poly-lysine. Also, the neuronal cells looked much healthier when grown in the presence of neurobasal medium with B27 supplement, as compared to growth medium alone. In the pilot studies, we varied the time cells were incubated after NMDA exposure, measuring LDH 24-40 hours later. Based on the results from the pilot studies, we used astrocyte-coated plates and a mix of growth medium with neurobasal medium + B27 supplement for the main study. After NMDA exposure, cells were incubated for 24 hours before LDH assay, based on results from the pilot experiments.

For the main study, we set up two sets of 24-well plates with 4 plates/set. Each set had the standards NMDA, memantine and MK-801 and the MDT compounds at various concentrations. All measurements were performed in triplicate. MDT drugs #22, 24 and 50 were tested at concentrations 10, 30 and 100 μM, while MDT drugs #23, 30, 43 and 51 was tested at concentrations 1, 10, 30 and 100 μM. For the graphs, the data was normalized using Graphpad Prism according to the minimum (100%, control) and maximum (NMDA) LDH values for each data set, and $EC_{50}$ values were determined. All the compounds tested were able to provide some degree of neuroprotection, as seen in the graphs (the highest concentrations are close to 0 on the Y-axis). MDT compounds 30 and 51 demonstrated $EC_{50}$ values close to that of memantine. MDT compounds 23, 43, 24, 22 and 50 have decreasing order of potency compared to memantine. The average $EC_{50}$ values (±SD) for two experiments are 4.6 ± 1.4 μM for MDT-30; 11.1 ± 9.3 μM for MDT-23; 15.7 ± 9.1 μM for MDT-51; 28.4 ± 12.8 μM for MDT-43; 34.3 ± 11.3 μM for MDT-24; 56.5 ± 14.6 μM for MDT-22; and 106.9 ± 88.3 μM for MDT-50.

## 6. References

Meldrum B. and Garthwaite J. Excitatory amino acid neurotoxicity and neurodegenerative disease. Trends Pharm Sci 1990, 11:379-387.

Woodruff GN, Foster AC, Gill R, Kemp JA, Wong EH, Iversen LL. The interaction between MK-801 and receptors for N-methyl-D-aspartate: functional consequences. Neuropharmacology 1987 Jul;26(7B):903-9.

Wong EH, Kemp JA, Priestley T, Knight AR, Woodruff GN, Iversen LL. The anticonvulsant MK-801 is a potent N-methyl-D-aspartate antagonist. Proc Natl Acad Sci U S A 1986 Sep;83(18):7104-8.

Reisberg B. et al. Memantine in moderate-to-severe Alzheimer's disease. New Eng Jour Medicine 2003, 348: 1333-1341.

Rose K, Goldberg MP and Choi DW. Cytotoxicity in Murine Neocortical Cell Culture. From "Methods in Toxicology" CA Tyson and JM Frazier (eds), Academic Press, San Diego, CA 1993, pg 46-60.

Losi G, Lanza M, Makovec F, Artusi R, Caselli G and Puia G. Functional in vitro characterization of CR 3394: A novel voltage dependent N-methyl-D-aspartate (NMDA) receptor antagonist. Neuropharmacology 2006, 50:277-285.

**Table 30**

Pilot Study: Astrocyte-coated rat neurons, GM/NB+B-27 fed, 21 hour incubation

| Treatment | Total LDH (Avg) | Secreted LDH (Avg) | Secreted/ (total+secreted) (Avg) | % |
|---|---|---|---|---|
| Control Wash | 3.92 | 0.35 | 8.30 | 100 |
| NMDA 300 μM + 10 μM glycine | 3.25 | 0.44 | 11.95 | 144 |
| NMDA 500 μM + 10 μM glycine | 3.91 | 0.51 | 11.56 | 139 |
| NMDA 500 μM + MK801 1 μM | 3.71 | 0.43 | 10.39 | 125 |
| NMDA 500 uM + Memantine 10 μM | 3.89 | 0.47 | 10.73 | 129 |
| NMDA 500 uM + Memantine 100 μM | 4.02 | 0.42 | 9.47 | 114 |
| NMDA 300 μM - 21 hr | 3.22 | 0.52 | 13.82 | 166 |
| NMDA 500 μM - 21 hr | 3.65 | 0.51 | 12.23 | 147 |

**Table 31**

Pilot Study: Poly-lysine-coated rat neurons, GM/NB+B-27 fed, 40 hour incubation

| Treatment | Total LDH (Avg) | Secreted LDH (Avg) | Secreted/ (total+secreted) (Avg) | % |
|---|---|---|---|---|
| Control Wash | 2.86 | 0.37 | 11.43 | 100 |
| NMDA 300 μM + 10 μM glycine | 2.56 | 0.39 | 13.33 | 116 |

(continued)

Pilot Study: Poly-lysine-coated rat neurons, GM/NB+B-27 fed, 40 hour incubation

| Treatment | Total LDH (Avg) | Secreted LDH (Avg) | Secreted/ (total+secreted) (Avg) | % |
|---|---|---|---|---|
| NMDA 500 uM + 10 uM glycine | 2.92 | 0.44 | 13.07 | 114 |
| NMDA 500 uM + MK801 1 uM | 2.90 | 0.36 | 11.04 | 96 |
| NMDA 500 uM + Memantine 10 μM | 2.80 | 0.41 | 12.77 | 111 |
| NMDA 500 uM + Memantine 100 uM | 2.39 | 0.52 | 17.86 | 156 |
| NMDA 300 uM - 40 hr | 2.61 | 0.45 | 14.55 | 127 |
| NMDA 500 uM - 40 hr | 2.91 | 0.48 | 14.35 | 125 |

**Table 32**

Pilot Study: Astrocyte-coated rat neurons, GM/NB+B-27 fed, 24 hour incubation

| Treatment | Total LDH (Avg) | Secreted LDH (Avg) | Secreted/ (total+secreted) (Avg) | % |
|---|---|---|---|---|
| Control Wash | 3.69 | 0.29 | 7.21 | 100 |
| NMDA 300 uM + 50 uM glycine | 3.10 | 0.76 | 19.61 | 271 |
| NMDA 300 uM + MK801 1 uM | 3.51 1 | 0.74 | 17.39 | 241 |
| NMDA 300 uM + Memantine 50 uM | 3.41 | 0.83 | 19.63 | 272 |
| MDT #6 30 uM | 3.55 | 0.85 | 19.24 | 266 |
| MDT #13 30 uM | 3.41 | 0.85 | 19.97 | 276 |
| MDT #22 30 uM | 3.09 | 0.77 | 19.87 | 275 |
| MDT #23 30 uM | 3.28 | 0.80 | 19.59 | 271 |

**Table 33**

Pilot Study: Plate #4, poly-lysine-coated rat neurons, GM/NB+B-27 fed, 24 hour incubation

| Treatment | Total LDH (Avg) | Secreted LDH (Avg) | Secreted/ (total+secreted) (Avg) | % |
|---|---|---|---|---|
| Control Wash | 3.37 | 0.41 | 10.90 | 100 |
| NMDA 300 uM + glycine 50 uM | 3.19 | 0.69 | 17.79 | 163 |
| NMDA 300 uM + MK801 1 uM | 3.34 | 0.62 | 15.63 | 143 |
| NMDA 300 uM + Memantine 50 uM | 3.36 | 0.75 | 18.20 | 166 |
| MDT #6 30 uM | 2.70 | 0.65 | 19.55 | 179 |
| MDT #13 30 uM | 2.99 | 0.54 | 15.25 | 139 |
| MDT #22 30 uM | 2.63 | 0.61 | 18.95 | 173 |
| MDT #23 30 uM | 3.09 | 0.76 | 19.74 | 181 |

**Table 34**

Pilot Study: Plate #5, astrocyte-coated rat neurons, GM fed, 24 hour incubation

| Treatment | Total LDH (Avg) | Secreted LDH (Avg) | Secreted/ (total+secreted) (Avg) | % |
|---|---|---|---|---|
| Control Wash | 2.92 | 0.18 | 5.85 | 100 |

(continued)

Pilot Study: Plate #5, astrocyte-coated rat neurons, GM fed, 24 hour incubation

| **Treatment** | **Total LDH (Avg)** | **Secreted LDH (Avg)** | **Secreted/ (total+secreted) (Avg)** | **%** |
|---|---|---|---|---|
| NMDA 300 uM + glycine 50 uM | 2.90 | 0.20 | 6.37 | 109 |
| NMDA 300 uM + MK801 1 uM | 3.18 | 0.18 | 5.43 | 92 |
| NMDA 300 uM + Memantine 50 uM | 3.06 | 0.20 | 6.20 | 106 |
| MDT #1 30 uM | 2.96 | 0.18 | 5.81 | 99 |
| MDT #2 30 uM | 3.09 | 0.23 | 6.93 | 118 |
| MDT #3 30 uM | 2.96 | 0.29 | 8.99 | 153 |
| MDT #5 30 uM | 3.27 | 0.25 | 7.21 | 123 |

**Example 25:**

**MES-induced and PTZ-induced seizure/death model and rotarod test for MDT-22, MDT-23 and MDT-30**

The ability of MDT-22, MDT-23 and MDT-30 to provide neuroprotection *in vivo* was monitored in the maximum electric shock (MES)-induced seizure/death model and in the pentylenetetrazol (PTZ)-induced seizure/death model. Side effects of MDT-22, MDT-23 and MDT-30 were evaluated using the rotarod test, which primarily assesses the balancing and coordination of mice.

In the experiments, all animals (C57/BL6 male mice, 8-10 weeks old) were first subjected to a rotarod test on the balance and coordination of the mice. Administration of the MDT compounds and vehicles was by an i.p. injection, and behavior was monitored for 20 minutes. A second rotarod was conducted to access the effect of drug/vehicle followed by the seizure induction. One set of animals was given an i.p injection of PTZ to induce seizure/death and were monitored for up to 30 minutes. Another set of animals was subjected to electric shock through ear electrodes at a consistent current of 40 mA, 0.3 seconds, maximal 750V and maximal 75W (Rodent Shocker, Type 221; Hugo Sachs, Freiburg, Germany).

Vehicles used were either saline at pH 5.2 or 2% DMSO in hydroxyl propyl cellulose (HPC). Because there were no significant differences between Vehicle I (Saline, pH 5.2) and Vehicle II (2% DMSO in HPC, pH 5.4) in this and the other tests, we combined the two vehicle groups in data analysis as Control. Memantine was dissolved in saline at either a low dose of 2 mg/ml for 10 mg/kg injection at 5 μl/g body weight, or a high dose of 6 mg/ml for 30 mg/kg injection at 5 μl/g body weight. MK801 was dissolved in saline at either a low dose of 0.1 mg/ml for 0.5 mg/kg injection at 5 μl/g body weight, or a high dose of 0.2 mg/ml for 1 mg/kg injection at 5 μl/g body weight. MDT-22, MDT-23 and MDT-30 were prepared in 2% DMSO in HPC at either a low dose of 2 mg/ml for 10 mg/kg injection at 5 μl/g body weight, or a high dose of 6 mg/ml for 30 mg/kg injection at 5 μl/g body weight. PTZ was prepared at 20 mg/ml in saline for a single injection of 100 mg/kg at 5 μl/g body weight.

**1. Maximum electric shock (MES)-induced seizure/death model**

The results of maximum electric shock studies are summarized in the Table 35. The MDT compounds showed a dose dependent reduction in MES-induced death with the efficacy order being MDT-30 > MDT-23 > MDT-22. Memantine at the low dose (10 mg/kg) protected all mice from death, while MK801 at test dose (0.5mg/kg) caused little effect compared to control.

**Table 35**

| Maximum electric shock (MES)-induced seizure/death model | | | | | | | | | |
|---|---|---|---|---|---|---|---|---|---|
| | **Control** | **MDT-22** | **MDT-22** | **MDT-23** | **MDT-23** | **MDT-30** | **MDT- 30** | **MK801** | **Memantine** |
| **Dose (mg/kg)** | NA | 30 | 10 | 30 | 10 | 30 | 10 | 0.5 | 10 |
| **Number of mice** | 12 | 6 | 6 | 6 | 6 | 6 | 6 | 6 | 6 |

(continued)

| Maximum electric shock (MES)-induced seizure/death model | | | | | | | | | |
|---|---|---|---|---|---|---|---|---|---|
| | **Control** | **MDT-22** | **MDT-22** | **MDT-23** | **MDT-23** | **MDT-30** | **MDT- 30** | **MK801** | **Memantine** |
| **# of extensions** | 12 | 5 | 6 | 6 | 6 | 2 | 6 | 3 | 4 |
| **# of deaths** | 7 | 2 | 3 | 1 | 4 | 0 | 0 | 3 | 0 |
| **Extension rate** | 1.00 | 0.83 | 1.00 | 1.00 | 1.00 | 0.33 | 1.00 | 0.50 | 0.67 |
| **Mortality rate** | 0.58 | 0.33 | 0.50 | 0.17 | 0.67 | 0 | 0 | 0.50 | 0 |

**2. Pentylenetetrazol (PTZ)-induced seizure/death model**

Similar to the result obtained from the MES test, the MDT compounds showed a certain reduction in PTZ-induced death with the efficacy order being MDT-30 > MDT-23 > MDT-22. Memantine produced a dose-dependent reduction in death, with full protection seen at the high dose, as also found for MK801 at test dose (1 mg/kg) (Table 36).

**Table 36**

| Pentylenetetrazol (PTZ)-induced seizure/death model | | | | | | | | | | |
|---|---|---|---|---|---|---|---|---|---|---|
| | **Control** | **MDT-22** | **MDT-22** | **MDT-23** | **MDT-23** | **MDT- 30** | **MDT-30** | **MK801** | **Memantine** | **Memantine** |
| **Dose (mg/kg)** | NA | 30 | 10 | 30 | 10 | 30 | 10 | 1.0 | 30 | 10 |
| **# of mice** | 14 | 6 | 6 | 6 | 6 | 6 | 6 | 6 | 6 | 6 |
| **# of deaths within 30 min** | 10 | 4 | 6 | 6 | 3 | 0 | 4 | 0 | 0 | 1 |
| **Mortality rate** | 0.71 | 0.67 | 1.00 | 1.00 | 0.50 | 0 | 0.67 | 0 | 0 | 0.17 |
| **Time to death (min)** | 9.90 ± 2.30 | 7.70 ± 2.10 | 9.60 ± 1.80 | 22.00 ± 2.90 | 14.00 ± 6.60 | NA | 8.30 ± 2.60 | NA | NA | NA |
| **# mice with L3 seizure** | 10 | 6 | 3 | 6 | 3 | 6 | 6 | NA | NA | NA |
| **L3 seizure rate** | 0.71 | 1.00 | 0.50 | 1.00 | 0.50 | 1.00 | 1.00 | NA | NA | NA |
| **L3 onset time (min)** | 4.70 ± 0.95 | 5.10 ± 2.30 | 2.80 ± 1.00 | 5.00 ± 1.90 | 3.00 ± 0.20 | 2.60 ± 0.70 | 2.10 ± 1.40 | NA | NA | NA |
| **# L3 per mouse** | 0.93 ± 0.26 | 1.67 ± 0.50 | 2.00 ± 0.58 | 3.83 ± 2.44 | 4.67 ± 1.65 | 6.50 ± 2.01 | 3.67 ± 1.08 | NA | NA | NA |
| **# mice with L4 seizure** | 12 | 6 | 6 | 6 | 6 | 6 | 5 | NA | NA | NA |
| **L4 seizure rate** | 0.86 | 1.00 | 1.00 | 1.00 | 1.00 | 1.00 | 0.83 | NA | NA | NA |
| **L4 onset time (min)** | 9.70 ± 230 | 10.20 ± 3.00 | 7.90 ± 1.70 | 19.80 ± 4.20 | 13.80 ± 4.40 | 12.90 ± 3.50 | 4.40 ± 0.90 | NA | NA | NA |
| **# L4 per mouse** | 1.25 ± 0.14 | 1.17 ± 0.19 | 3.85 ± 2.28 | 1.00 ± 0.0 | 1.67 ± 0.49 | 3.50 ± 0.62 | 2.40 ± 0.68 | NA | NA | NA |

Although the protection against death is the key endpoint, other effects of the compounds on PTZ-induced seizures were also assessed using multiple parameters. For the mice that did die within the 30 minute observation window, the time of death was also compared. Although all the MDT-23 (30mg/kg) treated mice died within 30 minutes, the average time of death was significantly longer than that of control (22 ± 2.9 vs. 9.9 ± 2.3 minutes; p = 0.005 unpaired t-test), suggesting a protection effect. The seizure occurrence rate, onset time and number of level 3 (L3) seizure (repetitive seizures but the animal can still stand) and level 4 (L4) seizure (repetitive seizures and the animal loses righting) are also analyzed. The MDT compound (30mg/kg) treated groups show a certain degree of improvement compared to control. No data from MK801 or memantine were included because the seizures occurring under these treatments are totally different from the control or MDT injected groups due to side effects ("high jumping").

**3. Rotarod test**

The side effects of the compounds was primarily evaluated using the rotarod test, which mainly assesses the balancing and coordination of mice. The comparisons were made on fall time of the mice between the first measure (pre-treatment) and second measure taken 20 min after the drug/vehicle injection in the same group (paired t-test). These results showed an improvement in performance (longer fall time) for control animals and low doses of all MDT compounds ($p \leq 0.05$ or better). High doses of MDT-23 and Memantine showed a loss of performance that was statistically significant only for the MDT compound ($P \leq 0.02$). Comparisons of the rotatod fall times after drug/vehicle dosing were made between drug treatment and control groups (unpaired t-test). There was no significant difference in rotarod performance after low dose drug treatment compared to control using MDT compounds, unlike either dose of Memantine ($p \leq 0.01$) (see column labeled "p vs. control"). This result shows that MDT compounds produce fewer side effects than Memantine at the low dose (10 mg/kg). At the high dose (30 mg/kg) MDT-23 and Memantine showed statistically significant decreases in rotarod performance ($p \leq 0.007$; p vs. control). Hyperactivity is a side effect that was observed only with MK-801 (1 mg/kg) and high dose Memantine (30 mg/kg). This behavior prevented testing using the rotarod of all the MK-801 treated mice and one of the high dose Memantine mice. The results are presented in Table 37.

**Table 37**

| Rotarod test | | | | | |
|---|---|---|---|---|---|
| | n | | Fall Time (min) | p (paired) | p (vs. control) |
| Saline | 24 | before | 39.26 ± 3.75 | | |
| | | after | 48.80 ± 3.50 | 0.0101 | 0.9967 |
| 2% DMSO in HPC | 20 | before | 39.24 ± 3.88 | | |
| | | after | 49.63 ± 4.64 | 0.0048 | 0.8851 |
| MDT-22 30 mg/kg | 12 | before | 45.03 ± 6.02 | | |
| | | after | 44.93 ± 5.45 | 0.9912 | 0.5258 |
| MDT-22 10 mg/kg | 12 | before | 33.56 ± 3.91 | | |
| | | after | 44.58 ± 4.58 | 0.0044 | 0.4747 |
| MDT-23 30 mg/kg | 15 | before | 39.77 ± 6.14 | | |
| | | after | 31.37 ± 3.83 | 0.0229 | 0.0067 |
| MDT-23 10 mg/kg | 12 | before | 28.63 ± 3.10 | | |
| | | after | 42.98 ± 2.82 | 0.0023 | 0.3074 |
| MDT-30 30 mg/kg | 11 | before | 37.55 ± 4.98 | | |
| | | after | 24.14 ± 3.94 | 0.0150 | 0.00017 |
| MDT-30 10 mg/kg | 12 | before | 35.44 ± 4.19 | | |
| | | after | 47.34 ± 5.53 | 0.0415 | 0.8183 |
| Memantine 30 mg/kg | 5 | before | 18.95 ± 2.80 | | |
| | | after | 10.10 ± 2.05 | 0.0576 | 0.00004 |

(continued)

| Rotarod test | | | | | |
|---|---|---|---|---|---|
| | n | | Fall Time (min) | p (paired) | p (vs. control) |
| Memantine 10 mg/kg | 14 | before | 38.03 ± 4.37 | | |
| | | after | 34.19 ± 4.55 | 0.3860 | 0.0155 |

**Example 26:**

**Assay for Neuronal Cell Function and Death**

To test the diamondoid derivatives of the present invention for their ability to prevent neurotoxicity, neuronal cell death may be assayed as follows.

Under general anesthesia, the fluorescent dye granular blue (Mackromolecular Chemin, Umstadt, FRG) may be injected as approximately a 2% (w/v) suspension in saline into the superior colliculus of 4- to 6-day-old Long-Evans rats (Charles River Laboratory, Wilmington, Mass.). Two to six days later, the animals may be sacrificed by decapitation and enucleated, and the retinas quickly removed. The retinas may be dissociated by mild treatment with the enzyme papain and cultured in Eagle's minimum essential medium (MEM, catalog #1090, Gibco, Grand Island, N.Y.) supplemented with 0.7% (w/v) methylcellulose, 0.3% (w/v) glucose, 2 mM glutamine, 1 .mu.g/ml gentamicin, and 5% (v/v) rat serum, as described in Lipton et al., J Physiol. 385:361, 1987. The cells are plated onto 75 mm.sup.2 glass coverslips coated with poly-L-lysine in 35 mm tissue culture dishes. The candidate diamondoid derivative is added (e.g., in a series of concentrations ranging from 1 nM-1 mM) in the presence or absence of compounds which activate the NMDA receptor-operated channel complex, and in high calcium, low magnesium medium (10 mM $CaCl_2$, 50 .mu.M $MgCl_2$) to enhance NMDA-receptor neurotoxicity in this preparation (Hahn et al., Proc. Natl. Acad. Sci. USA 85:6556, 1988; Levy et al., Neurology 40:852, 1990; Levy et al., Neurosci. Lett. 110:291, 1990). The degree of survival (under these ionic conditions or with added exogenous NMDA (200μM)) is compared to that in normal medium (1.8 mM $CaCl_2$, 0.8 mM $MgCl_2$), which minimizes NMDA receptor-mediated injury in this preparation (Hahn et al., cited above). Incubations last 16-24 h at 37 degrees Celsius in an atmosphere of 5% $CO_2$/95% air. The ability of retinal ganglion cells to take up and cleave fluorescein diacetate to fluorescein is used as an index of their viability as described in detail in Hahn et al., (Proc. Natl. Acad. Sci. USA 85:6556, 1988). Dye uptake and cleavage generally correlate well with normal electrophysiological properties assayed with patch electrodes.

To perform the viability test, the cell-culture medium may be exchanged for physiological saline containing 0.0005% fluorescein diacetate for 15-45 seconds, and then cells may be rinsed in saline. Retinal ganglion cell neurons that do not contain the fluorescein dye (and thus are not living) often remain visible under both phase-contrast and UV fluorescence optics, the latter because of the continued presence of the marker dye granular blue; other dead retinal ganglion cells disintegrate, leaving only cell debris. In contrast, the viable retinal ganglion cells display not only a blue color in the UV light but also a yellow-green fluorescence with filters appropriate for fluorescein. Thus, the use of two exchangeable fluorescence filter sets permits the rapid determination of viable ganglion cells in the cultures. The ganglion cells are often found as solitary neurons as well as neurons lying among other cells in small clusters.

A diamondoid diamantane, or triamantane derivative may be tested for utility in the method of the invention using any type of neuronal cell from the central nervous system, as long as the cell can be isolated intact by conventional techniques. In addition to the retinal cultures described above, hippocampal and cortical neurons may be used though any neuron may be used that possesses NMDA receptors (e.g., neurons from other regions of the brain). Such neurons may be prenatal or postnatal, and they may be from a human, rodent or other mammals. In one example, retinal cultures may be produced from postnatal mammals, as they are well-characterized and contain a central neuron (the retinal ganglion cell) that can be unequivocally identified with fluorescent labels. A substantial portion of retinal ganglion cells in culture display both functional synaptic activity and bear many, if not all, of the neurotransmitter receptors found in the intact central nervous system.

Measurement of Intracellular $Ca^{2+}$

The concentration of intracellular free $Ca^{2+}$ ([$Ca^{2+}$ ]i) may be measured in neonatal cortical neurons by digital imaging microscopy with the $Ca^{2+}$ sensitive fluorescent dye fura 2, as follows. The same cortical neuronal cultures as described above are used. During $Ca_2$+ measurements, unless otherwise stated the fluid bathing the neurons consists of Hanks' balanced salts: 137.6 mM NaCl, 1 mM $NaHCO_3$, 0.34 mM $Na_2$ $HPO_4$, 0.44 mM $KH_2$ $PO_4$, 5.36 mM KCl, 1.25 mM $CaCl_2$, 0.5 mM $MgSO_4$, 0.5 mM $MgCl_2$, 5 mM Hepes NaOH, 22.2 mM glucose, and sometimes with phenol red

indicator (0.001 % v/v); pH 7.2. NMDA (in the absence $Mg^{++}$), glutamate, and other substances may be applied to the neurons by pressure ejection after dilution in this bath solution. Neuronal [$Ca^{2+}$ ]i is analyzed with fura 2-acetoxymethyl ester (AM) as described [Grynkiewicz, et al., J. Biol. Chem. 260:3440 (1985); Williams et al., Nature 318:558 (1985); Connor et al., J. Neurosci. 7:1384 (1987); Connor et al., Science 240:649 (1988); Cohan et al., J. Neurosci. 7:3588 (1987); Mattson, et al., ibid, 9:3728 (1989)]. After adding Eagle's minimum essential medium containing 10 $\mu$M fura 2-AM to the neurons, the cultures are incubated at 37 degrees Celsius in a 5% $CO_2$ /95% air humidified chamber and then rinsed. The dye is loaded, trapped, and deesterified within 1 hour, as determined by stable fluorescence ratios and the effect of the $Ca^{2+}$ ionophore ionomycin on [$Ca^{2+}$ ]i is measured. During $Ca^{2+}$ imaging, the cells may be incubated in a solution of Hepes-buffered saline with Hanks' balanced salts. The [$Ca^{2+}$ ]i may be calculated from ratio images that are obtained by measuring the fluorescence at 500 nm that is excited by 350 and 380 nm light with a DAGE MTI 66 SIT or QUANTEX QX-100 Intensified CCD camera mounted on a Zeiss Axiovert 35 microscope. Exposure time for each picture is 500 milliseconds. Analysis may be performed with a Quantex (Sunnyvale, Calif.) QX7-210 image-processing system. As cells are exposed to ultraviolet light only during data collection (generally less than a total of 20 seconds per cell), bleaching of fura 2 is minimal. Delayed NMDA-receptor mediated neurotoxicity has been shown to be associated with an early increase in intracellular $Ca^{2+}$ concentration.

Correlation Between Channel-Blocking and Anticonvulsive Action

The correlation between the action of the tested diamondoid derivatives at the NMDA receptor channel (*in vitro*) and the anticonvulsive effect (*in vivo*) has been tested. For this purpose an xy diagram of both test parameters can be plotted. It shows that there is a correlation between the blocking of the NMDA receptor channel and the anticonvulsive action of the diamondoid of formula (I), (II) or (III).

Protection Against Cerebral Ischemia

Both carotid arteries are occluded in rats for 10 minutes. At the same time the blood pressure is reduced to 60-80 mg Hg by withdrawal of blood (Smith et al., 1984, Acta Neurol. Scand. 69: 385, 401). The ischemia is terminated by opening the carotids and reinfusion of the withdrawn blood. After seven days the brains of the test animals are histologically examined for cellular changes in the CA1-CA4 region of the hippocampus, and the percentage of destroyed neurons is determined. The action of the candidate diamondoid derivative is determined after a single administration of 5 mg/kg and 20 mg/kg one (1) hour prior to the ischemia.

**Example 27:**

**Treatment of Alzheimer's Disease**

The patient of this example is an 80 year old female patient, presenting with Alzheimer's Disease. Upon evaluation, she is administered tablets of a diamantane derivative at a dosage of 100 mg twice a day. After about two weeks of administration, her memory improves and she is able to accomplish household functions without assistanc

**Example 28:**

**Treatment of Stroke**

The patients of this example is a 50 year old male patient presenting at the hospital with symptoms indicating a stroke, including numbness and weakness on the left side of his body, trouble seeing and severe headache. He is parenterally administered a triamantane derivative. After two days, the symptoms of the stroke are abating and the patient exhibits greater recovery and freedom of movement than if he had not been given the triamantane derivative.

**References**

(1). P. A. Cahill, Tetra. Lett. 31 (38), pp. 5417-5420 (1990).

(2). P. Kovacic, C. T. Goralski, J. J. Hiller, J. A. Levisky, R. M. Lange, J. Amer. Chem. Soc. 87 (6), pp. 1262-1266 (1965).

(3). P. Kovacic, P. D. Roskos, J. Amer. Chem. Soc. 91 (23), pp. 6457-6460 (1969).

(4). P. Kovacic. P. D. Roskos, Tetra. Lett. (56) pp. 5833-5835 (1968).

While the present invention has been described with reference to specific embodiments, this application is intended to cover those various changes and substitutions that may be made by those of ordinary skill in the art without departing from the spirit and scope of the appended claims.

## Claims

1. A compound of Formula I:

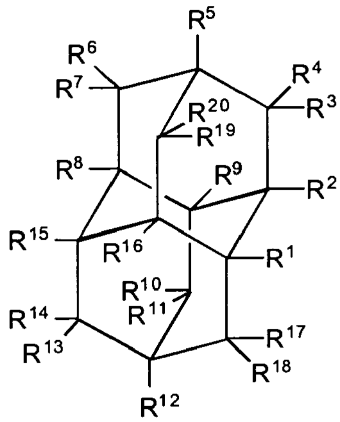

Formula I

wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;

provided that at least two of $R^1$, $R^2$, $R^5$, $R^8$, $R^9$, $R^{12}$, $R^{15}$, and $R^{16}$ are not hydrogen; and

that both $R^5$ and $R^{12}$ or $R^1$ and $R^8$ are not identical when the remaining of $R^1$, $R^2$, $R^8$, $R^9$, $R^{15}$, and $R^{16}$ are hydrogen;
and pharmaceutically acceptable salts thereof.

2. A compound of Formula I:

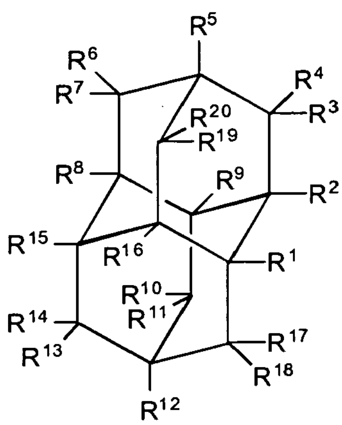

Formula I

wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;

provided that at least two of $R^1$, $R^2$, $R^5$, $R^8$, $R^9$, $R^{12}$, $R^{15}$, and $R^{16}$ are not hydrogen; and

that both $R^5$ and $R^{12}$ are not identical when $R^1$, $R^2$, $R^8$, $R^9$, $R^{15}$ and $R^{16}$ are hydrogen; and
that both $R^1$ and $R^8$ are not identical when $R^2$, $R^5$, $k^9$, $R^{12}$, $R^{15}$ and $R^{16}$ are hydrogen;
and pharmaceutically acceptable salts thereof.

**3.** The compound of claim 1 or 2, wherein at least three of $R^1$, $R^2$, $R^5$, $R^8$, $R^9$, $R^{12}$, $R^{15}$, and $R^{16}$ are not hydrogen.

**4.** The compound of claim 1 or 2, wherein at least four of $R^1$, $R^2$, $R^5$, $R^8$, $R^9$, $R^{12}$, $R^{15}$, and $R^{16}$ are not hydrogen.

**5.** The compound of claim 1 or 2, wherein five of $R^1$, $R^2$, $R^5$, $R^8$, $R^9$, $R^{12}$, $R^{15}$, and $R^{16}$ are not hydrogen.

**6.** The compound of claim 1 or 2, wherein $R^1$ and $R^5$ are aminoacyl and $R^2$, $R^8$, $R^9$, $R^{12}$, $R^{15}$, and $R^{16}$ are hydrogen or lower alkyl.

**7.** The compound of claim 1 or 2, wherein $R^5$ is amino and two of $R^1$, $R^2$, $R^8$ and $R^{15}$ are lower alkyl.

**8.** The compound of claim 7, wherein $R^1$ and $R^8$ are methyl.

**9.** The compound of claim 1 or 2, wherein $R^1$ and $R^{15}$ are methyl.

**10.** The compound of claim 1 or 2, wherein $R^9$ or $R^{15}$ is amino and $R^1$ is methyl.

**11.** The compound of claim 1 or 2, wherein $R^2$ or $R^{16}$ is amino and $R^1$ and $R^8$ are methyl.

**12.** The compound of claim 1 or 2, wherein at least one of $R^1$, $R^2$, $R^5$, $R^8$, $R^9$, $R^{12}$, $R^{15}$, and $R^{16}$ is independently selected from the group consisting of amino, nitroso, nitro, and aminoacyl and at least one of the remaining of $R^1$, $R^2$, $R^5$, $R^8$, $R^9$, $R^{12}$, $R^{15}$, and $R^{16}$ are lower alkyl.

**13.** The compound of claim 12, wherein at least two of the remaining of $R^1$, $R^2$, $R^5$, $R^8$, $R^9$, $R^{12}$, $R^{15}$, and $R^{16}$ are lower alkyl.

**14.** The compound of claim 12, wherein three of the remaining of $R^1$, $R^2$, $R^5$, $R^8$, $R^9$, $R^{12}$, $R^{15}$, and $R^{16}$ are lower alkyl.

**15.** The compound of claim 12, wherein at least one of $R^5$ and $R^{12}$ is independently selected from the group consisting of amino, nitroso, nitro, and aminoacyl and at least one of $R^1$, $R^2$, $R^8$, $R^9$, $R^{15}$, and $R^{16}$ is lower alkyl.

**16.** The compound of claim 15, wherein at least two of $R^1$, $R^2$, $R^8$, $R^9$, $R^{15}$, and $R^{16}$ are lower alkyl.

**17.** The compound of claim 15, wherein three of $R^1$, $R^2$, $R^8$, $R^9$, $R^{15}$, and $R^{16}$ are lower alkyl.

**18.** The compound of claim 1 or 2, wherein at least one of $R^1$, $R^2$, $R^5$, $R^8$, $R^9$, $R^{12}$, $R^{15}$, and $R^{16}$ is substituted lower alkyl.

**19.** The compound of claim 18, wherein two of $R^1$, $R^2$, $R^5$, $R^8$, $R^9$, $R^{12}$, $R^{15}$, and $R^{16}$ are substituted lower alkyl.

**20.** The compound of claim 1 or 2, wherein at least one of $R^1$, $R^2$, $R^5$, $R^8$, $R^9$, $R^{12}$, $R^{15}$, and $R^{16}$ is substituted lower alkyl and at least one of the remaining of $R^1$, $R^2$, $R^5$, $R^8$, $R^9$, $R^{12}$, $R^{15}$, and $R^{16}$ are independently selected from the group consisting of amino, nitroso, nitro, and aminoacyl.

**21.** A compound of Formula II:

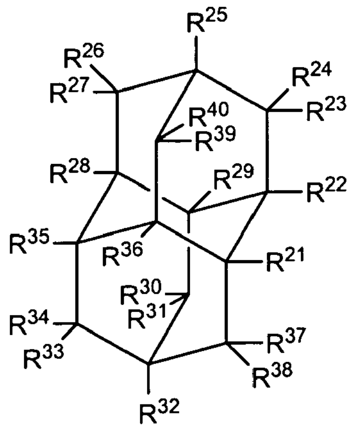

Formula II

wherein:

$R^{21}$, $R^{22}$, $R^{25}$, $R^{28}$, $R^{29}$, $R^{32}$, $R^{35}$, and $R^{36}$ are independently selected from the group consisting of hydrogen or substituted lower alkyl;
$R^{23}$, $R^{24}$, $R^{26}$, $R^{27}$, $R^{30}$, $R^{31}$, $R^{33}$, $R^{34}$, $R^{37}$, $R^{38}$, $R^{39}$, and $R^{40}$ are hydrogen;
provided that at least at least one of $R^{21}$, $R^{22}$, $R^{25}$, $R^{28}$, $R^{29}$, $R^{32}$, $R^{35}$, and $R^{36}$ is substituted lower alkyl;
and pharmaceutically acceptable salts thereof.

**22.** The compound of claim 21, wherein $R^{25}$ is substituted lower alkyl and $R^{21}$, $R^{22}$, $R^{28}$, $R^{29}$, $R^{32}$ $R^{35}$, and $R^{36}$ are hydrogen.

**23.** The compound of claim 21, wherein $R^{25}$ and $R^{32}$ are substituted lower alkyl.

**24.** The compound of claim 21, wherein $R^{21}$ is substituted lower alkyl and $R^{22}$, $R^{25}$, $R^{28}$, $R^{29}$, $R^{32}$, $R^{35}$, and $R^{36}$ are hydrogen.

**25.** The compound of claim 21, wherein $R^{25}$ and $R^{21}$ are substituted lower alkyl.

**26.** The compound of claim 21, wherein $R^{32}$ and $R^{21}$ are substituted lower alkyl.

**27.** The compound of claim 21, wherein the substituted lower alkyl group is substituted with one substitutent selected from the group consisting of amino, hydroxy, halo, nitroso, nitro, carboxy, acyloxy, acyl, aminoacyl, and aminocarbonyloxy.

**28.** The compound of claim 27, wherein the substituted lower alkyl group is substituted with one substitutent selected from the group consisting of amino, nitroso, nitro, and aminoacyl.

**29.** A compound having the structure:

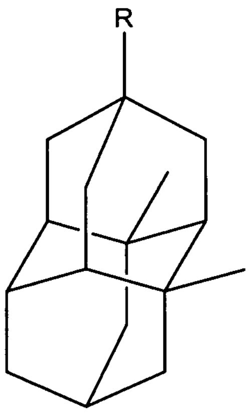
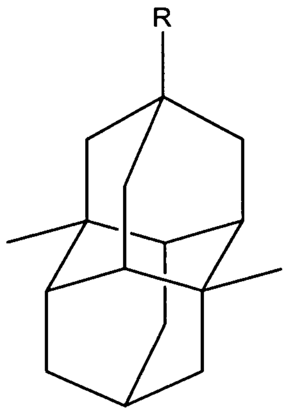
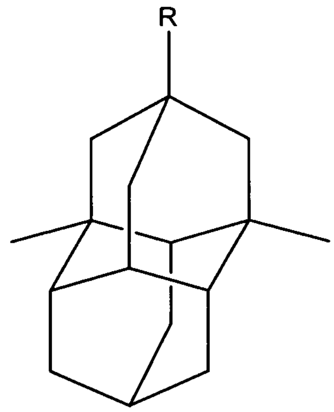

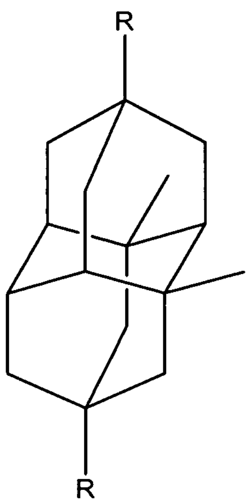
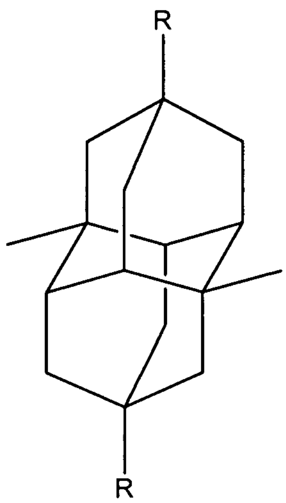
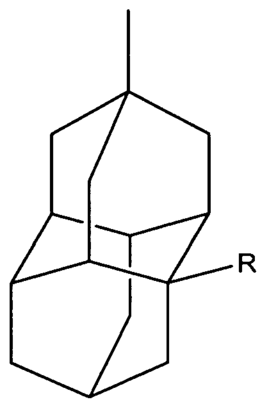
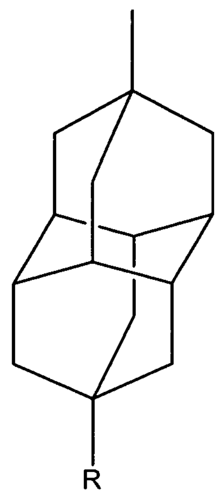

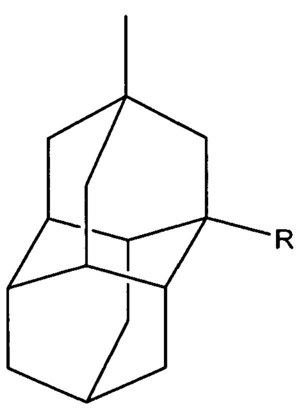
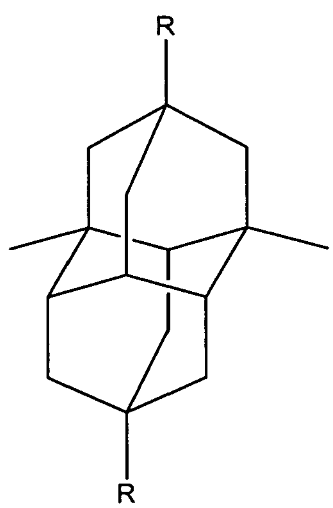

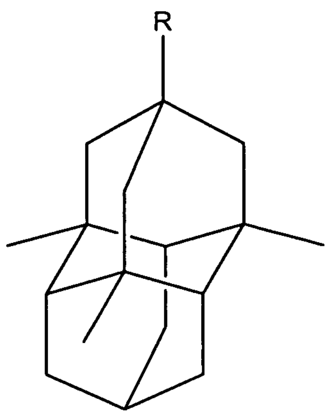
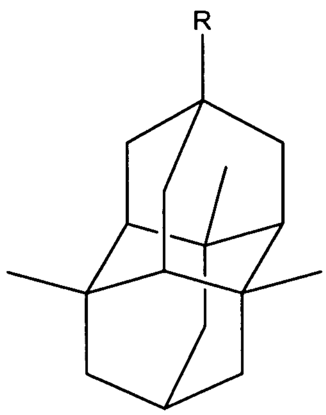
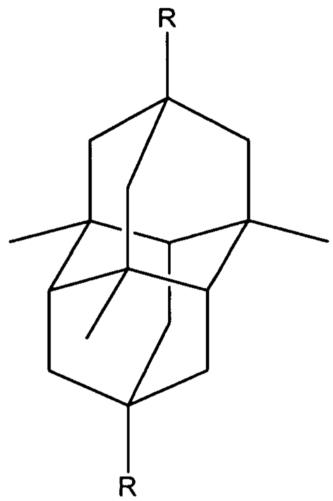

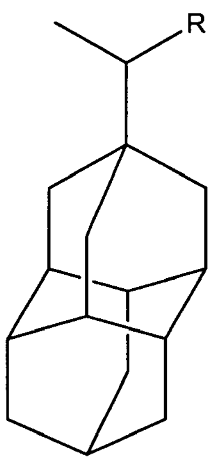
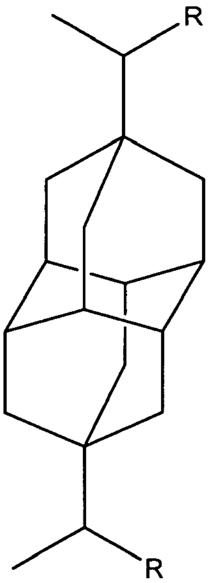
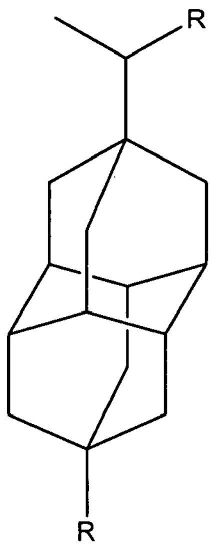
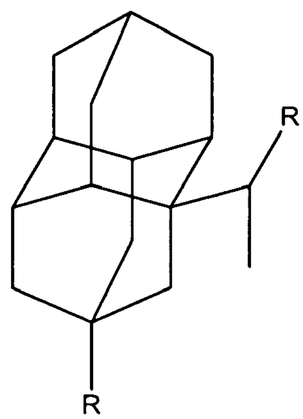

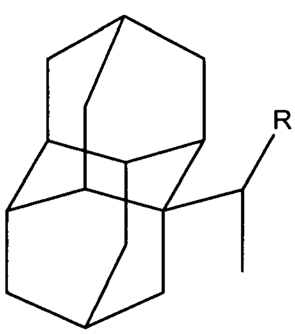
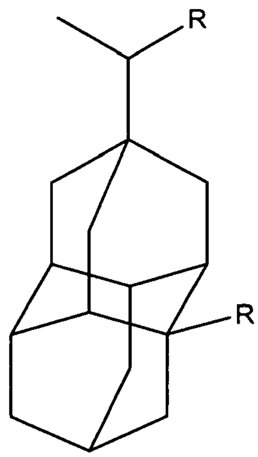
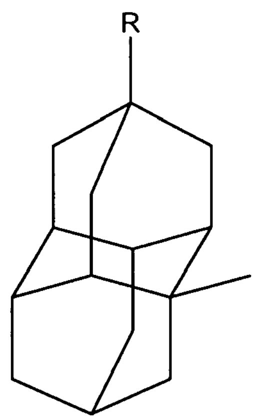

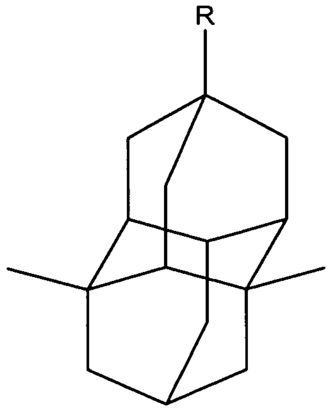 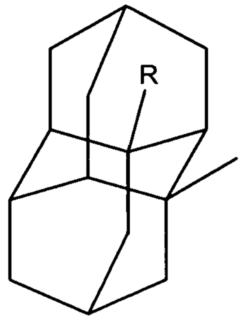 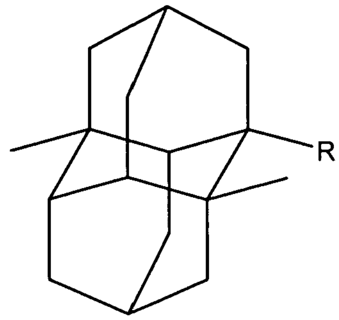

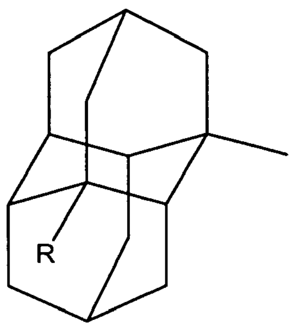 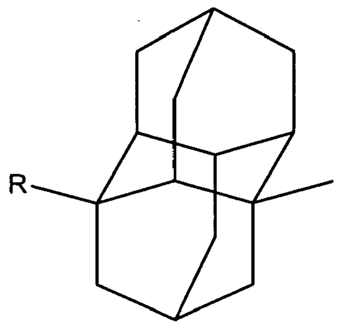 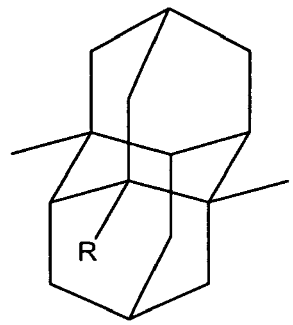

or

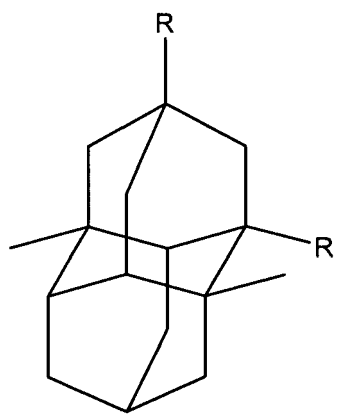

wherein R is independently hydroxy, carboxy, amino, nitroso, nitro or aminoacyl.

**30.** The compound according to claim 29 wherein R is selected from hydroxy, carboxy, amino, nitroso, nitro or aminoacyl.

**31.** A compound selected from the group consisting of 1-methyl-2-aminodiamantane; 1-methyl-4-aminodiamantane; 1-methyl-6-aminodiamantane; 1-methyl-7-aminodiamantane; 1-methyl-9-aminodiamantane; 1-methyl-11-aminodiamantane; 1-methyl-2,4-diaminodiamantane; 1-methyl-4,6-diaminodiamantane; 1-methyl-4,9-diaminodiamantane; 1-amino-2-methyldiamantane; 1-amino-4-methyldiamantane; 2-amino-4-methyldiamantane; 4-methyl-9-aminodiamantane; 1,6-dimethyl-2-aminodiamantane; 1,6-dimethyl-4-aminodiamantane; 1,6-dimethyl-12-aminodiamantane; 1,6-dimethyl-2,4-diaminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 4,9-dimethyl-1-aminodiamantane; 4,9-dimethyl-1-diamantanecarboxylic acid; 4,9-dimethyl-1,6-diaminodiamantane; 1,7-dimethyl-4-aminodiamantane; 1,4-diacetaminodiamantane; 1,7-dihydroxydiamantane; 4-nitrosodiamantane; 6-bromo-1-aminodiamantane; 1-aminomethyl-diamantane; 1-(1-aminoethyl)-diamantane; 4-aminomethyl-diamantane; 4-(1-aminoethyl)-diamantane; 1-aminomethyl-4,9-dimethyl-diamantane; 1-(1-aminopropyl)-diamantane; 4-aminomethyl-1,6-dimethyl-diamantane; 4-(1-aminopropyl)-diamantane; 1-(1-aminoethyl)-4,9-dimethyl-diamantane; 4-(1-aminoethyl)-1,6-dimethyl-diamantane; and pharmaceutically acceptable salts thereof.

**32.** A compound of Formula III:

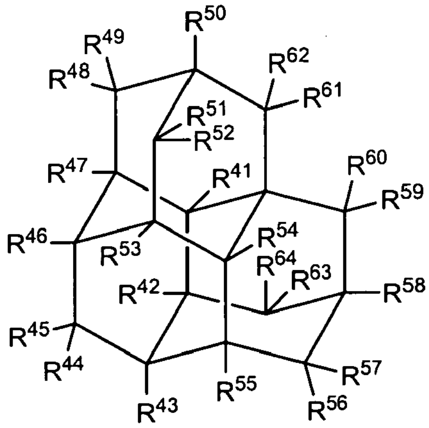

Formula III

wherein:

$R^{41}$, $R^{42}$, $R^{43}$, $R^{46}$, $R^{47}$, $R^{50}$, $R^{53}$, $R^{54}$, $R^{55}$, and $R^{58}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^{44}$, $R^{45}$, $R^{48}$, $R^{49}$, $R^{51}$, $R^{52}$, $R^{56}$, $R^{57}$, $R^{59}$, $R^{60}$, $R^{61}$, $R^{62}$, $R^{63}$, and $R^{64}$ are hydrogen;
provided that at least one of $R^{41}$, $R^{42}$, $R^{43}$, $R^{46}$, $R^{47}$, $R^{50}$, $R^{53}$, $R^{54}$, $R^{55}$, and $R^{58}$ is not hydrogen;
and pharmaceutically acceptable salts thereof.

**33.** The compound of claim 32, wherein at least two of $R^{41}$, $R^{42}$, $R^{43}$, $R^{46}$, $R^{47}$, $R^{50}$, $R^{53}$, $R^{54}$, $R^{55}$, and $R^{58}$ are not hydrogen.

**34.** The compound of claim 32, wherein at least three of $R^{41}$, $R^{42}$, $R^{43}$, $R^{46}$, $R^{47}$, $R^{50}$, $R^{53}$ , $R^{54}$ , $R^{55}$, and $R^{58}$ are not hydrogen.

**35.** The compound of claim 32, wherein $R^{50}$ is selected from the group consisting of amino, nitroso, nitro, and aminoacyl and at least one of $R^{41}$, $R^{42}$, $R^{43}$, $R^{46}$, $R^{47}$, $R^{50}$, $R^{53}$, $R^{54}$, $R^{55}$, and $R^{58}$ is lower alkyl.

**36.** The compound of claim 35, wherein at least two of $R^{41}$, $R^{42}$, $R^{43}$, $R^{46}$, $R^{47}$, $R^{50}$, $R^{53}$, $R^{54}$, $R^{55}$, and $R^{58}$ are lower alkyl.

**37.** The compound of claim 32 which is selected from the group consisting of 2-hydroxytriamantane; 3-hydroxytriamantane; 9-hydroxytriamantane; 9,15-dihydroxytriamantane; 2-aminotriamantane; 3-aminotriamantane; 9-aminotriamantane; 9,15-diaminotriamantane; and pharmaceutically acceptable salts thereof.

**38.** A compound of Formula Ia, or a pharmaceutically acceptable salt thereof, for treating a neurologic disorder in a subject in need thereof:

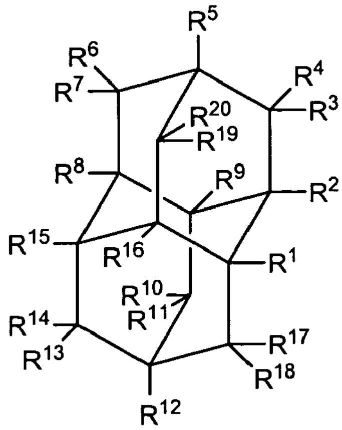

Formula Ia

wherein:

$R^1$, $R^2$, $R^5$, $R^8$, $R^9$, $R^{12}$, $R^{15}$, and $R^{16}$ are independently selected from the group consisting of hydrogen, hydroxy, lower alkyl, substituted lower alkyl, lower alkenyl, alkoxy, amino, nitroso, nitro, halo, cycloalkyl, carboxy, acyloxy, acyl, aminoacyl, and aminocarbonyloxy;
$R^3$, $R^4$, $R^6$, $R^7$, $R^{10}$, $R^{11}$, $R^{13}$, $R^{14}$, $R^{17}$, $R^{18}$, $R^{19}$ and $R^{20}$ are hydrogen;

provided that at least one of $R^1$, $R^2$, $R^5$, $R^8$, $R^9$, $R^{12}$, $R^{15}$, and $R^{16}$ are not hydrogen.

**39.** The compound for use as claimed in claim 40, wherein at least two of $R^1$, $R^2$, $R^5$, $R^8$, $R^9$, $R^{12}$, $R^{15}$, and $R^{16}$ are not hydrogen.

**40.** The compound for use as claimed in claim 38, wherein at least three of $R^1$, $R^2$, $R^5$, $R^8$, $R^9$, $R^{12}$, $R^{15}$, and $R^{16}$ are not hydrogen.

**41.** The compound for use as claimed in claim 38, wherein four of $R^1$, $R^2$, $R^5$, $R^8$, $R^9$, $R^{12}$, $R^{15}$, and $R^{16}$ are not hydrogen.

**42.** The compound for use as claimed in claim 38, wherein $R^1$ and $R^5$ are aminoacyl and $R^2$, $R^8$, $R^9$, $R^{12}$, $R^{15}$, and $R^{16}$ are hydrogen or lower alkyl.

**43.** The compound for use as claimed in claim 38, wherein $R^5$ is amino and two of $R^1$, $R^2$, $R^8$ and $R^{15}$ are lower alkyl.

**44.** The compound for use as claimed in claim 43, wherein $R^1$ and $R^8$ are methyl.

**45.** The compound for use as claimed in claim 43, wherein $R^1$ and $R^{15}$ are methyl.

**46.** The compound for use as claimed in claim 38, wherein $R^9$ or $R^{15}$ is amino and $R^1$ is methyl.

**47.** The compound for use as claimed in claim 38, wherein $R^2$ is amino, $R^1$ is methyl, and $R^8$ or $R^{15}$ is methyl.

**48.** The compound for use as claimed in claim 38, wherein at least one of $R^1$, $R^2$, $R^5$, $R^8$, $R^9$, $R^{12}$, $R^{15}$, and $R^{16}$ is independently selected from the group consisting of amino, nitroso, nitro, and aminoacyl and at least one of the remaining of $R^1$, $R^2$, $R^5$, $R^8$, $R^9$, $R^{12}$, $R^{15}$, and $R^{16}$ are lower alkyl.

**49.** The compound for use as claimed in claim 48, wherein at least two of the remaining of $R^1$, $R^2$, $R^5$, $R^8$, $R^9$, $R^{12}$, $R^{15}$, and $R^{16}$ are lower alkyl.

**50.** The compound for use as claimed in claim 48, wherein three of the remaining of $R^1$, $R^2$, $R^5$, $R^8$, $R^9$, $R^{12}$, $R^{15}$, and $R^{16}$ are lower alkyl.

**51.** The compound for use as claimed in claim 48, wherein at least one of $R^5$ and $R^{12}$ is independently selected from the group consisting of amino, nitroso, nitro, and aminoacyl and at least one of $R^1$, $R^2$, $R^8$, $R^9$, $R^{15}$, and $R^{16}$ is lower

alkyl.

**52.** The compound for use as claimed in claim 51, wherein at least two of $R^1$, $R^2$ $R^8$, $R^9$, $R^{15}$, and $R^{16}$ are lower alkyl.

**53.** The compound for use as claimed in claim 51, wherein three of $R^1$, $R^2$, $R^8$, $R^9$, $R^{15}$, and $R^{16}$ are lower alkyl.

**54.** The compound for use as claimed in claim 38, wherein at least one of $R^1$, $R^2$, $R^5$, $R^8$, $R^9$, $R^{12}$, $R^{15}$, and $R^{16}$ is substituted lower alkyl.

**55.** The compound for use as claimed in claim 54, wherein two of $R^1$, $R^2$, $R^5$, $R^8$, $R^9$, $R^{12}$, $R^{15}$, and $R^{16}$ are substituted lower alkyl.

**56.** The compound for use as claimed in claim 38, wherein at least one of $R^1$, $R^2$, $R^5$, $R^8$, $R^9$, $R^{12}$, $R^{15}$, and $R^{16}$ is substituted lower alkyl and at least one of the remaining of $R^1$, $R^2$, $R^5$, $R^8$, $R^9$, $R^{12}$, $R^{15}$, and $R^{16}$ are independently selected from the group consisting of amino, nitroso, nitro, and aminoacyl.

**57.** The compound for use as claimed in claim 38, wherein at least one of $R^1$, $R^2$, $R^5$, $R^8$, $R^9$, $R^{12}$, $R^{15}$, and $R^{16}$ is a substituted lower alkyl.

**58.** The compound for use as claimed in claim 57, wherein $R^5$ is substituted lower alkyl and $R^1$, $R^2$, $R^8$, $R^9$, $R^{12}$, $R^{15}$, and $R^{16}$ are hydrogen.

**59.** The compound for use as claimed in claim 57, wherein $R^5$ and $R^{12}$ are substituted lower alkyl.

**60.** The compound for use as claimed in claim 57, wherein $R^1$ is substituted lower alkyl and $R^2$, $R^5$, $R^8$, $R^9$, $R^{12}$, $R^{15}$, and $R^{16}$ are hydrogen.

**61.** The compound for use as claimed in claim 57, wherein $R^5$ and $R^1$ are substituted lower alkyl.

**62.** The compound for use as claimed in claim 57, wherein $R^{12}$ and $R^1$ are substituted lower alkyl.

**63.** The compound for use as claimed in claim 57, wherein the substituted lower alkyl group is substituted with one substitutent selected from the group consisting of amino, hydroxy, halo, nitroso, nitro, carboxy, acyloxy, acyl, aminoacyl, and aminocarbonyloxy.

**64.** The compound for use as claimed in claim 57, wherein the substituted lower alkyl group is substituted with one substitutent selected from the group consisting of amino, nitroso, nitro, and aminoacyl.

**65.** The compound for use as claimed in claim 38, wherein the compound of Formula Ia is selected from the group consisting of 1-aminodiamantane; 4-aminodiamantane; 1,6-diaminodiamantane; 4,9-diaminodiamantane; 1-methyl-2-aminodiamantane; 1-methyl-4-aminodiamantane; 1-methyl-6-aminodiamantane; 1-methyl-7-aminodiamantane; 1-methyl-9-aminodiamantane; 1-methyl-11-aminodiamantane; 1-methyl-2,4-diaminodiamantane; 1-methyl-4,6-diaminodiamantane; 1-methyl-4,9-diaminodiamantane; 1-amino-2-methyldiamantane; 1-amino-4-methyldiamantane; 2-amino-4-methyldiamantane; 4-methyl-9-aminodiamantane; 1,6-dimethyl-2-aminodiamantane; 1,6-dimethyl-4-aminodiamantane; 1,6-dimethyl-12-aminodiamantane; 1,6-dimethyl-2,4-diaminodiamantane; 1,6-dimethyl-2-hydroxydiamantane; 1,6-dimethyl-4-hydroxydiamantane; 1,6-dimethyl-4-diamantanecarboxylic acid; 4,9-dimethyl-1-hydroxydiamantane; 4,9-dimethyl-1-aminodiamantane; 4,9-dimethyl-1-diamantanecarboxylic acid; 4,9-dimethyl-1,6-diaminodiamantane; 1,7-dimethyl-4-aminodiamantane; 1-acetaminodiamantane; 4-acetaminodiamantane; 1,4-diacetaminodiamantane; 1,6-diacetaminodiamantane; 1-hydroxydiamantane; 4-hydroxydiamantane; 1,6-dihydroxydiamantane; 1,7-dihydroxydiamantane; 4,9-dihydroxydiamantane; 1-diamantanecarboxylic acid; sodium 1-diamantanecarboxylate; 4-diamantanecarboxylic acid; 1,6-diamantanedicarboxylic acid; sodium 1,6-diamantanedicarboxylate; 4,9-diamantanedicarboxylic acid; 1-nitrosodiamantane; 4-nitrosodiamantane; 6-bromo-1-aminodiamantane; 1-aminomethyl-diamantane; 1-(1-aminoethyl)-diamantane; 4-aminomethyl-diamantane; 4-(1-aminoethyl)-diamantane; 1-aminomethyl-4,9-dimethyl-diamantane; 1-(1-aminopropyl)-diamantane; 4-aminomethyl-1,6-dimethyl-diamantane; 4-(1-aminopropyl)-diamantane; 1-(1-aminoethyl)-4,9-dimethyl-diamantane; 4-(1-aminoethyl)-1,6-dimethyl-diamantane; and pharmaceutically acceptable salts thereof.

**66.** A pharmaceutical composition for the treatment of a neurologic disorder comprising a pharmaceutically effective

amount of the compound defined in claim 38, and one or more pharmaceutically acceptable excipients or carriers.

**67.** A compound of Formula III as defined in any one of claims 32-37, or a pharmaceutically acceptable salt thereof, for use in a method for treating a neurologic disorder in a subject in need thereof.

**68.** The compound for use as claimed in claim 38 or 67, wherein the neurologic disorder wherein the neurologic disorder is selected from the group consisting of pain, a neurodegenerative condition, a psychiatric condition, epilepsy, and narcolepsy.

**69.** The compound for use as claimed in claim 68, wherein the pain is neuropathic pain.

**70.** The compound for use as claimed in claim 68, wherein the neurodegenerative condition is selected from the group consisting of Alzheimer's Disease, Parkinson's Disease, stroke, AIDS related dementia, traumatic brain injury (TBI), and Huntington's Disease.

**71.** The compound for use as claimed in claim 68, wherein the psychiatric condition is substance abuse.

**72.** The compound for use as claimed in claim 71, wherein the substance abuse is alcohol abuse or drug abuse.

**73.** The compound for use as claimed in claim 38 or 67, wherein the subject is a mammal.

**74.** The compound for use as claimed in claim 73, wherein the mammal is a human.

**75.** The compound for use as claimed in claim 38 or 67, wherein the compound is administered parenterally.

**76.** A pharmaceutical composition for the treatment of a neurologic disorder comprising a pharmaceutically effective amount of the compound of claim 32, and one or more pharmaceutically acceptable excipients or carriers.

**FIG. 1**

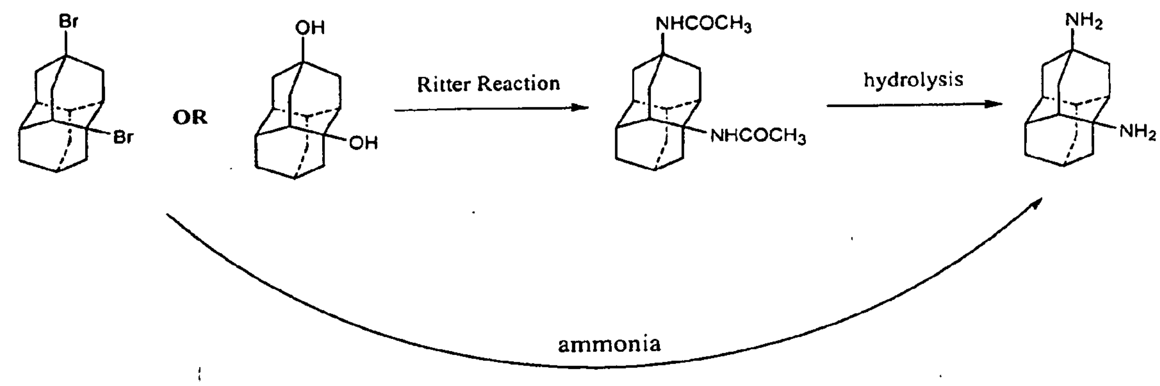
OR
Ritter Reaction
hydrolysis
ammonia

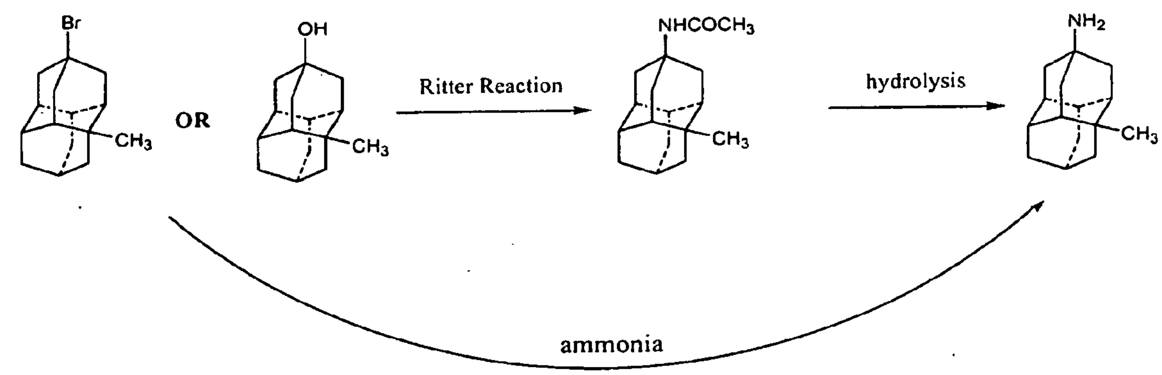
OR
Ritter Reaction
hydrolysis
ammonia

FIG. 2

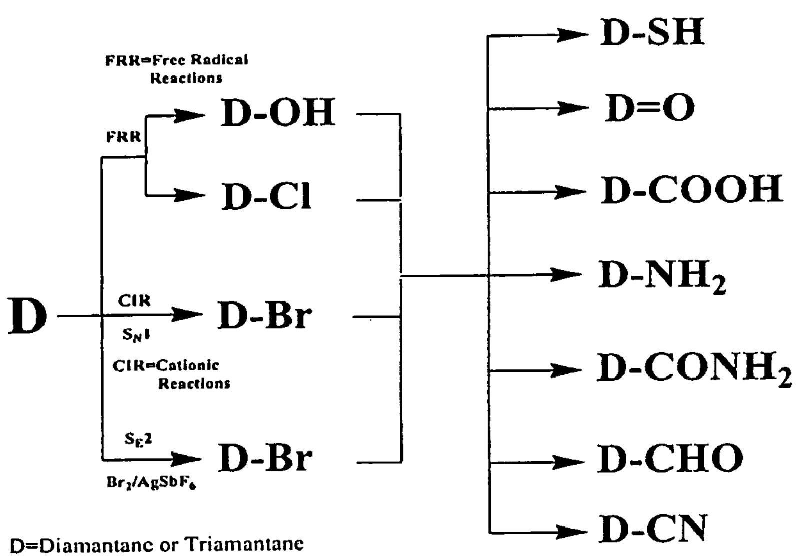
FRR=Free Radical Reactions
FRR
D-OH
D-Cl
D
CIR
$S_N1$
D-Br
CIR=Cationic Reactions
$S_E2$
D-Br
$Br_2/AgSbF_6$
D=Diamantane or Triamantane
D-SH
D=O
D-COOH
$D-NH_2$
$D-CONH_2$
D-CHO
D-CN

# FIG. 3

Representative Ways of Generation of Diamondoid Cations

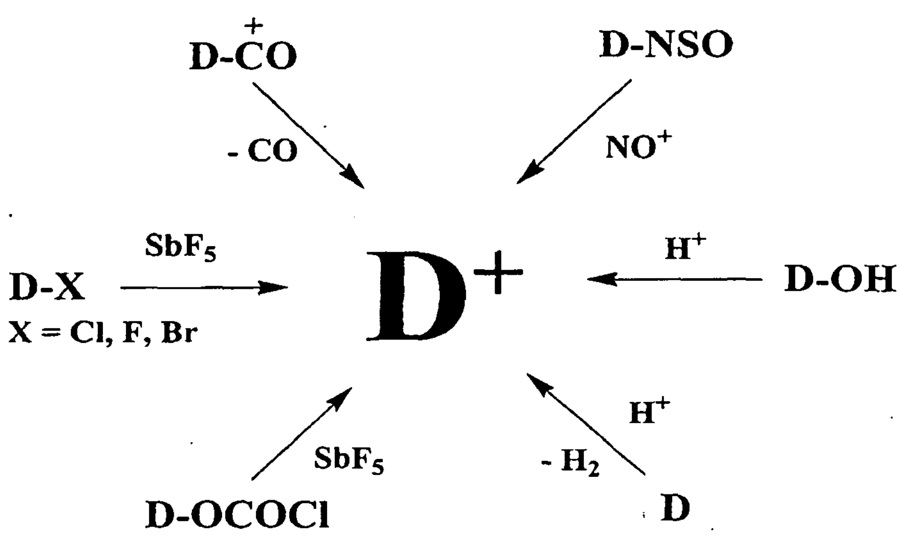

# FIG. 4

**Representative $S_N1$ Reactions of Diamondoid Carbocations**

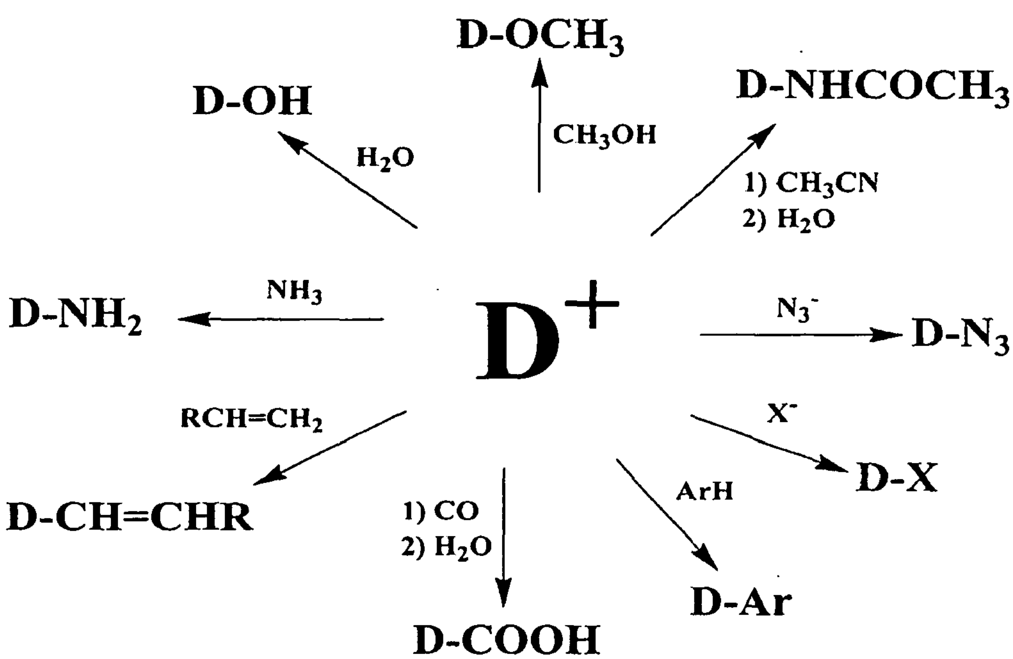

## FIG. 5

**Representative $S_E2$ Reactions of Diamondoids**

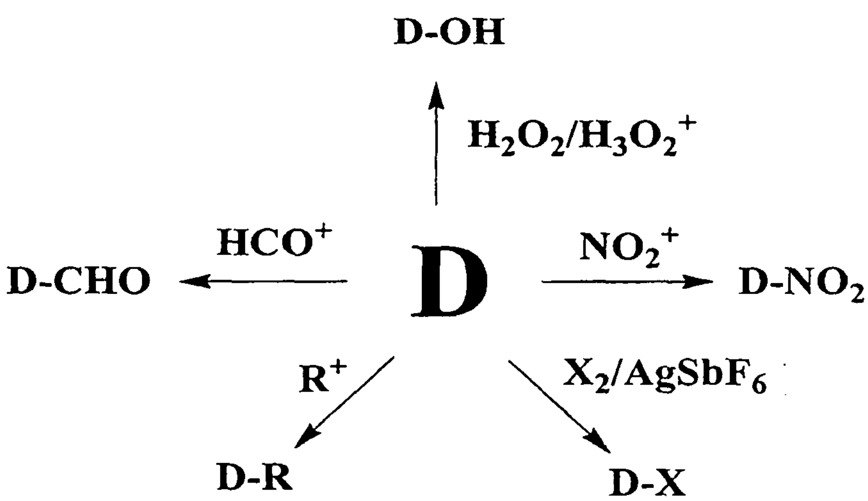

FIG. 6

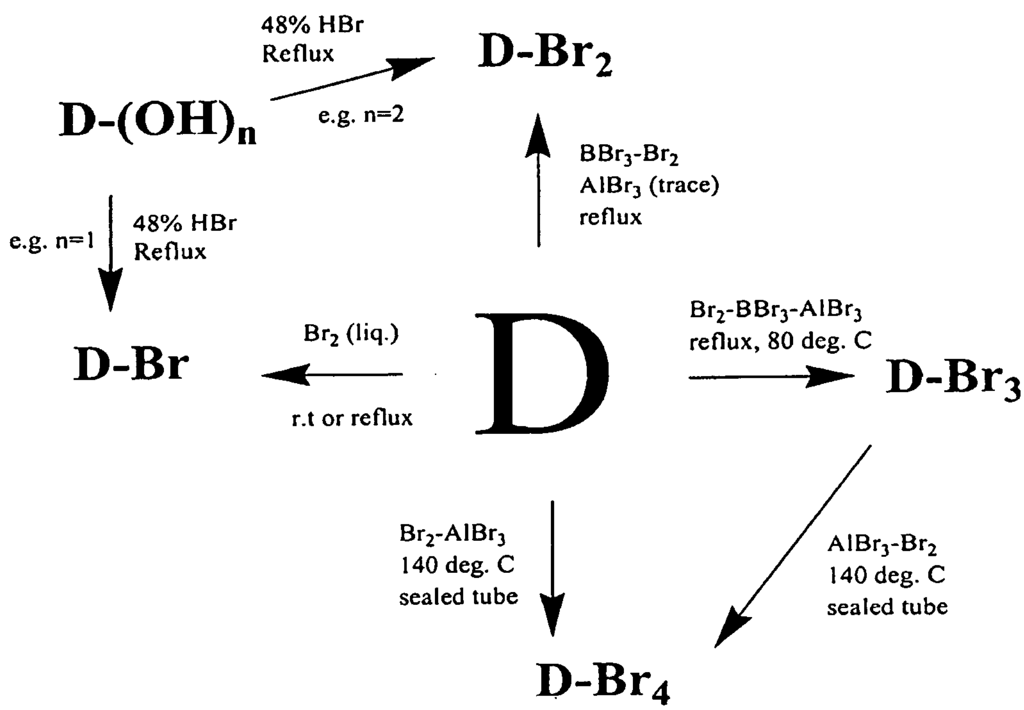
48% HBr
Reflux
e.g. n=2
D-Br2
D-(OH)n
BBr3-Br2
AlBr3 (trace)
reflux
e.g. n=1
48% HBr
Reflux
Br2-BBr3-AlBr3
reflux, 80 deg. C
Br2 (liq.)
D-Br
r.t or reflux
D
D-Br3
Br2-AlBr3
140 deg. C
sealed tube
AlBr3-Br2
140 deg. C
sealed tube
D-Br4

# FIG. 7

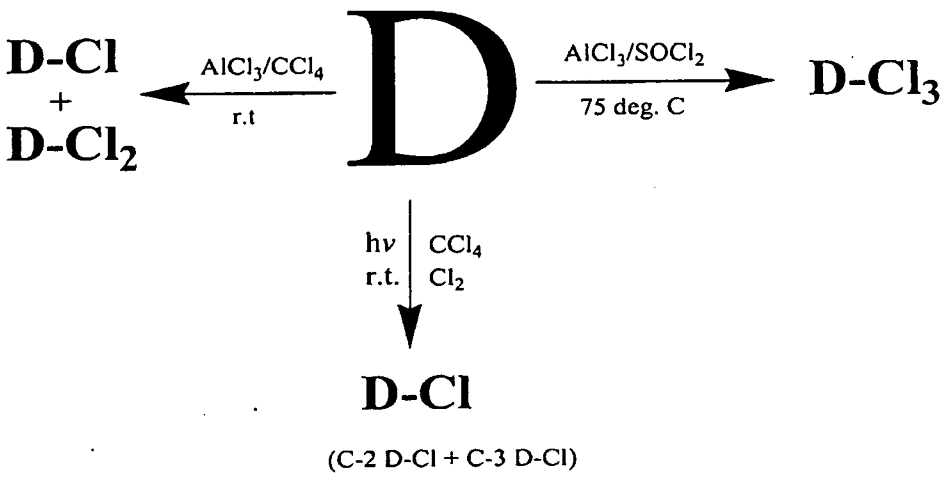
D-Cl
+
D-Cl2
AlCl3/CCl4
r.t
D
AlCl3/SOCl2
75 deg. C
D-Cl3
hν
r.t.
CCl4
Cl2
D-Cl
(C-2 D-Cl + C-3 D-Cl)

# FIG. 8

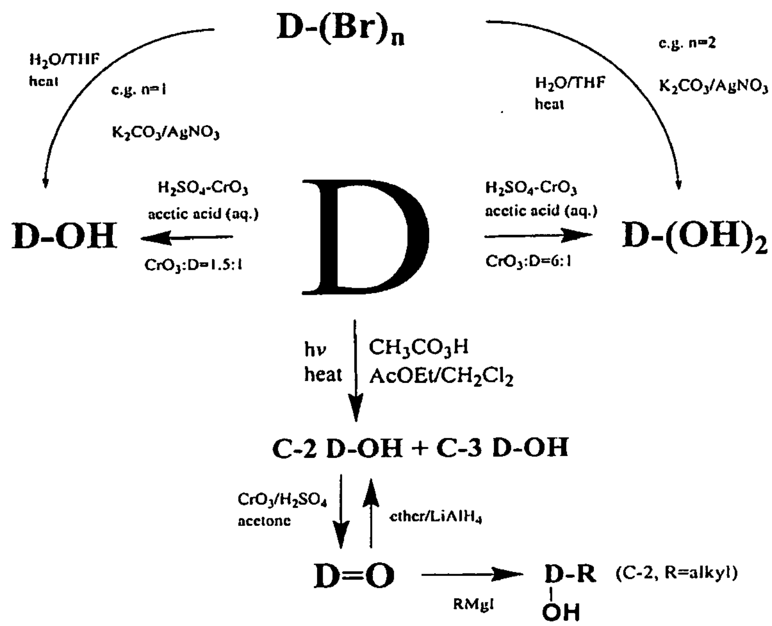

D-(Br)n
H2O/THF
heat
e.g. n=1
K2CO3/AgNO3
e.g. n=2
H2O/THF
heat
K2CO3/AgNO3
H2SO4-CrO3
acetic acid (aq.)
CrO3:D=1.5:1
D-OH
D
H2SO4-CrO3
acetic acid (aq.)
CrO3:D=6:1
D-(OH)2
hν
heat
CH3CO3H
AcOEt/CH2Cl2
C-2 D-OH + C-3 D-OH
CrO3/H2SO4
acetone
ether/LiAlH4
D=O
RMgI
D-R
OH
(C-2, R=alkyl)

# FIG. 9

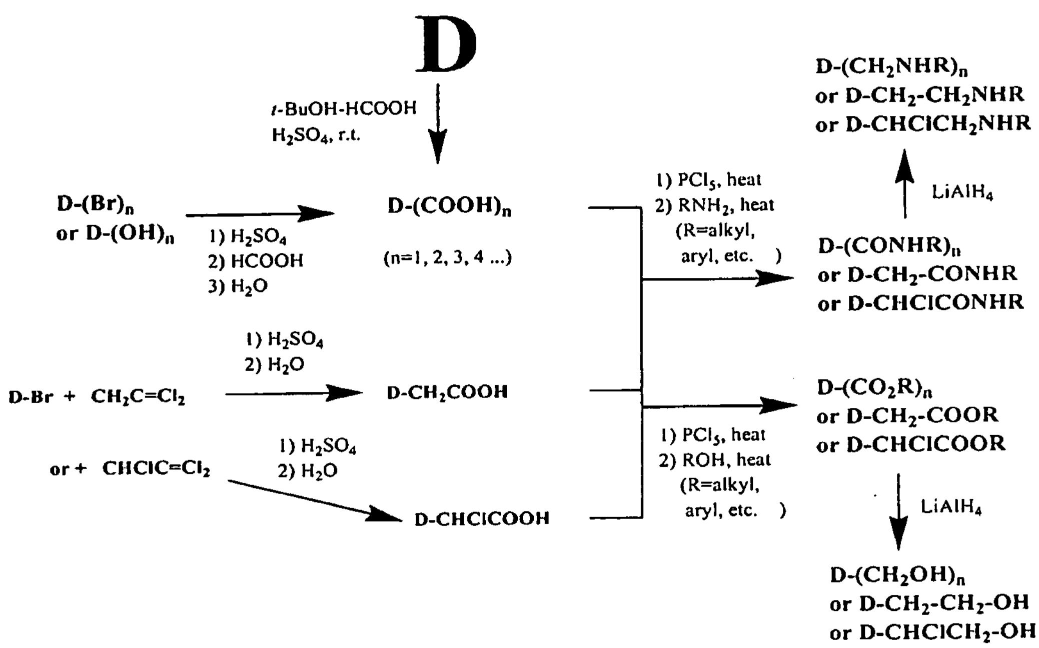
D
t-BuOH-HCOOH
$H_2SO_4$, r.t.
D-(Br)$_n$
or D-(OH)$_n$
1) $H_2SO_4$
2) HCOOH
3) $H_2O$
D-(COOH)$_n$
(n=1, 2, 3, 4 ...)
1) $H_2SO_4$
2) $H_2O$
D-Br + $CH_2C{=}Cl_2$
D-$CH_2COOH$
1) $H_2SO_4$
2) $H_2O$
or + $CHClC{=}Cl_2$
D-CHClCOOH
1) $PCl_5$, heat
2) $RNH_2$, heat
(R=alkyl,
aryl, etc. )
1) $PCl_5$, heat
2) ROH, heat
(R=alkyl,
aryl, etc. )
D-($CH_2$NHR)$_n$
or D-$CH_2$-$CH_2$NHR
or D-CHCl$CH_2$NHR
$LiAlH_4$
D-(CONHR)$_n$
or D-$CH_2$-CONHR
or D-CHClCONHR
D-($CO_2$R)$_n$
or D-$CH_2$-COOR
or D-CHClCOOR
$LiAlH_4$
D-($CH_2$OH)$_n$
or D-$CH_2$-$CH_2$-OH
or D-CHCl$CH_2$-OH

FIG. 10

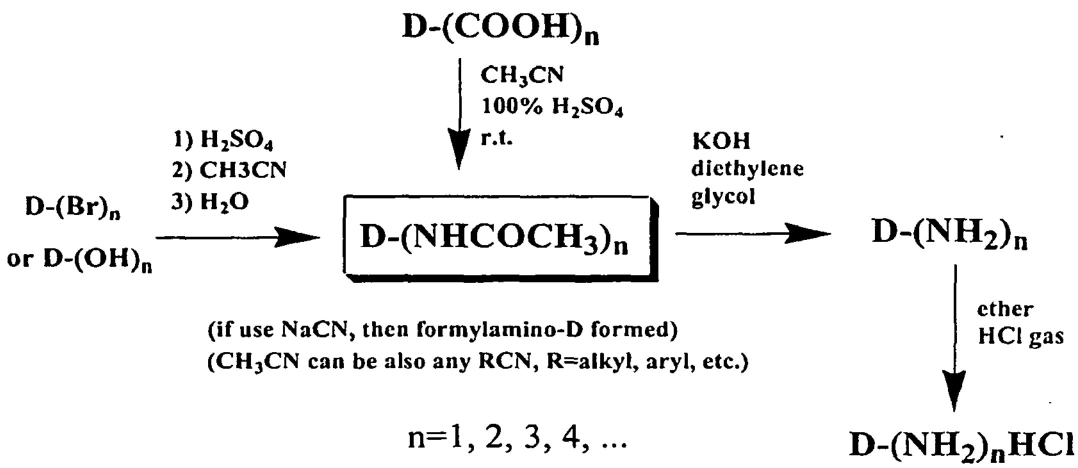
D-(COOH)n
CH3CN
100% H2SO4
r.t.
1) H2SO4
2) CH3CN
3) H2O
D-(Br)n
or D-(OH)n
D-(NHCOCH3)n
KOH
diethylene
glycol
D-(NH2)n
ether
HCl gas
D-(NH2)nHCl
(if use NaCN, then formylamino-D formed)
(CH3CN can be also any RCN, R=alkyl, aryl, etc.)
n=1, 2, 3, 4, ...

# FIG. 11

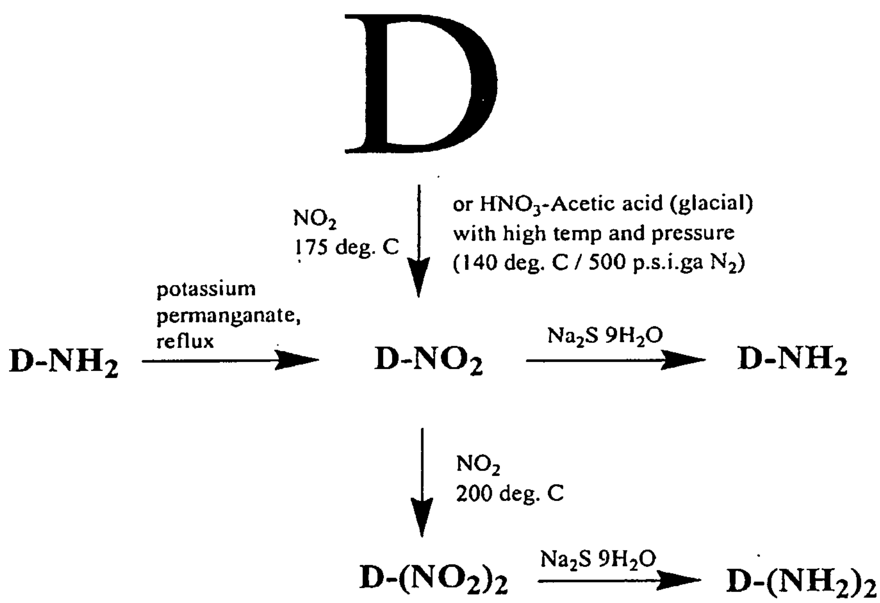
D
$NO_2$
175 deg. C
or $HNO_3$-Acetic acid (glacial)
with high temp and pressure
(140 deg. C / 500 p.s.i.ga $N_2$)
potassium
permanganate,
reflux
$D-NH_2$
$D-NO_2$
$Na_2S$ $9H_2O$
$D-NH_2$
$NO_2$
200 deg. C
$D-(NO_2)_2$
$Na_2S$ $9H_2O$
$D-(NH_2)_2$

# FIG. 12

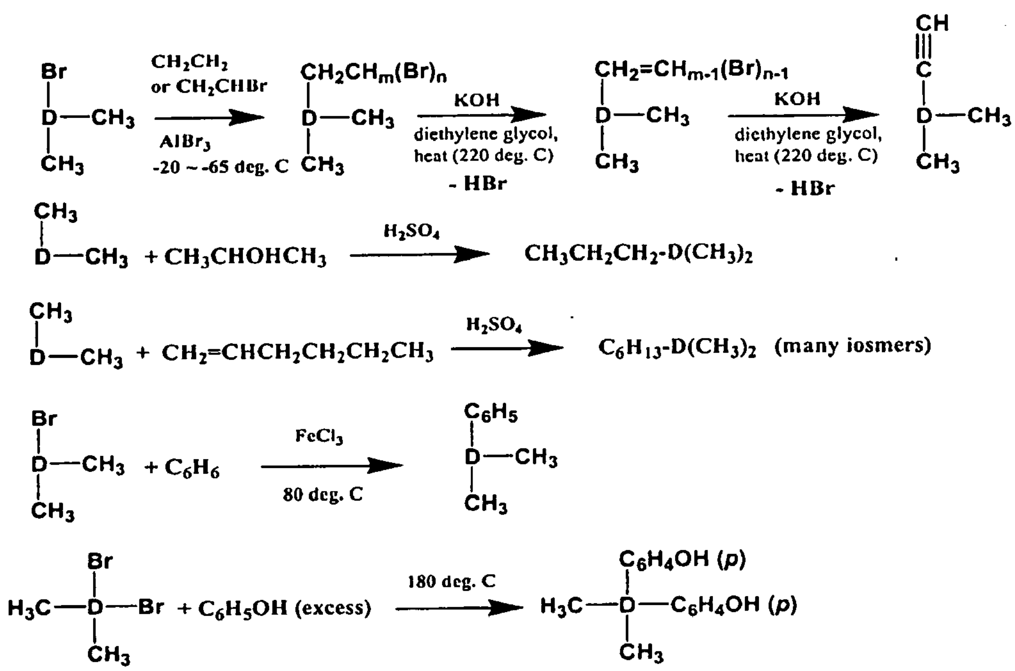
CH2CH2
or CH2CHBr
AlBr3
-20 ~ -65 deg. C
KOH
diethylene glycol,
heat (220 deg. C)
- HBr
KOH
diethylene glycol,
heat (220 deg. C)
- HBr
H2SO4
H2SO4
(many iosmers)
FeCl3
80 deg. C
180 deg. C

# FIG. 13

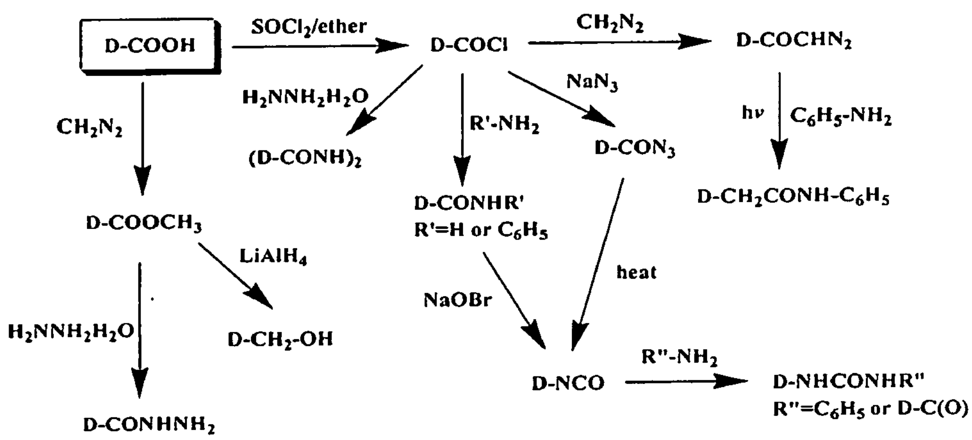
SOCl2/ether
CH2N2
H2NNH2H2O
R'-NH2
NaN3
hν
C6H5-NH2
CH2N2
LiAlH4
H2NNH2H2O
NaOBr
heat
R"-NH2

# FIG. 14

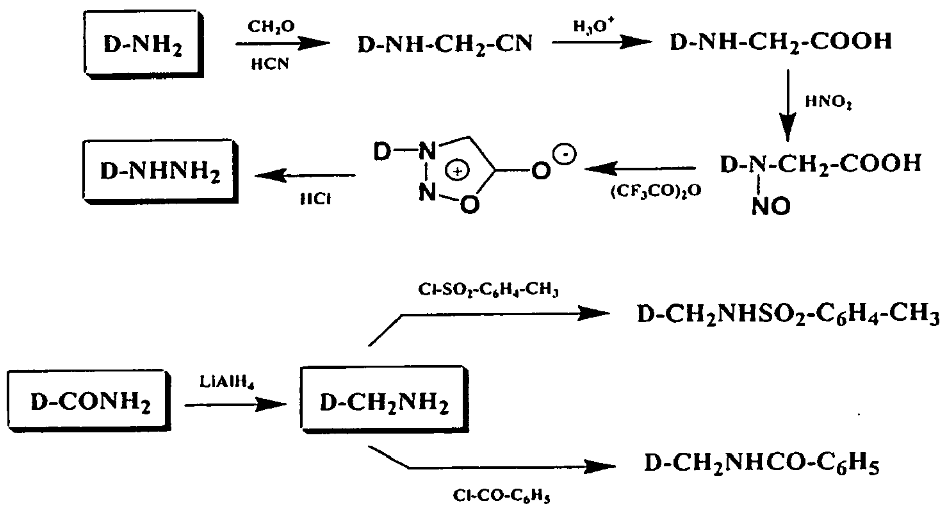
CH2O
HCN
H3O+
HNO2
(CF3CO)2O
HCl
LiAlH4
Cl-SO2-C6H4-CH3
Cl-CO-C6H5

FIG. 15

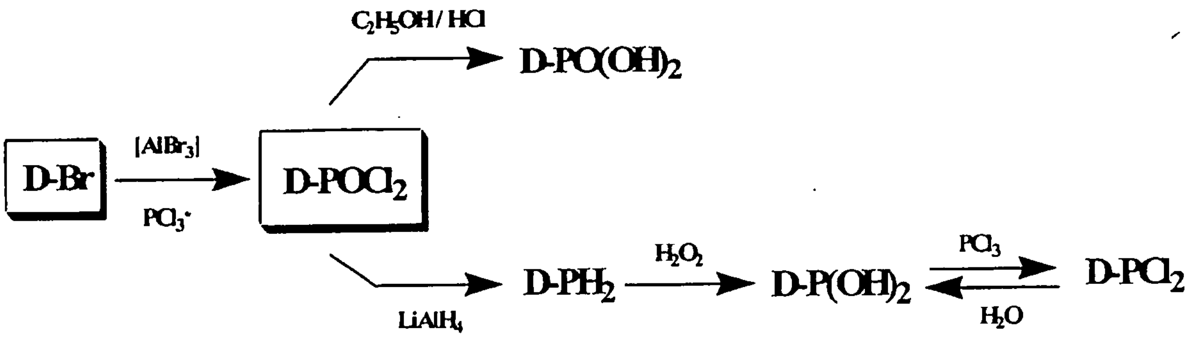
C2H5OH / HCl
D-PO(OH)2
D-Br
[AlBr3]
PCl3
D-POCl2
LiAlH4
D-PH2
H2O2
D-P(OH)2
PCl3
H2O
D-PCl2

# FIG. 16

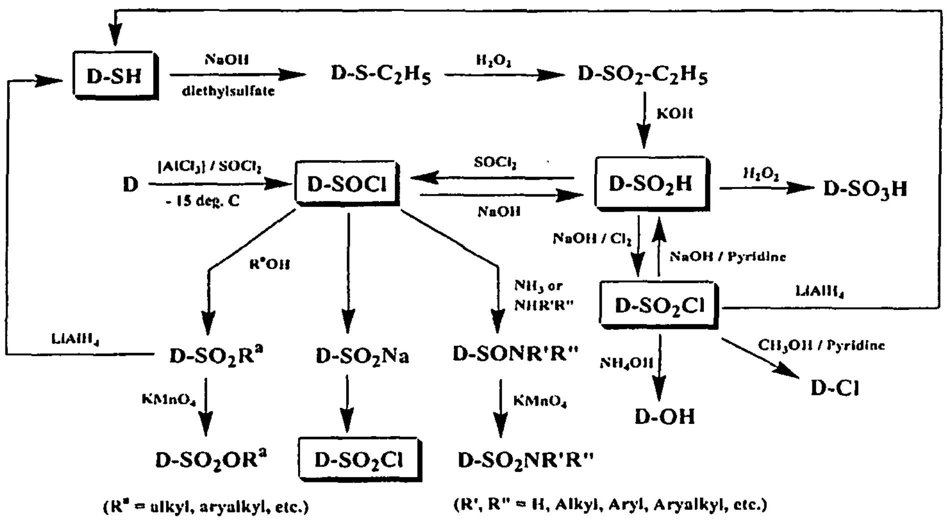
D-SH
NaOH
diethylsulfate
D-S-C2H5
H2O2
D-SO2-C2H5
KOH
D
[AlCl3] / SOCl2
- 15 deg. C
D-SOCl
SOCl2
NaOH
D-SO2H
H2O2
D-SO3H
NaOH / Cl2
NaOH / Pyridine
D-SO2Cl
LiAlH4
R^aOH
NH3 or
NHR'R"
LiAlH4
D-SO2R^a
D-SO2Na
D-SONR'R"
NH4OH
CH3OH / Pyridine
D-Cl
KMnO4
KMnO4
D-OH
D-SO2OR^a
D-SO2Cl
D-SO2NR'R"
(R^a = alkyl, aryalkyl, etc.)
(R', R" = H, Alkyl, Aryl, Aryalkyl, etc.)

FIG. 17

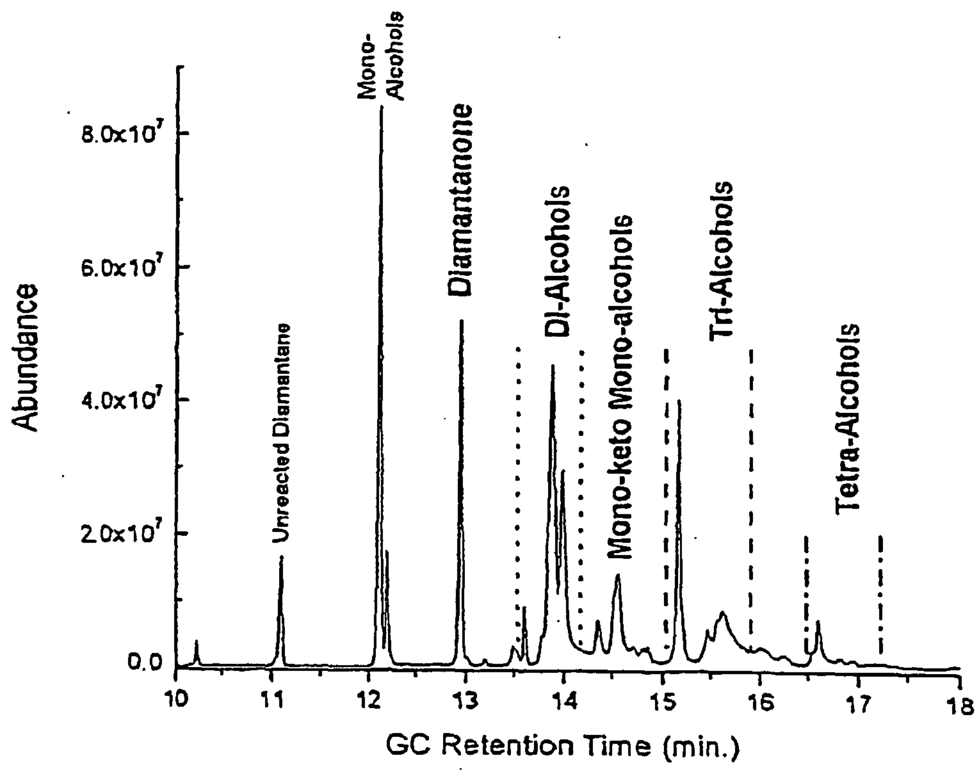
Abundance
8.0x10^7
6.0x10^7
4.0x10^7
2.0x10^7
0.0
Unreacted Diamantane
Mono-Alcohols
Diamantanone
Di-Alcohols
Mono-keto Mono-alcohols
Tri-Alcohols
Tetra-Alcohols
10
11
12
13
14
15
16
17
18
GC Retention Time (min.)

# Fig. 18

Crystal structure of 1,6-dibromodiamantane with atom numbering

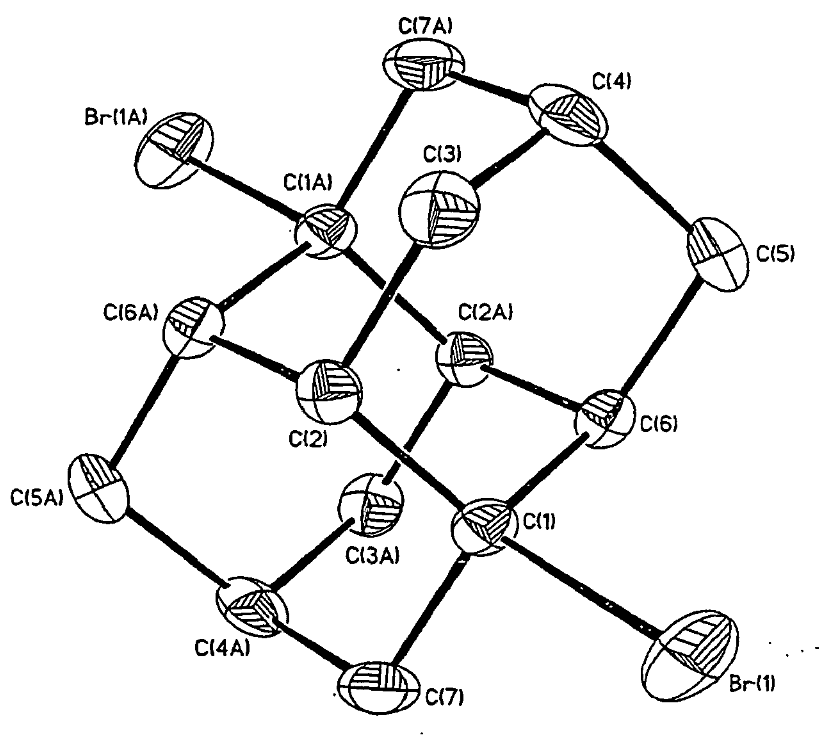

Fig. 19

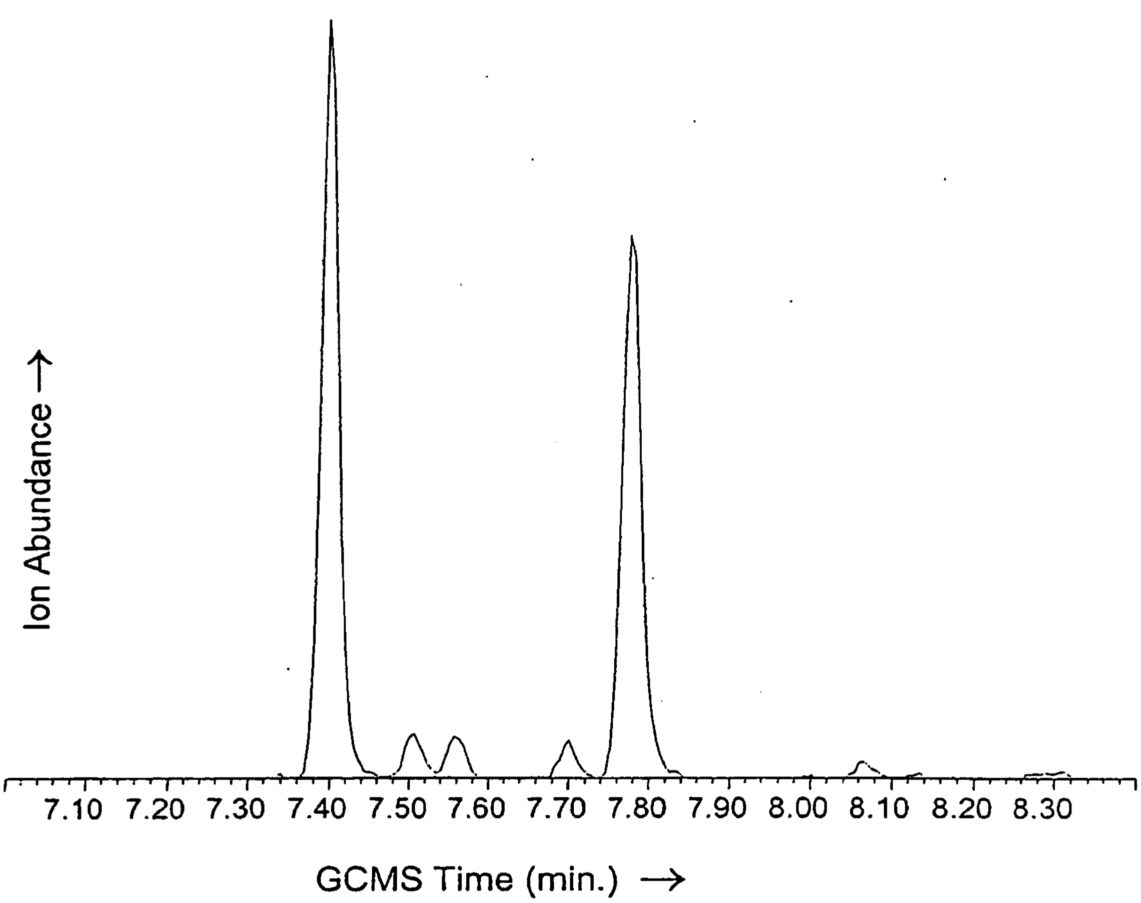
Ion Abundance →
7.10
7.20
7.30
7.40
7.50
7.60
7.70
7.80
7.90
8.00
8.10
8.20
8.30
GCMS Time (min.) →

Fig. 20

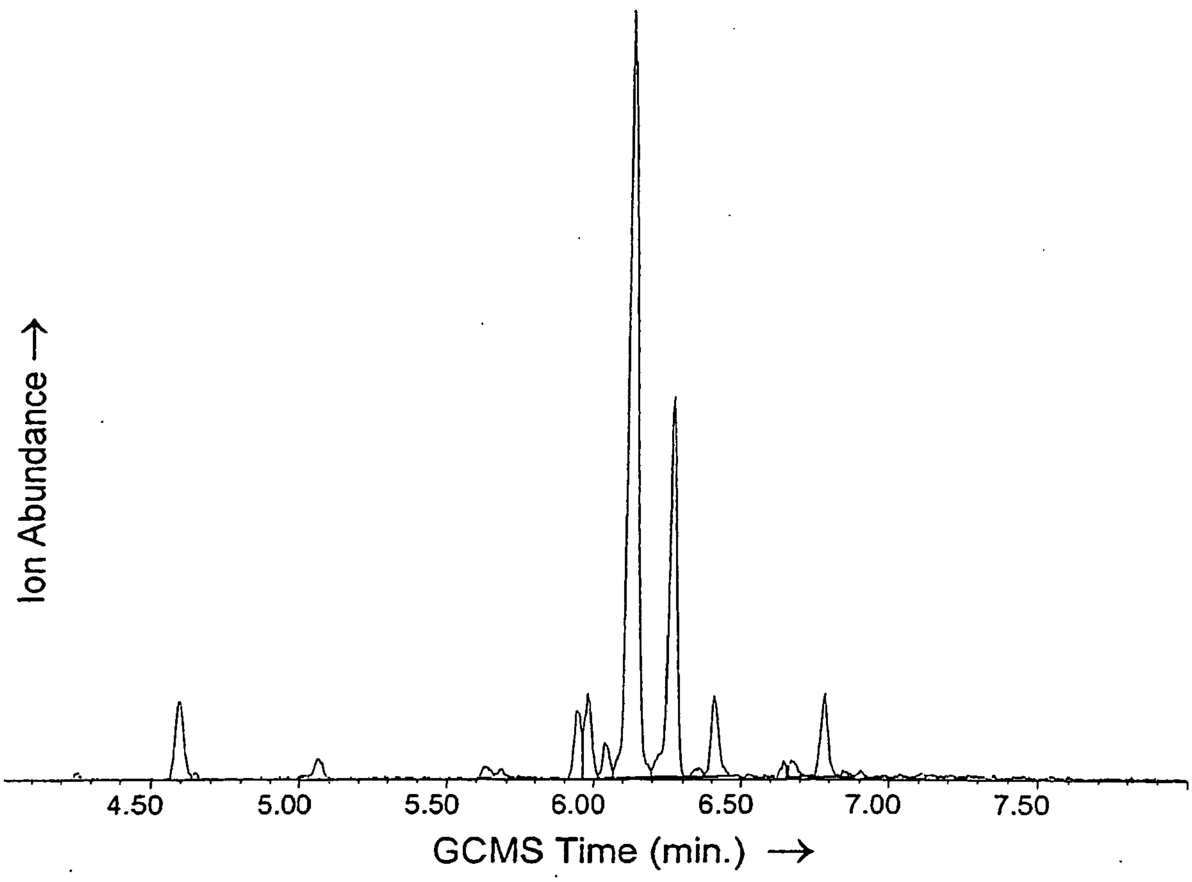

# Fig. 21

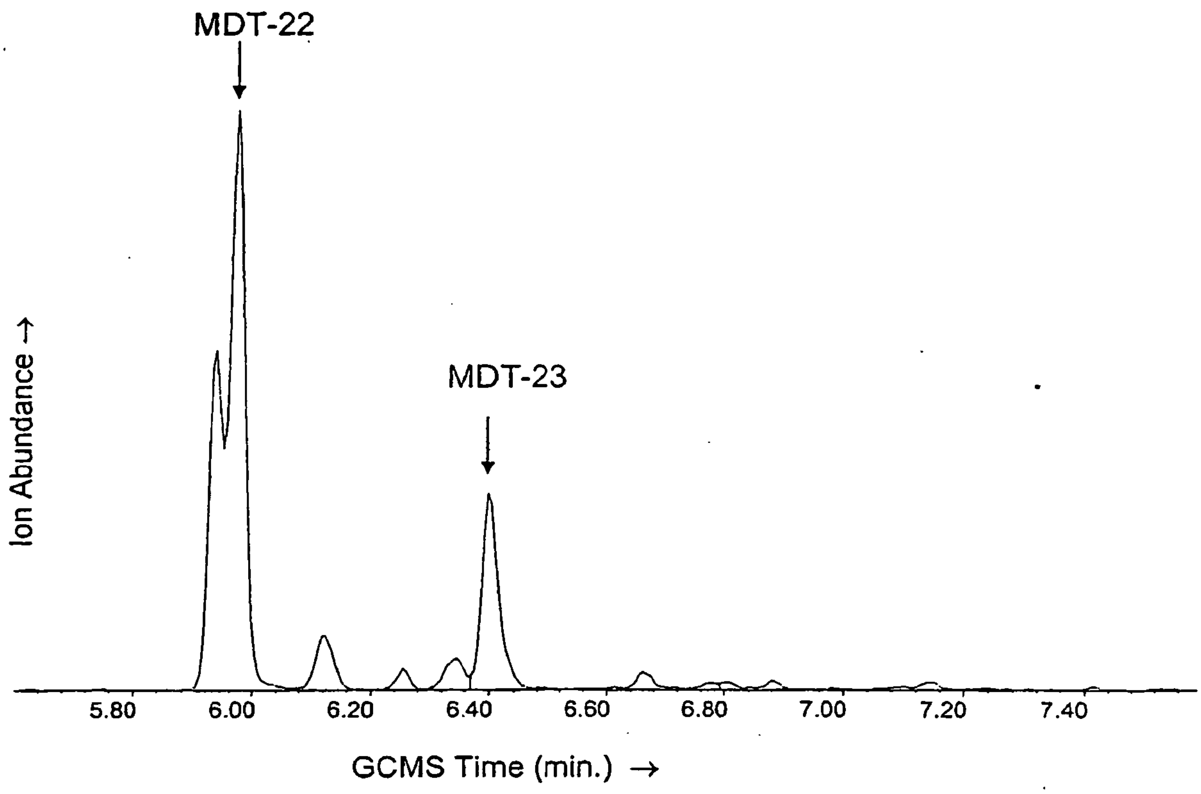
MDT-22
MDT-23
Ion Abundance →
5.80
6.00
6.20
6.40
6.60
6.80
7.00
7.20
7.40
GCMS Time (min.) →

Fig. 22

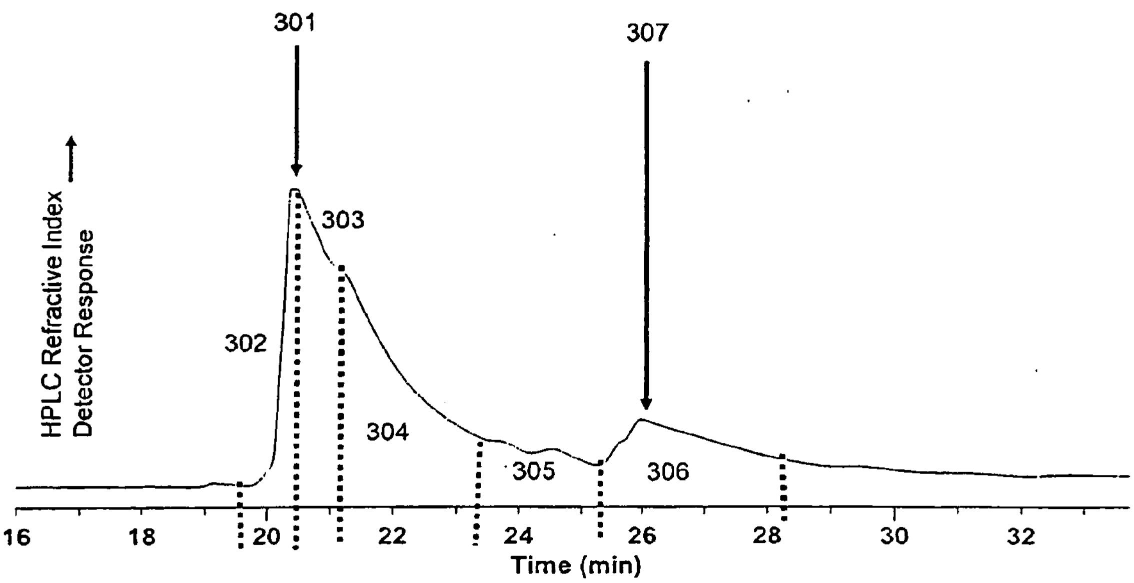
301
307
HPLC Refractive Index Detector Response
302
303
304
305
306
16
18
20
22
24
26
28
30
32
Time (min)

# Fig. 23

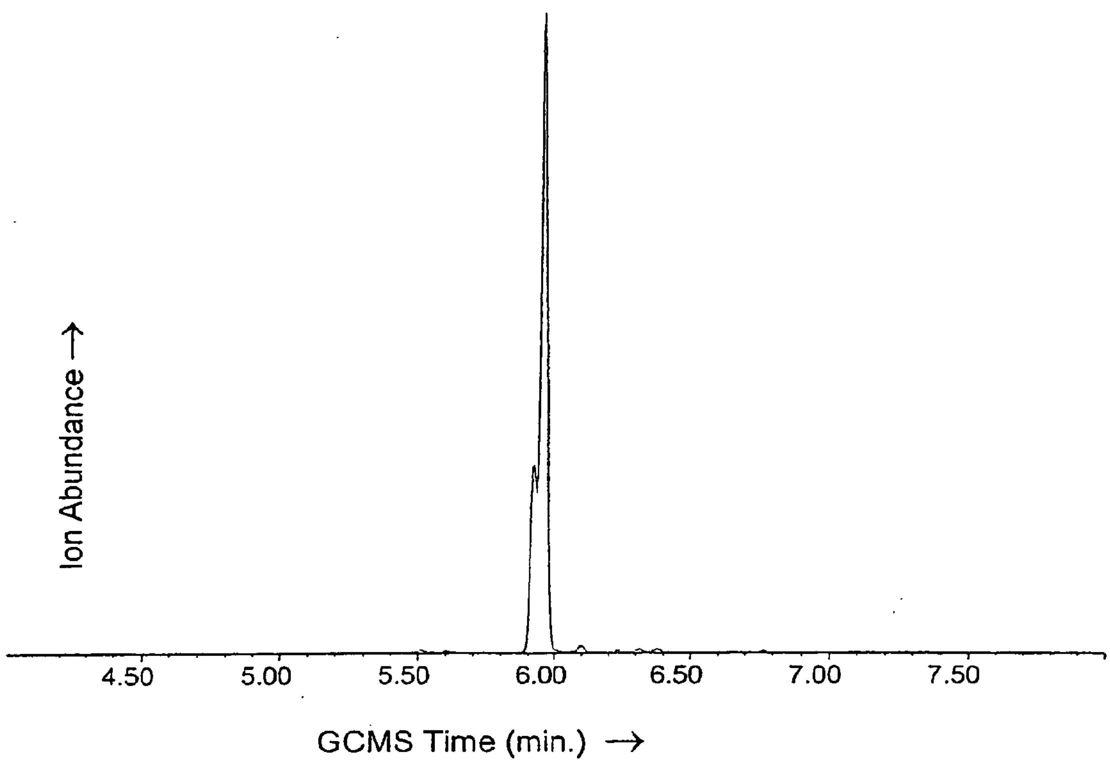

Fig. 24

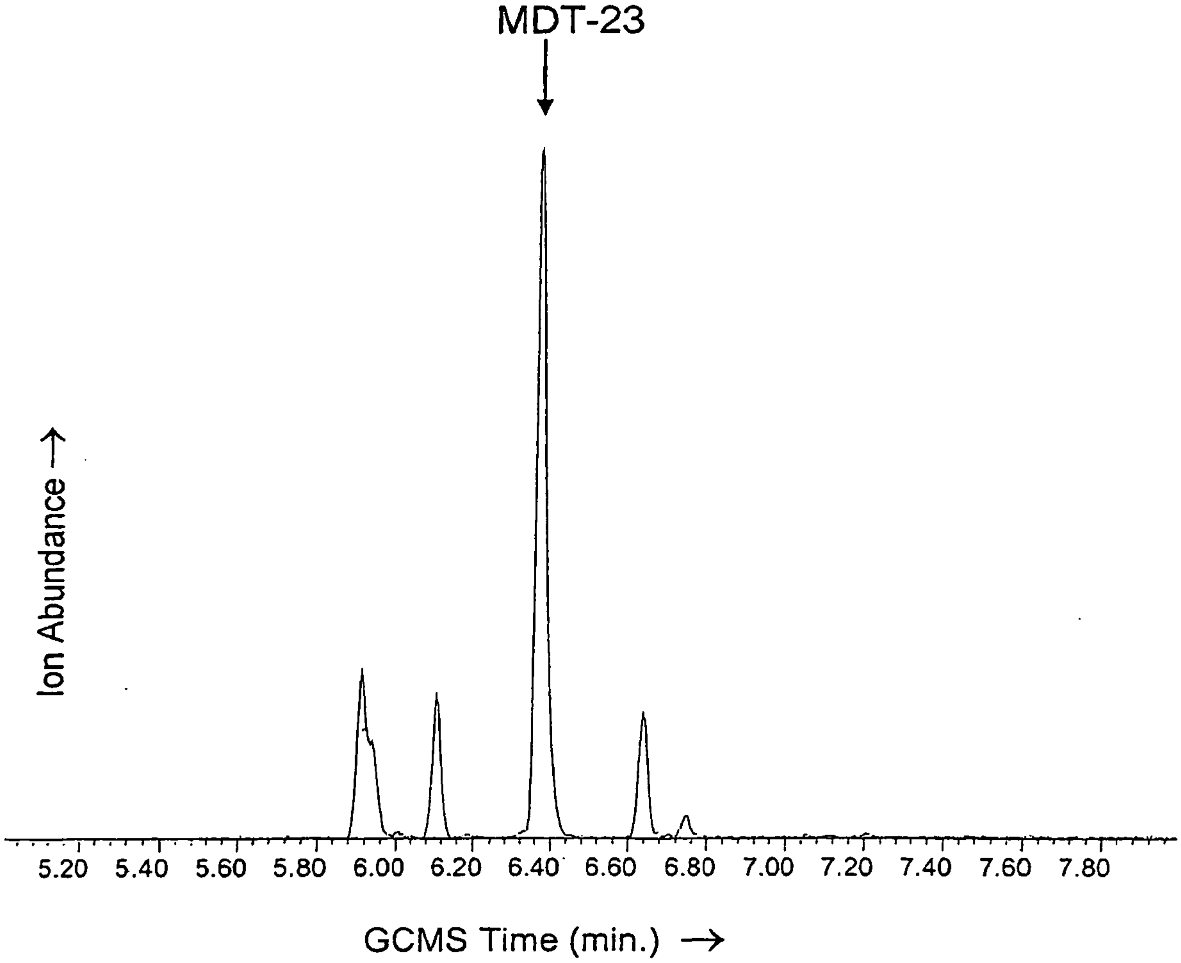
MDT-23
Ion Abundance →
5.20
5.40
5.60
5.80
6.00
6.20
6.40
6.60
6.80
7.00
7.20
7.40
7.60
7.80
GCMS Time (min.) →

Fig. 25

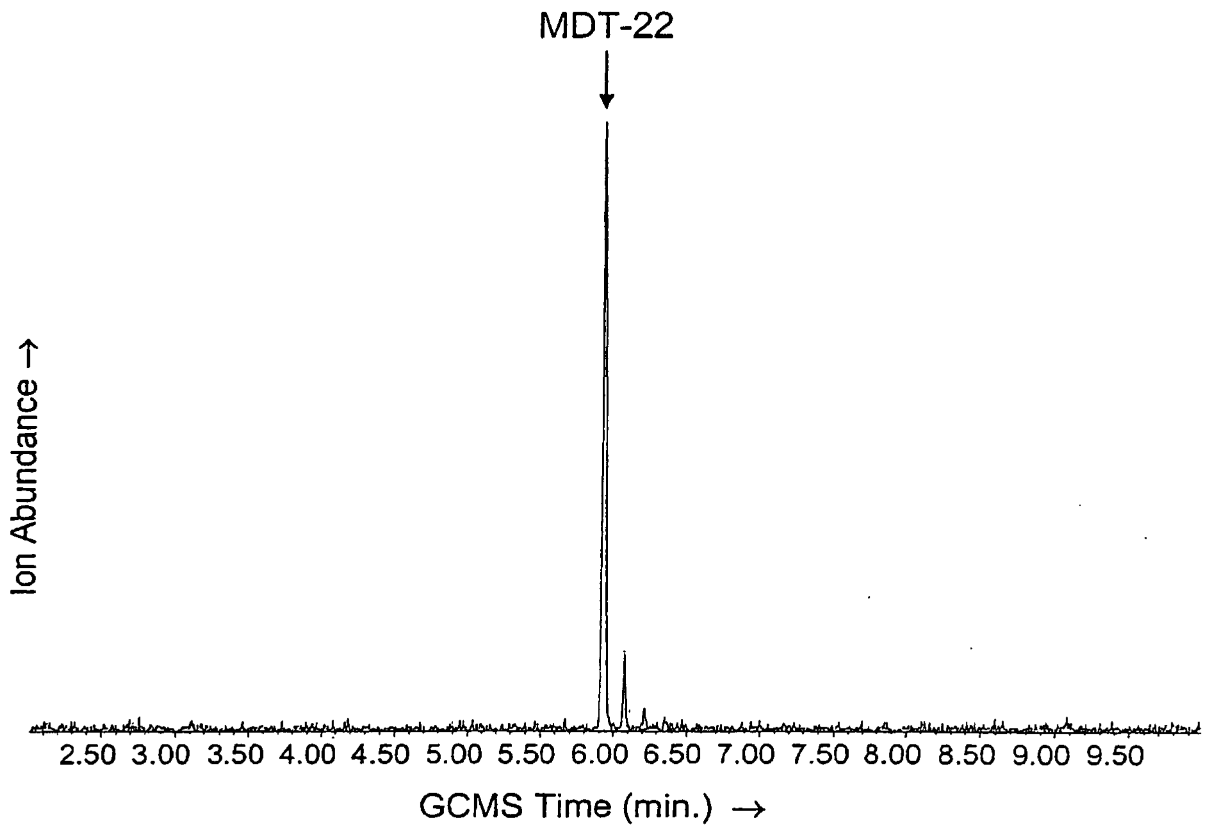
MDT-22
Ion Abundance →
2.50 3.00 3.50 4.00 4.50 5.00 5.50 6.00 6.50 7.00 7.50 8.00 8.50 9.00 9.50
GCMS Time (min.) →

# Fig. 26

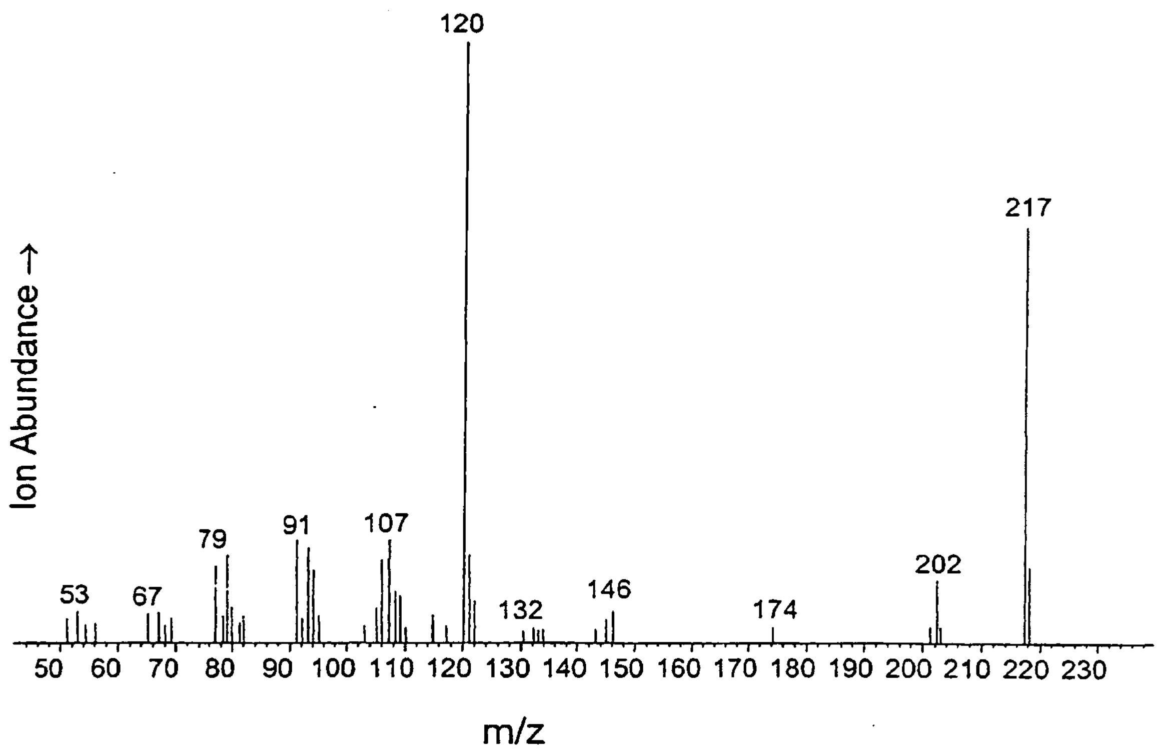
120
217
Ion Abundance →
53
67
79
91
107
132
146
174
202
50 60 70 80 90 100 110 120 130 140 150 160 170 180 190 200 210 220 230
m/z

# FIG. 27

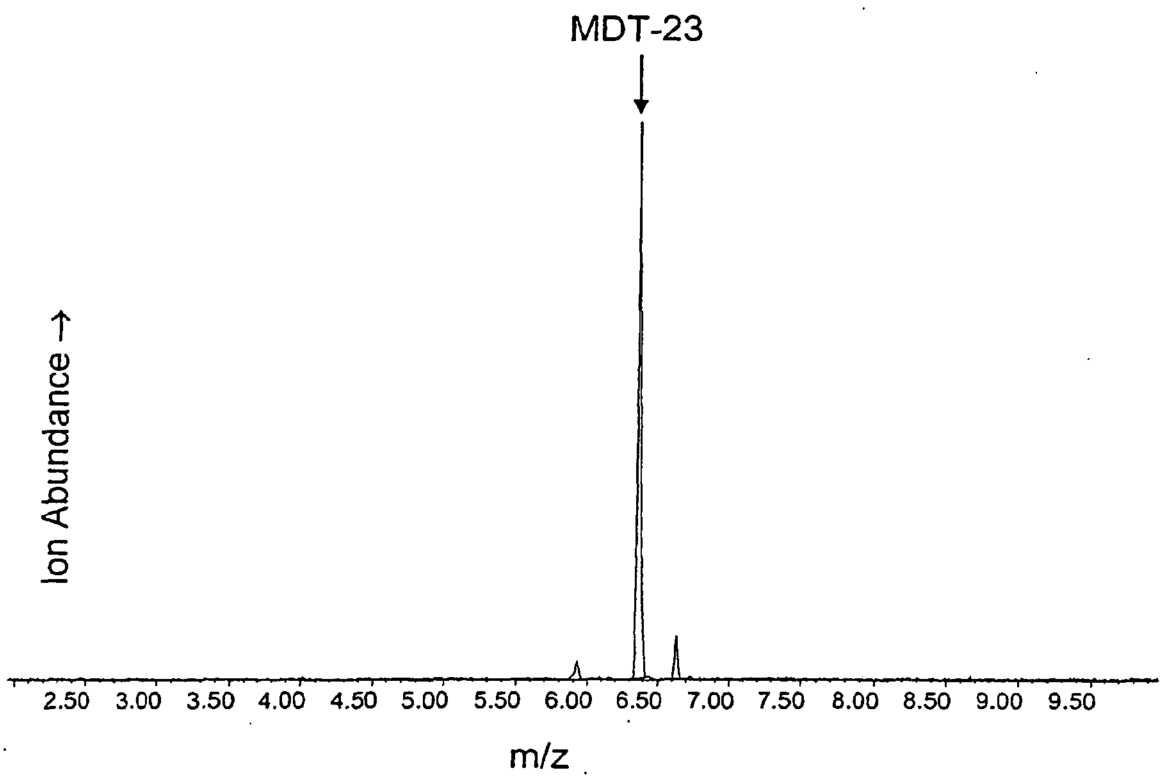
MDT-23
Ion Abundance →
2.50
3.00
3.50
4.00
4.50
5.00
5.50
6.00
6.50
7.00
7.50
8.00
8.50
9.00
9.50
m/z

FIG. 28

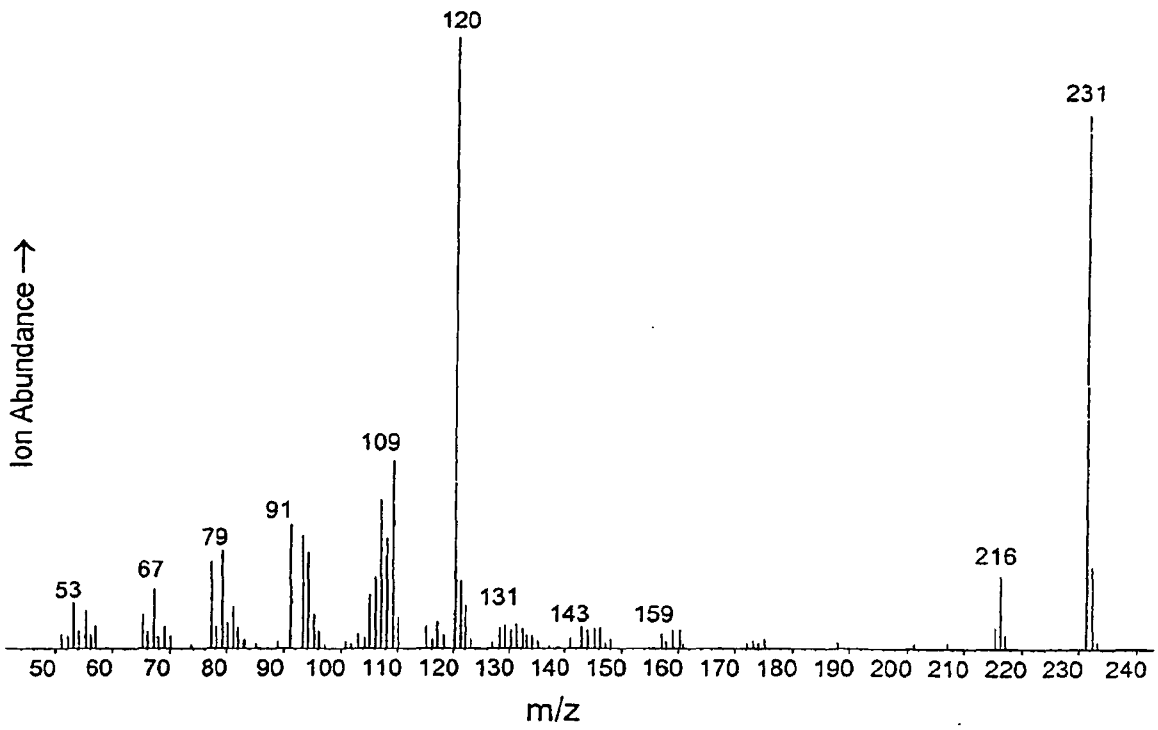
Ion Abundance →
120
231
109
91
79
67
53
131
143
159
216
50 60 70 80 90 100 110 120 130 140 150 160 170 180 190 200 210 220 230 240
m/z

FIG. 29

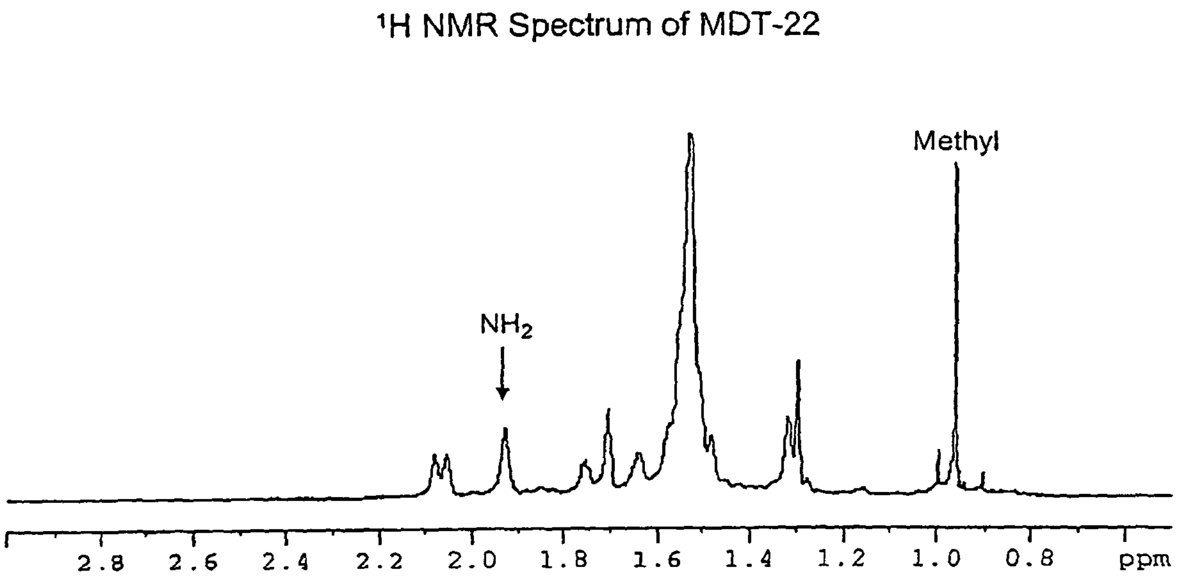

¹H NMR Spectrum of MDT-22
Methyl
NH2
2.8
2.6
2.4
2.2
2.0
1.8
1.6
1.4
1.2
1.0
0.8
ppm

# FIG. 30

$^{13}C$ NMR Spectrum of MDT-22

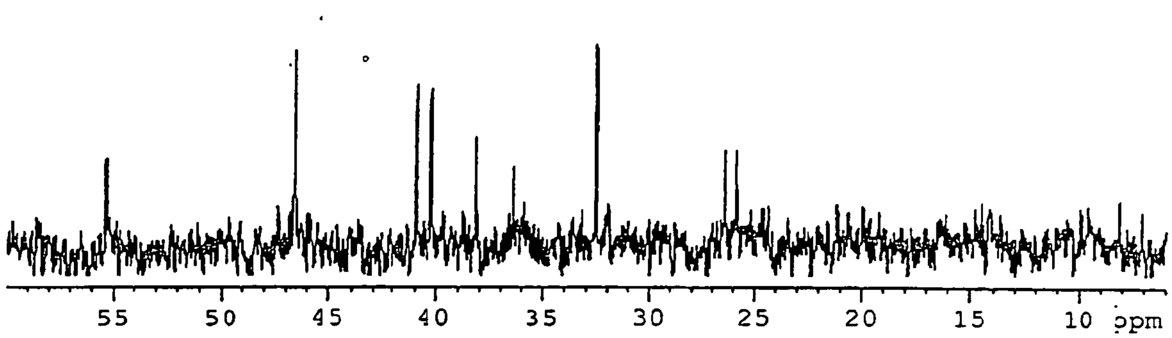

# FIG. 31

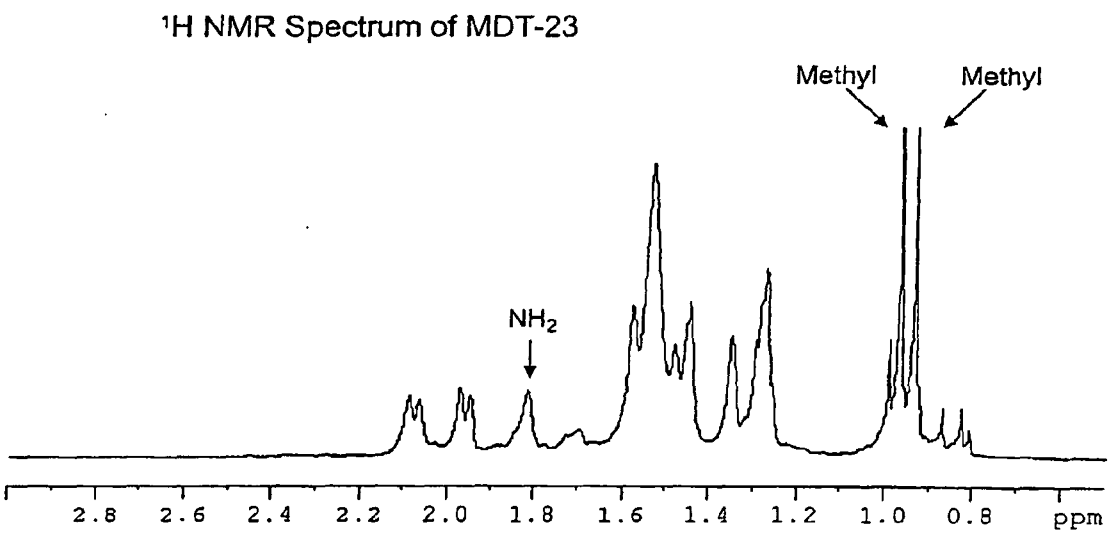

¹H NMR Spectrum of MDT-23
Methyl
Methyl
$NH_2$
2.8
2.6
2.4
2.2
2.0
1.8
1.6
1.4
1.2
1.0
0.8
ppm

FIG. 32

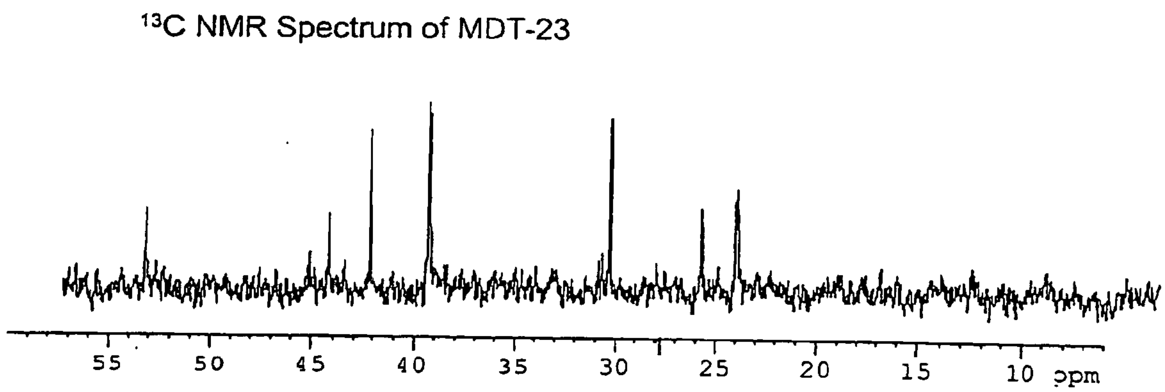

Fig. 33

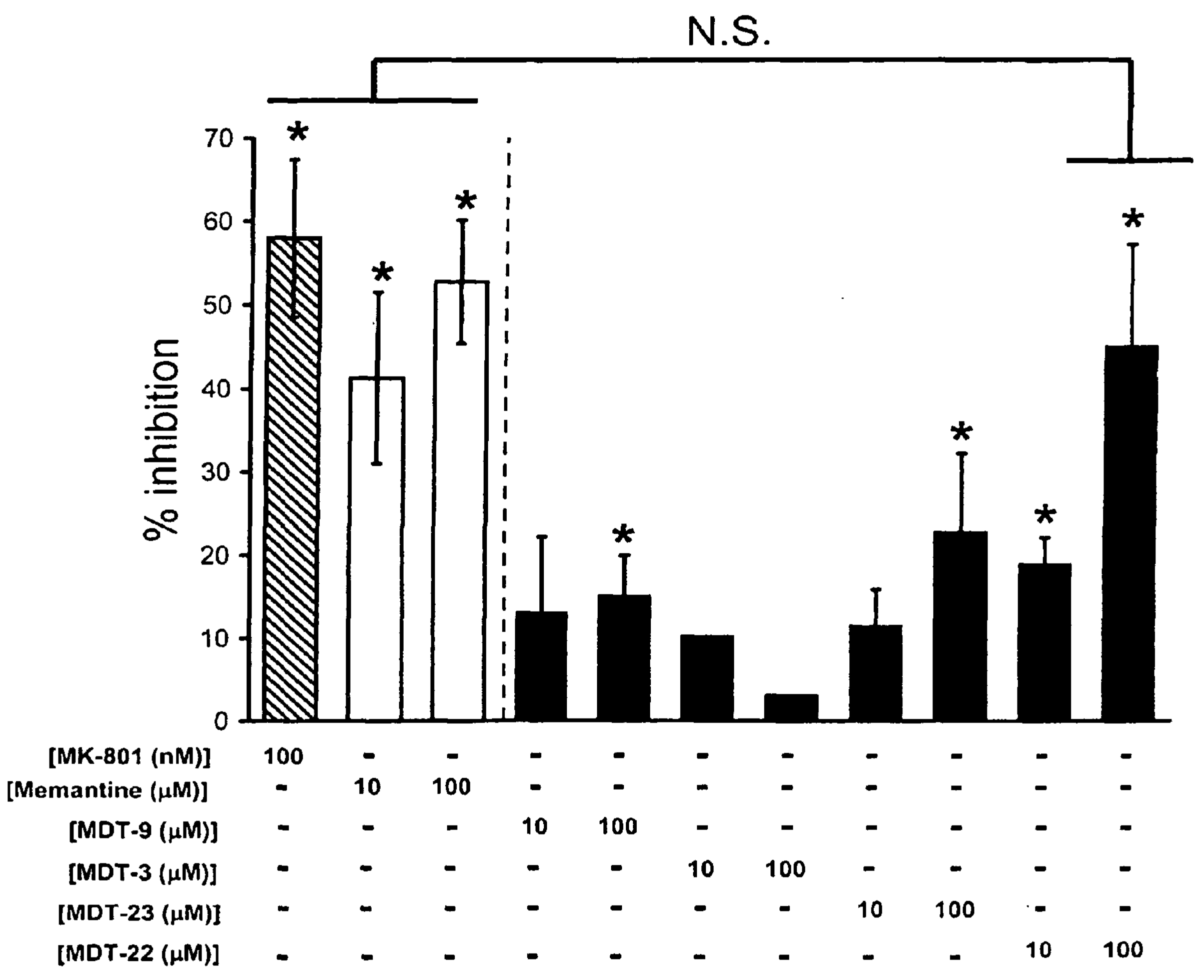
N.S.
% inhibition
70
60
50
40
30
20
10
0
*
[MK-801 (nM)] 100 - - - - - - - - - -
[Memantine (µM)] - 10 100 - - - - - - - -
[MDT-9 (µM)] - - - 10 100 - - - - - -
[MDT-3 (µM)] - - - - - 10 100 - - - -
[MDT-23 (µM)] - - - - - - - 10 100 - -
[MDT-22 (µM)] - - - - - - - - - 10 100

IR spectrum of 1, 6-dibromodiamantane

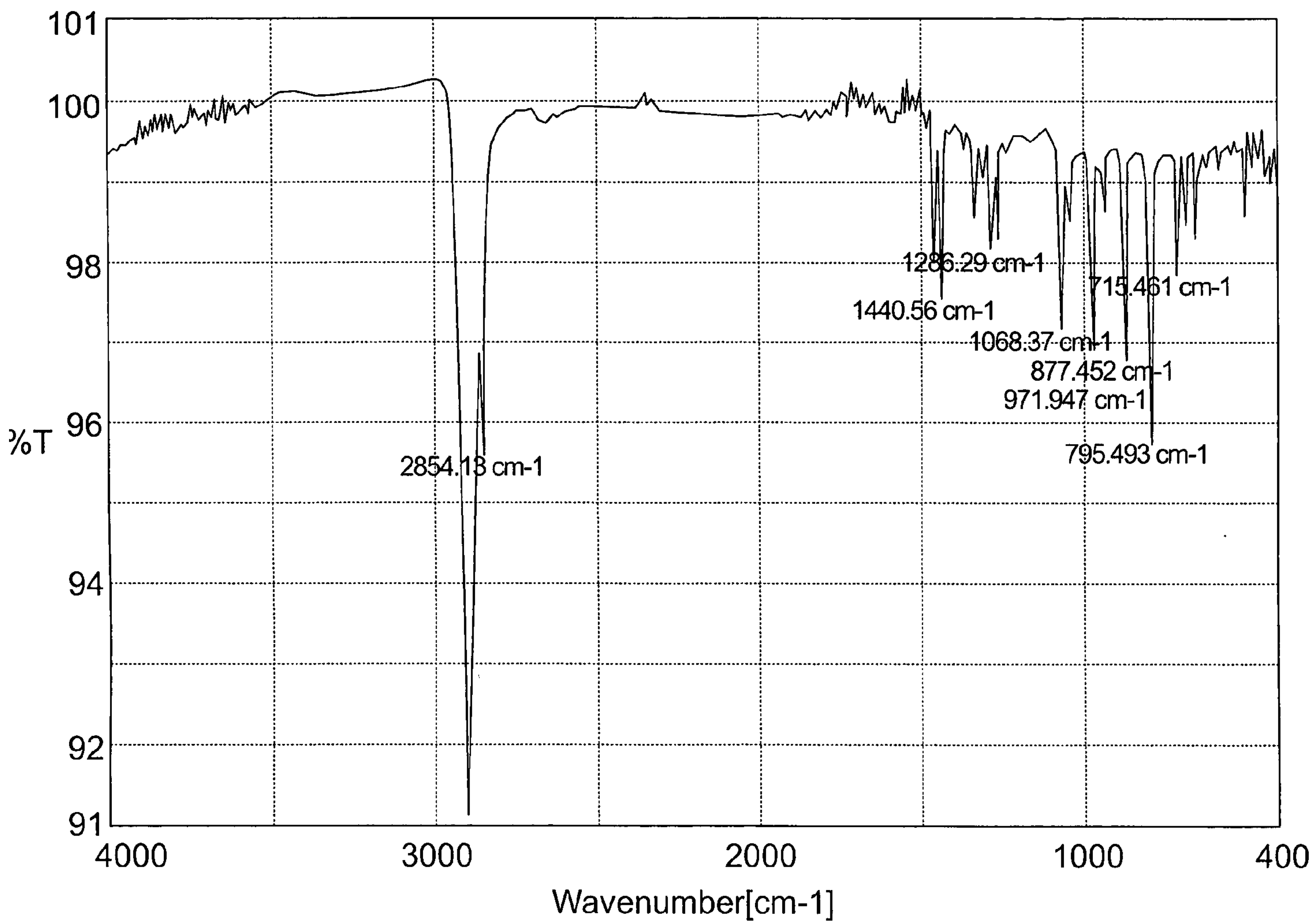


FIG. 34

FIG. 35

$^1$H-NMR of 1, 6-dibromodiamantane in $CDCl_3$

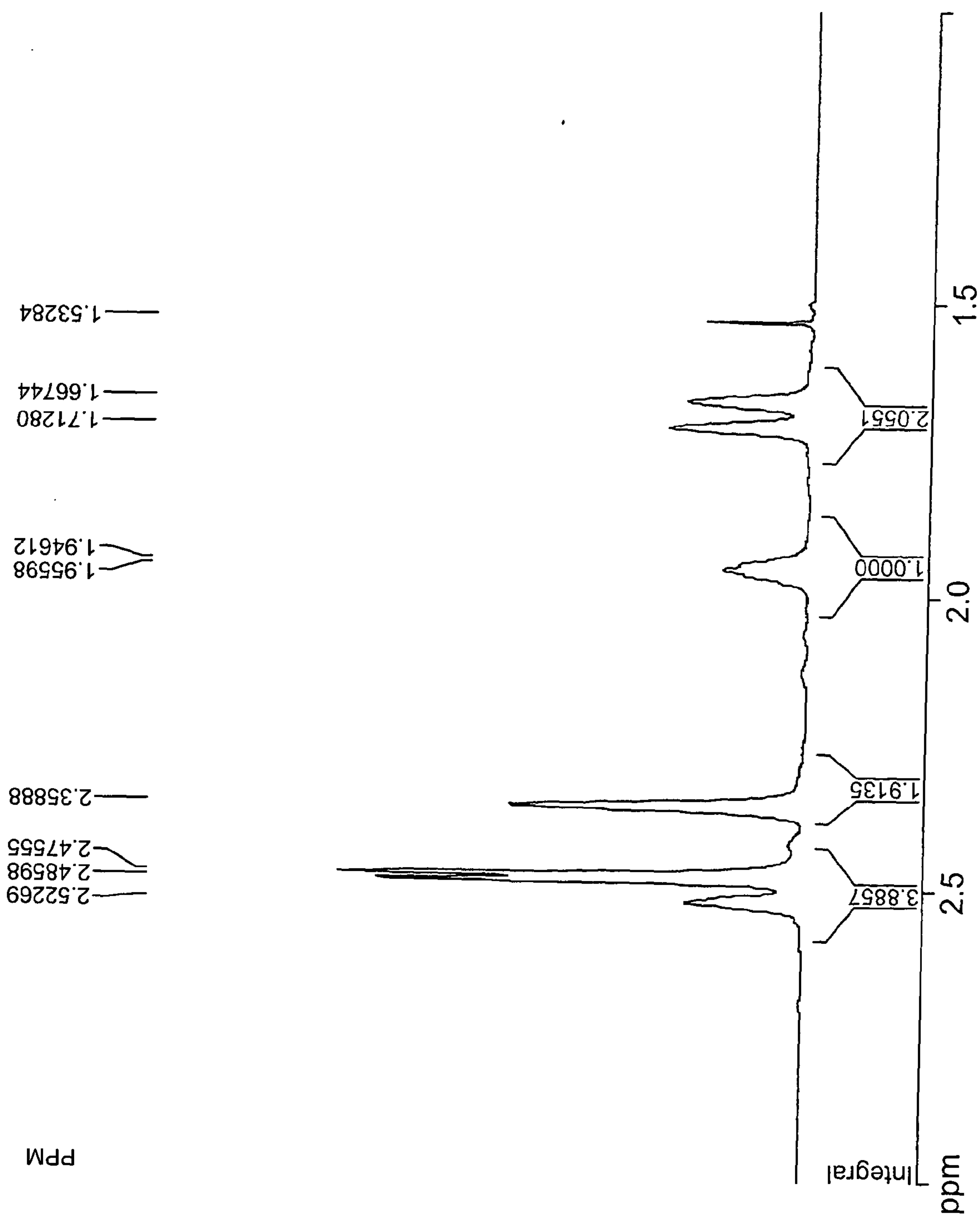

$^{13}$C-NMR of 1,6-dibromodiamantane in $CDCl_3$

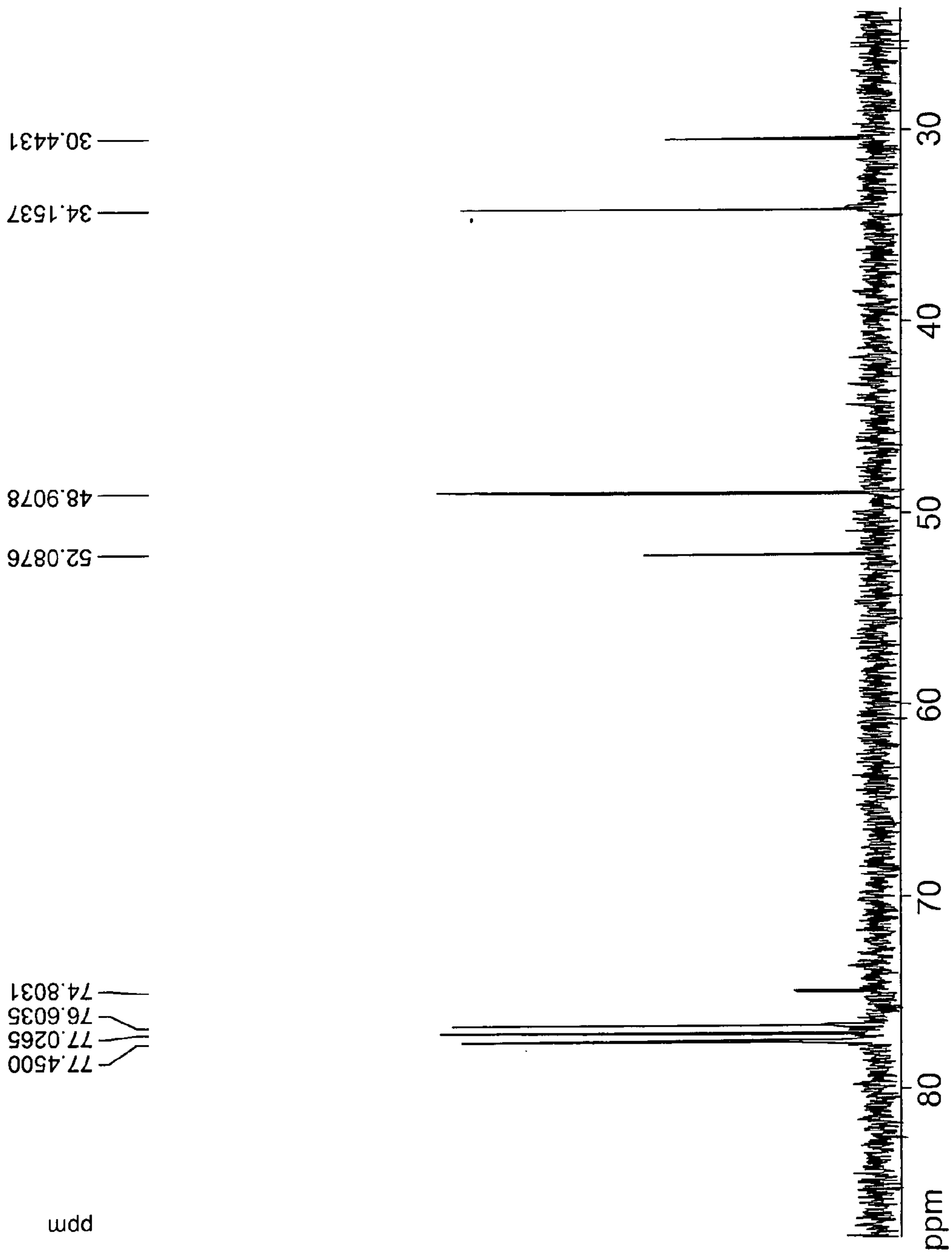


FIG. 36

$^{13}C$-$^{1}H$ Cosy of 1,6-dibromodiamantane

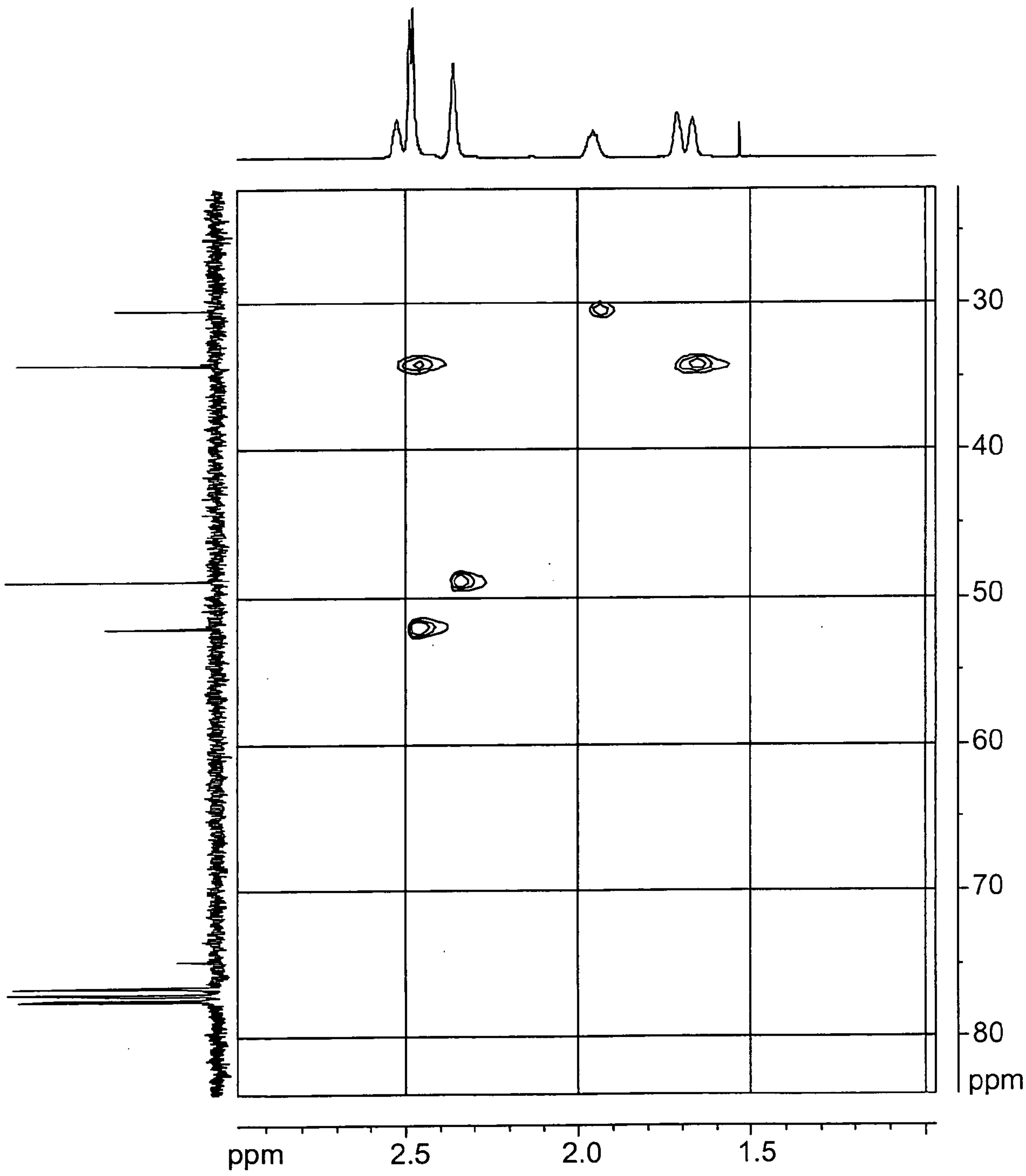


FIG. 37

GC-MS spectra of 1,6-dimethyl1-diamantane

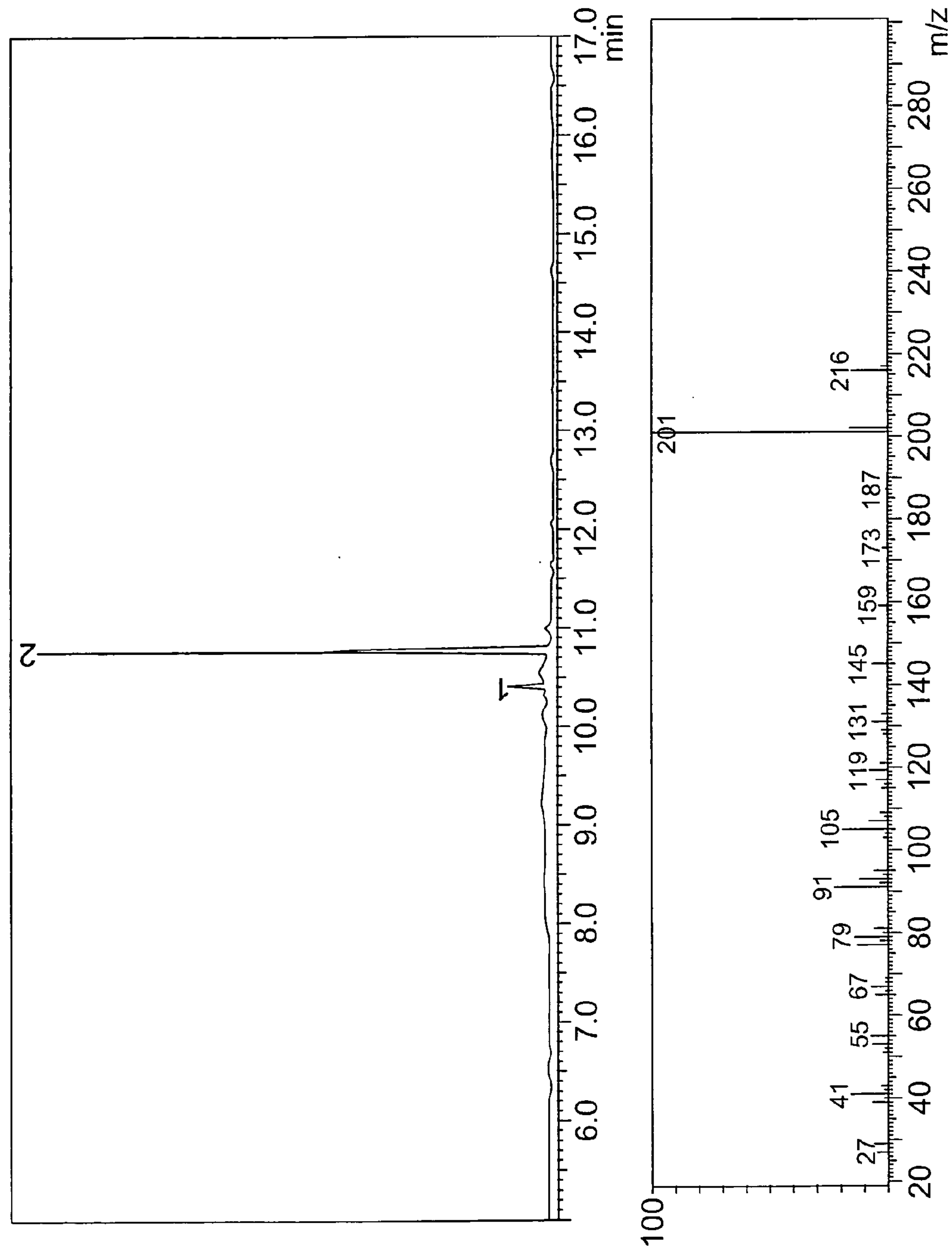


FIG. 38

$^1$H-NMR spectrum of 1,6-dimethyl-diamantane in $CDCl_3$

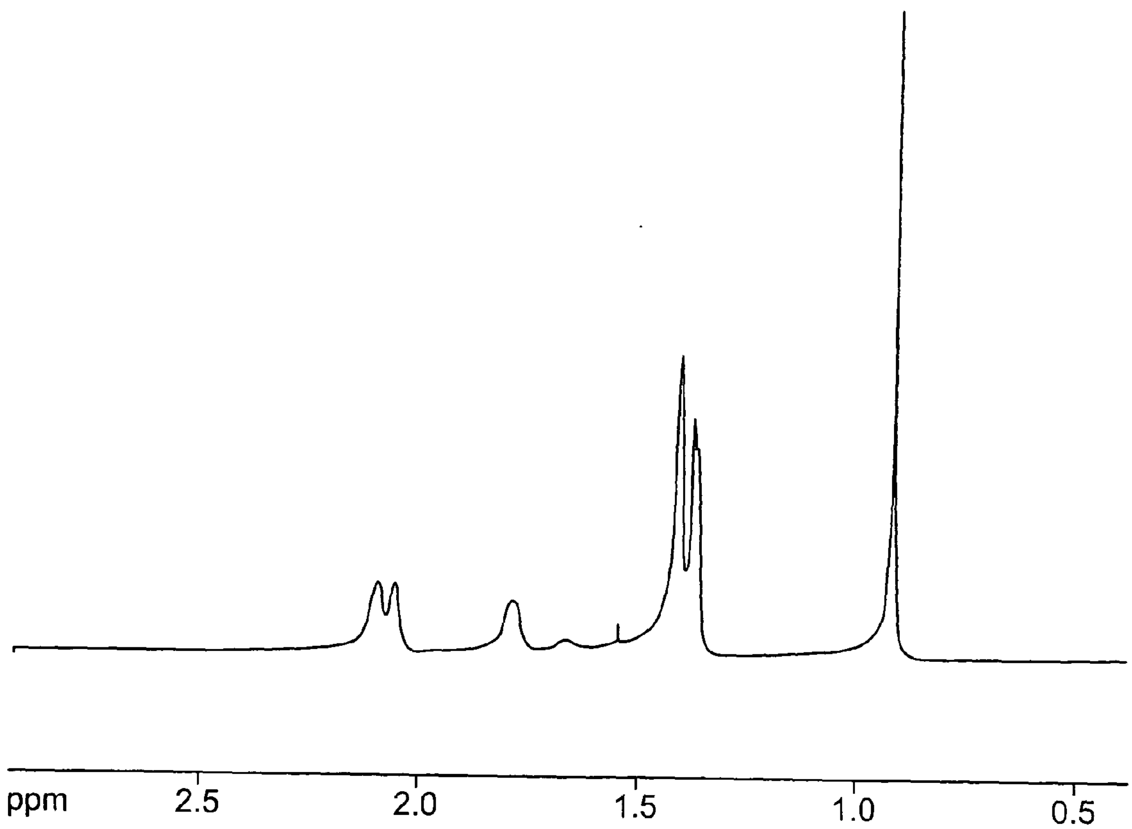


FIG. 39

$^{13}C$-NMR spectrum of 1,6-dimethyl-diamantane in $CDCl_3$

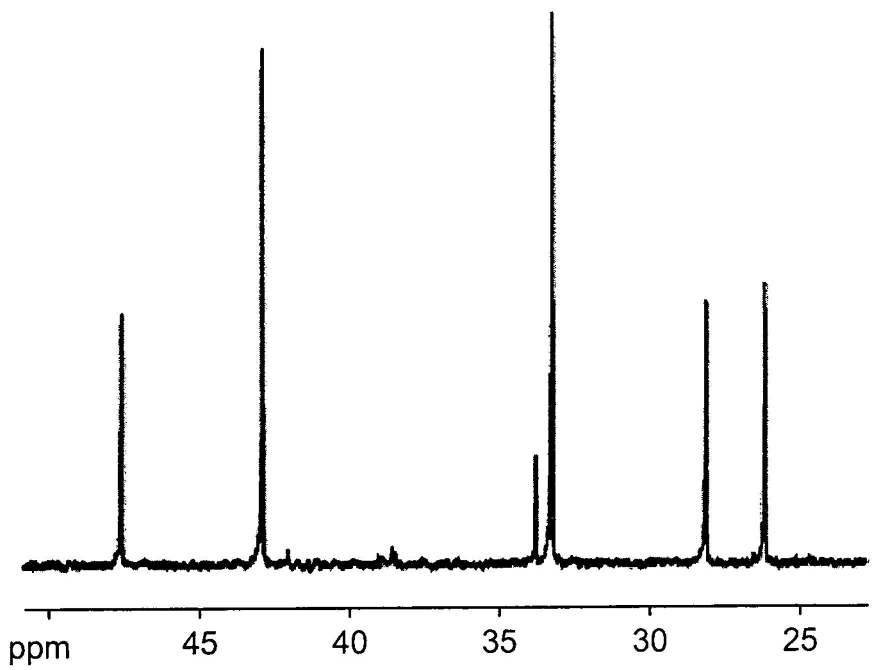


FIG. 40

$^1$H-NMR spectrum of 4-bromo-1, 6-dimethyl-diamantane in $CDCl_3$

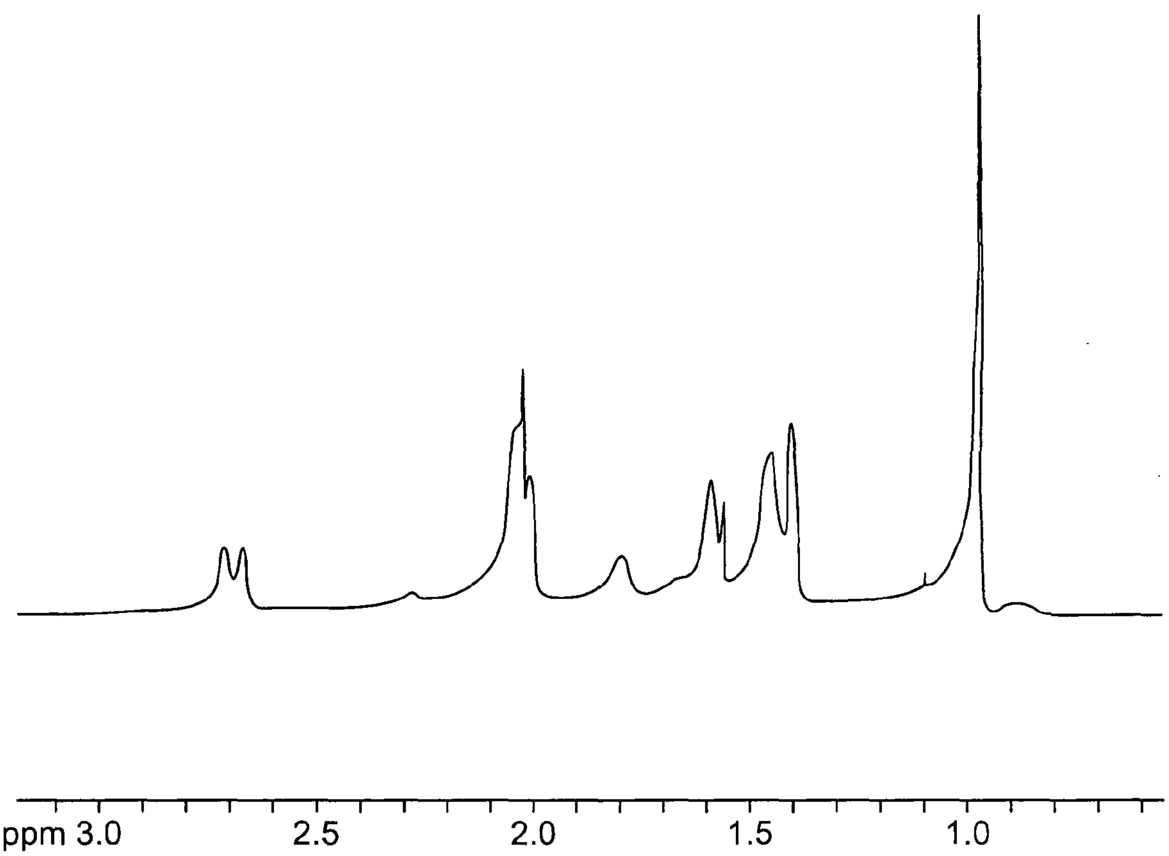


FIG. 41

$^{13}C$-NMR spectrum of 4-bromo-1,6-dimethy1-diamantane in $CDCl_3$

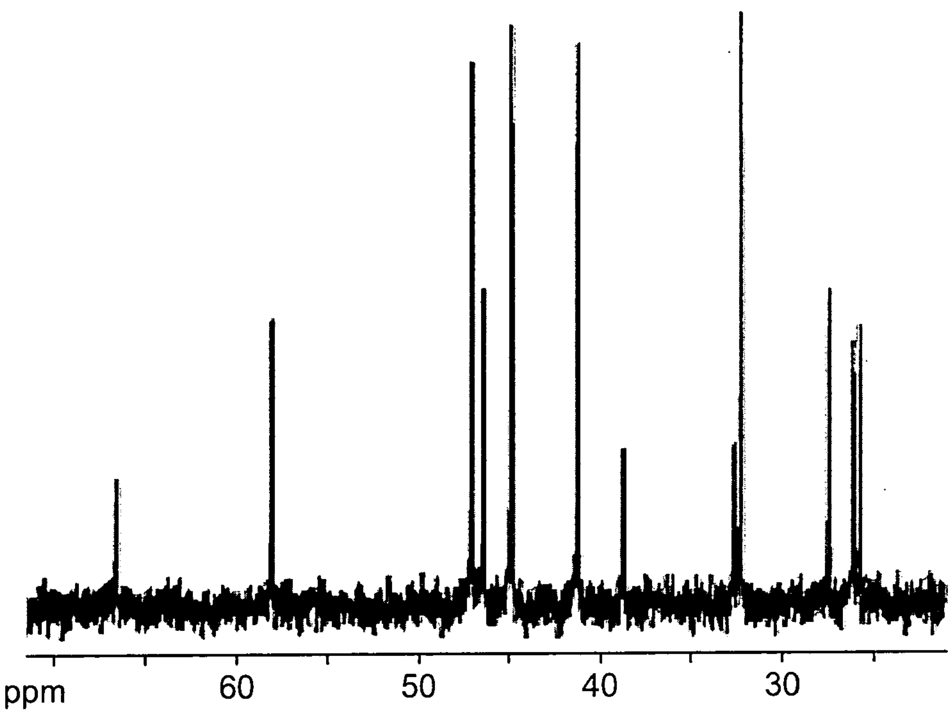


FIG. 42

$^{1}$H-NMR spectrum of 4-azido-1,6-dimethyl-diamantane in $CDCl_3$

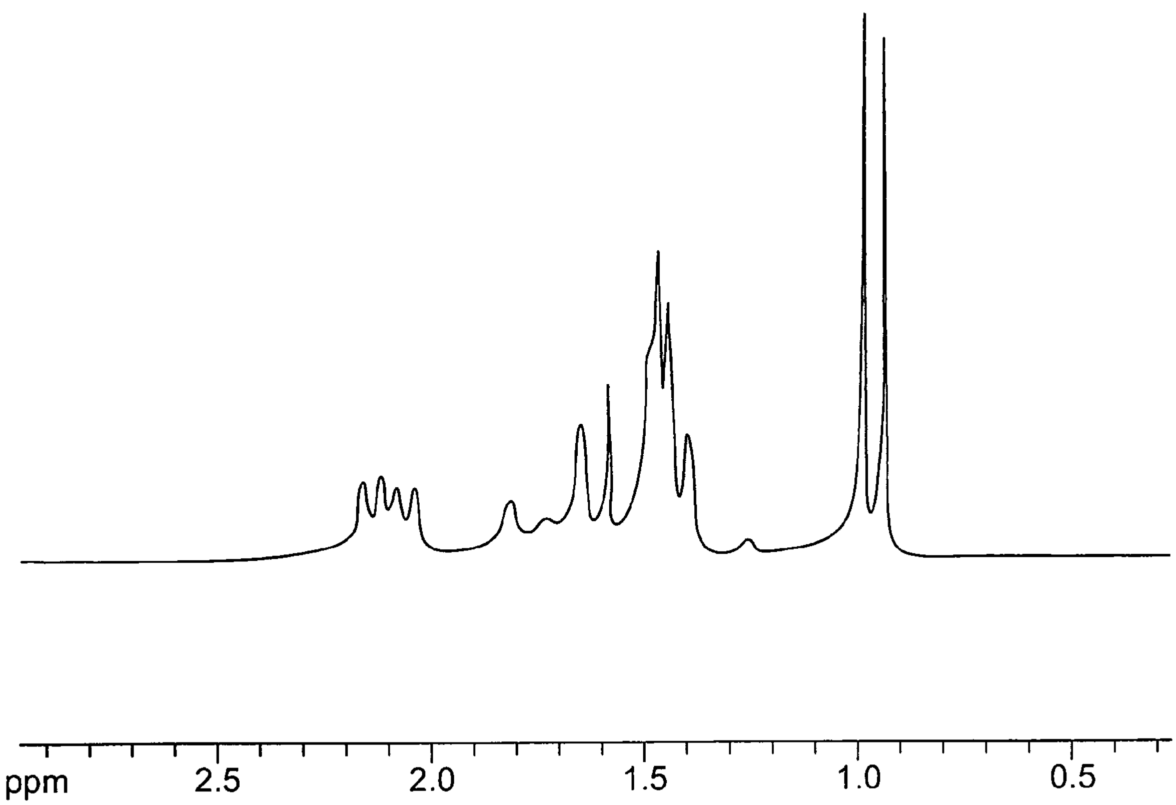


FIG. 43

$^{13}C$-NMR spectrum of 4-azido-1,6-dimethyl-diamantane in $CDCl_3$

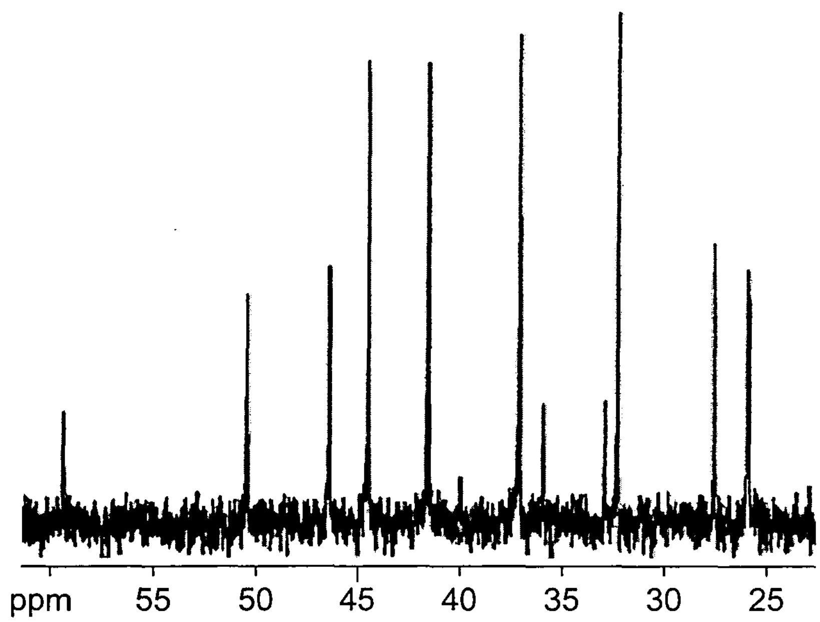


FIG. 44

**Figure 45.** GC-MS spectra of 4-amino-1,6-dimethyl-diamantane

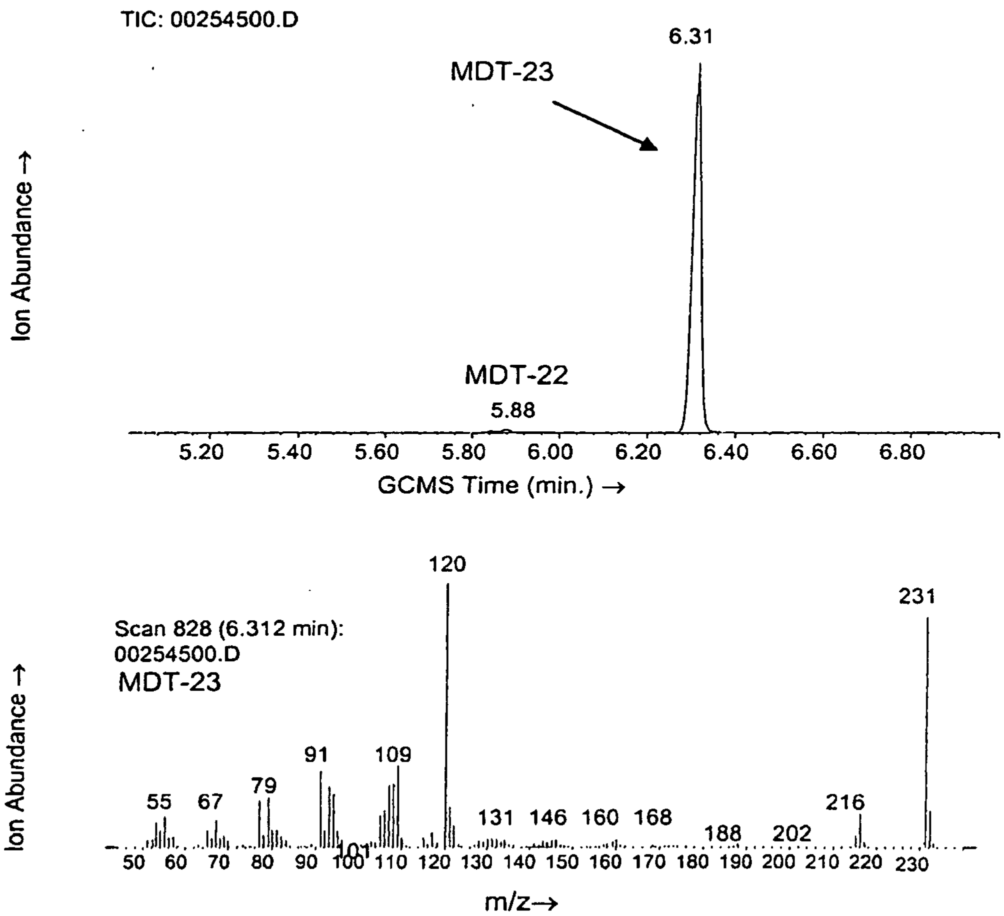

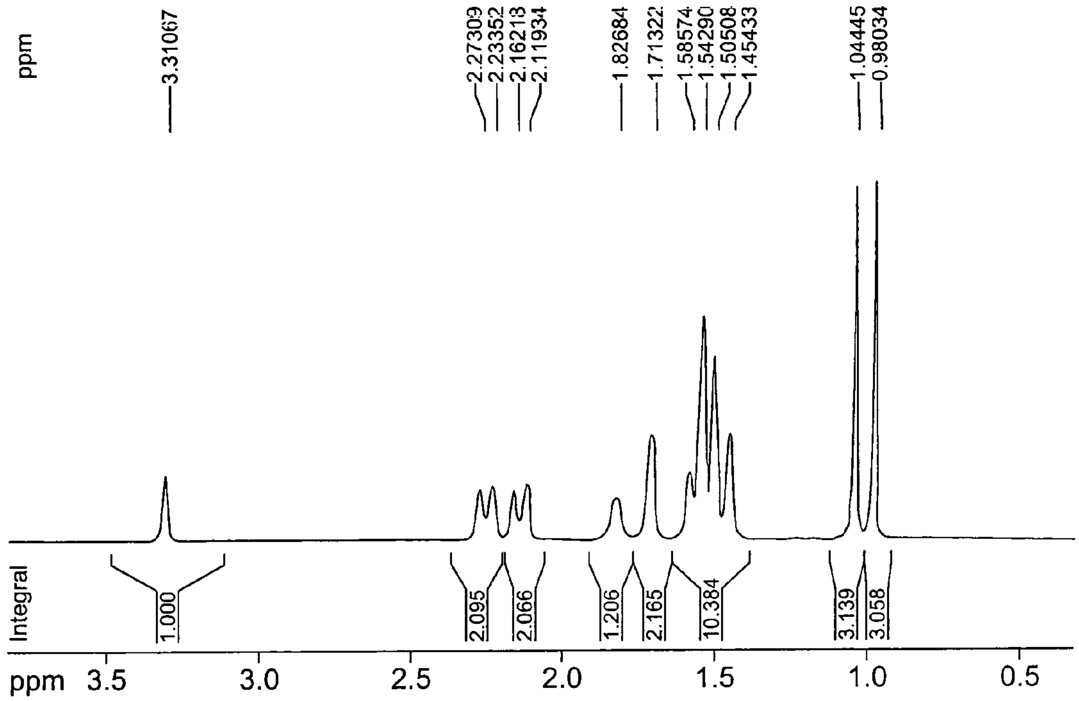
ppm
3.31067
2.27309
2.23352
2.16213
2.11934
1.82684
1.71322
1.58574
1.54290
1.50508
1.45433
1.04445
0.98034
Integral
1.000
2.095
2.066
1.206
2.165
10.384
3.139
3.058
ppm 3.5 3.0 2.5 2.0 1.5 1.0 0.5

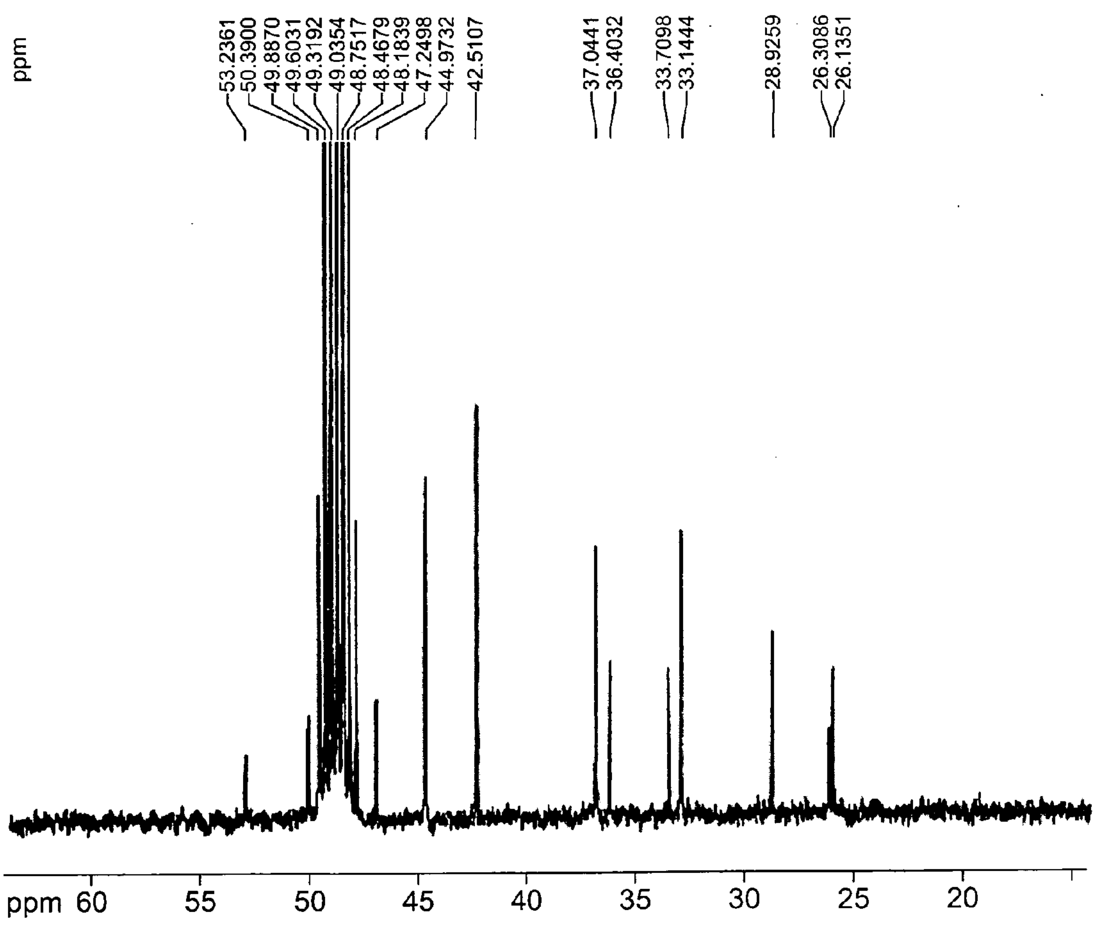
ppm
53.2361
50.3900
49.8870
49.6031
49.3192
49.0354
48.7517
48.4679
48.1839
47.2498
44.9732
42.5107
37.0441
36.4032
33.7098
33.1444
28.9259
26.3086
26.1351
ppm 60 55 50 45 40 35 30 25 20

FIG. 46

FIG. 47

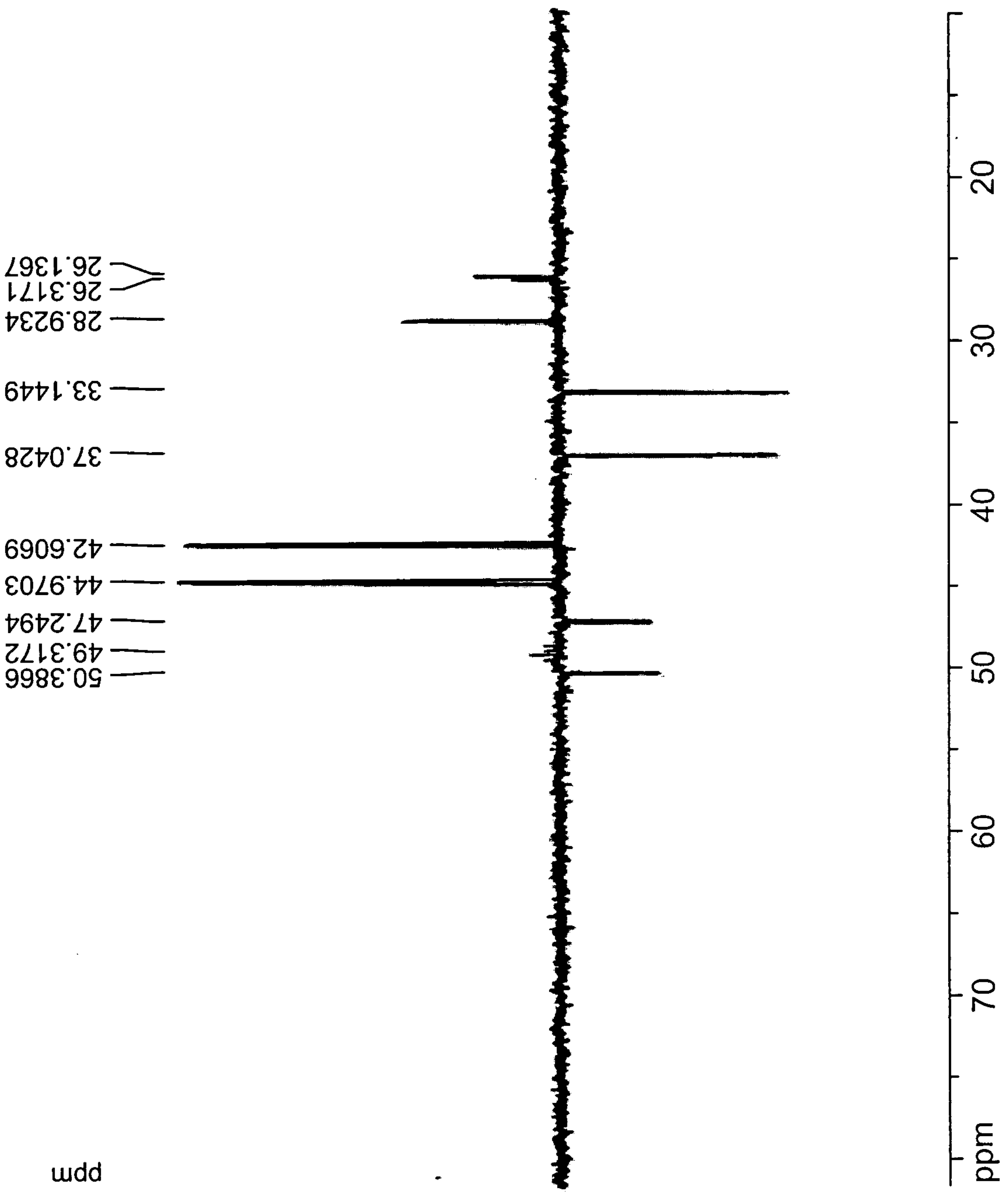
26.1367
26.3171
28.9234
33.1449
37.0428
42.6069
44.9703
47.2494
49.3172
50.3866
ppm
20
30
40
50
60
70
ppm

FIG. 48

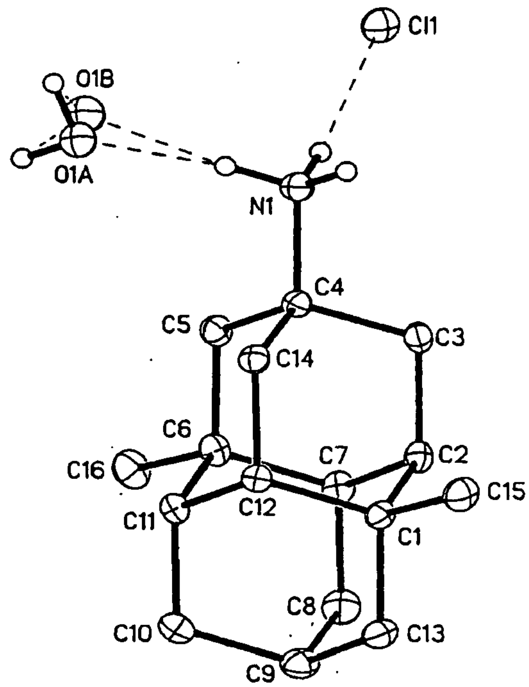

MDT-23

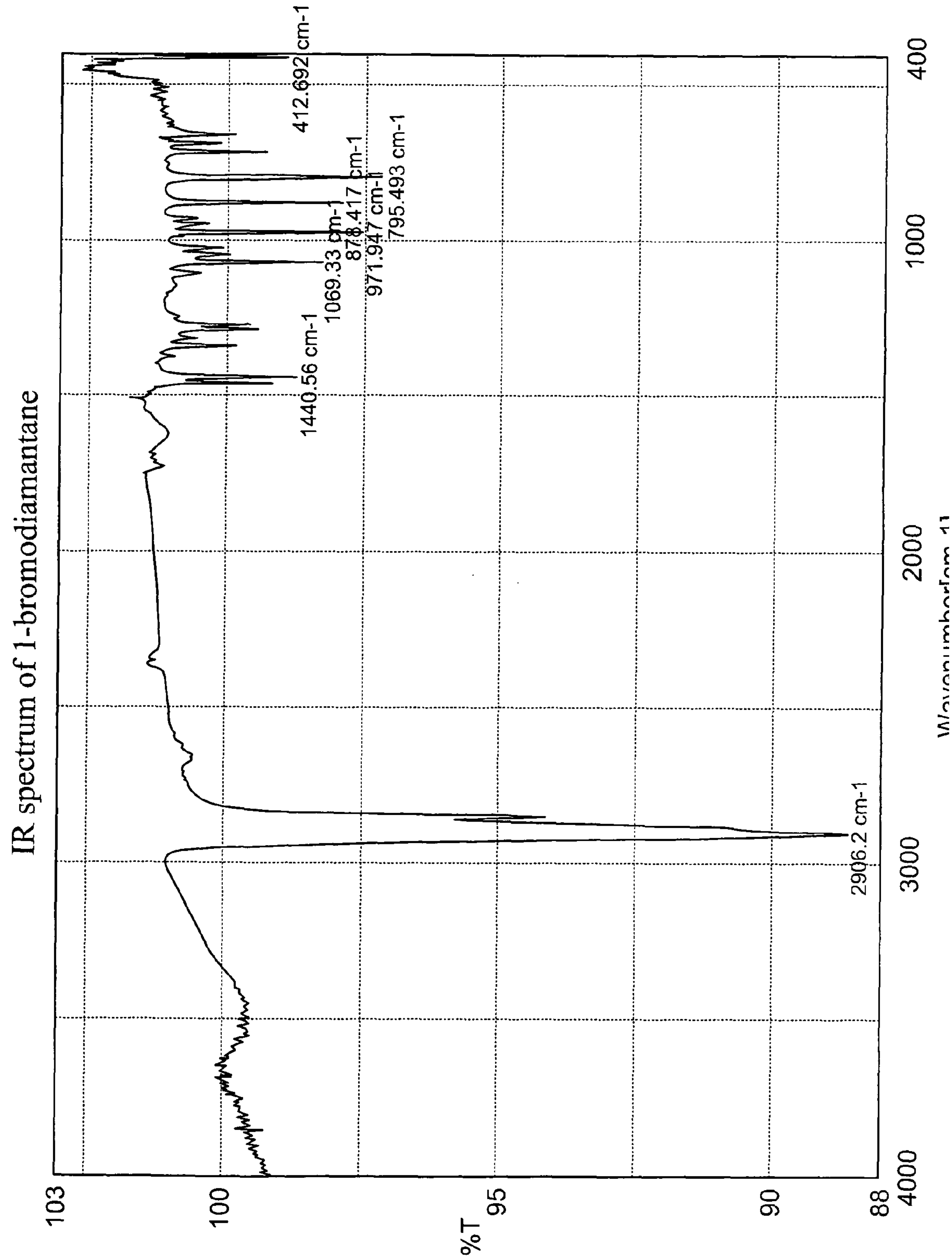
IR spectrum of 1-bromodiamantane
%T
103
100
95
90
88
4000
3000
2000
1000
400
2906.2 cm-1
1440.56 cm-1
1069.33 cm-1
971.947 cm-1
878.417 cm-1
795.493 cm-1
412.692 cm-1

FIG. 49

$^1H$-NMR of 1-bromodiamantane in $CDCl_3$

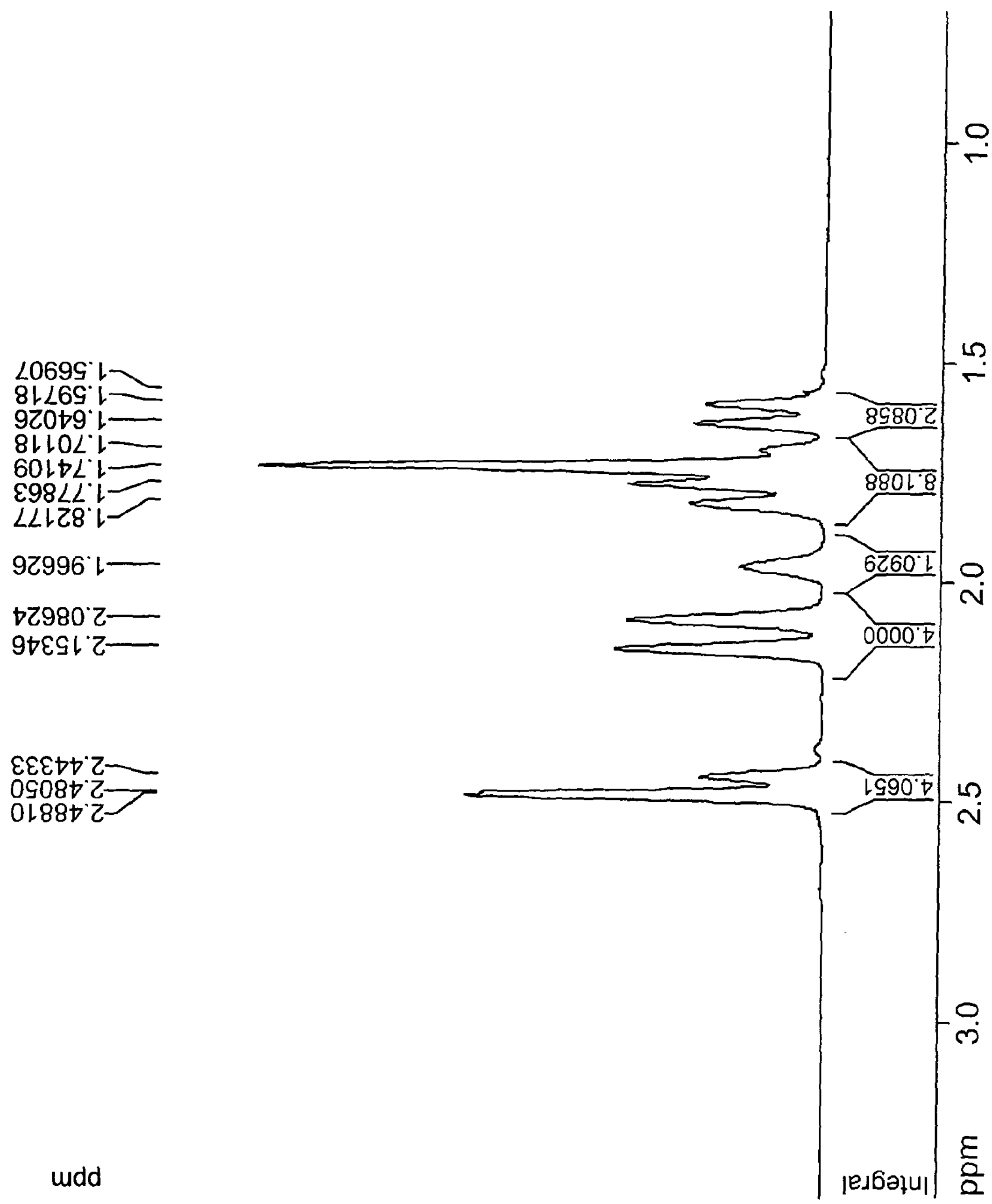


FIG. 50

FIG. 51

$^{13}$C-NMR of 1-bromodiamantane in $CDCl_3$

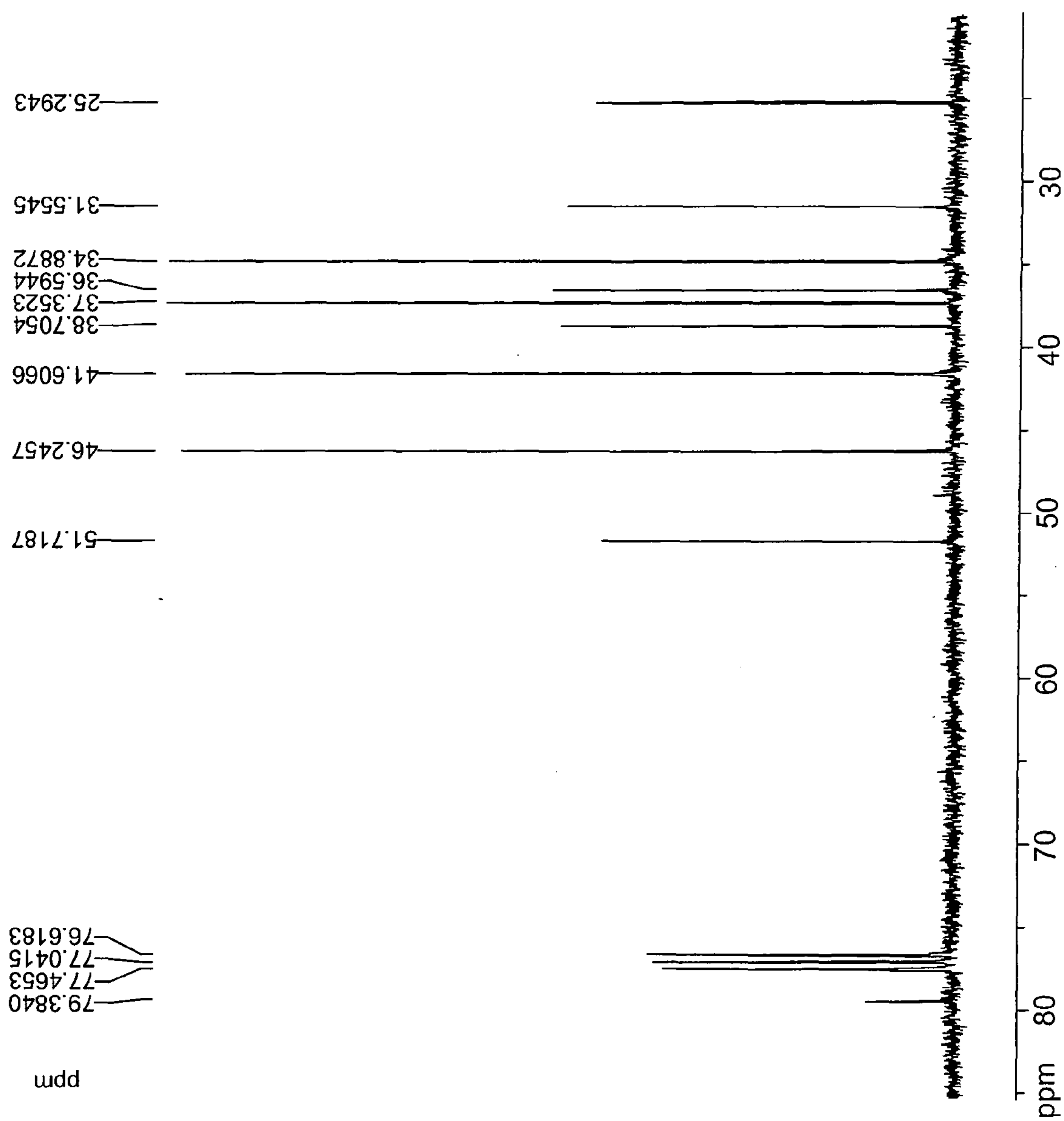

$^{13}C$-$^{1}H$ cosy of 1-bromodiamantane in $CDCl_3$

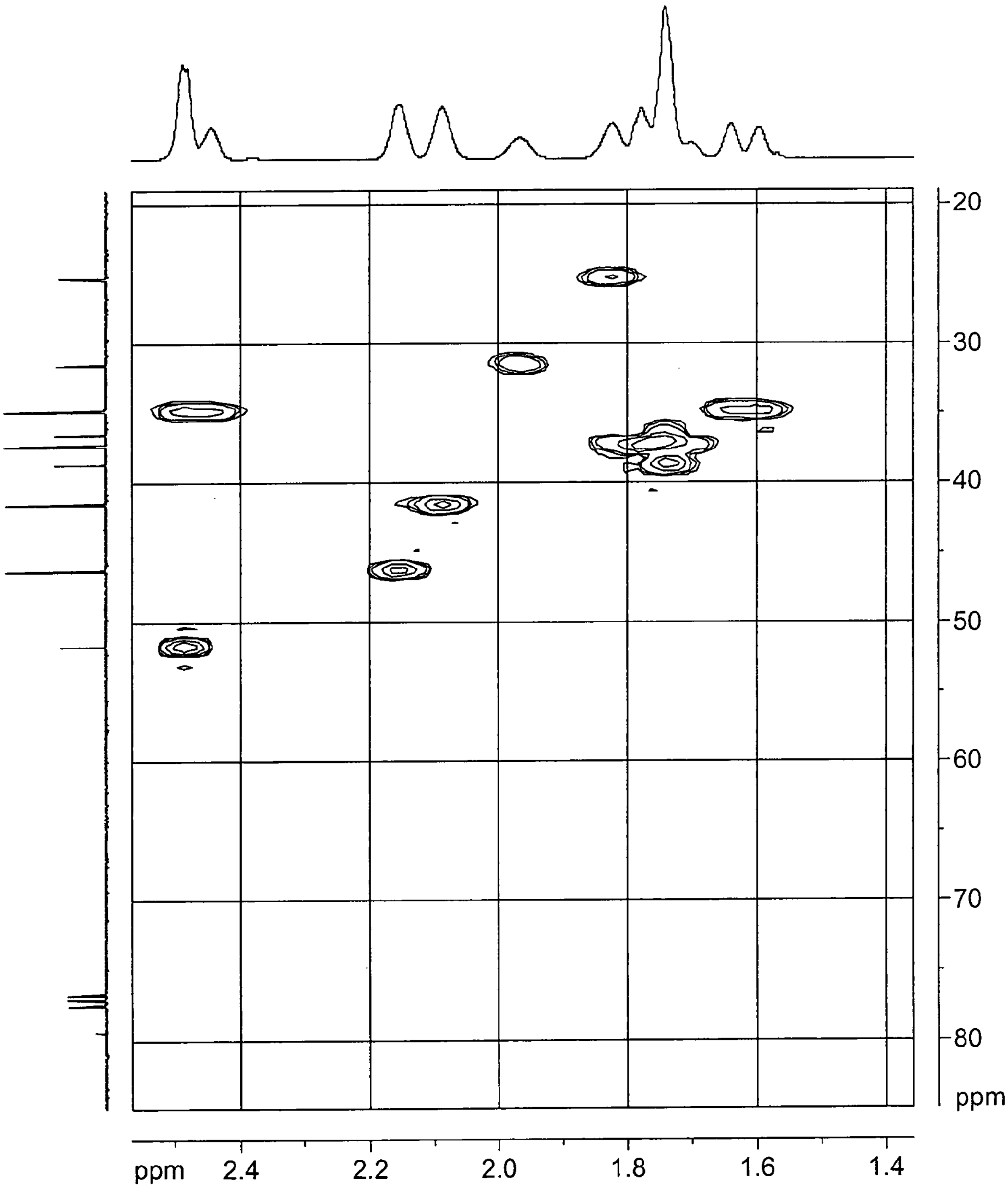


FIG. 52

Mass spectrum of 1-bromodiamantane

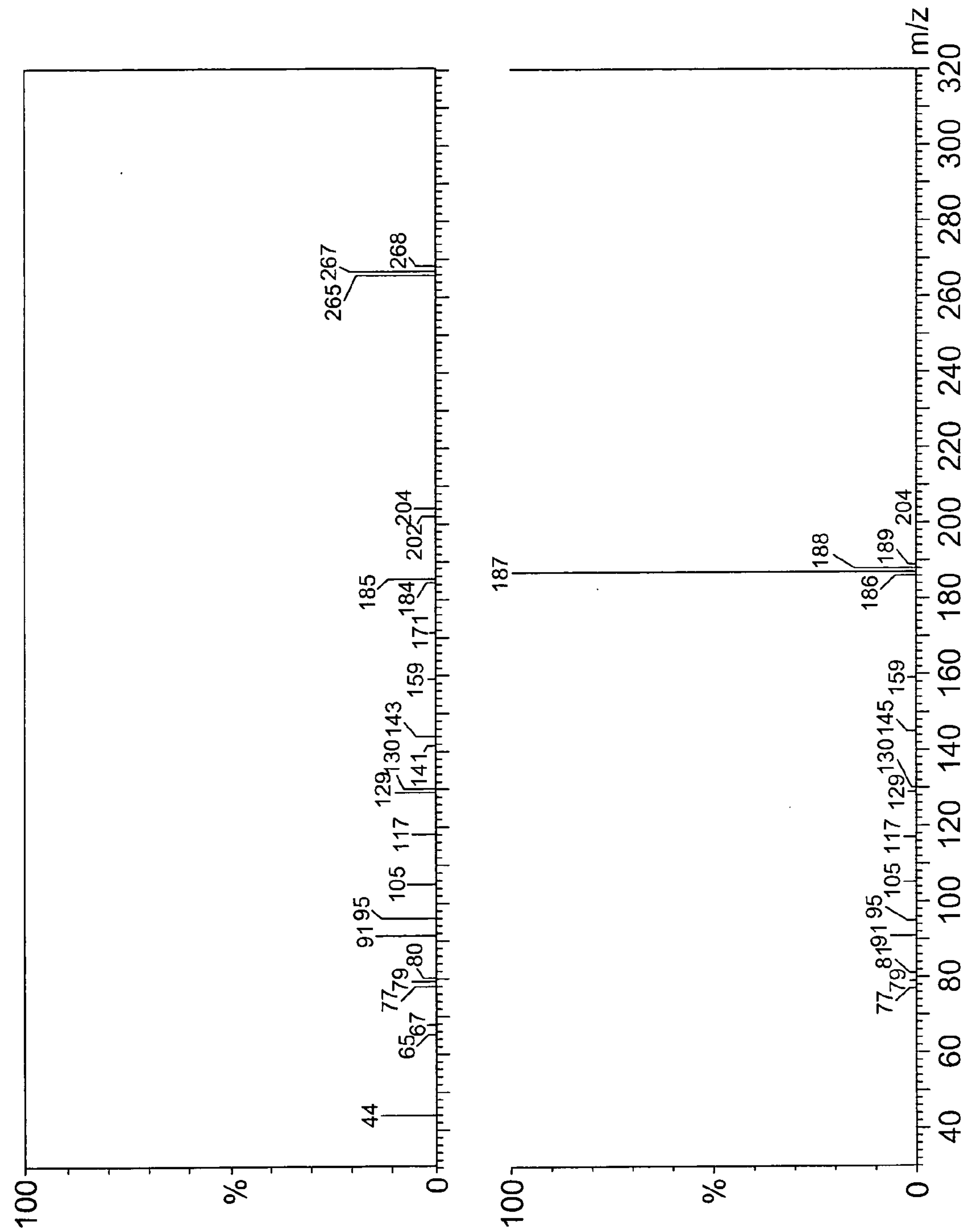


FIG. 53

$^1H$-NMR spectrum of 1-methyldiamantane in $CDCl_3$

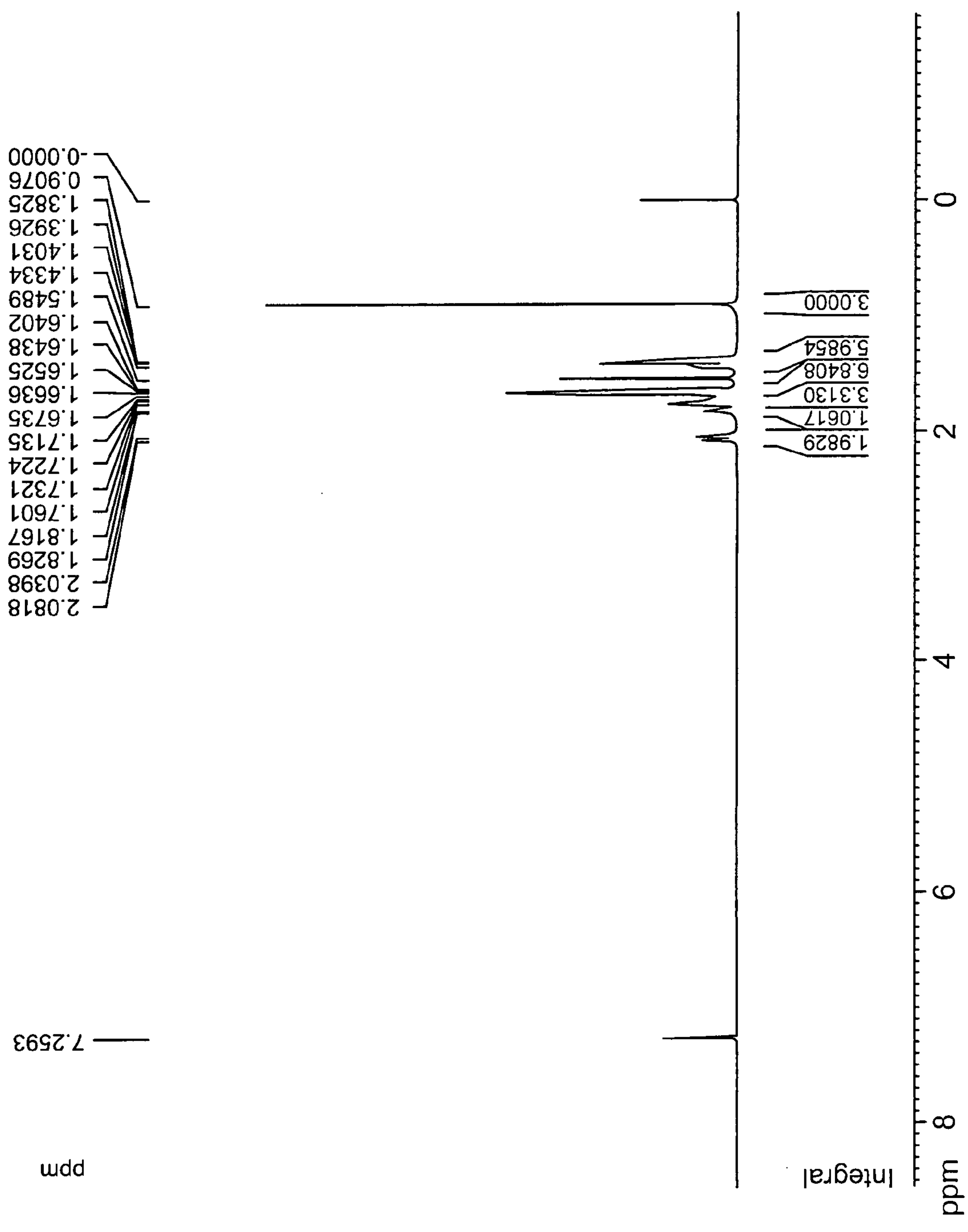


FIG. 54

$^{13}C$-NMR spectrum of 1-methyldiamantane in $CDCl_3$

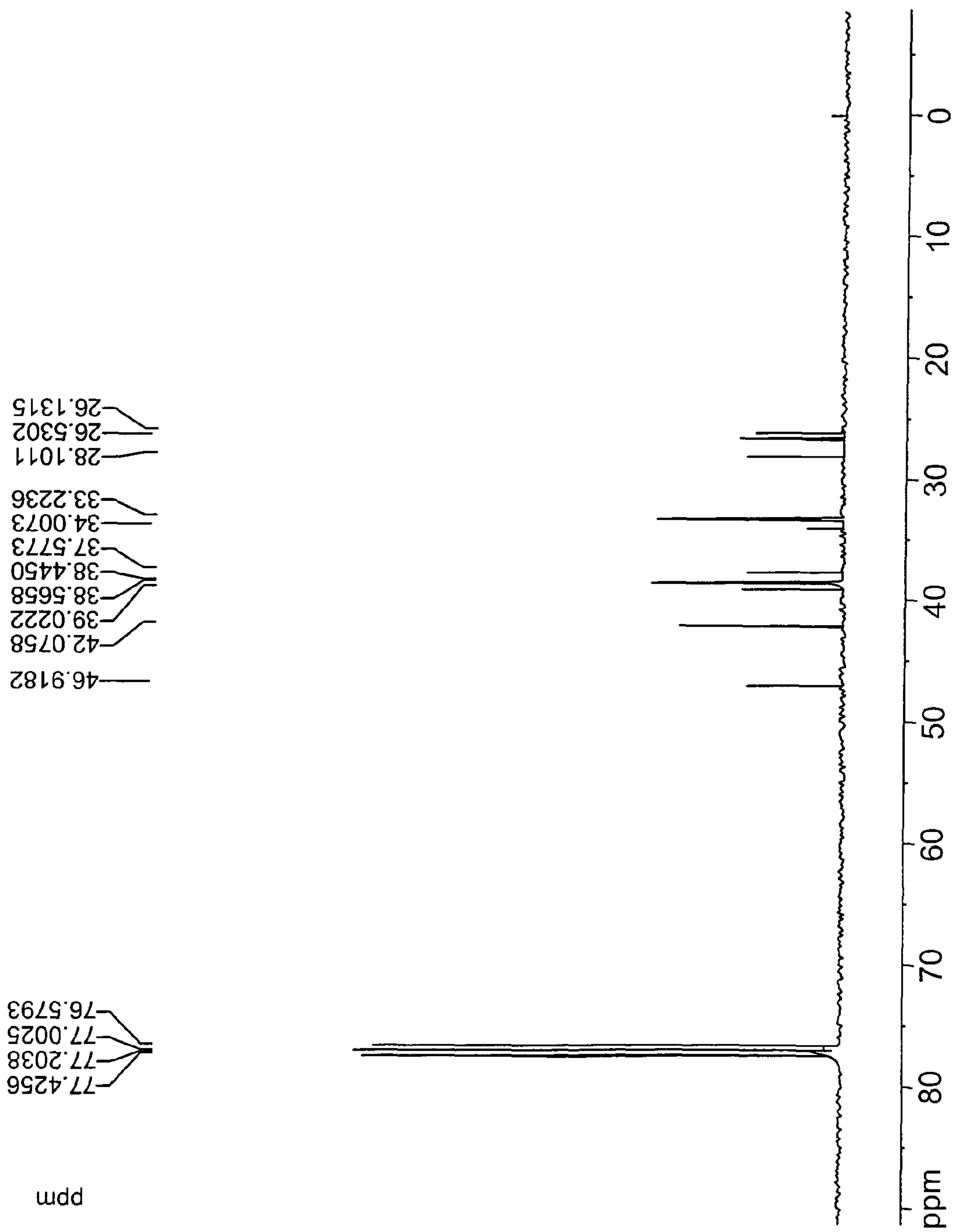


FIG. 55

$^1$H-NMR spectrum of 1-methyl-4-bromo-diamantane and 1-methyl-9-bromo-diamantane mixture in $CDCl_3$

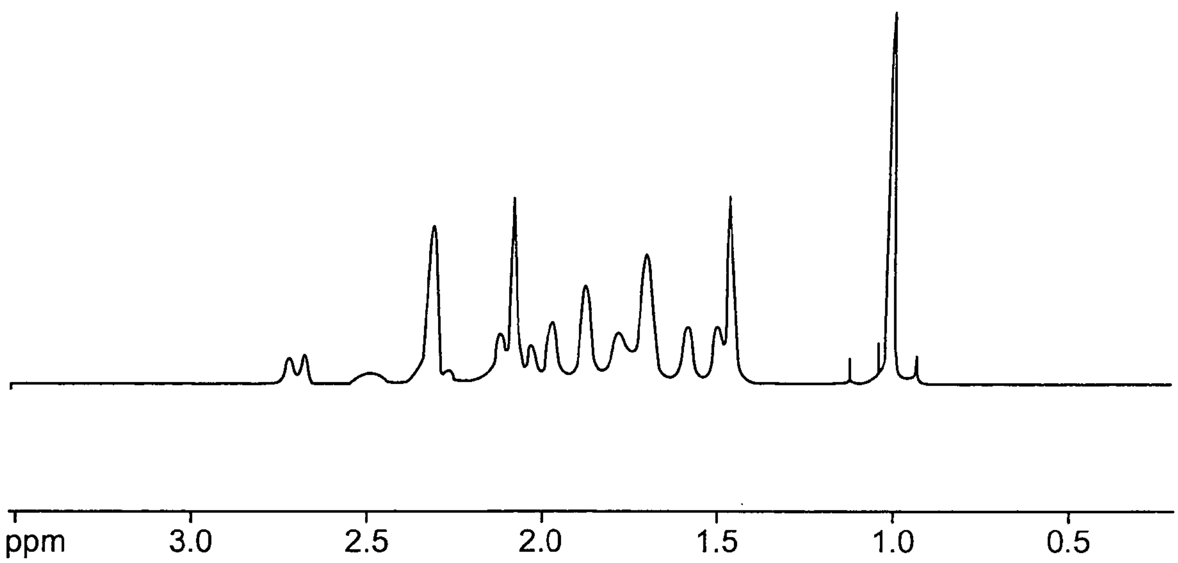


FIG. 56

$^{13}C$-NMR spectrum of 1-methyl-4-bromo-diamantane and 1-methyl-9-bromo-diamantane mixture in $CDCl_3$

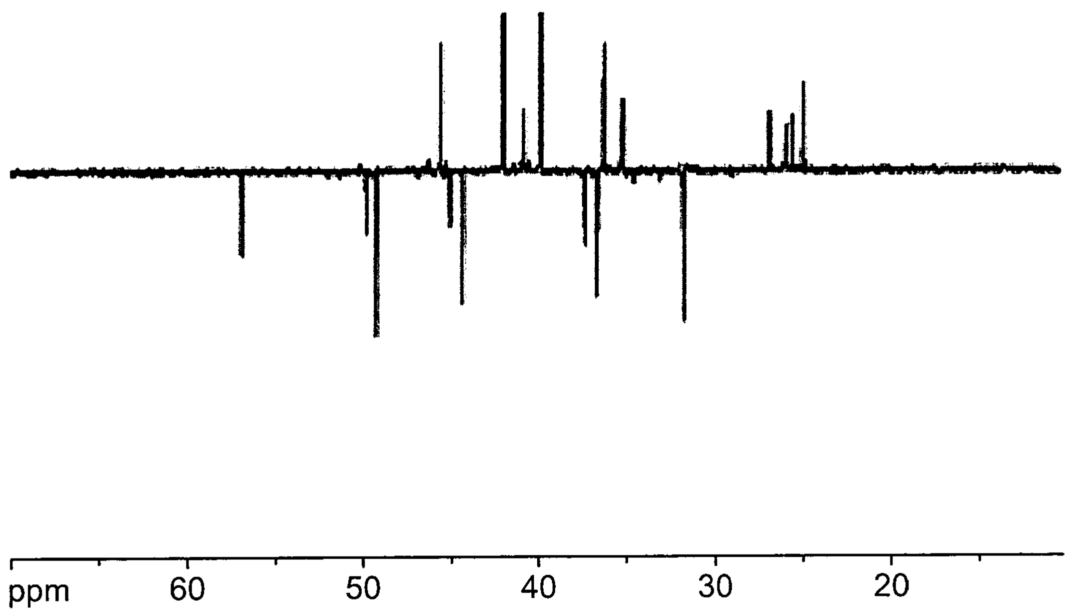


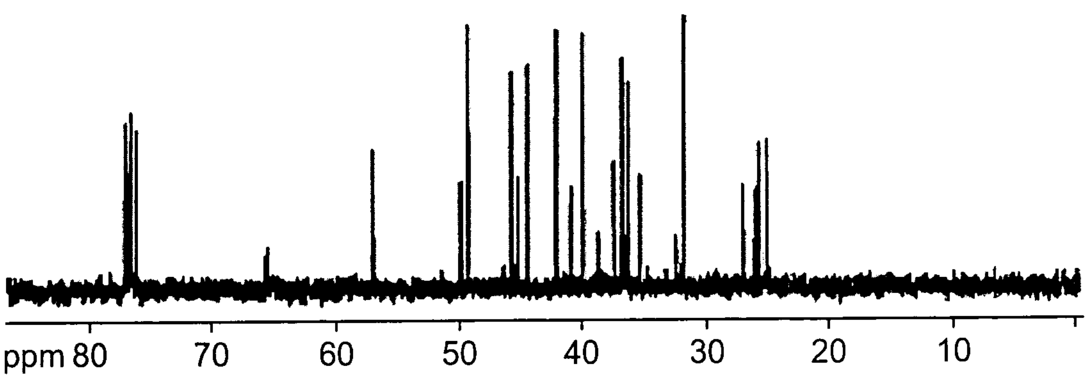


FIG. 57

IR spectrum of 1-methyl-4-azido-diamantane and 1-methyl-9-azido-diamantane mixture

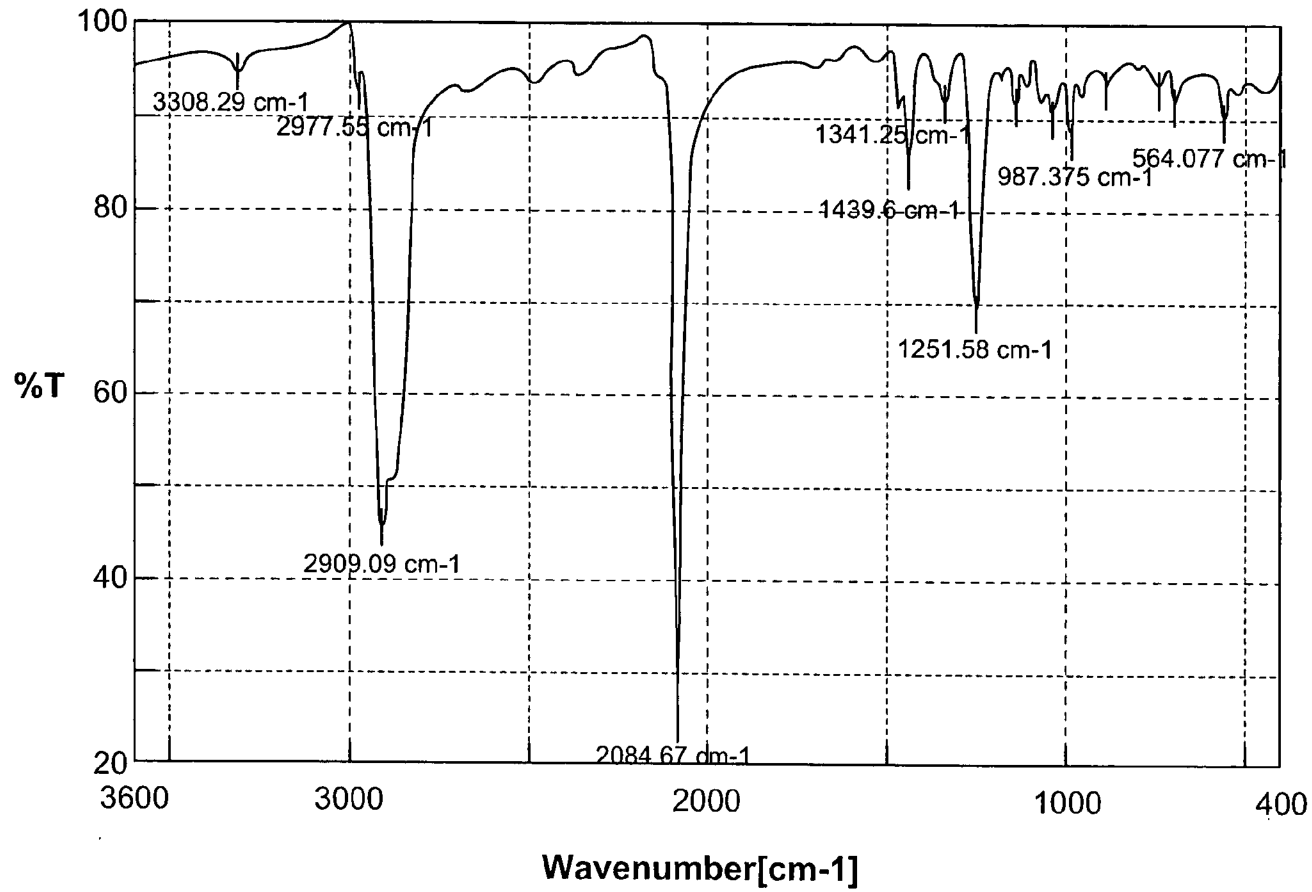


FIG. 58

$^1$H-NMR spectrum of 1-methyl-4-azido-diamantane and 1-methyl-9-azido-diamantane mixture in $CDCl_3$

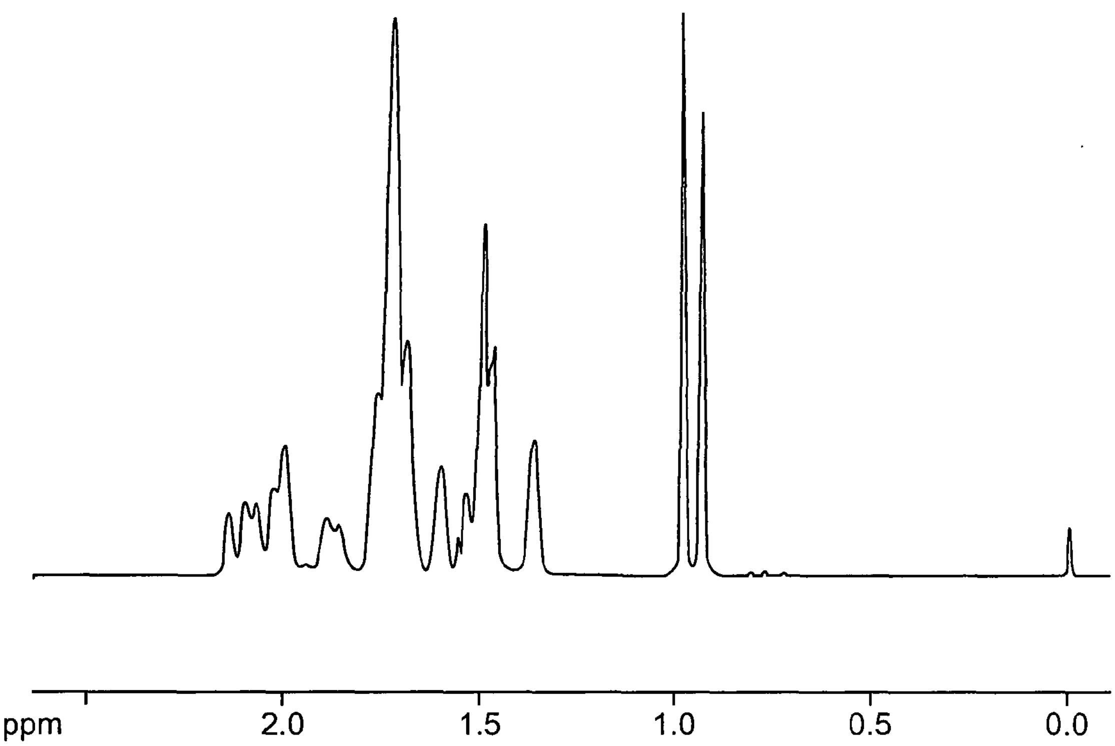


FIG. 59

$^{13}$C-NMR spectrum of 1-methyl-4-azido-diamantane and 1-methyl-9-azido-diamantane mixture in $CDCl_3$

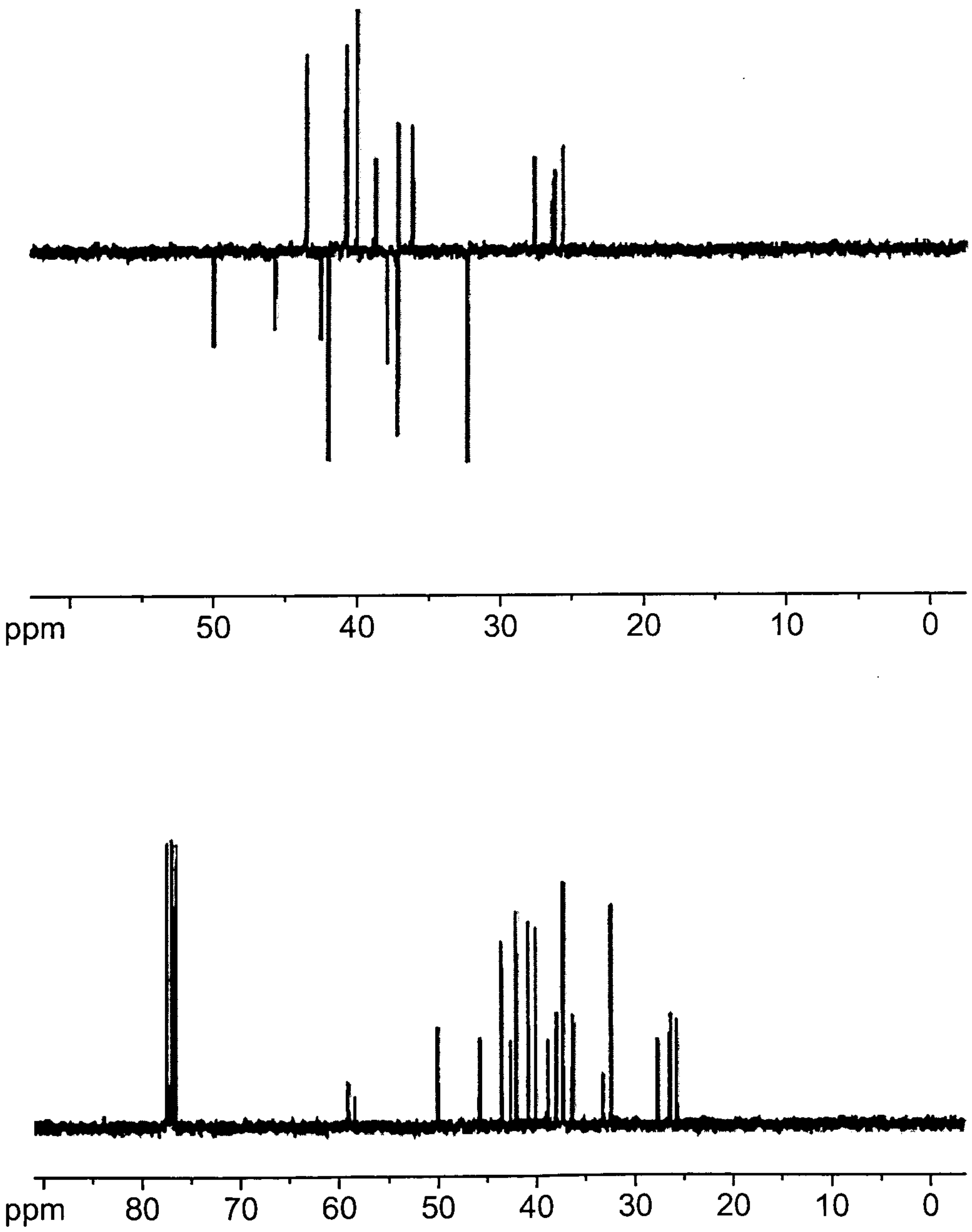


FIG. 60

Figure 61

A

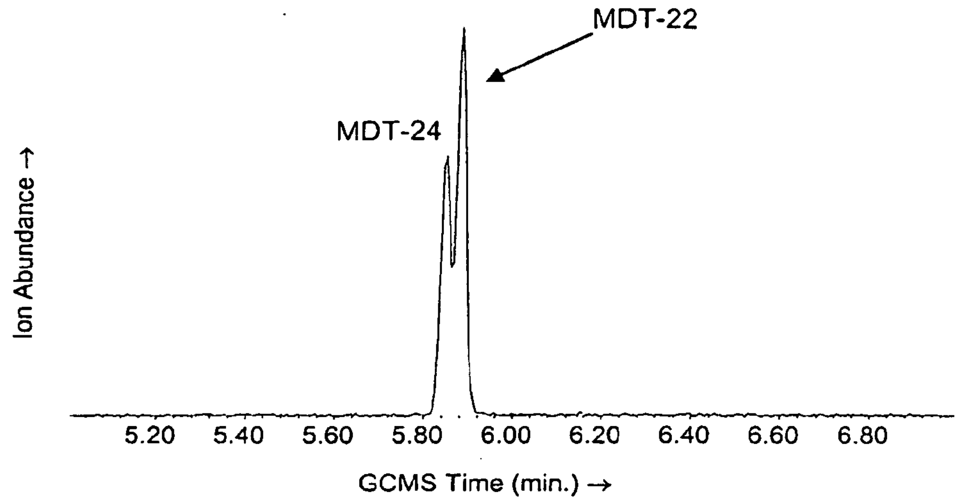


B

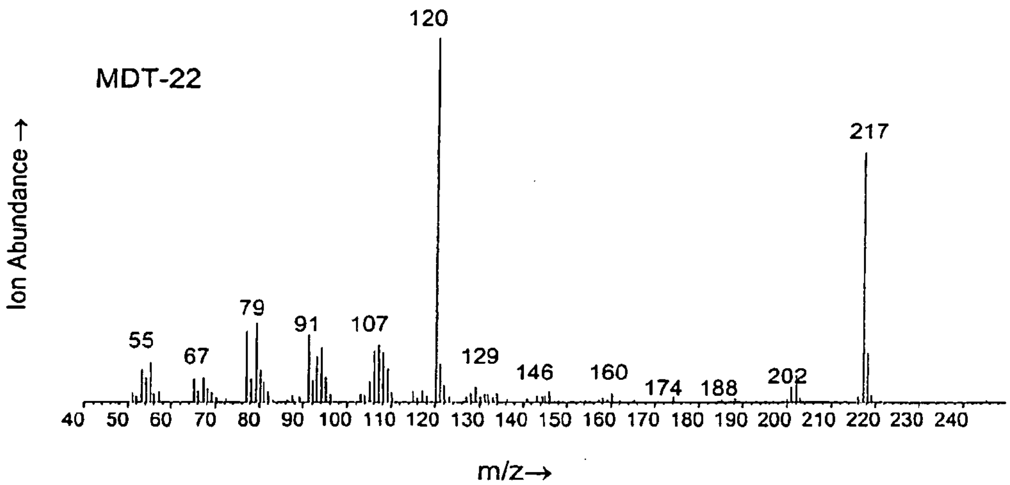


C

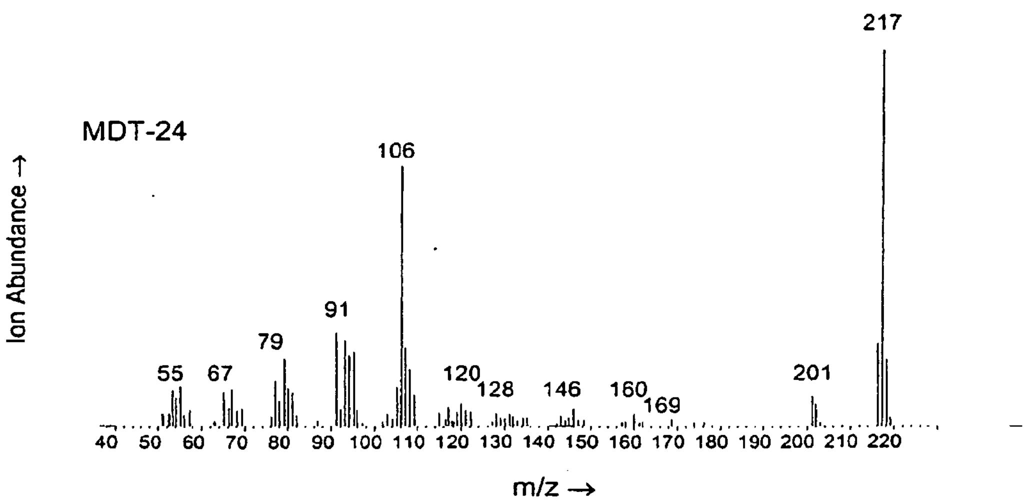

$^1$H-NMR spectrum of 1-methyl-4-amino-diamantane and 1-methyl-9-amino-diamantane mixture

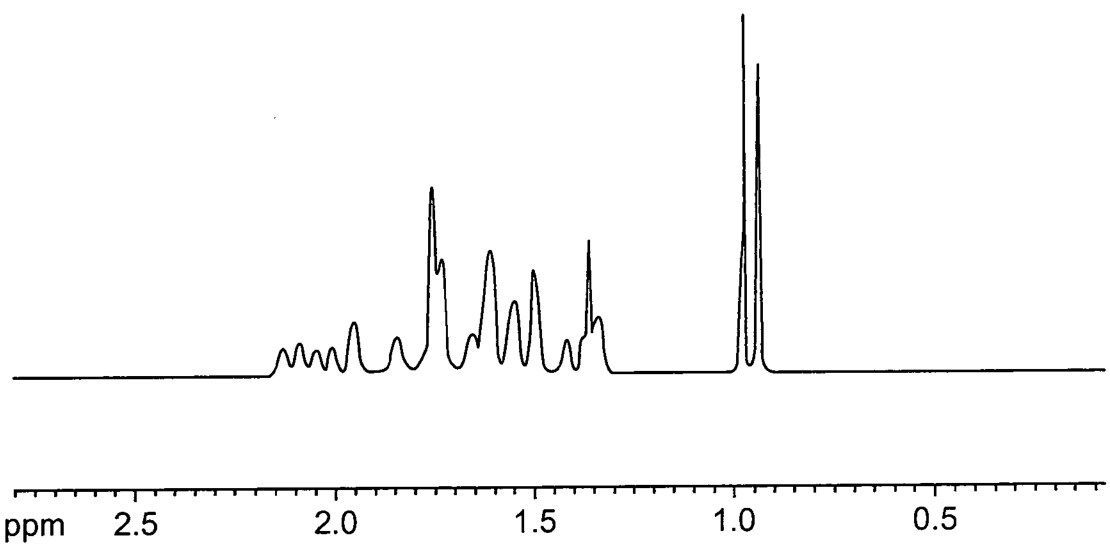


FIG. 62

$^{13}C$-NMR spectrum of 1-methyl-4-amino-diamantane and 1-methyl-9-amino-diamantane mixture

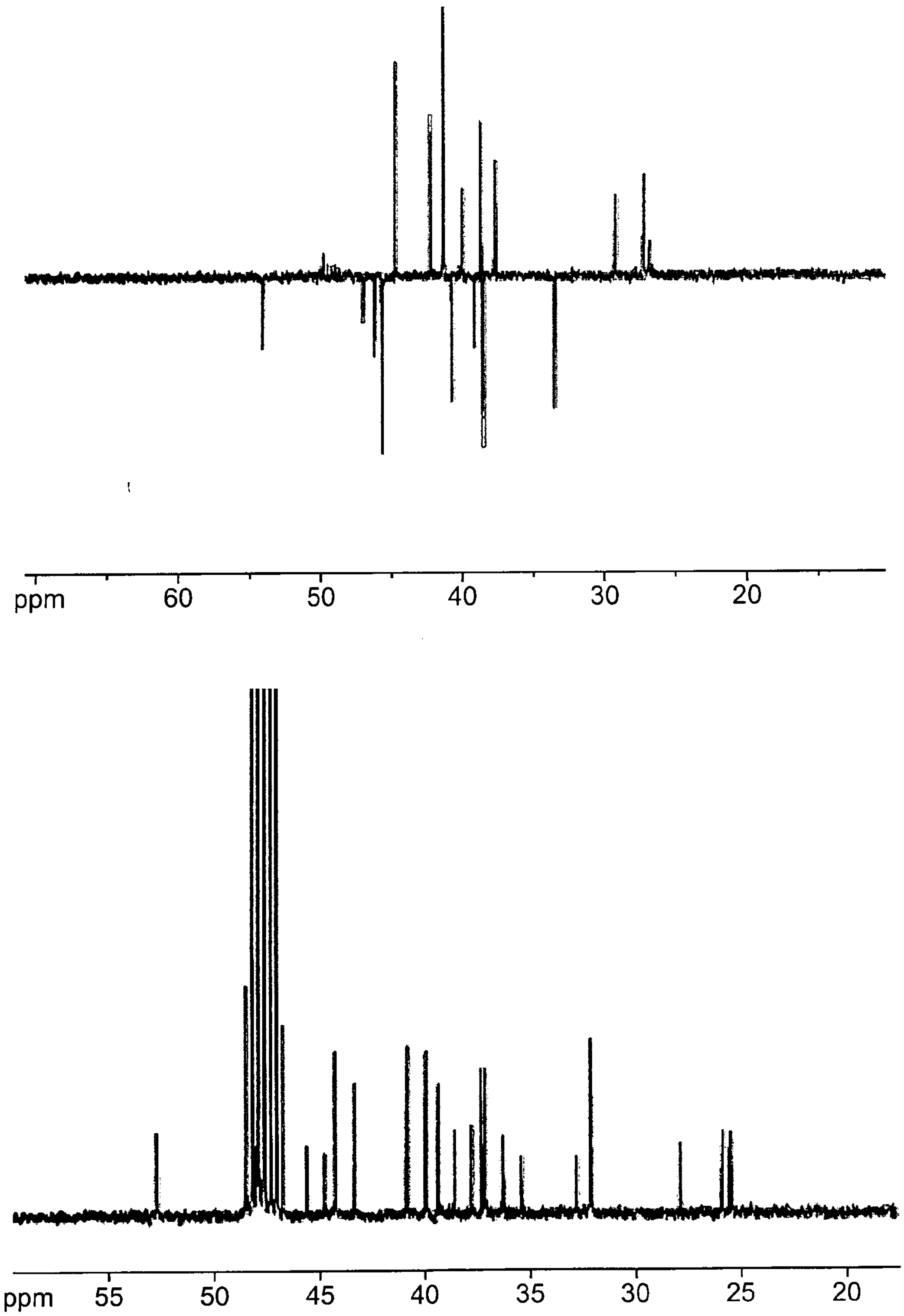


FIG. 63

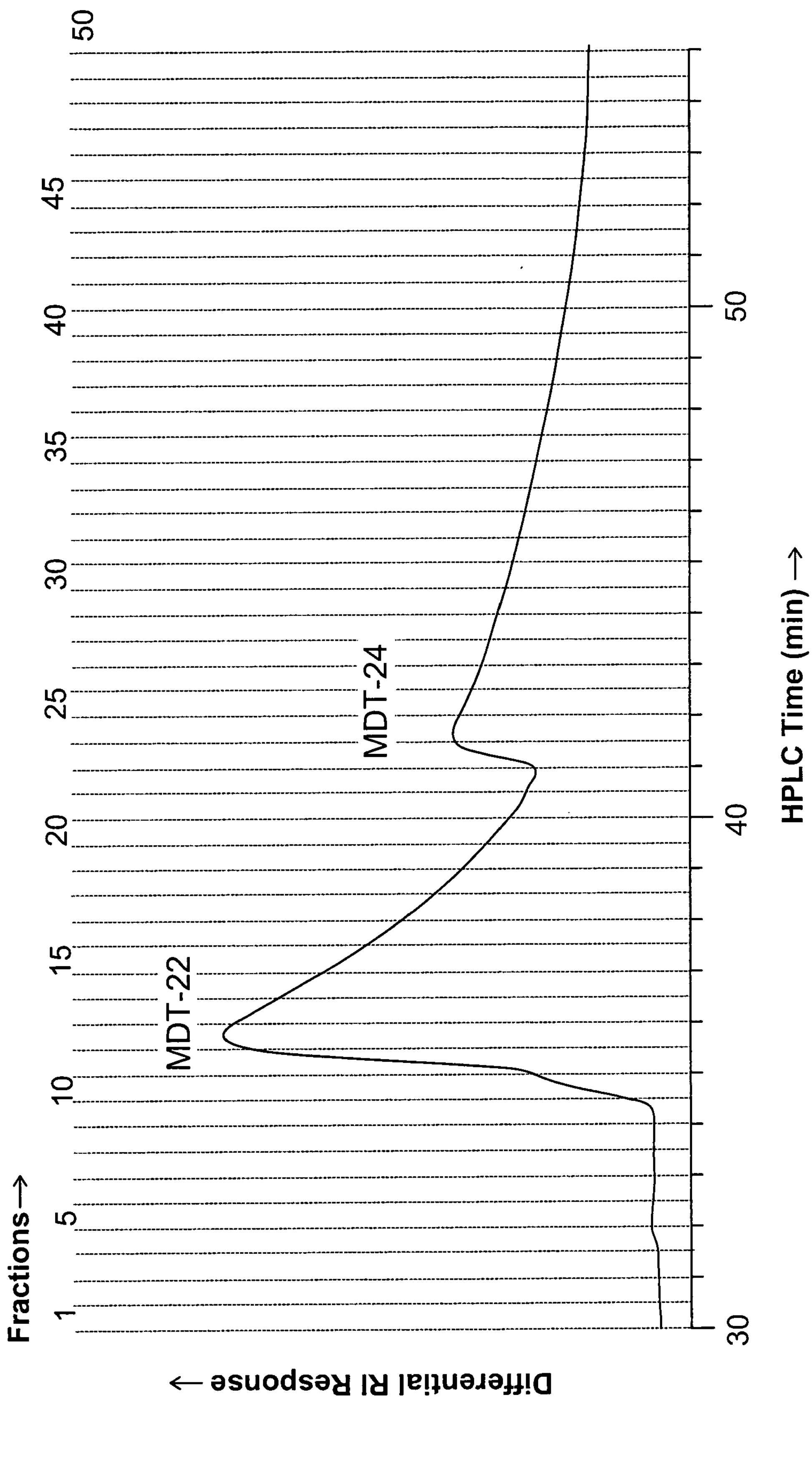
Fractions→
1
5
10
15
20
25
30
35
40
45
50
MDT-22
MDT-24
30
40
50
HPLC Time (min) →
← Differential RI Response →

FIG. 64

Figure 65

A

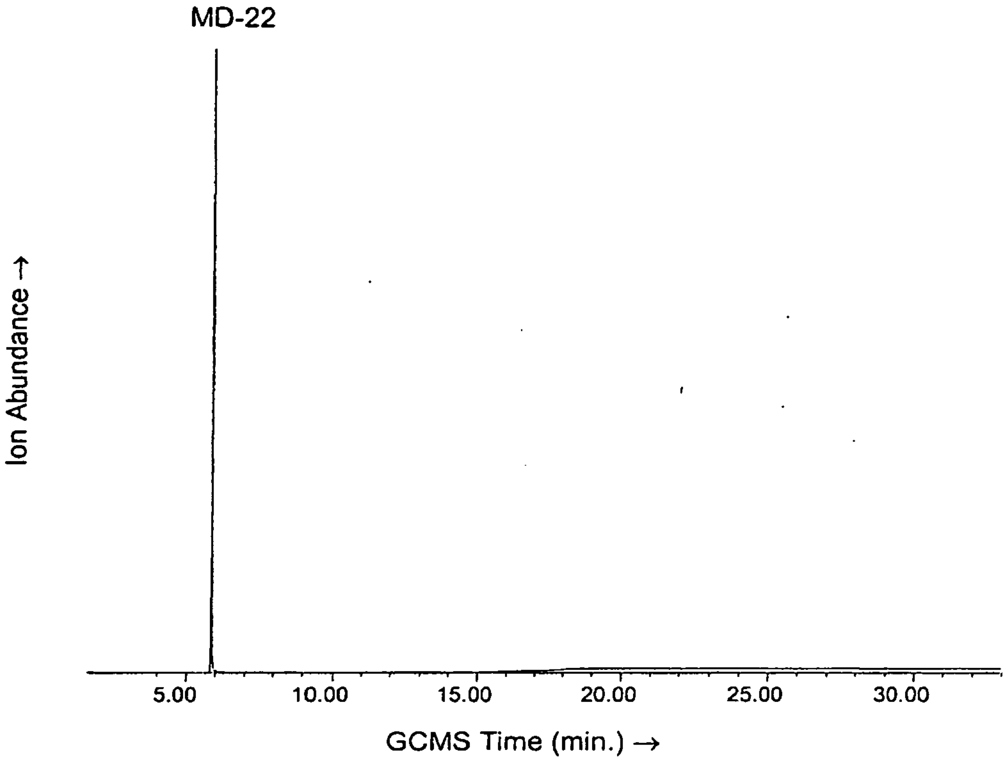
MD-22
Ion Abundance →
5.00
10.00
15.00
20.00
25.00
30.00
GCMS Time (min.) →

B

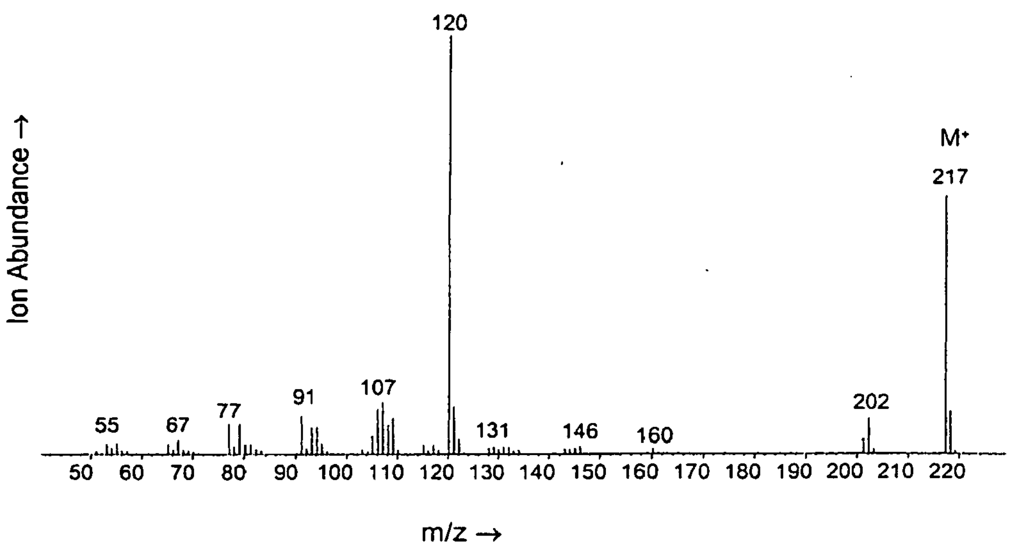
120
M+
217
Ion Abundance →
55
67
77
91
107
131
146
160
202
50 60 70 80 90 100 110 120 130 140 150 160 170 180 190 200 210 220
m/z →

Figure 66

A

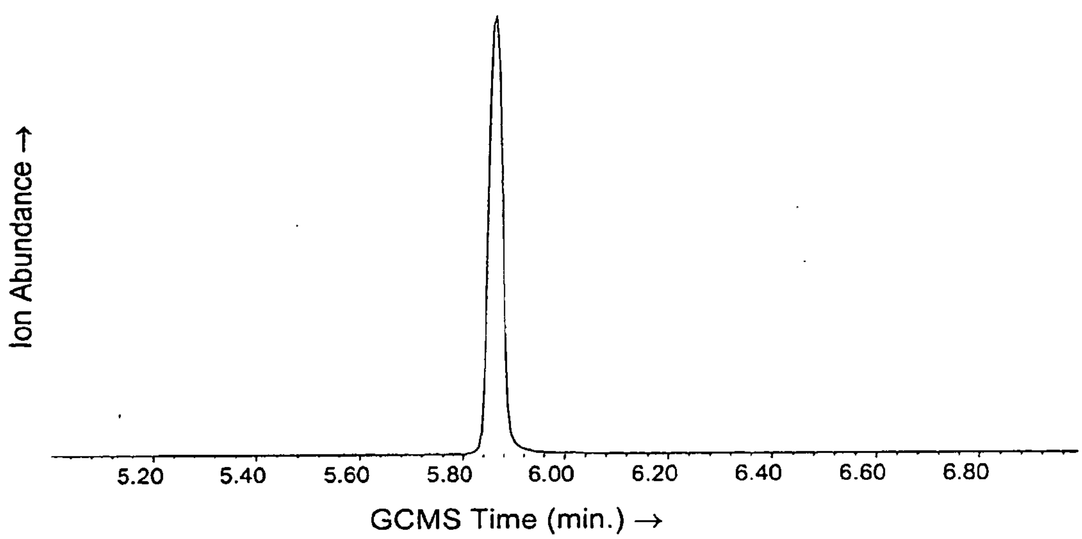
Ion Abundance →
5.20
5.40
5.60
5.80
6.00
6.20
6.40
6.60
6.80
GCMS Time (min.) →

B

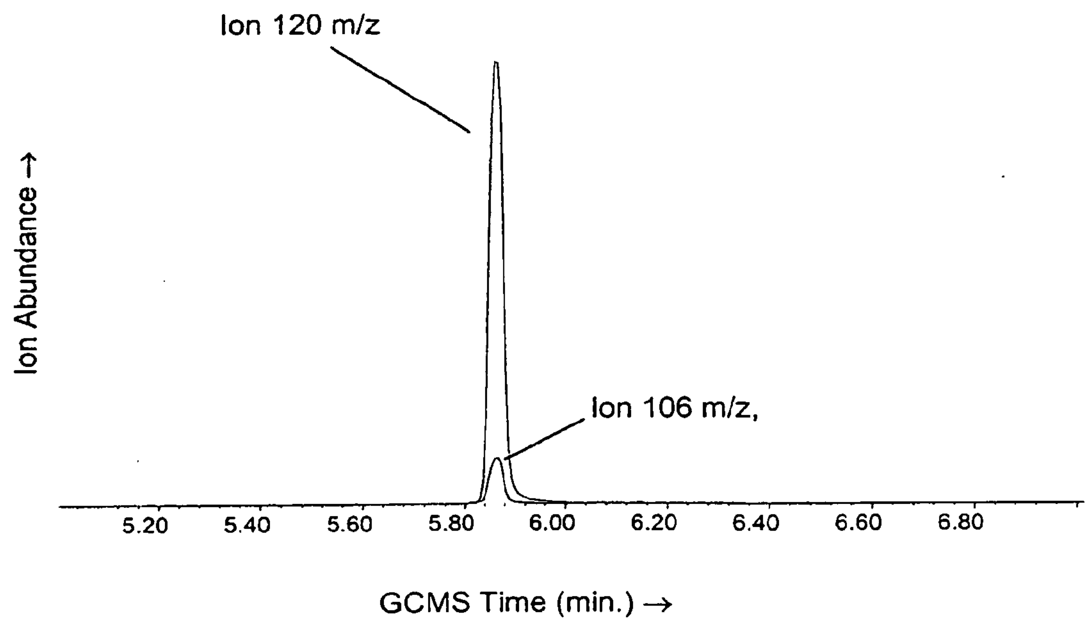
Ion 120 m/z
Ion 106 m/z,
Ion Abundance →
5.20
5.40
5.60
5.80
6.00
6.20
6.40
6.60
6.80
GCMS Time (min.) →

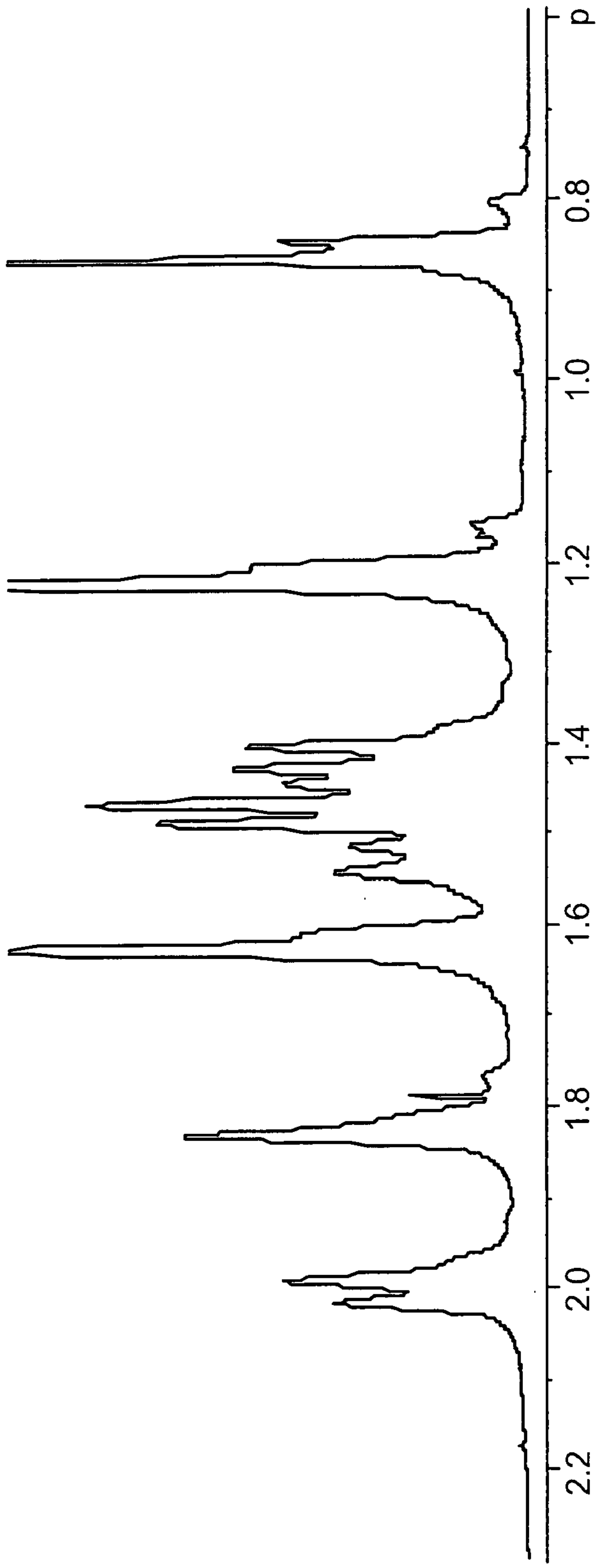


FIG. 67

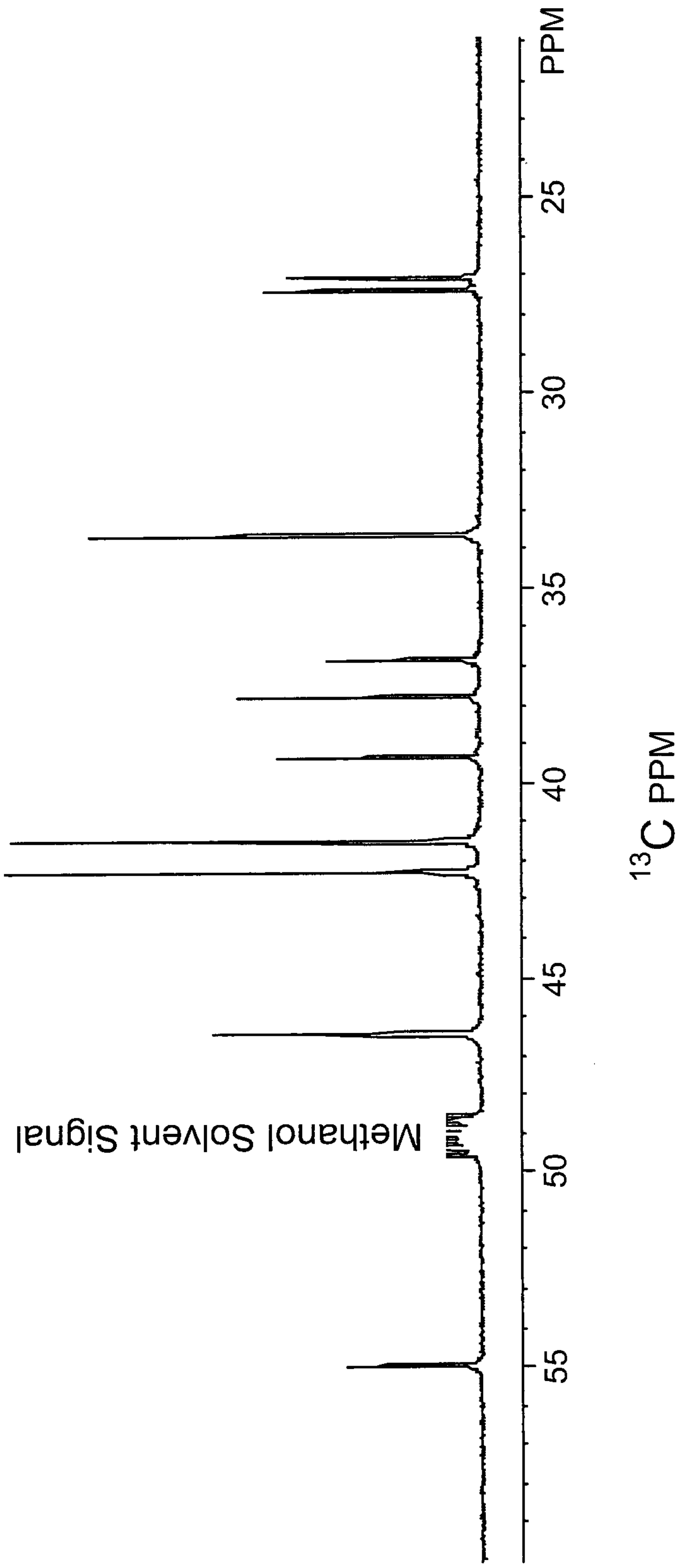


FIG. 68

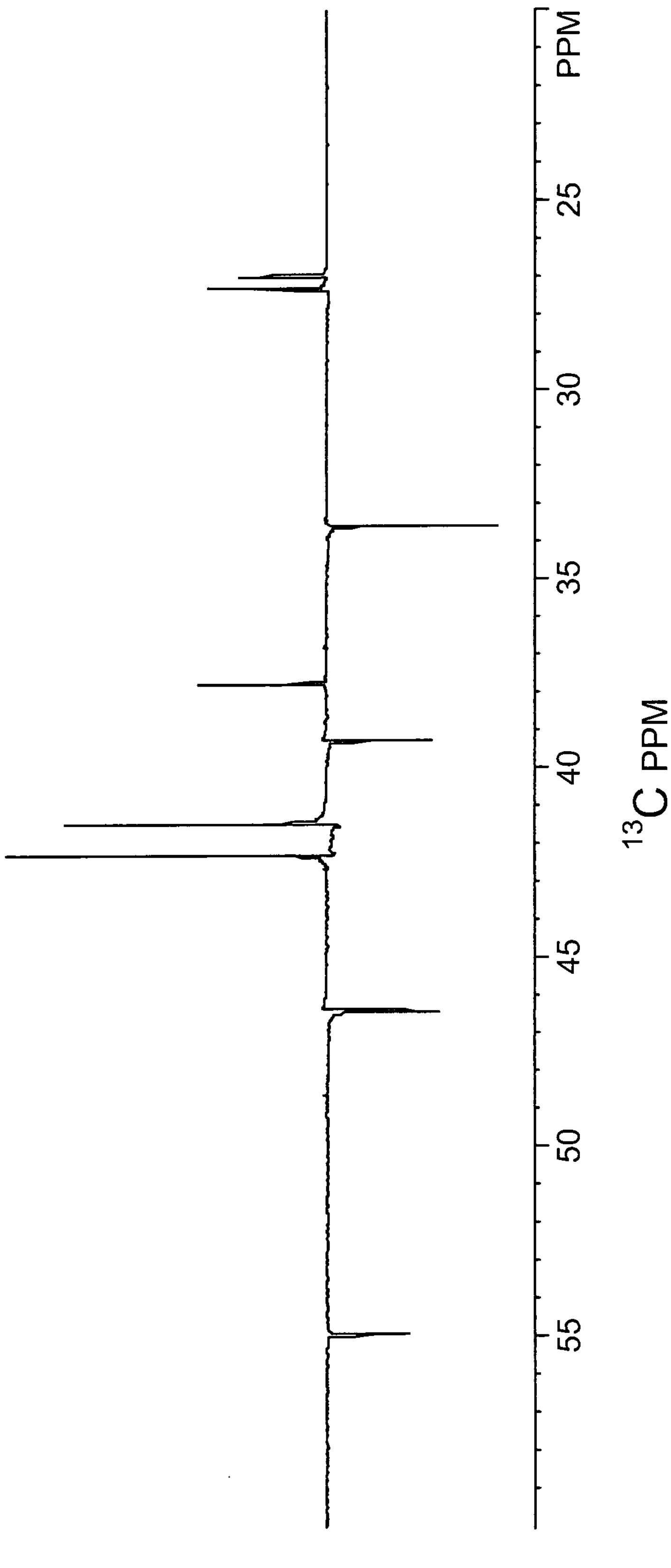


FIG. 69

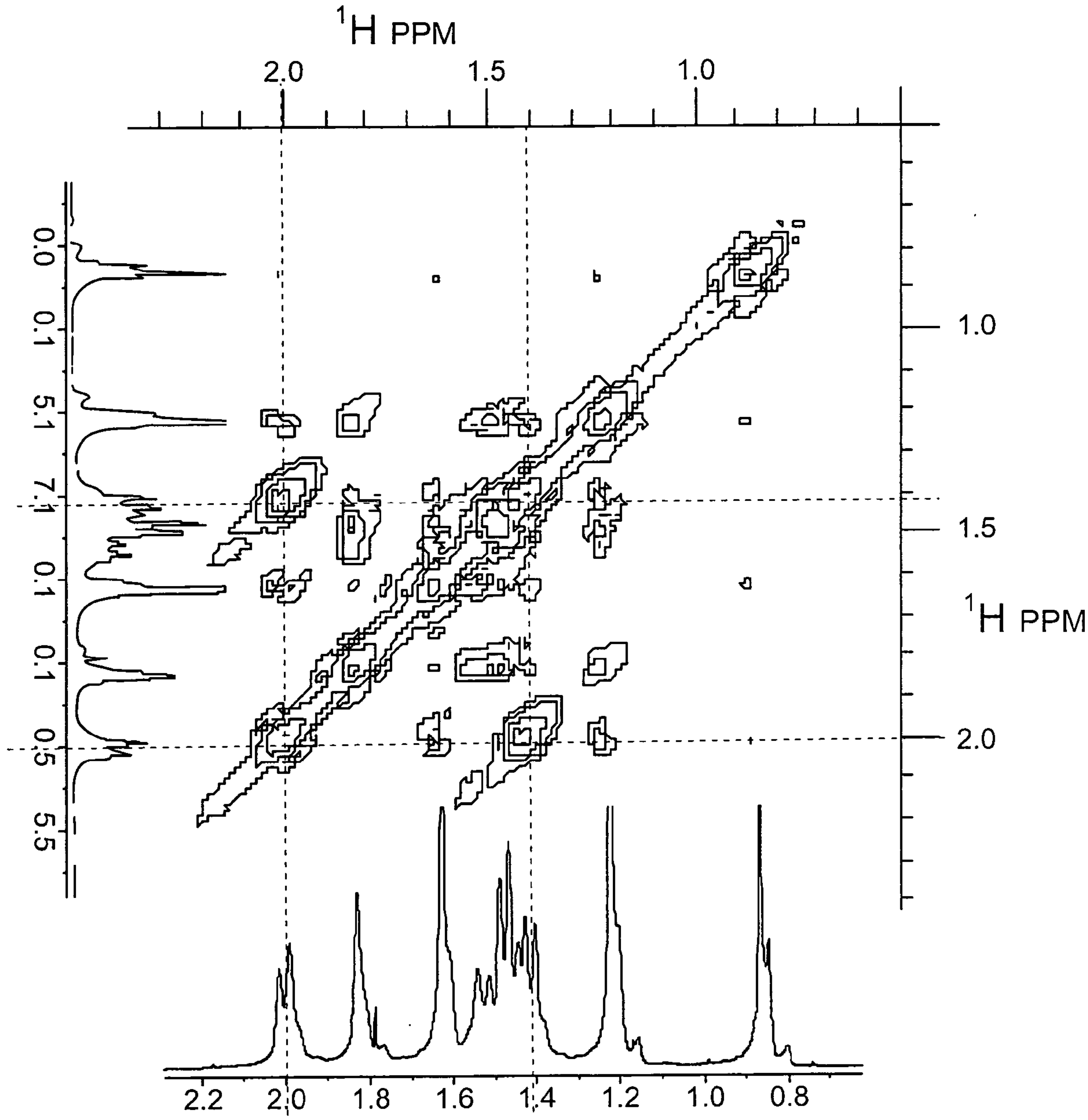


FIG. 70

Figure 71

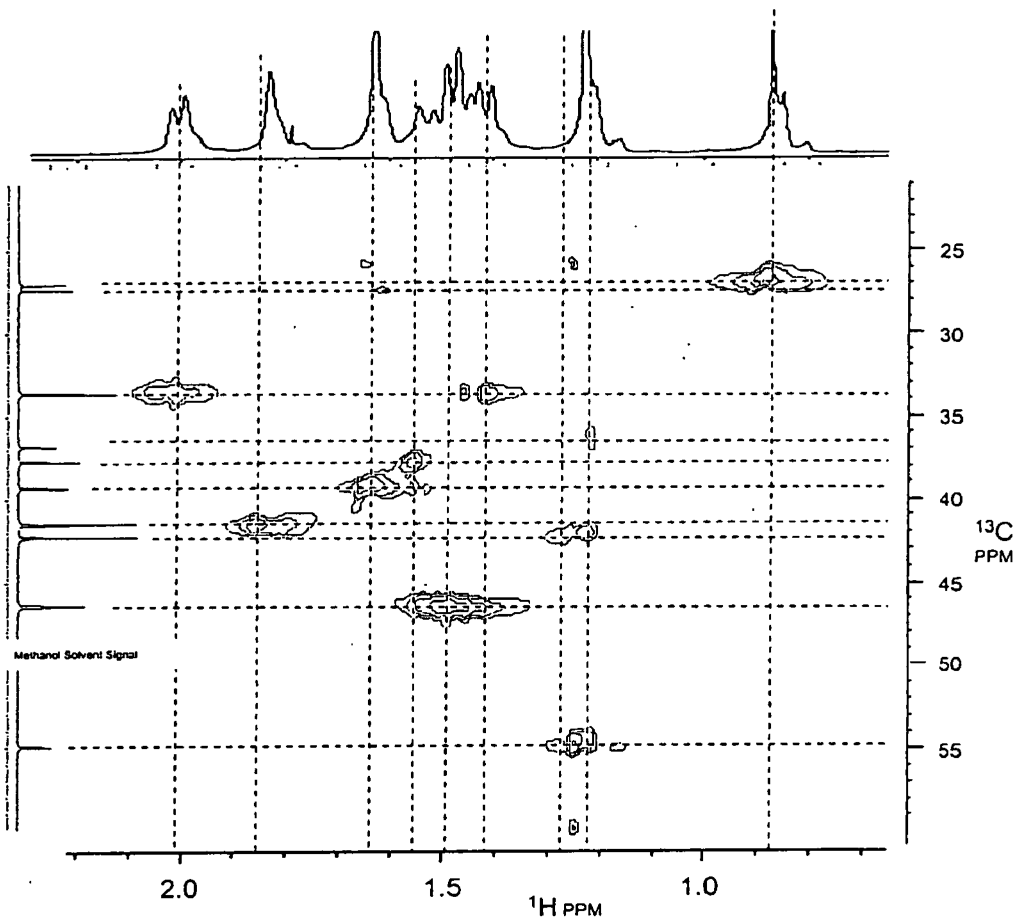
Methanol Solvent Signal
25
30
35
40
45
50
55
13C
PPM
2.0
1.5
1.0
1H PPM

FIG. 72

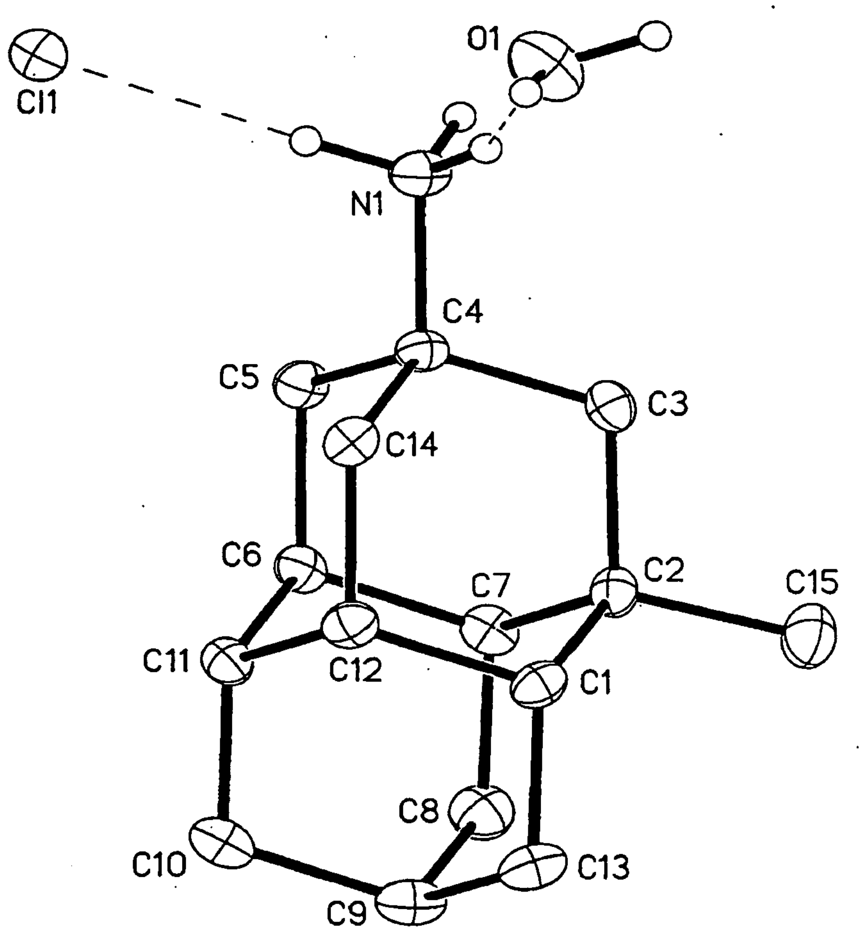

MDT-22

Figure 73

A

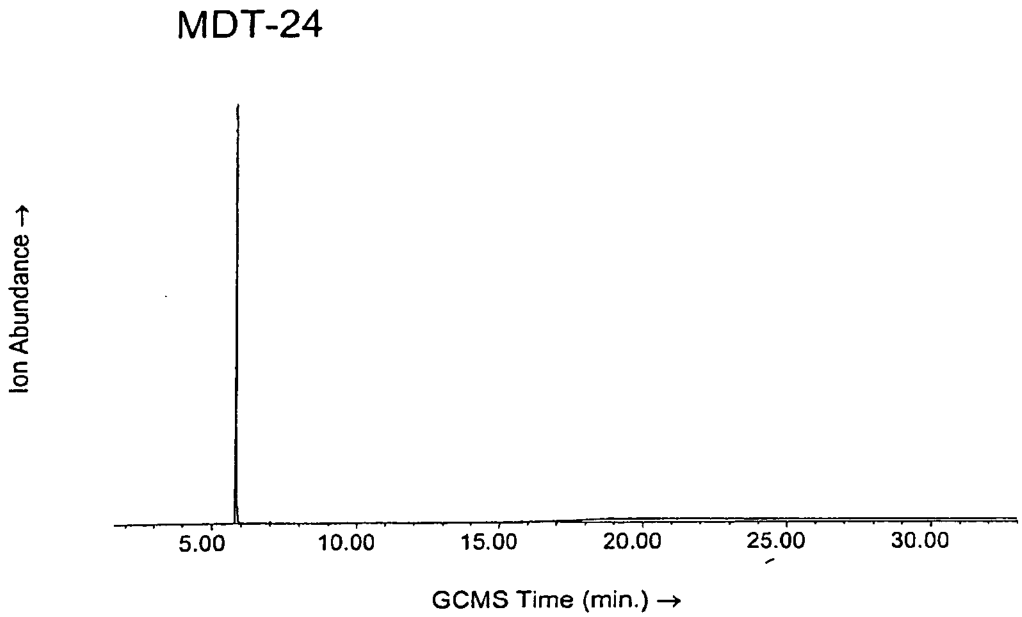
MDT-24
Ion Abundance →
5.00
10.00
15.00
20.00
25.00
30.00
GCMS Time (min.) →

B

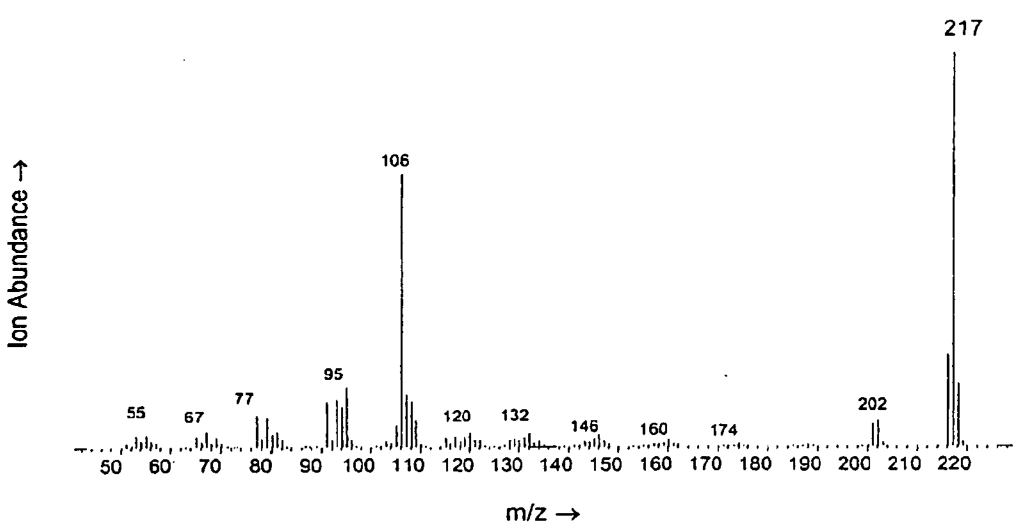
217
106
95
77
55
67
120
132
146
160
174
202
Ion Abundance →
50 60 70 80 90 100 110 120 130 140 150 160 170 180 190 200 210 220
m/z →

# Figure 74

A

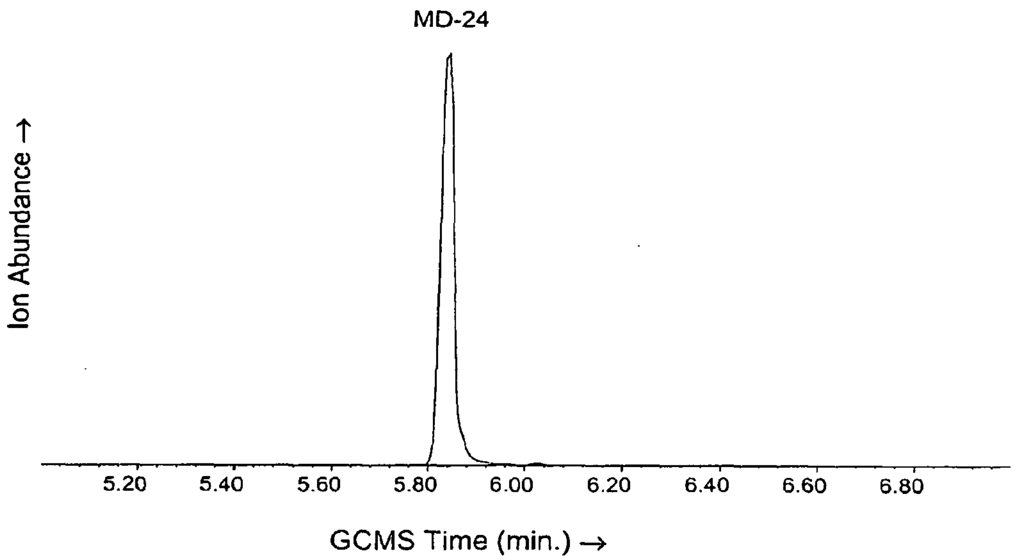
MD-24
Ion Abundance →
5.20
5.40
5.60
5.80
6.00
6.20
6.40
6.60
6.80
GCMS Time (min.) →

B

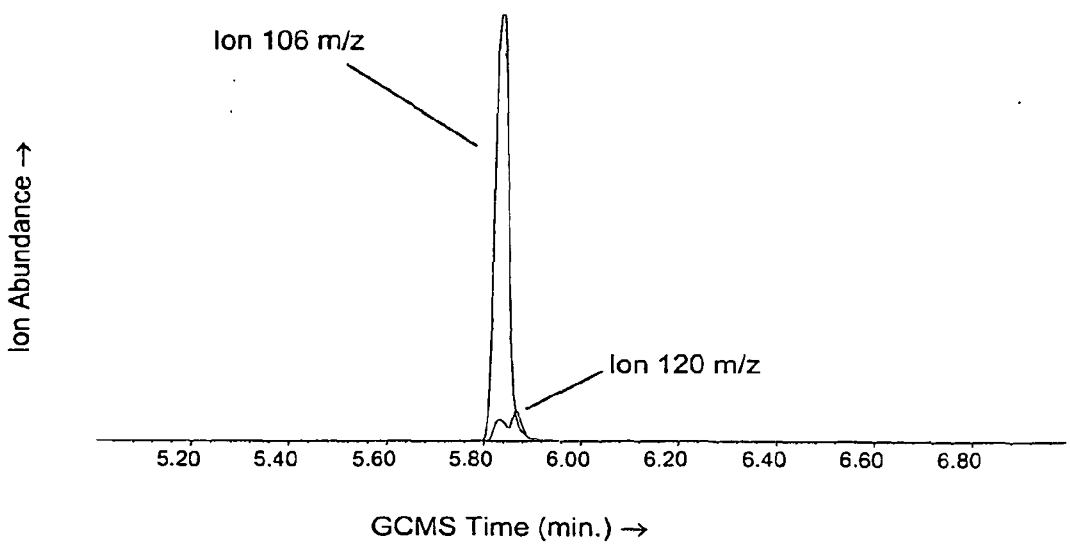
Ion 106 m/z
Ion 120 m/z
Ion Abundance →
5.20
5.40
5.60
5.80
6.00
6.20
6.40
6.60
6.80
GCMS Time (min.) →

FIG. 75

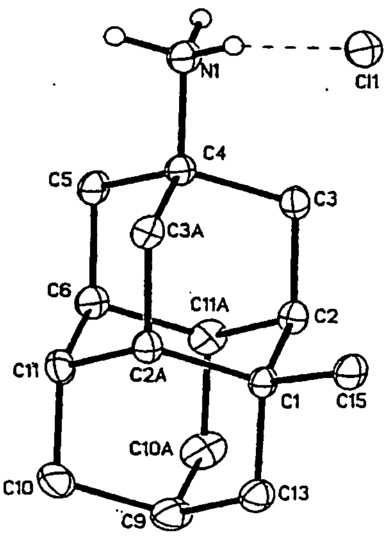

Molecule 1

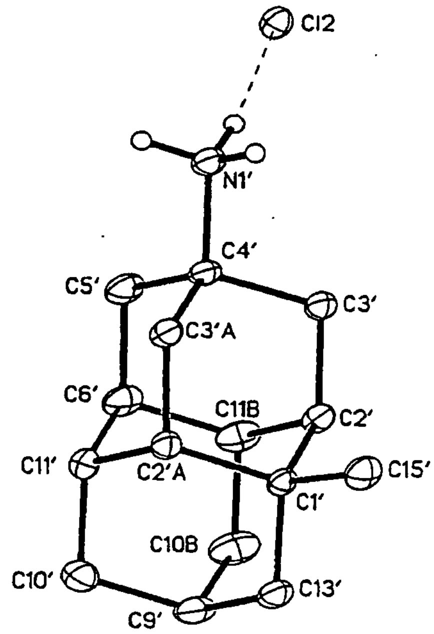

Molecule 2

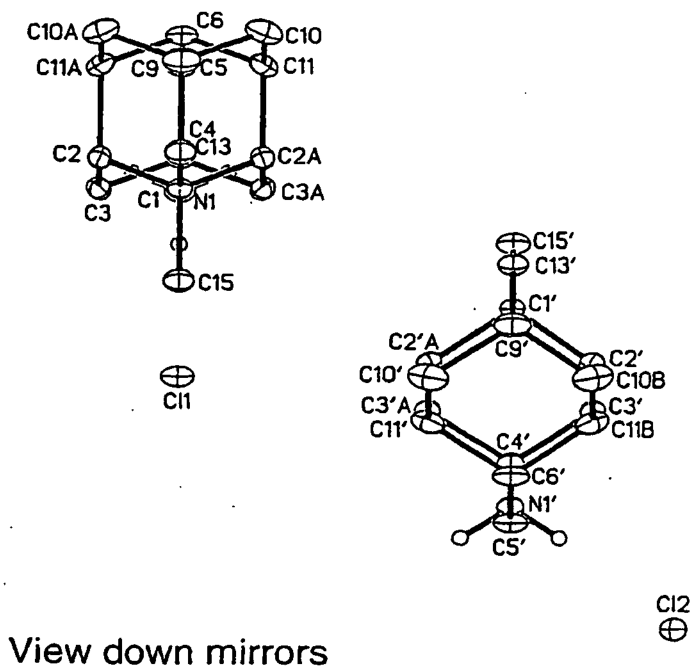

View down mirrors

MDT-24

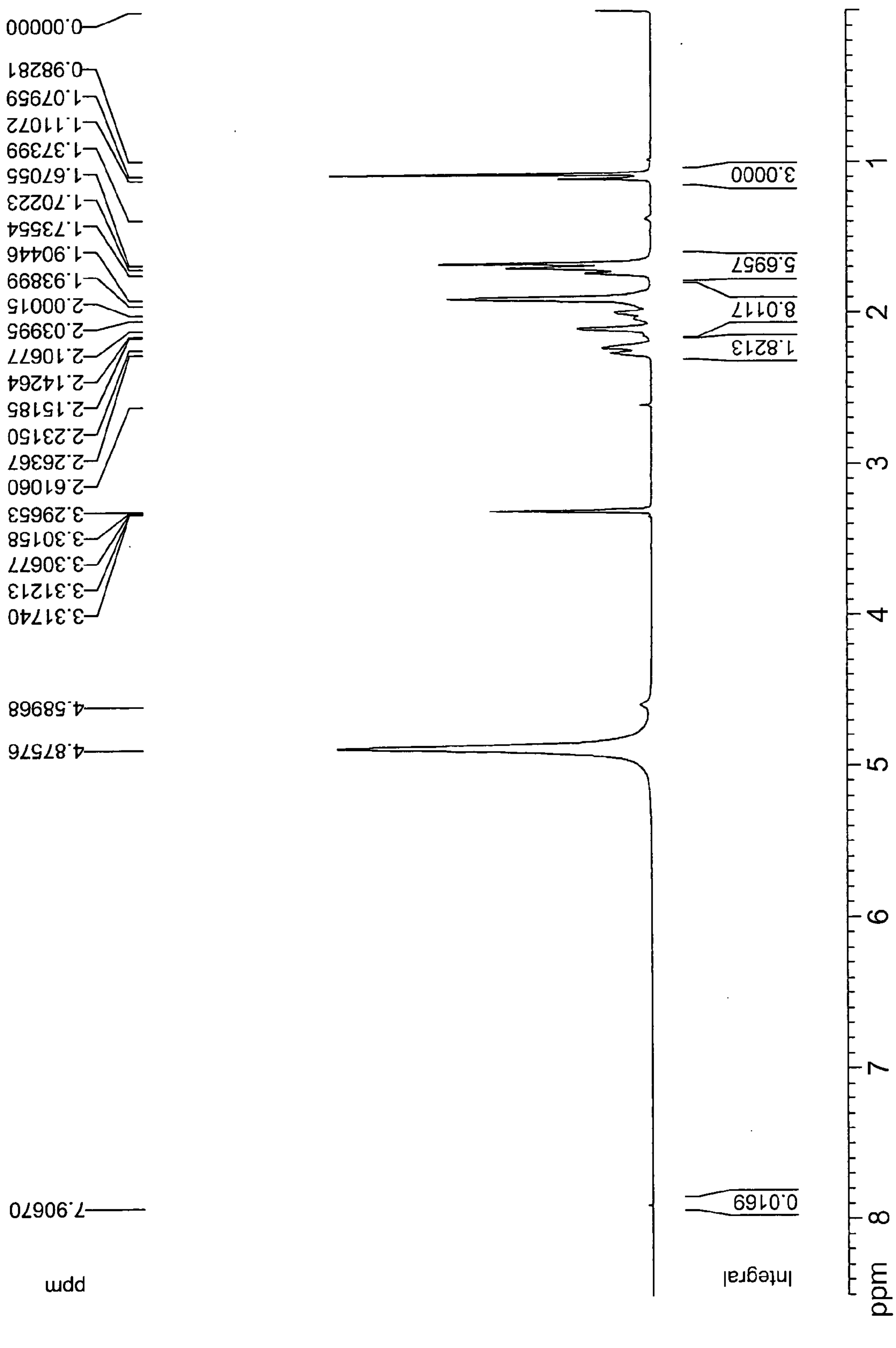
0.00000
0.98281
1.07959
1.11072
1.37399
1.67055
1.70223
1.73554
1.90446
1.93899
2.00015
2.03995
2.10677
2.14264
2.15185
2.23150
2.26367
2.61060
3.29653
3.30158
3.30677
3.31213
3.31740
4.58968
4.87576
7.90670
ppm
3.0000
5.6957
8.0117
1.8213
0.0169
Integral
1
2
3
4
5
6
7
8
ppm

FIG. 76

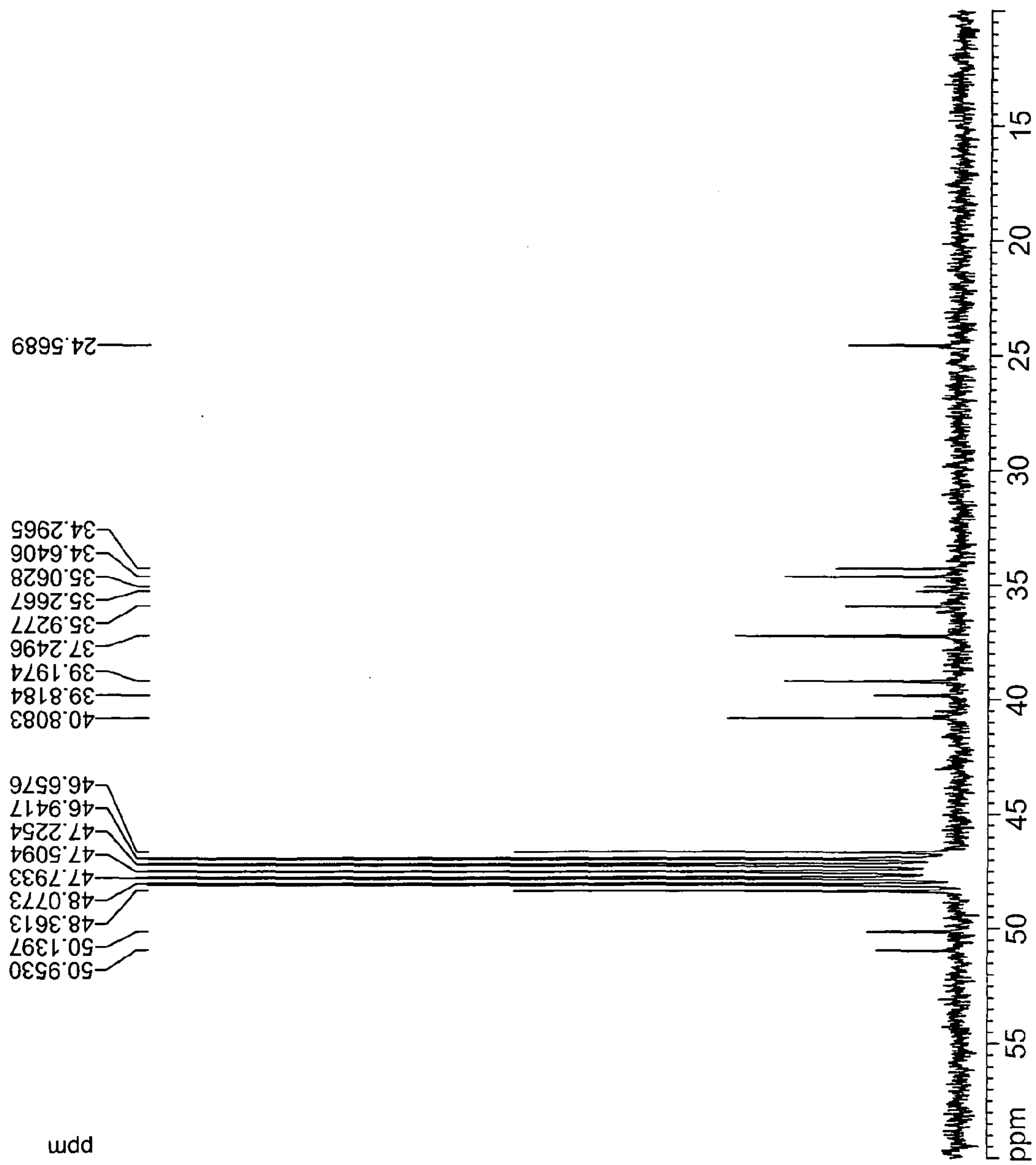
24.5689
34.2965
34.6406
35.0628
35.2667
35.9277
37.2496
39.1974
39.8184
40.8083
46.6576
46.9417
47.2254
47.5094
47.7933
48.0773
48.3613
50.1397
50.9530
ppm
15
20
25
30
35
40
45
50
55
ppm

FIG. 77

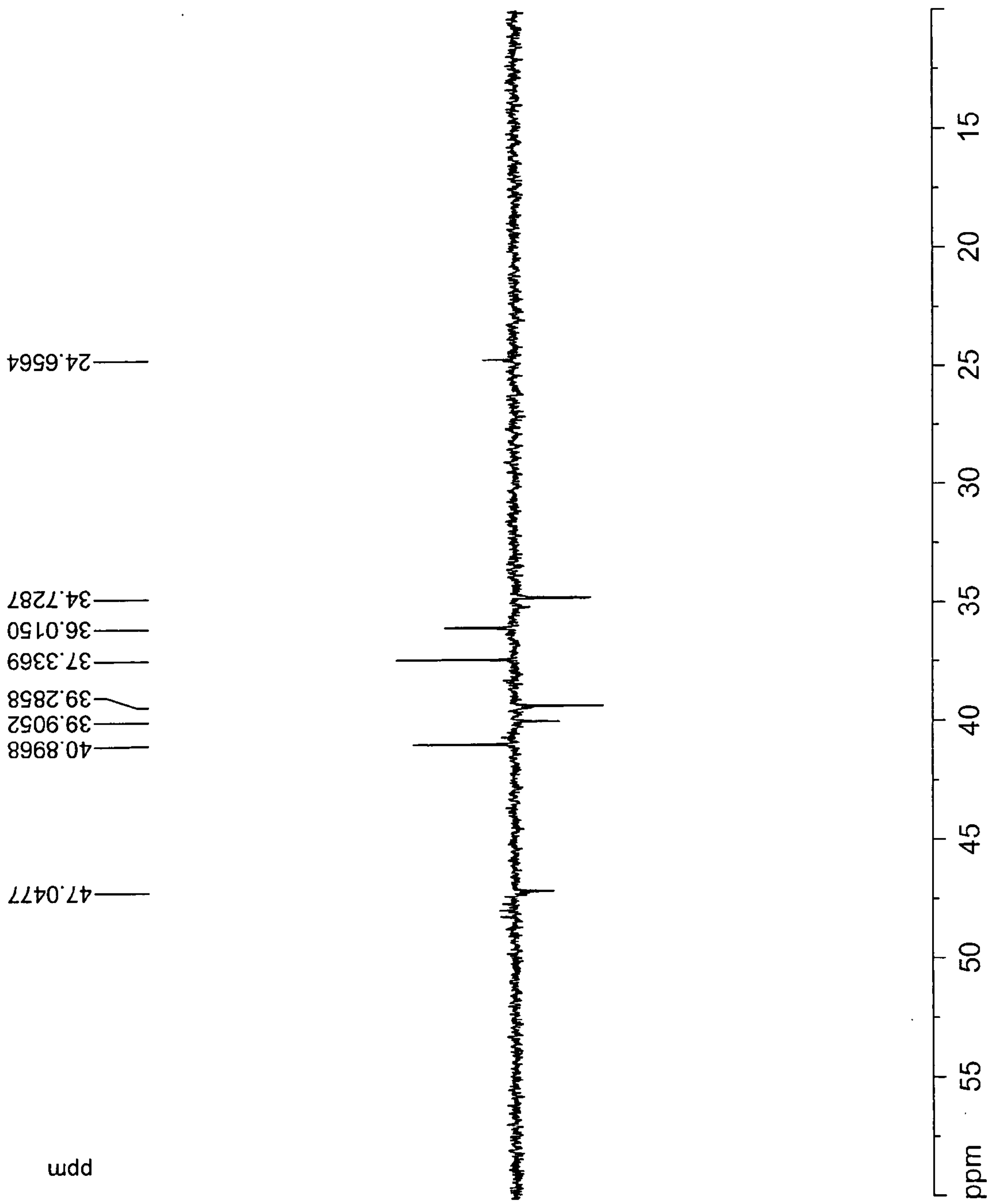
24.6564
34.7287
36.0150
37.3369
39.2858
39.9052
40.8968
47.0477
ppm
15
20
25
30
35
40
45
50
55
ppm

FIG. 78

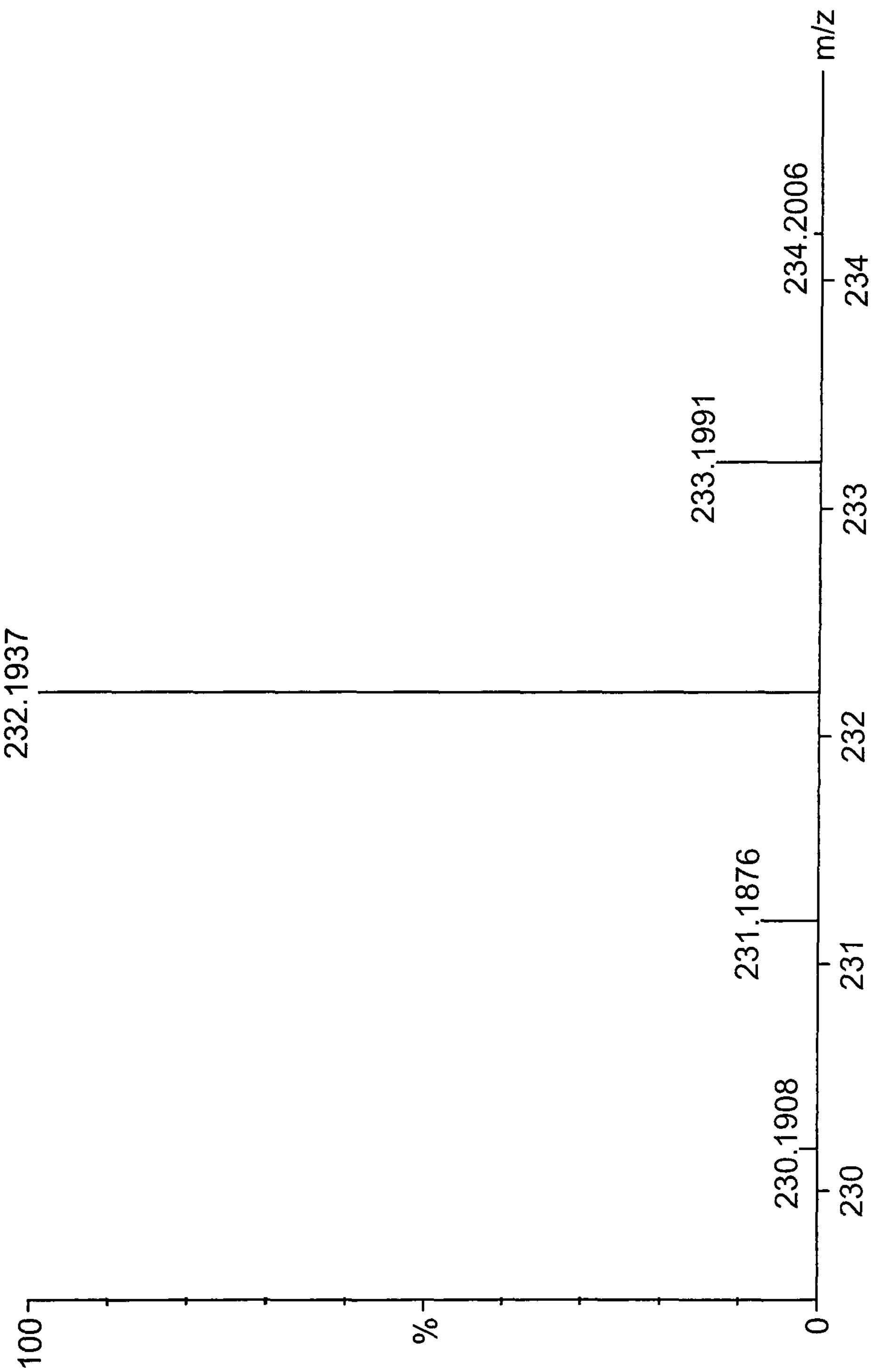
100
%
0
230
231
232
233
234
m/z
230.1908
231.1876
232.1937
233.1991
234.2006

FIG. 79

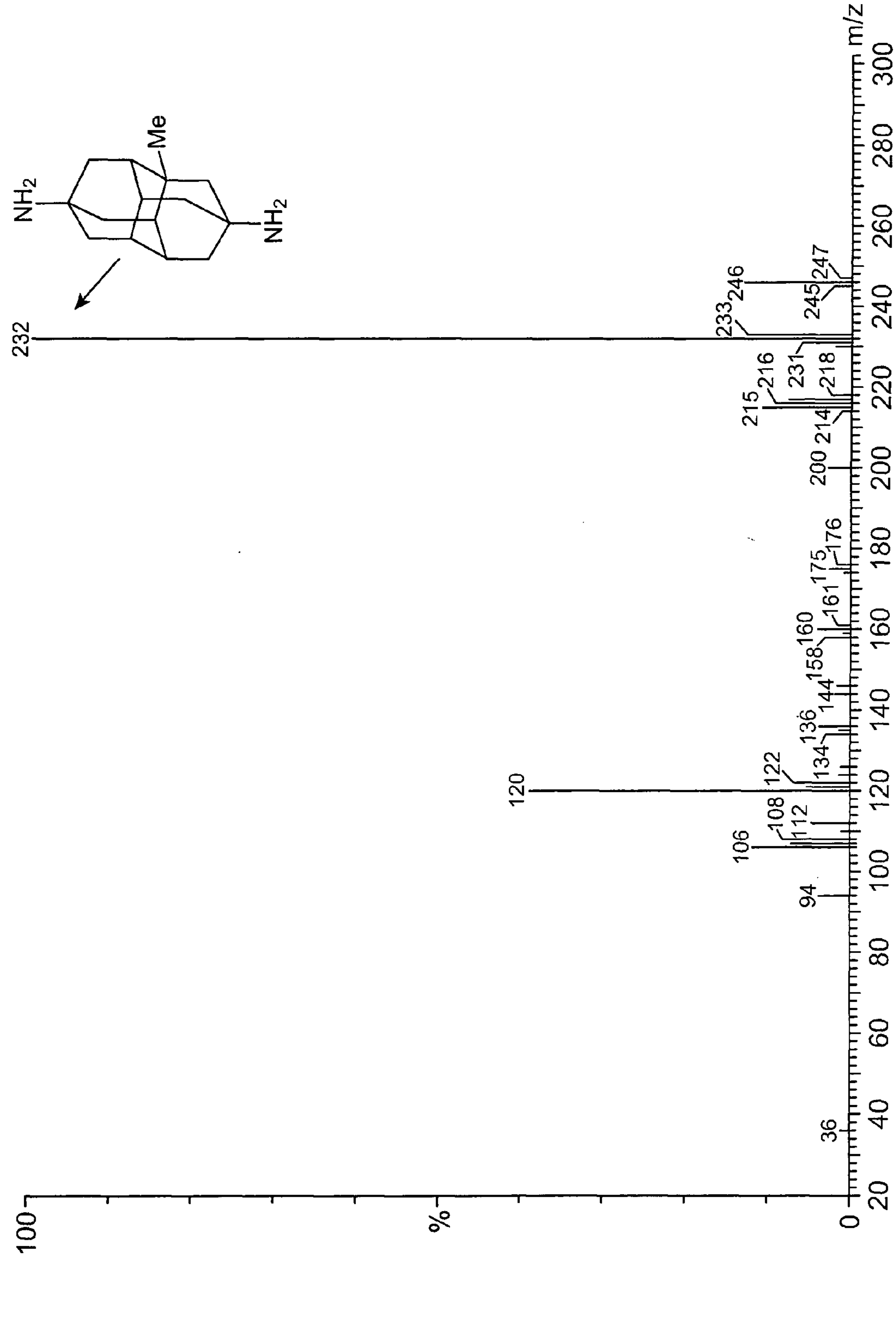
100
%
0
20
40
60
80
100
120
140
160
180
200
220
240
260
280
300
m/z
36
94
106
108
112
120
122
134
136
144
158
160
161
175
176
200
214
215
216
218
231
232
233
245
246
247

FIG. 80

Figure 81

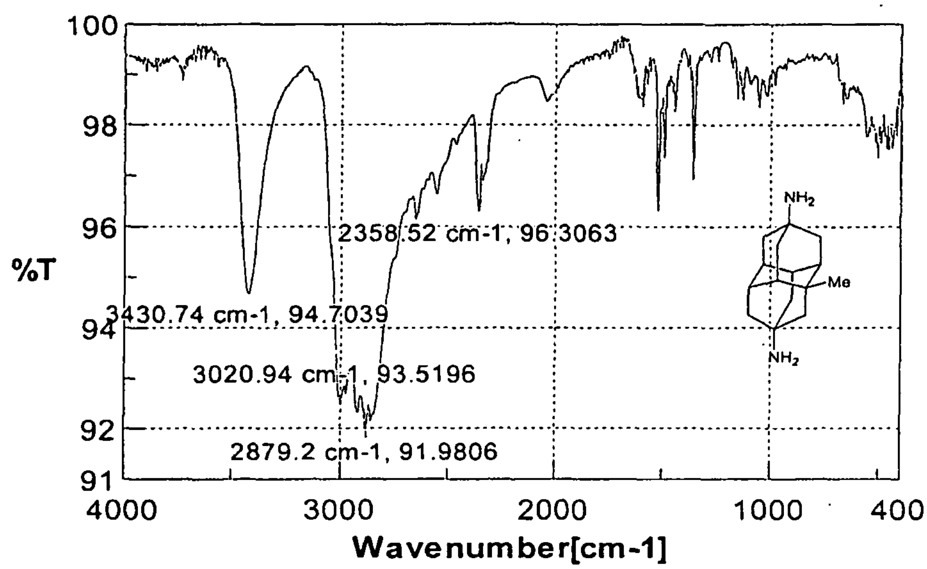
100
98
96
94
92
91
%T
2358.52 cm-1, 96.3063
3430.74 cm-1, 94.7039
3020.94 cm-1, 93.5196
2879.2 cm-1, 91.9806
4000
3000
2000
1000
400
Wavenumber[cm-1]

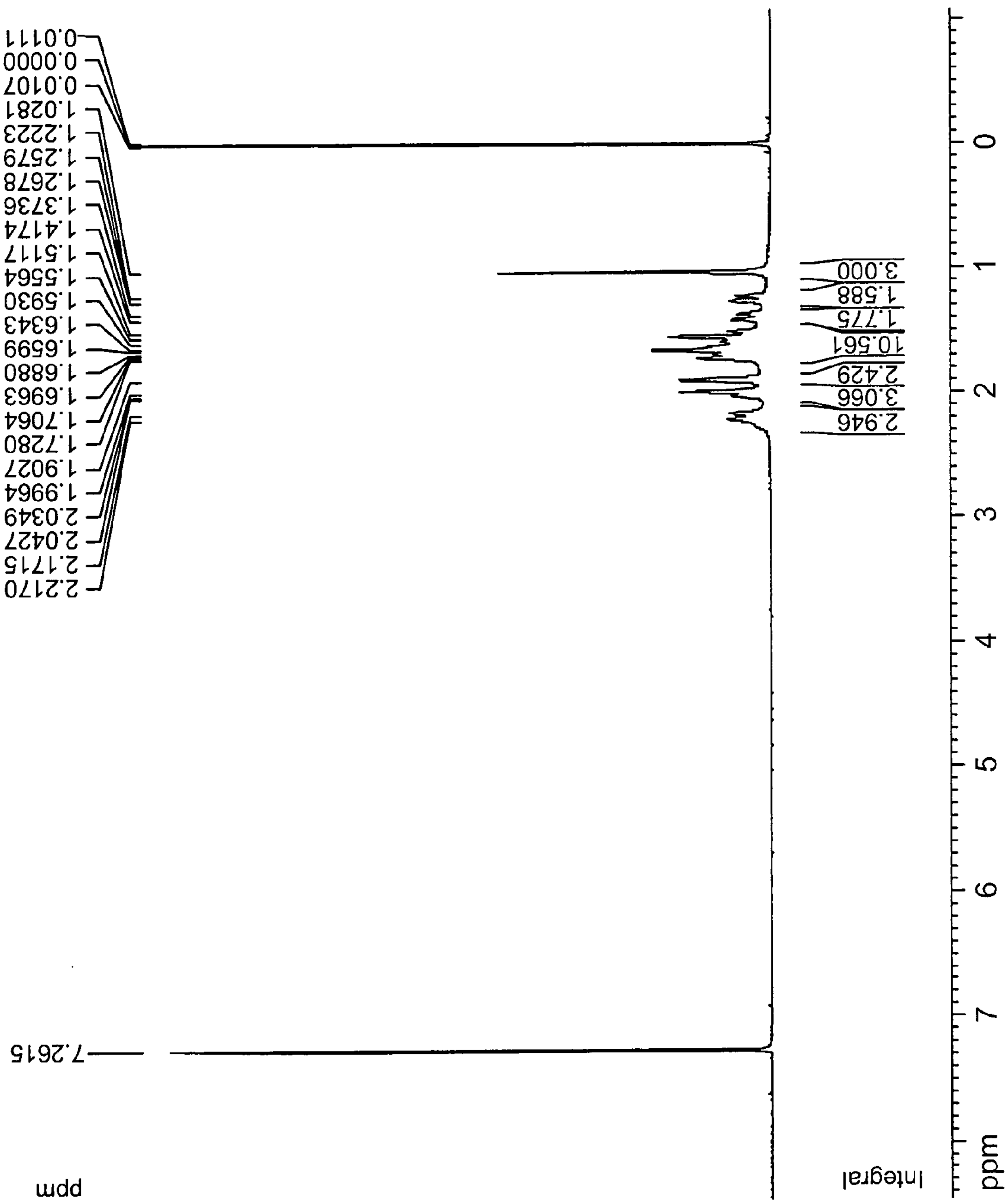

0.0111
0.0000
0.0107
1.0281
1.2223
1.2579
1.2678
1.3736
1.4174
1.5117
1.5564
1.5930
1.6343
1.6599
1.6880
1.6963
1.7064
1.7280
1.9027
1.9964
2.0349
2.0427
2.1715
2.2170
7.2615
ppm
3.000
1.588
1.775
10.561
2.429
3.066
2.946
Integral
0
1
2
3
4
5
6
7
ppm


FIG. 82

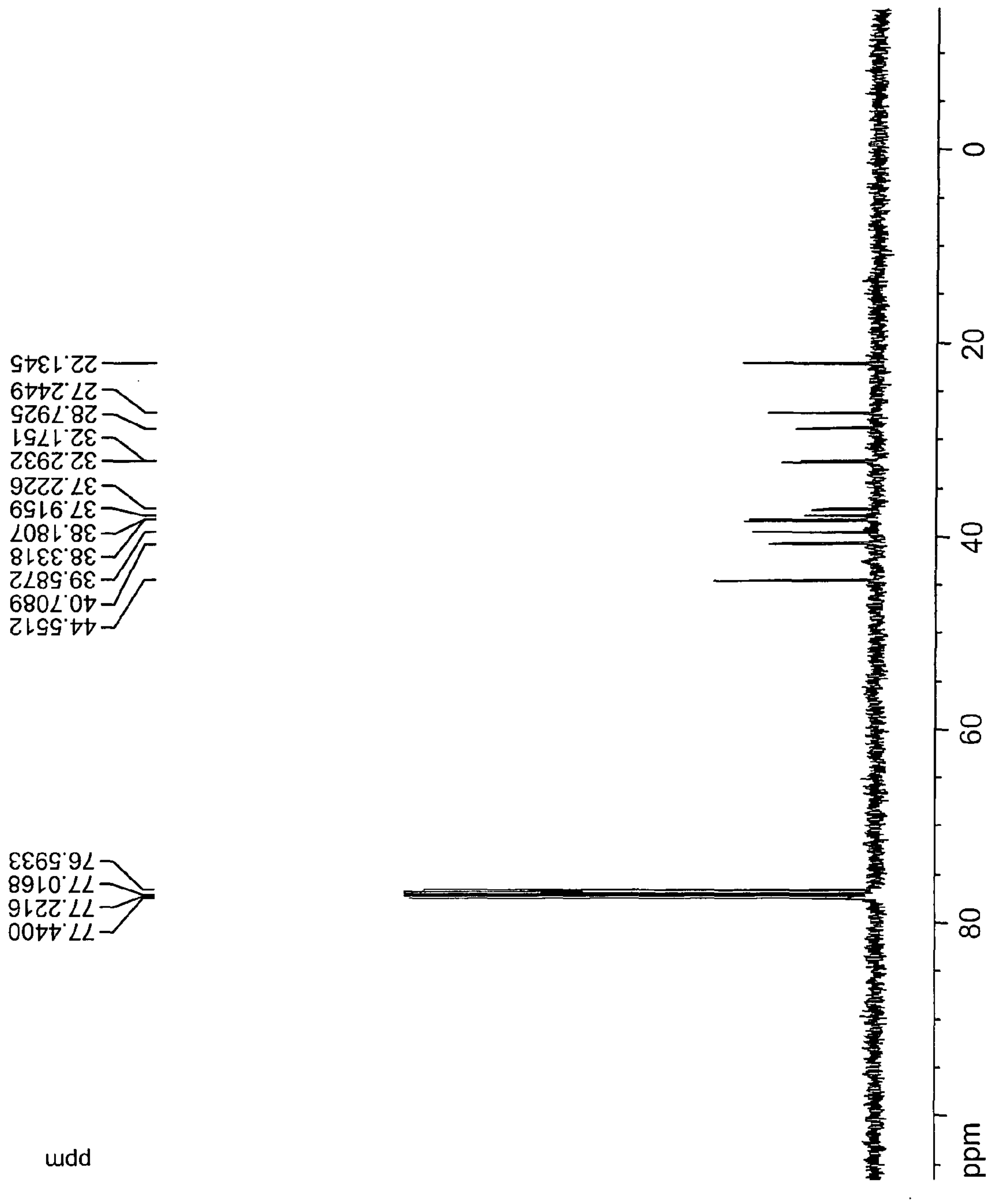
22.1345
27.2449
28.7925
32.1751
32.2932
37.2226
37.9159
38.1807
38.3318
39.5872
40.7089
44.5512
76.5933
77.0168
77.2216
77.4400
ppm
0
20
40
60
80
ppm

FIG. 83

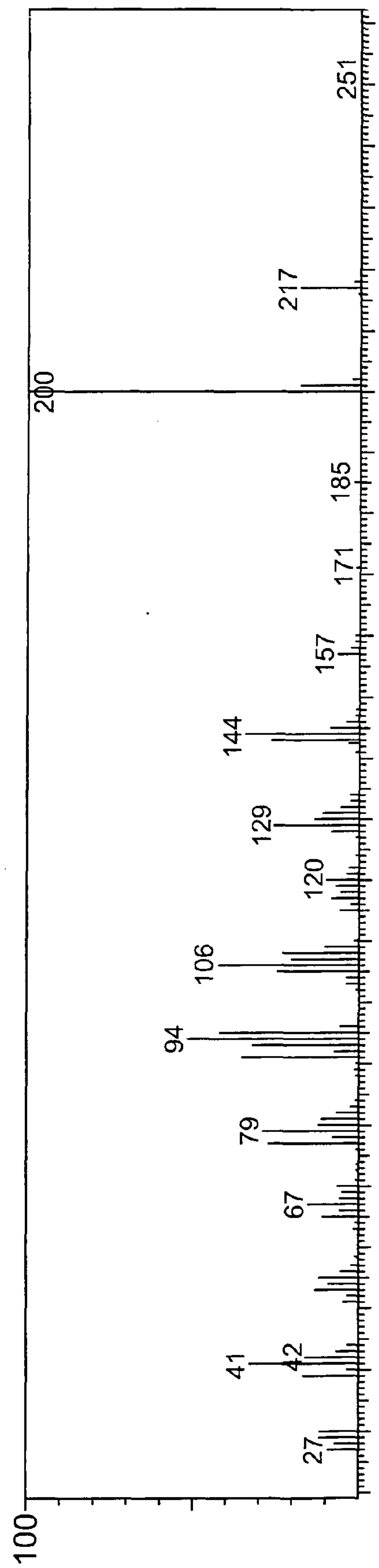


FIG. 84

FIG. 85

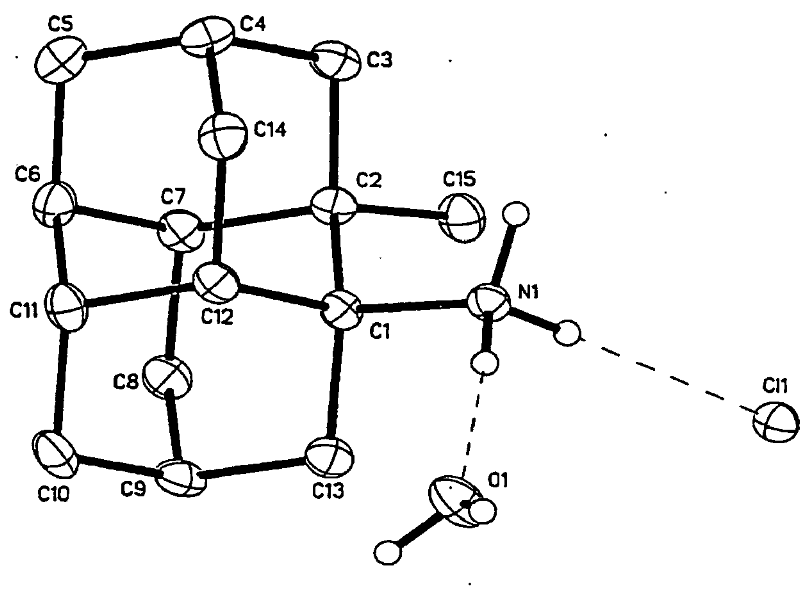

MDT-30

Figure 86

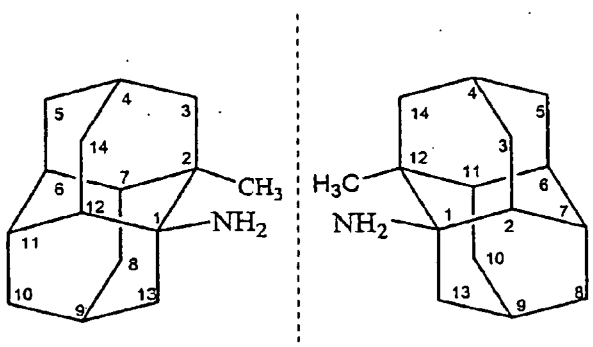

MDT-30: Racemic mixture of 1-amino-2-methyldiamantane and 1-amino12-methyldiamantane

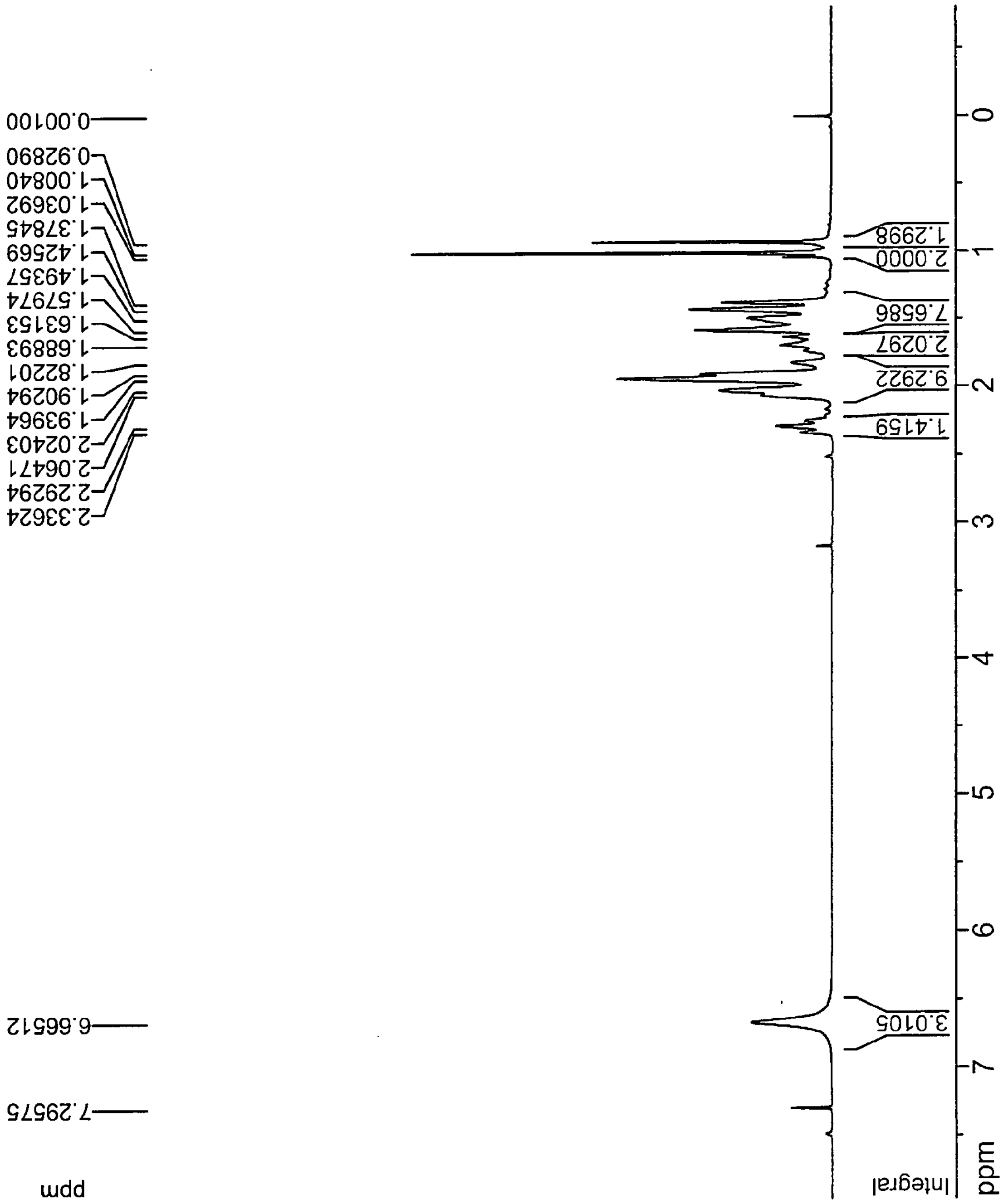
0.00100
0.92890
1.00840
1.03692
1.37845
1.42569
1.49357
1.57974
1.63153
1.68893
1.82201
1.90294
1.93964
2.02403
2.06471
2.29294
2.33624
6.66512
7.29575
ppm
1.2998
2.0000
7.6586
2.0297
9.2922
1.4159
3.0105
Integral
0
1
2
3
4
5
6
7
ppm

FIG. 87

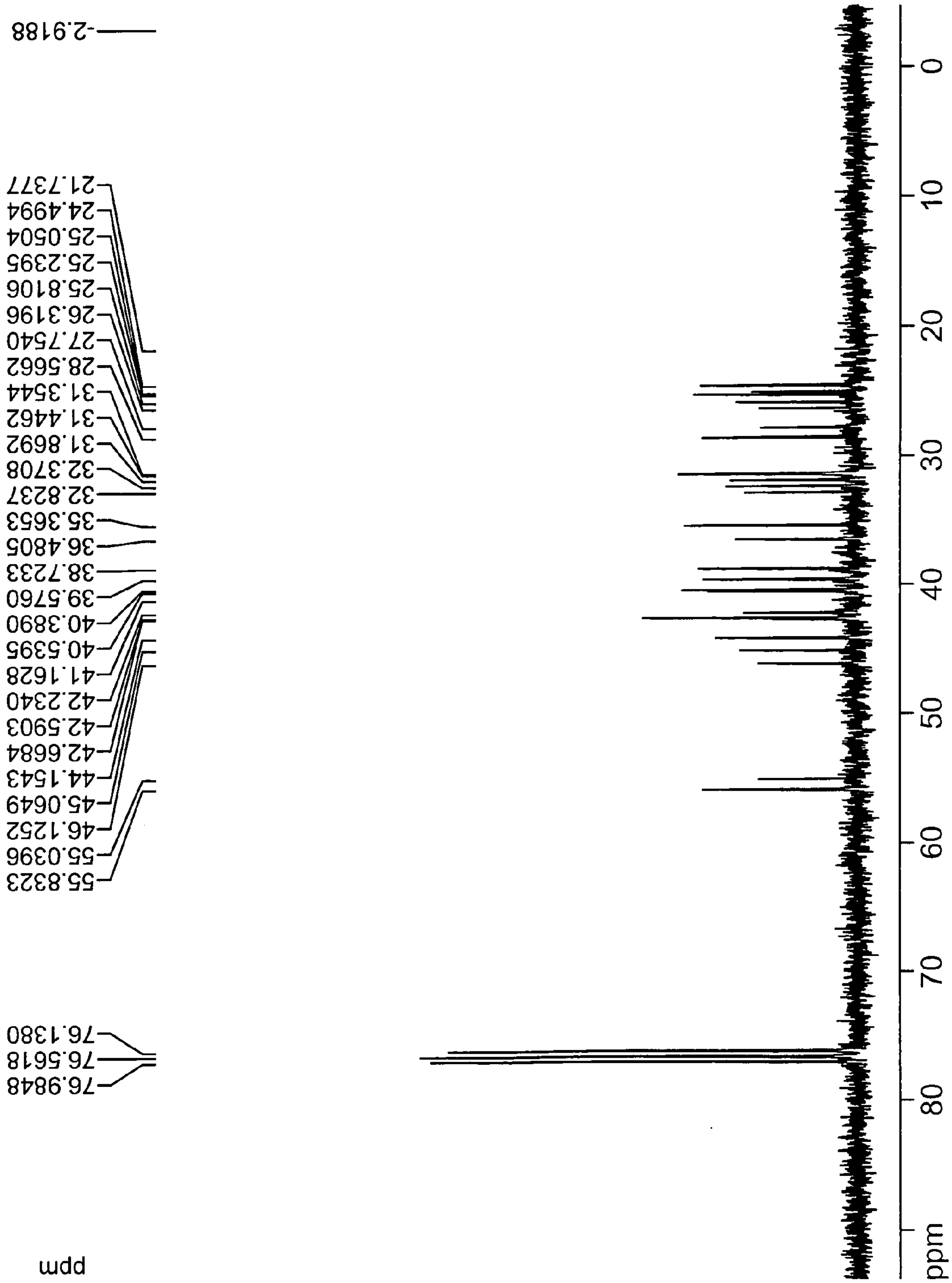
-2.9188
21.7377
24.4994
25.0504
25.2395
25.8106
26.3196
27.7540
28.5662
31.3544
31.4462
31.8692
32.3708
32.8237
35.3653
36.4805
38.7233
39.5760
40.3890
40.5395
41.1628
42.2340
42.5903
42.6684
44.1543
45.0649
46.1252
55.0396
55.8323
76.1380
76.5618
76.9848
ppm
0
10
20
30
40
50
60
70
80
ppm

FIG. 88

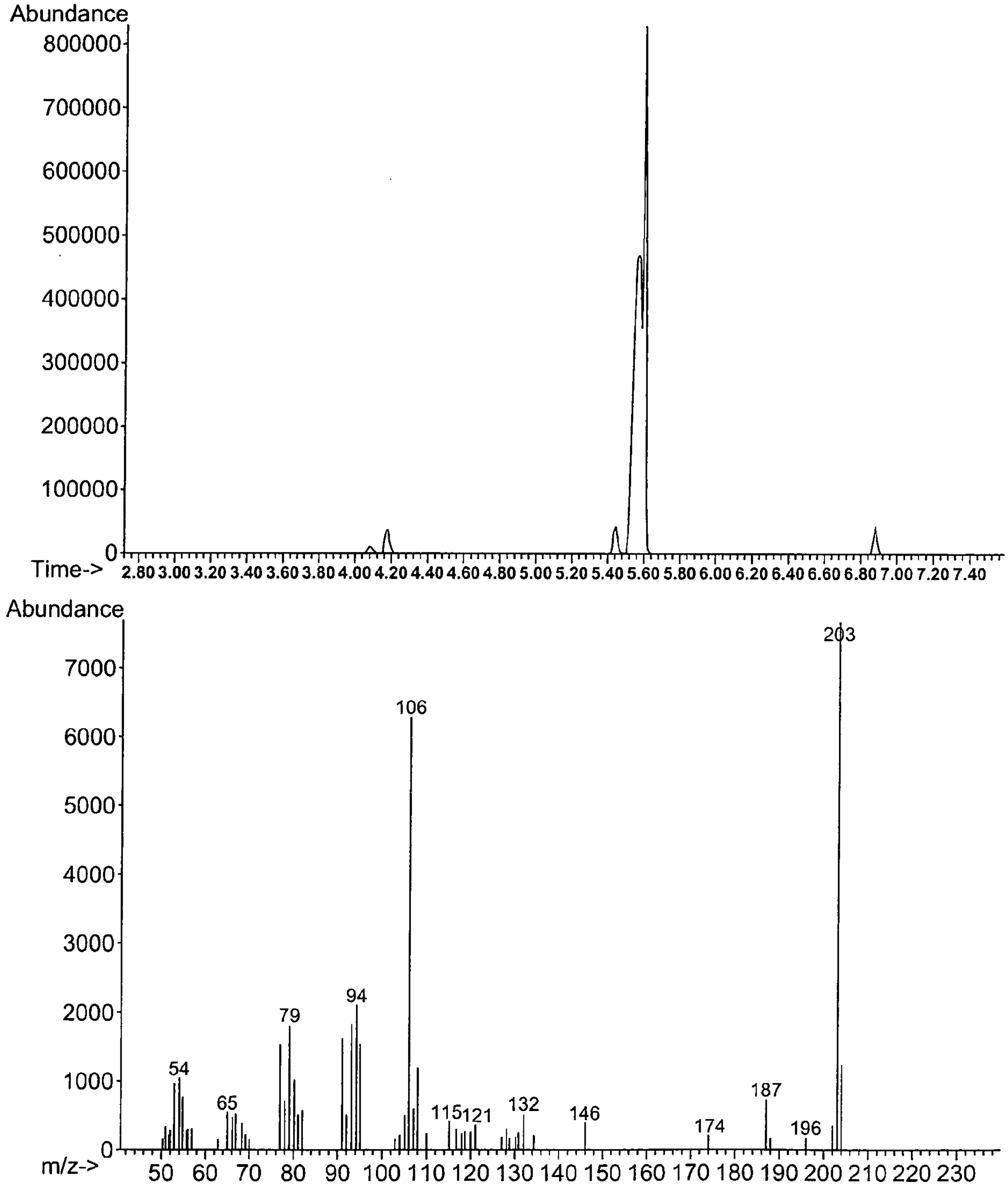

Abundance
800000
700000
600000
500000
400000
300000
200000
100000
0
Time->
2.80 3.00 3.20 3.40 3.60 3.80 4.00 4.20 4.40 4.60 4.80 5.00 5.20 5.40 5.60 5.80 6.00 6.20 6.40 6.60 6.80 7.00 7.20 7.40
Abundance
7000
6000
5000
4000
3000
2000
1000
0
m/z->
50 60 70 80 90 100 110 120 130 140 150 160 170 180 190 200 210 220 230
54
65
79
94
106
115
121
132
146
174
187
196
203


FIG. 89

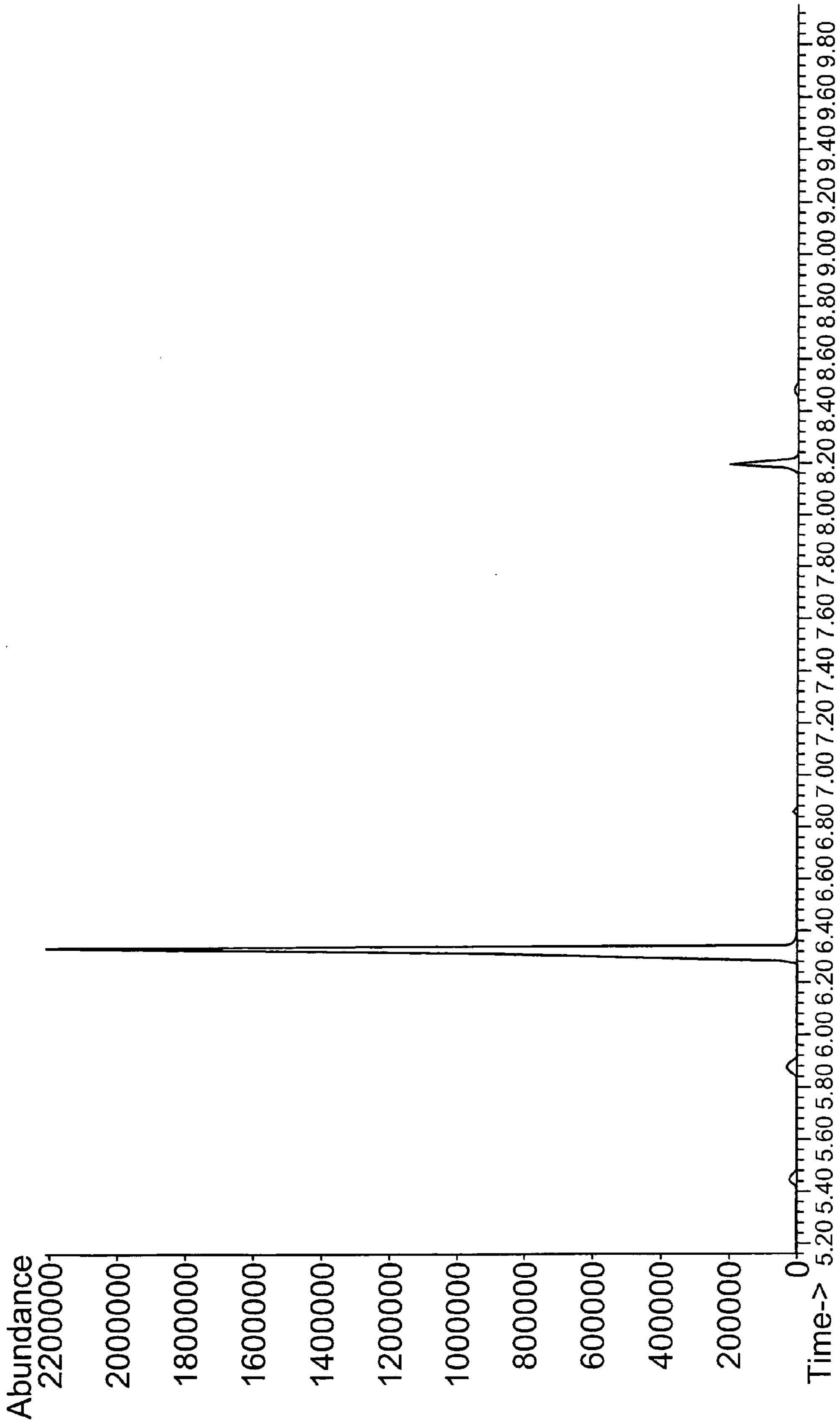
Abundance
2200000
2000000
1800000
1600000
1400000
1200000
1000000
800000
600000
400000
200000
0
Time->
5.20 5.40 5.60 5.80 6.00 6.20 6.40 6.60 6.80 7.00 7.20 7.40 7.60 7.80 8.00 8.20 8.40 8.60 8.80 9.00 9.20 9.40 9.60 9.80

FIG. 90

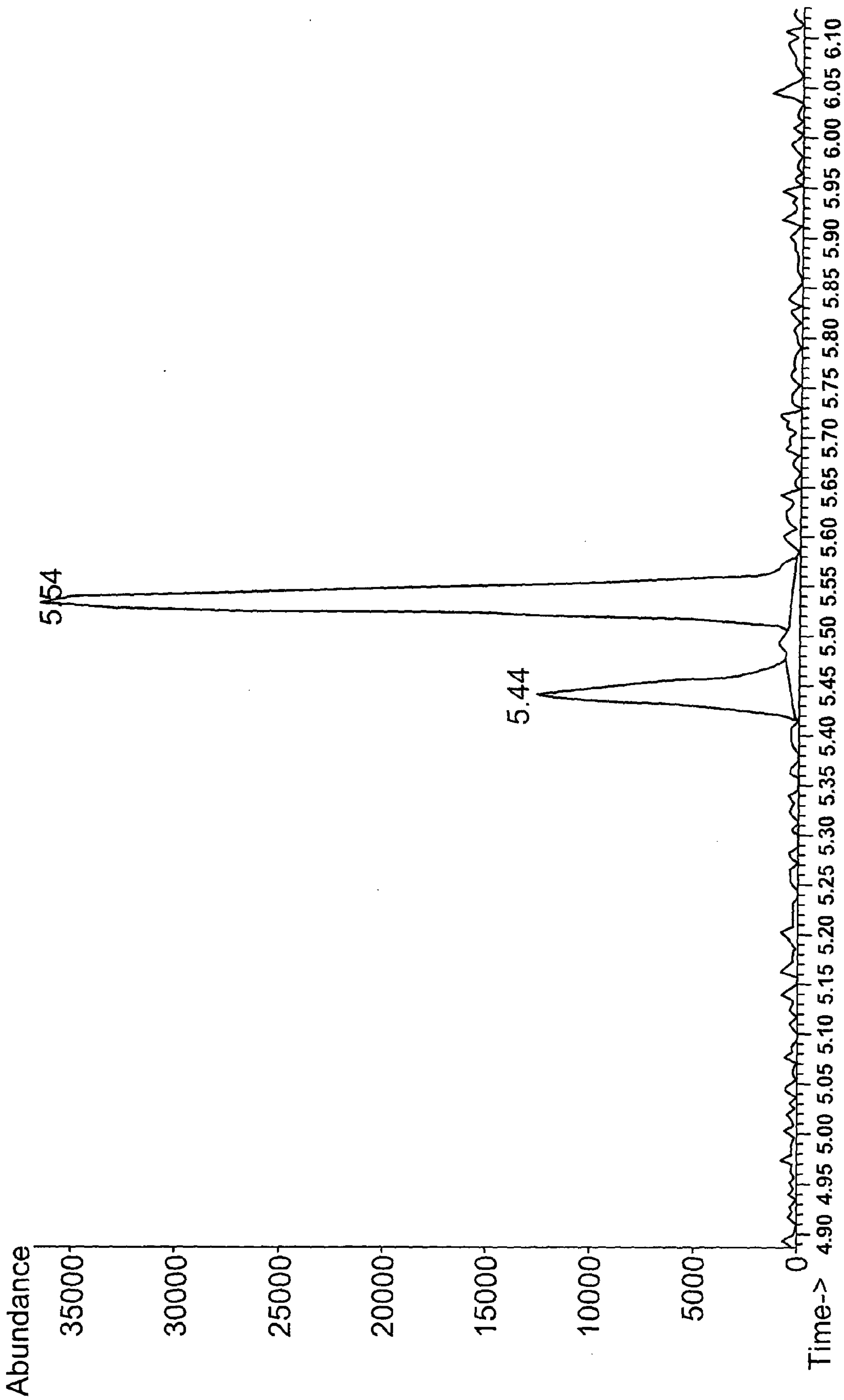
Abundance
35000
30000
25000
20000
15000
10000
5000
0
Time->
4.90 4.95 5.00 5.05 5.10 5.15 5.20 5.25 5.30 5.35 5.40 5.45 5.50 5.55 5.60 5.65 5.70 5.75 5.80 5.85 5.90 5.95 6.00 6.05 6.10
5.44
5.54

FIG. 91

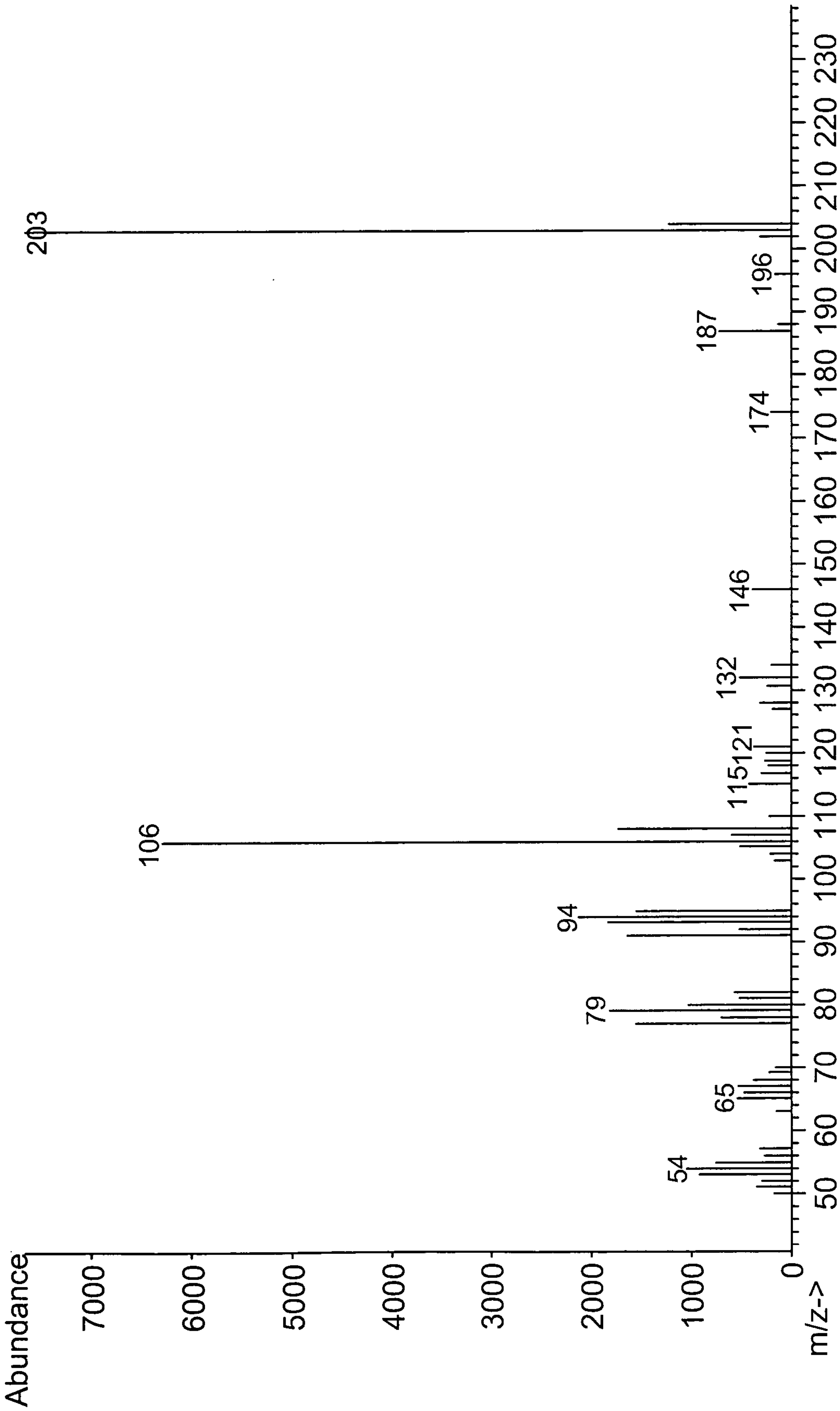
Abundance
7000
6000
5000
4000
3000
2000
1000
0
m/z->
50
60
70
80
90
100
110
120
130
140
150
160
170
180
190
200
210
220
230
54
65
79
94
106
115
121
132
146
174
187
196
203

FIG. 92

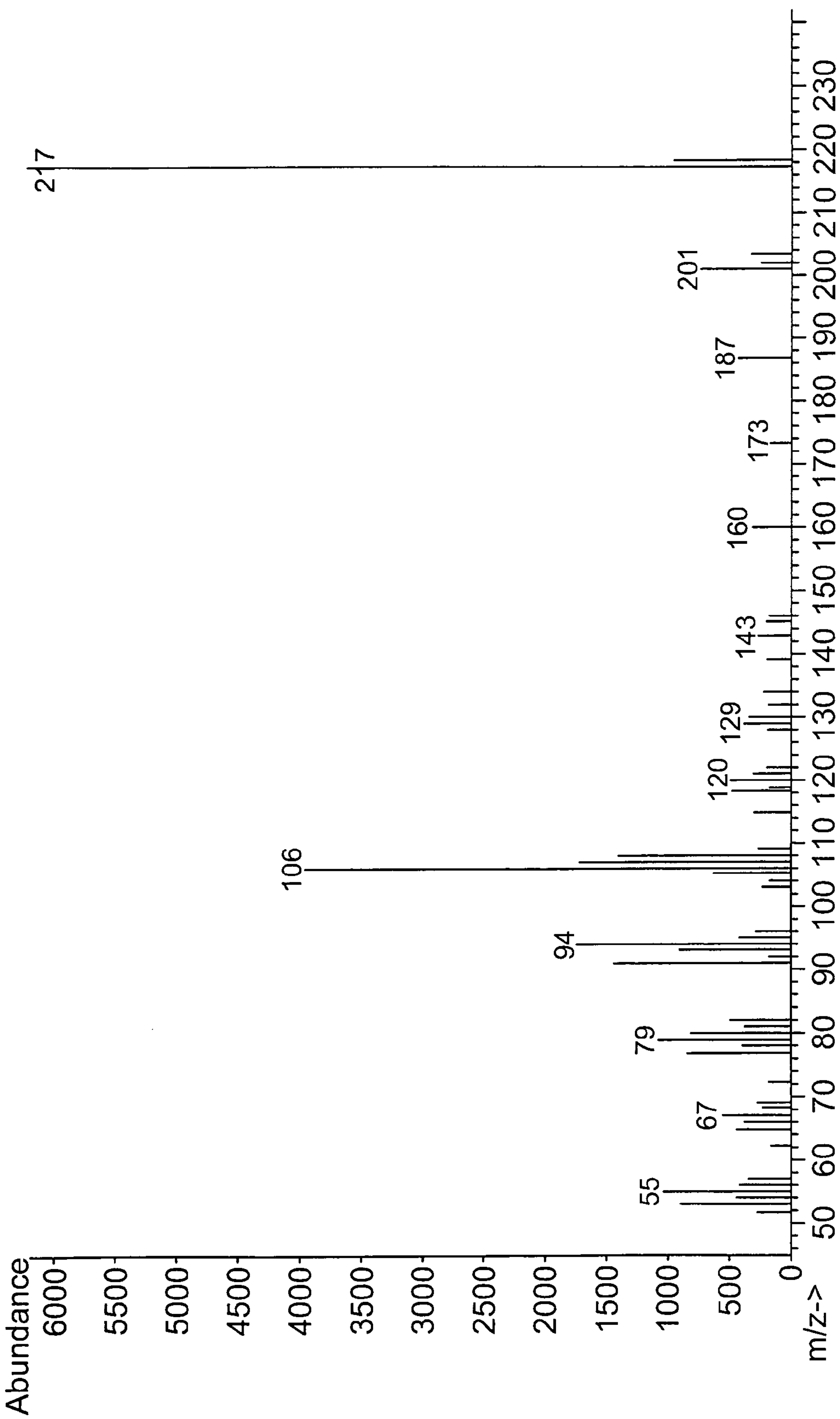
Abundance
6000
5500
5000
4500
4000
3500
3000
2500
2000
1500
1000
500
0
m/z->
50
60
70
80
90
100
110
120
130
140
150
160
170
180
190
200
210
220
230
55
67
79
94
106
120
129
143
160
173
187
201
217

FIG. 93

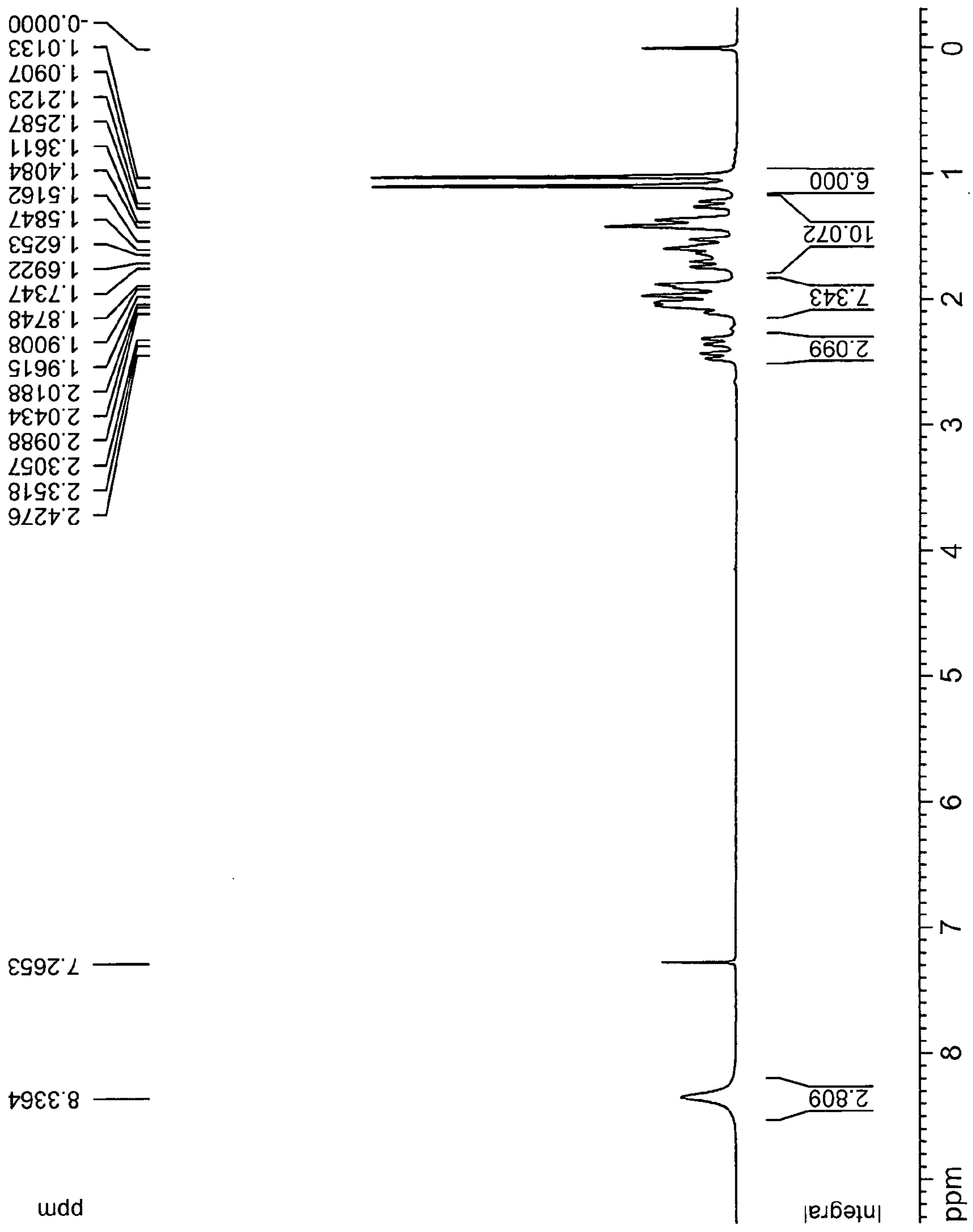
-0.0000
1.0133
1.0907
1.2123
1.2587
1.3611
1.4084
1.5162
1.5847
1.6253
1.6922
1.7347
1.8748
1.9008
1.9615
2.0188
2.0434
2.0988
2.3057
2.3518
2.4276
7.2653
8.3364
ppm
6.000
10.072
7.343
2.099
2.809
Integral
0
1
2
3
4
5
6
7
8
ppm

FIG. 94

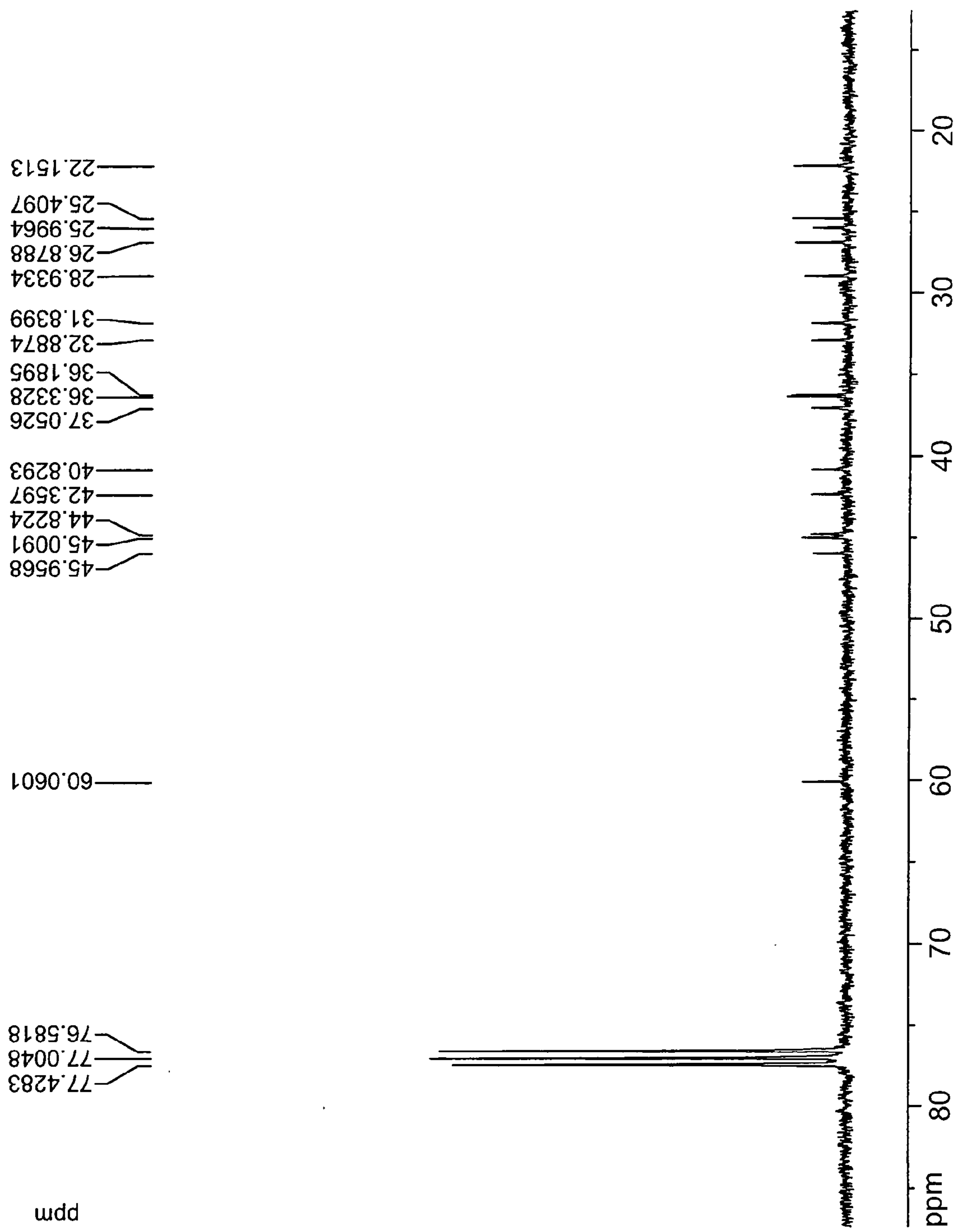
22.1513
25.4097
25.9964
26.8788
28.9334
31.8399
32.8874
36.1895
36.3328
37.0526
40.8293
42.3597
44.8224
45.0091
45.9568
60.0601
76.5818
77.0048
77.4283
ppm
20
30
40
50
60
70
80
ppm

FIG. 95

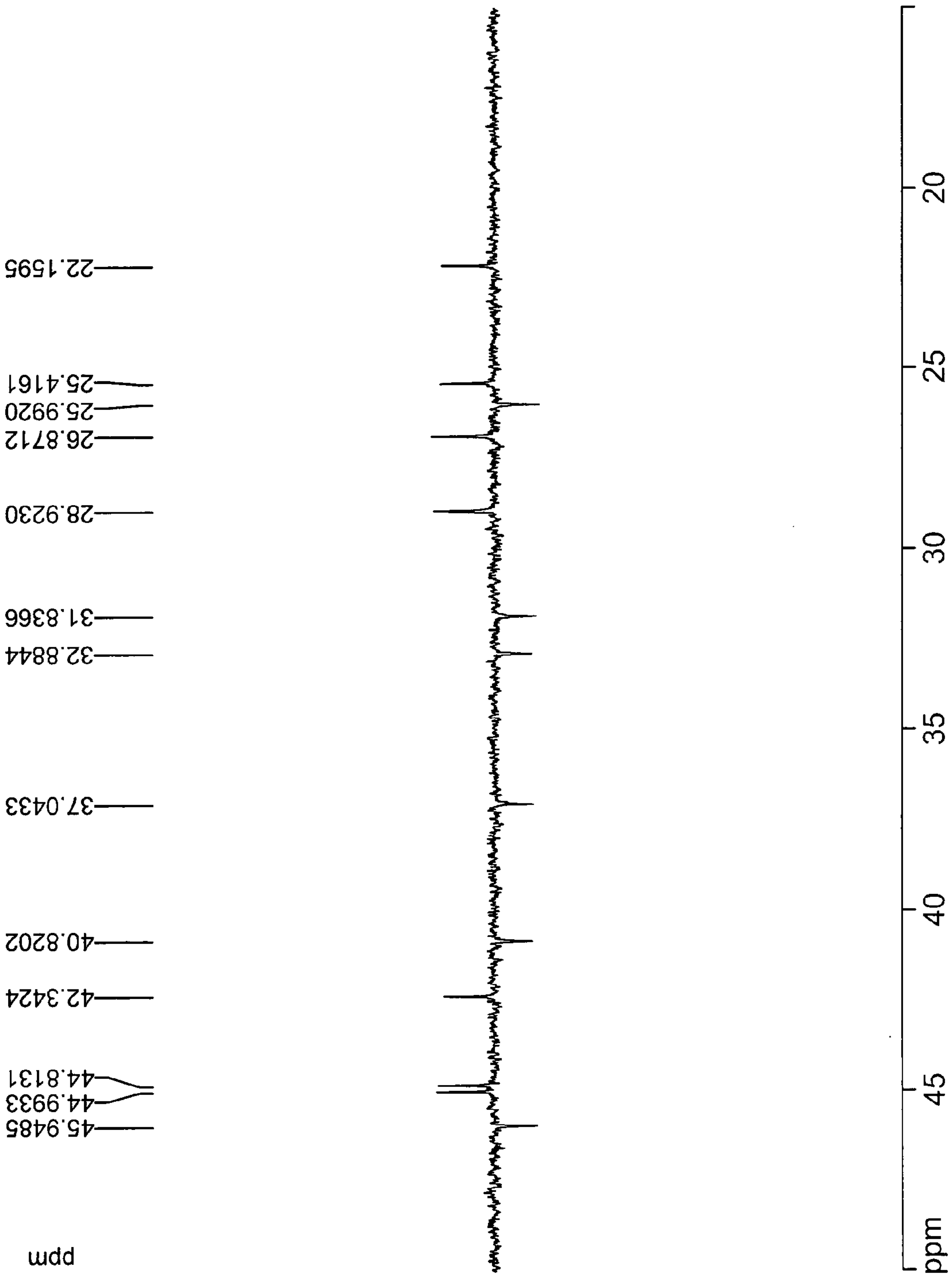

22.1595
25.4161
25.9920
26.8712
28.9230
31.8366
32.8844
37.0433
40.8202
42.3424
44.8131
44.9933
45.9485
ppm
20
25
30
35
40
45
ppm


FIG. 96

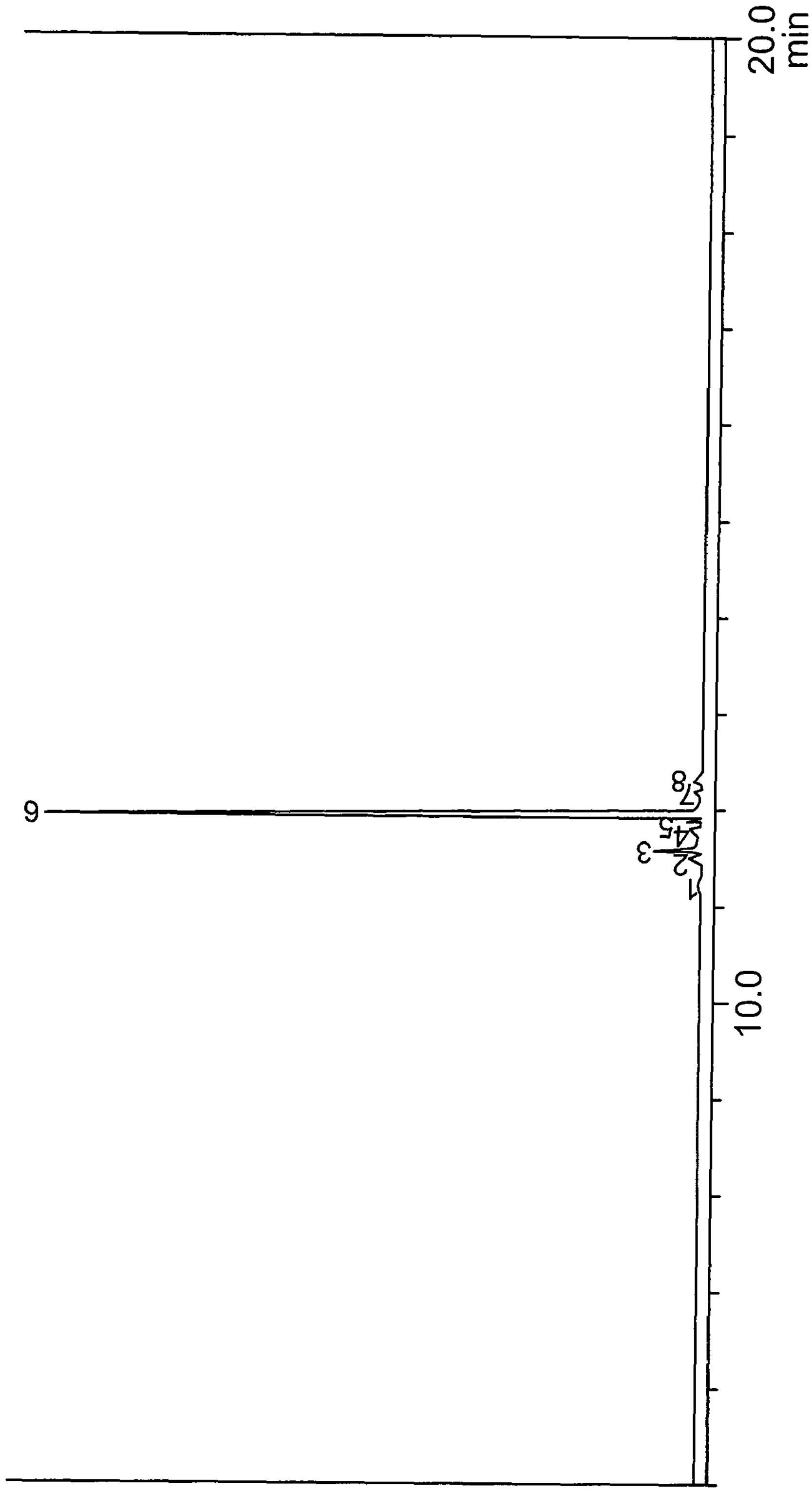


FIG. 97

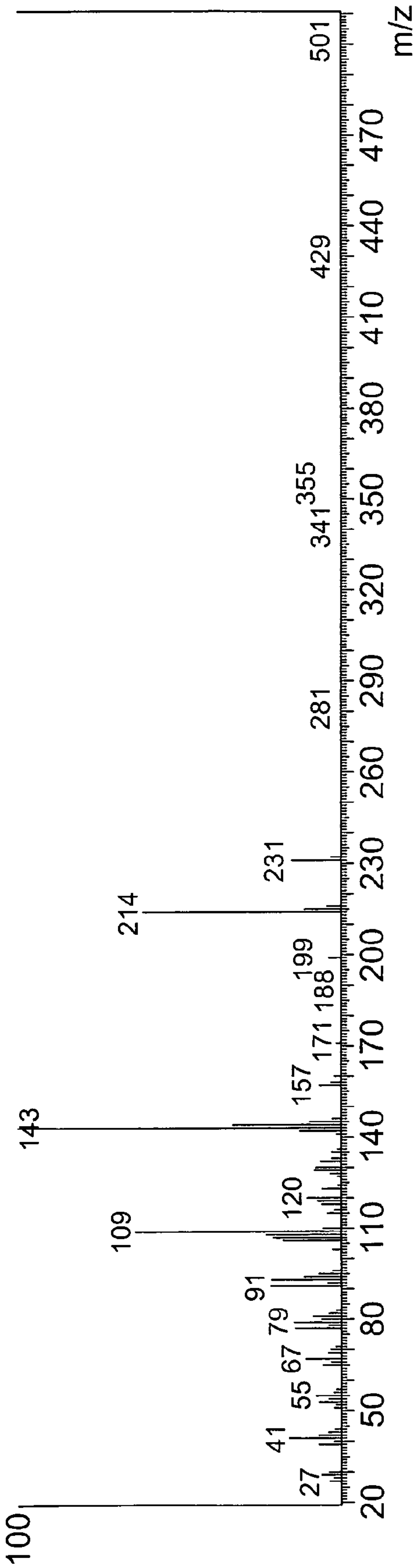
100
m/z
20
50
80
110
140
170
200
230
260
290
320
350
380
410
440
470
27
41
55
67
79
91
109
120
143
157
171
188
199
214
231
281
341
355
429
501

FIG. 98

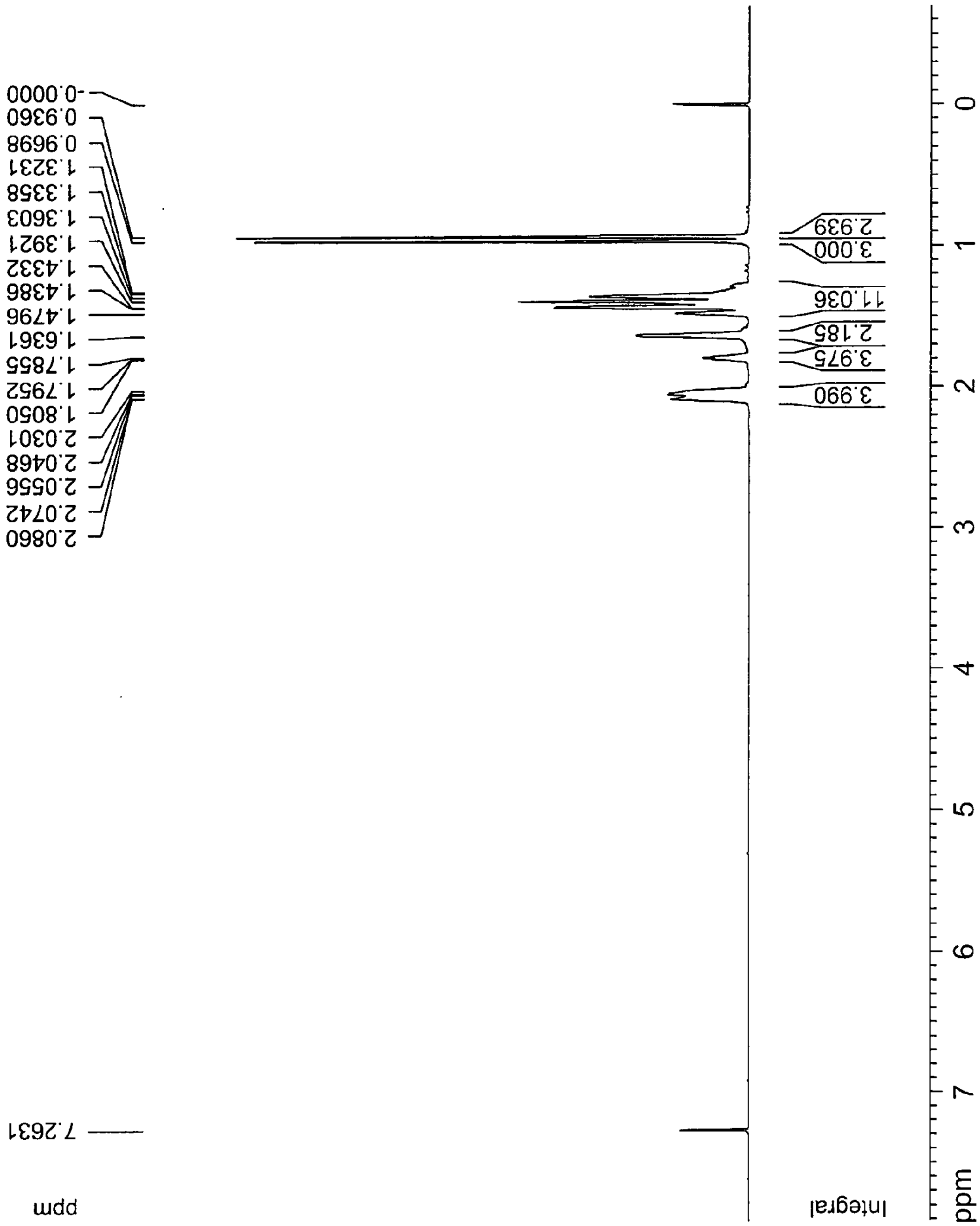
-0.0000
0.9360
0.9698
1.3231
1.3358
1.3603
1.3921
1.4332
1.4386
1.4796
1.6361
1.7855
1.7952
1.8050
2.0301
2.0468
2.0556
2.0742
2.0860
7.2631
ppm
2.939
3.000
11.036
2.185
3.975
3.990
Integral
0
1
2
3
4
5
6
7
ppm

FIG. 99

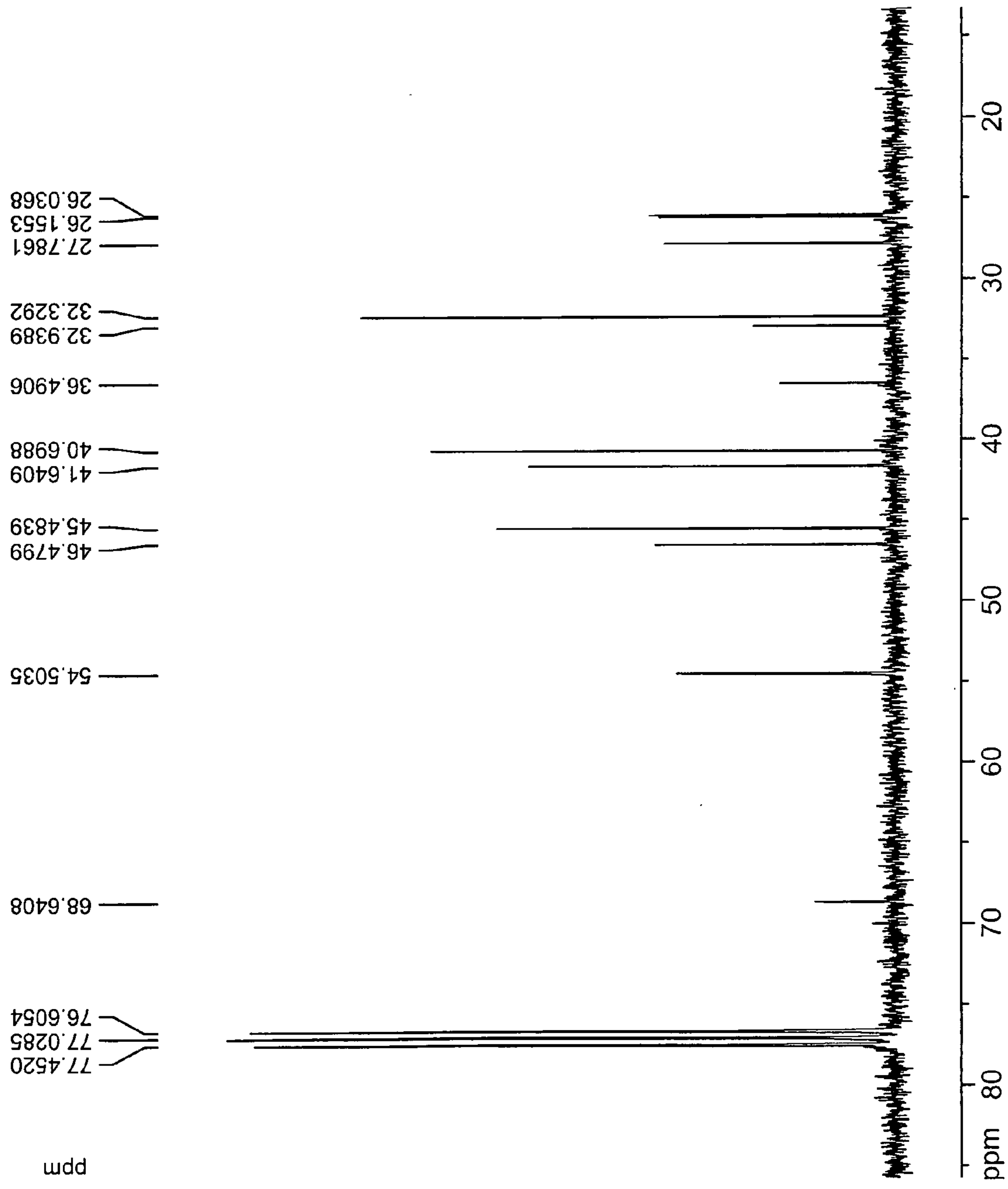

26.0368
26.1553
27.7861
32.3292
32.9389
36.4906
40.6988
41.6409
45.4839
46.4799
54.5035
68.6408
76.6054
77.0285
77.4520
ppm
20
30
40
50
60
70
80
ppm


FIG. 100

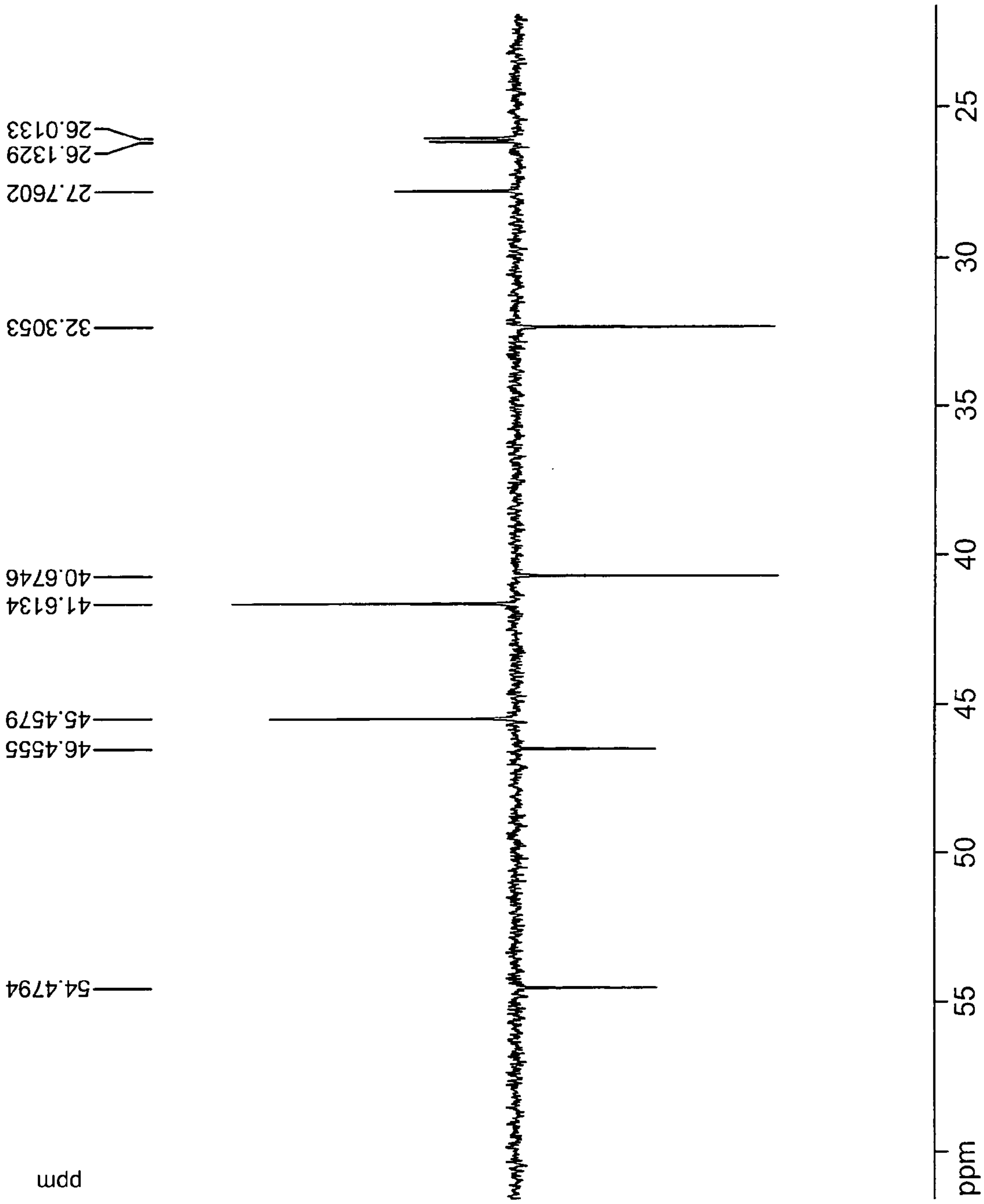
26.0133
26.1329
27.7602
32.3053
40.6746
41.6134
45.4579
46.4555
54.4794
ppm
25
30
35
40
45
50
55
ppm

FIG. 101

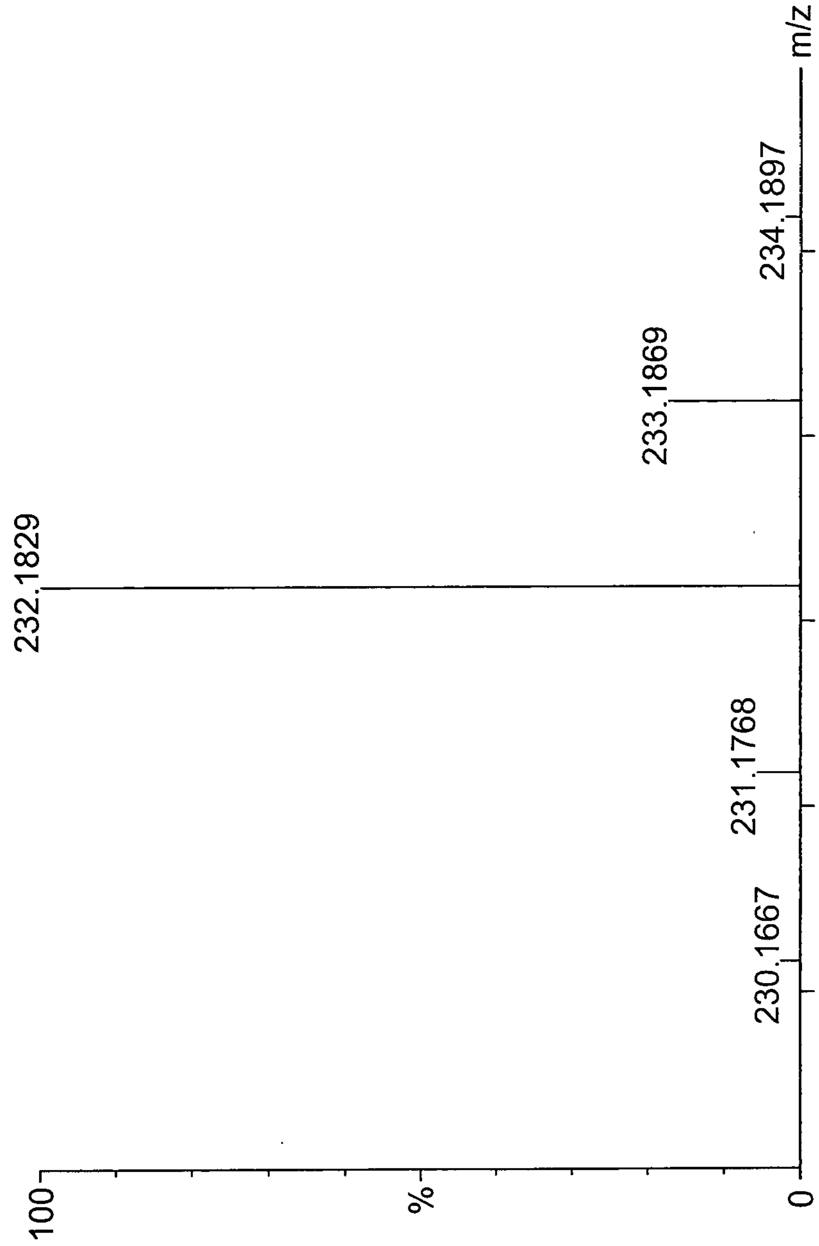

100
%
0
230.1667
231.1768
232.1829
233.1869
234.1897
230
231
232
233
234
m/z


FIG. 102

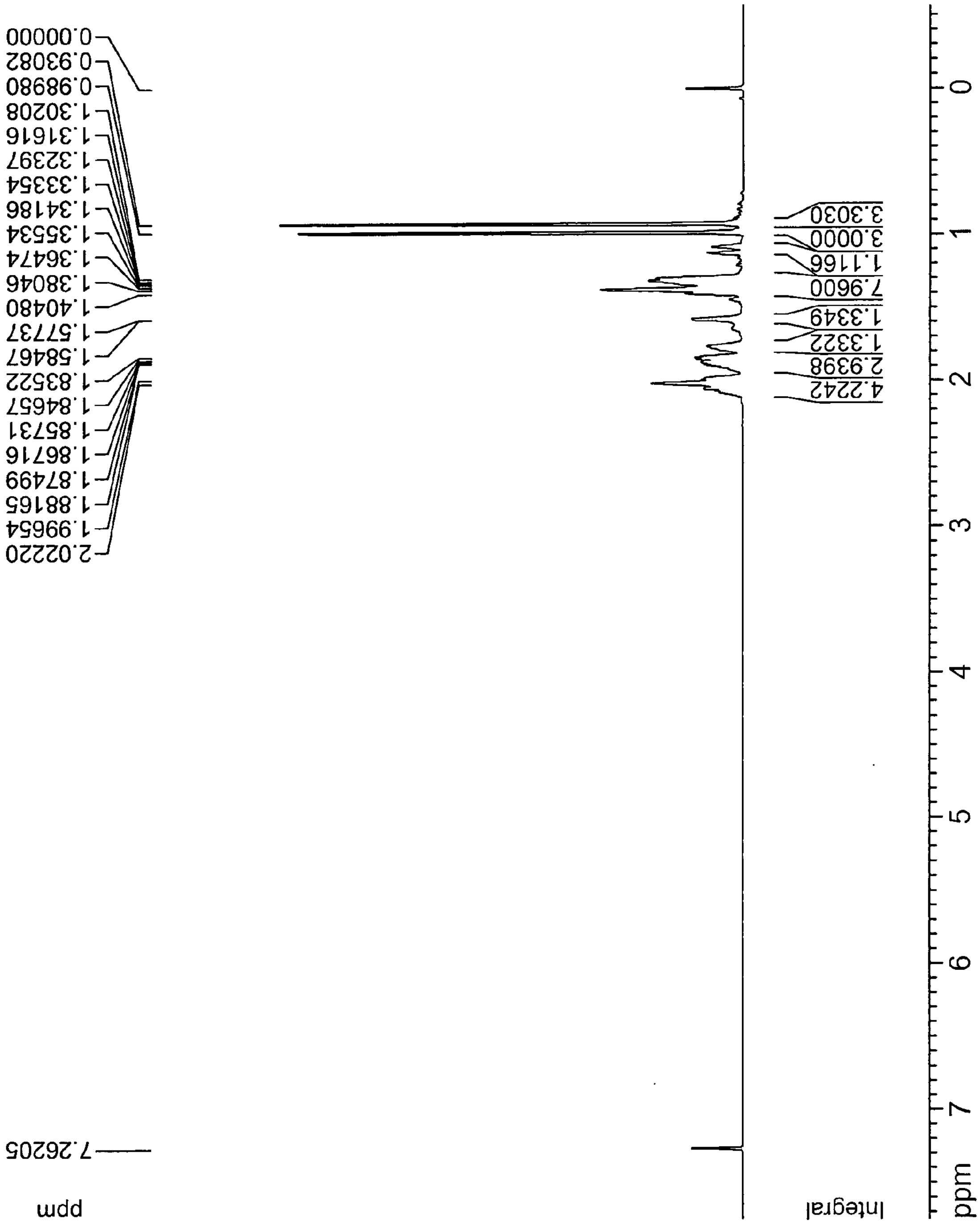
ppm
0.00000
0.93082
0.98980
1.30208
1.31616
1.32397
1.33354
1.34186
1.35534
1.36474
1.38046
1.40480
1.57737
1.58467
1.83522
1.84657
1.85731
1.86716
1.87499
1.88165
1.99654
2.02220
7.26205
Integral
3.3030
3.0000
1.1166
7.9600
1.3349
1.3322
2.9398
4.2242
ppm
0
1
2
3
4
5
6
7

FIG. 103

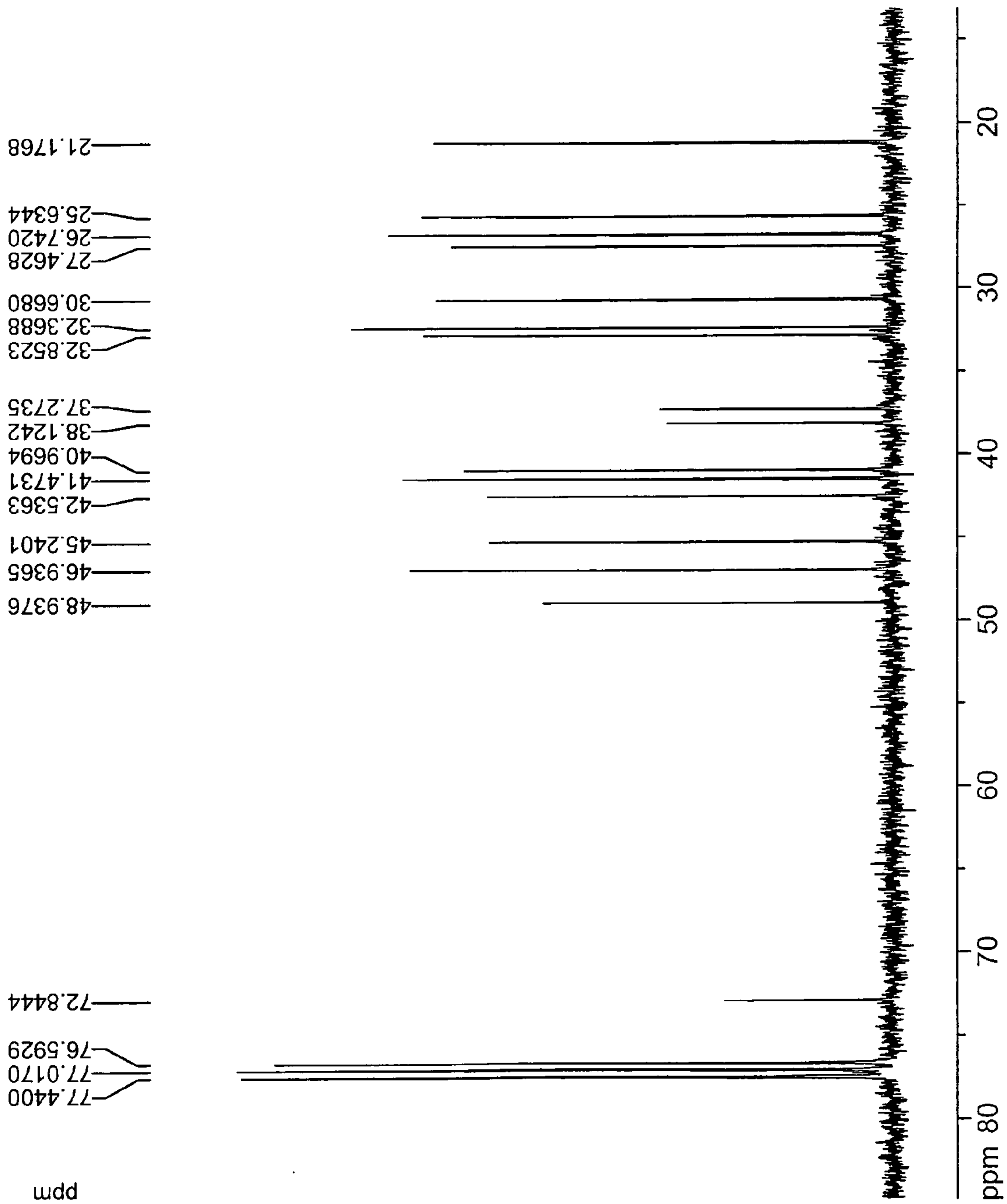
21.1768
25.6344
26.7420
27.4628
30.6680
32.3688
32.8523
37.2735
38.1242
40.9694
41.4731
42.5363
45.2401
46.9365
48.9376
72.8444
76.5929
77.0170
77.4400
ppm
20
30
40
50
60
70
ppm 80

FIG. 104

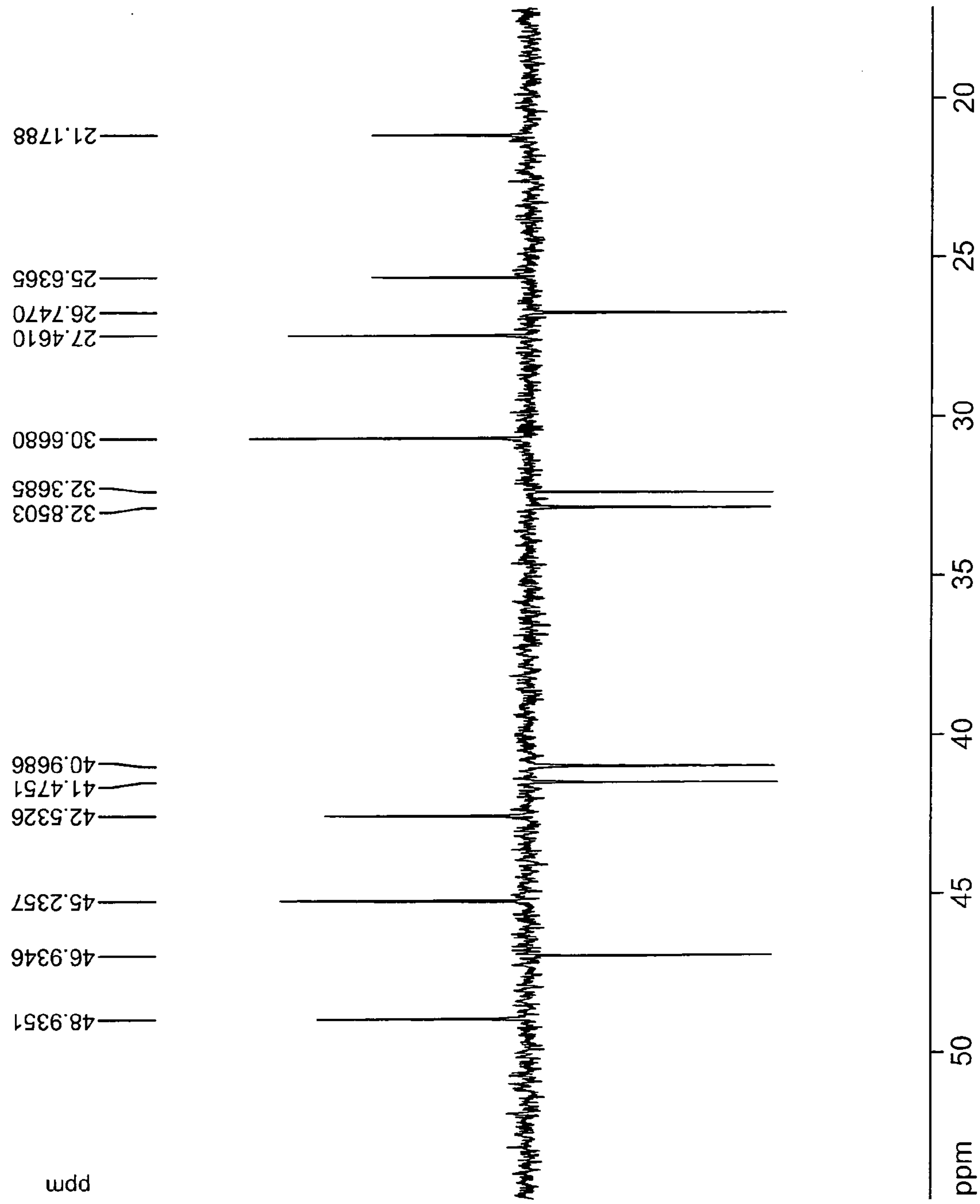
21.1788
25.6365
26.7470
27.4610
30.6680
32.3685
32.8503
40.9686
41.4751
42.5326
45.2357
46.9346
48.9351
ppm
20
25
30
35
40
45
50
ppm

FIG. 105

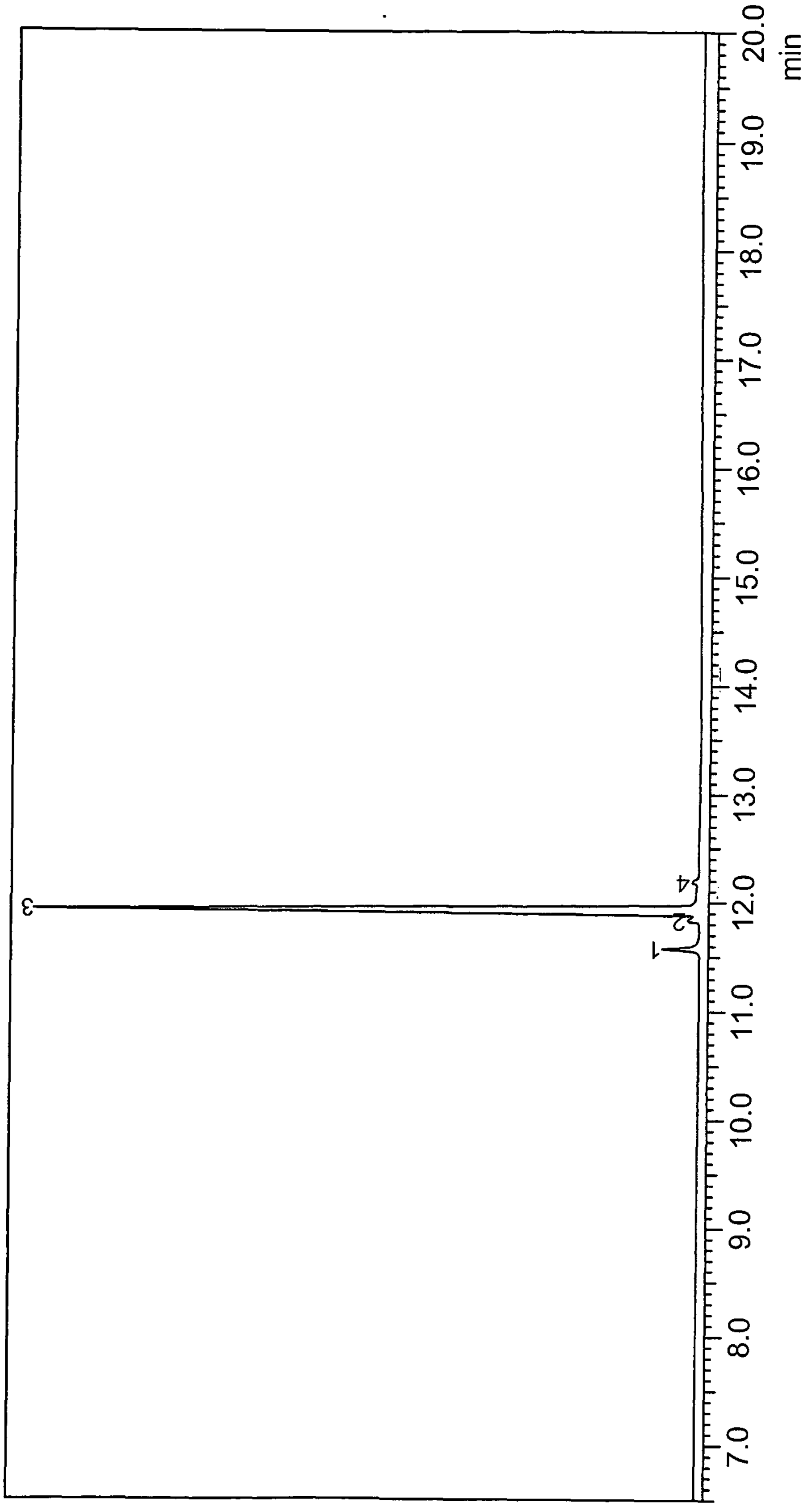


FIG. 106

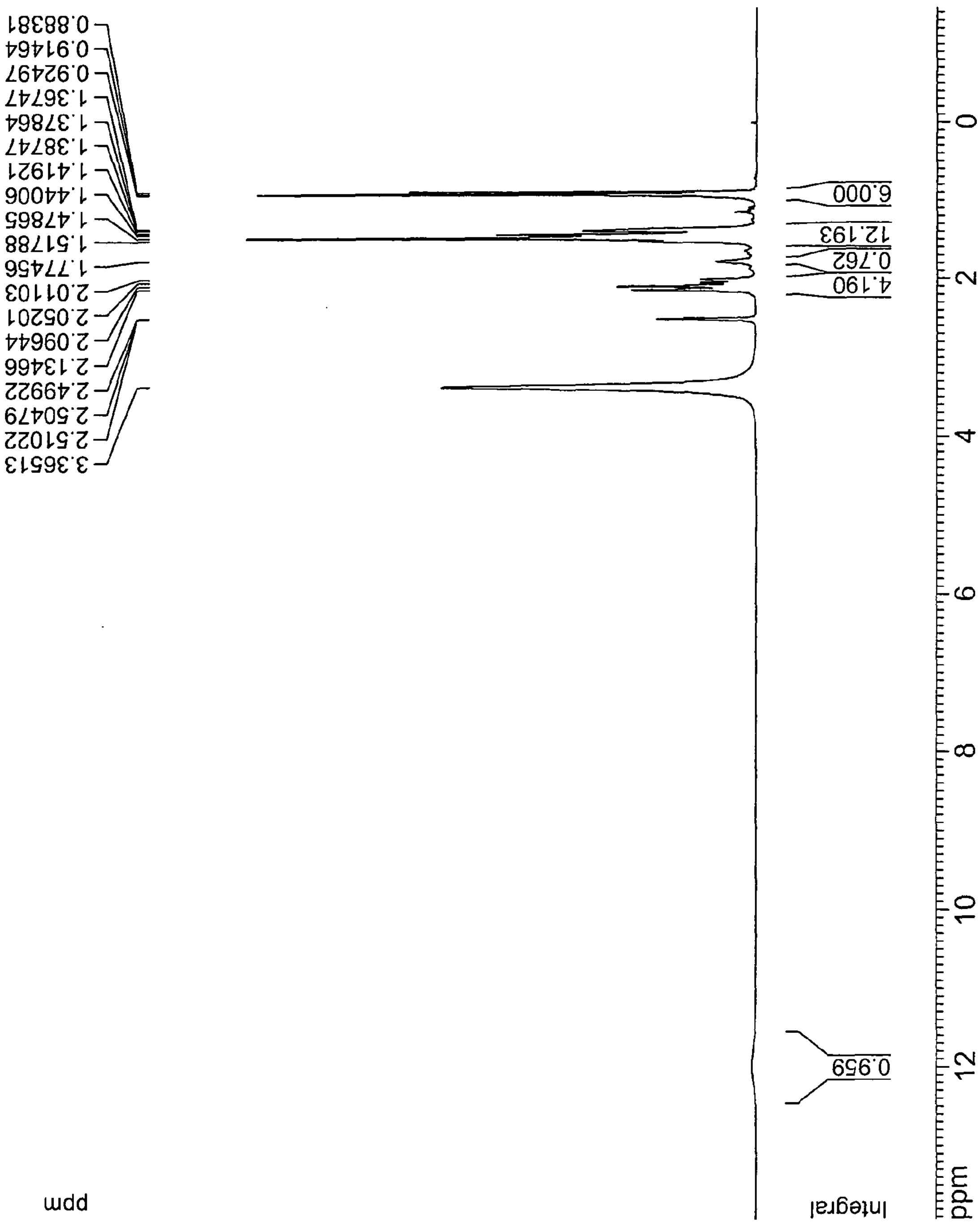
0.88381
0.91464
0.92497
1.36747
1.37864
1.38747
1.41921
1.44006
1.47865
1.51788
1.77456
2.01103
2.05201
2.09644
2.13466
2.49922
2.50479
2.51022
3.36513
ppm
6.000
12.193
0.762
4.190
0.959
Integral
0
2
4
6
8
10
12
ppm

FIG. 107

FIG. 108

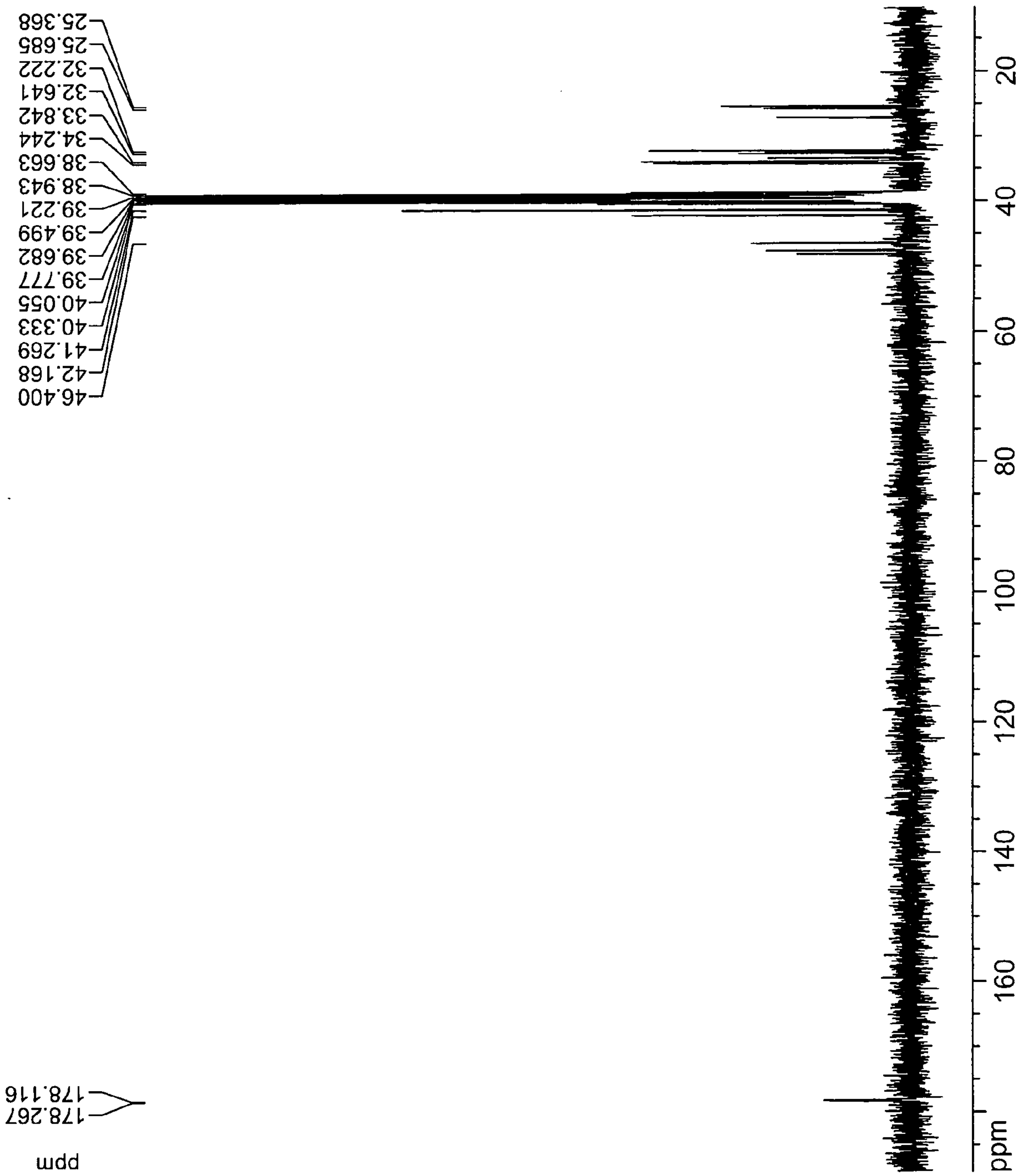
25.368
25.685
32.222
32.641
33.842
34.244
38.663
38.943
39.221
39.499
39.682
39.777
40.055
40.333
41.269
42.168
46.400
178.116
178.267
ppm
20
40
60
80
100
120
140
160
ppm

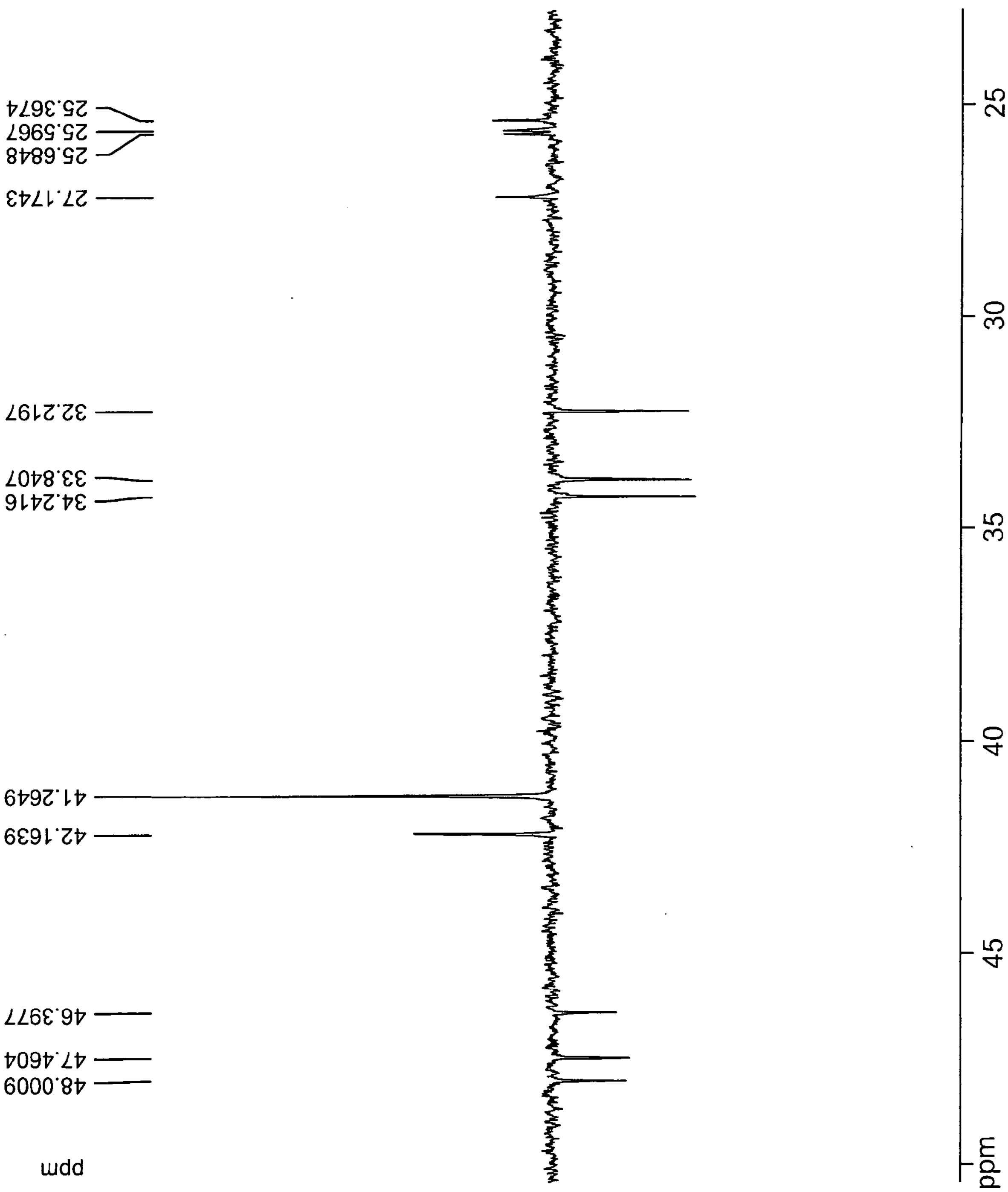
25.3674
25.5967
25.6848
27.1743
32.2197
33.8407
34.2416
41.2649
42.1639
46.3977
47.4604
48.0009
ppm
25
30
35
40
45
ppm

FIG. 109

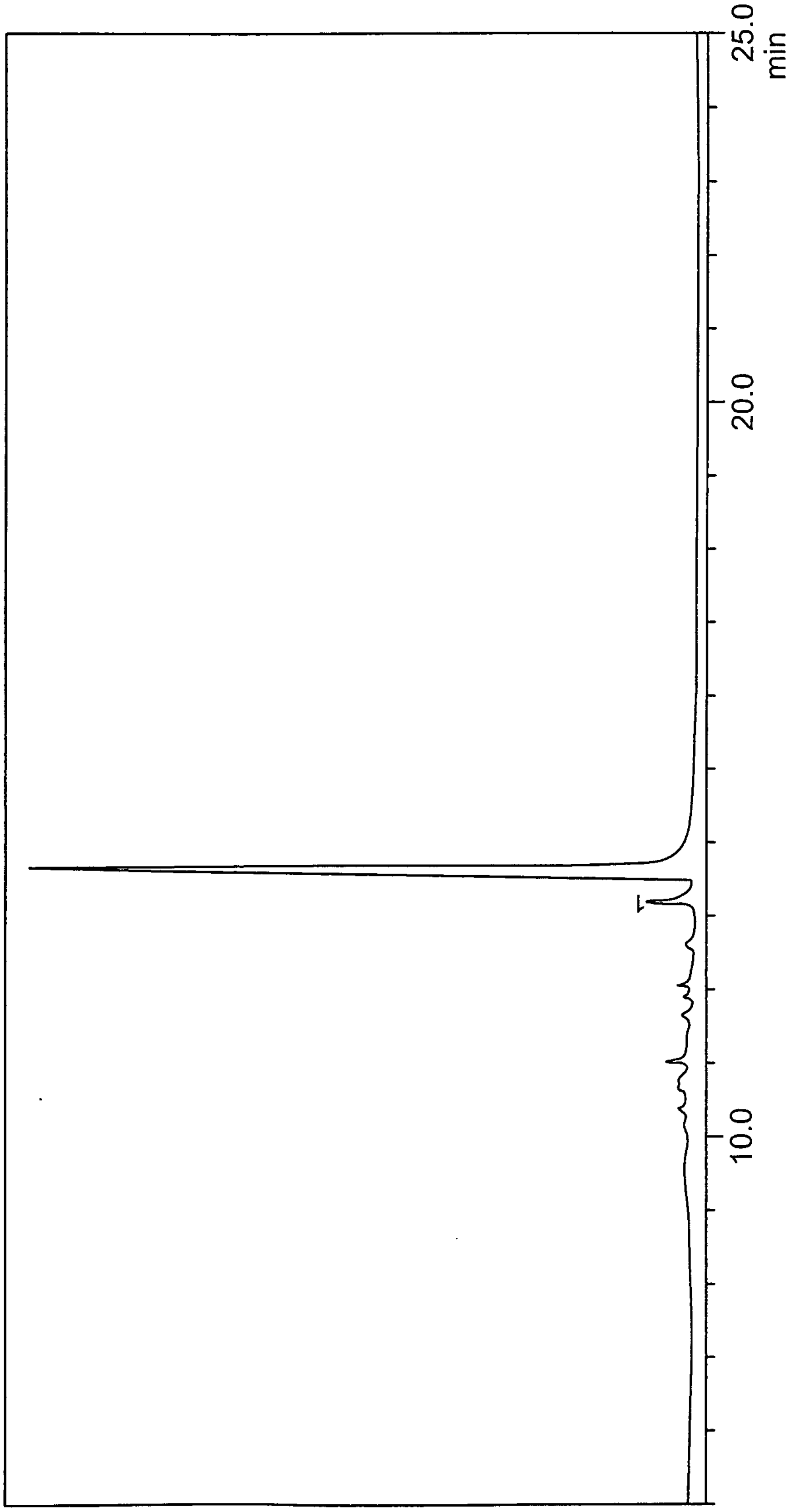


FIG. 110

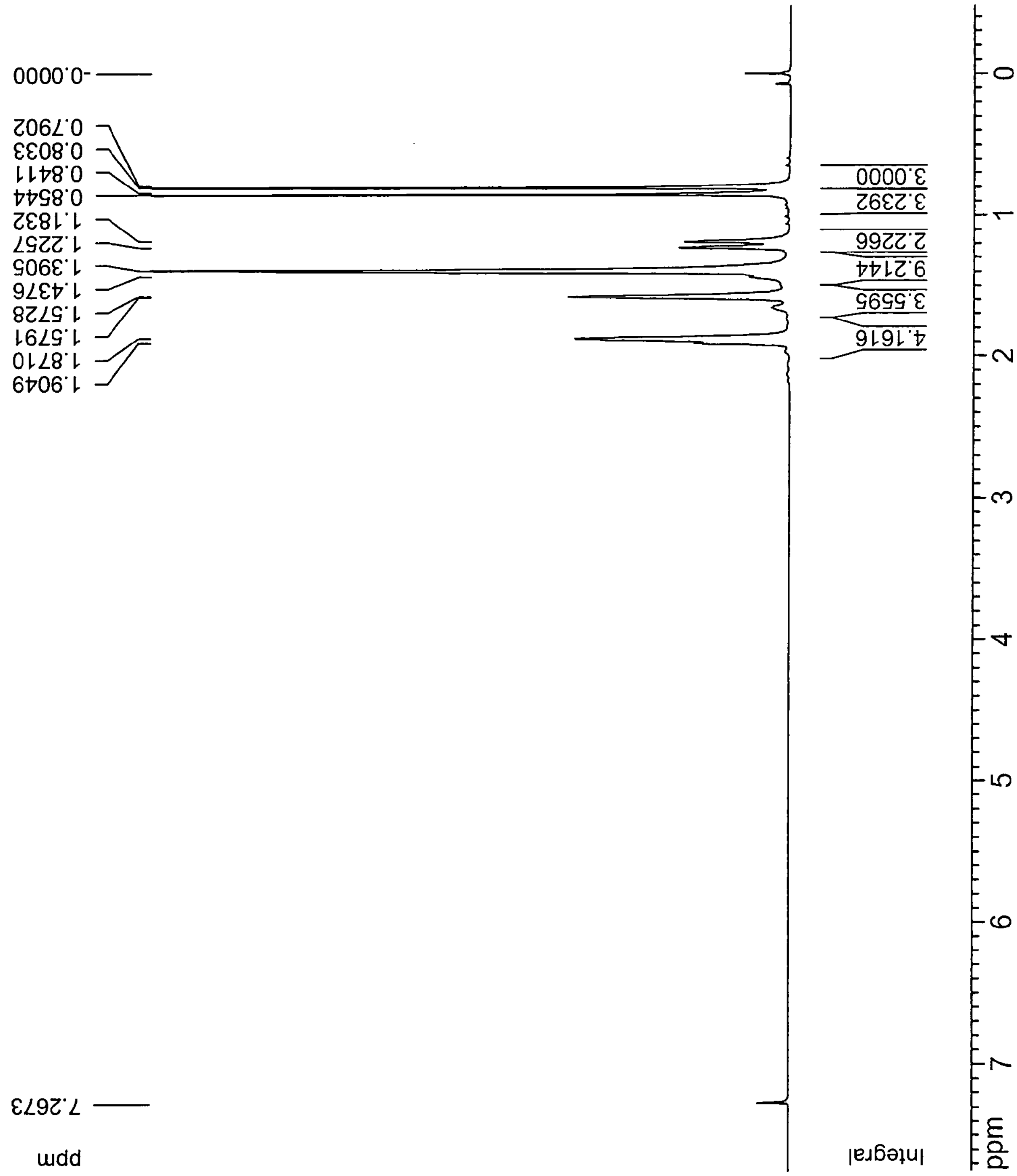
-0.0000
0.7902
0.8033
0.8411
0.8544
1.1832
1.2257
1.3905
1.4376
1.5728
1.5791
1.8710
1.9049
7.2673
ppm
3.0000
3.2392
2.2266
9.2144
3.5595
4.1616
Integral
0
1
2
3
4
5
6
7
ppm

FIG. 111

FIG. 112

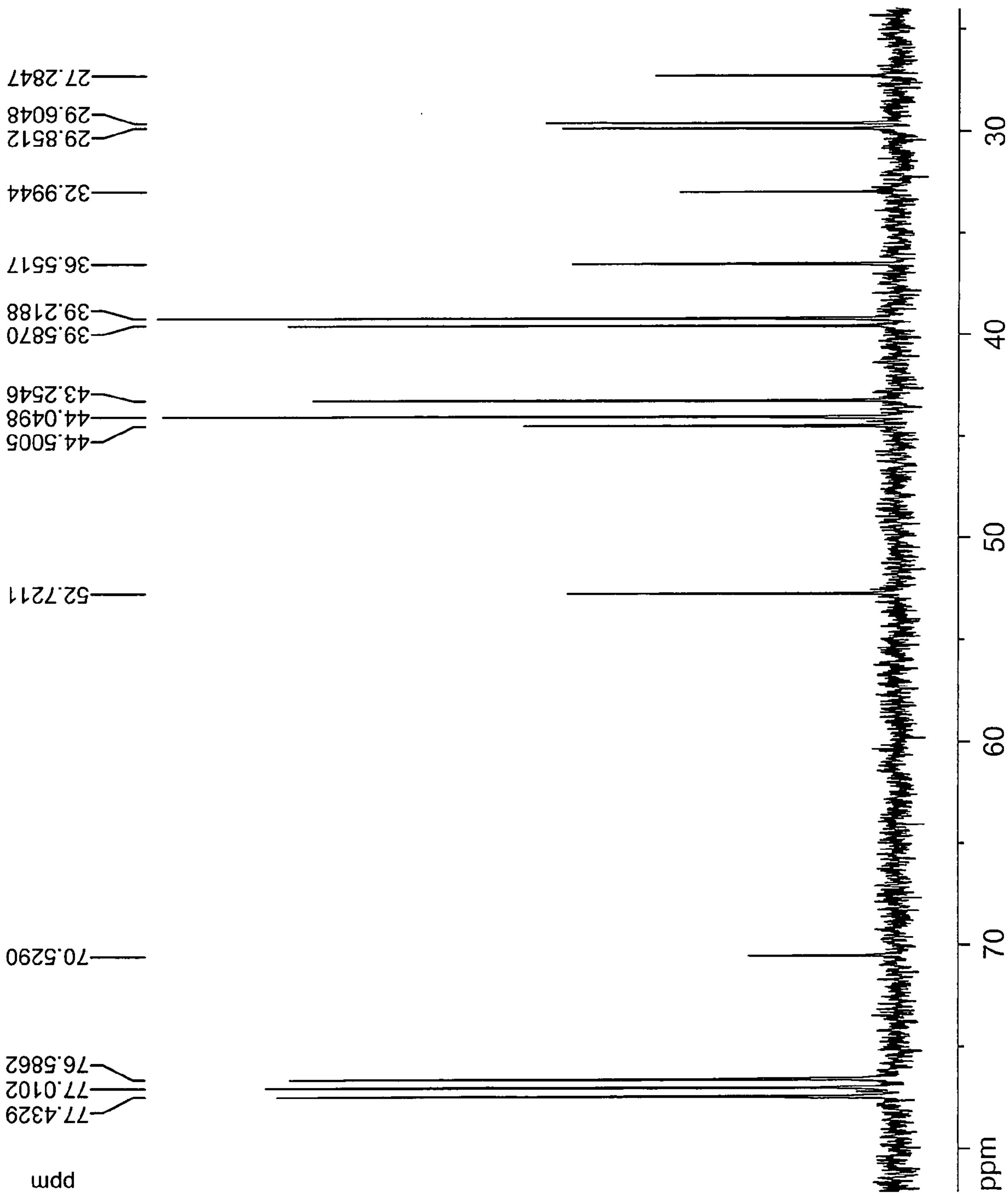
27.2847
29.6048
29.8512
32.9944
36.5517
39.2188
39.5870
43.2546
44.0498
44.5005
52.7211
70.5290
76.5862
77.0102
77.4329
ppm
30
40
50
60
70
ppm

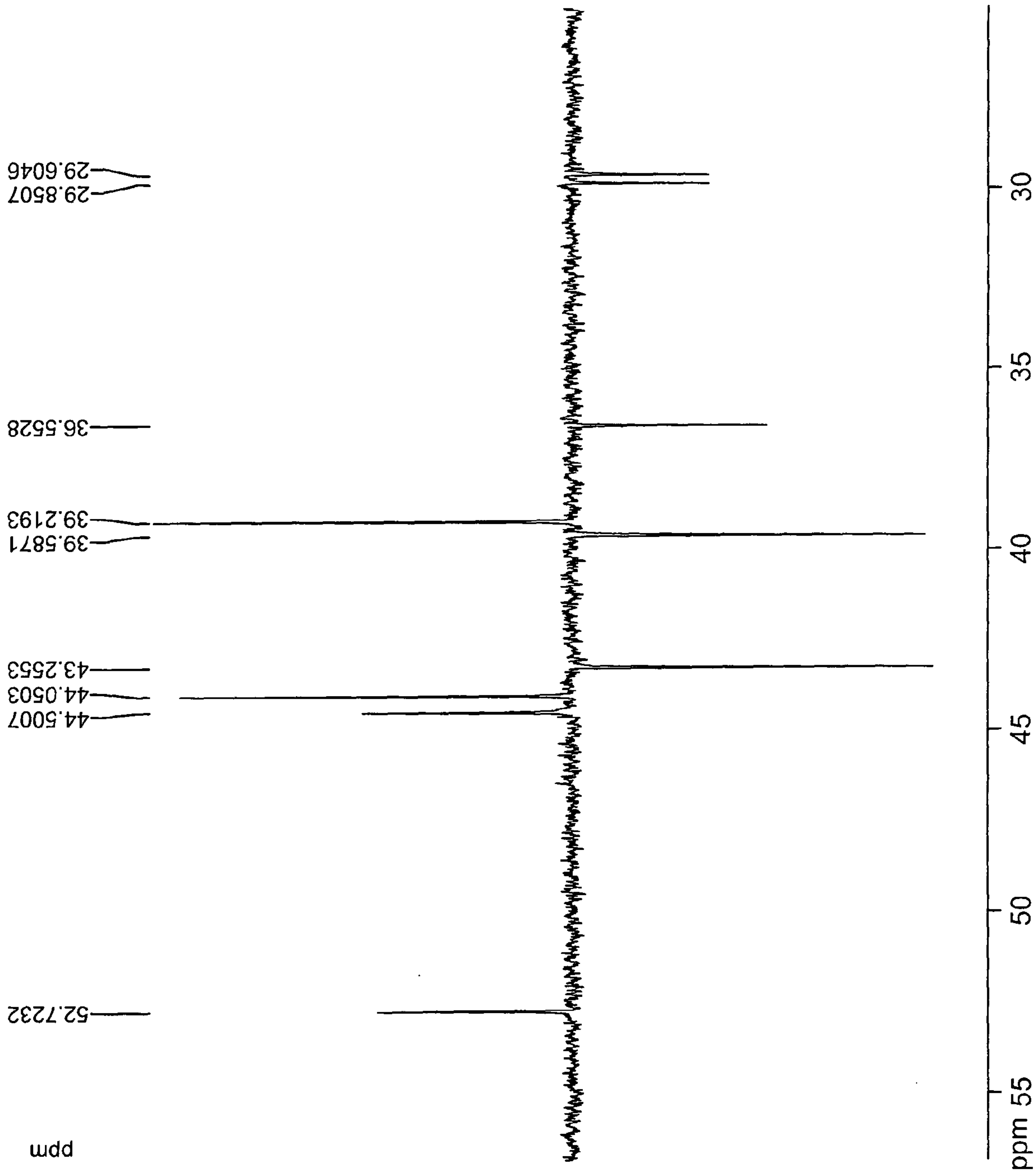
29.6046
29.8507
36.5528
39.2193
39.5871
43.2553
44.0503
44.5007
52.7232
ppm
30
35
40
45
50
55
ppm

FIG. 113

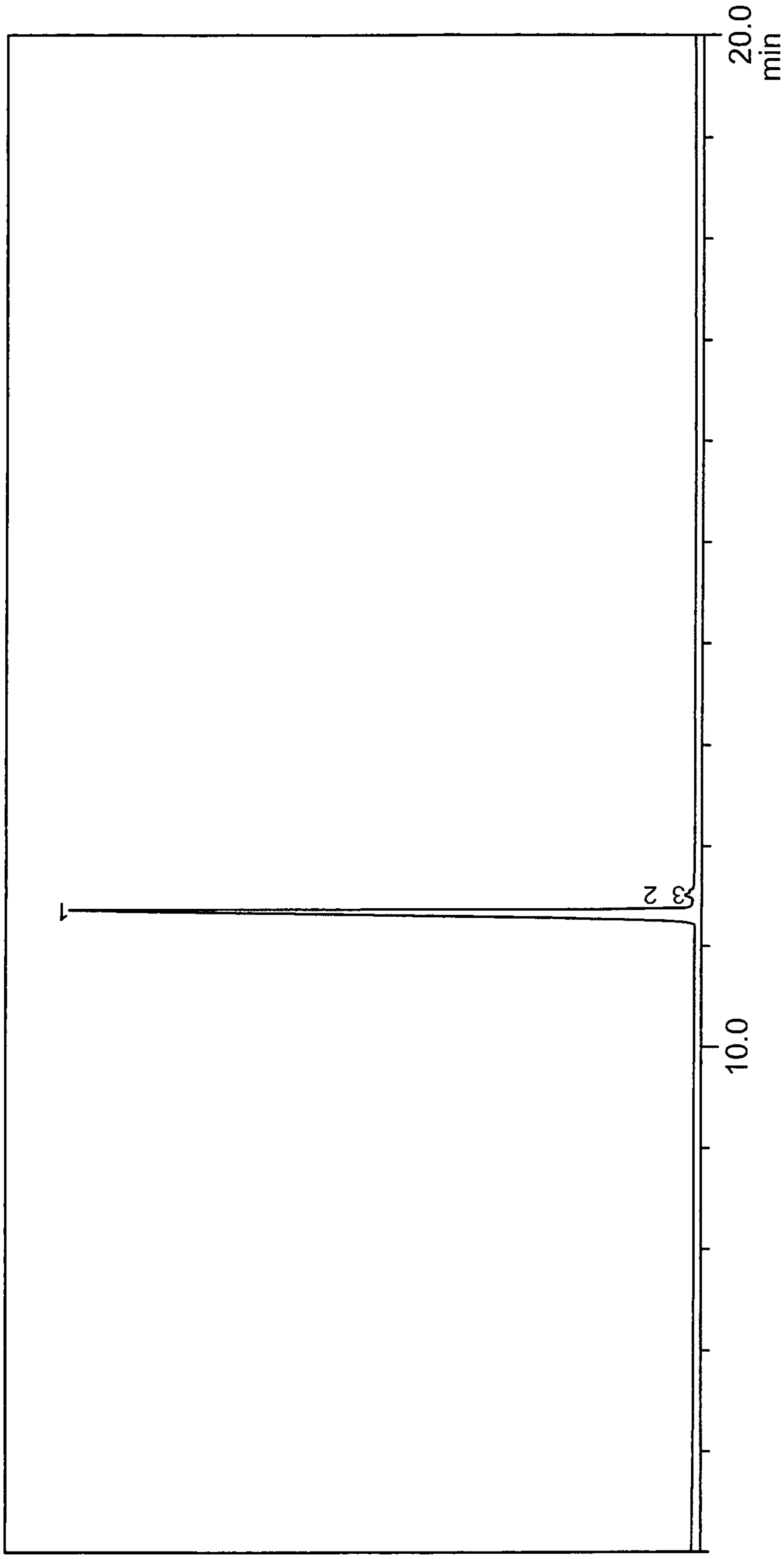


FIG. 114

FIG. 115

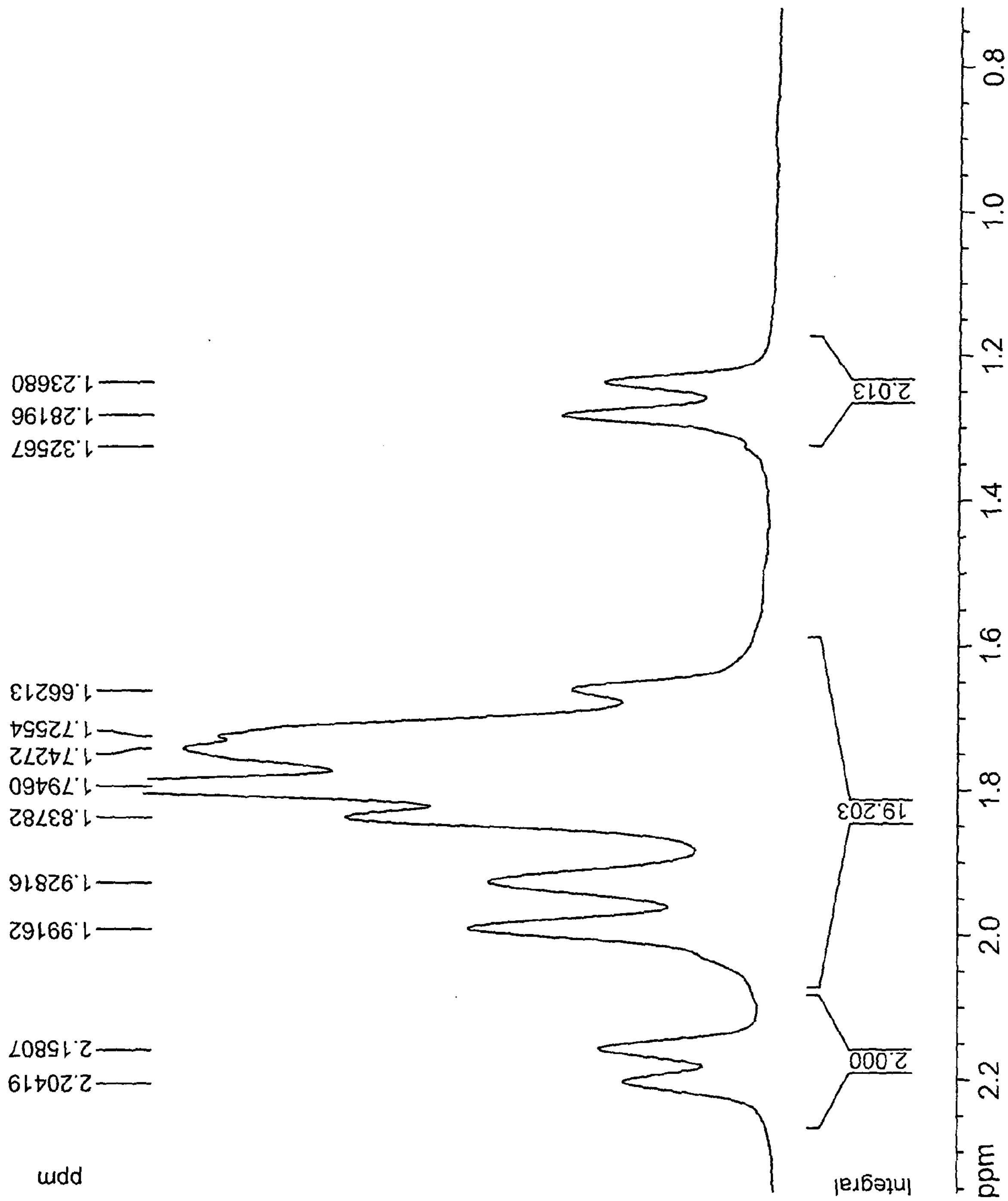

1.23680
1.28196
1.32567
1.66213
1.72554
1.74272
1.79460
1.83782
1.92816
1.99162
2.15807
2.20419
ppm
2.013
19.203
2.000
Integral
0.8
1.0
1.2
1.4
1.6
1.8
2.0
2.2
ppm

FIG. 116

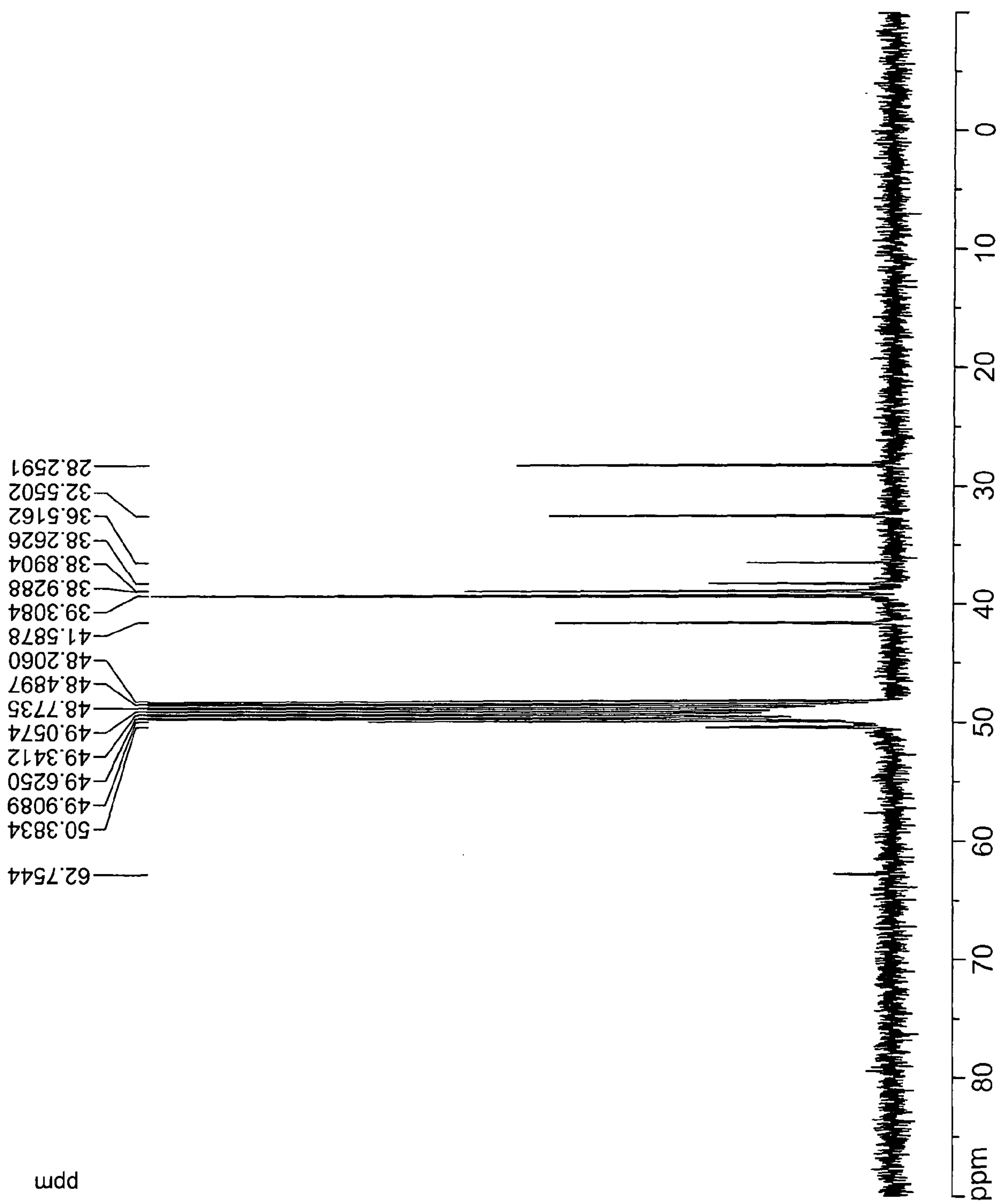
28.2591
32.5502
36.5162
38.2626
38.8904
38.9288
39.3084
41.5878
48.2060
48.4897
48.7735
49.0574
49.3412
49.6250
49.9089
50.3834
62.7544
ppm
0
10
20
30
40
50
60
70
80
ppm

FIG. 117

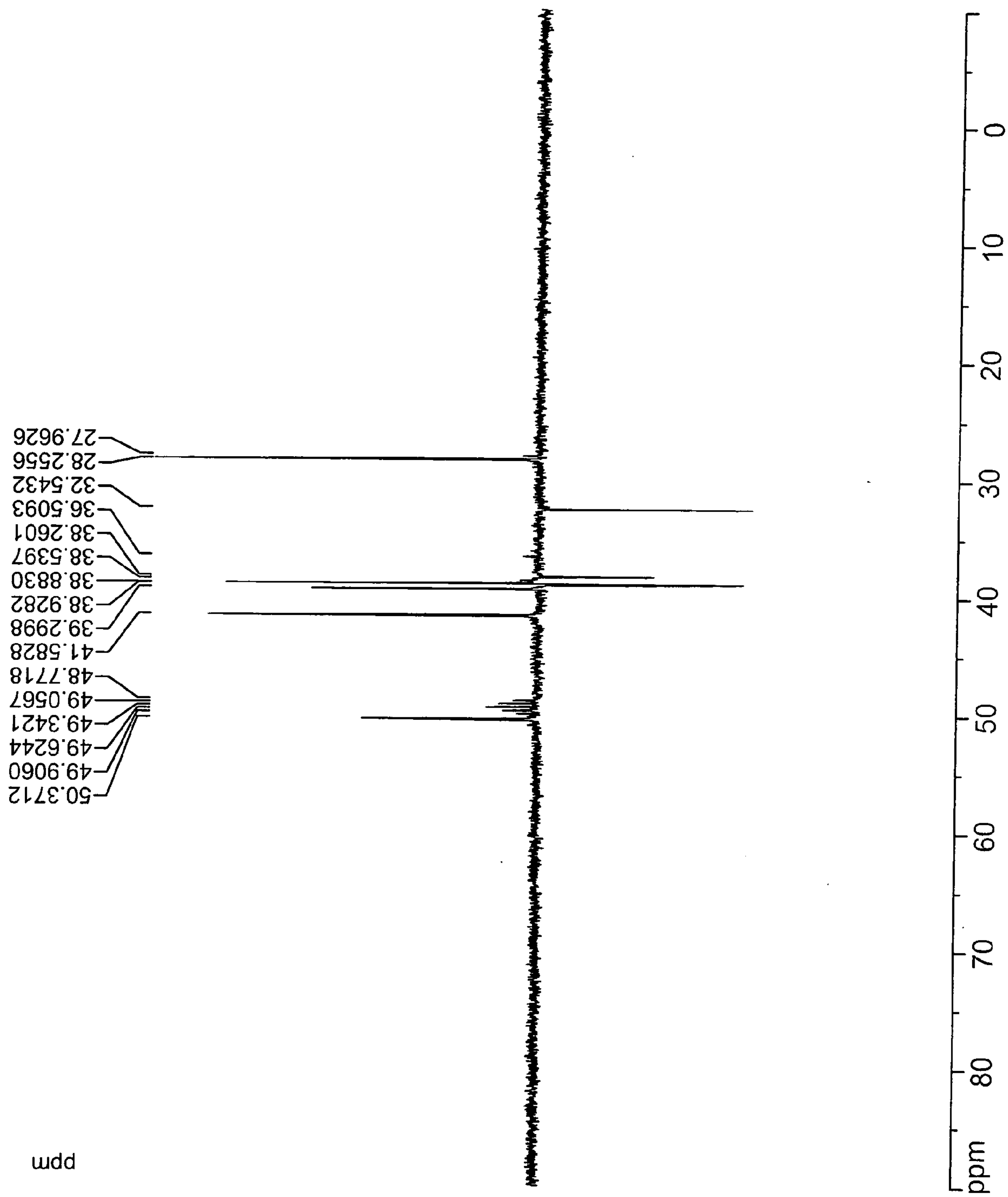
27.9626
28.2556
32.5432
36.5093
38.2601
38.5397
38.8830
38.9282
39.2998
41.5828
48.7718
49.0567
49.3421
49.6244
49.9060
50.3712
ppm
0
10
20
30
40
50
60
70
80
ppm

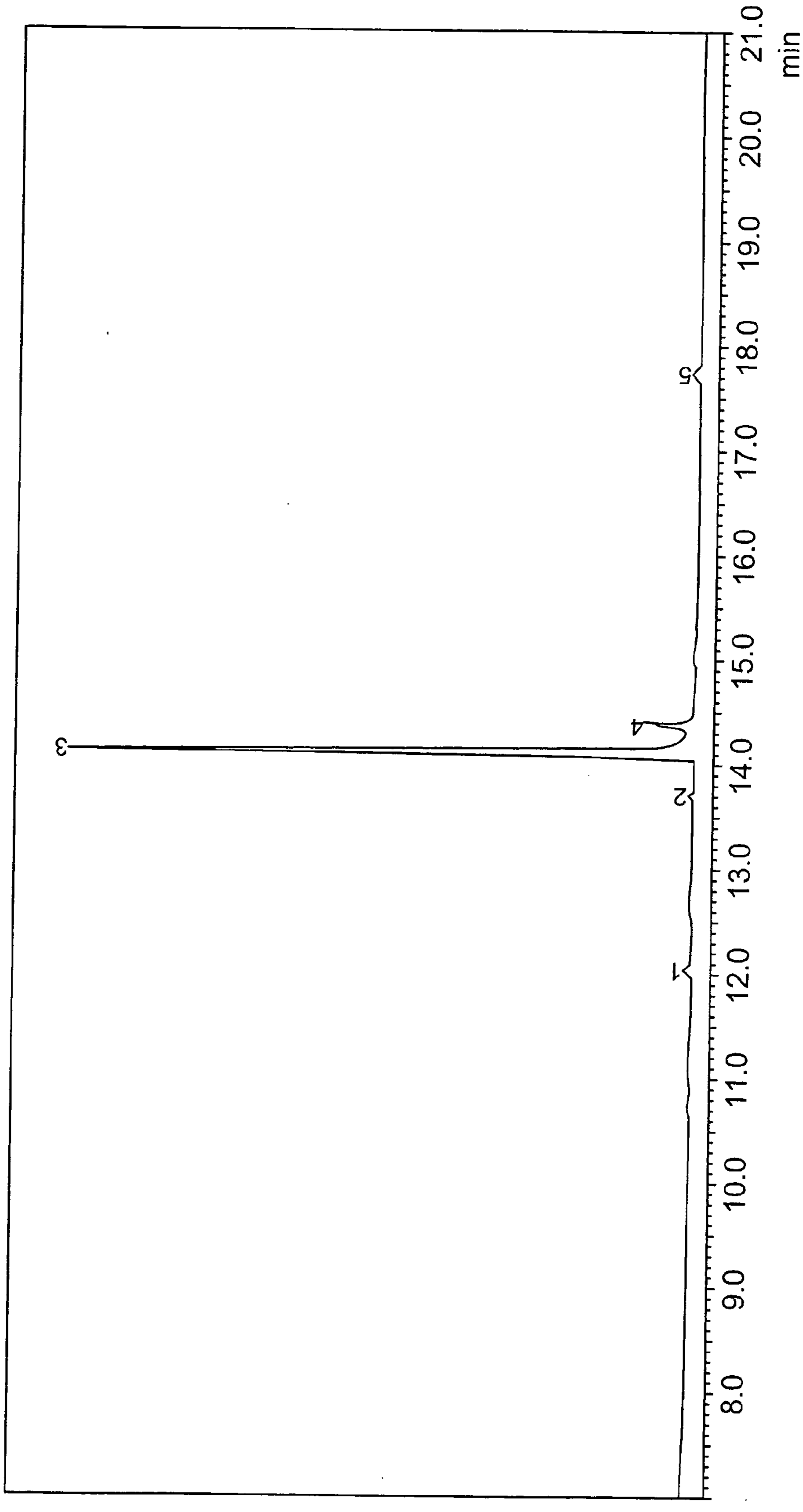


FIG. 118

FIG. 119

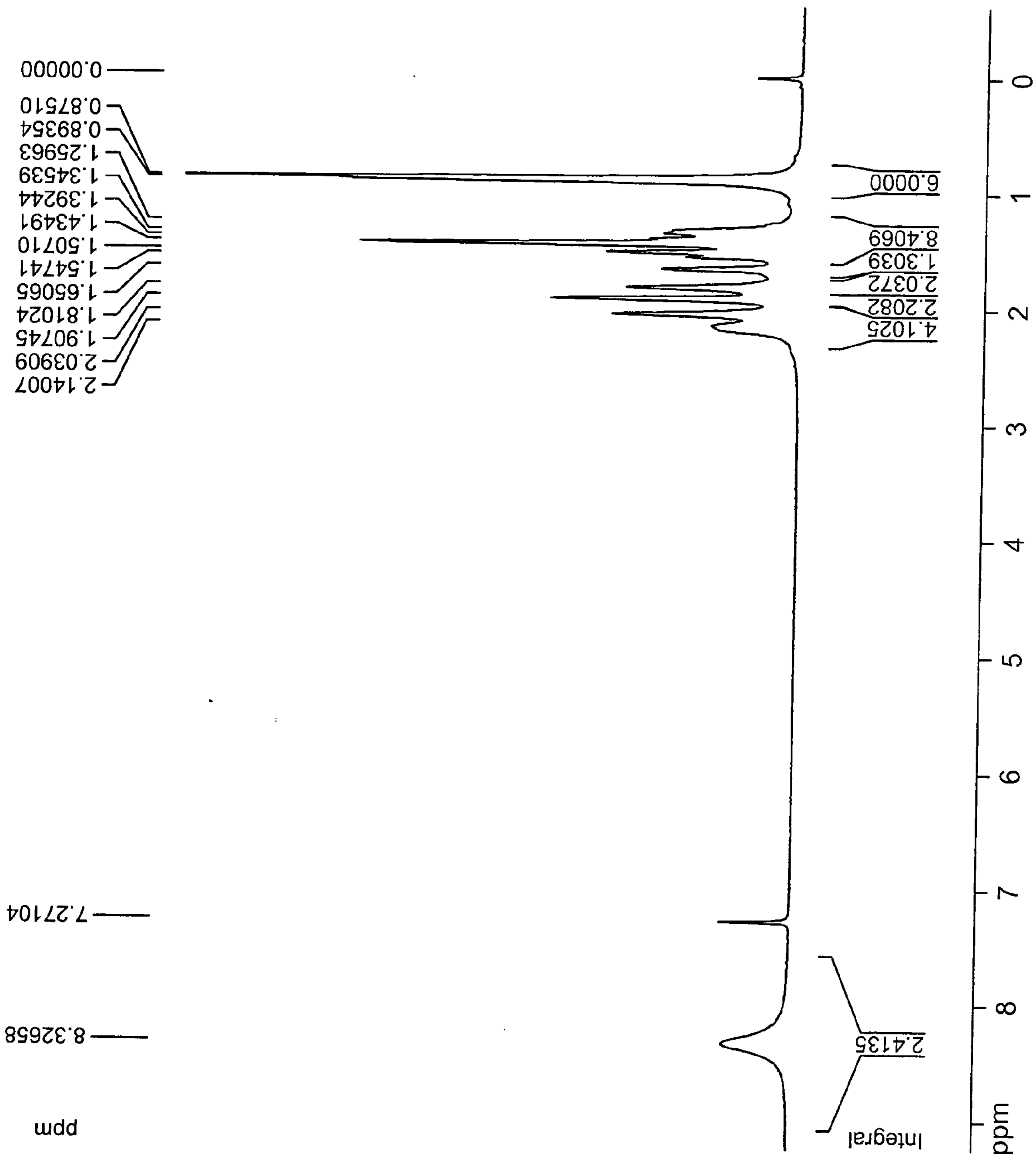
ppm
0.00000
0.87510
0.89354
1.25963
1.34539
1.39244
1.43491
1.50710
1.54741
1.65065
1.81024
1.90745
2.03909
2.14007
7.27104
8.32658
6.0000
8.4069
1.3039
2.0372
2.2082
4.1025
2.4135
Integral
ppm

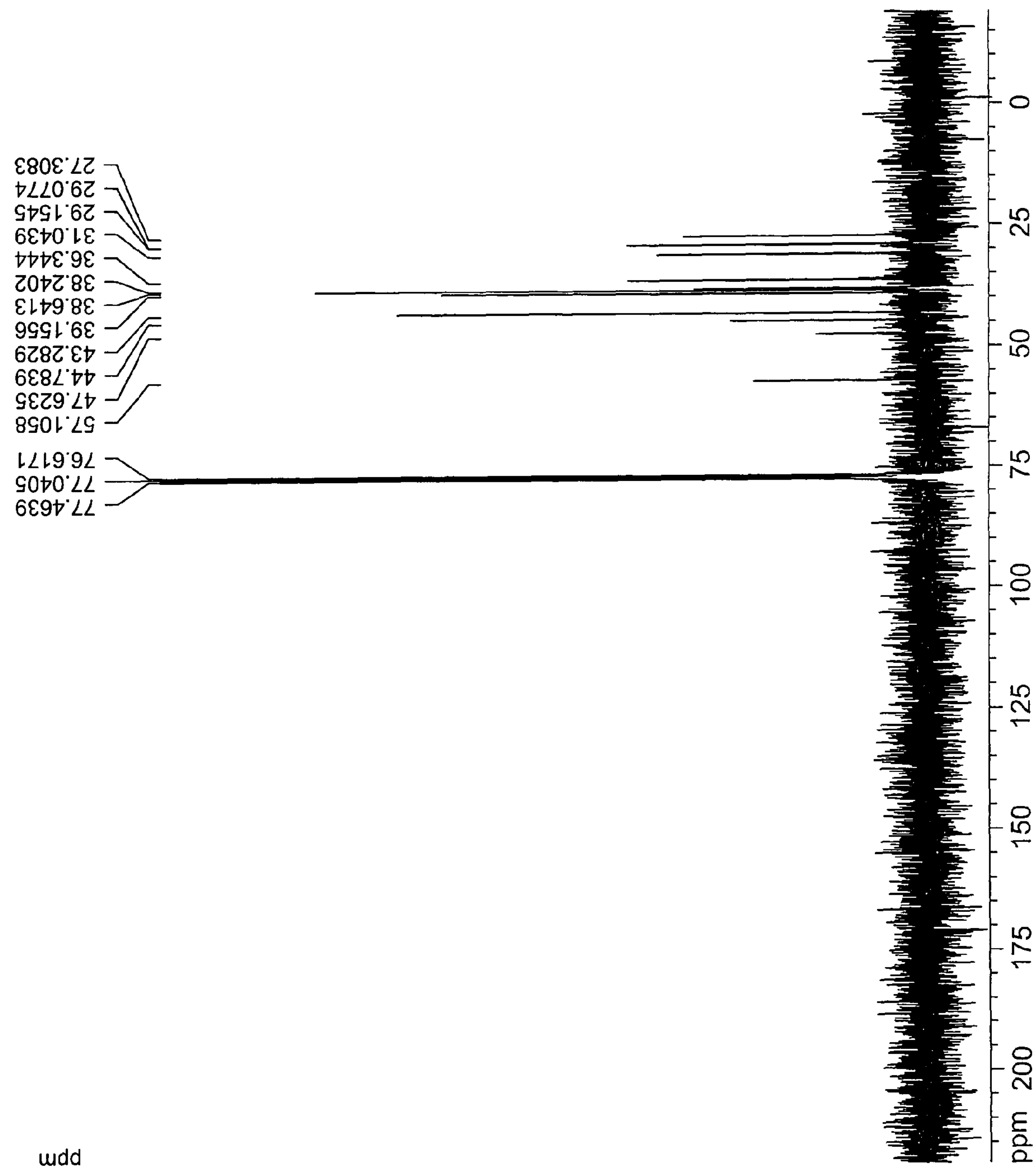
27.3083
29.0774
29.1545
31.0439
36.3444
38.2402
38.6413
39.1556
43.2829
44.7839
47.6235
57.1058
76.6171
77.0405
77.4639
ppm
0
25
50
75
100
125
150
175
200
ppm

FIG. 120

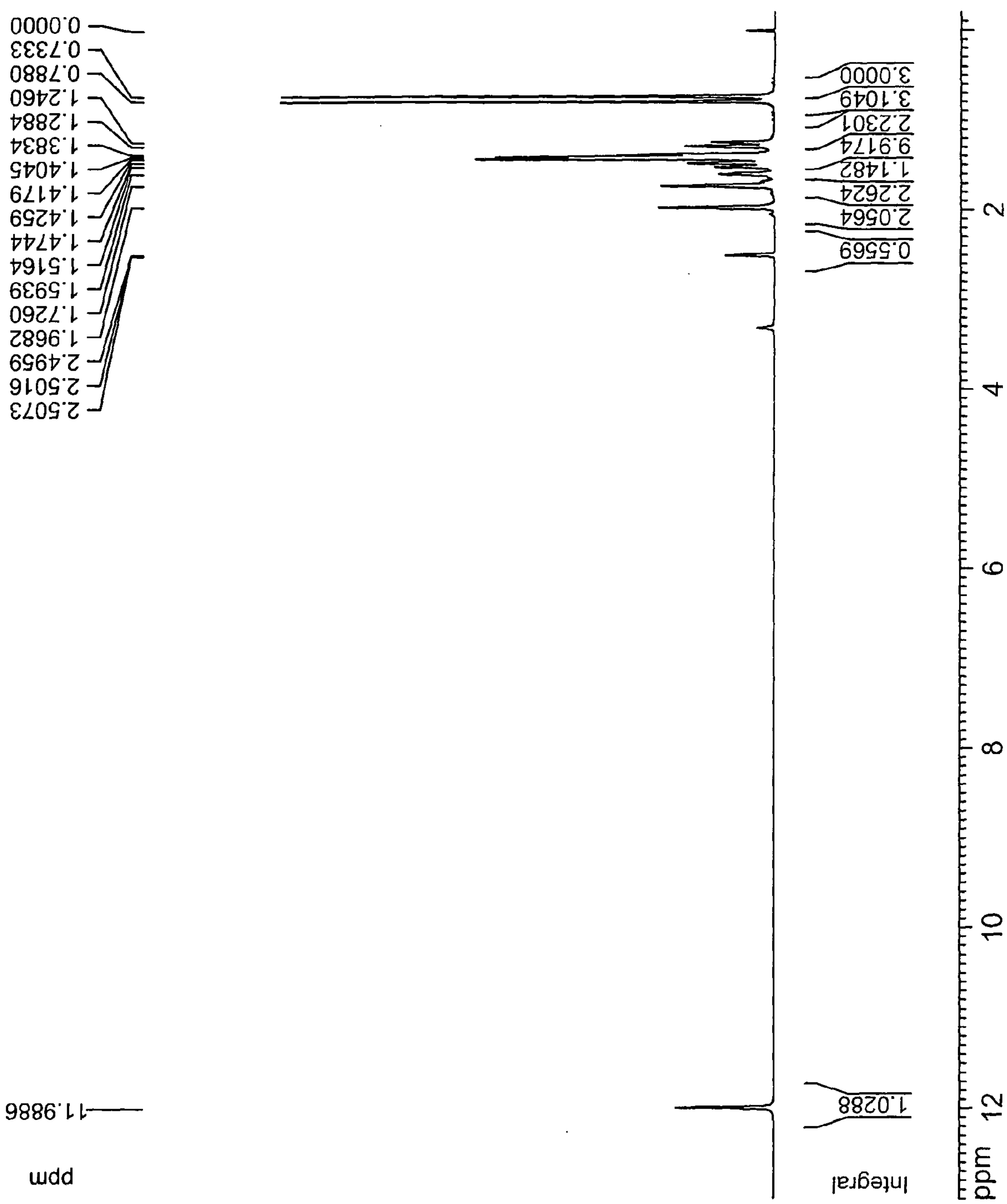
0.0000
0.7333
0.7880
1.2460
1.2884
1.3834
1.4045
1.4179
1.4259
1.4744
1.5164
1.5939
1.7260
1.9682
2.4959
2.5016
2.5073
11.9886
ppm
3.0000
3.1049
2.2301
9.9174
1.1482
2.2624
2.0564
0.5569
1.0288
Integral
2
4
6
8
10
12
ppm

FIG. 121

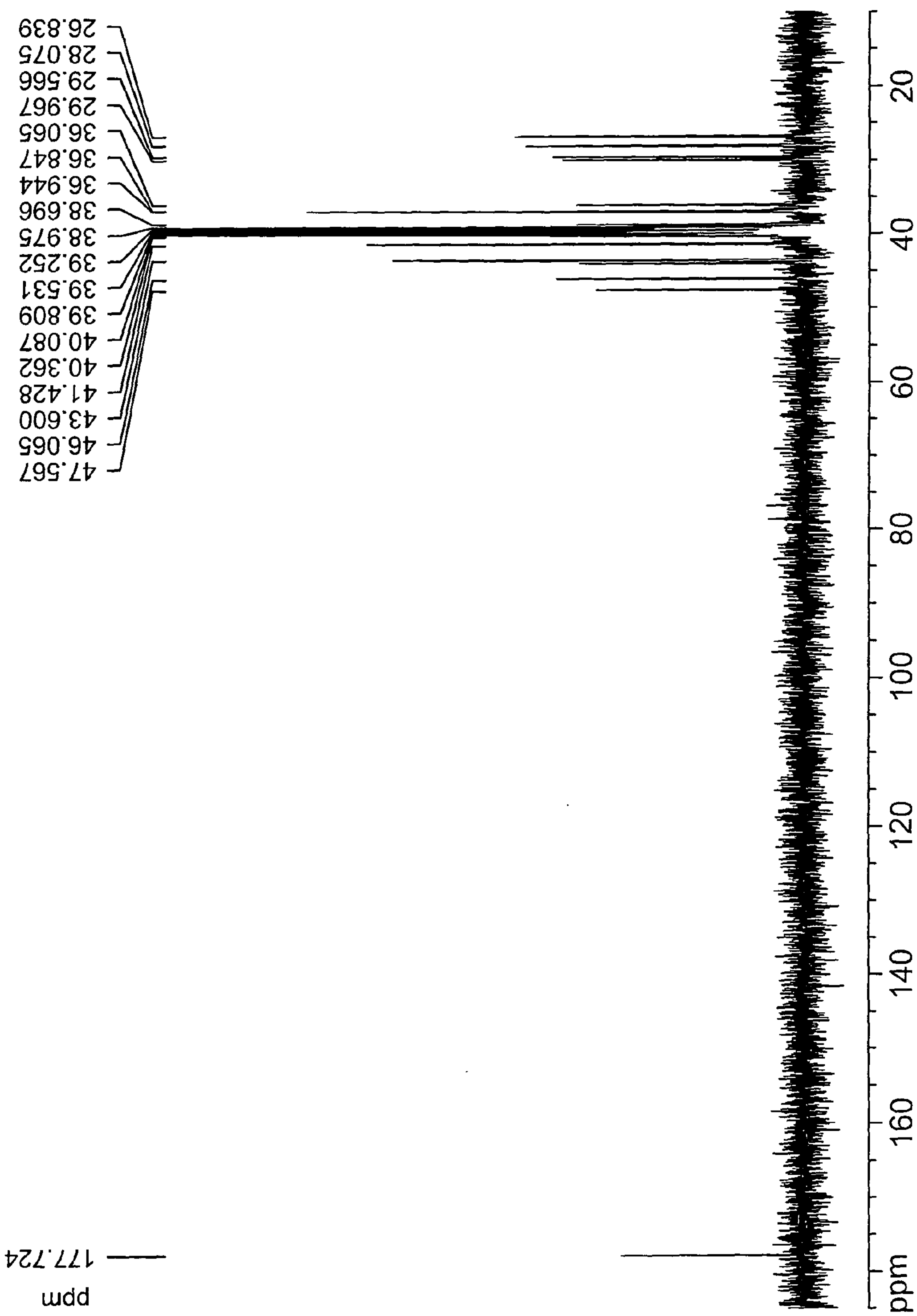
26.839
28.075
29.566
29.967
36.065
36.847
36.944
38.696
38.975
39.252
39.531
39.809
40.087
40.362
41.428
43.600
46.065
47.567
177.724
ppm
20
40
60
80
100
120
140
160
ppm

FIG. 122

FIG. 123

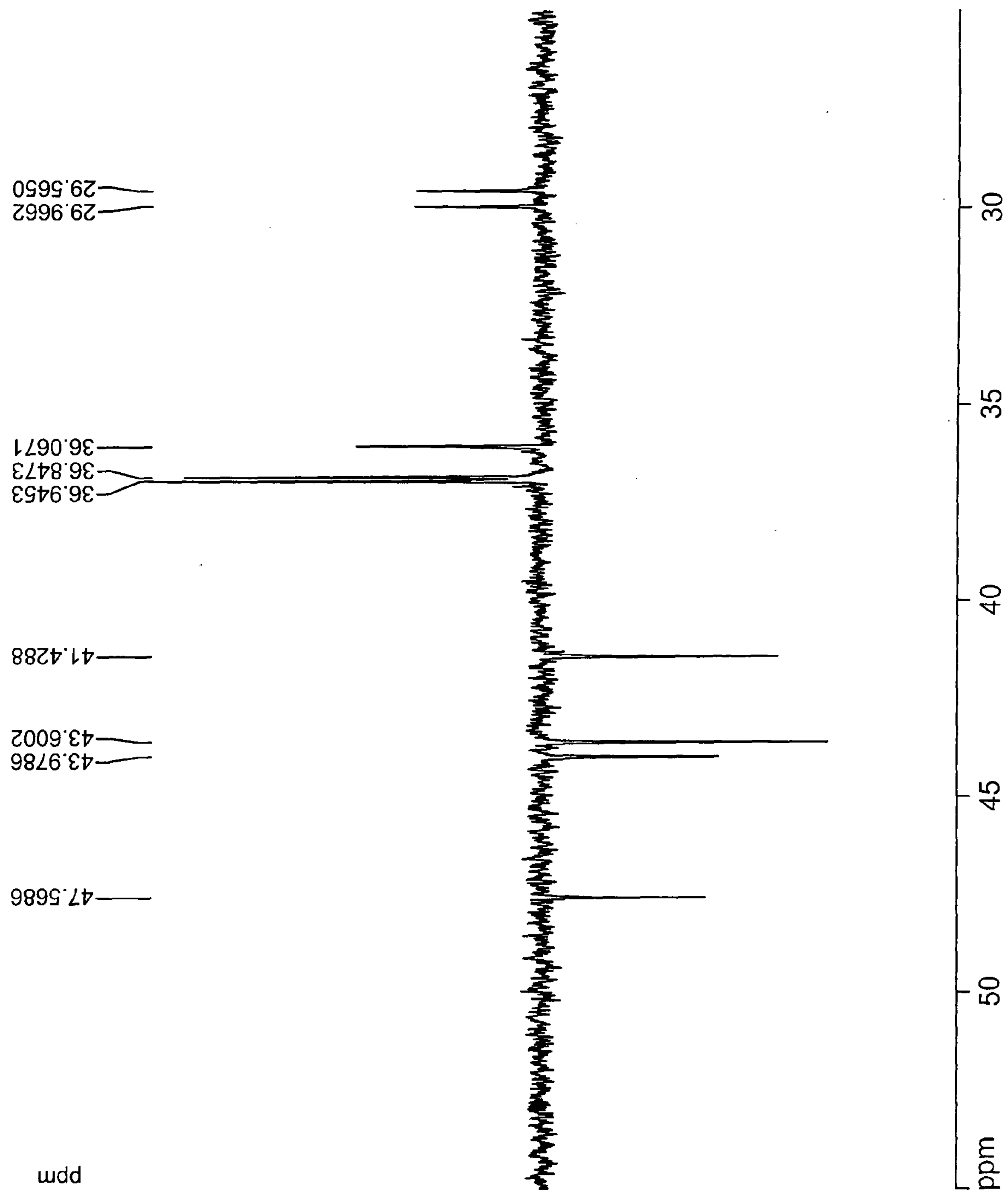
29.5650
29.9662
36.0671
36.8473
36.9453
41.4288
43.6002
43.9786
47.5686
ppm
30
35
40
45
50
ppm

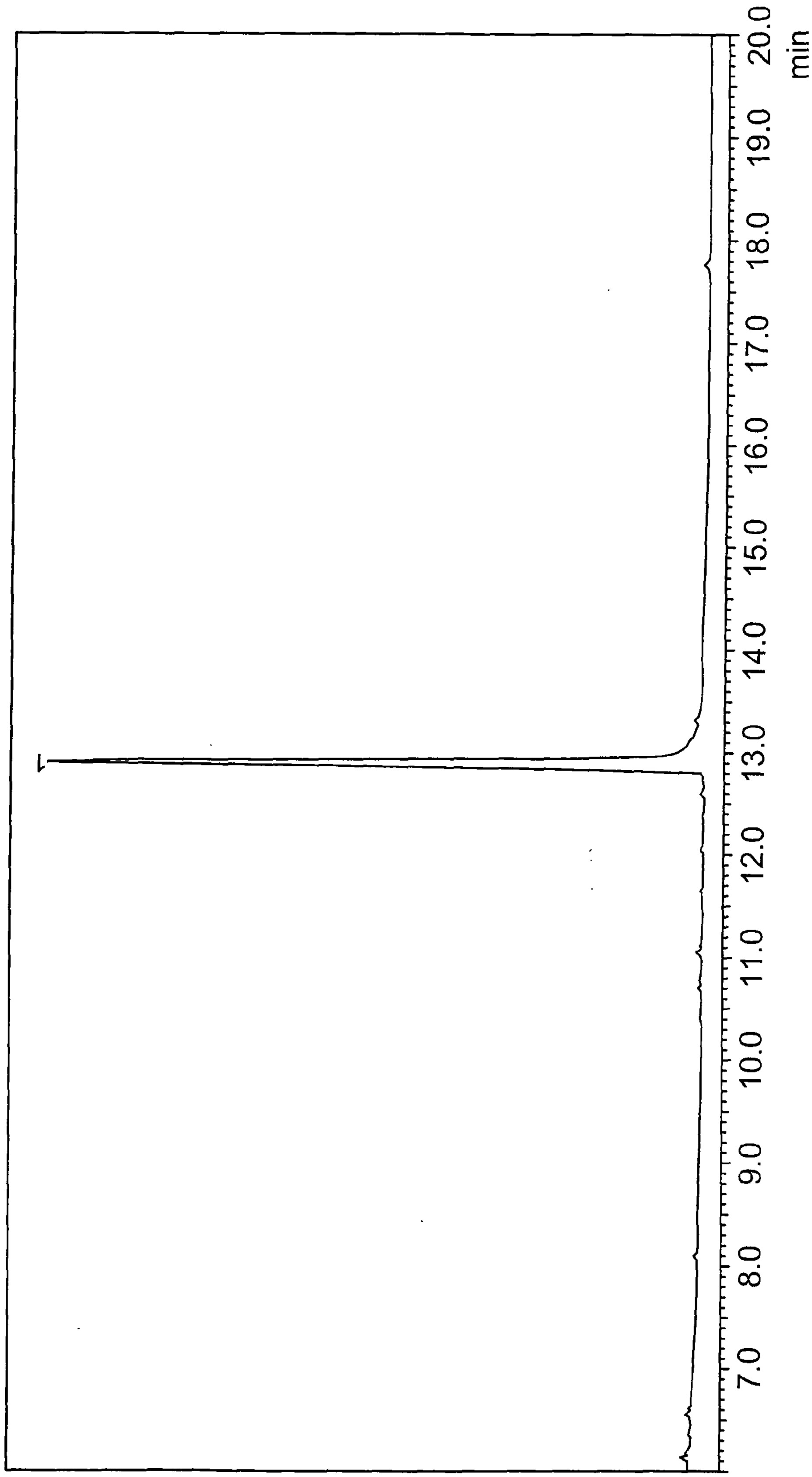


FIG. 124

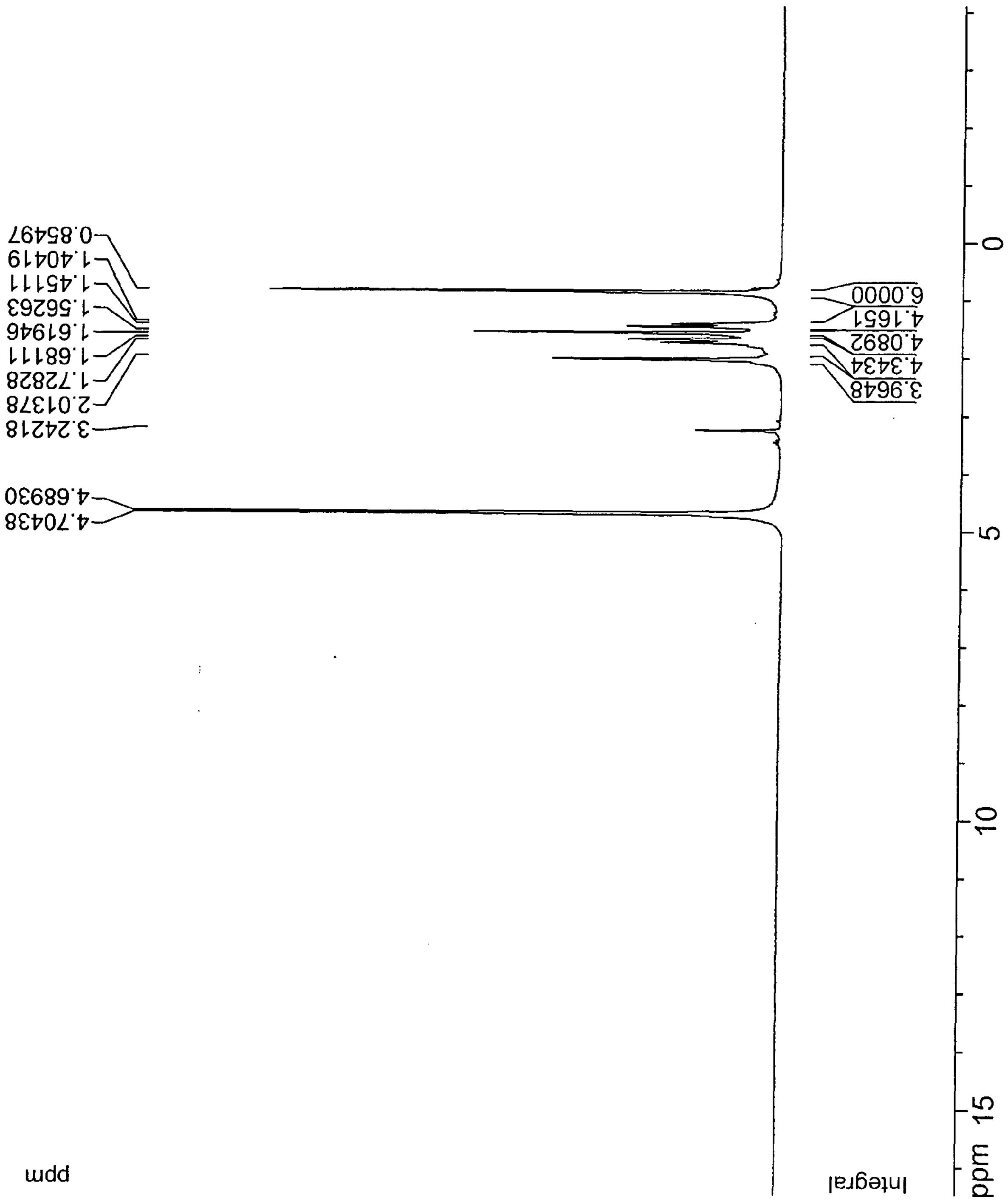
0.85497
1.40419
1.45111
1.56263
1.61946
1.68111
1.72828
2.01378
3.24218
4.68930
4.70438
6.0000
4.1651
4.0892
4.3434
3.9648
0
5
10
15
ppm
Integral
ppm

FIG. 125

FIG. 126

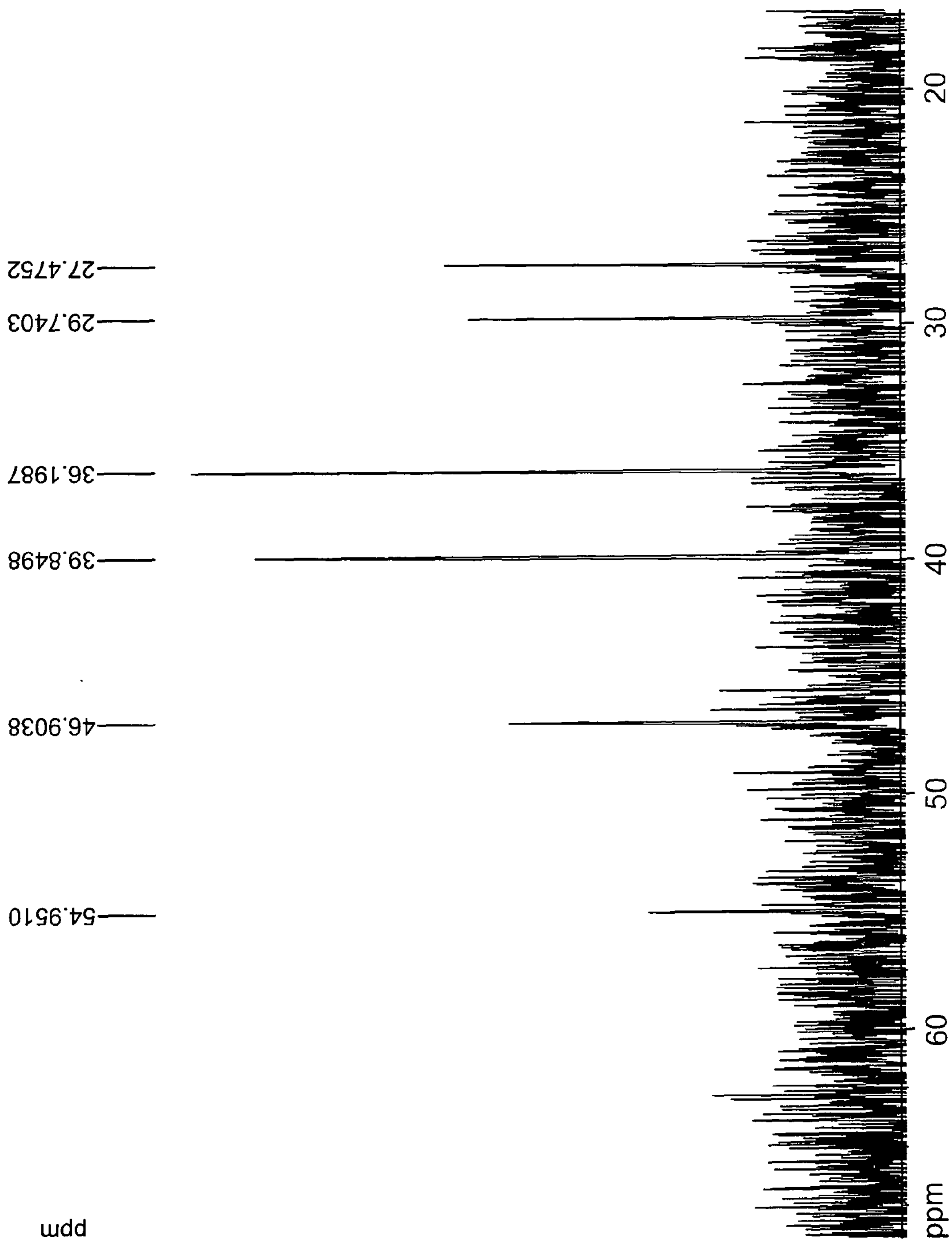
27.4752
29.7403
36.1987
39.8498
46.9038
54.9510
ppm
20
30
40
50
60
ppm

FIG. 127

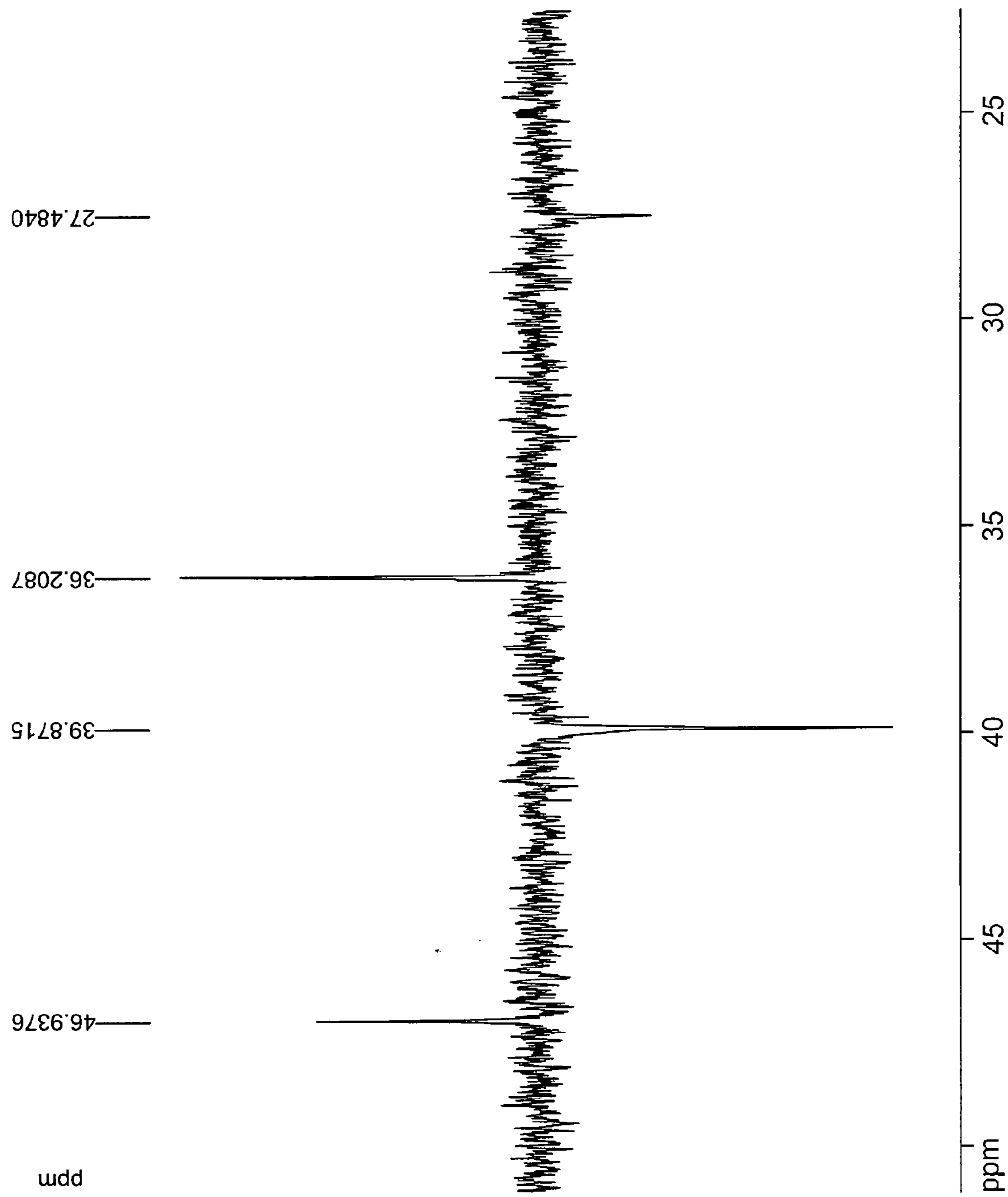

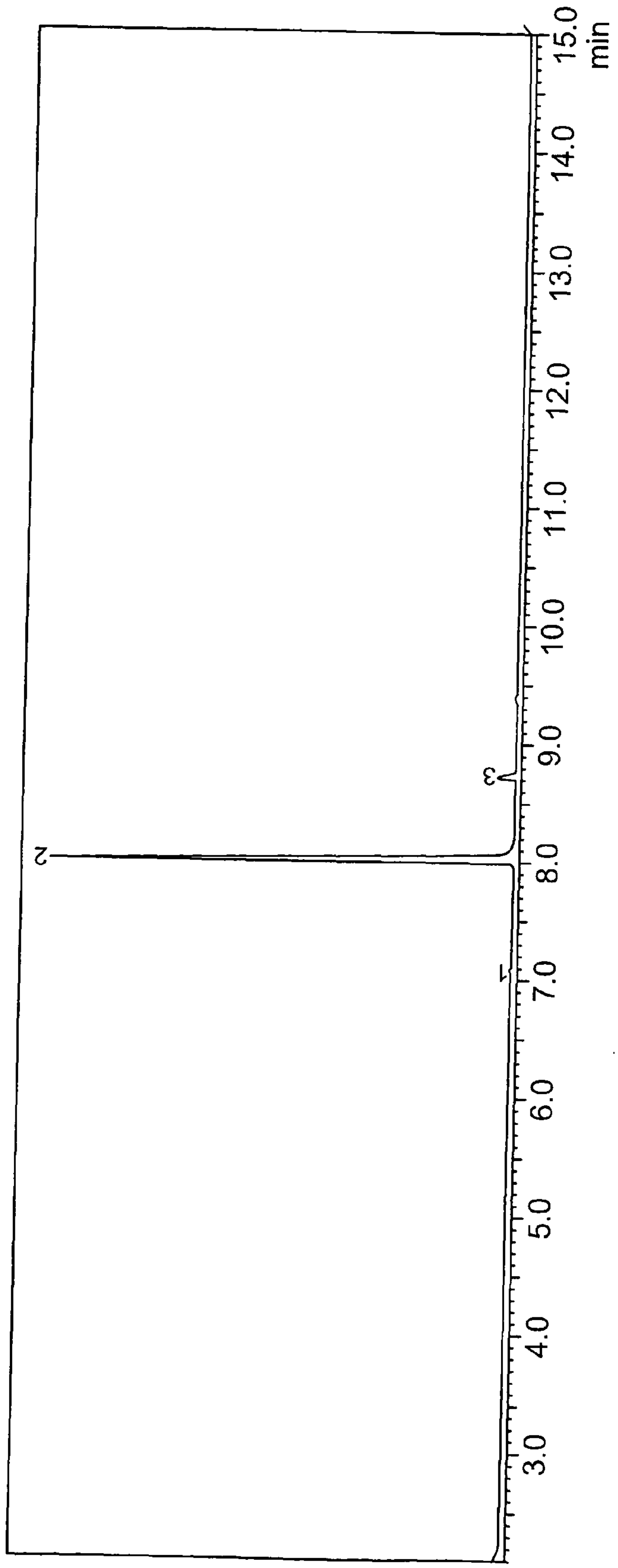


FIG. 128

GC-MS of methylated triamantane mixture precursor

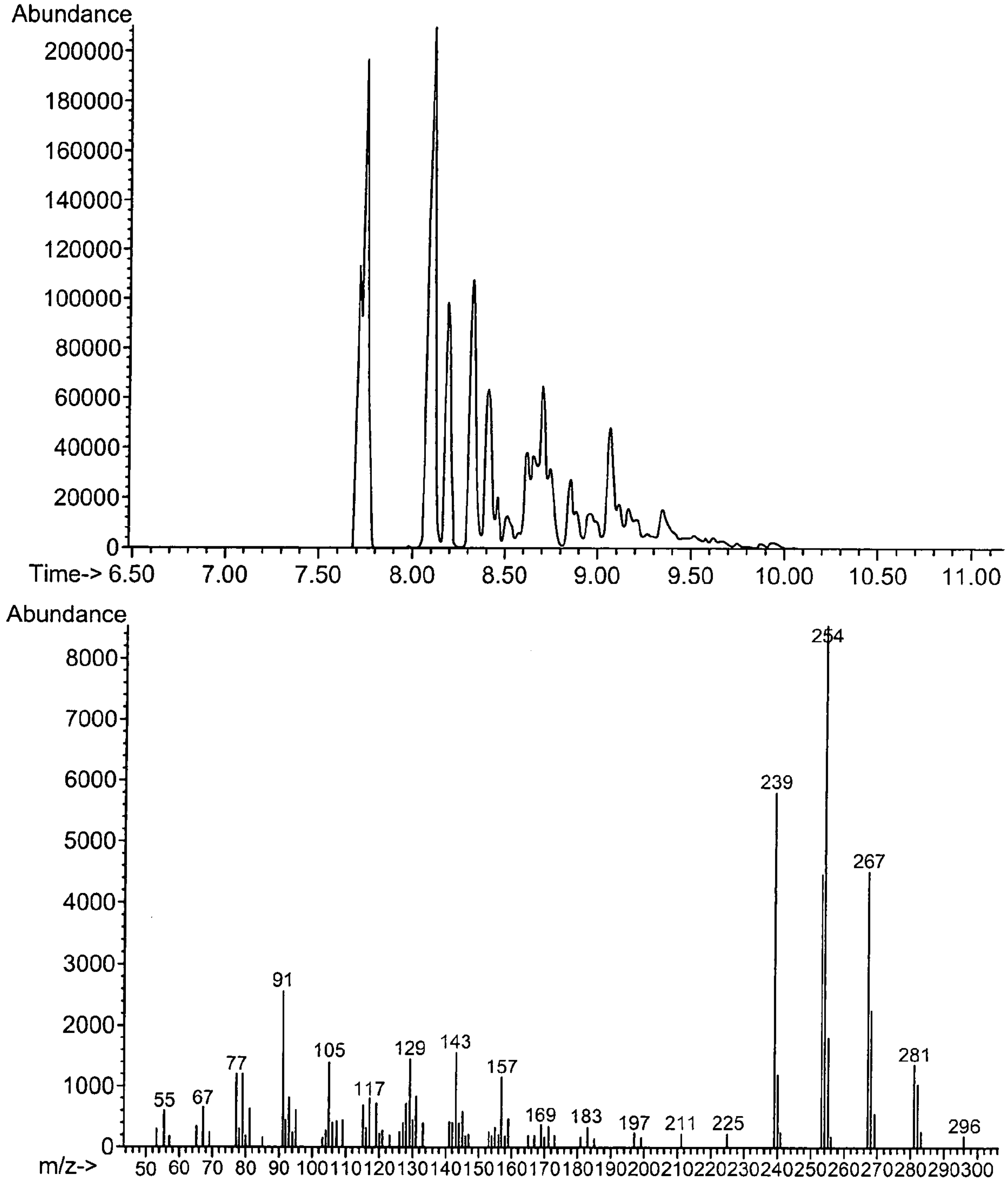


FIG. 129

GC-MS of aminated methylated triamantane mixture (free base) before formation of HCl salt

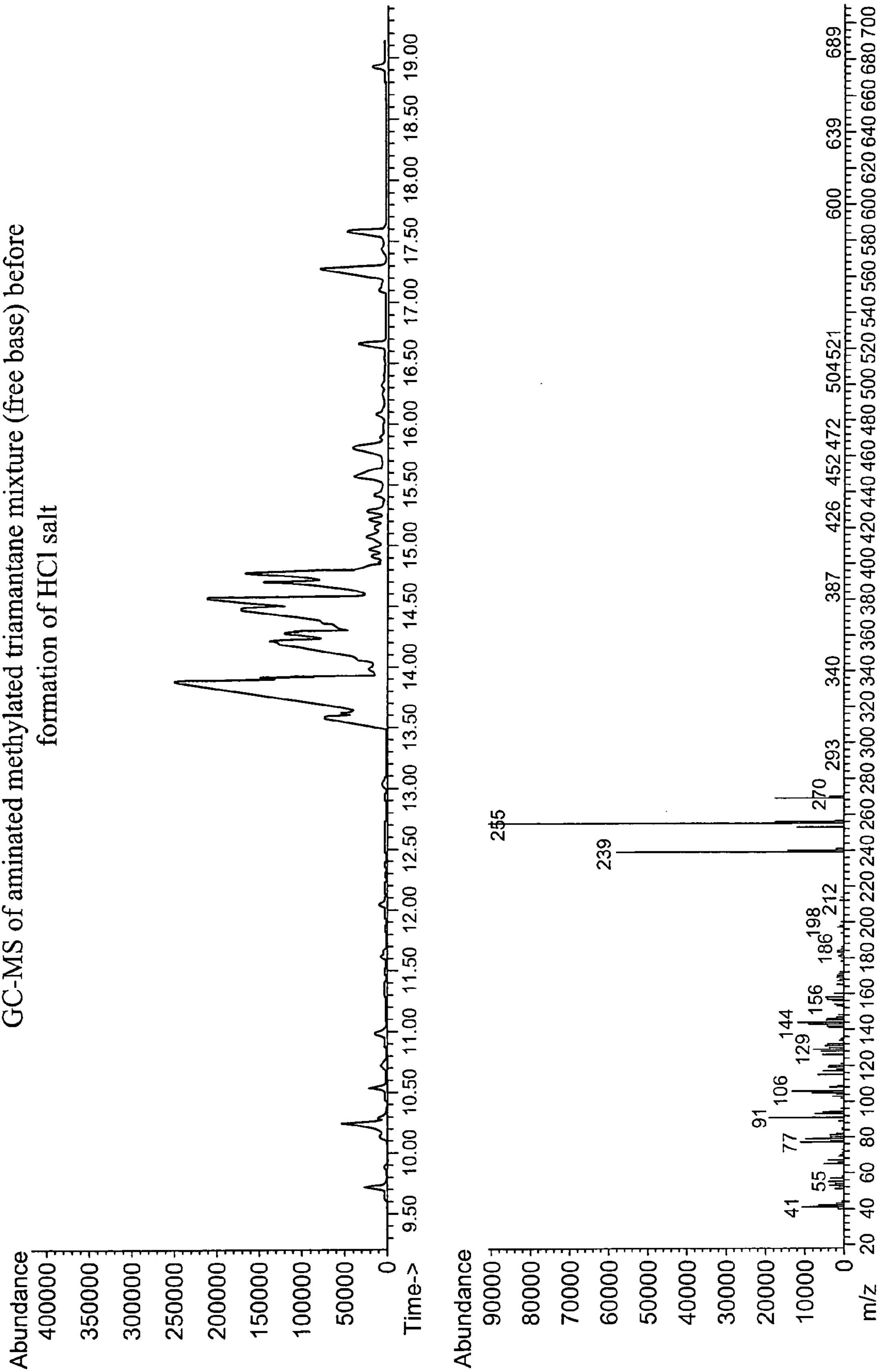


FIG. 130

GC-MS of aminated methylated triamantane mixture after formation of HCl salt then neutralized with ammonium hydroxide to convert into free base

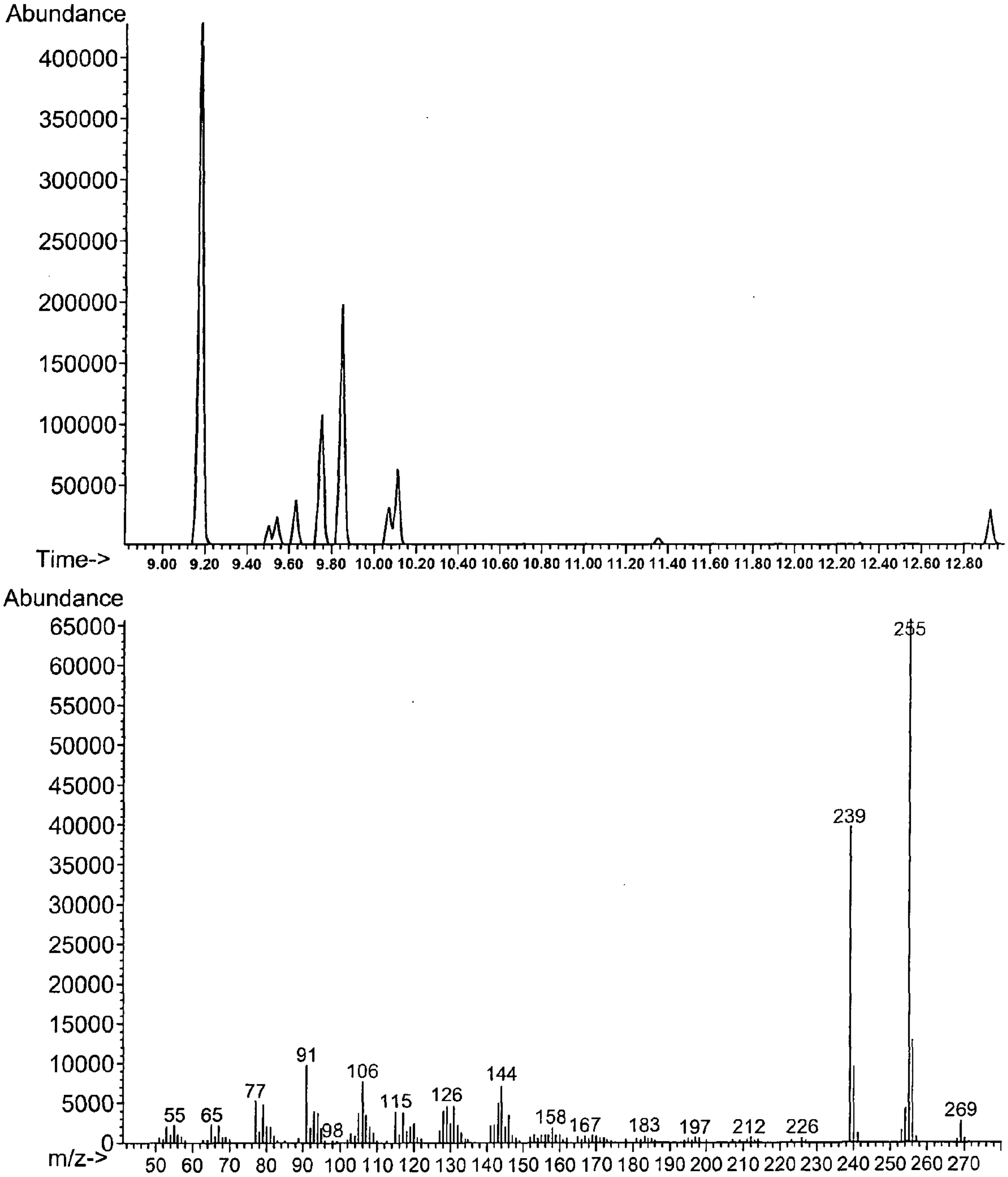


FIG. 131

Figure 132

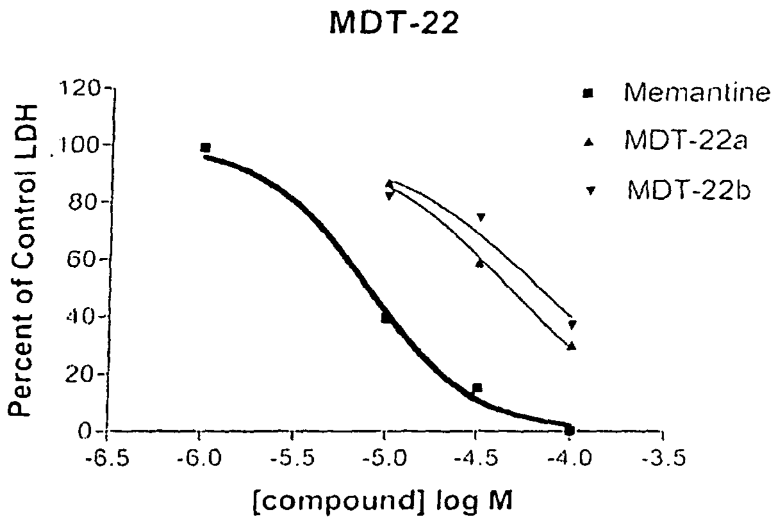


| | Memantine | MDT-22 |
|---|---|---|
| $EC_{50}$ | $7.9 \times 10^{-6}$ | $56.5 \times 10^{-6}$ |

Figure 133

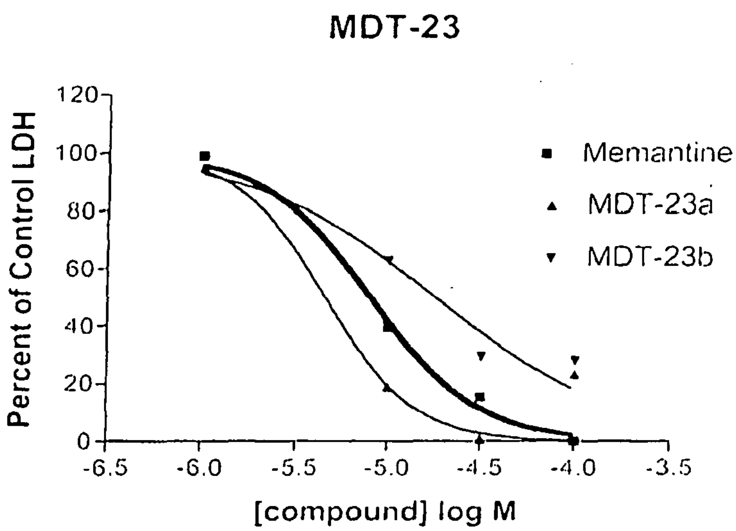


| | Memantine | MDT-23 |
|---|---|---|
| $EC_{50}$ | $7.9 \times 10^{-6}$ | $11.1 \times 10^{-6}$ |

Figure 134

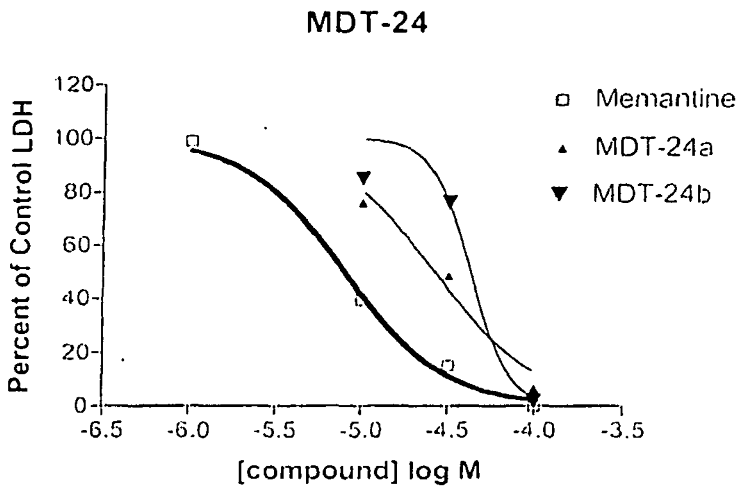


| | Memantine | MDT-24 |
|---|---|---|
| $EC_{50}$ | $7.9 \times 10^{-6}$ | $34.3 \times 10^{-6}$ |

Figure 135

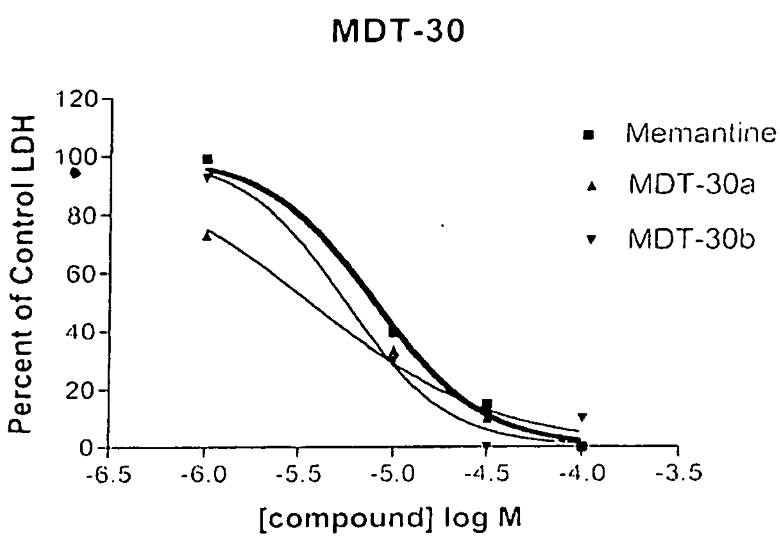


| | Memantine | MDT-30 |
|---|---|---|
| $EC_{50}$ | $7.9 \times 10^{-6}$ | $4.6 \times 10^{-6}$ |

Figure 136

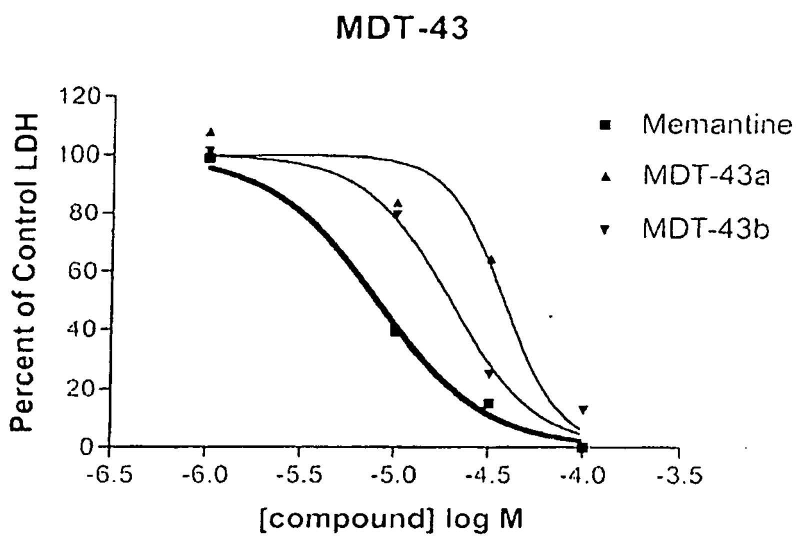


| | Memantine | MDT-43 |
|---|---|---|
| $EC_{50}$ | $7.9 \times 10^{-6}$ | $28.4 \times 10^{-6}$ |

Figure 137

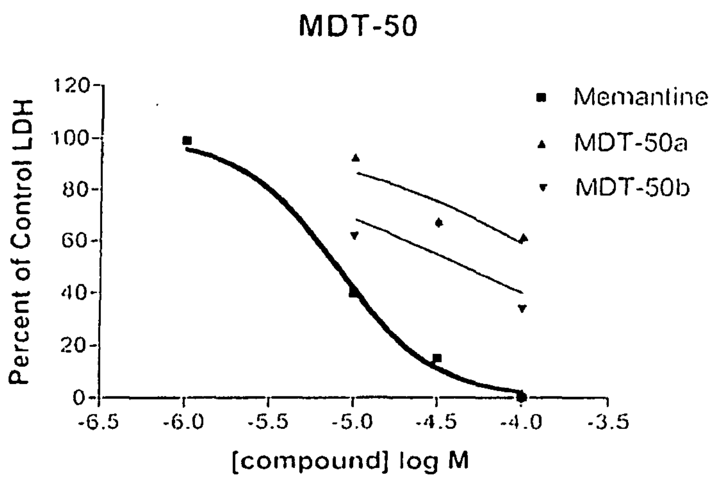


| | Memantine | MDT-50 |
|---|---|---|
| $EC_{50}$ | $7.9 \times 10^{-6}$ | $106.9 \times 10^{-6}$ |

Figure 138

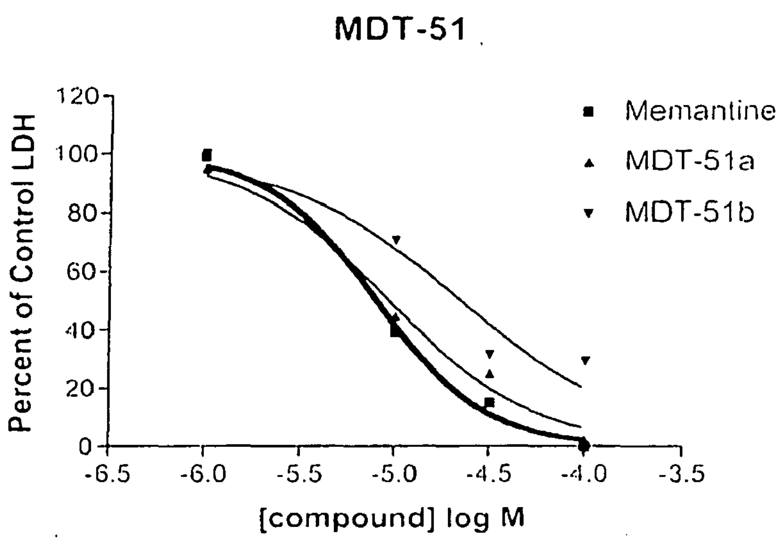


| | Memantine | MDT-51 |
|---|---|---|
| $EC_{50}$ | $7.9 \times 10^{-6}$ | $15.7 \times 10^{-6}$ |

European Patent Office

# EUROPEAN SEARCH REPORT

Application Number
EP 08 25 1149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/122054 A (CHEVRON USA INC [US]; LIU SHENGGAO [US]; LAM FREDERICK W [US]; SCIAMAN) 16 November 2006 (2006-11-16)<br>* pages 1-87; claims 1-70; examples 1-8 * | 1-76 | INV.<br>C07C35/44<br>C07C61/125<br>C07C207/02<br>C07C211/19<br>C07C211/38<br>C07C233/06<br>C07C233/41<br><br>ADD.<br>A61K31/16<br>A61K31/13<br>A61K31/045<br>A61K31/19<br>A61P25/28 |
| X | EP 1 760 057 A (TOKUYAMA CORP [JP]) 7 March 2007 (2007-03-07)<br>* examples 6-11,14-16,23-28 * | 1-3, 38-64, 66-76 | |
| X | VODICKA K ET AL: "Synthesis of diamantanedicarboxylic acids with the carboxy groups bonded at tertiary carbon atoms"<br>COLLECTION OF CZECHOSLOVAK CHEMICAL COMMUNICATIONS, INSTITUTE OF ORGANIC CHEMISTRY & BIOCHEMISTRY, PRAGUE,<br>vol. 48, no. 4,<br>1 January 1983 (1983-01-01), pages 1162-1172, XP009086796<br>ISSN: 0010-0765<br>* figures 1-3 * | 1,2, 38-64, 66-76 | TECHNICAL FIELDS SEARCHED (IPC)<br>C07C<br>A61K<br>A61P |
| X | GUND T M ET AL: "The ionic bromination of diamantane"<br>TETRAHEDRON LETTERS, ELSEVIER, AMSTERDAM,<br>no. 19, 1 January 1971 (1971-01-01), pages 1583-1586, XP002467814<br>ISSN: 0040-4039<br>* compounds III,VII,VIII * | 1-4, 38-64, 66-76 | |
| X | BLANEY F ET AL: "HYDROXYLATION OF DIAMANTAN-1-OL AND DIAMANTAN-4-OL WITH THE FUNGUS RHIZOPUS-NIGRICANS"<br>JOURNAL OF THE CHEMICAL SOCIETY CHEMICAL COMMUNICATIONS, no. 8, 1974, pages 297-298, XP002489885<br>ISSN: 0022-4936<br>* compounds 3,4,6,8 * | 1,2, 38-64, 66-76 | |

The present search report has been drawn up for all claims

1

| Place of search | Date of completion of the search | Examiner |
|---|---|---|
| The Hague | 29 July 2008 | Scheid, Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 08 25 1149

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2008

| Patent document cited in search report | Publication date | Patent family member(s) | Publication date |
|---|---|---|---|
| WO 2006122054 A | 16-11-2006 | CA 2607241 A1 | 16-11-2006 |
| | | EP 1885689 A1 | 13-02-2008 |
| | | KR 20080026096 A | 24-03-2008 |
| EP 1760057 A | 07-03-2007 | WO 2006001398 A1 | 05-01-2006 |
| | | KR 20070028449 A | 12-03-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 43046406 A
- US 67816905 P
- US 78226506 P
- US 5576355 A
- US 4600782 A
- US 3657273 A
- US 5414189 A
- US 6861569 B
- US 3773919 A
- EP 133988 A
- US 5023252 A
- US 5011472 A
- US 4235871 A
- US 4501728 A
- US 4837028 A


### Non-patent literature cited in the description


- HARRISON'S PRINCIPLES OF INTERNAL MEDICINE. McGraw-Hill, Inc, 1994, 2203-2204
- **FORT, JR. et al.** Adamantane: Consequences of the Diamondoid Structure. *Chem. Rev.,* 1964, vol. 64, 277-300
- **MAYER, M. L. ; N. ARMSTRONG.** Structure and function of glutamate receptor ion channels. *Annu Rev Physiol,* 2004, vol. 66, 161-81
- **HERIN, G. A. ; E. AIZENMAN.** Amino terminal domain regulation of NMDA receptor function. *Eur J Pharmacol,* 2004, vol. 500 (1-3), 101-11
- **MAYER, M. L.** Glutamate receptor ion channels. *Curr Opin Neurobiol,* 2005, vol. 15 (3), 282-8
- **KEW, J. N. ; J. A. KEMP.** Ionotropic and metabotropic glutamate receptor structure and pharmacology. *Psychopharmacology(Berl),* 2005, vol. 179 (1), 4-29
- **WATKINS, J. C. ; D. E. JANE.** The glutamate story. *Br J Pharmacol,* 2006, vol. 147 (1), 100-8
- **KALBAUGH, T. L. ; H. M. VANDONGEN et al.** Ligand-binding residues integrate affinity and efficacy in the NMDA receptor. *Mol Pharmacol,* 2004, vol. 66 (2), 209-19
- **CHEN, P. E. ; D. J. WYLLIE.** Pharmacological insights obtained from structure-function studies of ionotropic glutamate receptors. *Br J Pharmacol.,* 2006, vol. 147, 839
- **GIBB, A. J.** NMDA receptor subunit gating--uncovered. *Trends Neurosci,* 2004, vol. 27 (1), 7-10
- **MAGLEBY, K. L.** Modal gating of NMDA receptors. *Trends Neurosci,* 2004, vol. 27 (5), 231-3
- **LIPTON SA.** Paradigm shift in neuroprotection by NMDA receptor blockade: Memantine and beyond. *Nat Rev Drug Disc,* 2006, vol. 5, 160
- **BLEICH, S. ; K. ROMER et al.** Glutamate and the glutamate receptor system: a target for drug action. *Int J Geriatr Psychiatry,* 2003, vol. 18 (1), S33-40
- **TARIOT PN ; FARLOW MR.** Memantine treatment in patients with moderate to severe Alzheimer disease already receiving donepezil: a randomized controlled trial. *JAMA,* 21 January 2004, vol. 291 (3), 317-24
- **FOSTER, A. C. ; J. A. KEMP.** Glutamate- and GABA-based CNS therapeutics. *Curr Opin Pharmacol,* 2006, vol. 6 (1), 7-17
- **MUIR, K. W.** Glutamate-based therapeutic approaches: clinical trials with NMDA antagonists. *Curr Opin Pharmacol,* 2006, vol. 6 (1), 53-60
- **ROGAWSKI, M. A.** Low affinity channel blocking (uncompetitive) NMDA receptor antagonists as therapeutic agents--toward an understanding of their favorable tolerability. *Amino Acids,* 2000, vol. 19 (1), 133-49
- **CALABRESI, P. ; D. CENTONZE et al.** Ionotropic glutamate receptors: still a target for neuroprotection in brain ischemia? Insights from in vitro studies. *Neurobiol Dis,* 2003, vol. 12 (1), 82-8
- **HOYTE, L. ; P. A. BARBER et al.** The rise and fall of NMDA antagonists for ischemic stroke. *Curr Mol Med,* 2004, vol. 4 (2), 131-6
- **JOHNSON, J. W. ; S. E. KOTERMANSKI.** Mechanism of action of memantine. *Curr Opin Pharmacol,* 2006, vol. 6 (1), 61-7
- **LIPTON, S. A.** Paradigm shift in neuroprotection by NMDA receptor blockade: memantine and beyond. *Nat Rev Drug Discov,* 2006, vol. 5 (2), 160-70
- **LEGGE J.** The potential role of ketamine in hospice analgesia: a literature review. *Consult Pharm,* 2006, vol. 21, 51-7

- **YEH CC.** Preincisional dextromethorphan combined with thoracic epidural anesthesia and analgesia improves postoperative pain and bowel function in patients undergoing colonic surgery. *Anesth Analg,* 2005, vol. 100, 1384-9
- **WU CT.** The interaction effect of perioperative cotreatment with dextromethorphan and intravenous lidocaine on pain relief and recovery of bowel function after laparoscopic cholecystectomy. *Anesth Analg,* 2005, vol. 100, 448-53
- **WEINBROUM AA ; BEN-ABRAHAM R.** Dextromethorphan and dexmedetomidine: new agents for the control of perioperative pain. *Eur J Surg.,* 2001, vol. 167, 563-9
- **DUEDAHL, T H.** A qualitative systematic review of perioperative dextromethorphan in post-operative pain. *Acta Anaesthesiol Scand,* 2006, vol. 50, 1-13
- **HEMPENSTALL K.** Analgesic therapy in postherpetic neuralgia: a quantitative systematic review. *PLoS Med,* 2005, vol. 2
- **GALER BS.** MorphiDex (morphine sulfate/dextromethorphan hydrobromide combination) in the treatment of chronic pain: three multicenter, randomized, double-blind, controlled clinical trials fail to demonstrate enhanced opioid analgesia or reduction in tolerance. *Pain,* 2005, vol. 115, 284-95
- Dextromethorphan/quinidine: AVP 923, dextromethorphan/cytochrome P450-2D6 inhibitor, quinidine/dextromethorphan. *Drugs R D.,* 2005, vol. 6 (3), 174-7
- **FILBERT J.** *Med Chem Biol Radiol Def,* 2005, http://jmedhchemdef.org
- *Organic Syntheses,* 1973, vol. 53, 30-34
- *Tetrahedron Letters,* 1970, 3877-3880
- *Journal of the American Chemical Society,* 1965, vol. 87, 4, 917-918
- *Journal of the American Chemical Society,* 1966, vol. 88, 16, 3862-3863
- Fieser's Reagents for Organic Synthesis. John Wiley and Sons, 1991, vol. 1-15
- Rodd's Chemistry of Carbon Compounds. Elsevier Science Publishers, 1989, vol. 1-5
- Organic Reactions. John Wiley and Sons, 1991, vol. 1-40
- March's Advanced Organic Chemistry. John Wiley and Sons
- Larock's Comprehensive Organic Transformations. VCH Publishers Inc, 1989
- **T.W. GREENE ; G.M. WUTS.** Protecting Groups in Organic Synthesis. Wiley, 1991
- Recent developments in the adamantane and related polycyclic hydrocarbons. **R. C. BINGHAM ; P. V. R. SCHLEYER.** Chemistry of Adamantanes. Springer-Verlag, 1971
- **I. K. MOISEEV ; N. V. MAKAROVA ; M. N. ZEMTSOVA.** Reactions of adamantanes in electrophilic media. *Russian Chemical Review,* 1999, vol. 68 (12), 1001-1020
- Cage hydrocarbons. John Wiley & Son, Inc, 1990
- **H. W. GELUK ; V. G. KEISER.** *Organic Synthesis,* 1973, vol. 53, 8
- **ROGAWSKI, M. A.** Low affinity channel blocking (uncompetitive) NMDA receptor antagonists as therapeutic agents-toward an understanding of their favorable tolerability. *Amino Acids,* 2000, vol. 19 (1), 133-49
- **DANYSZ, W. ; C. G. PARSONS et al.** Aminoadamantanes as NMDA receptor antagonists and antiparkinsonian agents--preclinical studies. *Neurosci Biobehav Rev,* 1997, vol. 21 (4), 455-68
- **LIPTON, S. A.** The molecular basis of memantine action in Alzheimer's disease and other neurologic disorders: low-affinity, uncompetitive antagonism. *Curr Alzheimer Res,* 2005, vol. 2 (2), 155-65
- **ROGAWSKI, M. A. ; G. L. WENK.** The neuropharmacological basis for the use of memantine in the treatment of Alzheimer's disease. *CNS Drug Rev,* 2003, vol. 9 (3), 275-308
- **LIPTON, S. A.** Failures and successes of NMDA receptor antagonists: molecular basis for the use of open-channel blockers like memantine in the treatment of acute and chronic neurologic insults. *NeuroRx,* 2004, vol. 1 (1), 101-10
- **ANDERSON ER.** Memantine protects hippocampal neuronal function in muringe human immunodeficiency virus type I encephalitis. *J Neurosci,* 2004, vol. 24, 7194
- **ALISKY, J. M.** Could cholinesterase inhibitors and memantine alleviate HIV dementia?. *J Acquir Immune Defic Syndr,* 2005, vol. 38 (1), 113-4
- **KAUL, M. ; J. ZHENG et al.** HIV-1 infection and AIDS: consequences for the central nervous system. *Cell Death Differ,* 2005, vol. 12 (1), 878-92
- **PARSONS, C. G.** NMDA receptors as targets for drug action in neuropathic pain. *Eur J Pharmacol,* 2001, vol. 429 (1-3), 71-8
- **DANYSZ, W. ; C. G. PARSONS et al.** Amino-alkyl-cyclohexanes as a novel class of uncompetitive NMDA receptor antagonists. *Curr Pharm Des,* 2002, vol. 8 (10), 835-43
- **LOSI G.** Functional in vitro characterization of CR 3394: A novel voltage dependent N-metliyl-D-aspartate (NMDA) receptor antagonist. *Neuropharmacol,* 2006, vol. 50, 277
- **PARSONS.** NMDA receptors as targets for drug action in neuropathic pain. *Eur J Pharmacol,* 2001, vol. 429, 71
- **DANYSZ W ; PARSONS CG.** Neuroprotective potential of ionotrophic glutamate receptor antagonists. *Neurotox Res,* 2002, vol. 4, 119

- **PLANELLS-CASES R.** A novel N-methyl-D-aspartate receptor open channel blocker with in vivo neuroprotectant activity. *J Pharmacol Exp Thera,* 2002, vol. 302, 163
- **BLEICH S.** Glutamate and the glutamate receptor system: a target for drug action. *Int J Geriatr Psychiatry,* 2003, vol. 18, S33
- **MUIR KW.** Glutamate-based therapeutic approaches: clinical trials with NMDA antagonists. *Curr Opin Pharmacol,* 2006, vol. 6, 53
- **JOHNSON JW ; KOTERMANSKI SE.** Mechanism of action of memantine. *Cur Opin Pharmacol,* 2006, vol. 6, 61
- **CHEN N.** Site within N-methyl-D-aspartate receptor pore modulates channel gating. *Mol Pharmacol,* 2004, vol. 65, 157
- **YUAN H.** Conserved structural and functional control of N-methyl-d-aspartate receptor gating by transmembrane domain M3. *J Biol Chem,* 2005, vol. 280, 29708
- **THOMAS CG et al.** Probing N-methyl-D-aspartate receptor desensitization with the substituted-cysteine accessibility method. *Mol Pharmacol.,* April 2006, vol. 69 (4), 1296-303
- **SOBOLEVSKY A ; KOSHELEV.** Two blocking sites of amino-adamantane derivatives in open N-methyl-d-aspartate channels. *Biophysical J,* 1998, vol. 74, 1305
- **KASHIWAGI K.** Channel blockers acting at N-methyl-D-aspartate receptors: Differential effects of mutation in the vestibule and ion channel pore. *Mol Pharmacol,* 2002, vol. 61, 533
- **CHEN H-S ; LIPTON SA.** Pharmacological implications of two distinct mechanism of interaction of memantine with N-methyl-d-aspartate-gated channels. *J Pharmacol Exp Thera,* 2005, vol. 314, 961
- **BOLSHAKOV KV.** Design of antagonists for NMDA and AMPA receptors. *Neuropharmacol,* 2005, vol. 42, 144
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547-556
- **PETER R. SCHREINER et al.** *J. Org. Chem.,* 2006, vol. 71 (18), 6709-6720
- **DINGLEDINE, R. ; N. W. KLECKNER ; C. J. MCBAIN.** The glycine coagonist site of the NMDA receptor. *Adv. Exp. Med. Biol.,* 1990, vol. 268, 17-26
- **LODGE D. ; N.A. ANIS.** Effects of phencyclidine on excitatory amino acid activation of spinal interneurones in the cat. *Eur. J. Pharmacol.,* 1982, vol. 77, 203-204
- **MOTHET, J. P. ; A. T. PARENT ; H. WOLOSKER ; R. O. BRADY ; D. J. LINDEN ; C. D. FERRIS ; M. A. ROGAWSKI ; S. H. SNYDER.** D-serine is an endogenous ligand for the glycine site of the N-methyl-D-aspartate receptor. *Proc. Natl. Acad. Sci. USA,* 2000, vol. 97, 4926-4931
- **EVANS, J.G. ; G. WILCOCK ; J. BIRKS.** Evidence-based pharmacotherapy of Alzheimer's disease. *Int J Neuropsychopharmacol,* 2004, vol. 7 (3), 351-69
- **MAHANT, N. et al.** Huntington's disease: clinical correlates of disability and progression. *Neurology,* 2003, vol. 61 (8), 1085-92
- **HENRY, J.D. ; J.R. CRAWFORD.** Verbal fluency deficits in Parkinson's disease: a meta-analysis. *J Int Neuropsychol Soc,* 2004, vol. 10 (4), 608-22
- **DOWNES, J.J. et al.** Impaired extra-dimensional shift performance in medicated and unmedicated Parkinson's disease: evidence for a specific attentional dysfunction. *Neuropsychologia,* 1989, vol. 27 (11-12), 1329-43
- **LAWRENCE, A.D. et al.** Visual object and visuospatial cognition in Huntington's disease: implications for information processing in corticostriatal circuits. *Brain,* 2000, vol. 123 (7), 1349-64
- **FARLOW, M.R.** NMDA receptor antagonists. A new therapeutic approach for Alzheimer's disease. *Geriatrics,* 2004, vol. 59 (6), 22-7
- **LIPTON SA.** Paradigm shift in neuroprotection by NMDA receptor blockade: memantine and beyond. *Nat Rev Drug Discov.,* February 2006, vol. 2, 160-70
- **XIE, X. ; CROWDER, T.L. ; YAMANAKA, A. ; MORAIRTY, S.R. ; LEWINTER, R.D. ; SAKURAI, T. ; T.S. LILDUFF.** GABAB receptor-mediated modulation of hypocretin/orexin neurones in mouse hypothalamus. *J Physiol,* 2006
- **MELDRUM B. ; GARTHWAITE J.** Excitatory amino acid neurotoxicity and neurodegenerative disease. *Trends Pharm Sci,* 1990, vol. 11, 379-387
- **WOODRUFF GN ; FOSTER AC ; GILL R ; KEMP JA ; WONG EH ; IVERSEN LL.** The interaction between MK-801 and receptors for N-methyl-D-aspartate: functional consequences. *Neuropharmacology,* July 1987, vol. 26 (7B), 903-9
- **WONG EH ; KEMP JA ; PRIESTLEY T ; KNIGHT AR ; WOODRUFF GN ; IVERSEN LL.** The anticonvulsant MK-801 is a potent N-methyl-D-aspartate antagonist. *Proc Natl Acad Sci U S A,* September 1986, vol. 83 (18), 7104-8
- **REISBERG B. et al.** Memantine in moderate-to-severe Alzheimer's disease. *New Eng Jour Medicine,* 2003, vol. 348, 1333-1341
- Cytotoxicity in Murine Neocortical Cell Culture. **ROSE K ; GOLDBERG MP ; CHOI DW.** Methods in Toxicology. Academic Press, 1993, 46-60

- **LOSI G ; LANZA M ; MAKOVEC F ; ARTUSI R ; CASELLI G ; PUIA G.** Functional in vitro characterization of CR 3394: A novel voltage dependent N-methyl-D-aspartate (NMDA) receptor antagonist. *Neuropharmacology,* 2006, vol. 50, 277-285
- **LIPTON et al.** *J Physiol.,* 1987, vol. 385, 361
- **HAHN et al.** *Proc. Natl. Acad. Sci. USA,* 1988, vol. 85, 6556
- **LEVY et al.** *Neurology,* 1990, vol. 40, 852
- **LEVY et al.** *Neurosci. Lett.,* 1990, vol. 110, 291
- **GRYNKIEWICZ et al.** *J. Biol. Chem.,* 1985, vol. 260, 3440
- **WILLIAMS et al.** *Nature,* 1985, vol. 318, 558
- **CONNOR et al.** *J. Neurosci.,* 1987, vol. 7, 1384
- **CONNOR et al.** *Science,* 1988, vol. 240, 649
- **COHAN et al.** *J. Neurosci.,* 1987, vol. 7, 3588
- **MATTSON et al.** *J.NEUROSCI,* 1989, vol. 9, 3728
- **SMITH et al.** *Acta Neurol. Scand.,* 1984, vol. 69, 385-401
- **P. A. CAHILL.** *Tetra. Lett.,* 1990, vol. 31 (38), 5417-5420
- **P. KOVACIC ; C. T. GORALSKI ; J. J. HILLER ; J. A. LEVISKY ; R. M. LANGE.** *J. Amer. Chem. Soc.,* 1965, vol. 87 (6), 1262-1266
- **P. KOVACIC ; P. D. ROSKOS.** *J. Amer. Chem. Soc.,* 1969, vol. 91 (23), 6457-6460
- **P. KOVACIC. ; P. D. ROSKOS.** *Tetra. Lett.,* 1968, vol. 56, 5833-5835